(19) Europäisches Patentamt
European Patent Office
Office européen des brevets

(11) EP 3 644 999 B1

(12) **EUROPEAN PATENT SPECIFICATION**

(45) Date of publication and mention of the grant of the patent:
**14.12.2022 Bulletin 2022/50**

(21) Application number: **18823719.2**

(22) Date of filing: **29.06.2018**

(51) International Patent Classification (IPC):
***A61K 9/00*** (2006.01) ***A61K 9/19*** (2006.01)
***A61K 47/12*** (2006.01) ***A61K 47/20*** (2006.01)
***A61K 31/454*** (2006.01) ***A61K 39/395*** (2006.01)
***A61K 47/69*** (2017.01) ***C08B 37/16*** (2006.01)
***C08L 5/16*** (2006.01) ***A61P 35/02*** (2006.01)
***A61K 47/40*** (2006.01)

(52) Cooperative Patent Classification (CPC):
**A61K 31/454; A61K 9/0019; A61K 9/19; A61K 47/12; A61K 47/20; A61K 47/40; A61K 47/6951; A61P 35/02; C08B 37/0015; C08L 5/16**

(86) International application number:
**PCT/US2018/040286**

(87) International publication number:
**WO 2019/006299 (03.01.2019 Gazette 2019/01)**

(54) **COMPOSITIONS AND METHODS OF USE OF 2-(4-CHLOROPHENYL)-N-((2-(2,6-DIOXOPIPERIDIN-3-YL)-1-OXOISOINDOLIN-5-YL) METHYL) -2,2-DIFLUOROACETAMIDE**

ZUSAMMENSETZUNGEN UND VERFAHREN ZUR VERWENDUNG VON 2-(4-CHLORPHENYL)-N-((2-(2,6-DIOXOPIPERIDIN-3-YL)-1-OXOISOINDOLIN-5-YL) METHYL)-2,2-DIFLUORACETAMID

COMPOSITIONS ET METHODES D'UTILISATION DE 2-(4-CHLOROPHÉNYL)-N-((2-(2,6-DIOXOPIPÉRIDIN-3-YL)-1-OXOISOINDOLIN-5-YL)MÉTHYL)-2, 2-DIFLUOROACÉTAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 US 201762527744 P**
**05.04.2018 US 201862653436 P**
**17.05.2018 US 201862673064 P**

(43) Date of publication of application:
**06.05.2020 Bulletin 2020/19**

(73) Proprietor: **Celgene Corporation**
**Summit, NJ 07901 (US)**

(72) Inventors:
- **BUCHHOLZ, Tonia, J.**
**Moss Beach**
**CA 94038 (US)**
- **CARMICHAEL, James**
**Edwalton**
**NG12 4AL (GB)**
- **CARRANCIO, Soraya**
**San Diego**
**CA 92122 (US)**
- **FAN, Jinhong**
**San Mateo**
**CA 94403 (US)**
- **GUPTA, Rajan**
**Bridgewater**
**NJ 08807 (US)**
- **LU, Gang**
**San Diego**
**CA 92130 (US)**
- **MACBETH, Kyle**
**San Francisco**
**CA 94103 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PACE, Emily**
  **Orinda**
  **CA 94563 (US)**
- **PIERCE, Daniel**
  **Belmont**
  **CA 94002 (US)**
- **POURDEHNAD, Michael**
  **San Francisco**
  **CA 94114 (US)**
- **PU, Yu**
  **East Hanover**
  **NJ 07936 (US)**
- **WANG, Peng**
  **Westfield**
  **NJ 07090 (US)**
- **WU, Naijun**
  **Princeton**
  **NJ 08540 (US)**
- **YAO, Sheena**
  **San Francisco**
  **CA 94122 (US)**

(74) Representative: **Jones Day**
**Rechtsanwälte, Attorneys-at-Law, Patentanwälte**
**Prinzregentenstrasse 11**
**80538 München (DE)**



(56) References cited:
**WO-A1-2016/007848** **WO-A1-2017/120437**
**US-A- 5 571 534** **US-A1- 2017 007 590**
**US-A1- 2017 197 934**

- **Loftsson Thorsteinn ET AL: "Cyclodextrins in drug delivery (Review) UNGAP -COST Action 16205 View project Cyclon Hit View project Cyclodextrins in drug delivery", Expert Opin. Drug Deliv, 1 January 2005 (2005-01-01), pages 335-351, XP055816518, DOI: 10.1517/17425247.2.1.335.Source: Retrieved from the Internet: URL:https://www.researchgate.net/profile/Thorsteinn-Loftsson/publication/7471382_Cyclodextrins_in_drug_delivery_Review/links/59f2e9b0aca272cdc7d033c4/Cyclodextrins-in-drug-delivery-Review.pdf [retrieved on 2021-06-22]**
- **YOKOO et al.: "2-Hydroxypropyl-beta-Cyclodextrin Acts as a Novel Anticancer Agent", PLOS ONE, vol. 10, no. 11, 4 November 2015 (2015-11-04), pages 1-20, XP055320893,**

## Description

### RELATED APPLICATIONS

This application claims the benefit of U.S. provisional application nos. 62/527,744, filed June 30, 2017, 62/653,436, filed April 5, 2018, and 62/673,064, filed May 17, 2018.

### FIELD

Provided are formulations and dosage forms of 2-(4-chlorophenyl)-N-((2-(2,6-dioxopiperidin-3-yl)-1-oxoisoindolin-5-yl)methyl)-2,2-difluoroacetamide or a stereoisomer or a mixture of stereoisomers, pharmaceutically acceptable salt, tautomer, solvate, hydrate, co-crystal, clathrate, or polymorph thereof. Dosage forms for treating, managing, and/or preventing cancer are also provided herein. Thus, provided herein are said formulations and dosage forms for use in methods of treating, managing, and/or preventing cancer.

Also provided is a combination of 2-(4-chlorophenyl)-N-((2-(2,6-dioxopiperidin-3-yl)-1-oxoisoindolin-5-yl)methyl)-2,2-difluoroacetamide or a stereoisomer or a mixture of stereoisomers, an isotopologue, pharmaceutically acceptable salt, tautomer, solvate, hydrate, co-crystal, clathrate, or polymorph thereof and a second agent for use in treating, preventing, managing, and/or ameliorating a cancer. Thus, provided herein is a combination of 2-(4-chlorophenyl)-N-((2-(2,6-dioxopiperidin-3-yl)-1-oxoisoindolin-5-yl)methyl)-2,2-difluoroacetamide or a stereoisomer or a mixture of stereoisomers, an isotopologue, pharmaceutically acceptable salt, tautomer, solvate, hydrate, co-crystal, clathrate, or polymorph thereof and a second agent for use in such methods.

### BACKGROUND

2-(4-Chlorophenyl)-N-((2-(2,6-dioxopiperidin-3-yl)-1-oxoisoindolin-5-yl)methyl)-2,2-difluoroacetamide or a stereoisomer or mixture of stereoisomers, pharmaceutically acceptable salt, tautomer, prodrug, solvate, hydrate, co-crystal, clathrate, or polymorph thereof has been shown to have anti-cancer activities. Exemplary formulations of the compound are disclosed in U.S. Publication No. 2017-0196847, filed on January 6, 2017.

There is a need for further methods and formulations of 2-(4-chlorophenyl)-N-((2-(2,6-dioxopiperidin-3-yl)-1-oxoisoindolin-5-yl)methyl)-2,2-difluoroacetamide or a stereoisomer or mixture of stereoisomers, pharmaceutically acceptable salt, tautomer, prodrug, solvate, hydrate, co-crystal, clathrate, or polymorph thereof for treatment of cancer.

### BRIEF SUMMARY

Compound 1 used in the formulations and methods herein is described in U.S. Patent No. 9,499,514 and International Publication No. WO 2016/007848. In one embodiment, Compound 1 is polymorph Form A, Form B, Form C, Form D, Form E or an amorphous form of 2-(4-chlorophenyl)-N-((2-(2,6-dioxopiperidin-3-yl)-1-oxoisoindolin-5-yl)methyl)-2,2-difluoroacetamide. In one embodiment, Compound 1 is polymorph Form C of 2-(4-chlorophenyl)-N-((2-(2,6-dioxopiperidin-3-yl)-1-oxoisoindolin-5-yl)methyl)-2,2-difluoroacetamide. The polymorphs of 2-(4-chlorophenyl)-N-((2-(2,6-dioxopiperidin-3-yl)-1-oxoisoindolin-5-yl)methyl)-2,2-difluoroacetamide are described herein and in U.S. Publication No. 2017-0197934, filed on January 6, 2017.

In one embodiment, which is not claimed, provided herein are formulations comprising Compound 1 in an amount of about 0.05-0.2%, a citrate buffer in an amount of about 3%-6%, hydroxypropyl β-cyclodextrin in an amount of about 92-98%, and no more than about 1% dimethyl sulfoxide based on total weight of the formulation. In one embodiment, the citrate buffer comprises anhydrous citric acid and anhydrous sodium citrate.

In one embodiment, which is not claimed, provided herein are formulations comprising Compound 1 in an amount of about 0.05-0.25%, hydroxypropyl β-cyclodextrin in an amount of about 99.1-99.9%, and no more than about 0.5% formic acid based on total weight of the formulation.

In one embodiment, provided herein are formulations comprising Compound 1 in an amount of about 0.01-0.15%, hydroxypropyl β-cyclodextrin in an amount of about 99.1-99.99%.

In one embodiment, provided herein are formulations comprising Compound 1 in an amount of about 0.01-0.15%, hydroxypropyl β-cyclodextrin in an amount of about 99.1-99.99%, and no more than about 0.5% formic acid based on total weight of the formulation.

In one embodiment, which is not claimed, provided herein are formulations comprising Compound 1 in an amount of about 0.05-0.2%, a citrate buffer in an amount of about 3%-6%, sulfobutyl ether-beta-cyclodextrin in an amount of about 92-98%, and no more than about 1% dimethyl sulfoxide based on total weight of the formulation. In one embodiment, the citrate buffer comprises anhydrous citric acid and anhydrous sodium citrate.

In one embodiment, which is not claimed, provided herein are formulations comprising Compound 1 in an

amount of about 0.05-0.25%, sulfobutyl ether-beta-cyclodextrin in an amount of about 99.1-99.9%, and no more than about 0.5% formic acid based on total weight of the formulation.

In one embodiment, the formulations are for use in treating, preventing, managing, and/or ameliorating cancers, including solid tumors and hematological cancers, or one or more symptoms or causes thereof, by administering Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors. Thus, provided herein is Compound 1 for use in such methods, wherein the method comprises administering Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

In one embodiment, the medical uses provided herein comprise administering a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

In certain embodiments, the formulations provided herein comprise a solid form of 2-(4-chlorophenyl)-N-((2-(2,6-dioxopiperidin-3-yl)-1-oxoisoindolin-5-yl)methyl)-2,2-difluoroacetamide. In certain embodiments, the formulations provided herein comprise an amorphous form of 2-(4-chlorophenyl)-N-((2-(2,6-dioxopiperidin-3-yl)-1-oxoisoindolin-5-yl)methyl)-2,2-difluoroacetamide.

In certain embodiments, provided herein is a unit dosage form comprising a formulation, wherein the formulation comprises Compound 1, a buffer and a bulking agent.

In one aspect, the formulations containing therapeutically effective concentrations of Compound 1 are administered to an individual exhibiting the symptoms of the disease or disorder to be treated. The amounts are effective to ameliorate or eliminate one or more symptoms of the disease or disorder.

Further provided, but not claimed, is a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects approval by the agency of manufacture, use of sale for human administration. The pack or kit can be labeled with information regarding mode of administration, sequence of drug administration (e.g., separately, sequentially or concurrently), or the like.

These and other aspects of the subject matter described herein will become evident upon reference to the following detailed description.

**BRIEF DESCRIPTION OF THE DRAWINGS**

FIG. 1 depicts an X-ray powder diffractogram stack plot of Forms A, B, C, D, and E of Compound 1.
FIG. 2 depicts an X-ray powder diffractogram (XRPD) plot of Form A of Compound 1.
FIG. 3 depicts a SEM image of Form A of Compound 1.
FIG. 4 depicts a thermogravimetrical analysis (TGA) plot of Form A of Compound 1.
FIG. 5 depicts a differential scanning calorimetry (DSC) thermogram plot of Form A of Compound 1.
FIG. 6 provides a dynamic vapor sorption (DVS) isotherm plot of Form A of Compound 1.
FIG. 7 provides a $^1$H NMR spectrum of Form A of Compound 1.
FIG. 8 depicts the comparison of the X-ray powder diffractogram plots of Form A of Compound 1 before (a) and after (b) compression.
FIG. 9 depicts an XRPD plot of Form B of Compound 1.
FIG. 10 depicts a SEM image of Form B of Compound 1.
FIG. 11 depicts a TGA thermogram plot of Form B of Compound 1.
FIG. 12 depicts a DSC thermogram plot of Form B of Compound 1.
FIG. 13 provides a DVS isotherm plot of Form B of Compound 1.
FIG. 14 provides a $^1$H NMR spectrum of Form B of Compound 1.
FIG. 15 depicts the comparison of the X-ray powder diffractogram plots of Form B of Compound 1 before (a) and after (b) compression.
FIG. 16 depicts an XRPD plot of Form C of Compound 1.
FIG. 17 depicts a SEM image of Form C of Compound 1.
FIG. 18 depicts a TGA thermogram plot of Form C of Compound 1.
FIG. 19 depicts a DSC thermogram of Form C of Compound 1.
FIG. 20 provides a DVS isotherm plot of Form C of Compound 1.

FIG. 21 provides a $^{1}H$ NMR spectrum of Form C of Compound 1.

FIG. 22 depicts the comparison of the X-ray powder diffractogram plots of Form C of Compound 1 before (a) and after (b) compression.

FIG. 23 depicts an XRPD plot of Form D of Compound 1.

FIG. 24 depicts a TGA thermogram plot of Form D of Compound 1.

FIG. 25 depicts an XRPD plot of Form E of Compound 1.

FIG. 26 depicts a TGA thermogram plot of Form E of Compound 1.

FIG. 27 depicts the modulated DSC thermogramplot of amorphous Compound 1.

FIG. 28 depicts an XRPD plot of amorphous Compound 1.

FIG. 29 depicts a $^{1}H$ NMR spectrum of amorphous Compound 1.

FIG. 30 provides solubility of Compound 1 in various types, brands and percentages of cyclodextrin along with different solvents and solvent to cyclodextrin ratio.

FIG. 31 provides final pH of the bulk solution vs. citrate buffer pH and strength.

FIG. 32 provides lyophilization profile of 1st scale up batch for formulation Ib.

FIG. 33 provides residual solvent as a function of lyophilization process time for formulation Ib.

FIG. 34 provides lyophilization profile of 2nd scale up batch for formulation Ib.

FIG. 35 provides process diagram of formulation Ia.

FIG. 36 provides process diagram of formulation Ib.

FIG. 37 shows increase in solubility of Compound 1 as a function of Kleptose concentration at 25°C (bottom-up).

FIG. 38 demonstrates the effect of increase in Kleptose concentration on Compound 1 precipitation.

FIG. 39 shows Compound 1 precipitation in Kleptose solutions at refrigerated condition.

FIG. 40 illustrates the contour of design space for Compond 1 and Kleptose formulations.

FIG. 41 demonstrates the decrease in removal of formic acid with the increase of Kleptose amount by the same lyophilization cycle.

FIG. 42 demonstrates the impact of cake thickness on the residual formic acid level.

FIG. 43 shows residual formic acid per mg dose vs. Kleptose concentration in prototypes of formulation Ic.

FIG. 44 demonstrates that the osmolality of reconstituted solutions is linearly correlated with the Kleptose concentration.

FIG. 45 provides product temperature profiles for lyophilization of lab scale batch 1 for formulation Ic.

FIG. 46 provides product temperature profiles for lyophilization of lab scale batch 2 for formulation Ic.

FIG. 47 provides product temperature profiles for lyophilization of development batch Ic-1 for formulation Ic.

FIG. 48 provides product temperature profiles for lyophilization of development batch Ic-2 for formulation Ic.

FIG. 49 provides product temperature profiles for lyophilization of development batch Ic-3 for formulation Ic.

FIG. 50 provides a plot of residual moisture as a function of lyophilization cycle time for development batches Ic-1-F1, Ic-1-F2, Ic-2-F1, and Ic-2-F2, for formulation Ic.

FIG. 51 provides a plot of residual formic acid as a function of secondary drying time for development batches Ic-1-F1, Ic-1-F2, Ic-2-F1, Ic-2-F2, Ic-3-F1 and Ic-3-F2 for formulation Ic.

FIG. 52 provides product temperature profiles for lyophilization of batch C1 for formulation Ic.

FIG. 53 provides product temperature profiles for lyophilization of batch C2 for formulation Ic.

FIG. 54 illustrates lyophilized cake appearance for batches C1 and C2 for formulation Ic.

FIG. 55 provides a process diagram for preparation of formulation Ic.

FIG. 56 provides cell proliferation dose response (ECso) of Compound 1 in the presence of various concentrations of second agents. Data demonstrates the $EC_{50}$ shifts toward lower values in the presence of the second agents, indicating a synergistic activity of Compound 1 with the second agent. The synergy was confirmed by Bliss analysis.

FIG. 57 provides cell proliferation dose response curves for combinations of Compound 1 with midostaurin and ruxolitinib in the MOLM-13 cell line.

FIG. 58 provides a summary of the synergy observed for Compound 1 when used in combination with everolimus or temsirolimus, as measured by $EC_{50}$ shifts and by Bliss analysis in solid tumor cell lines.

FIG. 59 provides cell proliferation dose response curves for combinations of Compound 1 with everolimus in various solid tumor cell lines.

FIG. 60 provides the effect of Compound 1 alone, and in combinations with everolimus (RAD, 2 nM, 20 nM and 200 nM) on proliferation of BON cells, 24 h post treatment.

FIG. 61 provides the effect of Compound 1 alone, and in combinations with everolimus (RAD, 2 nM, 20 nM and 200 nM) on proliferation of BON cells, 120 h post treatment on 2D plates.

FIG. 62 provides the effect of Compound 1 alone, and in combinations with everolimus (RAD, 2 nM, 20 nM and 200 nM) on proliferation of BON cells, 120 h post treatment.

FIG. 63 provides the effect of for Compound 1 alone, and in combinations with everolimus (RAD, 2 nM, 20 nM and 200 nM) on proliferation of BON cells, 96 h post treatment on 3D plates.

FIGs. 64A and 64B provide the effect of Compound 1 alone, and in combinations with everolimus (RAD, 2 nM, 20 nM and 200 nM) on proliferation of BON cells, 120 h post treatment on 3D plates.

FIGs. 65A and 65B provide the dose-response of Compound 1 and everolimus in a 3D ex-vivo proliferation assay showing the $IC_{50}$ and maximal inhibition of the compounds in the GA0087 model in duplicate experiments.

FIG. 66 provides the dose dependent response of cisplatin (reference compound) in a 3D ex-vivo GA0087 proliferation assay showing the $IC_{50}$ and maximal inhibition of cisplatin model (duplicate experiments).

FIGs. 67A and 67B provide the effect of Compound 1 in a matrix combination assay with everolimus in a 3D GA0087 cell proliferation model (duplicate experiments).

FIGs. 68A and 68B provide the combination index as calculated by Chou and Talalay method for Compound 1 and everolimus in a 3D GA0087 cell proliferation model (duplicate experiments).

FIG. 69 provides the effect of Compound 1 and everolimus, alone and in combination, on tumor volume in the GA0087 model.

FIG. 70 provides the effect of Compound 1 on colony numbers in samples from myelofibrosis patients in a colony forming assay.

FIG. 71 provides the effect of Compound 1 on cell viability of BaF3 cells expressing hCRBN, hCRNB and wild type JAK2, or hCRBN and JAK2-V617F.

FIG. 72 provides the effect of ruxolitinib on cell viability of BaF3 cells expressing hCRBN, hCRNB and wild type JAK2, or hCRBN and JAK2-V617F.

FIG. 73 provides the effect of NS-18 on cell viability of BaF3 cells expressing hCRBN, hCRNB and wild type JAK2, or hCRBN and JAK2-V617F.

FIG. 74 provides the effect of momelotinib on cell viability of BaF3 cells expressing hCRBN, hCRNB and wild type JAK2, or hCRBN and JAK2-V617F.

FIG. 75 provides the effect of pacritinib on cell viability of BaF3 cells expressing hCRBN, hCRNB and wild type JAK2, or hCRBN and JAK2-V617F.

FIG. 76 provides the effect of fedratinib on cell viability of BaF3 cells expressing hCRBN, hCRNB and wild type JAK2, or hCRBN and JAK2-V617F.

FIG. 77 provides the effect of everolimus on cell viability of BaF3 cells expressing hCRBN, hCRNB and wild type JAK2, or hCRBN and JAK2-V617F.

FIG. 78 provides the effect of a combination of Compound 1 and NS-018 on cell viability of hCRBN, JAK2, JAK2-V617F newly transduced; IL3 dependent, and JAK2-V617F; IL3 independent BaF cell lines.

FIG. 79 provides the effect of a combination of Compound 1 and low dose NS-018 on cell viability of hCRBN, JAK2, JAK2-V617F newly transduced; IL3 dependent, and JAK2-V617F; IL3 independent BaF cell lines.

FIG. 80 provides the effect of a combination of Compound 1 and ruxolitinib on cell viability of hCRBN, JAK2, JAK2-V617F newly transduced; IL3 dependent, and JAK2-V617F; IL3 independent BaF cell lines.

FIG. 81 provides the effect of a combination of Compound 1 and low dose ruxolitinib on cell viability of hCRBN, JAK2, JAK2-V617F newly transduced; IL3 dependent, and JAK2-V617F; IL3 independent BaF cell lines.

FIG. 82 provides the effect of a combination of Compound 1 and momelotinib on cell viability of hCRBN, JAK2, JAK2-V617F newly transduced; IL3 dependent, and JAK2-V617F; IL3 independent BaF cell lines.

FIG. 83 provides the effect of a combination of Compound 1 and pacritinib on cell viability of hCRBN, JAK2, JAK2-V617F newly transduced; IL3 dependent, and JAK2-V617F; IL3 independent BaF cell lines.

FIG. 84 provides the effect of a combination of Compound 1 and fedratinib on cell viability of hCRBN, JAK2, JAK2-V617F newly transduced; IL3 dependent, and JAK2-V617F; IL3 independent BaF cell lines.

FIG. 85 provides the effect of a combination of Compound 1 and everolimus on cell viability of hCRBN, JAK2, JAK2-V617F newly transduced; IL3 dependent, and JAK2-V617F; IL3 independent BaF cell lines.

FIG. 86 provides the effect of NS-018 and ruxolitinib as single agents on cell viability of JAK2V617F AML cell lines.

FIG. 87 provides the effect of a combination of Compound 1 and NS-018 on cell viability of JAK2 V617F in HEL, SET-2 and MUTZ-8 AML cell lines.

FIG. 88 provides the effect of a combination of Compound 1 and ruxolitinib on cell viability of JAK2 V617F in HEL, SET-2 and MUTZ-8 AML cell lines.

FIG. 89 provides the effect of a combination of Compound 1 and everolimus on cell viability of of HEL, SET-2 and MUTZ-8 AML cells.

FIG. 90 provides an overview of the effect of JAK2 inhibitors in combination with Compound 1 in JAK2 V617F cells, wherein synergy was scored by ECso shift and Bliss method.

FIG. 91 provides dosing schedules for a combination of Compound 1 and IDH2 inhibitor enasidenib (AG-221).

FIG. 92 provides results of a qualitative analysis of phenotypes in a flow cytometry assay.

FIG. 93 provides the differentiation effect of Compound 1 and enasidenib (AG-221) combination on TF-1:IDH2RI40Q stem and progenitor cells ($CD34^+$), and $CD34^-$/$CD235^+$ erythroblast cells as shown in scatter plots from Schedule A.

FIG. 94 provides the differentiation effect of Compound 1 and enasidenib on TF-1:IDH2RI40Q stem and progenitor

cells ($CD34^+/CD38^+$), and HSC ($CD34^+/CD38^-$), and non stem/progenitor $CD34^-/CD38^-$ cells using Schedules A, B or C.

FIG. 95 provides the differentiation effect of Compound 1 and enasidenib on $CD235a^+$ (Glycophorins A) erythroblasts.

FIG. 96 provides the effect of Compound 1 and enasidenib on GSPT1 degradation in several subsets of cells in a TF1 assay.

FIG. 97 provides the effect of Compound 1 and enasidenib on GSPT1 degradation in several subsets of cells in a TF1 assay.

FIG. 98 provides the effect of Compound 1 and enasidenib on proliferation of cells, as shown by total cell count and undifferentiated HSC ($CD34^+/CD38^-$) and progenitors ($CD34^+/CD38^+$) cell count.

FIG. 99 shows that Compound 1 in combination with RAD resulted in significant decrease in GSPT1 protein and changes in phosphorylated proteins regulating translation and metabolism. Western blot analysis was performed on BON cell lysates treated for 120 h with the indicated concentrations of vehicle, RAD and/or Compound 1 and probed with indicated antibodies. Actin was used as a loading control.

FIGs. 100A-100E show the combination effect of treatment with Compound 1 and inhibitors targeting mTOR, FLT3, JAK2, or JAK3 on cell proliferation in U937 AML cells. FIG. 100A shows Compound 1 combination with mTOR inhibitor everolimus; FIG. 100B shows Compound 1 combination with FLT3 inhibitor quizartinib; FIG. 100C shows Compound 1 combination with JAK2 inhibitor ruxolitinib; FIG. 100D shows Compound 1 combination with JAK2 inhibitor AZD1480 ; and FIG. 100E shows Compound 1 combination with JAK3 inhibitor tofacitinib.

FIG. 101 shows the combination effect of treatment with Compound 1 and mTOR, FLT3, JAK2, or JAK3 inhibitors on GSPT1 expression, mTOR activation, ATF4 induction and Caspase-3 cleavage in U937 AML cells.

FIGs. 102A-102G show the effect of Compound 1 with and without venetoclax at the indicated concentrations on AML cell line proliferation when incubated for 48 hours.

FIG. 103 shows the relative ATP levels as a measure of viability in response to several dose combinations of Compound 1 and venetoclax.

FIG. 104 shows western blot analysis measuring GSPT1, Mcl-1, Bcl-2, cleaved caspase 3, and GAPDH protein levels in KG-1 cells 16 hours after treatment with a set of doses of Compound 1, venetoclax and combinations of Compound 1 and venetoclax.

FIGs. 105A and 105B show the live cell analyses of KG-1 confluency (FIG. 105A) and apoptotic event counts (FIG. 105B) after treatment with Compound 1, venetoclax and combinations of Compound 1 and venetoclax.

FIG. 106 shows the combination effect of treatment with Compound 1 and everolimus on GSPT1 expression, mTOR activation, Mcl-1 expression and Caspase-3 cleavage.

FIG. 107 shows the effect of Compound 1 on bone marrow mononuclear cells or isolated $CD34^+$ blast cells from 2 different myelodysplastic syndrome patients assayed by liquid culture.

FIG. 108 shows the effect of Compound 1 on bone marrow mononuclear cells from myelodysplastic syndrome patients assayed by liquid culture (A) or colony forming assay (B).

FIG. 109 shows the effect of Compound 1 on caspase-3 activation and GSPT1 degradation in bone marrow mononuclear cells from a myelodysplastic syndrome patient, when tested as a single agent or in combination with 111 nM everolimus after 24 hours of compound(s) exposure.

## DETAILED DESCRIPTION

### Definitions

Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs. In general, the technical teaching of one embodiment can be combined with that disclosed in other embodiments provided herein.

The use of the word "a" or "an" when used in conjunction with the term "comprising" in the claims and/or the specification can mean "one", but it is also consistent with the meaning of "one or more", "at least one" and "one or more than one."

As used herein, the terms "comprising" and "including" can be used interchangeably. The terms "comprising" and "including" are to be interpreted as specifying the presence of the stated features or components as referred to, but does not preclude the presence or addition of one or more features, or components, or groups thereof. Additionally, the terms "comprising" and "including" are intended to include examples encompassed by the term "consisting of'. Consequently, the term "consisting of' can be used in place of the terms "comprising" and "including" to provide for more specific embodiments of the invention.

The term "consisting of' means that a subject-matter has at least 90%, 95%, 97%, 98% or 99% of the stated

features or components of which it consists. In another embodiment the term "consisting of' excludes from the scope of any succeeding recitation any other features or components, excepting those that are not essential to the technical effect to be achieved.

As used herein, the terms "or" is to be interpreted as an inclusive "or" meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive. E.g., "treating, preventing or managing" or similar listings means: "treating; preventing; managing; treating and preventing; treating and managing; preventing and managing; treating, preventing and managing".

The term "Compound 1" refers to"2-(4-chlorophenyl)-N-((2-(2,6-dioxopiperidin-3-yl)-1-oxoisoindolin-5-yl)methyl)-2,2-difluoroacetamide" having the structure:

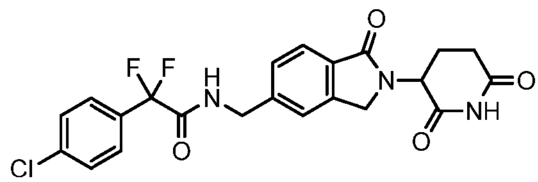

,

and its stereoisomers or mixture of stereoisomers, pharmaceutically acceptable salts, tautomers, prodrugs, solvates, hydrates, co-crystals, clathrates, or polymorphs thereof. In certain embodiments, Compound 1 refers to 2-(4-chlorophenyl)-N-((2-(2,6-dioxopiperidin-3-yl)-1-oxoisoindolin-5-yl)methyl)-2,2-difluoroacetamide and its tautomers. In certain embodiments, Compound 1 refers to a polymorph of 2-(4-chlorophenyl)-N-((2-(2,6-dioxopiperidin-3-yl)-1-oxoisoindolin-5-yl)methyl)-2,2-difluoroacetamide, such as Form A, B, C, D, or E, or a mixture thereof. In certain embodiments, Compound 1 refers to polymorph Form C of 2-(4-chlorophenyl)-N-((2-(2,6-dioxopiperidin-3-yl)-1-oxoisoindolin-5-yl)methyl)-2,2-difluoroacetamide. In certain embodiments, Compound 1 refers to an amorphous form of 2-(4-chlorophenyl)-N-((2-(2,6-dioxopiperidin-3-yl)-1-oxoisoindolin-5-yl)methyl)-2,2-difluoroacetamide. In one embodiment, the stereoisomer is an enantiomer.

Unless specifically stated otherwise, where a compound may assume alternative tautomeric, regioisomeric and/or stereoisomeric forms, all alternative isomers are intended to be encompassed within the scope of the claimed subject matter. For example, where a compound can have one of two tautomeric forms, it is intended that both tautomers be encompassed herein.

Thus, the compounds herein may be enantiomerically pure, or be stereoisomeric or diastereomeric mixtures. As used herein and unless otherwise indicated, the term "stereoisomerically pure" means a composition that comprises one stereoisomer of a compound and is substantially free of other stereoisomers of that compound. For example, a stereoisomerically pure composition of a compound having one chiral center will be substantially free of the opposite enantiomer of the compound. A stereoisomerically pure composition of a compound having two chiral centers will be substantially free of other diastereomers of the compound. A typical stereoisomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound. A stereoisomerically pure compound as used herein comprises greater than about 80% by weight of one stereoisomer of the compound, more preferably greater than about 90% by weight of one stereoisomer of the compound, even more preferably greater than about 95% by weight of one stereoisomer of the compound, and most preferably greater than about 97% by weight of one stereoisomer of the compound. As used herein and unless otherwise indicated, the term "stereoisomerically enriched" means a composition that comprises greater than about 60% by weight of one stereoisomer of a compound, preferably greater than about 70% by weight, more preferably greater than about 80% by weight of one stereoisomer of a compound. As used herein and unless otherwise indicated, the term "enantiomerically pure" means a stereoisomerically pure composition of a compound having one chiral center. Similarly, the term "stereoisomerically enriched" means a stereoisomerically enriched composition of a compound having one chiral center. As used herein, stereoisomeric or diastereomeric mixtures means a composition that comprises more than one stereoisomer of a compound. A typical stereoisomeric mixture of a compound comprises about 50% by weight of one stereoisomer of the compound and about 50% by weight of other stereoisomers of the compound, or comprises greater than about 50% by weight of one stereoisomer of the compound and less than about 50% by weight of other stereoisomers of the compound, or comprises greater than about 45% by weight of one stereoisomer of the compound and less than about 55% by weight of the other stereoisomers of the compound, or

comprises greater than about 40% by weight of one stereoisomer of the compound and less than about 60% by weight of the other stereoisomers of the compound, or comprises greater than about 35% by weight of one stereoisomer of the compound and less than about 65% by weight of the other stereoisomers of the compound.

As used herein, API refers to Compound 1. In certain embodiments, API refers to 2-(4-chlorophenyl)-N-((2-(2,6-dioxopiperidin-3-yl)-1-oxoisoindolin-5-yl)methyl)-2,2-difluoroacetamide.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. 1972, 11:942-944).

As used herein, and unless otherwise specified, the term "lyophilize" refers to the process of isolating a solid substance from solution and/or removal of solvent. In some embodiments, this may be achieved by various techniques known to one of skill in the art, including, for example, evaporation (*e.g.,* under vacuum, for example by freeze drying, and/or freezing the solution and vaporizing the frozen solvent under vacuum or reduced pressure conditions, *etc.*)

As used herein, the term "cosolvent" refers to a solvent that aids the solubilization of an active agent in water during manufacturing a formulation provided herein. The cosolvent can be a solvent that also provides sufficient stability of the intermediate formulation during manufacture. The cosolvent can also be removed from the formulation, or reduced to an acceptable level, during manufacture. Examples of cosolvents include acetonitrile, chloroform, tert-butanol, methanol, tetrahydrofuran, formic acid, acetic acid, acetone, anisole, butanol, butyl acetate, tert-butylmethyl ether, ethanol, ethyl acetate, ethyl ether, ethyl formate, heptanes, isobutyl acetate, isopropyl acetate, methyl acetate, 3-methyl-butanol, methylethyl ketone, methylisobutyl ketone, 2-methyl-1-propanol, pentane, 1-pentanol, 1-propanol, 2-propanol, and propyl acetate.

As used herein, and unless otherwise specified, the term "substantially free of' means containing no more than an insignificant amount. In some embodiments, a composition or preparation is "substantially free of' a recited element if it contains less than 5%, 4%, 3%, 2%, or 1%, by weight of the element. In some embodiments, the composition or preparation contains less than 0.9%, 0.8%, 0.7%, 0.6%, 0.5%, 0.4%, 0.3%, 0.2%, 0.1% or less of the recited element. In some embodiments, the composition or preparation contains an undetectable amount of the recited element.

As used herein, "reconstituted aqueous solution" or "reconstituted aqueous composition" or "reconstituted aqueous formulation" refers to an aqueous solution obtained by dissolving a lyophilized formulation provided herein in an aqueous solvent.

The term "aqueous diluent" used herein refers to an aqueous liquid capable of being included in a parenteral formulation. Such aqueous diluents can include, for example, water, saline, ½ normal saline or dextrose if desired, as well as any of the known ancillary preservatives or excipients commonly found as part of parenteral formulations. Exemplary aqueous diluents include water, 5% dextrose solution, and the like.

As used herein, and unless otherwise specified, the term "parenteral" includes subcutaneous, intravenous, intramuscular, intra-artricular, intra-synovial, intrasternal, intrathecal, intrahepatic, intralesional and intracranial injection or infusion techniques.

As used herein, and unless otherwise specified, the expression "unit dose" refers to a physically discrete unit of a formulation appropriate for a subject to be treated *(e.g.,* for a single dose); each unit containing a predetermined quantity of an active agent selected to produce a desired therapeutic effect (it being understood that multiple doses may be required to achieve a desired or optimum effect), optionally together with a pharmaceutically acceptable carrier, which may be provided in a predetermined amount. The unit dose may be, for example, a volume of liquid (e.g. an acceptable carrier) containing a predetermined quantity of one or more therapeutic agents, a predetermined amount of one or more therapeutic agents in solid form, a sustained release formulation or drug delivery device containing a predetermined amount of one or more therapeutic agents, etc. It will be appreciated that a unit dose may contain a variety of components in addition to the therapeutic agent(s). For example, acceptable carriers (e.g., pharmaceutically acceptable carriers), diluents, stabilizers, buffers, preservatives, etc., may be included as described *infra.* It will be understood, however, that the total daily usage of a formulation of the present disclosure will be decided by the attending physician within the scope of sound medical judgment. The specific effective dose level for any particular subject or organism may depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts.

As used herein, the term "solid form" refers a crystal form or an amorphous form or a mixture thereof of 2-(4-chlorophenyl)-N-((2-(2,6-dioxopiperidin-3-yl)-1-oxoisoindolin-5-yl)methyl)-2,2-difluoroacetamide or a stereoisomer or mixture of stereoisomers, pharmaceutically acceptable salt, tautomer, solvate, hydrate, co-crystal, clathrate, or polymorph thereof.

As used herein, unless otherwise specified, the term "pharmaceutically acceptable salt(s)," as used herein includes, but is not limited to, salts of acidic or basic moieties of Compound 1. Basic moieties are capable of forming a

wide variety of salts with various inorganic and organic acids. The acids that can be used to prepare pharmaceutically acceptable acid addition salts of such basic compounds are those that form non-toxic acid addition salts, *e.g.,* salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (*e.g.,* 1,1'-methylene-bis-(2-hydroxy-3-naphthoate) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts.

As used herein, and unless otherwise specified, the term "solvate" means a compound provided herein or a salt thereof that further includes a stoichiometric or non-stoichiometric amount of solvent bound by non-covalent intermolecular forces. Where the solvent is water, the solvate is a hydrate.

As used herein and unless otherwise indicated, the term "prodrug" means a derivative of a compound that can hydrolyze, oxidize, or otherwise react under biological conditions (in-vitro or in-vivo) to provide the compound. Examples of prodrugs include, but are not limited to, derivatives of compounds described herein (*e.g.,* Compound 1) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues.

A "pharmaceutically acceptable excipient," refers to a substance that aids the administration of an active agent to a subject by for example modifying the stability of an active agent or modifying the absorption by a subject upon administration. A pharmaceutically acceptable excipient typically has no significant adverse toxicological effect on the patient. Examples of pharmaceutically acceptable excipients include, for example, water, NaCl (including salt solutions), normal saline solutions, ½ normal saline, sucrose, glucose, bulking agents, buffers, binders, fillers, disintegrants, lubricants, coatings, sweeteners, flavors, alcohols, oils, gelatins, carbohydrates such as amylose or starch, fatty acid esters, hydroxymethycellulose, polyvinyl pyrrolidine, and colors, and the like. One of skill in the art will recognize that other pharmaceutical excipients known in the art are useful in the present invention and include those listed in for example the Handbook of Pharmaceutical Excipients, Rowe R.C., Shesky P.J., and Quinn M.E., 6th Ed., The Pharmaceutical Press, RPS Publishing (2009). The terms "bulking agent", and "buffer" are used in accordance with the plain and ordinary meaning within the art.

As used herein, and unless otherwise specified, the term "about," when used in connection with doses, amounts, or weight percent of ingredients of a composition or a dosage form, means dose, amount, or weight percent that is recognized by those of ordinary skill in the art to provide a pharmacological effect equivalent to that obtained from the specified dose, amount, or weight percent which is encompassed. Specifically, the term "about" contemplates a dose, amount, or weight percent within 10%, or 5% of the specified dose, amount, or weight percent which is encompassed.

As used herein, and unless otherwise specified, the term "stable," when used in connection with a liquid formulation or a dosage form, means that the active ingredient of the formulation or dosage form remains solubilized for a specified amount of time and does not significantly degrade or aggregate or become otherwise modified *(e.g.,* as determined, for example, by HPLC). In some embodiments, about 70% or greater, about 80% or greater or about 90% or greater of the compound remains solubilized after the specified period. Stability can also refer to the compatibility of pharmaceutically acceptable excipients described herein. Accordingly, a dosage form can be considered stable when the combined pharmaceutically acceptable excipients and active agent(s) described herein do not degrade or otherwise modify (e.g., react with) the effectiveness or therapeutic value of an active agent described herein.

As used herein, and unless otherwise specified, the term "stable," when used in connection with a solid formulation or a dosage form, means that the active ingredient of the formulation or dosage form does not significantly degrade, decompose or become otherwise modified *(e.g.,* as determined, for example, by HPLC). In some embodiments, about 85% or greater, about 90% or greater, about 95% or greater or about 98% or greater of the active ingredient remains unchanged after the specified period. Stability can also refer to the compatibility of pharmaceutically acceptable excipients described herein. Accordingly, a dosage form can be considered stable when the combined pharmaceutically acceptable excipients and active agent(s) described herein do not degrade or otherwise modify (e.g., react with) the effectiveness or therapeutic value of an active agent described herein.

As used herein, "administer" or "administration" refers to the act of physically delivering a substance as it exists outside the body into a subject. Administration includes all forms known in the art for delivering therapeutic agents, including but not limited to topical, mucosal, injections, intradermal, intravenous, intramuscular delivery or other method of physical delivery described herein or known in the art (e.g., implantation of a slow-release device, such as a miniosmotic pump to a subject; liposomal formulations; buccal; sublingual; palatal; gingival; nasal; vaginal; rectal; intraarteriole; intraperitoneal; intraventricular; intracranial; or transdermal).

"Anti-cancer agents" refer to anti-metabolites (e.g., 5-fluoro-uracil, methotrexate, fludarabine), antimicrotubule

agents (e.g., vinca alkaloids such as vincristine, vinblastine; taxanes such as paclitaxel, docetaxel), alkylating agents (e.g., cyclophosphamide, melphalan, carmustine, nitrosoureas such as bischloroethylnitrosurea and hydroxyurea), platinum agents (e.g. cisplatin, carboplatin, oxaliplatin, JM-216 or satraplatin, CI-973), anthracyclines (e.g., doxorubicin, daunorubicin), antitumor antibiotics (e.g., mitomycin, idarubicin, adriamycin, daunomycin), topoisomerase inhibitors (e.g., etoposide, camptothecins), anti-angiogenesis agents (e.g. Sutent®, sunitinib malate, and Bevacizumab) or any other cytotoxic agents (estramustine phosphate, prednimustine), hormones or hormone agonists, antagonists, partial agonists or partial antagonists, kinase inhibitors, checkpoint inhibitors, and radiation treatment.

By "co-administer" it is meant that compounds, compositions or agents described herein are administered at the same time, just prior to, or just after the administration of one or more additional compounds, compositions or agents, including for example an anti-cancer agent. Co-administration is meant to include simultaneous or sequential administration of compounds, compositions or agents individually or in combination (more than one compound or agent). Co-administration includes administering two compounds, compositions or agents simultaneously, approximately simultaneously (*e.g.,* within about 1, 5, 10, 15, 20, or 30 minutes of each other), or sequentially in any order. Thus, co-administration can include administering one active agent *(e.g.* a compound described herein) within 0.5, 1, 2, 4, 6, 8, 10, 12, 16, 20, or 24 hours of a second active agent. Co-administration can also be accomplished by co-formulation, *e.g.,* preparing a single dosage form including both active agents. The active agents can be formulated separately. In such instances, the active agents are admixed and included together in the final form of the dosage unit. Alternatively, co-administration as described herein can include administering two separate unit dosage forms of at least two separate active agents (*e.g.,* Compound 1 and a second active agent described herein).

As used herein, the term "daily" is intended to mean that a therapeutic compound, such as Compound 1, is administered once or more than once each day for a period of time. The term "continuous" is intended to mean that a therapeutic compound, such as Compound 1, is administered daily for an uninterrupted period of at least 10 days to 52 weeks. The term "intermittent" or "intermittently" as used herein is intended to mean stopping and starting at either regular or irregular intervals. For example, intermittent administration of Compound 1 is administration for one to six days per week, administration in cycles (*e.g.,* daily administration for one to ten consecutive days of a 28 day cycle, then a rest period with no administration for rest of the 28 day cycle or daily administration for two to eight consecutive weeks, then a rest period with no administration for up to one week), or administration on alternate days. The term "cycling" as used herein is intended to mean that a therapeutic compound, such as Compound 1, is administered daily or continuously but with a rest period.

A "cycling therapy" refers to a regimen or therapy that includes an administration period as described herein and a rest period as described herein.

The term "administration period" as used herein refers to a period of time a subject is continuously or actively administered a compound or composition described herein.

The term "rest period" as used herein refers to a period of time, often following an administration period, where a subject is not administered a compound or composition described herein (e.g. discontinuation of treatment). In certain embodiments, a "rest period" refers to a period of time where a single agent is not administered to a subject or treatment using a particular compound is discontinued. In such embodiments, a second therapeutic agent (e.g., a different agent than the compound or composition administered in the previous administration period) can be administered to the subject.

An "effective amount" is an amount sufficient to achieve the effect for which it is administered (*e.g.,* treat a disease or reduce one or more symptoms of a disease or condition). Thus, administration of an "amount" of a compound described herein to a subject refers to administration of "an amount effective," to achieve the desired therapeutic result. A "therapeutically effective amount" of a compound described herein for purposes herein is thus determined by such considerations as are known in the art. The term "therapeutically effective amount" of a composition described herein refers to the amount of the composition that, when administered, is sufficient to treat one or more of the symptoms of a disease described herein (*e.g.,* cancer, for example AML, MDS, MPN or solid tumors). Administration of a compound described herein can be determined according to factors such as, for example, the disease state, age, sex, and weight of the individual. A therapeutically effective amount also refers to any toxic or detrimental effects of Compound 1 are outweighed by the therapeutically beneficial effects.

As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a patient with such a disease or disorder. In some embodiments, the terms refer to the administration of a compound provided herein, with or without other additional active agent, after the onset of symptoms of the particular disease. In one embodiment, the disease is leukemia, including, but not limited to, chronic lymphocytic leukemia (CLL), chronic myelocytic leukemia (CML), acute lymphoblastic leukemia (ALL), acute myeloid leukemia or acute myeloblastic leukemia (AML). In one embodiment, the leukemia can be relapsed, refractory or resistant to at least one anti-cancer therapy. In one embodiment, the disease is AML, including, a subtype of AML discussed herein. In one embodiment, the disease is myelodysplastic syndrome MDS, including, a subtype of

MDS discussed herein.

As used herein, and unless otherwise specified, the terms "prevent," "preventing" and "prevention" refer to the prevention of the onset, recurrence or spread of a disease or disorder, or of one or more symptoms thereof. In certain embodiments, the terms refer to the treatment with or administration of a compound provided herein, with or without other additional active compound, prior to the onset of symptoms, particularly to patients at risk of diseases or disorders provided herein. The terms encompass the inhibition or reduction of a symptom of the particular disease. Patients with familial history of a disease in particular are candidates for preventive regimens in certain embodiments. In addition, patients who have a history of recurring symptoms are also potential candidates for the prevention. In this regard, the term "prevention" may be interchangeably used with the term "prophylactic treatment." In one embodiment, the disease is leukemia, including, but is not limited to, chronic lymphocytic leukemia, chronic myelocytic leukemia, acute lymphoblastic leukemia, acute myeloid leukemia, and acute myeloblastic leukemia. In one embodiment, the leukemia can be relapsed, refractory or resistant to at least one anti-cancer therapy. In one embodiment, the disease is AML, including, a subtype of AML discussed herein. In one embodiment, the disease is MDS, including, a subtype of MDS discussed herein.

As used herein, and unless otherwise specified, the terms "manage," "managing" and "management" refer to preventing or slowing the progression, spread or worsening of a disease or disorder, or of one or more symptoms thereof. Often, the beneficial effects that a patient derives from a prophylactic and/or therapeutic agent do not result in a cure of the disease or disorder. In this regard, the term "managing" encompasses treating a patient who had suffered from the particular disease in an attempt to prevent or minimize the recurrence of the disease, or lengthening the time during which the remains in remission. In one embodiment, the disease is leukemia, including, but not limited to, chronic lymphocytic leukemia, chronic myelocytic leukemia, acute lymphoblastic leukemia, acute myeloid leukemia, and acute myeloblastic leukemia. In one embodiment, the leukemia can be relapsed, refractory or resistant to at least one anti-cancer therapy. In one embodiment, the disease is AML, including, a subtype of AML discussed herein. In one embodiment, the disease is MDS, including a subtype of MDS discussed herein.

As used herein, "induction therapy" refers to the first treatment given for a disease, or the first treatment given with the intent of inducing complete remission in a disease, such as cancer. When used by itself, induction therapy is the one accepted as the best available treatment. For example, induction therapy for AML comprises treatment with cytarabine for 7 days plus treatment with an anthracycline, such as daunorubicin or idarubicin, for 3 days. If residual leukemia is detected, patients are treated with another chemotherapy course, termed reinduction. If the patient is in complete remission after induction therapy, then additional consolidation and/or maintenance therapy is given to prolong remission or to potentially cure the patient.

As used herein, "consolidation therapy" refers to the treatment given for a disease after remission is first achieved. For example consolidation therapy for cancer is the treatment given after the cancer has disappeared after initial therapy. Consolidation therapy may include radiation therapy, stem cell transplant, or treatment with cancer drug therapy. Consolidation therapy is also referred to as intensification therapy and post-remission therapy.

As used herein, "maintenance therapy" refers to the treatment given for a disease after remission or best response is achieved, in order to prevent or delay relapse. Maintenance therapy can include chemotherapy, hormone therapy or targeted therapy.

"Remission" as used herein, is a decrease in or disappearance of signs and symptoms of a cancer, for example, multiple myeloma. In partial remission, some, but not all, signs and symptoms of the cancer have disappeared. In complete remission, all signs and symptoms of the cancer have disappeared, although the cancer still may be in the body.

The terms "subject," "patient," "subject in need thereof," and "patient in need thereof' are herein used interchangeably and refer to a living organism suffering from one or more of the diseases described herein (*e.g.,* AML) that can be treated by administration of a composition described herein. Non-limiting examples of organisms include humans, other mammals, bovines, rats, mice, dogs, monkeys, goat, sheep, cows, deer, and other non-mammalian animals. In embodiments, a subject is human. A human subject can be between the ages of about 1 year old to about 100 years old. In embodiments, subjects herein can be characterized by the disease being treated *(e.g.,* a "AML subject", a "cancer subject", or a "leukemia subject").

As used herein, the term "tumor," refers to all neoplastic cell growth and proliferation, whether malignant or benign, and all pre-cancerous and cancerous cells and tissues. "Neoplastic," as used herein, refers to any form of dysregulated or unregulated cell growth, whether malignant or benign, resulting in abnormal tissue growth. Thus, "neoplastic cells" include malignant and benign cells having dysregulated or unregulated cell growth.

As used herein, "hematologic malignancy" refers to cancer of the body's blood-forming and immune system-the bone marrow and lymphatic tissue. Such cancers include leukemias, lymphomas (Non-Hodgkin's Lymphoma), Hodgkin's disease (also called Hodgkin's Lymphoma) and myeloma. In one embodiment, the myeloma is multiple myeloma. In some embodiments, the leukemia is, for example, acute myelogenous leukemia (AML), acute lymphocytic leukemia (ALL), adult T-cell leukemia, chronic lymphocytic leukemia (CLL), hairy cell leukemia, myelodysplasia, myeloproliferative disorders or myeloproliferative neoplasm (MPN), chronic myelogenous leukemia (CML), myelodysplastic syndrome

(MDS), human lymphotropic virus-type 1 (HTLV 1) leukemia, mastocytosis, or B-cell acute lymphoblastic leukemia. In some embodiments, the lymphoma is, for example, diffuse large B-cell lymphoma (DLBCL), B-cell immunoblastic lymphoma, small non-cleaved cell lymphoma, human lymphotropic virus-type 1 (HTLV-1) leukemia/lymphoma, adult T-cell lymphoma, peripheral T-cell lymphoma (PTCL), cutaneous T-cell lymphoma (CTCL), mantle cell lymphoma (MCL), Hodgkin lymphoma (HL), non-Hodgkin lymphoma (NHL), AIDS-related lymphoma, follicular lymphoma, small lymphocytic lymphoma, T-cell/histiocyte rich large B-cell lymphoma, transformed lymphoma, primary mediastinal (thymic) large B-cell lymphoma, splenic marginal zone lymphoma, Richter's transformation, nodal marginal zone lymphoma, or ALK-positive large B-cell lymphoma. In one embodiment, the hematological cancer is indolent lymphoma including, for example, DLBCL, follicular lymphoma, or marginal zone lymphoma. In one embodiment, the hematological malignancy is AML. In another embodiment, the hematological malignancy is MDS.

The term "leukemia" refers to malignant neoplasms of the blood-forming tissues. The leukemia includes, but is not limited to, chronic lymphocytic leukemia, chronic myelocytic leukemia, acute lymphoblastic leukemia, acute myeloid leukemia, and acute myeloblastic leukemia. The leukemia can be relapsed, refractory or resistant to at least one anti-cancer therapy.

In one embodiment, the subject has AML, including, for example, the following subtypes of AML. The term "acute myelogenous or myeloid leukemia" refers to hematological conditions characterized by proliferation and accumulation of primarily undifferentiated or minimally differentiated myeloid cells in the bone marrow, and includes subtypes categorized by either the FAB (French, American, British) or WHO classification system. As described herein, the AML includes the following subtypes based on the FAB classification: M0 (AML minimally differentiated); M1 (AML with minimal maturation); M2 (AML with maturation); M3 (Acute promyelocytic leukemia); M4 (Acute myelomonocytic leukemia); M4 (eosAcute myelomonocytic leukemia with eosinophilia); M5 (Acute monocytic leukemia); M6 (Acute erythroid leukemia); and M7 (Acute megakaryoblastic leukemia). As described herein, the AML includes the following subtypes based on the WHO classification: AML with recurrent genetic abnormalities (AML with translocation between chromosomes 8 and 21); AML with translocation or inversion in chromosome 16; AML with translocation between chromosomes 9 and 11; APL (M3) with translocation between chromosomes 15 and 17; AML with translocation between chromosomes 6 and 9; AML with translocation or inversion in chromosome 3); AML (megakaryoblastic) with a translocation between chromosomes 1 and 22; AML with myelodysplasia-related changes; AML related to previous chemotherapy or radiation (Alkylating agent-related AML; Topoisomerase II inhibitor-related AML); AML not otherwise categorized (AML that does not fall into the above categories, i. e. AML minimally differentiated (M0); AML with minimal maturation (M1); AML with maturation (M2); Acute myelomonocytic leukemia (M4); Acute monocytic leukemia (M5); Acute erythroid leukemia (M6); Acute megakaryoblastic leukemia (M7); Acute basophilic leukemia; Acute panmyelosis with fibrosis); Myeloid Sarcoma (also known as granulocytic sarcoma, chloroma or extramedullary myeloblastoma); and Undifferentiated and biphenotypic acute leukemias (also known as mixed phenotype acute leukemias). *(see* https://www.cancer.org/cancer/acute-myelo idleukemia/detection-diagnosis-staging/how-classified.html, last accessed May 25, 2017).

In certain embodiments, the risk groups for AML based on cytogenetics are as described below:

| **Risk Status** | **Cytogenetics** | **Molecular Abnormalities**[a] |
|---|---|---|
| Favorable-risk | Core binding factor: inv(16)[b,c,d]or t(16; 16)[b,c,a] or t(8;21)[b,d] or t(15;17)[d] | Normal cytogenetics: NPM1 mutation in the absence of FLT3-ITD or isolated biallelic CEBPA mutation |
| Intermediate-risk | Normal cytogenetics +8 alone<br><br>t(9;11)<br>Other non-defined | Core binding factor with c-KIT mutation[b] |

(continued)

| Risk Status | Cytogenetics | Molecular Abnormalities[a] |
|---|---|---|
| Poor-risk | Complex (≥ 3 clonal chromosomal abnormalities)<br>Monosomal karyotype<br>-5, 5q-, -7, 7q-<br>11q23 - non t(9;11)<br>inv(3), t(3;3)<br>t(6;9)<br>t(9;22)[e] | Normal cytogenetics: with FLT3-ITD mutation [f] TP53 mutation |

[a] The molecular abnormalities included in this table reflect those for which validated assays are available in standardized commercial laboratories.
[b] Emerging data indicate that the presence of KIT mutations in patients with t(8;21), and to a lesser extent inv(16), confers a higher risk of relapse. These patients are considered intermediate risk and should be considered for hematopoietic stem cell transplant (HSCT) or clinical trials, if available. Other cytogenetic abnormalities in addition to these finding do not alter risk status.
[c] Paschka P, et al. Blood 2013; 121:170-177.
[d] Other cytogenetic abnormalities in addition to these findings do not alter better risk status
[e] For Philadelphia+ acute myeloid leukemia (AML) t(9;22), manage as myeloid blast crisis in chronic myeloid leukemia (CML), with addition of tyrosine kinase inhibitors.

In one embodiment, the subject has MDS, including, for example, the following subtypes of MDS. The term "myelodysplastic syndrome" refers to hematological conditions characterized by abnormalities in the production of one or more of the cellular components of blood (red cells, white cells (other than lymphocytes) and platelets (or their progenitor cells, megakaryocytes)). The ineffective hematopoiesis in the bone marrow (BM) and peripheral blood cytopenias in MDS manifest clinically as anemia, neutropenia, and/or thrombocytopenia of variable frequency and severity. Anemia is the most frequent laboratory finding and it often progresses to red blood cell (RBC) transfusion dependence. Other less common presenting clinical features related to the cytopenias are an increased risk of infection and/or hemorrhage and a propensity to progress to acute myeloid leukemia (AML) (Catenacci, et al. Blood Rev 2005;19:301-319).

MDS includes the following disorders: refractory anemia (RA); RA with ringed sideroblasts (RARS); RA with excess of blasts (RAEB); refractory cytopenia with multilineage dysplasia (RCMD), refractory cytopenia with unilineage dysplasia (RCUD); unclassifiable myelodysplastic syndrome (MDS-U), myelodysplastic syndrome associated with an isolated del(5q) chromosome abnormality, therapy-related myeloid neoplasms and chronic myelomonocytic leukemia (CMML). The MDS as used herein also includes very low risk, low risk, intermediate risk, high risk and very high risk MDS. In some embodiments, the MDS is primary or *de novo* MDS. In other embodiments, the MDS is secondary.

In certain embodiments, MDS is classified based on the World Health Organization (WHO) classification of MDS as described below:

**WHO classifications for MDS**

| WHO myeloid neoplasm and acute leukemia classification | Dysplastic findings | Cytopenias[a] | PB and BM findings and cytogenetics |
|---|---|---|---|
| MDS with single lineage dysplasia (MDS-SLD) | 1 | 1 or 2 | BM <5%, PB <1%, no Auer Rods<br>Any cytogenetics, unless fulfills all criteria for MDS with isolated del(5q) |
| MDS with ring sideroblasts (MDS-RS)[b]<br>MDS-RS and single lineage dysplasia<br>MDS-RS and multilineage dysplasia | 1<br>2 or 3 | 1 or 2<br>3 | BM <5%, PB <1%, no Auer Rods<br>Any cytogenetics, unless fulfills all criteria for MDS with isolated del(5q) |
| MDS with multilineage dysplasia (MDS-MLD) | 2 or 3 | 1-3 | BM <5%, PB <1%, no Auer Rods Any cytogenetics, unless fulfills all criteria for MDS with isolated del(5q) |

(continued)

| WHO myeloid neoplasm and acute leukemia classification | Dysplastic findings | Cytopenias[a] | PB and BM findings and cytogenetics |
|---|---|---|---|
| MDS with excess blasts (MDS-EB) | | | |
| MDS-EB-1 | 0-3 | 1-3 | BM 5-9% or PB 2-4%, no Auer Rods<br>Any cvtogenetics |
| MDS-EB-2 | 0-3 | 1-3 | BM 10-19% or PB 5-19% or Auer Rods<br>Any cytogenetics |
| MDS with isolated del(5q) | 1-3 | 1-2 | BM <5%, PB <1%, no Auer Rods<br>del(5q) alone or with 1 additional abnormality except -7 or del(7q) |
| MDS, unclassifiable (MDS-U) | | | |
| MDS-U with 1% blood blasts | 1-3 | 1-3 | BM <5%, PB =1%[c], no Auer Rods<br><br>Any cytogenetics |
| MDS-U with SLD and pancytopenia | 1 | 3 | BM <5%, PB <1%, no Auer Rods<br>Any cytogenetics |
| MDS-U based on defining cytogenetic abnormality | 0 | 1-3 | BM <5%, PB <1%, no Auer Rods<br>MDS-defininp abnormality[d] |

[a]Cytopenias defined as : hemoglobin, <10 g/dL, platelet count, <100 × $10^9$/L; and absolute neutrophil count, <1.8 × $10^9$/L. Rarely, MDS may present with mild anemia or thrombocytopenia above these levels. Peripheral blood monocytes must be < 1 × $10^9$/L.
[b]Cases with ≥ 15% ring sideroblasts by definition have significant erythroid dysplasia, and are classified as MDS-RS-SLD.
[c]One percent PB blasts must be recorded on at least 2 separate occasions.
[d]Abnormality must be demonstrated by conventional karyotyping, not by FISH or sequencing. The presence of +8, -Y, of del(20q) is not considered to be MDS-defining in the absence of diagnostic morphologic features of MDS. Arber, et al. Blood 2016; 127(20):2391-2405, and Vardiman, et al. Blood. 2009; 114(5):937-51.

As used herein, "promyelocytic leukemia" or "acute promyelocytic leukemia" refers to a malignancy of the bone marrow in which there is a deficiency of mature blood cells in the myeloid line of cells and an excess of immature cells called promyelocytes. It is usually marked by an exchange of regions of chromosomes 15 and 17.

As used herein, "acute lymphocytic leukemia (ALL)", also known as "acute lymphoblastic leukemia" refers to a malignant disease caused by the abnormal growth and development of early nongranular white blood cells, or lymphocytes.

As used herein, "T- cell leukemia" refers to a disease in which certain cells of the lymphoid system called T lymphocytes or T cells are malignant. T cells are white blood cells that normally can attack virus-infected cells, foreign cells, and cancer cells and produce substances that regulate the immune response.

The term "relapsed" refers to a situation where patients who have had a remission of leukemia after therapy have a return of leukemia cells in the marrow and a decrease in normal blood cells.

The term "refractory or resistant" refers to a circumstance where patients, even after intensive treatment, have residual leukemia cells in their marrow.

The term "drug resistance" refers to the condition when a disease does not respond to the treatment of a certain drug or drugs. Drug resistance can be either intrinsic, which means the disease has never been responsive to the particular drug or drugs, or it can be acquired, which means the disease ceases responding to particular a drug or drugs that the disease had previously responded to. In certain embodiments, drug resistance is intrinsic. In certain embodiments, the drug resistance is acquired.

As used herein, and unless otherwise specified, a "therapeutically effective amount" of a compound is an amount sufficient to provide a therapeutic benefit in the treatment or management of a disease or disorder, or to delay or minimize one or more symptoms associated with the disease or disorder. A therapeutically effective amount of a compound means an amount of therapeutic agent, alone or in combination with other therapies, which provides a therapeutic benefit in the treatment or management of the disease or disorder. The term "therapeutically effective amount"

can encompass an amount that improves overall therapy, reduces or avoids symptoms or causes of disease or disorder, or enhances the therapeutic efficacy of another therapeutic agent.

As used herein, and unless otherwise specified, a "prophylactically effective amount" of a compound is an amount sufficient to prevent a disease or disorder, or prevent its recurrence. A prophylactically effective amount of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

As used herein, ECOG status refers to Eastern Cooperative Oncology Group (ECOG) Performance Status (Oken M, et al Toxicity and response criteria of the Eastern Cooperative Oncology Group. Am J Clin Oncol 1982;5(6):649-655), as shown below:

| Score | Description |
|---|---|
| **0** | Fully active, able to carry on all pre-disease performance without restriction |
| **1** | Restricted in physically strenuous activity but ambulatory and able to carry out work of a light or sedentary nature, eg, light housework, office work. |
| **2** | Ambulatory and capable of all self-care but unable to carry out any work activities. Up and about more than 50% of waking hours. |
| **3** | Capable of only limited self-care, confined to bed or chair more than 50% of waking hours. |
| **4** | Completely disabled. Cannot carry on any self-care. Totally confined to bed or chair |
| **5** | Dead |

In the context of a cancer, treatment or inhibition may be assessed by inhibition of disease progression, inhibition of tumor growth, reduction of primary tumor, relief of tumorrelated symptoms, inhibition of tumor secreted factors, delayed appearance of primary or secondary tumors, slowed development of primary or secondary tumors, decreased occurrence of primary or secondary tumors, slowed or decreased severity of secondary effects of disease, arrested tumor growth and regression of tumors, increased Time To Progression (TTP), increased Progression Free Survival (PFS), increased Overall Survival (OS), among others. OS as used herein means the time from treatment onset until death from any cause. TTP as used herein means the time from treatment onset until tumor progression; TTP does not include deaths. Time to Remission (TTR) as used herein means the time from treatment onset until remisison, for example, complete or partial remission. As used herein, PFS means the time from treatment onset until tumor progression or death. In one embodiment, PFS rates will be computed using the Kaplan-Meier estimates. Event-free survival (EFS) means the time from study entry until any treatment failure, including disease progression, treatment discontinuation for any reason, or death. Relapse-free survival (RFS) means the length of time after the treatment ends that the patient survives without any signs or symptoms of that cancer. Overall response rate (ORR) means the sum of the percentage of patients who achieve complete and partial responses. Complete remission rate (CRR) refers to the percentage of patients achieving complete remission (CR). Duration of response (DoR) is the time from achieving a response until relapse or disease progression. Duration of remission is the time from achieving remission, for example, complete or partial remission, until relapse. In the extreme, complete inhibition, is referred to herein as prevention or chemoprevention. In this context, the term "prevention" includes either preventing the onset of clinically evident cancer altogether or preventing the onset of a preclinically evident stage of a cancer. Also intended to be encompassed by this definition is the prevention of transformation into malignant cells or to arrest or reverse the progression of premalignant cells to malignant cells. This includes prophylactic treatment of those at risk of developing a cancer.

For leukemia, in particular AML, response to treatment can be assessed based on the International Working Group Response Criteria in AML (Cheson et al. J Clin Oncol 2003; 21(24):4642-9).

Hematologic Response According to IWG Criteria for AML

| Response Criterion | Time of Assessment | Neutrophi ls (μL) | Platelets (μL) | Bone Marrow Blasts (%) | Other |
|---|---|---|---|---|---|
| Early Treatment assessment | 7-10 days after therapy | NA | NA | < 5 | |

(continued)

| Response Criterion | Time of Assessment | Neutrophi ls (μL) | Platelets (μL) | Bone Marrow Blasts (%) | Other |
|---|---|---|---|---|---|
| Morphologic Leukemia-free State | Varies by protocol | NA | NA | < 5 | Flow cytometry EMD |
| Morphologic CR | Varies by protocol | ≥ 1,000 | ≥100,000 | < 5 | Transfusion EMD |
| Cytogenetic CR (CRc) | Varies by protocol | ≥ 1,000 | ≥100,000 | < 5 | Cytogenetics -normal, EMD |
| Molecular CR (CRm) | Varies by protocol | ≥ 1,000 | ≥100,000 | < 5 | Molecularnegative, EMD |
| Morphologic CR with incomplete blood recovery (CRi) | Varies by protocol | Fulfill all criteria for CR except for residual neutropenia (< 1,000/ μL) or thrombocytopenia (< 100,000/μL). | | | |
| Partial Remission | Varies by protocol | ≥ 1,000 | ≥100,000 | Decrease ≥ 50 resulting in 5 to 25 | Blasts ≤ 5% if Auer rod positive |
| Relapse after CR | Varies by protocol | Reappearance of leukemic blasts in the peripheral blood or ≥ 5% blasts in the bone marrow not attributable to any other cause (eg, bone marrow regeneration after consolidation therapv). | | | |
| Key: CR = complete remission; EMD = extramedullary disease; IWG = International Working Group; NA = not applicable. | | | | | |

The treatment of lymphoma may be assessed by the International Workshop Criteria (IWC) for NHL *(see* Cheson BD, et al. J. Clin. Oncol: 2007: (25) 579-586), using the response and endpoint definitions shown below:

| Response | Definition | Nodal Masses | Spleen, liver | Bone Marrow |
|---|---|---|---|---|
| CR | Disappearance of all evidence of disease | (a) FDG-avid or PET positive prior to therapy; mass of any size permitted if PET negative (b) Variably FDG-avid or PET negative; regression to normal size on CT | Not palpable, nodules disappeared | Infiltrate cleared on repeat biopsy; if indeterminate by morphology, immunohistochemistry should be negative |
| PR | Regression of measurable disease and no new sites | ≥50% decrease in SPD of up to 6 largest dominant masses; no increase in size of other nodes<br>(a) FDG-avid or PET positive prior to therapy; one or more PET positive at previously involved site (b) Variably FDG-avid or PET negative; regression on CT | ≥50% decrease in SPD of nodules (for single nodule in greatest transverse diameter); no increase in size of liver or spleen | Irrelevant if positive prior to therapy; cell type should be specified |
| SD | Failure to attain CR/PR or PD | (a) FDG-avid or PET positive prior to therapy; PET positive at prior sites of disease and no new sites on CT or PET (b) Variably FDG-avid or PET negative; no change in size of previous lesions on CT | | |

(continued)

| Response | Definition | Nodal Masses | Spleen, liver | Bone Marrow |
|---|---|---|---|---|
| PD or relapsed disease | Any new lesion or increase by ≥ 50% of previously involved sites from nadir | Appearance of a new lesion(s) ≥1.5 cm in any axis, ≥50% increase in SPD of more than one node, or ≥50% increase in longest diameter of a previously identifed node ≥1 cm in short axis Lesions PET positive if FDG-avid lymphoma or PET positive prior to therapy | ≥50% increase from nadir in the SPD of any previous lesions | New or recurrent involvement |
| Abbreviations: CR, complete remission; FDG, [18F]fluorodeoxyglucose; PET, positron emission tomography; CT, computed tomography; PR, partial remission; SPD, sum of the product of the diameters; SD, stable disease; PD, progressive disease. | | | | |

| End point | Patients | Definition | Measured from |
|---|---|---|---|
| **Primary** | | | |
| Overall survival | All | Death as a result of any cause | Entry onto study |
| Progressionfree survival | All | Disease progression or death as a result of any cause | Entry onto study |
| **Secondary** | | | |
| Event-free survival | All | Failure of treatment or death as result of any cause | Entry onto study |
| Time to progression | All | Time to progression or death as a result of lymphoma | Entry onto study |
| Disease-free survival | In CR | Time to relapse or death as a result of lymphoma or acute toxicity of treatment | Documentation of response |
| Response duration | In CR or PR | Time to relapse or progression | Documentation of response |
| Lymphomaspecific survival | All | Time to death as a result of lymphoma | Entry onto study |
| Time to next treatment | All | Time to new treatment | End of primary treatment |
| Abbreviations: CR: complete remission; PR: partial remission. | | | |

In one embodiment, the end point for lymphoma is evidence of clinical benefit. Clinical benefit may reflect improvement in quality of life, or reduction in patient symptoms, transfusion requirements, frequent infections, or other parameters. Time to reappearance or progression of lymphoma-related symptoms can also be used in this end point.

The treatment of CLL may be assessed by the International Workshop Guidelines for CLL *(see* Hallek M, et al. Blood, 2008; (111) 12: 5446-5456) using the response and endpoint definitions shown therein and in particular:

| Parameter | CR | PR | PD |
|---|---|---|---|
| **Group A** | | | |
| Lymphadenopathy† | None > 1.5 cm | Decrease ≥ 50% | Increase ≥ 50% |
| Hepatomegaly | None | Decrease ≥ 50% | Increase ≥ 50% |
| Splenomegaly | None | Decrease ≥ 50% | Increase ≥ 50% |

(continued)

| Parameter | CR | PR | PD |
|---|---|---|---|
| **Group A** | | | |
| Blood lymphocytes | < 4000/μL | Decrease ≥ 50% from baseline | Increase ≥ 50% over baseline |
| Marrow‡ | Normocellular, < 30% lymphocytes, no B-lymphoid nodules. Hypocellular marrow defines CRi (5.1.6). | 50% reduction in marrow infiltrate, or B-lymphoid nodules | |
| **Group B** | | | |
| Platelet count | > 100 000/μL | > 100 000/μL or increase ≥ 50% over baseline | Decrease of ≥ 50% from baseline secondary to CLL |
| Hemoglobin | > 11.0 g/dL | > 11 g/dL or increase ≥ 50% over baseline | Decrease of > 2 g/dL from baseline secondary to CLL |
| Neutrophils‡ | > 1500/μL | > 1500/μL or > 50% improvement over baseline | |

Group A criteria define the tumor load; Group B criteria define the function of the hematopoietic system (or marrow). CR (complete remission): all of the criteria have to be met, and patients have to lack disease-related constitutional symptoms; PR (partial remission): at least two of the criteria of group A plus one of the criteria of group B have to be met; SD is absence of progressive disease (PD) and failure to achieve at least a PR; PD: at least one of the above criteria of group A or group B has to be met. Sum of the products of multiple lymph nodes (as evaluated by CT scans in clinical trials, or by physical examination in general practice). These parameters are irrelevant for some response categories.

The treatment of MM may be assessed by the International Uniform Response Criteria for Multiple Myeloma (IURC) *(see* Durie et al. Leukemia, 2006; (10) 10: 1-7), using the response and endpoint definitions shown below:

| Response Subcategory | Response Criteria[a] |
|---|---|
| sCR | CR as defined below plus Normal FLC ratio and Absence of clonal cells in bone marrow[b] by immunohistochemistry or immunofluorescence[c] |
| CR | Negative immunofixation on the serum and urine and Disappearance of any soft tissue plasmacytomas and <5% plasma cells in bone marrow[b] |
| VGPR | Serum and urine M-protein detectable by immunofixation but not on electrophoresis or 90% or greater reduction in serum M-protein plus urine M-protein level <100mg per 24 h |
| PR | ≥50% reduction of serum M-protein and reduction in 24-h urinary M-protein by≥90% or to <200mg per 24 h If the serum and urine M-protein are unmeasurable,[d] a ≥50% decrease in the difference between involved and uninvolved FLC levels is required in place of the M-protein criteria If serum and urine M-protein are unmeasurable, and serum free light assay is also unmeasurable, ≥50% reduction in plasma cells is required in place of M-protein, provided baseline bone marrow plasma cell percentage was ≥30% In addition to the above listed criteria, if present at baseline, a ≥50% reduction in the size of soft tissue plasmacytomas is also required |

(continued)

| Response Subcategory | Response Criteria[a] |
|---|---|
| SD (not recommended for use as an indicator of response; stability of disease is best described by providing the time to progression estimates) | Not meeting criteria for CR, VGPR, PR or progressive disease |
| Abbreviations: CR, complete response; FLC, free light chain; PR, partial response; SD, stable disease; sCR, stringent complete response; VGPR, very good partial response; [a]All response categories require two consecutive assessments made at anytime before the institution of any new therapy; all categories also require no known evidence of progressive or new bone lesions if radiographic studies were performed. Radiographic studies are not required to satisfy these response requirements; [b]Confirmation with repeat bone marrow biopsy not needed; [c]Presence/absence of clonal cells is based upon the κ/λ ratio. An abnormal κ/λ ratio by immunohistochemistry and/or immunofluorescence requires a minimum of 100 plasma cells for analysis. An abnormal ratio reflecting presence of an abnormal clone is κ/λ of >4:1 or <1:2.[d]Measurable disease defined by at least one of the following measurements: Bone marrow plasma cells ≥30%; Serum M-protein ≥1 g/dl (≥10 gm/1)[10 g/1]; Urine M-protein ≥200 mg/24 h; Serum FLC assay: Involved FLC level ≥10 mg/dl (≥100 mg/1); provided serum FLC ratio is abnormal. | |

The treatment of a cancer may also be assessed by Response Evaluation Criteria in Solid Tumors (RECIST 1.1) (*see* Thereasse P., et al. J. of the National Cancer Institute; 2000; (92) 205-216 and Eisenhauer et al. European J. Cancer; 2009; (45) 228-247). Overall responses for all possible combinations of tumor responses in target and non-target lesions with our without the appearance of new lesions are as follows:

| Target lesions | Non-target lesions | New lesions | Overall response |
|---|---|---|---|
| CR | CR | No | CR |
| CR | Incomplete response/SD | No | PR |
| PR | Non-PD | No | PR |
| SD | Non-PD | No | SD |
| PD | Any | Yes or no | PD |
| Any | PD | Yes or no | PD |
| Any | Any | Yes | PD |
| CR = complete response; PR = partial response; SD = stable disease; and PD = progressive disease. | | | |

With respect to the evaluation of target lesions, complete response (CR) is the disappearance of all target lesions, partial response (PR) is at least a 30% decrease in the sum of the longest diameter of target lesions, taking as reference the baseline sum longest diameter, progressive disease (PD) is at least a 20% increase in the sum of the longest diameter of target lesions, taking as reference the smallest sum longest diameter recorded since the treatment started or the appearance of one or more new lesions and stable disease (SD) is neither sufficient shrinkage to qualify for partial response nor sufficient increase to qualify for progressive disease, taking as reference the smallest sum longest diameter since the treatment started.

With respect to the evaluation of non-target lesions, complete response is the disappearance of all non-target lesions and normalization of tumor marker level; incomplete response/stable disease is the persistence of one or more non-target lesion(s) and/or the maintenance of tumor marker level above the normal limits, and progressive disease (PD) is the appearance of one or more new lesions and/or unequivocal progression of existing non-target lesions.

The treatment of MDS may be assessed by International Working Group (IWG) Response Criteria for Myelodysplasia.

**Modified IWG Response Criteria for MDS**

| **Category** | **Response criteria (responses must last at least 4 weeks)** |
|---|---|
| Complete remission (CR) | Bone marrow: ≤ 5% myeloblasts with normal maturation of all cell lines[a] |
| | Persistent dysplasia will be noted[a,b]<br>Peripheral blood[c]<br>- Hemoglobin ≥ 11 g/dL<br>- Platelets ≥ 100 × $10^9$/L<br>- Neutrophils ≥ 1.0 × $10^9$/L[b]<br>- Blasts 0% |
| Partial remission (PR) | All CR criteria if abnormal before treatment, except:<br>Bone marrow blasts decreased by ≥ 50% over pretreatment but still > 5%<br>Cellularity and morphology not relevant |
| Marrow CR[b] ± Hematologic Improvement (HI) | Bone marrow: ≤ 5% myeloblasts and decrease by ≥ 50% over pretreatmentb Note: Blasts at baseline must be ≥ 5% in order for subject to be evaluable for Marrow CR[d]<br>Peripheral blood: if HI responses, they will be noted in addition to marrow CR[b] |
| Stable disease (SD) | Failure to achieve at least PR, but no evidence of progression for > 8 weeks |
| Failure | Death during treatment or disease progression characterized by worsening of cytopenias, increase in percentage of bone marrow blasts, or progression to a more advanced MDS FAB subtype than pretreatment |
| Relapse after CR or PR | At least 1 of the following:<br>• Return to pretreatment bone marrow blast percentage<br>• Decrement of ≥50% from maximum remission/response levels in granulocytes or platelets<br>• Reduction of Hgb concentration by ≥ 1.5 g/dL or transfusion dependence |
| Cytogenetic Response | Complete - Disappearance of the chromosomal abnormality without appearance of new ones<br>Partial - At least 50% reduction of the chromosomal abnormality |

| **Category** | **Response criteria (responses must last at least 4 weeks)** |
|---|---|
| Disease Progression (PD) | For patients with:<br>• Less than 5% blasts: ≥ 50% increase in blasts to > 5% blasts<br>• 5% - 10% blasts: ≥ 50% increase in blasts to > 10% blasts<br>• 10% - 20% blasts: ≥ 50% increase in blasts to > 20% blasts<br>Any of the following:<br>• At least 50% decrement from maximum remission/response levels in granulocytes or platelets<br>• Reduction in Hgb concentration by ≥ 2 g/dL<br>• Transfusion dependence |
| Disease transformation | Transformation to AML (20% or more BM or PB blasts)[d] |
| Hematologic Improvement (HI) | |

(continued)

| Category | Response criteria (responses must last at least 4 weeks) |
|---|---|
| Erythroid response (HI-E) (Pretreatment < 11 g/dL) | Hgb increase by ≥ 1.5 g/dL<br>Relevant reduction of units of RBC transfusions by an absolute number of at least 4 RBC transfusions/8 weeks compared with the pretreatment transfusion number in the previous 8 weeks. Only RBC transfusions given for a Hgb of ≤ 9.0 g/dL pretreatment will count in the RBC transfusion evaluation |
| Platelet response (HI-P) (Pretreatment < 100 × $10^9$/L) | Absolute increase of ≥ 30 × $10^9$/L for patients starting with > 20 × $10^9$/L<br>Increase from < 20 × $10^9$/L to > 20 × $10^9$/L and by at least 100% |
| Neutrophil response (HI-N) (Pretreatment < 1.0 × $10^9$/L) | At least 100% increase and an absolute increase of > 0.5 × $10^9$/L |
| Progression/relapse after HI | At least one of the following:<br>• At least 50% decrement from maximum response levels in granulocytes or platelets<br>• Reduction in Hgb by ≥ 1.5 g/dL<br>• Transfusion dependence |

BM = bone marrow; CR = complete remission; FAB = French-American-British; Hgb = hemoglobin; HI = hematologic improvement; IWG = International Working Group; MDS = myelodysplastic syndromes; PB = peripheral blood; PD = Disease Progression; PR = partial remission; RBC = red blood cell.
[a] Dysplastic changes should consider the normal range of dysplastic changes (modification).
[b] Modification to IWG response criteria.
[c] In some circumstances, protocol therapy may require the initiation of further treatment (eg, consolidation, maintenance) before the 4-week period. Such subjects can be included in the response category into which they fit at the time the therapy is started. Transient cytopenias during repeated chemotherapy courses should not be considered as interrupting durability of response, as long as they recover to the improved counts of the previous course.
[d] Sponsor modification of IWG criteria.
Sources: Cheson, 2006 and Vardiman, 2008.

**RBC and Platelet Transfusion Independence**

| | At Screening | During Study Treatment |
|---|---|---|
| RBC transfusion independence | Subjects who received < 4 RBC units during the previous 56 days | Subjects who experienced a Hgb increase of 1.5 g/dL over baseline and who received no RBC transfusions during a 56-day period on treatment. Note: Only RBC transfusions given for a Hgb of ≤ 9.0 g/dL within 3 days prior to the transfusion will count in the RBC transfusion response evaluation |
| RBC transfusion dependence | Subjects who received ≥ 4 RBC units during the previous 56 days | |
| Platelet transfusion independence | Subjects who received < 2 platelet transfusions during the previous 56 days | Subjects who received no platelet transfusions during a 56-day period on treatment |

(continued)

| | At Screening | During Study Treatment |
|---|---|---|
| Platelet transfusion dependence | Subjects who received ≥ 2 platelet transfusions during the previous 56 days. | |
| RBC = red blood cell; Hgb = hemoglobin.<br>[a] RBC transfusion independence and RBC transfusion dependence are defined according to modified IWG criteria.<br>[b] Platelet transfusion independence and platelet transfusion dependence are defined by the Sponsor.<br>Source: Cheson, et al. Blood. 2006; 108(2):419-25. | | |

Revised International Prognostic Scoring System is used for prognosis of MDS as follows:

**IPSS-R Cytogenetic Risk Group**

| Cytogenetic Prognostic Subgroups | Cytogenetic Abnormalities |
|---|---|
| Very good | -Y, del(11q) |
| Good | Normal, del(5q), del(12p), del(20q), double including del(5q) |
| Intermediate | del(7q), +8, +19, i(17q), any other single or double independent clones |
| Poor | -7, inv(3)/t(3q)/del(3q), double including -7/del(7q), Complex: 3 abnormalities |
| Very poor | Complex: > 3 abnormalities |
| Source: Greenburg, et al. Blood. 2012;120(12):2454-65. | |

**IPSS-R Prognostic Score Values**

| Prognostic variable | 0 | 0.5 | 1 | 1.5 | 2 | 3 | 4 |
|---|---|---|---|---|---|---|---|
| Cytogenetics | Very Good | - | Good | - | Inter - mediate | Poor | Very Poor |
| Bone Marrow Blast (%) | ≤ 2 | - | > 2 - < 5 | - | 5 - 10 | > 10 | - |
| Hemoglobin (g/dL) | ≥10 | - | 8 - < 10 | <8 | - | - | - |
| Platelets (× $10^9$/L) | ≥100 | 50 - < 100 | < 50 | - | - | - | - |
| ANC (× $10^9$/L) | ≥0.8 | < 0.8 | - | - | - | - | - |
| Source: Greenburg, et al. Blood. 2012;120(12):2454-65. | | | | | | | |

The total IPSS-R score is calculated as the sum of the cytogenetics, bone marrow blast percentage, hemoglobin, platelets and ANC individual scores.

**IPSS-R Prognostic Risk Categories/Scores**

| Risk Category | Risk Score |
|---|---|
| Very Low | ≤ 1.5 |

(continued)

| Risk Category | Risk Score |
|---|---|
| Low | > 1.5 - 3 |
| Intermediate | > 3 - 4.5 |
| High | > 4.5 - 6 |
| Very High | > 6 |
| Source: Greenburg, et al. Blood. 2012;120(12): 2454-65. | |

**IPSS-R: Prognostic Risk Category Clinical Outcomes**

| Prognostic variable | No. pts | Very Low | Low | Intermediate | High | Very High |
|---|---|---|---|---|---|---|
| Patients, % | 7012 | 19% | 38% | 20% | 13% | 10% |
| Median Overall Survival (years) | - | 8.8 | 5.3 | 3.0 | 1.6 | 0.8 |
| Median time to 25% AML evolution | - | Not reached | 10.8 | 3.2 | 1.4 | 0.73 |
| Source: Greenberg, et al. Blood. 2012;120(12):2454-65 | | | | | | |

**Compound**

The compound suitable for use in the methods and formulations provided herein is Compound 1: 2-(4-chlorophenyl)-N-((2-(2,6-dioxopiperidin-3-yl)-1-oxoisoindolin-5-yl)methyl)-2,2-difluoroacetamide having the structure:

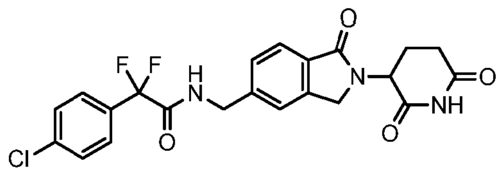

or its stereoisomers or mixture of stereoisomers, isotopologues, pharmaceutically acceptable salts, tautomers, solvates, hydrates, co-crystals, clathrates, or polymorphs thereof. In certain embodiments, Compound 1 refers to 2-(4-chlorophenyl)-N-((2-(2,6-dioxopiperidin-3-yl)-1-oxoisoindolin-5-yl)methyl)-2,2-difluoroacetamide.

Compound 1 can be prepared according to the methods described in the Examples provided herein or as described in U.S. Patent No. 9,499; 514. The compound can also be synthesized according to other methods apparent to those of skill in the art based upon the teaching herein.

In certain embodiments, Compound 1 is a solid. In certain embodiments, Compound 1 is a hydrate. In certain embodiments, Compound 1 is solvated. In certain embodiments, Compound 1 is anhydrous.

In certain embodiments, Compound 1 is amorphous. In certain embodiments, Compound 1 is crystalline. In certain embodiments, Compound 1 is in a crystalline form described in U.S. Publication No. 2017-0197934 filed on January 6, 2017. Exemplary solid forms are described on page nos. 86-101.

The solid forms of Compound 1 can be prepared according to the methods described in the disclosure of U.S. Publication No. 2017-0197934 filed on January 6, 2017. *See* page nos. 86-101. The solid forms can also be prepared according to other methods apparent to those of skill in the art.

In one embodiment, Compound 1 is polymorph Form A, Form B, Form C, Form D, Form E or an amorphous form of 2-(4-chlorophenyl)-N-((2-(2,6-dioxopiperidin-3-yl)-1-oxoisoindolin-5-yl)methyl)-2,2-difluoroacetamide. Polymorphs of 2-(4-chlorophenyl)-N-((2-(2,6-dioxopiperidin-3-yl)-1-oxoisoindolin-5-yl)methyl)-2,2-difluoroacetamide are briefly described herein.

**Form A of 2-(4-chlorophenyl)-N-((2-(2,6-dioxopiperidin-3-yl)-1-oxoisoindolin-5-yl)methyl)- 2,2-difluoroacetamide**

In certain embodiments, the formulations provided herein are prepared from Form A of Compound 1.

In one embodiment, Form A is an anhydrous form of Compound 1. In another embodiment, Form A of Compound 1 is crystalline.

In certain embodiments, Form A is obtained by crystallization from certain solvent systems, for example, solvent systems comprising one or more of the following solvents: acetone and the solvent mixture of isopropanol and water at room temperature. In certain embodiments, Form A is obtained as an intermediate solid form from slurries at elevated temperature, for example about 50 °C, in ethanol/water (1:1), acetone or acetonitrile.

In certain embodiments, Form A is substantially crystalline, as indicated by, *e.g.,* X-ray powder diffraction measurements. In one embodiment, Form A of Compound 1 has an X-ray powder diffraction pattern substantially as shown in **FIG. 2.**

In one embodiment, Form A of Compound 1 has one or more characteristic X-ray powder diffraction peaks at a two-theta angle of approximately 11.5, 15.6, 16.6, 17.2, 18.1, 19.0, 19.6, 21.1, 23.2 or 24.8 degrees 2θ as depicted in **FIG. 2.** In another embodiment, Form A of Compound 1 has one, two, three or four characteristic X-ray powder diffraction peaks at a two-theta angle of approximately 15.6, 16.6, 17.2 or 24.8 degrees 2θ. In another embodiment, Form A of Compound 1 has one, two, three, four, five, six or seven characteristic X-ray powder diffraction peaks as set forth in Table A. In another embodiment, Form A of Compound 1 has one, two, or three characteristic X-ray powder diffraction peaks as set forth in Table A.

**Table A**

| No. | Pos. [°2Th.] | d-spacing [Å] | Rel. Int. [%] |
|---|---|---|---|
| 1 | 7.23 | 12.2187 | 17.6 |
| 2 | 11.52 | 7.6789 | 29.7 |
| 3 | 15.22 | 5.8209 | 7.5 |
| 4 | 15.62 | 5.6720 | 31.2 |
| 5 | 16.58 | 5.3466 | 40.3 |
| 6 | 17.19 | 5.1576 | 100.0 |
| 7 | 18.08 | 4.9056 | 22.3 |
| 8 | 19.00 | 4.6702 | 19.6 |
| 9 | 19.60 | 4.5302 | 22.1 |
| 10 | 21.05 | 4.2197 | 29.2 |
| 11 | 21.74 | 4.0884 | 8.3 |
| 12 | 22.01 | 4.0388 | 7.1 |
| 13 | 22.47 | 3.9576 | 6.0 |
| 14 | 23.22 | 3.8312 | 28.6 |
| 15 | 24.17 | 3.6825 | 5.6 |
| 16 | 24.77 | 3.5945 | 57.2 |
| 17 | 25.59 | 3.4813 | 14.6 |
| 18 | 25.94 | 3.4356 | 10.5 |
| 19 | 26.63 | 3.3470 | 17.4 |
| 20 | 27.73 | 3.2172 | 10.0 |
| 21 | 28.51 | 3.1307 | 7.1 |
| 22 | 29.88 | 2.9906 | 19.3 |
| 23 | 30.76 | 2.9065 | 7.1 |

(continued)

| No. | Pos. [°2Th.] | d-spacing [Å] | Rel. Int. [%] |
|---|---|---|---|
| 24 | 31.59 | 2.8327 | 11.1 |
| 25 | 34.82 | 2.5766 | 4.8 |
| 26 | 36.05 | 2.4913 | 4.3 |

In one embodiment, Form A of Compound 1 has the SEM picture as shown in **FIG. 3.**

In one embodiment, the crystalline form of Compound 1 has a thermogravimetric (TGA) thermograph corresponding substantially to the representative TGA thermogram as depicted in **FIG. 4.** In certain embodiments, no TGA weight loss is observed for Form A.

In one embodiment, crystalline form A of Compound 1 has a DSC thermogram corresponding substantially as depicted in **FIG. 5.** In certain embodiments, Form A is characterized by a DSC plot comprising a melting event with an onset temperature of 229 °C and heat of fusion of 118 J/g.

In certain embodiments, Form A is characterized by dynamic vapor sorption analysis. A representative dynamic vapor sorption (DVS) isotherm plot is shown in **FIG. 6.** In certain embodiments, when the relative humidity ("RH") is increased from about 0% to about 90% RH, Form A exhibits less than 1.5%, less than 1.2% or about 1.2 %w/w water uptake. In certain embodiments, Form A comprises less than 0.1% water as determined in a coulometric Karl Fischer (KF) titrator equipped with an oven sample processor set at 225 °C.

In certain embodiments, no significant degradation or residual solvent for Form A is observed by $^1$H NMR **(FIG. 7).**

In certain embodiments, Form A of Compound 1 is characterized by its stability profile upon compression. In certain embodiments, Form A is stable, *e.g.,* its XRPD pattern remains substantially unchanged with broader diffraction peaks, upon application of 2000-psi pressure for about 1 minute **(FIG. 8).**

In still another embodiment, Form A of Compound 1 is substantially pure. In certain embodiments, the substantially pure Form A of Compound 1 is substantially free of other solid forms, e.g., amorphous form. In certain embodiments, the purity of the substantially pure Form A of Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.

Certain embodiments Form A of Compound 1 is substantially pure. In certain embodiments herein Form A of Compound 1 is substantially free of other solid forms comprising Compound 1 including, e.g., Forms B, C, D, E and/or an amorphous solid form comprising Compound 1. In certain embodiments, Form A is a mixture of solid forms comprising Compound 1, including, *e.g.,* a mixture comprising one or more of the following: Forms B, C, D, E and an amorphous solid form comprising Compound 1.

**Form B of 2-(4-chlorophenyl)-N-((2-(2,6-dioxopiperidin-3-yl)-1-oxoisoindolin-5-yl)methyl)-2,2-difluoroacetamide**

In certain embodiments, the formulations provided herein are prepared from anhydrous Form B of Compound 1.

In certain embodiments, Form B is obtained by anti-solvent recrystallization from certain solvent systems, for example, solvent systems comprising one or more of the following solvents: methanol/water, DMSO/isopropanol, DMSO/toluene, and DMSO/water. In certain embodiments, Form B is obtained by cooling recrystallization from THF/water (1:1).

In certain embodiments, Form B is crystalline, as indicated by, e.g., X-ray powder diffraction measurements. In one embodiment, Form B of Compound 1 has an X-ray powder diffraction pattern substantially as shown in **FIG. 9.**

In one embodiment, Form B of Compound 1 has one or more characteristic X-ray powder diffraction peaks at a two-theta angle of approximately 15.4, 16.3, 16.7, 17.7, 20.4, 25.6 or 27.5, degrees 2$\theta$ as depicted in **FIG. 9.** In another embodiment, Form B of Compound 1 has one, two, three or four characteristic X-ray powder diffraction peaks at a two-theta angle of approximately 16.7, 25.6, 15.4 or 16.3 degrees 2$\theta$. In another embodiment, Form B of Compound 1 has one, two, three, four, five, six or seven characteristic X-ray powder diffraction peaks as set forth in Table B. In another embodiment, Form B of Compound 1 has one, two, or three characteristic X-ray powder diffraction peaks as set forth in Table B.

**Table B**

| No. | Pos. [°2Th.] | d-spacing [Å] | Rel. Int. [%] |
|---|---|---|---|
| 1 | 7.01 | 12.6035 | 9.3 |
| 2 | 11.58 | 7.6444 | 8.3 |

(continued)

| No. | Pos. [°2Th.] | d-spacing [Å] | Rel. Int. [%] |
|---|---|---|---|
| 3 | 11.80 | 7.5027 | 6.8 |
| 4 | 12.73 | 6.9551 | 18.4 |
| 5 | 15.38 | 5.7601 | 34.8 |
| 6 | 16.32 | 5.4330 | 31.4 |
| 7 | 16.72 | 5.3012 | 100.0 |
| 8 | 17.72 | 5.0046 | 26.6 |
| 9 | 18.13 | 4.8930 | 19.8 |
| 10 | 18.77 | 4.7271 | 7.5 |
| 11 | 20.41 | 4.3516 | 22.0 |
| 12 | 21.02 | 4.2258 | 15.9 |
| 13 | 21.21 | 4.1881 | 13.5 |
| 14 | 21.93 | 4.0529 | 3.4 |
| 15 | 23.68 | 3.7581 | 14.2 |
| 16 | 25.01 | 3.5601 | 10.4 |
| 17 | 25.63 | 3.4755 | 37.3 |
| 18 | 26.19 | 3.4030 | 9.8 |
| 19 | 26.73 | 3.3349 | 8.5 |
| 20 | 27.45 | 3.2499 | 20.9 |
| 21 | 27.71 | 3.2193 | 9.4 |
| 22 | 28.22 | 3.1623 | 11.8 |
| 23 | 29.48 | 3.0296 | 4.7 |
| 24 | 30.10 | 2.9692 | 15.0 |
| 25 | 31.08 | 2.8775 | 18.3 |
| 26 | 31.65 | 2.8272 | 6.2 |
| 27 | 34.29 | 2.6150 | 3.4 |

In one embodiment, Form B of Compound 1 has the SEM picture as shown in **FIG. 10.** In one embodiment, a crystalline form of Compound 1 has a thermogravimetric (TGA) thermograph corresponding substantially to the representative TGA thermogram as depicted in **FIG. 11.** In certain embodiments, Form B shows no TGA weight loss below 170 °C. In certain embodiments, Form B shows a TGA weight loss of 0.4 % between 170~230 °C.
In one embodiment, crystalline Form B of Compound 1 has a DSC thermogram corresponding substantially as depicted in **FIG. 12.** In certain embodiments, Form B is characterized by a DSC plot comprising a melt/recrystallization event at 219~224 °C and a major melting event with a peak temperature of 231 °C.
In certain embodiments, Form B is characterized by dynamic vapor sorption analysis. A representative dynamic vapor sorption (DVS) isotherm plot is shown in **FIG. 13.** In certain embodiments, when the relative humidity ("RH") is increased from about 0% to about 90% RH, Form B exhibits about 1.4%w/w water uptake. In certain embodiments, Form B comprises less than 0.1% water as determined in a coulometric Karl Fischer (KF) titrator equipped with an oven sample processor set at 225 °C.
In certain embodiments, Form B shows no significant degradation or residual solvent by $^1H$ NMR **(FIG. 14).**
In certain embodiments, Form B of Compound 1 is characterized by its stability profile upon compression. In certain embodiments, Form B is stable, *e.g.,* its XRPD pattern remains substantially unchanged with broader diffraction peaks, upon application of 2000-psi pressure for about 1 minute **(FIG. 15).**
In still another embodiment, Form B of Compound 1 is substantially pure. In certain embodiments, the substantially pure Form B of Compound 1 is substantially free of other solid forms, e.g., amorphous form. In certain embodiments, the purity of the substantially pure Form B of Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.

Certain embodiments, Form B of Compound 1 is substantially pure. In certain embodiments, Form B of Compound 1 is substantially free of other solid forms comprising Compound 1 including, e.g., Forms A, C, D, E, and/or an amorphous solid form comprising Compound 1. In certain embodiments, Form B is a mixture of solid forms comprising Compound 1, including, *e.g.,* a mixture comprising one or more of the following: Forms A, C, D, E, and an amorphous solid form comprising Compound 1.

**Form C of 2-(4-chlorophenyl)-N-((2-(2,6-dioxopiperidin-3-yl)-1-oxoisoindolin-5-yl)methyl)-2,2-difluoroacetamide**

In certain embodiments, the formulations provided herein are prepared from anhydrous Form C of Compound 1. In certain embodiments, Form C is the most thermodynamically stable anhydrate among the crystal forms of Compound 1.

In certain embodiments, Form C is obtained by slurrying Compound 1 in certain solvent systems, for example, solvent systems comprising one or more of the following solvents: acetonitril/water, acetone, or ethanol/water for extended period of time.

In certain aspects, Form C is obtained by slurrying Form B (IX wt) in acetone (30 X vol) at an elevated temperature, for example, from 60-80 °C or 70-75 °C for at least 24 hours, and cooling the mixture to room temperature. In one aspect, the slurrying is conducted at a temperature of 70-75 °C under nitrogen pressure of 50-55-psi. In one aspect, the mixture is cooled to room temperature over at least 6 hours.

In certain embodiments, Form C is crystalline, as indicated by, e.g., X-ray powder diffraction measurements. In one embodiment, Form C of Compound 1 has an X-ray powder diffraction pattern substantially as shown in **FIG. 16.**

In one embodiment, Form C of Compound 1 has one or more characteristic X-ray powder diffraction peaks at a two-theta angle of approximately 7.4, 11.5, 15.8, 16.7, 16.9, 17.7, 18.4, 19.2, 19.5, 21.1, 23.4, 24.7, or 29.9, degrees 2$\theta$ as depicted in **FIG. 16.** In another embodiment, Form C of Compound 1 has one, two, three or four characteristic X-ray powder diffraction peaks at a two-theta angle of approximately 16.7, 16.9, 17.7 or 24.7 degrees 2$\theta$. In another embodiment, Form C of Compound 1 has one, two, three, four, five, six or seven characteristic X-ray powder diffraction peaks as set forth in Table C. In another embodiment, Form C of Compound 1 has one, two, or three characteristic X-ray powder diffraction peaks as set forth in Table C.

**Table C**

| No. | Pos. [°2Th.] | d-spacing [Å] | Rel. Int. [%] |
|---|---|---|---|
| 1 | 7.36 | 12.0091 | 32.0 |
| 2 | 9.14 | 9.6750 | 8.3 |
| 3 | 11.51 | 7.6855 | 44.7 |
| 4 | 12.22 | 7.2420 | 4.9 |
| 5 | 15.17 | 5.8398 | 8.4 |
| 6 | 15.82 | 5.6011 | 31.8 |
| 7 | 16.68 | 5.3140 | 57.1 |
| 8 | 16.92 | 5.2392 | 86.8 |
| 9 | 17.72 | 5.0057 | 100.0 |
| 10 | 18.39 | 4.8242 | 21.9 |
| 11 | 19.18 | 4.6268 | 36.4 |
| 12 | 19.45 | 4.5649 | 27.1 |
| 13 | 21.11 | 4.2077 | 40.4 |
| 14 | 21.82 | 4.0724 | 12.4 |
| 15 | 22.28 | 3.9902 | 12.0 |
| 16 | 22.57 | 3.9398 | 17.6 |

(continued)

| No. | Pos. [°2Th.] | d-spacing [Å] | Rel. Int. [%] |
|---|---|---|---|
| 17 | 23.36 | 3.8082 | 24.7 |
| 18 | 24.26 | 3.6695 | 7.1 |
| 19 | 24.71 | 3.6026 | 72.5 |
| 20 | 25.74 | 3.4615 | 16.9 |
| 21 | 26.03 | 3.4231 | 9.7 |
| 22 | 26.51 | 3.3627 | 17.7 |
| 23 | 27.88 | 3.1998 | 18.0 |
| 24 | 28.70 | 3.1104 | 6.9 |
| 25 | 29.91 | 2.9871 | 30.5 |
| 26 | 30.43 | 2.9375 | 10.7 |
| 27 | 30.83 | 2.9006 | 5.8 |
| 28 | 32.01 | 2.7960 | 16.6 |
| 29 | 37.94 | 2.3718 | 5.5 |

In one embodiment, Form C of Compound 1 has the SEM picture as shown in **FIG. 17.** In one embodiment, a crystalline form of Compound 1 has a thermogravimetric (TGA) thermograph corresponding substantially to the representative TGA thermogram as depicted in **FIG. 18.** In certain embodiments, Form C shows no TGA weight loss.
In one embodiment, crystalline Form C of Compound 1 has a DSC thermogram corresponding substantially as depicted in **FIG. 19.** In certain embodiments, Form C is characterized by a DSC plot comprising melting event with an onset temperature of 232 °C and heat of fusion of 126 J/g.
In certain embodiments, Form C is characterized by dynamic vapor sorption analysis. A representative dynamic vapor sorption (DVS) isotherm plot is shown in **FIG. 20.** In certain embodiments, when the relative humidity ("RH") is increased from about 0% to about 90% RH, Form C exhibits about 0.6%w/w water uptake. In certain embodiments, Form C comprises less than 0.1% water as determined in a coulometric Karl Fischer (KF) titrator equipped with an oven sample processor set at 225 °C.
In certain embodiments, Form C shows no significant degradation or residual solvent by $^1$H NMR **(FIG. 21).**
In certain embodiments, Form C of Compound 1 is characterized by its stability profile upon compression. In certain embodiments, Form C is stable, *e.g.,* its XRPD pattern remains substantially unchanged with broader diffraction peaks, upon application of 2000-psi pressure for about 1 minute **(FIG. 22).**
In still another embodiment, Form C of Compound 1 is substantially pure. In certain embodiments, the substantially pure Form C of Compound 1 is substantially free of other solid forms, e.g., amorphous form. In certain embodiments, the purity of the substantially pure Form C of Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.
In certain embodiments, Form C of Compound 1 is substantially pure. In certain embodiments, Form C of Compound 1 is substantially free of other solid forms comprising Compound 1 including, e.g., Forms A, B, D, E, and/or an amorphous solid form comprising Compound 1. In certain embodiments, Form C is a mixture of solid forms comprising Compound 1, including, *e.g.,* a mixture comprising one or more of the following: Forms A, B, D, E, and an amorphous solid form comprising Compound 1.

**Form D of 2-(4-chlorophenyl)-N-((2-(2,6-dioxopiperidin-3-yl)-1-oxoisoindolin-5-yl)methyl)-2,2-difluoroacetamide**

In certain embodiments, the formulations provided herein are prepared from Form D of Compound 1. In certain embodiments, Form D of Compound 1 is a DMSO solvate.
In certain embodiments, Form D is obtained by heating Form B in DMSO/methyl isobutyl ketone and cooling the solution.
In certain embodiments, Form D is crystalline, as indicated by, e.g., X-ray powder diffraction measurements. In one embodiment, Form D of Compound 1 has an X-ray powder diffraction pattern substantially as shown in **FIG. 23.**
In one embodiment, Form D of Compound 1 has one or more characteristic X-ray powder diffraction peaks at

a two-theta angle of approximately 14.1, 14.3, 18.8, 19.1, 23.6 or 24.0 degrees 2θ as depicted in **FIG. 23.** In another embodiment, Form D of Compound 1 has one, two, three or four characteristic X-ray powder diffraction peaks at a two-theta angle of approximately 14.1, 14.3, 18.8 or 19.1 degrees 2θ. In another embodiment, Form D of Compound 1 has one, two, three, four, five, six or seven characteristic X-ray powder diffraction peaks as set forth in Table D. In another embodiment, Form D of Compound 1 has one, two, or three characteristic X-ray powder diffraction peaks as set forth in Table D.

**Table D**

| No. | Pos. [°2Th.] | d-spacing [Å] | Rel. Int. [%] |
|---|---|---|---|
| 1 | 4.77 | 18.5435 | 3.0 |
| 2 | 9.57 | 9.2399 | 7.0 |
| 3 | 10.55 | 8.3876 | 3.1 |
| 4 | 11.95 | 7.4070 | 3.7 |
| 5 | 12.50 | 7.0808 | 3.5 |
| 6 | 14.06 | 6.2990 | 100.0 |
| 7 | 14.30 | 6.1927 | 92.9 |
| 8 | 16.13 | 5.4943 | 3.8 |
| 9 | 17.02 | 5.2097 | 8.4 |
| 10 | 17.50 | 5.0676 | 19.8 |
| 11 | 17.78 | 4.9881 | 8.0 |
| 12 | 18.09 | 4.9049 | 7.7 |
| 13 | 18.27 | 4.8561 | 9.0 |
| 14 | 18.75 | 4.7326 | 58.5 |
| 15 | 19.09 | 4.6482 | 63.5 |
| 16 | 21.04 | 4.2228 | 7.3 |
| 17 | 22.77 | 3.9053 | 10.9 |
| 18 | 23.58 | 3.7738 | 53.6 |
| 19 | 24.02 | 3.7045 | 24.6 |
| 20 | 24.90 | 3.5756 | 8.4 |
| 21 | 25.22 | 3.5310 | 10.0 |
| 22 | 26.37 | 3.3796 | 9.4 |
| 23 | 26.63 | 3.3470 | 7.9 |
| 24 | 28.21 | 3.1640 | 5.8 |
| 25 | 29.82 | 2.9958 | 3.0 |
| 26 | 30.16 | 2.9629 | 5.0 |
| 27 | 30.45 | 2.9361 | 6.7 |
| 28 | 32.48 | 2.7566 | 3.3 |
| 29 | 33.03 | 2.7120 | 8.1 |
| 30 | 33.69 | 2.6604 | 3.4 |
| 31 | 35.32 | 2.5413 | 3.0 |
| 32 | 37.96 | 2.3702 | 3.2 |
| 33 | 38.70 | 2.3269 | 3.0 |

In one embodiment, provided herein is a crystalline form of Compound 1 having a thermogravimetric (TGA) thermograph corresponding substantially to the representative TGA thermogram as depicted in **FIG. 24.** In certain embodiments, Form D shows TGA weight loss of about 14.1 % up to 140 °C.

In certain embodiments, Form D comprises DMSO in about 14.3 wt% as measured by gas chromatography.

In still another embodiment, Form D of Compound 1 is substantially pure. In certain embodiments, the substantially pure Form D of Compound 1 is substantially free of other solid forms, *e.g.*, amorphous form. In certain embodiments, the purity of the substantially pure Form D of Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.

In certain embodiments Form D of Compound 1 is substantially pure. In certain embodiments, Form D of Compound 1 is substantially free of other solid forms comprising Compound 1 including, *e.g.*, Forms A, B, C, E, and/or an amorphous solid form comprising Compound 1 as provided herein. In certain embodiments, Form D is a mixture of solid forms comprising Compound 1, including, *e.g.*, a mixture comprising one or more of the following: Forms A, B, C, E, and an amorphous solid form comprising Compound 1.

**Form E of 2-(4-chlorophenyl)-N-((2-(2,6-dioxopiperidin-3-yl)-1-oxoisoindolin-5-yl)methyl)-2,2-difluoroacetamide**

In certain embodiments, the formulations provided herein are prepared from Form E of Compound 1. In certain embodiments, Form E of Compound 1 is a DMSO solvate.

In certain embodiments, Form E is obtained from Form C in DMSO/MIBK or DMSO/IPA or DMSO/anisole at room temperature.

In certain embodiments, Form E is crystalline, as indicated by, *e.g.*, X-ray powder diffraction measurements. In one embodiment, Form E of Compound 1 has an X-ray powder diffraction pattern substantially as shown in **FIG. 25.**

In one embodiment, Form E of Compound 1 has one or more characteristic X-ray powder diffraction peaks at a two-theta angle of approximately 10.5, 12.5, 16.1, 17.0, 18.5, 21.2, 21.7, 22.6, 22.9, 23.4, 23.8, 24.1, 25.1 or 26.7, degrees 2$\theta$ as depicted in **FIG. 25.** In another embodiment, Form E of Compound 1 has one, two, three or four characteristic X-ray powder diffraction peaks at a two-theta angle of approximately 16.1, 17.0, 21.2 or 22.9 degrees 2$\theta$. In another embodiment, Form E of Compound 1 has one, two, three, four, five, six or seven characteristic X-ray powder diffraction peaks as set forth in Table E. In another embodiment, Form E of Compound 1 has one, two, or three characteristic X-ray powder diffraction peaks as set forth in Table E.

**Table E**

| No. | Pos. [°2Th.] | d-spacing [Å] | Rel. Int. [%] |
|---|---|---|---|
| 1 | 4.20 | 21.0329 | 9.6 |
| 2 | 10.48 | 8.4394 | 32.0 |
| 3 | 12.54 | 7.0591 | 28.4 |
| 4 | 14.52 | 6.1023 | 9.9 |
| 5 | 15.51 | 5.7131 | 17.7 |
| 6 | 16.08 | 5.5121 | 100.0 |
| 7 | 16.97 | 5.2256 | 94.5 |
| 8 | 17.77 | 4.9908 | 17.1 |
| 9 | 18.48 | 4.8001 | 20.5 |
| 10 | 19.54 | 4.5422 | 14.7 |
| 11 | 21.15 | 4.2007 | 62.8 |
| 12 | 21.72 | 4.0924 | 20.8 |
| 13 | 22.64 | 3.9270 | 57.4 |
| 14 | 22.91 | 3.8826 | 59.9 |
| 15 | 23.43 | 3.7977 | 23.6 |
| 16 | 23.83 | 3.7348 | 23.2 |
| 17 | 24.13 | 3.6881 | 29.5 |

(continued)

| No. | Pos. [°2Th.] | d-spacing [Å] | Rel. Int. [%] |
|---|---|---|---|
| 18 | 25.14 | 3.5421 | 35.2 |
| 19 | 26.72 | 3.3362 | 49.5 |
| 20 | 27.68 | 3.2232 | 14.6 |
| 21 | 27.93 | 3.1949 | 15.3 |
| 22 | 28.86 | 3.0942 | 15.6 |
| 23 | 29.08 | 3.0703 | 18.3 |
| 24 | 30.12 | 2.9671 | 7.1 |
| 25 | 30.92 | 2.8923 | 12.8 |
| 26 | 32.35 | 2.7672 | 5.0 |
| 27 | 33.21 | 2.6979 | 6.9 |

In one embodiment, provided herein is a crystalline form of Compound 1 having a thermogravimetric (TGA) thermograph corresponding substantially to the representative TGA thermogram as depicted in **FIG. 26.** In certain embodiments, Form E shows TGA weight loss of about 19.4 % up to 120 °C. In certain embodiments, Form E shows additional weight loss of 24.9 % between 120 and 220 °C.

In one embodiment, Form E of Compound 1 is substantially pure. In certain embodiments, the substantially pure Form E of Compound 1 is substantially free of other solid forms, *e.g.*, amorphous form. In certain embodiments, the purity of the substantially pure Form E of Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.

In certain embodiments, Form E of Compound 1 is substantially pure. In certain embodiments herein, Form E of Compound 1 is substantially free of other solid forms comprising Compound 1 including, *e.g.*, Forms A, B, C, D and/or an amorphous solid form comprising Compound 1. In certain embodiments, Form E is a mixture of solid forms comprising Compound 1, including, *e.g.*, a mixture comprising one or more of the following: Forms A, B, C, D and an amorphous solid form comprising Compound 1.

**Amorphous Form of 2-(4-chlorophenyl)-N-((2-(2,6-dioxopiperidin-3-yl)-1-oxoisoindolin-5-yl)methyl)-2,2-difluoroacetamide**

In certain embodiments, the formulations provided herein comprise amorphous Compound 1.

In certain embodiments, provided herein are methods for making the amorphous form by heating Compound 1 in THF and water and cooling the solution.

In one embodiment, provided herein is an amorphous solid form of Compound 1 having a modulated DSC thermogram as depicted in **FIG. 27.**

In one embodiment, amorphous Compound 1 has an X-ray powder diffraction pattern substantially as shown in **FIG. 28.**

In one embodiment, amorphous Compound 1 has a $^{1}$H NMR spectrum substantially as shown in **FIG. 29.**

In still another embodiment, amorphous Compound 1 is substantially pure. In certain embodiments, the substantially pure amorphous Compound 1 is substantially free of other solid forms, e.g., Form A, Form B, Form C, Form D or Form E. In certain embodiments, the purity of the substantially pure amorphous Compound 1 is no less than about 95% pure, no less than about 96% pure, no less than about 97% pure, no less than about 98% pure, no less than about 98.5% pure, no less than about 99% pure, no less than about 99.5% pure, or no less than about 99.8% pure.

**Formulations of Compound 1**

In one aspect, provided herein are stable formulations of Compound 1. In one embodiment, the formulations of Compound 1 comprise a solid form of 2-(4-chlorophenyl)-N-((2-(2,6-dioxopiperidin-3-yl)-1-oxoisoindolin-5-yl)methyl)-2,2-difluoroacetamide. In one embodiment, the formulations of Compound 1 comprise an amorphous form of 2-(4-chlorophenyl)-N-((2-(2,6-dioxopiperidin-3-yl)-1-oxoisoindolin-5-yl)methyl)-2,2-difluoroacetamide.

In certain embodiments, the formulations are prepared with dimethylsulfoxide as a co-solvent or a processing

aid. In certain embodiments, the formulations are prepared with formic acid as co-solvent or a processing aid. In certain embodiments, the formulations are prepared without any co-solvent or processing aid.

In certain embodiments, the formulations comprise dimethylsulfoxide as a co-solvent or a processing aid. In certain embodiments, the formulations comprise formic acid as a co-solvent or a processing aid. In certain embodiments, the formulations do not comprise any co-solvent or processing aid.

In certain embodiments, the formulations provided herein are lyophilized formulations. In certain embodiments, the formulations provided herein are reconstituted formulations obtained in a pharmaceutically acceptable solvent to produce a pharmaceutically acceptable solution.

**Formulation Ia** (not according to the invention)

In one embodiment, provided herein are formulations comprising Compound 1 in an amount of about 0.05-0.2%, a citrate buffer in an amount of about 3%-6%, and hydroxypropyl β-cyclodextrin (HPBCD) in an amount of about 92-98% based on total weight of the formulation.

In one embodiment, provided herein are formulations comprising Compound 1 in an amount of about 0.05-0.2%, a citrate buffer in an amount of about 3%-6%, and sulfobutyl ether-beta-cyclodextrin in an amount of about 92-98% based on total weight of the formulation.

In one embodiment, provided herein are formulations comprising Compound 1 in an amount of about 0.05-0.2%, a citrate buffer in an amount of about 3%-6%, HPBCD in an amount of about 92-98%, and no more than about 1% dimethyl sulfoxide based on total weight of the formulation.

In one embodiment, provided herein are formulations comprising Compound 1 in an amount of about 0.05-0.2%, a citrate buffer in an amount of about 3%-6%, sulfobutyl ether-beta-cyclodextrin in an amount of about 92-98%, and no more than about 1% dimethyl sulfoxide based on total weight of the formulation.

In one embodiment, provided herein are formulations comprising Compound 1 in an amount of about 0.08-0.15%, a citrate buffer in an amount of about 3%-6%, and HPBCD in an amount of about 94-96%, based on total weight of the formulation.

In one embodiment, provided herein are formulations comprising Compound 1 in an amount of about 0.08-0.15%, a citrate buffer in an amount of about 3%-6%, and sulfobutyl ether-beta-cyclodextrin in an amount of about 94-96%, and based on total weight of the formulation.

In one embodiment, provided herein are formulations comprising Compound 1 in an amount of about 0.08-0.15%, a citrate buffer in an amount of about 3%-6%, HPBCD in an amount of about 94-96%, and no more than about 1% dimethyl sulfoxide based on total weight of the formulation.

In one embodiment, provided herein are formulations comprising Compound 1 in an amount of about 0.08-0.15%, a citrate buffer in an amount of about 3%-6%, sulfobutyl ether-beta-cyclodextrin in an amount of about 94-96%, and no more than about 1% dimethyl sulfoxide based on total weight of the formulation.

In one aspect, the formulation provided herein comprises Compound 1 in an amount of about 0.08 to about 0.15% based on the total weight of the formulation. In certain embodiments, the amount of Compound 1 is from about 0.09% to about 0.15 %, about 0.1% to about 0.13% or about 0.11% to about 0.12% based on the total weight of the formulation. In certain embodiments, the amount of Compound 1 is about 0.05%, 0.07%, 0.09%, 0.11%, 0.12%, 0.13%, or 0.15% based on the total weight of the formulation. In one embodiment, the amount of Compound 1 in the formulation is about 0.12% based on the total weight of the formulation.

In another aspect, provided herein is a formulation that comprises Compound 1 in an amount of about 0.5 mg to about 2 mg in a 20 cc vial. In still another aspect is a formulation that comprises Compound 1 in an amount of about 0.5 mg to about 1.5 mg, about 0.75 mg to about 1.25 mg, or about 0.8 mg to about 1.1 mg in a 20 cc vial. In one aspect Compound 1 is present in an amount of about 0.7, 0.75, 0.76, 0.8, 0.9, 1.0, 1.05 or 1.2 mg in a 20 cc vial. In one aspect Compound 1 is present in an amount of about 1.05 mg in a 20 cc vial.

In one aspect, the formulations provided herein contain a citrate buffer. In one aspect, the amount of citrate buffer in the formulations provided herein is from about 3% to about 6% based on total weight of the formulation. In one aspect, the amount of citrate buffer in the formulations provided herein is about 3%, 3.5%, 4%, 4.2%, 4.5% or 5% based on total weight of the formulation. In one aspect, the amount of citrate buffer in the formulations provided herein is about 4.2 % based on total weight of the formulation. In one aspect, the amount of citrate buffer in the formulations provided herein is about 37 mg in a 20cc vial.

In one embodiment, the citrate buffer comprises anhydrous citric acid and anhydrous sodium citrate. In certain embodiments, the amount of anhydrous citric acid is from about 1.5% to about 3%, about 1.75% to about 2.75%, or about 2% to about 2.5% based on total weight of the formulation. In certain embodiments, the amount of anhydrous citric acid in the formulation is about 1.5%, 1.75%, 2%, 2.1%, or 2.5% based on total weight of the formulation. In one embodiment, the amount of anhydrous citric acid in the formulation is about 2%, 2.1%, 2.22% or 2.3% based on total weight of the formulation. In one embodiment, the amount of anhydrous citric acid in the formulation is about 2.10%

based on total weight of the formulation.

In still another aspect is a formulation that comprises anhydrous citric acid in an amount of about 16 mg to about 20 mg in a 20 cc vial. In one embodiment, the amount of anhydrous citric acid is about 16, 17, 18, 18.2, 18.4, 18.6, 18.8, 19 or 20 mg in a 20 cc vial. In one embodiment, the amount of anhydrous citric acid is about 18.6mg in a 20 cc vial.

In certain embodiments, the amount of anhydrous sodium citrate is from about 1.5% to about 3%, about 1.75% to about 2.75%, or about 2% to about 2.5% based on total weight of the formulation. In certain embodiments, the amount of anhydrous sodium citrate in the formulation is about 1.5%, 1.75%, 2%, 2.1%, or 2.5% based on total weight of the formulation. In one embodiment, the amount of anhydrous sodium citrate in the formulation is about 2%, 2.05%, 2.08% or 2.1% based on total weight of the formulation. In one embodiment, the amount of anhydrous sodium citrate in the formulation is about 2.08% based on total weight of the formulation.

In still another aspect is a formulation that comprises anhydrous sodium citrate in an amount of about 16 mg to about 20 mg in a 20 cc vial. In one embodiment, the amount of anhydrous sodium citrate is about 16, 17, 18, 18.2, 18.4, 18.6, 18.8, 19 or 20 mg in a 20 cc vial. In one embodiment, the amount of anhydrous sodium citrate is about 18.4 mg in a 20 cc vial.

In certain embodiments, the amount of HPBCD in the formulations provided herein is about 94 to about 97% based on total weight of the formulation. In one embodiment, the amount of HPBCD in the formulations provided herein is about 94.5%, 95%, 95.5%, or 96% based on total weight of the formulation. In one embodiment, the amount of HPBCD in the formulations provided herein is about 95% based on total weight of the formulation.

In certain embodiments, the amount of sulfobutyl ether-beta-cyclodextrin in the formulations provided herein is about 94 to about 97% based on total weight of the formulation. In one embodiment, the amount of sulfobutyl ether-beta-cyclodextrin in the formulations provided herein is about 94.5%, 95%, 95.5%, or 96% based on total weight of the formulation. In one embodiment, the amount of sulfobutyl ether-beta-cyclodextrin in the formulations provided herein is about 95% based on total weight of the formulation.

In another aspect is a formulation that comprises HPBCD in an amount of about 800-900 mg in a 20 cc vial. In another aspect is a formulation that comprises HPBCD in an amount of about 810-880 mg, 820-860 mg or 830-850 mg in a 20 cc vial. In another aspect is a formulation that comprises HPBCD in an amount of about 840 mg in a 20 cc vial.

In another aspect is a formulation that comprises sulfobutyl ether-beta-cyclodextrin in an amount of about 800-900 mg in a 20 cc vial. In another aspect is a formulation that comprises sulfobutyl ether-beta-cyclodextrin in an amount of about 810-880 mg, 820-860 mg or 830-850 mg in a 20 cc vial. In another aspect is a formulation that comprises sulfobutyl ether-beta-cyclodextrin in an amount of about 840 mg in a 20 cc vial.

In another aspect is a formulation that comprises Kleptose®HPB in an amount of about 840 mg in a 20 cc vial.

In one embodiment, the formulations comprise dimethyl sulfoxide in an amount of no more than about 1.5% based on total weight of the formulation. In one embodiment, the formulations comprise dimethyl sulfoxide in an amount of up to 0.1%, 0.2%, 0.3%, 0.4%, 0.6%, 0.7%, 0.8%, 0.9% or 1% based on total weight of the formulation. In one embodiment, the formulations comprise no more than about 0.1%, 0.2%, 0.3%, 0.4%, 0.6%, 0.7%, 0.8%, 0.9% or 1% dimethyl sulfoxide based on total weight of the formulation. In one embodiment, the formulations comprise dimethyl sulfoxide in an amount of up to about 0.1 to about 1.5% based on total weight of the formulation. In one embodiment, the amount of dimethyl sulfoxide in the formulations provided herein is about 0.1 to about 1.3% based on total weight of the formulation. In one embodiment, the amount of dimethyl sulfoxide in the formulations provided herein is about 0.1%, 0.2%, 0.3%, 0.4%, 0.6%, 0.7%, 0.8%, 0.9% or 1% based on total weight of the formulation. In one embodiment, the formulations provided herein do not contain any dimethyl sulfoxide. In one embodiment, the amount of dimethyl sulfoxide in the formulations provided herein is about 0.4% to 0.8% based on total weight of the formulation.

In another aspect is a formulation that comprises dimethyl sulfoxide in an amount of about 4 to 7 mg in a 20 cc vial. In another aspect is a formulation that comprises dimethyl sulfoxide in an amount of about 4.5-6.5 mg, or 5 to 6 mg in a 20 cc vial.

In certain embodiments, the formulation provided herein is lyophilized, and the lyophilized formulation upon reconstitution has a pH of about 4 to 5. In certain embodiments, the formulation upon reconstitution has a pH of about 4.2 to 4.4. In one embodiment, the lyophilized formulation upon reconstitution has a pH of about 4, 4.1, 4.2, 4.3, 4.4, 4.5, 4.6, 4.7, 4.8, 4.9 or 5.

In certain embodiments, the lyophilized formulation upon reconstitution has an osmolality of about 250-290 mOsm/kg. In certain embodiments, the lyophilized formulation upon reconstitution has an osmolality of about 260-280 mOsm/kg.

In certain embodiments, provided herein is a container comprising a formulation provided herein. In one aspect, the container is a glass vial. In one aspect, the container is a 20 cc glass vial.

In one aspect provided herein is a formulation in a 20 cc vial that comprises: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide and a pharmaceutically acceptable carrier or excipient that includes a bulking agent as described herein. In one embodiment, the formulation further comprises no more than about 7 mg dimethyl sulfoxide as residual solvent. In one embodiment, the formulation comprises no more than about 6 mg dimethyl sulfoxide as residual solvent. In one embodiment, the formulation comprises no more than about 5 mg dimethyl sulfoxide as residual solvent. In one embodiment, the formulation comprises no more than about 4 mg dimethyl sulfoxide as residual solvent. In one embodiment, the formulation comprises from about 3 mg to about 7 mg, about 4 mg to about 6 mg, about 4 mg to about 5 mg or about 5 mg to about 6 mg dimethyl sulfoxide as residual solvent. In one embodiment, the formulation comprises about 4, 4.5, 5, 5.3, 5.5, 5.7, 6 or 6.5 mg dimethyl sulfoxide as residual solvent.

In one embodiment, provided herein are formulations consisting essentially of Compound 1 in an amount of about 0.05-0.2%, a citrate buffer in an amount of about 3%-6%, and HPBCD in an amount of about 92-98% based on total weight of the formulation.

In one embodiment, provided herein are formulations consisting essentially of Compound 1 in an amount of about 0.05-0.2%, a citrate buffer in an amount of about 3%-6%, and sulfobutyl ether-beta-cyclodextrin in an amount of about 92-98% based on total weight of the formulation.

In one embodiment, provided herein are formulations consisting essentially of Compound 1 in an amount of about 0.05-0.2%, a citrate buffer in an amount of about 3%-6%, HPBCD in an amount of about 92-98%, and no more than about 1% dimethyl sulfoxide based on total weight of the formulation.

In one embodiment, provided herein are formulations consisting essentially of Compound 1 in an amount of about 0.05-0.2%, a citrate buffer in an amount of about 3%-6%, sulfobutyl ether-beta-cyclodextrin in an amount of about 92-98%, and no more than about 1% dimethyl sulfoxide based on total weight of the formulation.

In one aspect provided herein is a formulation in a 20 cc vial that comprises: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, a pharmaceutically acceptable carrier or excipient that includes a buffer and bulking agent as described herein, and about 5 mg to about 6 mg dimethyl sulfoxide as residual solvent. The buffer and bulking agent can be present at an amount as described herein.

In one aspect provided herein is a formulation in a 20 cc vial that comprises: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent as described herein. In one embodiment, the formulation in a 20 cc vial is reconstituted with 3.8 mL sterile water for injection.

In one aspect provided herein is a formulation in a 20 cc vial that consists essentially of: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent as described herein. In one embodiment, the formulation in a 20 cc vial is reconstituted with 3.8 mL sterile water for injection.

In one aspect provided herein is a formulation in a 20 cc vial that consists of: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent as described herein. In one embodiment, the formulation in a 20 cc vial is reconstituted with 3.8 mL sterile water for injection.

In one embodiment, provided herein is an aqueous formulation comprising Compound 1 in an amount of about 0.05-0.2% based on total weight of the solids, a citrate buffer in an amount of about 3%-6% based on total weight of the solids, HPBCD in an amount of about 92-98% based on total weight of the solids, and a diluent.

In one embodiment, provided herein is an aqueous formulation consisting essentially of Compound 1 in an amount of about 0.05-0.2% based on total weight of the solids, a citrate buffer in an amount of about 3%-6% based on total weight of the solids, HPBCD in an amount of about 92-98% based on total weight of the solids, and a diluent.

In one aspect provided herein is an aqueous formulation that comprises: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent and about 3.8 mL diluent.

In one aspect provided herein is an aqueous formulation that consists essentially of: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent and about 3.8 mL diluent.

In one aspect provided herein is an aqueous formulation that consists of: Compound 1 at an amount that provides 1.05 mg 2-(4-chlorophenyl)-N-((2-(2,6-dioxopiperidin-3-yl)-1-oxoisoindolin-5-yl)methyl)-2,2-difluoroacetamide, 18.6 mg anhydrous citric acid, 18.4 mg anhydrous sodium citrate, 840 mg HPBCD, and about 5 mg to about 6 mg dimethyl sulfoxide as residual solvent and about 3.8 mL diluent.

In certain embodiments, the formulation has a composition as described in Table 43.

**Formulation Ib**

In one embodiment, provided herein are formulations comprising Compound 1 in an amount of about 0.01-0.15%, hydroxypropyl β-cyclodextrin in an amount of about 99.1-99.99%. In one embodiment, provided herein are formulations comprising Compound 1 in an amount of about 0.01-0.15%, hydroxypropyl β-cyclodextrin in an amount of about 99.1-99.99%, and no more than about 0.5% formic acid based on total weight of the formulation.

In one embodiment, which is not part of the claims, provided herein are formulations comprising Compound 1 in an amount of about 0.05-0.25% and HPBCD in an amount of about 99.1-99.9% based on total weight of the formulation.

In one embodiment, which is not part of the claims, provided herein are formulations comprising Compound 1 in an amount of about 0.05-0.25%, HPBCD in an amount of about 99.1-99.9%, and no more than about 0.5% formic acid based on total weight of the formulation.

In one embodiment, which is not part of the claims, provided herein are formulations comprising Compound 1 in an amount of about 0.05-0.25% and HPBCD in an amount of about 99.75-99.9% based on total weight of the formulation.

In one embodiment, which is not part of the claims, provided herein are formulations comprising Compound 1 in an amount of about 0.05-0.25%, HPBCD in an amount of about 99.75-99.9%, and no more than about 0.5% formic acid based on total weight of the formulation.

In one embodiment, which is not part of the claims, provided herein are formulations comprising Compound 1 in an amount of about 0.05-0.25%, HPBCD in an amount of about 99.75-99.9%, and no more than about 0.2% formic acid based on total weight of the formulation.

In one embodiment, provided herein are formulations comprising Compound 1 in an amount of about 0.08-0.15% and HPBCD in an amount of about 99.8-99.9% based on total weight of the formulation.

In one embodiment, provided herein are formulations comprising Compound 1 in an amount of about 0.08-0.15%, HPBCD in an amount of about 99.8-99.9%, and no more than about 0.5% formic acid based on total weight of the formulation.

In one embodiment, provided herein are formulations comprising Compound 1 in an amount of about 0.08-0.15%, HPBCD in an amount of about 99.8-99.9%, and no more than about 0.12% formic acid based on total weight of the formulation.

In one embodiment, provided herein are formulations comprising Compound 1 in an amount of about 0.12% and HPBCD in an amount of about 99.88% based on total weight of the formulation.

In one embodiment, provided herein are formulations comprising Compound 1 in an amount of about 0.08-0.15% and sulfobutyl ether-beta-cyclodextrin in an amount of about 99.8-99.9% based on total weight of the formulation.

In one embodiment, provided herein are formulations comprising Compound 1 in an amount of about 0.08-0.15%, sulfobutyl ether-beta-cyclodextrin in an amount of about 99.8-99.9%, and no more than about 0.5% formic acid based on total weight of the formulation.

In one embodiment, provided herein are formulations comprising Compound 1 in an amount of about 0.12% and sulfobutyl ether-beta-cyclodextrin in an amount of about 99.88% based on total weight of the formulation.

In one aspect, the formulation provided herein comprises Compound 1 in an amount of about 0.08 to about 0.15% based on the total weight of the formulation. In certain embodiments, the amount of Compound 1 is from about 0.09% to about 0.15 %, about 0.1% to about 0.13% or about 0.11% to about 0.12% based on the total weight of the formulation. In certain embodiments, the amount of Compound 1 is about 0.05%, 0.07%, 0.09%, 0.11%, 0.12%, 0.13%, or 0.15% based on the total weight of the formulation. In one embodiment, the amount of Compound 1 in the formulation is about 0.12% based on the total weight of the formulation.

In another aspect, provided herein is a formulation that comprises Compound 1 in an amount of about 0.5 mg to about 2 mg in a 20 cc vial. In still another aspect is a formulation that comprises Compound 1 in an amount of about 0.5 mg to about 1.5 mg, about 0.75 mg to about 1.25 mg, or about 0.8 mg to about 1.1 mg in a 20 cc vial. In one aspect Compound 1 is present in an amount of about 0.7, 0.75, 0.76, 0.8, 0.9, 1.0, 1.05 or 1.2 mg in a 20 cc vial. In one aspect Compound 1 is present in an amount of about 1 mg in a 20 cc vial.

In one embodiment, the amount of HPBCD in the formulations provided herein is about 99.1%, 99.3%, 99.5%, 99.7% or 99.9% based on total weight of the formulation. In one embodiment, the amount of HPBCD in the formulations provided herein is about 99.5% based on total weight of the formulation. In another aspect is a formulation that comprises HPBCD in an amount of about 750-850 mg in a 20 cc vial. In another aspect is a formulation that comprises HPBCD in an amount of about 790-840 mg, 780-830 mg or 790-810 mg in a 20 cc vial. In another aspect is a formulation that comprises HPBCD in an amount of about 800 mg in a 20 cc vial.

In another aspect is a formulation that comprises Kleptose®HPB in an amount of about 800 mg in a 20 cc vial.

In one embodiment, the amount of sulfobutyl ether-beta-cyclodextrin in the formulations provided herein is about 99.1%, 99.3%, 99.5%, 99.7% or 99.9% based on total weight of the formulation. In one embodiment, the amount of sulfobutyl ether-beta-cyclodextrin in the formulations provided herein is about 99.5% based on total weight of the formulation.

In another aspect is a formulation that comprises sulfobutyl ether-beta-cyclodextrin in an amount of about 750-850 mg in a 20 cc vial. In another aspect is a formulation that comprises sulfobutyl ether-beta-cyclodextrin in an amount of about 790-840 mg, 780-830 mg or 790-810 mg in a 20 cc vial. In another aspect is a formulation that comprises sulfobutyl ether-beta-cyclodextrin in an amount of about 800 mg in a 20 cc vial.

In another aspect is a formulation that comprises Kleptose®HPB in an amount of about 800 mg in a 20 cc vial.

In one embodiment, the formulations comprise formic acid in no more than about 0.5% based on total weight of the formulation. In one embodiment, the formulations comprise formic acid in an amount of up to about 0.05%, 0.07%, 0.09%, 0.1%, 0.2%, 0.3%, 0.4% or 0.5% based on total weight of the formulation. In one embodiment, the formulations comprise formic acid in no more than about 0.05%, 0.07%, 0.09%, 0.1%, 0.2%, 0.3%, 0.4% or 0.5% based on total weight of the formulation. In one embodiment, the amount of formic acid in the formulations provided herein is about 0.05 to about 0.5% based on total weight of the formulation. In one embodiment, the amount of formic acid in the formulations provided herein is about 0.05 to about 0.1% based on total weight of the formulation. In one embodiment, the amount of formic acid in the formulations provided herein is about 0.05%, 0.07%, 0.09%, 0.1%, 0.2%, 0.3%, 0.4%or 0.5% based on total weight of the formulation. In one embodiment, the formulations provided herein do not contain any formic acid. In one embodiment, the amount of formic acid in the formulations provided herein is about 0.05% to 0.09% based on total weight of the formulation.

In another aspect is a formulation that comprises formic acid in an amount of no more than about 1 mg in a 20 cc vial. In another aspect is a formulation that comprises formic acid in an amount of up to about 0.2, 0 5, 0.7, 0.9 mg or 1 mg in a 20 cc vial. In another aspect is a formulation that comprises formic acid in an amount of about 0.3-0.9 mg, or 0.4 to 0.8 mg in a 20 cc vial.

In another aspect, provided herein is a formulation that comprises Compound 1 in an amount of about 1 mg and HPBCD in an amount of about 800 mg in a 20 cc vial.

In another aspect, provided herein is a formulation that comprises Compound 1 in an amount of about 1 mg, HPBCD in an amount of about 800 mg and formic acid in an amount of about 0.9 mg in a 20 cc vial.

In certain embodiments, the formulation has a composition as described in the Table 43.

**Formulation Ic**

In one embodiment, provided herein are formulations comprising Compound 1 in an amount of about 0.01-0.08% and HPBCD in an amount of about 99.40-99.99% based on total weight of the formulation.

In one embodiment, provided herein are formulations comprising Compound 1 in an amount of about 0.01-0.08%, HPBCD in an amount of about 99.40-99.99%, and no more than about 0.5% formic acid based on total weight of the formulation.

In one embodiment, provided herein are formulations comprising Compound 1 in an amount of about 0.03-0.06% and HPBCD in an amount of about 99.60-99.99% based on total weight of the formulation.

In one embodiment, provided herein are formulations comprising Compound 1 from about 0.01 to about 0.08%, hydroxypropyl β-cyclodextrin from about 99.40% to about 99.99%, and formic acid from about 0.1 to about 0.3% based on total weight of the formulation

In one aspect, the formulation provided herein comprises Compound 1 in an amount of about 0.02 to about 0.06% based on the total weight of the formulation. In certain embodiments, the amount of Compound 1 is from about 0.03% to about 0.06 %, or about 0.04% to about 0.06% based on the total weight of the formulation. In certain embodiments, the amount of Compound 1 is about 0.03%, 0.04%, 0.05% or 0.06% based on the total weight of the formulation. In one embodiment, the amount of Compound 1 in the formulation is about 0.05% based on the total weight of the formulation.

In another aspect, provided herein is a formulation that comprises Compound 1 in an amount of about 0.75 mg to about 1.5 mg in a 20 cc vial. In still another aspect is a formulation that comprises Compound 1 in an amount of about 0.75 mg to about 1.25 mg in a 20 cc vial. In one aspect Compound 1 is present in an amount of about 0.75, 0.8, 0.9, 1.0, 1.05 or 1.2 mg in a 20 cc vial. In one aspect Compound 1 is present in an amount of about 1 mg in a 20 cc vial.

In one embodiment, the amount of HPBCD in the formulations provided herein is about 99.40 to about 99.99% based on total weight of the formulation. In one embodiment, the amount of HPBCD in the formulations provided herein is about 99.5, 99.6, 99.7, 99.8, 99.9, 99.95, or 99.99% based on total weight of the formulation. In another aspect is a formulation that comprises HPBCD in an amount of about 1800-1900 mg in a 20 cc vial. In another aspect is a formulation that comprises HPBCD in an amount of about 1850-1900 mg in a 20 cc vial. In another aspect is a formulation that comprises HPBCD in an amount of about 1875 mg in a 20 cc vial.

In one embodiment, the formulations comprise formic acid in no more than about 0.5% based on total weight of the formulation. In one embodiment, the formulations comprise formic acid in an amount of up to about 0.05%, 0.07%, 0.09%, 0.1%, 0.2%, 0.3%, 0.4% or 0.5% based on total weight of the formulation. In one embodiment, the formulations comprise formic acid in no more than about 0.05%, 0.07%, 0.09%, 0.1%, 0.2%, 0.3%, 0.4% or 0.5% based on total

weight of the formulation. In one embodiment, the amount of formic acid in the formulations provided herein is about 0.05 to about 0.3% based on total weight of the formulation. In one embodiment, the amount of formic acid in the formulations provided herein is about 0.05 to about 0.25% based on total weight of the formulation. In one embodiment, the amount of formic acid in the formulations provided herein is about 0.05%, 0.07%, 0.09%, 0.1%, 0.2%, or 0.3% based on total weight of the formulation. In one embodiment, the formulations provided herein do not contain any formic acid. In one embodiment, the amount of formic acid in the formulations provided herein is about 0.11% to 0.3% based on total weight of the formulation.

In another aspect is a formulation that comprises formic acid in an amount of no more than about 4 mg in a 20 cc vial. In another aspect is a formulation that comprises formic acid in an amount of up to about 1, 1.8, 2, 2.1, 2.5, 3, 3.5, 3.8, 3.9, 4, 4.5, 4.9 mg or 5 mg in a 20 cc vial. In another aspect is a formulation that comprises formic acid in an amount of about 1-1.8 mg, 2.1-3.8 mg, or 3.9-4.9 mg in a 20 cc vial.

In another aspect, provided herein is a formulation that comprises Compound 1 in an amount of about 1 mg, and HPBCD in an amount of about 1875 mg in a 20 cc vial.

In another aspect, provided herein is a formulation that comprises Compound 1 in an amount of about 1 mg, HPBCD in an amount of about 1875 mg and formic acid in an amount of about 2.1-3.8 mg in a 20 cc vial.

In certain embodiments, the formulation has a composition as described in the Table 64.

**Formulations without co-solvent** (formulations comprising 0.01-0.15 w% Compound 1 and 99.1-99.99 w% of either hydroxypropyl β-cyclodextrin or sulfobutyl ether-beta-cyclodextrin are falling within the scope of the claims)

In one embodiment, provided herein are formulations comprising Compound 1 in an amount of about 0.15-0.5%, a citrate buffer in an amount of about 15% to about 35%, and HPBCD in an amount of about 92% to about 98%, based on total weight of the formulation. In one embodiment, the citrate buffer comprises anhydrous citric acid and anhydrous sodium citrate.

In one embodiment, provided herein are formulations comprising Compound 1 in an amount of about 0.25-0.30%, a citrate buffer in an amount of about 30-32%, and HPBCD in an amount of about 67-69%, based on total weight of the formulation.

In one embodiment, provided herein are formulations comprising Compound 1 in an amount of about 0.30-0.33%, a citrate buffer in an amount of about 17-18%, and HPBCD in an amount of about 80-85%, based on total weight of the formulation.

**Exemplary Formulations**

In one embodiment, provided herein are formulations consisting essentially of Compound 1 in an amount of about 0.05-0.25% and HPBCD in an amount of about 99.75-99.95% based on total weight of the formulation.

In one embodiment, provided herein are formulations consisting essentially of Compound 1 in an amount of about 0.05-0.25% and HPBCD in an amount of about 99.75-99.99% based on total weight of the formulation.

In one embodiment, provided herein are formulations consisting essentially of Compound 1 in an amount of about 0.05-0.25% and sulfobutyl ether-beta-cyclodextrin in an amount of about 99.75-99.95%, based on total weight of the formulation.

In one aspect provided herein is a formulation in a 20 cc vial that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, and about 0.6 mg formic acid as described herein. In one embodiment, the formulation in a 20 cc vial is reconstituted with 4.5 mL sterile water for injection.

In one aspect provided herein is a formulation in a 20 cc vial that consists essentially of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, and about 0.6 mg formic acid as described herein. In one embodiment, the formulation in a 20 cc vial is reconstituted with 4.5 mL sterile water for injection.

In one aspect provided herein is a formulation in a 20 cc vial that consists of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, and about 0.6 mg formic acid as described herein. In one embodiment, the formulation in a 20 cc vial is reconstituted with 4.5 mL sterile water for injection.

In one aspect provided herein is a formulation in a 20 cc vial that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg sulfobutyl ether-beta-cyclodextrin, and about 0.6 mg formic acid as described herein. In one embodiment, the formulation in a 20 cc vial is reconstituted with 4.5 mL sterile water for injection.

In one aspect provided herein is a formulation in a 20 cc vial that consists essentially of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg sulfobutyl ether-beta-cyclodextrin, and about 0.6 mg formic acid as described herein. In one embodiment, the formulation in a 20 cc vial is reconstituted with 4.5 mL sterile water for injection.

In one aspect provided herein is a formulation in a 20 cc vial that consists of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg sulfobutyl ether-beta-cyclodextrin, and about 0.6 mg formic acid as described herein. In one embodiment, the formulation in a 20 cc vial is reconstituted with 4.5 mL sterile water for injection.

In one aspect provided herein is a formulation in a 20 cc vial that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 1875 mg HPBCD, and about 2.1-3.8 mg formic acid as described herein. In one embodiment, the formulation in a 20 cc vial is reconstituted with 12.5 ml Normal Saline for injection.

In one aspect provided herein is a formulation in a 20 cc vial that consists essentially of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 1875 mg HPBCD, and about 2.1-3.8 mg formic acid as described herein. In one embodiment, the formulation in a 20 cc vial is reconstituted with 12.5 ml Normal Saline for injection.

In one aspect provided herein is a formulation in a 20 cc vial that consists of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 1875 mg HPBCD, and about 2.1-3.8 mg formic acid as described herein. In one embodiment, the formulation in a 20 cc vial is reconstituted with 12.5 ml Normal Saline for injection.

In one embodiment, provided herein is an aqueous formulation comprising Compound 1 in an amount of about 0.05-0.25% based on total weight of the solids, and HPBCD in an amount of about 99.1-99.9% based on total weight of the solids, and a diluent.

In one embodiment, provided herein is an aqueous formulation comprising Compound 1 in an amount of about 0.05-0.25% based on total weight of the solids, and HPBCD in an amount of about 99.75-99.95% based on total weight of the solids, and a diluent.

In one embodiment, provided herein is an aqueous formulation consisting essentially of Compound 1 in an amount of about 0.05-0.25% based on total weight of the solids, and HPBCD in an amount of about 99.75-99.95% based on total weight of the solids, and a diluent.

In one aspect provided herein is an aqueous formulation that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, about 0.6 mg formic acid and about 4.5 mL diluent.

In one aspect provided herein is an aqueous formulation that consists of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, about 0.6 mg formic acid and about 4.5 mL diluent.

In one embodiment, provided herein is an aqueous formulation comprising Compound 1 in an amount of about 0.01-0.08% based on total weight of the solids, and HPBCD in an amount of about 99.50-99.99% based on total weight of the solids, and a diluent.

In one embodiment, provided herein is an aqueous formulation comprising Compound 1 in an amount of about 0.01-0.08% based on total weight of the solids, and HPBCD in an amount of about 99.50-99.99% based on total weight of the solids, and a diluent.

In one embodiment, provided herein is an aqueous formulation consisting essentially of Compound 1 in an amount of about 0.01-0.08% based on total weight of the solids, and HPBCD in an amount of about 99.50-99.99% based on total weight of the solids, and a diluent.

In one aspect provided herein is an aqueous formulation that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, about 0.6 mg formic acid and about 4.5 mL diluent.

In one aspect provided herein is an aqueous formulation that consists of: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, 800 mg HPBCD, about 0.6 mg formic acid and about 4.5 mL diluent.

In certain embodiments, the formulation has a composition as described in the Table 43. In certain embodiments, the formulation has a composition as described in the Table 64.

In certain embodiments, the formulation provided herein is lyophilized, and the lyophilized formulation upon reconstitution has a pH of about 2.5 to 4. In certain embodiments, the lyophilized formulation upon reconstitution has a pH of about 2.5 to 3.5. In certain embodiments, the lyophilized formulation upon reconstitution has a pH of about 3.0 to 3.6. In one embodiment, the lyophilized formulation upon reconstitution has a pH of about 2.5, 3, 3.2, 3.4, 3.6, 3.8 or 4. In one embodiment, the lyophilized formulation upon reconstitution has a pH of about 2.5, 2.8, 3, 3.2, 3.4, 3.6, 3.8 or 4.

In certain embodiments, the lyophilized formulation upon reconstitution has an osmolality of about 260-290 mOsm/kg. In certain embodiments, the lyophilized formulation upon reconstitution has an osmolality of about 280 mOsm/kg. In certain embodiments, the lyophilized formulation upon reconstitution has an osmolality of about 260-370

mOsm/kg. In certain embodiments, the lyophilized formulation upon reconstitution has an osmolality of about 360 mOsm/kg. In certain embodiments, the lyophilized formulation upon reconstitution has an osmolality of about 350-450 mOsm/kg. In certain embodiments, the lyophilized formulation upon reconstitution has an osmolality of about 416 mOsm.

In certain embodiments, the lyophilized formulation is reconstituted with half normal saline (0.45% sodium chloride sterile solution for injection) and has an osmolality of about 280-320 mOsm/kg upon reconstitution. In certain embodiments, the lyophilized formulation is reconstituted with half normal saline (0.45% sodium chloride sterile solution for injection), and has a pH of 3.0-3.2 and an osmolality of about 280-320 mOsm/kg upon reconstitution. In certain embodiments, the lyophilized formulation is reconstituted with 4.5 mL of half normal saline (0.45% sodium chloride sterile solution for injection), and has a pH of 3.0-3.2 and an osmolality of about 280-320 mOsm/kg upon reconstitution. In one embodiment, the reconstituted solution of the required dose is diluted with normal saline (0.9% sodium chloride sterile solution for injection) in an infusion bag to a volume to 50 mL for 30-minute intravenous administration.

In certain embodiments, the lyophilized formulation is reconstituted with normal saline and has an osmolality of about 440 mOsm/kg upon reconstitution. In one embodiment, the reconstituted solution of the required dose is diluted with normal saline to a volume to 50 mL to obtain a dosing solution having an osmolality of about 310-380 mOsm/kg. In one embodiment, the reconstituted solution of the required dose is diluted with normal saline to a volume to 50 mL to obtain a dosing solution having an osmolality of about 310-355 mOsm/kg. In one embodiment, the reconstituted solution of the required dose is diluted with normal saline to a volume to 50 mL to obtain a dosing solution having an osmolality of about 317-371 mOsm/kg. In one embodiment, the reconstituted solution of the required dose is diluted with normal saline to a volume to 50 mL to obtain a dosing solution having an osmolality of about 317 mOsm/kg. In one embodiment, the reconstituted solution of the required dose is diluted with normal saline to a volume to 50 mL to obtain a dosing solution having an osmolality of about 371 mOsm/kg. In one embodiment, the osmolality of the dosing solution is no more than 352 mOsm/kg. In one embodiment, the osmolality of the dosing solution having a dose of 4.8 mg Compound 1 is 352 mOsm/kg.

In certain embodiments, provided herein is a container comprising a formulation provided herein. In one aspect, the container is a glass vial. In one aspect, the container is a 20 cc glass vial.

In one aspect provided herein is a formulation in a 20 cc vial that comprises: Compound 1 at an amount that provides 1 mg 2-(4-chlorophenyl)-N-((2-(2,6-dioxopiperidin-3-yl)-1-oxoisoindolin-5-yl)methyl)-2,2-difluoroacetamide, and a bulking agent as described herein. In one embodiment, the formulation further comprises no more than about 5 mg formic acid as residual solvent. In one embodiment, the formulation further comprises no more than about 4 mg formic acid as residual solvent. In one embodiment, the formulation further comprises no more than about 3 mg formic acid as residual solvent. In one embodiment, the formulation further comprises no more than about 2 mg formic acid as residual solvent. In one embodiment, the formulation further comprises no more than about 1.5 mg formic acid as residual solvent. In one embodiment, the formulation further comprises no more than about 1 mg formic acid as residual solvent. In one embodiment, the formulation further comprises no more than about 0.8 mg formic acid as residual solvent. In one embodiment, the formulation comprises from about 0.4 mg to about 1.5 mg, about 0.5 mg to about 1 mg, or about 0.5 mg to about 0.9 mg formic acid as residual solvent. In one embodiment, the formulation comprises about 0.4 mg, about 0.6 mg, about 0.8 mg, about 1 mg or about 1.5 mg formic acid as residual solvent. In one embodiment, the formulation comprises formic acid as residual solvent in an amount from about 1.0 mg/mg of Compound 1 to about 1.8 mg/mg of Compound 1, about 2.1 mg/mg of Compound 1 to about 3.8 mg/mg of Compound 1, or about 3.9 mg/mg of Compound 1 to about 4.9 mg/mg of Compound 1.

The formulations of Compound 1 provided herein can be administered to a patient in need thereof using standard therapeutic methods for delivering Compound 1 including, but not limited to, the methods described herein. In one embodiment, the formulations provided herein are reconstituted in a pharmaceutically acceptable solvent to produce a pharmaceutically acceptable solution, wherein the solution is administered (such as by intravenous injection) to the patient.

In one aspect, the formulations provided herein lyophilized, and the lyophilized formulations are suitable for reconstitution with a suitable diluent to the appropriate concentration prior to administration. In one embodiment, the lyophilized formulation is stable at room temperature. In one embodiment, the lyophilized formulation is stable at room temperature for up to about 24 months. In one embodiment, the lyophilized formulation is stable at room temperature for up to about 24 months, up to about 18 months, up to about 12 months, up to about 6 months, up to about 3 months or up to about 1 month. In one embodiment, the lyophilized formulation is stable upon storage under accelerated condition of 40 °C/75% RH for up to about 12 months, up to about 6 months or up to about 3 months.

The lyophilized formulation provided herein can be reconstituted for parenteral administration to a patient using any pharmaceutically acceptable diluent. Such diluents include, but are not limited to Sterile Water for Injection (SWFI), Dextrose 5% in Water (D5W), or a cosolvent system. Any quantity of diluent may be used to reconstitute the lyophilized formulation such that a suitable solution for injection is prepared. Accordingly, the quantity of the diluent must be sufficient to dissolve the lyophilized formulation. In one embodiment, 1-5 mL or 1 to 4 mL of a diluent are used to reconstitute the lyophilized formulation to yield a final concentration of, about 0.05-0.3 mg/mL or about 0.15-0.25 mg/mL of Compound

1. In certain embodiments, the final concentration of Compound 1 in the reconstituted solution is about 0.25 mg/mL. In certain embodiments, the final concentration of Compound 1 in the reconstituted solution is about 0.20 mg/mL. In certain embodiments, the volume of the reconstitution diluent varies between 3 ml and 5 ml to yield a final concentration of 0.15-0.3 mg/mL. In certain embodiments, depending on the required dose, multiple vials may be used for reconstitution.

The reconstituted solutions of lyophilized formulation can be stored and used within up to about 24 hours, about 12 hours or about 8 hours. In one embodiment, the reconstituted aqueous solution is stable at room temperature from about 1-24, 2-20, 2-15, 2-10 hours upon reconsititution. In one embodiment, the reconstituted aqueous solution is stable at room temperature for up to about 20, 15, 12, 10, 8, 6, 4 or 2 hours upon reconsititution. In some embodiments, the solution is used within 8 hour of preparation. In some embodiments, the solution is used within 5 hour of preparation. In some embodiments, the solution is used within 1 hour of preparation.

**Process for Making Formulations**

The formulations provided herein can be prepared by any of the methods known in the art and as described herein, but all methods include the step of bringing the active ingredient into association with the pharmaceutically acceptable excipient, which constitutes one or more necessary ingredients (such as bulking agent and/or buffer). According to the claims, Compound 1 is dissolved in formic acid to obtain a premix, and the premix is added to a solution of hydroxypropyl β-cyclodextrin in water to obtain a drug solution.

Alternatively, the formulations provided herein may be prepared by dissolving Compound 1, a bulking agent and a citrate buffer in water and dimethyl sulfoxide (DMSO) to obtain a solution, and optionally lyophilizing the solution. FIG. 37 and 38 provide flow charts illustrating exemplary processes to prepare the formulations provided herein.

In one embodiment, which is not claimed, the process for preparing the formulation comprises:
dissolving HPBCD in a citrate buffer to obtain a buffer solution, dissolving Compound 1 in DMSO to obtain a premix, adding the premix to the buffer solution to obtain a solution; and optionally lyophilizing the solution to produce the lyophilized formulation.

For example, the process comprises dissolving Kleptose® HPB in a 20 mM, pH 4 - 4.5 citrate buffer to obtain a buffer solution, dissolving Compound 1 in DMSO to obtain an active premix, adding the premix to the buffer solution to obtain a mixture, adding water to the mixture to obtain a bulk solution, filtering the bulk solution through one or more 0.45 μm and 0.22 μm filters to obtain a filtered solution, filling the filtered solution into a vial, and lyophilizing the solution. In one embodiment, the solution is filtered through one 0.45 μm and two 0.22 μm filters. In one embodiment, the process comprises dissolving Kleptose® HPB in a 20 mM, pH 4.3 citrate buffer to obtain a buffer solution, dissolving Compound 1 in DMSO to obtain an active premix, adding the premix to the buffer solution to obtain a mixture, adding water to the mixture to obtain a bulk solution, filtering the bulk solution through one 0.45 μm filter and two 0.22 μm filters to obtain a filtered solution, filling the filtered solution into a 20 cc glass vial, and optionally lyophilizing the solution. In one embodiment, the vial is sealed under nitrogen after lyophilization.

According to the invention, the formulations provided herein are prepared by dissolving Compound 1 in formic acid to obtain a premix, dissolving HPBCD in water to obtain a solution, adding the premix to the solution to obtain a drug solution; and optionally lyophilizing the drug solution to produce the lyophilized formulation.

In one aspect, the formulations provided herein are prepared by dissolving Compound 1in formic acid to obtain an active premix, dissolving Kleptose® HPB in water to obtain a Kleptose solution, adding the premix to the Kleptose solution to obtain a mixture, adding water to the mixure to obtain a bulk solution, filtering the bulk solution through one or more 0.45 μm and 0.22 μm filters to obtain a filtered solution, filling the filtered solution into a vial, and lyophilizing the solution. In one embodiment, the solution is filtered through one 0.45 μm and two 0.22 μm filters. In one embodiment, the process comprises dissolving Compound 1in formic acid to obtain an active premix, dissolving Kleptose® HPB in water to obtain a Kleptose solution, adding the premix to the Kleptose solution to obtain a mixture, adding water to the mixure to obtain a bulk solution, filtering the bulk solution through one 0.45 μm and two 0.22 μm filters to obtain a filtered solution, filling the filtered solution into a 20 cc glass vial, and lyophilizing the solution. In one embodiment, the vial is sealed under nitrogen after lyophilization.

In one aspect, the lyophilization process contains three stages: freezing, primary drying, and secondary drying. A liquid formulation is transformed to a lyophilized powder form by going through complete solidification through freezing stage, sublimation of ice and solvents through primary drying, and desorption of residual moisture and solvents through secondary drying. The shelf temperature and chamber pressure in the primary drying and secondary drying are controlled to obtain the desired quality of the finished drug product. In one aspect of the process, the cake appearance and structure was characterized by visual inspection.

**Kits** (not claimed)

Pharmaceutical packs or kits which comprise pharmaceutical compositions or dosage forms provided herein

are also provided. Exemplary kits include notice in the form prescribed by a governmental agency regulating the manufacture, use or sale of pharmaceuticals products, which notice reflects approval by the agency of manufacture, use or sale for human administration.

**Methods of Use and Compound 1 for use in such methods**

Compound 1 as provided herein can be used in all methods provided herein. Provided herein is Compound 1 for use in methods of treating, preventing, managing, and/or ameliorating cancers, including solid tumors and hematological cancers, or one or more symptoms or causes thereof, wherein Compound 1 is administered in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors

In one embodiment, provided herein is a formulation of Compound 1 for use in a method of treating and preventing cancer, which comprises administering to a patient a formulation of Compound 1 provided herein.

In another embodiment, provided herein is Compound 1 for use in a method of managing cancer, which comprises administering to a patient a formulation of Compound 1 provided herein.

In one embodiment, the formulations for use provided herein comprise administering a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

Also exemplarily shown herein are the formulations for use in methods of treating patients who have been previously treated for cancer but are non-responsive to cancer therapies, as well as those who have not previously been treated. Also encompassed are methods of treating patients regardless of patient's age, although some diseases or disorders are more common in certain age groups.

The formulations may be for use in methods of treating patients who have undergone surgery in an attempt to treat the disease or condition at issue, as well as those who have not. Because patients with cancer have heterogeneous clinical manifestations and varying clinical outcomes, the treatment given to a patient may vary, depending on his/her prognosis. The skilled clinician will be able to readily determine without undue experimentation specific secondary agents, types of surgery, and types of non-drug based standard therapy that can be effectively used to treat an individual patient with cancer.

Provided herein is Compound 1 for use in methods for improving the Eastern Cooperative Oncology Group Performance Status (ECOG) of a cancer patient, comprising administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in methods for improving the Eastern Cooperative Oncology Group Performance Status (ECOG) of a cancer patient, comprising administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in improving the Eastern Cooperative Oncology Group Performance Status (ECOG) of a cancer patient, comprising administering an effective amount of a formulation of Compound 1 to the patient.

In one embodiment, provided herein are said formulations for use in methods for inhibition of disease progression, inhibition of tumor growth, reduction of primary tumor, relief of tumor-related symptoms, inhibition of tumor secreted factors, delaying appearance of primary or secondary tumors, slowing development of primary or secondary tumors, decreasing occurrence of primary or secondary tumors, slowing or decreasing severity of secondary effects of disease, arresting tumor growth and regression of tumors, increasing time to progression, increasing progression free survival, increasing overall survival or one or more thereof, in a cancer patient, comprising administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is Compound 1 for use in all such methods in a cancer patient, comprising administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment, provided herein are said formulations for use in methods for inhibition of disease progression, inhibition of tumor growth, reduction of primary tumor, relief of tumor-related symptoms, inhibition of tumor secreted factors, delaying appearance of primary or secondary tumors, slowing development of primary or secondary tumors, decreasing occurrence of primary or secondary tumors, slowing or decreasing severity of secondary effects of disease,

arresting tumor growth and regression of tumors, increasing time to progression, increasing progression free survival, increasing overall survival in a cancer patient, or one or more thereof, comprising administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is Compound 1 for use in all such methods in a cancer patient, or one or more thereof, comprising administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment, provided herein are said formulations for use in methods for inhibition of disease progression, inhibition of tumor growth, reduction of primary tumor, relief of tumor-related symptoms, inhibition of tumor secreted factors, delaying appearance of primary or secondary tumors, slowing development of primary or secondary tumors, decreasing occurrence of primary or secondary tumors, slowing or decreasing severity of secondary effects of disease, arresting tumor growth and regression of tumors, increasing time to progression, increasing progression free survival, increasing overall survival in a cancer patient, or one or more thereof, comprising administering an effective amount of a formulation of Compound 1 to the patient. Provided herein is Compound 1 for use in all such methods in a cancer patient, or one or more thereof, comprising administering an effective amount of a formulation of Compound 1 to the patient.

In certain embodiments, the cancer is a solid tumor or a hematological cancer. In certain embodiments, the cancer is interleukin-3 (IL-3) independent. In certain embodiments, the cancer is a solid tumor. In certain embodiments, the solid tumor is metastatic. In certain embodiments, the solid tumor is drug-resistant.

In certain embodiments, cancer refers to a disease of skin tissues, organs, blood, and vessels. In certain embodiments, the cancer is a solid tumor, including, but not limited to, cancers of the bladder, bone, blood, brain, breast, cervix, chest, colon, endometrium, esophagus, eye, head, kidney, liver, lymph nodes, lung, mouth, neck, ovaries, pancreas, prostate, rectum, stomach, testis, throat, and uterus. Specific cancers include, but are not limited to, advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, colorectal cancer, including stage 3 and stage 4, unresectable colorectal carcinoma, metastatic hepatocellular carcinoma, Kaposi's sarcoma, karyotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, malignant melanoma, malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unresectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, carcinoma, including papillary thyroid carcinoma, follicular thyroid carcinoma, and medullary thyroid carcinoma, and leiomyoma.

In certain embodiments, the cancer is a solid tumor, including, but not limited to, cancers of the skin, central nervous system, soft tissue, salivary gland, ovary, kidney, lung, bone, stomach, endometrium, pancreas, urinary tract, thyroid, upper aerodigestive tract, breast, large intestine, oesophagus, prostate, liver, autonomic ganglia, and malignant pleural mesothelioma.

In certain embodiments, the solid tumor is hepatocellular carcinoma, prostate cancer, ovarian cancer, or glioblastoma.

In certain embodiments, the solid tumor is breast cancer, kidney cancer, pancreatic cancer, gastrointestinal cancer, lung cancer, neuroendocrine tumor (NET), or renal cell carcinoma (RCC).

In certain embodiments, the cancer is a hematological cancer. In certain embodiments, the hematological cancer is metastatic. In certain embodiments, the hematological cancer is drug resistant to at least one anti-cancer therapy. In certain embodiments the hematological cancer is relapsed or refractory to at least one anti-cancer therapy.

In one embodiment, the hematological cancer is multiple myeloma (MM). In one embodiment, the hematological cancer is relapsed/refractory (R/R) MM. In one embodiment, the patient having R/R MM has impaired renal function.

In one embodiment provided herein is the inventive formulation for use in a method for achieving a stringent complete remission (sCR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is Compound 1 for use in a method for achieving a stringent complete remission (sCR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a stringent complete remission (sCR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is a formulation of Compound 1 for use in a method for achieving a stringent complete remission (sCR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a stringent complete remission (sCR) in anMM patient, wherein the method comprises administering an effective amount of a formulation of Compound 1 to the patient. Provided herein is a formulation of Compound 1 for use in a method for achieving a stringent complete remission (sCR) in anMM patient, wherein the method comprises administering an effective amount of a formulation of Compound 1 to the patient.

In one embodiment provided herein is the inventive formulation for use in a method for achieving a complete remission (CR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is Compound 1 for use in a method for achieving a complete remission (CR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a complete remission (CR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is a formulation of Compound 1 for use in a method for achieving a complete remission (CR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a complete remission (CR) in an MM patient, wherein the method comprises administering an effective amount of a formulation of Compound 1 to the patient. Provided herein is a formulation of Compound 1 for use in a method for achieving a complete remission (CR) in an MM patient, wherein the method comprises administering an effective amount of a formulation of Compound 1 to the patient.

In one embodiment provided herein is the inventive formulation for use in a method for achieving a very good partial response (VGPR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is Compound 1 for use in a method for achieving a very good partial response (VGPR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a very good partial response (VGPR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is a formulation of Compound 1 for use in a method for achieving a very good partial response (VGPR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is the inventive formulation for use in a method for achieving a very good partial response (VGPR) in an MM patient, wherein the method comprises administering an effective amount of a formulation of Compound 1 to the patient. Provided herein is a formulation of Compound 1 for use in a method for achieving a very good partial response (VGPR) in an MM patient.

Provided herein is Compound 1 for use in a method for achieving a partial response (PR) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial response (PR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is a formulation of Compound 1for use in a method for achieving a partial response (PR) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial response (PR) in an MM patient, wherein the method comprises administering an effective amount of a formulation of Compound 1 to the patient. Provided herein is a formulation of Compound 1 for use in a method for achieving a partial response in an MM patient.

Provided herein is Compound 1 for use in a method for achieving a stable disease (SD) in an MM patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a stable disease (SD) in an MM patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a stable disease (SD) in an MM patient, wherein the method comprises administering an effective amount of a formulation of Compound 1 to the patient. Provided herein is a formulation of Compound 1 for use in a method for achieving a stable disease in an MM patient.

In one embodiment, the hematological cancer is acute myelogenous leukemia (AML). In one embodiment, the hematological cancer is acute lymphocytic leukemia (ALL). In one embodiment, the hematological cancer is adult T-cell leukemia. In one embodiment, the hematological cancer is chronic lymphocytic leukemia (CLL). In one embodiment, the hematological cancer is hairy cell leukemia. In one embodiment, the hematological cancer is myelodysplasia. In one embodiment, the hematological cancer is a myeloproliferative disorder or myeloproliferative neoplasm (MPN). In one embodiment, the hematological cancer is chronic myelogenous leukemia (CML). In one embodiment, the hematological cancer is myelodysplastic syndrome (MDS). In one embodiment, the hematological cancer is human lymphotropic virus-type 1 (HTLV-1) leukemia. In one embodiment, the hematological cancer is mastocytosis. In one embodiment, the hematological cancer is B-cell acute lymphoblastic leukemia. In one embodiment, the hematological cancer is CLL.

In one embodiment, provided herein is a formulation of Compound 1 for use in methods of treating, preventing, managing, and/or ameliorating a cancer selected from diffuse large B-cell lymphoma (DLBCL), B-cell immunoblastic lymphoma, small non-cleaved cell lymphoma, human lymphotropic virus-type 1 (HTLV-1) leukemia/lymphoma, adult T-cell lymphoma, mantle cell lymphoma (MCL), Hodgkin lymphoma (HL), non-Hodgkin lymphoma (NHL), AIDS-related lymphoma, follicular lymphoma, small lymphocytic lymphoma, T-cell/histiocyte rich large B-cell lymphoma, transformed lymphoma, primary mediastinal (thymic) large B-cell lymphoma, splenic marginal zone lymphoma, Richter's transformation, nodal marginal zone lymphoma, and ALK-positive large B-cell lymphoma in a subject, comprising the step of administering to the subject an amount of a formulation of Compound 1 provided herein effective to treat, prevent and/or manage the cancer. Thus, provided herein is a formulation of Compound 1 for use in all said methods of treating, preventing, managing, and/or ameliorating a cancer, wherein the cancer is selected from diffuse large B-cell lymphoma (DLBCL), B-cell immunoblastic lymphoma, small non-cleaved cell lymphoma, human lymphotropic virus-type 1 (HTLV-1) leukemia/lymphoma, adult T-cell lymphoma, mantle cell lymphoma (MCL), Hodgkin lymphoma (HL), non-Hodgkin lymphoma (NHL), AIDS-related lymphoma, follicular lymphoma, small lymphocytic lymphoma, T-cell/histiocyte rich large

B-cell lymphoma, transformed lymphoma, primary mediastinal (thymic) large B-cell lymphoma, splenic marginal zone lymphoma, Richter's transformation, nodal marginal zone lymphoma, and ALK-positive large B-cell lymphoma in a subject,. In some embodiments, the formulation for use in said methods comprise the step of administering to the subject a formulation of Compound 1 provided herein in combination with a second active agent in amounts effective to treat, prevent and/or manage the cancer. In one embodiment, the hematological cancer is HL. In one embodiment, the hematological cancer is NHL. In one embodiment, the hematological cancer is indolent lymphoma including, for example, DLBCL, follicular lymphoma, and marginal zone lymphoma.

Provided herein is Compound 1 for use in a method for achieving a complete remission (CR) in an NHL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a complete remission (CR) in an NHL patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a complete remission (CR) in an NHL patient, wherein the method comprises administering an effective amount of a formulation of Compound 1 to the patient. Provided herein is a formulation of Compound 1 for use in a method for achieving a complete remission (CR) in an NHL patient.

Provided herein is Compound 1 for use in a method for achieving a partial remission (PR) in an NHL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a partial remission (PR) in an NHL patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial remission (PR) in an NHL patient, wherein the method comprises administering an effective amount of a formulation of Compound 1 to the patient. Provided herein is a formulation of Compound 1 for use in a method for achieving a partial remission (PR) in an NHL patient.

Provided herein is Compound 1 for use in a method for achieving a stable disease (SD) in an NHL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a stable disease (SD) in an NHL patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a stable disease (SD) in an NHL patient, wherein the method comprises administering an effective amount of a formulation of Compound 1 to the patient. Provided herein is a formulation of Compound 1 for use in a method for achieving a stable disease (SD) in an NHL patient.

In one embodiment, provided herein is Compound 1 for use in methods of treating, preventing, managing, and/or ameliorating leukemia by administering a therapeutically active amount of Compound 1 to a subject. Thus, provided herein is Compound 1 for use in such methods of treating, preventing, managing, and/or ameliorating leukemia. In one embodiment, the leukemia is acute myeloid leukemia (AML). In one embodiment, the AML is relapsed or refractory AML. In one embodiment, the AML is newly diagnosed AML. In another embodiment, the AML has FAB classification M0/1. In another embodiment, the AML has FAB classification M2. In another embodiment, the AML has FAB classification M3. In another embodiment, the AML has FAB classification M4. In another embodiment, the AML has FAB classification M5. In one embodiment, the AML is AML with at least one recurrent genetic abnormality (for example, AML with translocation between chromosomes 8 and 21; AML with translocation or inversion in chromosome 16; AML with translocation

between chromosomes 9 and 11; APL (M3) with translocation between chromosomes 15 and 17; AML with translocation between chromosomes 6 and 9; AML with translocation or inversion in chromosome 3); AML (megakaryoblastic) with a translocation between chromosomes 1 and 22; AML with myelodysplasia-related changes; AML related to previous chemotherapy or radiation (for example, alkylating agent-related AML; or Topoisomerase II inhibitor-related AML); AML not otherwise categorized (for example, AML that does not fall into the above categories, i. e. AML minimally differentiated (M0); AML with minimal maturation (M1); AML with maturation (M2); Acute myelomonocytic leukemia (M4); Acute monocytic leukemia (M5); Acute erythroid leukemia (M6); Acute megakaryoblastic leukemia (M7); Acute basophilic leukemia; or Acute panmyelosis with fibrosis); Myeloid Sarcoma (also known as granulocytic sarcoma, chloroma or extramedullary myeloblastoma); or Undifferentiated and biphenotypic acute leukemias (also known as mixed phenotype acute leukemias). In one embodiment, the AML is characterized by a mutant allele of IDH2. In one aspect of this embodiment, the mutant allele of IDH2 has an R140X mutation. In another aspect of this embodiment, the R140X mutation is a R140Q mutation. In another aspect of this embodiment, the R140X mutation is a R140W mutation. In another aspect of this embodiment, the R140X mutation is a R140L mutation. In another aspect of this embodiment, the mutant allele of IDH2 has an R172X mutation. In another aspect of this embodiment, the R172X mutation is a R172K mutation. In another aspect of this embodiment, the R172X mutation is a R172G mutation.

In one embodiment, the AML is relapsed AML after allogeneic HSCT. In one embodiment, the AML is second or later relapsed AML. In one embodiment, the AML is refractory to initial induction or re-induction treatment. In certain embodiments, the AML is refractory to at least one induction/reinduction or consolidation therapy. In one embodiment, the AML is refractory to or relapsed after hypomethylating agent (HMA). As used herein, HMA failure is defined as primary progression or lack of clinical benefit after a minimum of 6 cycles or unable to tolerate HMA due to toxicity. In one embodiment, the AML is relapsed within 1 year of initial treatment (excluding AML with favorable-risk status).

In some embodiments, the methods comprise the step of administering to the subject a formulation of Compound 1 provided herein in combination with a second active agent in amounts effective to treat, prevent and/or manage acute myeloid leukemia.

Provided herein is Compound 1 for use in a method for achieving a morphologic leukemia free state in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a morphologic leukemia free state in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a morphologic leukemia free state in an AML patient, wherein the method comprises administering an effective amount of a formulation of Compound 1 to the patient. Provided herein is a formulation of Compound 1 for use in a method for achieving a morphologic leukemia free state in an AML patient.

Provided herein is Compound 1 for use in a method for achieving a morphologic complete remission in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a morphologic complete remission in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a morphologic complete remission in an AML patient, wherein the method comprises administering an effective amount of a formulation of Compound 1 to the patient. Provided herein is a formulation of Compound 1 for use in a method for achieving a morphologic complete remission in an AML patient.

In one embodiment provided herein is Compound 1 for use in a method for achieving a cytogenetic complete remission (CRc) in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a cytogenetic complete

remission (CRc) in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a cytogenetic complete remission (CRc) in an AML patient, wherein the method comprises administering an effective amount of a formulation of Compound 1 to the patient. Provided herein is a formulation of Compound 1 for use in a method for achieving a cytogenetic complete remission (CRc) in an AML patient.

Provided herein is Compound 1 for use in a method for achieving a molecular complete remission (CRm) in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a molecular complete remission (CRm) in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a molecular complete remission (CRm) in an AML patient, wherein the method comprises administering an effective amount of a formulation of Compound 1 to the patient. Provided herein is a formulation of Compound 1 for use in a method for achieving a molecular complete remission (CRm) in an AML patient.

Provided herein is Compound 1 for use in a method for achieving a morphologic complete remission with incomplete blood recovery (CRi) in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a morphologic complete remission with incomplete blood recovery (CRi) in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a morphologic complete remission with incomplete blood recovery (CRi) in an AML patient, wherein the method comprises administering an effective amount of a formulation of Compound 1 to the patient. Provided herein is a formulation of Compound 1 for use in a method for achieving a morphologic complete remission with incomplete blood recovery (CRi) in an AML patient.

Provided herein is Compound 1 for use in a method for achieving a partial remission in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a partial remission (PR) in an AML patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial remission (PR) in an AML patient, wherein the method comprises administering an effective amount of a formulation of Compound 1 to the patient. Provided herein is a formulation of Compound 1 for use in a method for achieving a partial remission (PR) in an AML patient.

Provided herein is Compound 1 for use in a method for achieving a complete remission (CR) in an AML patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a complete remission (CR) in an AML patient, wherein the method comprises administering an effective amount of a formulation of Compound 1 in combination with one or more second agents selected from JAK inhibitors, FLT3 inhibitors, mTOR inhibitors, spliceosome

inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a complete remission (CR) in an AML patient, wherein the method comprises administering an effective amount of a formulation of Compound 1 to the patient. Provided herein is a formulation of Compound 1 for use in a method for achieving a complete remission (CR) in an AML patient.

In some embodiments, the formulation of Compound 1 for use in the methods provided herein encompass treating, preventing and/or managing acute lymphocytic leukemia (ALL) in a subject. In some embodiments, ALL includes leukemia that originates in the blast cells of the bone marrow (B-cells), thymus (T-cells), and lymph nodes. The ALL can be categorized according to the French-American-British (FAB) Morphological Classification Scheme as L1 - Mature-appearing lymphoblasts (T-cells or pre-B-cells), L2 - Immature and pleomorphic (variously shaped) lymphoblasts (T-cells or pre-B-cells), and L3 - Lymphoblasts (B-cells; Burkitt's cells). In one embodiment, the ALL originates in the blast cells of the bone marrow (B-cells). In one embodiment, the ALL originates in the thymus (T-cells). In one embodiment, the ALL originates in the lymph nodes. In one embodiment, the ALL is L1 type characterized by mature-appearing lymphoblasts (T-cells or pre-B-cells). In one embodiment, the ALL is L2 type characterized by immature and pleomorphic (variously shaped) lymphoblasts (T-cells or pre-B-cells). In one embodiment, the ALL is L3 type characterized by lymphoblasts (B-cells; Burkitt's cells). In certain embodiments, the ALL is T-cell leukemia. In one embodiment, the T-cell leukemia is peripheral T-cell leukemia. In another embodiment, the T-cell leukemia is T-cell lymphoblastic leukemia. In another embodiment, the T-cell leukemia is cutaneous T-cell leukemia. In another embodiment, the T-cell leukemia is adult T-cell leukemia. In certain embodiments, the methods of treating, preventing and/or managing ALL in a subject comprise the step of administering to the subject an amount of a formulation of Compound 1 provided herein effective to treat, prevent and/or manage ALL. In some embodiments, the methods comprise the step of administering to the subject a formulation of Compound 1 provided herein in combination with a second active agent in amounts effective to treat, prevent and/or manage ALL.

In some embodiments, the formulation of Compound 1 for use in the methods provided herein encompass treating, preventing and/or managing chronic myelogenous leukemia (CML) in a subject. The methods comprise the step of administering to the subject an amount of a formulation of Compound 1 provided herein effective to treat, prevent and/or manage CML. In some embodiments, the methods comprise the step of administering to the subject a formulation of Compound 1 provided herein in combination with a second active agent in amounts effective to treat, prevent and/or manage CML.

In some embodiments, the formulation of Compound 1 for use in the methods provided herein encompass treating, preventing and/or managing chronic lymphocytic leukemia (CLL) in a subject. The methods comprise the step of administering to the subject an amount of a formulation of Compound 1 provided herein effective to treat, prevent and/or manage chronic lymphocytic leukemia. In some embodiments, the methods comprise the step of administering to the subject a formulation of Compound 1 provided herein in combination with a second active agent in amounts effective to treat, prevent and/or manage CLL.

Provided herein is Compound 1 for use in a method for achieving a complete remission (CR) in a CLL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a complete remission (CR) in a CLL patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a method for achieving a complete remission (CR) in a CLL patient, wherein the formulation of Compound 1 for use in the method comprises administering an effective amount of a formulation of Compound 1 to the patient. Provided herein is a formulation of Compound 1 for use in a method for achieving a complete remission (CR) in a CLL patient.

Provided herein is Compound 1 for use in a method for achieving a partial remission (PR) in a CLL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a partial remission (PR) in a CLL patient, wherein the method comprises administering an effective amount of a formulation of Compound 1 in combination with one or more second agents selected from JAK inhibitors, FLT3 inhibitors, mTOR inhibitors, spliceosome

inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial remission (PR) in a CLL patient, wherein the method comprises administering an effective amount of a formulation of Compound 1 to the patient. Provided herein is a formulation of Compound 1 for use in a method for achieving a partial remission (PR) in a CLL patient.

Provided herein is Compound 1 for use in a method for achieving a stable disease (SD) in a CLL patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a stable disease (SD) in a CLL patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a stable disease (SD) in a CLL patient, wherein the method comprises administering an effective amount of a formulation of Compound 1 to the patient. Provided herein is Compound 1 for use in a method for achieving a stable disease (SD) in a CLL patient.

In one embodiment, provided herein is a formulation of Compound 1 for use in are methods of treating, preventing, managing, and/or ameliorating a myelodysplastic syndrome (MDS) by administering a therapeutically active amount of Compound 1 to a subject. In one embodiment provided herein is a method of treating MDS. Thus, provided herein is Compound 1 for use in such methods of treating, preventing, managing, and/or ameliorating MDS. In one embodiment, the MDS is relapsed, resistant or refractory MDS. In one embodiment, MDS is refractory anemia (RA); RA with ringed sideroblasts (RARS); RA with excess of blasts (RAEB); refractory cytopenia with multilineage dysplasia (RCMD), refractory cytopenia with unilineage dysplasia (RCUD); unclassifiable myelodysplastic syndrome (MDS-U), myelodysplastic syndrome associated with an isolated del(5q) chromosome abnormality, therapy-related myeloid neoplasms or chronic myelomonocytic leukemia (CMML). In some embodiments, the MDS is very low risk, low risk, intermediate risk, high risk or very high risk MDS. In one embodiment, the MDS is very low risk. In another embodiment, the MDS is low risk. In another embodiment, the MDS is intermediate risk. In another embodiment, the MDS is high risk. In another embodiment, the MDS is very high risk MDS. In one embodiment, the MDS is relapsed or refractory high risk MDS. In one embodiment, the MDS is with a score > 3.5 points in the Revised International Prognostic Scoring System (IPSS-R) (eg, IPSS-R intermediate risk (in combination with more than 10% bone marrow blasts or poor or very poor IPSS-R cytogenetic risk), IPSS-R high and IPSS-R very high risk]. In one embodiment, the MDS is not suitable for other established therapies (eg, transplant or hypomethylating agent). In some embodiments, the MDS is primary or de novo MDS. In other embodiments, the MDS is secondary MDS. In one embodiment, the MDS is refractory to initial induction or re-induction treatment. In certain embodiments, the MDS is refractory to at least one induction/reinduction or consolidation therapy. In certain embodiments, a formulation of Compound 1 for use in the methods of treating, preventing and/or managing MDS in a subject comprise the step of administering to the subject an amount of a formulation of Compound 1 provided herein effective to treat, prevent and/or manage MDS. In some embodiments, the methods comprise the step of administering to the subject a formulation of Compound 1 provided herein in combination with a second active agent in amounts effective to treat, prevent and/or manage MDS.

In one embodiment provided herein is a formulation of Compound 1 for use in a method for achieving a complete remission (CR) in an MDS patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a complete remission (CR) in an MDS patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient. Provided herein is a formulation of Compound 1 for use in a method for achieving a complete remission (CR) in an MDS patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a complete

remission (CR) in an MDS patient, wherein the method comprises administering an effective amount of a formulation of Compound 1 to the patient. Provided herein is a formulation of Compound 1 for use in a method for achieving a complete remission (CR) in an MDS patient.

Provided herein is Compound 1 for use in a method for achieving a marrow complete remission (mCR) in an MDS patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a marrow complete remission (mCR) in an MDS patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a marrow complete remission (mCR) in an MDS patient, wherein the method comprises administering an effective amount of a formulation of Compound 1 to the patient. Provided herein is a formulation of Compound 1 for use in a method for achieving a marrow complete remission (mCR) in an MDS patient.

Provided herein is Compound 1 for use in a method for achieving a partial remission (PR) in an MDS patient, wherein the method comprises administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1 for use in a method for achieving a partial remission (PR) in an MDS patient, wherein the method comprises administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment provided herein is a formulation of Compound 1 for use in a method for achieving a partial remission (PR) in an MDS patient, wherein the method comprises administering an effective amount of a formulation of Compound 1 to the patient. Provided herein is a formulation of Compound 1 for use in a method for achieving a partial remission (PR) in an MDS patient.

Provided herein is Compound 1 for use in methods for increasing overall survival, increasing relapse free survival, increasing progression free survival, increasing event-free survival, increasing duration of remission, increasing duration of response, or increasing time to transformation to AML in an MDS patient, comprising administering an effective amount of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

Provided herein is a formulation of Compound 1for use in methods for increasing overall survival, increasing relapse free survival, increasing progression free survival, increasing event-free survival, increasing duration of remission, increasing duration of response, or increasing time to transformation to AML in an MDS patient, comprising administering an effective amount of a formulation of Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors to the patient.

In one embodiment, provided herein is a formulation of Compound 1 for use in methods for increasing overall survival, increasing relapse free survival, increasing progression free survival, increasing event-free survival, increasing duration of remission, increasing duration of response, or increasing time to transformation to AML in an MDS patient, comprising administering an effective amount of a formulation of Compound 1 to the patient. Provided herein is a formulation of Compound 1 for use in methods for increasing overall survival, increasing relapse free survival, increasing progression free survival, increasing event-free survival, increasing duration of remission, increasing duration of response, or increasing time to transformation to AML in an MDS patient.

In some embodiments, the formulation of Compound 1 for use in the methods provided herein encompass treating, preventing and/or managing a myeloproliferative neoplasm. In one embodiment, the myeloproliferative neoplasm is polycythemia vera, primary or essential thrombocythemia, myelofibrosis, chronic myelogenous leukemia, chronic neutrophilic leukemia, juvenile myelomonocytic leukemia, chronic eosinophilic leukemia, or hyper eosinophilic syndrome. In one embodiment, the myeloproliferative neoplasm is polycythemia vera, primary or essential thrombocythemia, primary or idiopathic myelofibrosis, secondary myeolofibrosis, post polycythemia vera myelofibrosis, post essential thrombocythemia myelofibrosis, chronic myelogenous leukemia, chronic neutrophilic leukemia, juvenile myelomonocytic leukemia, chronic eosinophilic leukemia, or hyper eosinophilic syndrome. In one embodiment, the myeloproliferative neoplasm

is polycythemia vera. In one embodiment, the myeloproliferative neoplasm is primary or essential thrombocythemia. In one embodiment, the myeloproliferative neoplasm is myelofibrosis. In one embodiment, the myeloproliferative neoplasm is primary or idiopathic myelofibrosis. In one embodiment, the myeloproliferative neoplasm is secondary myeolofibrosis. In one embodiment, the myeloproliferative neoplasm is post polycythemia vera myelofibrosis. In one embodiment, the myeloproliferative neoplasm is post essential thrombocythemia myelofibrosis. In one embodiment, the myeloproliferative neoplasm is chronic myelogenous leukemia. In one embodiment, the myeloproliferative neoplasm is chronic neutrophilic leukemia. In one embodiment, the myeloproliferative neoplasm is juvenile myelomonocytic leukemia. In one embodiment, the myeloproliferative neoplasm is chronic eosinophilic leukemia. In one embodiment, the myeloproliferative neoplasm is hyper eosinophilic syndrome. In certain embodiments, the myeloproliferative neoplasm is interleukin-3 (IL-3) independent. In some embodiments, the myeloproliferative neoplasm is characterized by a JAK mutation, for example, a V617 mutation, such as V617F.

In certain embodiments, the formulation of Compound 1 for use in the methods of treating, preventing and/or managing a myeloproliferative neoplasm in a subject comprise the step of administering to the subject an amount of a formulation of Compound 1 provided herein effective to treat, prevent and/or manage myeloproliferative neoplasm. In some embodiments, the methods comprise the step of administering to the subject a formulation of Compound 1 provided herein in combination with a second active agent in amounts effective to treat, prevent and/or manage myeloproliferative neoplasm.

In one embodiment, the formulation of Compound 1 for use in the methods of treating, preventing and/or managing cancer provided herein comprise intravenous administration of a formulation of Compound 1. In one embodiment, the formulation of Compound 1 is dissolved in water to form an aqueous solution for intravenous administration in methods of treating, preventing and/or managing cancer provided herein.

In some embodiments, the methods comprise the step of administering to the subject a formulation of Compound 1 provided herein in combination with a second active agent in amounts effective to treat, prevent and/or manage cancer.

In certain embodiments, provided herein is a formulation of Compound 1 for use in methods of treating, preventing, and/or managing cancer in patients with impaired renal function. In certain embodiments, provided herein is a formulation of Compound 1 for use in methods of providing appropriate dose adjustments for patients with impaired renal function due to, but not limited to, disease, aging, or other patient factors.

In one embodiment, provided herein is a formulation of Compound 1 for use in methods of reducing GSPT1 levels in a subject, the methods comprising administering Compound 1 in combination with a second agent as described herein, to the subject. Thus, provided herein is Compound 1 for use in methods of reducing GSPT1 levels in a subject, the methods comprising administering Compound 1 in combination with a second agent as described herein, to the subject. In some embodiments, provided herein is a formulation of Compound 1 for use in a method of monitoring the efficacy of treatment with Compound 1 in combination with a second agent in the treatment of cancer in a subject, comprising: (a) administering Compound 1 and a second agent to the subject; (b) obtaining a sample from the subject; (c) determining the level of GSPT1 in the sample; (d) comparing the level of GSPT1 in the sample with the level of GSPT1 in a reference sample, wherein a decrease in the GSPT1 level in the sample as compared to in the reference sample is indicative of the efficacy of the treatment with Compound 1 and the second agent of the cancer in the subject. Thus, provided herein is Compound 1 for use in such a method of monitoring the efficacy of treatment with Compound 1 in combination with a second agent in the treatment of cancer in a subject. In yet another aspect, provided herein is a method of predicting the responsiveness of a subject having or suspected of having cancer to treatment with Compound 1 and a second agent, the method comprising (a) administering Compound 1 and a second agent to the subject; (b) obtaining a sample from the subject; (c) determining the level of GSPT1 in the sample; (d) diagnosing the subject as being likely to be responsive to treatment of the cancer with Compound 1 and the second agent if the level of GSPT1 in the sample is reduced compared to the GSPT1 level in a reference sample. Thus, provided herein is Compound 1 for use in a method of predicting the responsiveness of a subject having or suspected of having cancer to treatment with Compound 1 and a second agent.

In one embodiment, provided herein is a formulation of Compound 1 for use in methods of reducing Mcl-1 levels in a subject, the methods comprising administering Compound 1 in combination with a second agent as described herein, to the subject. Thus, provided herein is Compound 1 for use in such methods of reducing Mcl-1 levels in a subject, the methods comprising administering Compound 1 in combination with a second agent as described herein, to the subject. Provided herein is Compound 1 for use in a method of monitoring the efficacy of treatment with Compound 1 in combination with a second agent in the treatment of cancer in a subject. In yet another aspect, provided herein is a method of predicting the responsiveness of a subject having or suspected of having cancer to treatment with Compound 1 and a second agent, the method comprising (a) administering Compound 1 and a second agent to the subject; (b) obtaining a sample from the subject; (c) determining the level of Mcl-1 in the sample; (d) diagnosing the subject as being likely to be responsive to treatment of the cancer with Compound 1 and the second agent if the level of Mcl-1 in the sample is reduced compared to the Mcl-1 level in a reference sample. Thus, provided herein is Compound 1 for use in a method of predicting the responsiveness of a subject having or suspected of having cancer to treatment with Compound

1 and a second agent.

In some embodiments of the methods provided herein, the reference sample is obtained from the subject prior to administering Compound 1 and the second agent to the subject, and the reference sample is from the same source as the sample. In other embodiments of the methods provided herein, the reference sample is obtained from a second subject having cancer, and the reference sample is from the same source as the sample. In still other embodiments of the methods provided herein, the reference sample is obtained from a group of subjects having cancer, and the reference sample is from the same source as the sample.

In one embodiment, which is not claimed, provided herein is a method of identifying a cancer subject suitable for treatment with Compound 1 and a second agent comprising: (a) obtaining a sample from a subject having cancer; (b) determining the level of GSPT1 in the sample; (c) contacting the sample with Compound 1 and the second agent; (d) determining the level of GSPT1 in the sample after the contacting step; (e) identifying the subject as being likely to be responsive to treatment of the cancer with Compound 1 and the second agent if the level of GSPT1 in step (d) is reduced compared to the level of GSPT1 in step (b).

In one embodiment, which is not claimed, provided herein is a method of identifying a cancer subject suitable for treatment with Compound 1 and a second agent comprising: (a) obtaining a sample from a subject having cancer; (b) determining the level of Mcl-1 in the sample; (c) contacting the sample with Compound 1 and the second agent; (d) determining the level of Mcl-1 in the sample after the contacting step; (e) identifying the subject as being likely to be responsive to treatment of the cancer with Compound 1 and the second agent if the level of Mcl-1 in step (d) is reduced compared to the level of Mcl-1 in step (b). Thus, provided herein is Compound 1 for use in a method of identifying a cancer subject suitable for treatment with Compound 1 and a second agent.

The term "sample" as used herein refers to a material or mixture of materials obtained from a subject, including a sample of tissue or fluid origin, obtained, reached, or collected *in vivo* or *in situ.* A sample also includes samples from a region of a subject containing precancerous or cancer cells or tissues. Such samples can be, but are not limited to, organs, tissues, and cells isolated from a mammal. Exemplary samples include but are not limited to cell lysate, a cell culture, a cell line, a tissue, oral tissue, gastrointestinal tissue, an organ, an organelle, a biological fluid, a blood sample, a urine sample, a skin sample, and the like. In one embodiment, samples include, but are not limited to, whole blood, partially purified blood, PBMC, tissue biopsies including bone marrow core biopsy, bone marrow aspirate, isolated bone marrow mononuclear cells, circulating tumor cells and the like.

In some such embodiments, the second agent is selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors, as described herein.

In certain embodiments, a therapeutically or prophylactically effective amount of Compound 1 is from about 0.005 to about 20 mg per day, from about 0.05 to 20 mg per day, from about 0.01 to about 10 mg per day, from about 0.01 to about 7 mg per day, from about 0.01 to about 5 mg per day, from about 0.01 to about 3 mg per day, from about 0.05 to about 10 mg per day, from about 0.05 to about 7 mg per day, from about 0.05 to about 5 mg per day, from about 0.05 to about 3 mg per day, from about 0.1 to about 15 mg per day, from about 0.1 to about 10 mg per day, from about 0.1 to about 7 mg per day, from about 0.1 to about 5 mg per day, from about 0.1 to about 3 mg per day, from about 0.5 to about 10 mg per day, from about 0.05 to about 5 mg per day, from about 0.5 to about 3 mg per day, from about 0.5 to about 2 mg per day, from about 0.3 to about 10 mg per day, from about 0.3 to about 8.5 mg per day, from about 0.3 to about 8.1 mg per day, from about 0.6 to about 10 mg per day or from about 0.6 to about 5 mg per day. In one embodiment, a therapeutically or prophylactically effective amount of Compound 1 is from about 0.005 to about 20 mg per day. In one embodiment, a therapeutically or prophylactically effective amount of Compound 1 is, from about 0.05 to 20 mg per day. In one embodiment, a therapeutically or prophylactically effective amount of Compound 1 is from about 0.01 to about 10 mg per day. In one embodiment, a therapeutically or prophylactically effective amount of Compound 1 is from about 0.01 to about 7 mg per day. In one embodiment, a therapeutically or prophylactically effective amount of Compound 1 is from about 0.01 to about 5 mg per day. In one embodiment, a therapeutically or prophylactically effective amount of Compound 1 is from about 0.01 to about 3 mg per day. In one embodiment, a therapeutically or prophylactically effective amount of Compound 1 is from about 0.05 to about 10 mg per day. In one embodiment, a therapeutically or prophylactically effective amount of Compound 1 is from about 0.05 to about 7 mg per day. In one embodiment, a therapeutically or prophylactically effective amount of Compound 1 is from about 0.05 to about 5 mg per day. In one embodiment, a therapeutically or prophylactically effective amount of Compound 1 is from about 0.05 to about 3 mg per day. In one embodiment, a therapeutically or prophylactically effective amount of Compound 1 is from about 0.1 to about 15 mg per day. In one embodiment, a therapeutically or prophylactically effective amount of Compound 1 is from about 0.1 to about 10 mg per day. In one embodiment, a therapeutically or prophylactically effective amount of Compound 1 is from about 0.1 to about 7 mg per day. In one embodiment, a therapeutically or prophylactically effective amount of Compound 1 is from about 0.1 to about 5 mg per day. In one embodiment, a therapeutically or prophylactically effective amount of Compound 1 is from about 0.1 to about 3 mg per day. In one embodiment, a therapeutically or prophylactically effective amount of Compound 1 is from about 0.5 to about 10 mg per day. In one embodiment, a

therapeutically or prophylactically effective amount of Compound 1 is from about 0.5 to about 5 mg per day. In one embodiment, a therapeutically or prophylactically effective amount of Compound 1 is from about 0.5 to about 3 mg per day. In one embodiment, a therapeutically or prophylactically effective amount of Compound 1 is from about 0.5 to about 2 mg per day. In one embodiment, a therapeutically or prophylactically effective amount of Compound 1 is from about 0.3 to about 10 mg per day. In one embodiment, a therapeutically or prophylactically effective amount of Compound 1 is from about 0.3 to about 8.5 mg per day. In one embodiment, a therapeutically or prophylactically effective amount of Compound 1 is from about 0.3 to about 8.1 mg per day. In one embodiment, a therapeutically or prophylactically effective amount of Compound 1 is from about 0.6 to about 10 mg per day or from about 0.6 to about 5 mg per day.

In certain embodiments, the therapeutically or prophylactically effective amount is about 0.1, about 0.2, about 0.5, about 1, about 2, about 3, about 4, about 5, about 6, about 7, about 8, about 9, or about 10 mg per day. In some such embodiments, the therapeutically or prophylactically effective amount is about 0.5, about 0.6, about 0.75, about 1, about 2, about 3, about 4, about 5, about 6 or about 7 mg per day. In some such embodiments, the therapeutically or prophylactically effective amount is about 0.6, about 1.2, about 1.8, about 2.4, or about 3.6 mg per day. In certain embodiments, the therapeutically or prophylactically effective amount is about 0.1 mg per day. In certain embodiments, the therapeutically or prophylactically effective amount is about 0.2 mg per day. In certain embodiments, the therapeutically or prophylactically effective amount is about 0.5 mg per day. In certain embodiments, the therapeutically or prophylactically effective amount is about about 1 mg per day. In certain embodiments, the therapeutically or prophylactically effective amount is about about 2 mg per day. In certain embodiments, the therapeutically or prophylactically effective amount is about about 3 mg per day. In certain embodiments, the therapeutically or prophylactically effective amount is about about 4 mg per day. In certain embodiments, the therapeutically or prophylactically effective amount is about about 5 mg per day. In certain embodiments, the therapeutically or prophylactically effective amount is about about 6 mg per day. In certain embodiments, the therapeutically or prophylactically effective amount is about about 7 mg per day. In certain embodiments, the therapeutically or prophylactically effective amount is about about 8 mg per day. In certain embodiments, the therapeutically or prophylactically effective amount is about about 9 mg per day. In certain embodiments, the therapeutically or prophylactically effective amount is about about 10 mg per day.

In one embodiment, the recommended daily dose range of Compound 1, for the conditions described herein lie within the range of from about 0.01 mg to about 20 mg per day, preferably given as a single once-a-day dose, or in divided doses throughout a day. In one embodiment, the recommended daily dose range of Compound 1, for the conditions described herein lie within the range of from about 0.01 mg to about 15 mg per day, preferably given as a single once-a-day dose, or in divided doses throughout a day. In one embodiment, the recommended daily dose range of Compound 1, for the conditions described herein lie within the range of from about 0.01 mg to about 12 mg per day, preferably given as a single once-a-day dose, or in divided doses throughout a day. In some embodiments, the dosage ranges from about 0.1 mg to about 10 mg per day. In other embodiments, the dosage ranges from about 0.5 to about 5 mg per day. Specific doses per day include 0.1, 0.2, 0.5, 0.6, 1, 1.2, 1.5, 1.8, 2, 2.4, 2.5, 3, 3.5, 3.6, 4, 4.5, 5, 5.5, 6, 6.5, 7, 7.2, 7.5, 8, 8.5, 9, 9.5, 10, 10.5, 11, 11.5, 12, 12.5, 13, 13.5, 14, 14.4, 14.5 or 15 mg per day. In other embodiments, the dosage ranges from about 0.5 to about 5 mg per day. Specific doses per day include 0.1, 0.2, 0.5, 0.6, 1, 1.2, 1.5, 1.8, 2, 2.4, 2.5, 3, 3.5, 3.6, 4, 4.5, 5, 5.5, 6, 6.5, 7, 7.5, 8, 8.5, 9, 9.5 or 10 mg per day. In one embodiment, the dose per day is 0.1 mg per day. In one embodiment, the dose per day is 0.2 mg per day. In one embodiment, the dose per day is 0.5 mg per day. In one embodiment, the dose per day is 0.6 mg per day. In one embodiment, the dose per day is 1 mg per day. In one embodiment, the dose per day is 1.2 mg per day. In one embodiment, the dose per day is 1.5 mg per day. In one embodiment, the dose per day is 1.8 mg per day. In one embodiment, the dose per day is 2 mg per day. In one embodiment, the dose per day is 2.4 mg per day. In one embodiment, the dose per day is 2.5 mg per day. In one embodiment, the dose per day is 3 mg per day. In one embodiment, the dose per day is 3.5 mg per day. In one embodiment, the dose per day is 3.6 mg per day. In one embodiment, the dose per day is 4 mg per day. In one embodiment, the dose per day is 4.5 mg per day. In one embodiment, the dose per day is 5 mg per day. In one embodiment, the dose per day is 5.5 mg per day. In one embodiment, the dose per day is 6 mg per day. In one embodiment, the dose per day is 6.5 mg per day. In one embodiment, the dose per day is 7 mg per day. In one embodiment, the dose per day is 7.2 mg per day. In one embodiment, the dose per day is 7.5 mg per day. In one embodiment, the dose per day is 8 mg per day. In one embodiment, the dose per day is 8.5 mg per day. In one embodiment, the dose per day is 9 mg per day. In one embodiment, the dose per day is 9.5 mg per day. In one embodiment, the dose per day is 10 mg per day. In one embodiment, the dose per day is 12 mg per day. In one embodiment, the dose per day is 10 mg per day. In one embodiment, the dose per day is 12 mg per day. In one embodiment, the dose per day is 14.4 mg per day. In one embodiment, the dose per day is 15 mg per day.

In a specific embodiment, the recommended starting dosage may be 0.1, 0.5, 0.6, 0.7, 1, 1.2, 1.5, 1.8, 2, 2.4, 2.5, 3, 3.5, 3.6, 4, 4.5, 5, 5.5, 6, 6.5 or 7 mg per day. In another embodiment, the recommended starting dosage may be 0.1, 0.5, 0.6, 1, 1.2, 1.8, 2, 2.4, 3, 3.6, 4, or 5 mg per day. In one embodiment, the dose may be escalated to 7, 8, 9 10, 12, or 15 mg/day. In one embodiment, the dose may be escalated to 7, 8, 9 or 10 mg/day.

In a specific embodiment, Compound 1 can be administered in an amount of about 0.1 mg/day to patients with

leukemia, including AML. In a particular embodiment, Compound 1 can be administered in an amount of about 1 mg/day to patients with leukemia, including AML In a particular embodiment, Compound 1 can be administered in an amount of about 3 mg/day to patients with leukemia, including AML. In a particular embodiment, Compound 1 can be administered in an amount of about 4 mg/day to patients with leukemia, including AML. In a particular embodiment, Compound 1 provided herein can be administered in an amount of about 5 mg/day to patients with leukemia, including AML. In a particular embodiment, Compound 1 provided herein can be administered in an amount of about 6 mg/day to patients with leukemia, including AML. In a particular embodiment, Compound 1 provided herein can be administered in an amount of about 7 mg/day to patients with leukemia, including AML. In a particular embodiment, Compound 1 provided herein can be administered in an amount of about 10 mg/day to patients with leukemia, including AML. In a particular embodiment, Compound 1 provided herein can be administered in an amount of about 12 mg/day to patients with leukemia, including AML. In a particular embodiment, Compound 1 provided herein can be administered in an amount of about 15 mg/day to patients with leukemia, including AML

In a specific embodiment, Compound 1 can be administered in an amount of about 0.1 mg/day to patients with MDS. In a particular embodiment, Compound 1 can be administered in an amount of about 1 mg/day to patients with MDS. In a particular embodiment, Compound 1 can be administered in an amount of about 3 mg/day to patients with MDS. In a particular embodiment, Compound 1 can be administered in an amount of about 4 mg/day to patients with MDS. In a particular embodiment, Compound 1 provided herein can be administered in an amount of about 5 mg/day to patients with MDS. In a particular embodiment, Compound 1 provided herein can be administered in an amount of about 6 mg/day to patients with MDS. In a particular embodiment, Compound 1 provided herein can be administered in an amount of about 7 mg/day to patients with MDS. In a particular embodiment, Compound 1 provided herein can be administered in an amount of about 10 mg/day to patients with MDS. In a particular embodiment, Compound 1 provided herein can be administered in an amount of about 12 mg/day to patients with MDS. In a particular embodiment, Compound 1 provided herein can be administered in an amount of about 15 mg/day to patients with MDS.

In certain embodiments, the therapeutically or prophylactically effective amount is from about 0.001 to about 20 mg/kg/day, from about 0.01 to about 15 mg/kg/day, from about 0.01 to about 10 mg/kg/day, from about 0.01 to about 9 mg/kg/day, 0.01 to about 8 mg/kg/day, from about 0.01 to about 7 mg/kg/day, from about 0.01 to about 6 mg/kg/day, from about 0.01 to about 5 mg/kg/day, from about 0.01 to about 4 mg/kg/day, from about 0.01 to about 3 mg/kg/day, from about 0.01 to about 2 mg/kg/day, from about 0.01 to about 1 mg/kg/day, or from about 0.01 to about 0.05 mg/kg/day. In certain embodiments, the therapeutically or prophylactically effective amount is from about 0.001 to about 20 mg/kg/day. In certain embodiments, the therapeutically or prophylactically effective amount is from about 0.01 to about 15 mg/kg/day. In certain embodiments, the therapeutically or prophylactically effective amount is from about 0.01 to about 10 mg/kg/day. In certain embodiments, the therapeutically or prophylactically effective amount is from about 0.01 to about 9 mg/kg/day. In certain embodiments, the therapeutically or prophylactically effective amount is 0.01 to about 8 mg/kg/day. In certain embodiments, the therapeutically or prophylactically effective amount is from about 0.01 to about 7 mg/kg/day. In certain embodiments, the therapeutically or prophylactically effective amount is from about 0.01 to about 6 mg/kg/day. In certain embodiments, the therapeutically or prophylactically effective amount is from about 0.01 to about 5 mg/kg/day. In certain embodiments, the therapeutically or prophylactically effective amount is from about 0.01 to about 4 mg/kg/day. In certain embodiments, the therapeutically or prophylactically effective amount is from about 0.01 to about 3 mg/kg/day. In certain embodiments, the therapeutically or prophylactically effective amount is from about 0.01 to about 2 mg/kg/day. In certain embodiments, the therapeutically or prophylactically effective amount is from about 0.01 to about 1 mg/kg/day. In certain embodiments, the therapeutically or prophylactically effective amount is from about 0.01 to about 0.05 mg/kg/day.

The administered dose can also be expressed in units other than mg/kg/day. For example, doses for parenteral administration can be expressed as $mg/m^2/day$. One of ordinary skill in the art would readily know how to convert doses from mg/kg/day to $mg/m^2/day$ to given either the height or weight of a subject or both (*see,* www.fda.gov/cder/cancer/animalframe.htm). For example, a dose of 1 mg/kg/day for a 65 kg human is approximately equal to 38 $mg/m^2/day$.

In certain embodiments, the amount of Compound 1 administered is sufficient to provide a plasma concentration of the compound at steady state, ranging from about 0.001 to about 500 μM, about 0.002 to about 200 μM, about 0.005 to about 100 μM, about 0.01 to about 50 μM, from about 1 to about 50 μM, about 0.02 to about 25 μM, from about 0.05 to about 20 μM, from about 0.1 to about 20 μM, from about 0.5 to about 20 μM, or from about 1 to about 20 μM. In certain embodiments, the amount of Compound 1 administered is sufficient to provide a plasma concentration of the compound at steady state, ranging from about 0.001 to about 500 μM, about 0.002 to about 200 μM, about 0.005 to about 100 μM, about 0.01 to about 50 μM, from about 1 to about 50 μM, about 0.02 to about 25 μM, from about 0.05 to about 20 μM, from about 0.1 to about 20 μM, from about 0.5 to about 20 μM, or from about 1 to about 20 μM.

In other embodiments, the amount of a formulation of Compound 1 administered is sufficient to provide a plasma concentration of the compound at steady state, ranging from about 5 to about 100 nM, about 5 to about 50 nM, about 10 to about 100 nM, about 10 to about 50 nM or from about 50 to about 100 nM. In other embodiments, the amount of a formulation of Compound 1 administered is sufficient to provide a plasma concentration of the compound at steady state, ranging from about 5 to about 100 nM. In other embodiments, the amount of a formulation of Compound 1

administered is sufficient to provide a plasma concentration of the compound at steady state, ranging from about 5 to about 50 nM. In other embodiments, the amount of a formulation of Compound 1 administered is sufficient to provide a plasma concentration of the compound at steady state, ranging from about 10 to about 100 nM. In other embodiments, the amount of a formulation of Compound 1 administered is sufficient to provide a plasma concentration of the compound at steady state, ranging from about 10 to about 50 nM. In other embodiments, the amount of a formulation of Compound 1 administered is sufficient to provide a plasma concentration of the compound at steady state, ranging from about 50 to about 100 nM.

As used herein, the term "plasma concentration at steady state" is the concentration reached after a period of administration of a formulation provided herein. Once steady state is reached, there are minor peaks and troughs on the time dependent curve of the plasma concentration of the solid form.

In certain embodiments, the amount of a formulation of Compound 1 administered is sufficient to provide a maximum plasma concentration (peak concentration) of the compound, ranging from about 0.001 to about 500 μM, about 0.002 to about 200 μM, about 0.005 to about 100 μM, about 0.01 to about 50 μM, from about 1 to about 50 μM, about 0.02 to about 25 μM, from about 0.05 to about 20 μM, from about 0.1 to about 20 μM, from about 0.5 to about 20 μM,or from about 1 to about 20 μM. In certain embodiments, the amount of a formulation of Compound 1 administered is sufficient to provide a maximum plasma concentration (peak concentration) of the compound, ranging from about 0.001 to about 500 μM. In certain embodiments, the amount of a formulation of Compound 1 administered is sufficient to provide a maximum plasma concentration (peak concentration) of the compound, ranging from about 0.002 to about 200 μM. In certain embodiments, the amount of a formulation of Compound 1 administered is sufficient to provide a maximum plasma concentration (peak concentration) of the compound, ranging from about 0.005 to about 100 μM. In certain embodiments, the amount of a formulation of Compound 1 administered is sufficient to provide a maximum plasma concentration (peak concentration) of the compound, ranging from about 0.01 to about 50 μM. In certain embodiments, the amount of a formulation of Compound 1 administered is sufficient to provide a maximum plasma concentration (peak concentration) of the compound, ranging from about 1 to about 50 μM. In certain embodiments, the amount of a formulation of Compound 1 administered is sufficient to provide a maximum plasma concentration (peak concentration) of the compound, ranging from about 0.02 to about 25 μM. In certain embodiments, the amount of a formulation of Compound 1 administered is sufficient to provide a maximum plasma concentration (peak concentration) of the compound, ranging from about 0.05 to about 20 μM. In certain embodiments, the amount of a formulation of Compound 1 administered is sufficient to provide a maximum plasma concentration (peak concentration) of the compound, ranging from about 0.1 to about 20 μM. In certain embodiments, the amount of a formulation of Compound 1 administered is sufficient to provide a maximum plasma concentration (peak concentration) of the compound, ranging from about 0.5 to about 20 μM. In certain embodiments, the amount of a formulation of Compound 1 administered is sufficient to provide a maximum plasma concentration (peak concentration) of the compound, ranging from about 1 to about 20 μM.

In certain embodiments, the amount of a formulation of Compound 1 administered is sufficient to provide a minimum plasma concentration (trough concentration) of the compound, ranging from about 0.001 to about 500 μM, about 0.002 to about 200 μM, about 0.005 to about 100 μM, about 0.01 to about 50 μM, from about 1 to about 50 μM, about 0.01 to about 25 μM, from about 0.01 to about 20 μM, from about 0.02 to about 20 μM, from about 0.02 to about 20 μM, or from about 0.01 to about 20 μM. In certain embodiments, the amount of a formulation of Compound 1 administered is sufficient to provide a minimum plasma concentration (trough concentration) of the compound, ranging from about 0.001 to about 500 μM. In certain embodiments, the amount of a formulation of Compound 1 administered is sufficient to provide a minimum plasma concentration (trough concentration) of the compound, ranging from about 0.002 to about 200 μM. In certain embodiments, the amount of a formulation of Compound 1 administered is sufficient to provide a minimum plasma concentration (trough concentration) of the compound, ranging from about 0.005 to about 100 μM. In certain embodiments, the amount of a formulation of Compound 1 administered is sufficient to provide a minimum plasma concentration (trough concentration) of the compound, ranging from about 0.01 to about 50 μM. In certain embodiments, the amount of a formulation of Compound 1 administered is sufficient to provide a minimum plasma concentration (trough concentration) of the compound, ranging from about 1 to about 50 μM, about 0.01 to about 25 μM. In certain embodiments, the amount of a formulation of Compound 1 administered is sufficient to provide a minimum plasma concentration (trough concentration) of the compound, ranging from about 0.01 to about 20 μM. In certain embodiments, the amount of a formulation of Compound 1 administered is sufficient to provide a minimum plasma concentration (trough concentration) of the compound, ranging from about 0.02 to about 20 μM. In certain embodiments, the amount of a formulation of Compound 1 administered is sufficient to provide a minimum plasma concentration (trough concentration) of the compound, ranging from about 0.02 to about 20 μM. In certain embodiments, the amount of a formulation of Compound 1 administered is sufficient to provide a minimum plasma concentration (trough concentration) of the compound, ranging from about 0.01 to about 20 μM.

In certain embodiments, the amount of a formulation of Compound 1 administered is sufficient to provide an area under the curve (AUC) of the compound, ranging from about 100 to about 100,000 ng*hr/mL, from about 1,000 to

about 50,000 ng*hr/mL, from about 5,000 to about 25,000 ng*hr/mL, or from about 5,000 to about 10,000 ng*hr/mL. In certain embodiments, the amount of a formulation of Compound 1 administered is sufficient to provide an area under the curve (AUC) of the compound, ranging from about 100 to about 100,000 ng*hr/mL. In certain embodiments, the amount of a formulation of Compound 1 administered is sufficient to provide an area under the curve (AUC) of the compound, ranging from about 1,000 to about 50,000 ng*hr/mL. In certain embodiments, the amount of a formulation of Compound 1 administered is sufficient to provide an area under the curve (AUC) of the compound, ranging from about 5,000 to about 25,000 ng*hr/mL. In certain embodiments, the amount of a formulation of Compound 1 administered is sufficient to provide an area under the curve (AUC) of the compound, ranging from about 5,000 to about 10,000 ng*hr/mL.

In certain embodiments, the patient to be treated with one of the methods provided herein has not been treated with anti-cancer therapy prior to the administration of a formulation of Compound 1 provided herein. In certain embodiments, the patient to be treated with one of the methods provided herein has been treated with anti-cancer therapy prior to the administration of a formulation of Compound 1 provided herein. In certain embodiments, the patient to be treated with one of the methods provided herein has developed drug resistance to the anti-cancer therapy.

The formulation of Compound 1 for use in methods provided herein encompass treating a patient regardless of patient's age, although some diseases or disorders are more common in certain age groups.

The formulation of Compound 1 provided herein can be delivered as a single dose such as, *e.g.,* a single bolus injection, or over time, such as, *e.g.,* continuous infusion over time or divided bolus doses over time. The formulation of Compound 1 can be administered repeatedly if necessary, for example, until the patient experiences stable disease or regression, or until the patient experiences disease progression or unacceptable toxicity. For example, stable disease for solid tumors generally means that the perpendicular diameter of measurable lesions has not increased by 25% or more from the last measurement. Response Evaluation Criteria in Solid Tumors (RECIST) Guidelines, Journal of the National Cancer Institute 92(3): 205-216 (2000). Stable disease or lack thereof is determined by methods known in the art such as evaluation of patient symptoms, physical examination, visualization of the tumor that has been imaged using X-ray, CAT, PET, or MRI scan and other commonly accepted evaluation modalities.

The formulation of Compound 1 provided herein can be administered once daily (QD), or divided into multiple daily doses such as twice daily (BID), three times daily (TID), and four times daily (QID). In addition, the administration can be continuous (*i.e.,* daily for consecutive days or every day), intermittent, *e.g.,* in cycles (*i.e.,* including days, weeks, or months of rest without drug). As used herein, the term "daily" is intended to mean that a therapeutic compound is administered once or more than once each day, for example, for a period of time. The term "continuous" is intended to mean that a therapeutic compound is administered daily for an uninterrupted period of at least 10 days to 52 weeks. The term "intermittent" or "intermittently" as used herein is intended to mean stopping and starting at either regular or irregular intervals. For example, intermittent administration of the formulation of Compound 1 is administration for one to six days per week, administration in cycles (*e.g.,* daily administration for one to ten consecutive days of a 28 day cycle, then a rest period with no administration for rest of the 28 day cycle; or daily administration for two to eight consecutive weeks, then a rest period with no administration for up to one week), or administration on alternate days. Cycling therapy with Compound 1 is discussed elsewhere herein.

In some embodiments, the frequency of administration is in the range of about a daily dose to about a monthly dose. In certain embodiments, administration is once a day, twice a day, three times a day, four times a day, once every other day, twice a week, once every week, once every two weeks, once every three weeks, or once every four weeks. In one embodiment, Compound 1 is administered once a day. In another embodiment, Compound 1 is administered twice a day. In yet another embodiment, Compound 1 provided herein is administered three times a day. In still another embodiment, Compound 1 provided herein is administered four times a day. In still another embodiment, Compound 1 provided herein is administered once every other day. In still another embodiment, Compound 1 provided herein is administered twice a week. In still another embodiment, Compound 1 provided herein is administered once every week. In still another embodiment, Compound 1 provided herein is administered once every two weeks. In still another embodiment, Compound 1 provided herein is administered once every three weeks. In still another embodiment, Compound 1 provided herein is administered once every four weeks.

In certain embodiments, a formulation of Compound 1 provided herein is administered once per day from one day to six months, from one week to three months, from one week to four weeks, from one week to three weeks, or from one week to two weeks. In certain embodiments, a formulation of Compound 1 provided herein is administered once per day for one week, two weeks, three weeks, or four weeks. In one embodiment, a formulation of Compound 1 provided herein is administered once per day for 1 day. In one embodiment, a formulation of Compound 1 provided herein is administered once per day for 2 days. In one embodiment, a formulation of Compound 1 provided herein is administered once per day for 3 days. In one embodiment, a formulation of Compound 1 provided herein is administered once per day for 4 days. In one embodiment, a formulation of Compound 1 provided herein is administered once per day for 5 days. In one embodiment, a formulation of Compound 1 provided herein is administered once per day for 6 days. In one embodiment, a formulation of Compound 1 provided herein is administered once per day for one week. In one embodiment, a formulation of Compound 1 provided herein is administered once per day for up to 10 days. In another embodiment, a formulation of Compound 1 provided herein is administered once per day for two weeks. In yet another embodiment, a formulation of Compound 1 provided herein is administered once per day for three weeks. In still another embodiment, a formulation of Compound 1 provided herein is administered once per day for four weeks.

**Combination Therapy** All methods of treatment provided herein are to be understood as a formulation of Compound 1 for use in such methods of treatment.

In one embodiment, provided herein is a method of treating, preventing, and/or managing cancer, comprising administering to a patient Compound 1 in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors, and optionally in combination with radiation therapy, blood transfusions, or surgery. Examples of second active agents are disclosed herein.

In one embodiment, provided herein is a method of treating, preventing, and/or managing cancer, comprising administering to a patient a formulation of Compound 1 provided herein in combination with one or more second active agents, and optionally in combination with radiation therapy, blood transfusions, or surgery. Examples of second active agents are disclosed herein.

As used herein, the term "in combination" includes the use of more than one therapy (*e.g.*, one or more prophylactic and/or therapeutic agents). However, the use of the term "in combination" does not restrict the order in which therapies (*e.g.*, prophylactic and/or therapeutic agents) are administered to a patient with a disease or disorder. E.g., "in combination" may include administration as a mixture, simultaneous administration using separate formulations, and consecutive administration in any order. "Consecutive" means that a specific time has passed between the administration of the active agents. For example, "consecutive" may be that more than 10 minutes have passed between the administration of the separate active agents. The time period can then be more than 10 min, more than 30 minutes, more than 1 hour, more than 3 hours, more than 6 hours or more than 12 hours. E.g., a first therapy *(e.g.,* a prophylactic or therapeutic agent such as a formulation of Compound 1 provided herein) can be administered prior to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy *(e.g.,* a prophylactic or therapeutic agent) to the subject. Triple therapy is also contemplated herein.

In one embodiment, administration of Compound 1, including a formulation of Compound 1 provided herein, and one or more second active agents to a patient can occur simultaneously or sequentially by the same or different routes of administration. In one embodiment, administration of Compound 1, including a formulation of Compound 1 provided herein, and one or more second active agents to a patient can occur simultaneously or sequentially by the same or different routes of administration. The suitability of a particular route of administration employed for a particular active agent will depend on the active agent itself (*e.g.*, whether it can be administered orally without decomposing prior to entering the blood stream) and the cancer being treated.

The route of administration of Compound 1, including a formulation of Compound 1 provided herein, is independent of the route of administration of a second therapy. Thus, in one embodiment, Compound 1, including a formulation of Compound 1 provided herein, is administered intravenously, and the second therapy can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form. In one embodiment, Compound 1, including a formulation of Compound 1 provided herein, and a second therapy are administered by the same mode of administration, by IV. In another embodiment, Compound 1, including a formulation of Compound 1 provided herein, is administered by one mode of administration, e.g., by IV, whereas the second agent (an anti-cancer agent) is administered by another mode of administration, e.g., orally.

In one embodiment, the second active agent is administered intravenously or subcutaneously and once or twice daily in an amount of from about 1 to about 1000 mg, from about 5 to about 500 mg, from about 10 to about 350 mg, or from about 50 to about 200 mg. The specific amount of the second active agent will depend on the specific agent used, the type of disease being treated and/or managed, the severity and stage of disease, and the amount of Compound 1 and any optional additional active agents concurrently administered to the patient.

One or more second active ingredients or agents can be used together with Compound 1 in the methods and compositions provided herein. Second active agents can be large molecules *(e.g.,* proteins) or small molecules *(e.g.,* synthetic inorganic, organometallic, or organic molecules).

Examples of large molecule active agents include, but are not limited to, hematopoietic growth factors, cytokines, and monoclonal and polyclonal antibodies, particularly, therapeutic antibodies to cancer antigens. Typical large molecule active agents are biological molecules, such as naturally occurring or synthetic or recombinant proteins. Proteins that

are particularly useful in the methods and compositions provided herein include proteins that stimulate the survival and/or proliferation of hematopoietic precursor cells and immunologically active poietic cells *in vitro* or *in vivo.* Other useful proteins stimulate the division and differentiation of committed erythroid progenitors in cells *in vitro* or *in vivo.* Particular proteins include, but are not limited to: interleukins, such as IL-2 (including recombinant IL-II ("rIL2") and canarypox IL-2), IL-10, IL-12, and IL-18; interferons, such as interferon alfa-2a, interferon alfa-2b, interferon alfa-nl, interferon alfa-n3, interferon beta-I a, and interferon gamma-I b; GM-CF and GM-CSF; and EPO.

In certain embodiments, GM-CSF, G-CSF, SCF or EPO is administered subcutaneously during about five days in a four or six week cycle in an amount ranging from about 1 to about 750 mg/m$^2$/day, from about 25 to about 500 mg/m$^2$/day, from about 50 to about 250 mg/m$^2$/day, or from about 50 to about 200 mg/m$^2$/day. In certain embodiments, GM-CSF may be administered in an amount of from about 60 to about 500 mcg/m$^2$ intravenously over 2 hours or from about 5 to about 12 mcg/m$^2$/day subcutaneously. In certain embodiments, G-CSF may be administered subcutaneously in an amount of about 1 mcg/kg/day initially and can be adjusted depending on rise of total granulocyte counts. The maintenance dose of G-CSF may be administered in an amount of about 300 (in smaller patients) or 480 mcg subcutaneously. In certain embodiments, EPO may be administered subcutaneously in an amount of 10,000 Unit 3 times per week.

Particular proteins that can be used in the methods and compositions include, but are not limited to: filgrastim, which is sold in the United States under the trade name Neupogen® (Amgen, Thousand Oaks, CA); sargramostim, which is sold in the United States under the trade name Leukine® (Immunex, Seattle, WA); and recombinant EPO, which is sold in the United States under the trade name Epogen® (Amgen, Thousand Oaks, CA).

Recombinant and mutated forms of GM-CSF can be prepared as described in U.S. patent nos. 5,391,485; 5,393,870; and 5,229,496. Recombinant and mutated forms of G-CSF can be prepared as described in U.S. patent nos. 4,810,643; 4,999,291; 5,528,823; and 5,580,755.

Also provided for use in combination with Compound 1, including a formulation of Compound 1, are native, naturally occurring, and recombinant proteins. Further encompassed are mutants and derivatives (*e.g.*, modified forms) of naturally occurring proteins that exhibit, *in vivo,* at least some of the pharmacological activity of the proteins upon which they are based. Examples of mutants include, but are not limited to, proteins that have one or more amino acid residues that differ from the corresponding residues in the naturally occurring forms of the proteins. Also encompassed by the term "mutants" are proteins that lack carbohydrate moieties normally present in their naturally occurring forms (*e.g.*, nonglycosylated forms). Examples of derivatives include, but are not limited to, pegylated derivatives and fusion proteins, such as proteins formed by fusing IgG1 or IgG3 to the protein or active portion of the protein of interest. *See, e.g.,* Penichet, M.L. and Morrison, S.L., J. Immunol. Methods 248:91-101 (2001).

Antibodies that can be used in combination with Compound 1, including a formulation of Compound 1 provided herein, include monoclonal and polyclonal antibodies. Examples of antibodies include, but are not limited to, trastuzumab (Herceptin®), rituximab (Rituxan®), bevacizumab (Avastin™), pertuzumab (Omnitarg™), tositumomab (Bexxar®), edrecolomab (Panorex®), and G250. The formulation of Compound 1 can also be combined with, or used in combination with, anti-TNF-a antibodies, and/or anti-EGFR antibodies, such as, for example, Erbitux® or panitumumab.

Large molecule active agents may be administered in the form of anti-cancer vaccines. For example, vaccines that secrete, or cause the secretion of, cytokines such as IL-2, G-CSF, and GM-CSF can be used in the methods and pharmaceutical compositions provided. *See, e.g.,* Emens, L.A., et al., Curr. Opinion Mol. Ther. 3(1):77-84 (2001).

Second active agents that are small molecules can also be used to alleviate adverse effects associated with the administration of a formulation of Compound 1 provided herein. However, like some large molecules, many are believed to be capable of providing a synergistic effect when administered with (*e.g.*, before, after, or simultaneously) Compound 1, including a formulation of Compound 1 provided herein. Examples of small molecule second active agents include, but are not limited to, anti-cancer agents, antibiotics, immunosuppressive agents, and steroids.

In certain embodiments, the second agent is an HSP inhibitor, a proteasome inhibitor, a FLT3 inhibitior or an mTOR inhibitor. In some embodiments, the mTOR inhibitor is a mTOR kinase inhibitor.

Examples of anti-cancer agents to be used within the methods or compositions described herein include, but are not limited to: acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); chlorambucil; cirolemycin; cisplatin; cladribine; clofarabine; crisnatol mesylate; cyclophosphamide; Ara-C; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone;

fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; omacetaxine; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sorafenib; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs to be included within the methods herein include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; Ara-C ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (*e.g.,* Gleevec®); imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (Genasense®); $O^6$-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosane polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer.

In certain embodiments, the second agent is selected from one or more checkpoint inhibitors. In one embodiment, one checkpoint inhibitor is used in combination with Compound 1 or a formulation of Compound 1 in the methods provided herein. In another embodiment, two checkpoint inhibitors are used in combination with Compound 1 or a formulation of Compound 1 in connection with the methods provided herein. In yet another embodiment, three or more checkpoint inhibitors are used in combination with Compound 1 or a formulation of Compound 1 in connection with the methods provided herein.

As used herein, the term "immune checkpoint inhibitor" or "checkpoint inhibitor" refers to molecules that totally or partially reduce, inhibit, interfere with or modulate one or more checkpoint proteins. Without being limited by a particular theory, checkpoint proteins regulate T-cell activation or function. Numerous checkpoint proteins are known, such as CTLA-4 and its ligands CD80 and CD86; and PD-1 with its ligands PD-L1 and PD-L2 (Pardoll, Nature Reviews Cancer, 2012, 12, 252-264). These proteins appear responsible for co-stimulatory or inhibitory interactions of T-cell responses. Immune checkpoint proteins appear to regulate and maintain self-tolerance and the duration and amplitude of physiological immune responses. Immune checkpoint inhibitors include antibodies or are derived from antibodies.

In one embodiment, the checkpoint inhibitor is a CTLA-4 inhibitor. In one embodiment, the CTLA-4 inhibitor is an anti-CTLA-4 antibody. Examples of anti-CTLA-4 antibodies include, but are not limited to, those described in US Patent Nos: 5,811,097; 5,811,097; 5,855,887; 6,051,227; 6,207,157; 6,682,736; 6,984,720; and 7,605,238, all of which are incorporated herein in their entireties. In one embodiment, the anti-CTLA-4 antibody is tremelimumab (also known as ticilimumab or CP-675,206). In another embodiment, the anti-CTLA-4 antibody is ipilimumab (also known as MDX-010 or MDX-101). Ipilimumab is a fully human monoclonal IgG antibody that binds to CTLA-4. Ipilimumab is marketed under the trade name Yervoy™.

In one embodiment, the checkpoint inhibitor is a PD-1/PD-L1 inhibitor. Examples of PD-1/PD-L1 inhibitors include, but are not limited to, those described in US Patent Nos. 7,488,802; 7,943,743; 8,008,449; 8,168,757; 8,217,149, and PCT Patent Application Publication Nos. WO2003042402, WO2008156712, WO2010089411, WO2010036959, WO2011066342, WO2011159877, WO2011082400, and WO2011161699.

In one embodiment, the checkpoint inhibitor is a PD-1 inhibitor. In one embodiment, the PD-1 inhibitor is an anti-PD-1 antibody. In one embodiment, the anti-PD-1 antibody is BGB-A317, nivolumab (also known as ONO-4538, BMS-936558, or MDX1106) or pembrolizumab (also known as MK-3475, SCH 900475, or lambrolizumab). In one embodiment, the anti-PD-1 antibody is nivolumab. Nivolumab is a human IgG4 anti-PD-1 monoclonal antibody, and is marketed under the trade name Opdivo™. In another embodiment, the anti-PD-1 antibody is pembrolizumab. Pembrolizumab is a humanized monoclonal IgG4 antibody and is marketed under the trade name Keytruda™. In yet another embodiment, the anti-PD-1 antibody is CT-011, a humanized antibody. CT-011 administered alone has failed to show response in treating acute myeloid leukemia (AML) at relapse. In yet another embodiment, the anti-PD-1 antibody is AMP-224, a fusion protein. In another embodiment, the PD-1 antibody is BGB-A317. BGB-A317 is a monoclonal antibody in which the ability to bind Fc gamma receptor I is specifically engineered out, and which has a unique binding signature to PD-1 with high affinity and superior target specificity.

In one embodiment, the checkpoint inhibitor is a PD-L1 inhibitor. In one embodiment, the PD-L1 inhibitor is an anti-PD-LI antibody. In one embodiment, the anti-PD-LI antibody is MEDI4736 (durvalumab). In another embodiment, the anti-PD-LI antibody is BMS-936559 (also known as MDX-1105-01). In yet another embodiment, the PD-L1 inhibitor is atezolizumab (also known as MPDL3280A, and Tecentriq®).

In one embodiment, the checkpoint inhibitor is a PD-L2 inhibitor. In one embodiment, the PD-L2 inhibitor is an

anti-PD-L2 antibody. In one embodiment, the anti-PD-L2 antibody is rHIgM12B7A.

In one embodiment, the checkpoint inhibitor is a lymphocyte activation gene-3 (LAG-3) inhibitor. In one embodiment, the LAG-3 inhibitor is IMP321, a soluble Ig fusion protein (Brignone et al., J. Immunol., 2007, 179, 4202-4211). In another embodiment, the LAG-3 inhibitor is BMS-986016.

In one embodiment, the checkpoint inhibitor is a B7 inhibitor. In one embodiment, the B7 inhibitor is a B7-H3 inhibitor or a B7-H4 inhibitor. In one embodiment, the B7-H3 inhibitor is MGA271, an anti-B7-H3 antibody (Loo et al., Clin. Cancer Res., 2012, 3834).

In one embodiment, the checkpoint inhibitor is a TIM3 (T-cell immunoglobulin domain and mucin domain 3) inhibitor (Fourcade et al., J. Exp. Med., 2010, 207, 2175-86; Sakuishi et al., J. Exp. Med., 2010, 207, 2187-94).

In one embodiment, the checkpoint inhibitor is an OX40 (CD134) agonist. In one embodiment, the checkpoint inhibitor is an anti-OX40 antibody. In one embodiment, the anti-OX40 antibody is anti-OX-40. In another embodiment, the anti-OX40 antibody is MEDI6469.

In one embodiment, the checkpoint inhibitor is a GITR agonist. In one embodiment, the checkpoint inhibitor is an anti-GITR antibody. In one embodiment, the anti-GITR antibody is TRX518.

In one embodiment, the checkpoint inhibitor is a CD137 agonist. In one embodiment, the checkpoint inhibitor is an anti-CD137 antibody. In one embodiment, the anti-CD137 antibody is urelumab. In another embodiment, the anti-CD137 antibody is PF-05082566.

In one embodiment, the checkpoint inhibitor is a CD40 agonist. In one embodiment, the checkpoint inhibitor is an anti-CD40 antibody. In one embodiment, the anti-CD40 antibody is CF-870,893.

In one embodiment, the checkpoint inhibitor is recombinant human interleukin-15 (rhIL-15).

In one embodiment, the checkpoint inhibitor is an IDO inhibitor. In one embodiment, the IDO inhibitor is INCB024360. In another embodiment, the IDO inhibitor is indoximod.

In certain embodiments, the combination therapies provided herein include two or more of the checkpoint inhibitors described herein (including checkpoint inhibitors of the same or different class). Moreover, the combination therapies described herein can be used in combination with second active agents as described herein where appropriate for treating diseases described herein and understood in the art.

In certain embodiments, Compound 1 can be used in combination with one or more immune cells expressing one or more chimeric antigen receptors (CARs) on their surface (e.g., a modified immune cell). Generally, CARs comprise an extracellular domain from a first protein e.g., an antigen-binding protein), a transmembrane domain, and an intracellular signaling domain. In certain embodiments, once the extracellular domain binds to a target protein such as a tumor-associated antigen (TAA) or tumor-specific antigen (TSA), a signal is generated via the intracellular signaling domain that activates the immune cell, e.g., to target and kill a cell expressing the target protein.

Extracellular domains: The extracellular domains of the CARs bind to an antigen of interest. In certain embodiments, the extracellular domain of the CAR comprises a receptor, or a portion of a receptor, that binds to said antigen. In certain embodiments, the extracellular domain comprises, or is, an antibody or an antigen-binding portion thereof. In specific embodiments, the extracellular domain comprises, or is, a single chain Fv (scFv) domain. The single-chain Fv domain can comprise, for example, a $V_L$ linked to $V_H$ by a flexible linker, wherein said $V_L$ and $V_H$ are from an antibody that binds said antigen.

In certain embodiments, the antigen recognized by the extracellular domain of a polypeptide described herein is a tumor-associated antigen (TAA) or a tumor-specific antigen (TSA). In various specific embodiments, the tumor-associated antigen or tumor-specific antigen is, without limitation, Her2, prostate stem cell antigen (PSCA), alpha-fetoprotein (AFP), carcinoembryonic antigen (CEA), cancer antigen-125 (CA-125), CA19-9, calretinin, MUC-1, B cell maturation antigen (BCMA), epithelial membrane protein (EMA), epithelial tumor antigen (ETA), tyrosinase, melanoma-24 associated antigen (MAGE), CD19, CD22, CD27, CD30, CD34, CD45, CD70, CD99, CD117, EGFRvIII (epidermal growth factor variant III), mesothelin, PAP (prostatic acid phosphatase), prostein, TARP (T cell receptor gamma alternate reading frame protein), Trp-p8, STEAPI (six-transmembrane epithelial antigen of the prostate 1), chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-I), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, or an abnormal p53 protein. In certain other embodiments, the TAA or TSA recognized by the extracellular domain of a CAR is integrin $\alpha v\beta 3$ (CD61), galactin, or Ral-B.

In certain embodiments, the TAA or TSA recognized by the extracellular domain of a CAR is a cancer/testis (CT) antigen, e.g., BAGE, CAGE, CTAGE, FATE, GAGE, HCA661, HOM-TES-85, MAGEA, MAGEB, MAGEC, NA88, NY-ES0-1, NY-SAR-35, OY-TES-1, SPANXBI, SPA17, SSX, SYCPI, or TPTE.

In certain other embodiments, the TAA or TSA recognized by the extracellular domain of a CAR is a carbohydrate or ganglioside, e.g., fuc-GMI, GM2 (oncofetal antigen-immunogenic-1; OFA-I-1); GD2 (OFA-I-2), GM3, GD3, and the like.

In certain other embodiments, the TAA or TSA recognized by the extracellular domain of a CAR is alpha-actinin-4, Bage-l, BCR-ABL, Bcr-Abl fusion protein, beta-catenin, CA 125, CA 15-3 (CA 27.29\BCAA), CA 195, CA 242, CA-50, CAM43, Casp-8, cdc27, cdk4, cdkn2a, CEA, coa-l, dek-can fusion protein, EBNA, EF2, Epstein Barr virus antigens, ETV6-AML1 fusion protein, HLA-A2, HLA-All, hsp70-2, KIAA0205, Mart2, Mum-1, 2, and 3, neo-PAP, myosin class I, OS-9, pml-RARα fusion protein, PTPRK, K-ras, N-ras, triosephosphate isomerase, Gage 3,4,5,6,7, GnTV, Herv-K-mel, Lage-1, NA-88, NY-Eso-1/Lage-2, SP17, SSX-2, TRP2-Int2, gp100 (Pmel17), tyrosinase, TRP-1, TRP-2, MAGE-1, MAGE-3, RAGE, GAGE-1, GAGE-2, p15(58), RAGE, SCP-1, Hom/Mel-40, PRAME, p53, HRas, HER-2/neu, E2A-PRL, H4-RET, IGH-IGK, MYL-RAR, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, 13-Catenin, Mum-1, p16, TAGE, PSMA, CT7, telomerase, 43-9F, 5T4, 791Tgp72, 13HCG, BCA225, BTAA, CD68\KP1, C0-029, FGF-5, G250, Ga733 (EpCAM), HTgp-175, M344, MA-50, MG7-Ag, MOV18, NB\70K, NY-C0-1, RCAS1, SDCCAG16, TA-90, TAAL6, TAG72, TLP, or TPS.

In various specific embodiments, the tumor-associated antigen or tumor-specific antigen is an AML-related tumor antigen, as described in S. Anguille et al, Leukemia (2012), 26, 2186-2196.

Other tumor-associated and tumor-specific antigens are known to those in the art.

Receptors, antibodies, and scFvs that bind to TSAs and TAAs, useful in constructing chimeric antigen receptors, are known in the art, as are nucleotide sequences that encode them.

In certain specific embodiments, the antigen recognized by the extracellular domain of a chimeric antigen receptor is an antigen not generally considered to be a TSA or a TAA, but which is nevertheless associated with tumor cells, or damage caused by a tumor. In certain embodiments, for example, the antigen is, e.g., a growth factor, cytokine or interleukin, e.g., a growth factor, cytokine, or interleukin associated with angiogenesis or vasculogenesis. Such growth factors, cytokines, or interleukins can include, e.g., vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8). Tumors can also create a hypoxic environment local to the tumor. As such, in other specific embodiments, the antigen is a hypoxia-associated factor, e.g., HIF-1α, HIF-1β, HIF-2α, HIF-2β, HIF-3α, or HIP-3β. Tumors can also cause localized damage to normal tissue, causing the release of molecules known as damage associated molecular pattern molecules (DAMPs; also known as alarmins). In certain other specific embodiments, therefore, the antigen is a DAMP, e.g., a heat shock protein, chromatin-associated protein high mobility group box 1 (HMGB 1), S100A8 (MRP8, calgranulin A), S100A9 (MRP14, calgranulin B), serum amyloid A (SAA), or can be a deoxyribonucleic acid, adenosine triphosphate, uric acid, or heparin sulfate.

Transmembrane domain: In certain embodiments, the extracellular domain of the CAR is joined to the transmembrane domain of the polypeptide by a linker, spacer or hinge polypeptide sequence, e.g., a sequence from CD28 or a sequence from CTLA4. The transmembrane domain can be obtained or derived from the transmembrane domain of any transmembrane protein, and can include all or a portion of such transmembrane domain. In specific embodiments, the transmembrane domain can be obtained or derived from, e.g., CD8, CD 16, a cytokine receptor, and interleukin receptor, or a growth factor receptor, or the like.

Intracellular signaling domains: In certain embodiments, the intracellular domain of a CAR is or comprises an intracellular domain or motif of a protein that is expressed on the surface of T cells and triggers activation and/or proliferation of said T cells. Such a domain or motif is able to transmit a primary antigen-binding signal that is necessary for the activation of a T lymphocyte in response to the antigen's binding to the CAR's extracellular portion. Typically, this domain or motif comprises, or is, an ITAM (immunoreceptor tyrosine-based activation motif). ITAM-containing polypeptides suitable for CARs include, for example, the zeta CD3 chain (CD3ζ) or ITAM-containing portions thereof. In a specific embodiment, the intracellular domain is a CD3ζ intracellular signaling domain. In other specific embodiments, the intracellular domain is from a lymphocyte receptor chain, a TCR/CD3 complex protein, an Fe receptor subunit or an IL-2 receptor subunit. In certain embodiments, the CAR additionally comprises one or more co-stimulatory domains or motifs, e.g., as part of the intracellular domain of the polypeptide. The one or more co-stimulatory domains or motifs can be, or can comprise comprise, one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence, or other costimulatory domain or motif, or any combination thereof.

The CAR may also comprise a T cell survival motif. The T cell survival motif can be any polypeptide sequence or motif that facilitates the survival of the T lymphocyte after stimulation by an antigen. In certain embodiments, the T cell survival motif is, or is derived from, CD3, CD28, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.

The modified immune cells expressing the CARs can be, e.g., T lymphocytes (T cells, e.g., CD4+ T cells or CD8+ T cells), cytotoxic lymphocytes (CTLs) or natural killer (NK) cells. T lymphocytes used in the compositions and methods provided herein may be naive T lymphocytes or MHC-restricted T lymphocytes. In certain embodiments, the T lymphocytes are tumor infiltrating lymphocytes (TILs). In certain embodiments, the T lymphocytes have been isolated

from a tumor biopsy, or have been expanded from T lymphocytes isolated from a tumor biopsy. In certain other embodiments, the T cells have been isolated from, or are expanded from T lymphocytes isolated from, peripheral blood, cord blood, or lymph. Immune cells to be used to generate modified immune cells expressing a CAR can be isolated using art-accepted, routine methods, e.g., blood collection followed by apheresis and optionally antibody-mediated cell isolation or sorting.

The modified immune cells are preferably autologous to an individual to whom the modified immune cells are to be administered. In certain other embodiments, the modified immune cells are allogeneic to an individual to whom the modified immune cells are to be administered. Where allogeneic T lymphocytes or NK cells are used to prepare modified T lymphocytes, it is preferable to select T lymphocytes or NK cells that will reduce the possibility of graft-versus-host disease (GVHD) in the individual. For example, in certain embodiments, virus-specific T lymphocytes are selected for preparation of modified T lymphocytes; such lymphocytes will be expected to have a greatly reduced native capacity to bind to, and thus become activated by, any recipient antigens. In certain embodiments, recipient-mediated rejection of allogeneic T lymphocytes can be reduced by co-administration to the host of one or more immunosuppressive agents, e.g., cyclosporine, tacrolimus, sirolimus, cyclophosphamide, or the like.

T lymphocytes, e.g., unmodified T lymphocytes, or T lymphocytes expressing CD3 and CD28, or comprising a polypeptide comprising a CD3ζ signaling domain and a CD28 co-stimulatory domain, can be expanded using antibodies to CD3 and CD28, e.g., antibodies attached to beads; see, e.g., U.S. Patent Nos. 5,948,893; 6,534,055; 6,352,694; 6,692,964; 6,887,466; and 6,905,681.

The modified immune cells, e.g., modified T lymphocytes, can optionally comprise a "suicide gene" or "safety switch" that enables killing of substantially all of the modified immune cells when desired. For example, the modified T lymphocytes, in certain embodiments, can comprise an HSV thymidine kinase gene (HSV-TK), which causes death of the modified T lymphocytes upon contact with gancyclovir. In another embodiment, the modified T lymphocytes comprise an inducible caspase, e.g., an inducible caspase 9 (icaspase9), e.g., a fusion protein between caspase 9 and human FK506 binding protein allowing for dimerization using a specific small molecule pharmaceutical. See Straathof et al., Blood 105(11):4247-4254 (2005).

Specific second active agents useful in the methods or compositions include, but are not limited to, rituximab, oblimersen (Genasense®), remicade, docetaxel, celecoxib, melphalan, dexamethasone (Decadron®), steroids, gemcitabine, cisplatinum, temozolomide, etoposide, cyclophosphamide, temodar, carboplatin, procarbazine, gliadel, tamoxifen, topotecan, methotrexate, Arisa®, taxol, taxotere, fluorouracil, leucovorin, irinotecan, xeloda, interferon alpha, pegylated interferon alpha *(e.g.,* PEG INTRON-A), capecitabine, cisplatin, thiotepa, fludarabine, carboplatin, liposomal daunorubicin, Ara-C, doxetaxol, pacilitaxel, vinblastine, IL-2, GM-CSF, dacarbazine, vinorelbine, zoledronic acid, palmitronate, biaxin, busulphan, prednisone, bisphosphonate, arsenic trioxide, vincristine, doxorubicin (Doxil®), paclitaxel, ganciclovir, adriamycin, estramustine sodium phosphate (Emcyt®), sulindac, and etoposide.

In certain embodiments of the methods provided herein, use of a second active agent in combination with Compound 1, including a formulation of Compound 1 provided herein, may be modified or delayed during or shortly following administration of Compound 1, including a formulation of Compound 1 provided herein, as deemed appropriate by the practitioner of skill in the art. In certain embodiments, subjects being administered Compound 1, including a formulation of Compound 1 provided herein, alone or in combination with other therapies may receive supportive care including antiemetics, myeloid growth factors, and transfusions of platelets, when appropriate. In some embodiments, subjects being administered Compound 1, including a formulation of Compound 1 provided herein, may be administered a growth factor as a second active agent according to the judgment of the practitioner of skill in the art. In some embodiments, provided is administration of Compound 1, including a formulation of Compound 1 provided herein, in combination with erythropoietin or darbepoetin (Aranesp).

In one aspect, provided herein is a method of treating, preventing, managing, and/or ameliorating locally advanced or metastatic transitional cell bladder cancer comprising administering a formulation of Compound 1 with gemcitabine, cisplatinum, 5-fluorouracil, mitomycin, methotrexate, vinblastine, doxorubicin, carboplatin, thiotepa, paclitaxel, docetaxel, atezolizumab, avelumab, durvalumab, keytruda (pembrolizumab) and/or nivolumab.

In one aspect, methods of treating, preventing, managing, and/or ameliorating a cancer provided herein comprise administering a formulation of Compound 1 in combination with a second active ingredient as follows: temozolomide to pediatric patients with relapsed or progressive brain tumors or recurrent neuroblastoma; celecoxib, etoposide and cyclophosphamide for relapsed or progressive CNS cancer; temodar to patients with recurrent or progressive meningioma, malignant meningioma, hemangiopericytoma, multiple brain metastases, relapsed brain tumors, or newly diagnosed glioblastoma multiforms; irinotecan to patients with recurrent glioblastoma; carboplatin to pediatric patients with brain stem glioma; procarbazine to pediatric patients with progressive malignant gliomas; cyclophosphamide to patients with poor prognosis malignant brain tumors, newly diagnosed or recurrent glioblastoma multiforms; Gliadel® for high grade recurrent malignant gliomas; temozolomide and tamoxifen for anaplastic astrocytoma; or topotecan for gliomas, glioblastoma, anaplastic astrocytoma or anaplastic oligodendroglioma.

In one aspect, methods of treating, preventing, managing, and/or ameliorating a metastatic breast cancer

provided herein comprise administering a formulation of Compound 1 with methotrexate, cyclophosphamide, capecitabine, 5-fluorouracil, taxane, temsirolimus, ABRAXANE® (paclitaxel protein-bound particles for injectable suspension) (albumin-bound), lapatinib, herceptin, pamidronate disodium, eribulin mesylate, everolimus, gemcitabine, palbociclib, ixabepilone, kadcyla, pertuzumab, theotepa, anastrozole, docetaxel, doxorubicin hydrochloride, epirubicin hydrochloride, toremifene, fulvestrant, goserelin acetate, ribociclib, megestrol acetate, vinblastin, aromatase inhibitors, such as letrozole, exemestane, selective estrogen modulators, estrogen receptor antagonists, anthracyclines, emtansine, and/or pexidartinib to patients with metastatic breast cancer.

In one aspect, methods of treating, preventing, managing, and/or ameliorating neuroendocrine tumors provided herein comprise administering a formulation of Compound 1 with at least one of everolimus, avelumab, sunitinib, nexavar, leucovorin, oxaliplatin, temozolomide, capecitabine, bevacizumab, doxorubicin (Adriamycin), fluorouracil (Adrucil, 5-fluorouracil), streptozocin (Zanosar), dacarbazine, sandostatin, lanreotide, and/or pasireotide to patients with neuroendocrine tumors.

In one aspect, methods of treating, preventing, managing, and/or ameliorating a metastatic breast cancer provided herein comprise administering a formulation of Compound 1 with methotrexate, gemcitabine, cisplatin, cetuximab, 5-fluorouracil, bleomycin, docetaxel, carboplatin, hydroxyurea, pembrolizumab and/or nivolumab to patients with recurrent or metastatic head or neck cancer.

In one aspect, methods of treating, preventing, managing, and/or ameliorating a pancreatic cancer provided herein comprise administering a formulation of Compound 1 with gemcitabine, ABRAXANE®, 5-fluorouracil, afinitor, irinotecan, mitomycin C, sunitinib, sunitinibmalate, and/or tarceva to patients with pancreatic cancer.

In one aspect, methods of treating, preventing, managing, and/or ameliorating a colon or rectal cancer provided herein comprise administering a formulation of Compound 1 with ARISA®, avastatin, oxaliplatin, 5-fluorouracil, irinotecan, capecitabine, cetuximab, ramucirumab, panitumumab, bevacizumab, leucovorin calcium, lonsurf, regorafenib, ziv-aflibercept, taxol, and/or taxotere.

In one aspect, methods of treating, preventing, managing, and/or ameliorating a refractory colorectal cancer provided herein comprise administering a formulation of Compound 1 with capecitabine and/or vemurafenib to patients with refractory colorectal cancer, or patients who fail first line therapy or have poor performance in colon or rectal adenocarcinoma.

In one aspect, methods of treating, preventing, managing, and/or ameliorating a colorectal cancer provided herein comprise administering a formulation of Compound 1 with fluorouracil, leucovorin, and/or irinotecan to patients with colorectal cancer, including stage 3 and stage 4, or to patients who have been previously treated for metastatic colorectal cancer.

In certain embodiments, a formulation of Compound 1 provided herein is administered to patients with refractory colorectal cancer in combination with capecitabine, xeloda, and/or irinotecan.

In certain embodiments, a formulation of Compound 1 provided herein is administered with capecitabine and irinotecan to patients with refractory colorectal cancer or to patients with unresectable or metastatic colorectal carcinoma.

In one aspect, the methods provided herein comprise administering a formulation of Compound 1with interferon alpha or capecitabine to patients with unresectable or metastatic hepatocellular carcinoma; or with cisplatin and thiotepa, or with sorafenib tosylate to patients with primary or metastatic liver cancer.

In one aspect, the methods provided herein comprise administering a formulation of Compound 1 with doxorubicin, paclitaxel, vinblastine, pegylated interferon alpha and/or recombinant interferon alpha-2b to patients with Kaposi's sarcoma.

In one aspect, the methods provided herein comprise administering a formulation of Compound 1 with at least one of enasidenib, arsenic trioxide, fludarabine, carboplatin, daunorubicin, cyclophosphamide, cytarabine, doxorubicin, idarubicin, mitoxantrone hydrochloride, thioguanine, vincristine, midostaurin and/or topotecan to patients with acute myeloid leukemia, including refractory or relapsed or high-risk acute myeloid leukemia.

In one aspect, the methods provided herein comprise administering a formulation of Compound 1 with at least one of enasidenib, liposomal daunorubicin, topotecan and/or cytarabine to patients with unfavorable karyotype acute myeloblastic leukemia.

In one aspect, the methods provided herein comprise administering Compound 1 with an IDH2 inhibitor to a patient having leukemia, wherein the leukemia is characterized by the presence of a mutant allele of IDH2. Exemplary IDH2 inhibitors are disclosed in US Patent Nos. 9,732,062; 9,724,350; 9,738,625; and 9,579,324; and US Publication Nos. 2016-0159771and US 2016-0158230 A1. In one aspect, the methods provided herein comprise administering Compound 1 with enasidenib to a patient having leukemia, wherein the leukemia is characterized by the presence of a mutant allele of IDH2. In certain embodiments, the combination of Compound 1 and an IDH2 inhibitor increases differentiated cells (CD34-/CD38) and erythroblasts in a patient having acute myeloid leukemia, wherein the acute myeloid leukemia is characterized by the presence of IDH2 R140Q. In certain embodiments, the combination of Compound 1 and an IDH2 inhibitor reduces progenitor cells (CD34+/CD38+) and HSC in a patient having acute myeloid leukemia, wherein the acute myeloid leukemia is characterized by the presence of IDH2 R140Q.

In one aspect, the methods provided herein comprise administering Compound 1 with enasidenib to a patient having acute myeloid leukemia, wherein the acute myeloid leukemia is characterized by the presence of a mutant allele of IDH2. In one embodiment, the mutant allele of IDH2 is IDH2 R140Q or R172K.

In one aspect, the methods provided herein comprise administering a formulation of Compound 1 with enasidenib to a patient having leukemia, wherein the leukemia is characterized by the presence of a mutant allele of IDH2. In one aspect, the methods provided herein comprise administering a formulation of Compound 1 with enasidenib to a patient having acute myeloid leukemia, wherein the acute myeloid leukemia is characterized by the presence of a mutant allele of IDH2. In one embodiment, the mutant allele of IDH2 is IDH2 R140Q or R172K.

In one aspect, the methods provided herein comprise administering Compound 1 with 6-(6-(trifluoromethyl)pyridin-2-yl)-$N^2$-(2-(trifluoromethyl)pyridin-4-yl)-1,3,5-triazine-2,4-diamine (Compound 2) to a patient having leukemia, wherein the leukemia is characterized by the presence of a mutant allele of IDH2. In one aspect, the methods provided herein comprise administering Compound 1 with Compound 2 to a patient having acute myeloid leukemia, wherein the acute myeloid leukemia is characterized by the presence of a mutant allele of IDH2. In one embodiment, the mutant allele of IDH2 is IDH2 R140Q or R172K.

In one aspect, the methods provided herein comprise administering a formulation of Compound 1 with Compound 2 to a patient having leukemia, wherein the leukemia is characterized by the presence of a mutant allele of IDH2. In one aspect, the methods provided herein comprise administering a formulation of Compound 1 with Compound 2 to a patient having acute myeloid leukemia, wherein the acute myeloid leukemia is characterized by the presence of a mutant allele of IDH2. In one embodiment, the mutant allele of IDH2 is IDH2 R140Q or R172K.

In one aspect, the methods provided herein comprise administering a formulation of Compound 1 with methotrexate, mechlorethamine hydrochloride, afatinib dimaleate, pemetrexed, bevacizumab, carboplatin, cisplatin, ceritinib, crizotinib, ramucirumab, pembrolizumab, docetaxel, vinorelbine tartrate, gemcitabine, ABRAXANE®, erlotinib, geftinib, irinotecan, everolimus, alectinib, brigatinib, nivolumab, osimertinib, atezolizumab, necitumumab and/or to patients with non-small cell lung cancer.

In one aspect, the methods provided herein comprise administering a formulation of Compound 1with carboplatin and irinotecan to patients with non-small cell lung cancer.

In one aspect, the methods provided herein comprise administering a formulation of Compound 1 with doxetaxol to patients with non-small cell lung cancer who have been previously treated with carbo/etoposide and radiotherapy.

In one aspect, the methods provided herein comprise administering a formulation of Compound 1 with carboplatin and/or taxotere, or in combination with carboplatin, pacilitaxel and/or thoracic radiotherapy to patients with non-small cell lung cancer.

In one aspect, the methods provided herein comprise administering a formulation of Compound 1 with taxotere to patients with stage IIIB or IV non-small cell lung cancer.

In one aspect, the methods provided herein comprise administering a formulation of Compound 1 with oblimersen (Genasense®), methotrexate, mechlorethamine hydrochloride, etoposide, topotecan and/or doxorubicin to patients with small cell lung cancer.

In one aspect, the methods provided herein comprise administering a formulation of Compound 1 with Venetoclax, ABT-737 (Abbott Laboratories) and/or obatoclax (GX15-070) to patients with lymphoma and other blood cancers.

In one aspect, the methods provided herein comprise administering a formulation of Compound 1 with a second active ingredient such as vinblastine or fludarabine adcetris, ambochlorin, becenum, bleomycin, brentuximab vedotin, carmustinem chlorambucil, cyclophosphamide, dacarbazine, doxorubicin, lomustine, matulane, mechlorethamine hydrochloride, prednisone, procarbazine hydrochloride, vincristine, methotrexate, nelarabin, belinostat, bendamustine HCl, tositumomab, and iodine 131 tositumomab, denileukin diftitox, dexamethasone, pralatrexate, prelixafor, obinutuzumab, ibritumomab, tiuxefan, ibritinib, idelasib, intron A, romidepsin, lenalidomide, rituximab, and/or vorinostat to patients with various types of lymphoma, including, but not limited to, Hodgkin's lymphoma, non-Hodgkin's lymphoma, cutaneous T-Cell lymphoma, cutaneous B-Cell lymphoma, diffuse large B-Cell lymphoma or relapsed or refractory low grade follicular lymphoma.

In one aspect, the methods provided herein comprise administering a formulation of Compound 1 with taxotere, dabrafenib, imlygic, ipilimumab, pembrolizumab, nivolumab, trametinib, vemurafenib, talimogene laherparepvec, IL-2, IFN, GM-CSF, and/or dacarbazine, aldesleukin, cobimetinib, Intron A®, peginterferon Alfa-2b, and/or trametinib to patients with various types or stages of melanoma.

In one aspect, the methods provided herein comprise administering a formulation of Compound 1 with vinorelbine or pemetrexed disodium to patients with malignant mesothelioma, or stage IIIB non-small cell lung cancer with pleural implants or malignant pleural effusion mesothelioma syndrome.

In one aspect, the methods of treating patients with various types or stages of multiple myeloma provided herein comprise administering a formulation of Compound 1 with with dexamethasone, zoledronic acid, palmitronate, GM-CSF, biaxin, vinblastine, melphalan, busulphan, cyclophosphamide, IFN, prednisone, bisphosphonate, celecoxib, arsenic trioxide, PEG INTRON-A, vincristine, becenum, bortezomib, carfilzomib, doxorubicin, panobinostat, lenalidomide, pomalidomide, thalidomide, mozobil, carmustine, daratumumab, elotuzumab, ixazomib citrate, plerixafor or a combination thereof.

In certain embodiments, a formulation of Compound 1 provided herein is administered to patients with various types or stages of multiple myeloma in combination with chimeric antigen receptor (CAR) T-cells. In certain embodiments the CAR T cell in the combination targets B cell maturation antigen (BCMA), and in more specific embodiments, the CAR T cell is bb2121 or bb21217. In some embodiments, the CAR T cell is JCARH125.

In certain embodiments, a formulation of Compound 1 provided herein is administered to patients with relapsed or refractory multiple myeloma in combination with doxorubicin (Doxil®), vincristine and/or dexamethasone (Decadron®).

In certain embodiments, the methods provided herein comprise administering a formulation of Compound 1 to patients with various types or stages of ovarian cancer such as peritoneal carcinoma, papillary serous carcinoma, refractory ovarian cancer or recurrent ovarian cancer, in combination with taxol, carboplatin, doxorubicin, gemcitabine, cisplatin, xeloda, paclitaxel, dexamethasone, avastin, cyclophosphamide, topotecan, olaparib, thiotepa, melphalan, niraparib tosylate monohydrate, rubraca or a combination thereof.

In certain embodiments, the methods provided herein comprise administering a formulation of Compound 1 to patients with various types or stages of prostate cancer, in combination with xeloda, 5 FU/LV, gemcitabine, irinotecan plus gemcitabine, cyclophosphamide, vincristine, dexamethasone, GM-CSF, celecoxib, taxotere, ganciclovir, paclitaxel, adriamycin, docetaxel, estramustine, Emcyt, denderon, zytiga, bicalutamide, cabazitaxel, degarelix, enzalutamide, zoladex, leuprolide acetate, mitoxantrone hydrochloride, prednisone, sipuleucel-T, radium 223 dichloride, or a combination thereof.

In certain embodiments, the methods provided herein comprise administering a formulation of Compound 1 to patients with various types or stages of renal cell cancer, in combination with capecitabine, IFN, tamoxifen, IL-2, GM-CSF, Celebrex®, flutamide, goserelin acetate, nilutamide or a combination thereof.

In certain embodiments, the methods provided herein comprise administering a formulation of Compound 1 to patients with various types or stages of gynecologic, uterus or soft tissue sarcoma cancer in combination with IFN, dactinomycin, doxorubicin, imatinib mesylate, pazopanib, hydrochloride, trabectedin, eribulin mesylate, olaratumab, a COX-2 inhibitor such as celecoxib, and/or sulindac.

In one aspect, the methods provided herein comprise administering a formulation of Compound 1 to patients with various types or stages of solid tumors in combination with celecoxib, etoposide, cyclophosphamide, docetaxel, apecitabine, IFN, tamoxifen, IL-2, GM-CSF, or a combination thereof.

In one aspect, the methods provided herein comprise administering a formulation of Compound 1 to patients with scleroderma or cutaneous vasculitis in combination with celebrex, etoposide, cyclophosphamide, docetaxel, apecitabine, IFN, tamoxifen, IL-2, GM-CSF, or a combination thereof.

In one aspect, the methods provided herein comprise administering a formulation of Compound 1 to patients with MDS in combination with azacitidine, cytarabine, daunorubicin, decitabine, idarubicin, lenalidomide, enasidenib, or a combination thereof.

In one aspect, the methods provided herein comprise administering Compound 1 to patients with hematological cancer in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors. In one aspect, the methods provided herein comprise administering a formulation of Compound 1 to patients with a hematological cancer in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

In one aspect, the methods provided herein comprise administering Compound 1 to patients with leukemia in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors. In certain embodiments, a formulation of Compound 1 provided herein is administered to patients with leukemia in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

In one aspect, the methods provided herein comprise administering Compound 1 to patients with AML in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors. In certain embodiments, a formulation of Compound 1 provided herein is administered to patients with AML in combination with one or more second agents selected from JAK inhibitors, FLT3 inhibitors, mTOR inhibitors, spliceosome inhibitors, BET inhibitors, SMG1 inhibitors, ERK inhibitors, LSD1 inhibitors, BH3 mimetics, topoisomerase inhibitors, and RTK inhibitors.

In one aspect, the methods provided herein comprise administering Compound 1 to patients with leukemia in combination with an mTOR inhibitor. In certain embodiments, a formulation of Compound 1 provided herein is administered to patients with leukemia in combination with an mTOR inhibitor. In certain embodiments, the mTOR inhibitor is selected from everolimus, MLN-0128 and AZD8055. In some embodiments, the mTOR inhibitor is an mTOR kinase inhibitor. In certain embodiments, the mTOR kinase inhibitor is selected from 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223) and 1-ethyl-7-(2-methyl-6-(1H-1,2,4-triazol-3-yl)pyridin-3-yl)-3,4-dihydropyrazino[2,3-b]pyrazin-2(1H)-one (CC-115). In certain embodiments, Compound 1 is administered to patients with leukemia in combination with 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223). In certain embodiments, Compound 1 is administered to patients with leukemia in combination with 1-ethyl-7-(2-methyl-6-(1H-1,2,4-triazol-3-yl)pyridin-3-yl)-3,4-dihydropyrazino[2,3-b]pyrazin-2(1H)-one (CC-115). In certain embodiments, Compound 1 is administered to patients with leukemia in combination with everolimus. In certain embodiments, Compound 1 is administered to patients with leukemia in combination with MLN-0128. In certain embodiments, Compound 1 is administered to patients with leukemia in combination with AZD8055.

In one aspect, the methods provided herein comprise administering Compound 1 to patients with AML in combination with an mTOR inhibitor. In certain embodiments, a formulation of Compound 1 provided herein is administered to patients with AML in combination with an mTOR inhibitor. In certain embodiments, the mTOR inhibitor is selected from everolimus, MLN-0128 and AZD8055. In some embodiments, the mTOR inhibitor is an mTOR kinase inhibitor. In certain embodiments, the mTOR kinase inhibitor is selected from 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223) and 1-ethyl-7-(2-methyl-6-(1H-1,2,4-triazol-3-yl)pyridin-3-yl)-3,4-dihydropyrazino[2,3-b]pyrazin-2(1H)-one (CC-115). In certain embodiments, Compound 1 is administered to patients with AML in combination with 1-ethyl-7-(2-methyl-6-(1H-1,2,4-triazol-3-yl)pyridin-3-yl)-3,4-dihydropyrazino[2,3-b]pyrazin-2(1H)-one. In certain embodiments, Compound 1 is administered to patients with AML in combination with everolimus. In certain embodiments, everolimus is administered to patients with AML prior to administration of Compound 1. In certain embodiments, Compound 1 is administered to patients with AML in combination with MLN-0128. In certain embodiments, Compound 1 is administered to patients with AML in combination with AZD8055.

In one aspect, the methods provided herein comprise administering Compound 1 to patients with MPN in combination with a JAK inhibitor. In certain embodiments, a formulation of Compound 1 provided herein is administered to patients with MPN in combination with a JAK inhibitor. In one aspect the JAK inhibitor is selected from a JAK1 inhibitor, a JAK2 inhibitor and a JAK3 inhibitor. In certain embodiments, the JAK inhibitor is selected from tofacitinib, momelotinib, filgotinib, decernotinib, barcitinib, ruxolitinib, fedratinib, NS-018 and pacritinib. In certain embodiments, the JAK inhibitor is selected from tofacitinib, momelotinib, ruxolitinib, fedratinib, NS-018 and pacritinib. In certain embodiments, Compound 1 is administered to patients with MPN in combination with tofacitinib. In certain embodiments, Compound 1 is administered to patients with MPN in combination with momelotinib. In certain embodiments, Compound 1 is administered to patients with MPN in combination with filgotinib. In certain embodiments, Compound 1 is administered to patients with MPN in combination with decernotinib. In certain embodiments, Compound 1 is administered to patients with MPN in combination with barcitinib. In certain embodiments, Compound 1 is administered to patients with MPN in combination with ruxolitinib. In certain embodiments, Compound 1 is administered to patients with MPN in combination with fedratinib. In certain embodiments, Compound 1 is administered to patients with MPN in combination with NS-018. In certain embodiments, Compound 1 is administered to patients with MPN in combination with pacritinib. In certain embodiments, the MPN is IL-3 independent. In certain embodiments, the MPN is characterized by a JAK 2 mutation, for example, a $JAK2^{V617F}$ mutation.

In one aspect, the methods provided herein comprise administering Compound 1 to patients with myelofibrosis in combination with a JAK inhibitor. In certain embodiments, a formulation of Compound 1 provided herein is administered to patients with myelofibrosis in combination with a JAK inhibitor. In one aspect the JAK inhibitor is selected from a JAK1 inhibitor, a JAK2 inhibitor and a JAK3 inhibitor. In certain embodiments, the JAK inhibitor is selected from tofacitinib, momelotinib, ruxolitinib, fedratinib, NS-018 and pacritinib. In certain embodiments, Compound 1 is administered to patients with myelofibrosis in combination with tofacitinib. In certain embodiments, Compound 1 is administered to patients with myelofibrosis in combination with momelotinib. In certain embodiments, Compound 1 is administered to patients with myelofibrosis in combination with ruxolitinib. In certain embodiments, Compound 1 is administered to patients with myelofibrosis in combination with fedratinib. In certain embodiments, Compound 1 is administered to patients with myelofibrosis in combination with NS-018. In certain embodiments, Compound 1 is administered to patients with myelofibrosis in combination with pacritinib. In certain embodiments, the myeolofibrosis is characterized by a JAK 2 mutation, for example, a JAK2V617F mutation. In some embodiments, the myelofibrosis is primary myelofibrosis. In other embodiments, the myelofibrosis is secondary myelofibrosis. In some such embodiments, the secondary myelofibrosis is post polycythemia vera myelofibrosis. In other embodiments, the secondary myelofibrosis is post essential thrombocythemia myelofibrosis.

In one aspect, the methods provided herein comprise administering Compound 1 to patients with leukemia in combination with a JAK inhibitor. In certain embodiments, a formulation of Compound 1 provided herein is administered to patients with leukemia in combination with a JAK inhibitor. In one aspect the JAK inhibitor is selected from a JAK1

inhibitor, a JAK2 inhibitor and a JAK3 inhibitor. In certain embodiments, the JAK inhibitor is selected from tofacitinib, momelotinib, filgotinib, decernotinib, barcitinib, ruxolitinib, fedratinib, NS-018 and pacritinib. In certain embodiments, the JAK inhibitor is selected from momelotinib, ruxolitinib, fedratinib, NS-018 and pacritinib. In certain embodiments, Compound 1 is administered to patients with leukemia in combination with tofacitinib. In certain embodiments, Compound 1 is administered to patients with leukemia in combination with momelotinib. In certain embodiments, Compound 1 is administered to patients with leukemia in combination with filgotinib. In certain embodiments, Compound 1 is administered to patients with leukemia in combination with decernotinib. In certain embodiments, Compound 1 is administered to patients with leukemia in combination with barcitinib. In certain embodiments, Compound 1 is administered to patients with leukemia in combination with ruxolitinib. In certain embodiments, Compound 1 is administered to patients with leukemia in combination with fedratinib. In certain embodiments, Compound 1 is administered to patients with leukemia in combination with NS-018. In certain embodiments, Compound 1 is administered to patients with leukemia in combination with pacritinib. In certain embodiments, the MPN is characterized by a JAK 2 mutation, for example, a JAK2V617F mutation.

In one aspect, the methods provided herein comprise administering Compound 1 to patients with AML in combination with a JAK inhibitor. In certain embodiments, a formulation of Compound 1 provided herein is administered to patients with AML in combination with a JAK inhibitor. In one aspect the JAK inhibitor is selected from a JAK1 inhibitor, a JAK2 inhibitor and a JAK3 inhibitor. In certain embodiments, the JAK inhibitor is selected from tofacitinib, momelotinib, filgotinib, decernotinib, barcitinib, ruxolitinib, fedratinib, NS-018 and pacritinib. In certain embodiments, the JAK inhibitor is selected from momelotinib, ruxolitinib, fedratinib, NS-018 and pacritinib. In certain embodiments, Compound 1 is administered to patients with AML in combination with tofacitinib. In certain embodiments, Compound 1 is administered to patients with AML in combination with momelotinib. In certain embodiments, Compound 1 is administered to patients with AML in combination with filgotinib. In certain embodiments, Compound 1 is administered to patients with AML in combination with decernotinib. In certain embodiments, Compound 1 is administered to patients with AML in combination with barcitinib. In certain embodiments, Compound 1 is administered to patients with AML in combination with ruxolitinib. In certain embodiments, Compound 1 is administered to patients with AML in combination with fedratinib. In certain embodiments, Compound 1 is administered to patients with AML in combination with NS-018. In certain embodiments, Compound 1 is administered to patients with AML in combination with pacritinib. In certain embodiments, the MPN is characterized by a JAK 2 mutation, for example, a JAK2V617F mutation.

In one aspect, the methods provided herein comprise administering Compound 1 to patients with leukemia in combination with a FLT3 kinase inhibitor. In certain embodiments, a formulation of Compound 1 provided herein is administered to patients with leukemia in combination with a FLT3 kinase inhibitor. In certain embodiments, the FLT3 kinase inhibitor is selected from quizartinib, sunitinib, sunitinib malate, midostaurin, pexidartinib, lestaurtinib, tandutinib, and crenolanib. In certain embodiments, Compound 1 is administered to patients with leukemia in combination with quizartinib. In certain embodiments, Compound 1 is administered to patients with leukemia in combination with sunitinib. In certain embodiments, Compound 1 is administered to patients with leukemia in combination with midostaurin. In certain embodiments, Compound 1 is administered to patients with leukemia in combination with pexidartinib. In certain embodiments, Compound 1 is administered to patients with leukemia in combination with lestaurtinib. In certain embodiments, Compound 1 is administered to patients with leukemia in combination with tandutinib. In certain embodiments, Compound 1 is administered to patients with leukemia in combination with crenolanib. In certain embodiments, the patient carries a FLT3-ITD mutation.

In one aspect, the methods provided herein comprise administering Compound 1 to patients with AML in combination with a FLT3 kinase inhibitor. In certain embodiments, a formulation of Compound 1 provided herein is administered to patients with AML in combination with a FLT3 kinase inhibitor. In certain embodiments, the FLT3 kinase inhibitor is selected from quizartinib, sunitinib, sunitinib malate, midostaurin, pexidartinib, lestaurtinib, tandutinib, quizartinib and crenolanib. In certain embodiments, Compound 1 is administered to patients with AML in combination with quizartinib. In certain embodiments, Compound 1 is administered to patients with AML in combination with sunitinib. In certain embodiments, Compound 1 is administered to patients with AML in combination with midostaurin. In certain embodiments, Compound 1 is administered to patients with AML in combination with pexidartinib. In certain embodiments, Compound 1 is administered to patients with AML in combination with lestaurtinib. In certain embodiments, Compound 1 is administered to patients with AML in combination with tandutinib. In certain embodiments, Compound 1 is administered to patients with AML in combination with crenolanib. In certain embodiments, the patient carries a FLT3-ITD mutation.

In certain embodiments, Compound 1 is administered to patients with leukemia in combination with a spliceosome inhibitor. In certain embodiments, Compound 1 is administered to patients with AML in combination with a spliceosome inhibitor. In certain embodiments, the spliceosome inhibitor is pladienolide B, 6-deoxypladienolide D, or H3B-8800.

In one aspect, the methods provided herein comprise administering Compound 1 to patients with leukemia in combination with an SMG1 kinase inhibitor. In certain embodiments, a formulation of Compound 1 provided herein is

administered to patients with leukemia in combination with an SMG1 kinase inhibitor. In one aspect, the methods provided herein comprise administering Compound 1 to patients with AML in combination with an SMG1 kinase inhibitor. In certain embodiments, a formulation of Compound 1 provided herein is administered to patients with AML in combination with an SMG1 kinase inhibitor. In certain embodiments, the SMG1 inhibitor is 1-ethyl-7-(2-methyl-6-(1H-1,2,4-triazol-3-yl)pyridin-3-yl)-3,4-dihydropyrazino[2,3-b]pyrazin-2(1H)-one, chloro-N,N-diethyl-5-((4-(2-(4-(3-methylureido)phenyl)pyridin-4-yl)pyrimidin-2-yl)amino)benzenesulfonamide (compound Ii), or a compound disclosed in A. Gopalsamy et al, Bioorg. Med Chem Lett. 2012, 22:6636-66412 (for example, chloro-N,N-diethyl-5-((4-(2-(4-(3-methylureido)phenyl)pyridin-4-yl)pyrimidin-2-yl)amino)benzenesulfonamide.

In one aspect, the methods provided herein comprise administering Compound 1 to patients with leukemia in combination with a BCL2 inhibitor. In certain embodiments, a formulation of Compound 1 provided herein is administered to patients with leukemia in combination with a BCL2 inhibitor. In certain embodiments, Compound 1 is administered to patients with AML in combination with a BCL2 inhibitor. In certain embodiments, a formulation of Compound 1 provided herein is administered to patients with AML in combination with a BCL2 inhibitor, for example, venetoclax or navitoclax. In certain embodiments, the BCL2 inhibitor is venetoclax.

In one embodiment, provided herein is a method for treating of AML that is resistant to treatment with a BCL2 inhibitor, comprising administering Compound 1. In one embodiment, provided herein is a method for treating of AML that has acquired resistance to venetoclax treatment, comprising administering Compound 1. In one embodiment, provided herein is a method for treating of AML that has acquired resistance to venetoclax treatment, comprising administering a combination of Compound 1 and a BCL2 inhibitor. In one embodiment, provided herein is a method for treating of AML that has acquired resistance to venetoclax treatment, comprising administering a combination of Compound 1 and venetoclax.

In one aspect, the methods provided herein comprise administering Compound 1 to patients with leukemia in combination with a topoisomerase inhibitor. In certain embodiments, a formulation of Compound 1 provided herein is administered to patients with leukemia in combination with a topoisomerase inhibitor. In certain embodiments, Compound 1 is administered to patients with AML in combination with a topoisomerase inhibitor. In certain embodiments, a formulation of Compound 1 provided herein is administered to patients with AML in combination with a topoisomerase inhibitor, for example, irinotecan, topotecan, camptothecin, lamellarin D, etoposide, teniposide, doxorubicin, daunorubicin, mitoxantrone, amsacrine, ellipticines, aurintricarboxylic acid, or HU-331. In certain embodiments, the topoisomerase inhibitor is topotecan.

In certain embodiments, Compound 1 is administered to patients with leukemia in combination with a BET inhibitor. In certain embodiments, Compound 1 is administered to patients with AML in combination with a BET inhibitor. In certain embodiments, the BET inhibitor is selected from GSK525762A, OTX015, BMS-986158, TEN-010, CPI-0610 , INCB54329, BAY1238097, FT-1101, C90010, ABBV-075, BI 894999, GS-5829, GSK1210151A (I-BET-151), CPI-203, RVX 208, XD46, MS436, PFI-1, RVX2135, ZEN3365, XD14, ARV-771, MZ-1, PLX5117, 4-[2-(cyclopropylmethoxy)-5-(methanesulfonyl)phenyl]-2-methylisoquinolin-1(2H)-one (Compound A), EP11313 and EP11336.

In certain embodiments, Compound 1 is administered to patients with leukemia in combination with an LSD1 inhibitor. In certain embodiments, Compound 1 is administered to patients with AML in combination with an LSD1 inhibitor. In certain embodiments, the LSD1 inhibitor is selected from ORY-1001, ORY-2001, INCB-59872, IMG-7289, TAK 418, GSK-2879552, and 4-[2-(4-amino-piperidin-1-yl)-5-(3-fluoro-4-methoxy-phenyl)-1-methyl-6-oxo-1,6-dihydropyrimidin-4-yl]-2-fluoro-benzonitrile or a salt thereof (e.g. besylate salt, Compound B).

In one aspect, the methods provided herein comprise administering Compound 1 to patients with leukemia in combination with triptolide, retaspimycin, alvespimycin, 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223), 1-ethyl-7-(2-methyl-6-(1H-1,2,4-triazol-3-yl)pyridin-3-yl)-3,4-dihydropyrazino[2,3-b]pyrazin-2(1H)-one (CC-115), rapamycin, MLN-0128, everolimus, AZD8055, pladienolide B, topotecan, thioguanine, mitoxantrone, etoposide, decitabine, daunorubicin, clofarabine, cladribine, 6-mercaptopurine, chloro-N,N-diethyl-5-((4-(2-(4-(3-methylureido)phenyl)pyridin-4-yl)pyrimidin-2-yl)amino)benzenesulfonamide (compound Ii), fedratinib, sunitinib, pexidartinib, midostaurin, lestaurtinib, momelotinib, quizartinib, and crenolanib.

In one aspect, the methods provided herein comprise administering Compound 1 to patients with AML in combination with triptolide, retaspimycin, alvespimycin, 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223), 1-ethyl-7-(2-methyl-6-(1H-1,2,4-triazol-3-yl)pyridin-3-yl)-3,4-dihydropyrazino[2,3-b]pyrazin-2(1H)-one (CC-115), rapamycin, MLN-0128, everolimus, AZD8055, pladienolide B, topotecan, thioguanine, mitoxantrone, etoposide, decitabine, daunorubicin, clofarabine, cladribine, 6-mercaptopurine, chloro-N,N-diethyl-5-((4-(2-(4-(3-methylureido)phenyl)pyridin-4-yl)pyrimidin-2-yl)amino)benzenesulfonamide (compound Ii), fedratinib, sunitinib, pexidartinib, midostaurin, lestaurtinib, momelotinib, quizartinib, and crenolanib.

In one aspect, the methods provided herein comprise administering Compound 1 to patients with cancer in combination with an mTOR inhibitor, wherein the cancer is selected from breast cancer, kidney cancer, pancreatic cancer, gastrointestinal cancer, lung cancer, neuroendocrine tumor (NET), and renal cell carcinoma (RCC). In certain embodiments, a formulation of Compound 1 provided herein is administered to patients with cancer in combination with

a topoisomerase inhibitor. In certain embodiments, a formulation of Compound 1 provided herein is administered to cancer patients in combination with an mTOR inhibitor, wherein the cancer is selected from breast cancer, kidney cancer, pancreatic cancer, gastrointestinal cancer, lung cancer, neuroendocrine tumor (NET), and renal cell carcinoma. In certain embodiments, the mTOR inhibitor is selected from everolimus, MLN-0128 and AZD8055. In some embodiments, the mTOR inhibitor is an mTOR kinase inhibitor. In certain embodiments, the mTOR kinase inhibitor is selected from 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223) and 1-ethyl-7-(2-methyl-6-(1H-1,2,4-triazol-3-yl)pyridin-3-yl)-3,4-dihydropyrazino[2,3-b]pyrazin-2(1H)-one (CC-115). In one embodiment, the mTOR kinase inhibitor is 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223). In one embodiment, the mTOR kinase inhibitor is 1-ethyl-7-(2-methyl-6-(1H-1,2,4-triazol-3-yl)pyridin-3-yl)-3,4-dihydropyrazino[2,3-b]pyrazin-2(1H)-one (CC-115). In one embodiment, the mTOR inhibitor is everolimus. In one embodiment, the mTOR inhibitor is temsirolimus. In one embodiment, the mTOR inhibitor is MLN-0128. In one embodiment, the mTOR inhibitor is AZD8055.

In certain embodiments, Compound 1 is administered to breast cancer patients in combination with everolimus. In certain embodiments, a formulation of Compound 1 provided herein is administered to breast cancer patients in combination with everolimus.

In certain embodiments, Compound 1 is administered to kidney cancer patients in combination with everolimus. In certain embodiments, a formulation of Compound 1 provided herein is administered to kidney cancer patients in combination with everolimus.

In certain embodiments, Compound 1 is administered to pancreatic cancer patients in combination with everolimus. In certain embodiments, a formulation of Compound 1 provided herein is administered to pancreatic cancer patients in combination with everolimus.

In certain embodiments, Compound 1 is administered to gastrointestinal cancer patients in combination with everolimus. In certain embodiments, a formulation of Compound 1 provided herein is administered to gastrointestinal cancer patients in combination with everolimus.

In certain embodiments, Compound 1 is administered to lung cancer patients in combination with everolimus. In certain embodiments, a formulation of Compound 1 provided herein is administered to lung cancer patients in combination with everolimus.

In certain embodiments, Compound 1 is administered to neuroendocrine tumor patients in combination with everolimus. In certain embodiments, a formulation of Compound 1 provided herein is administered to neuroendocrine tumor patients in combination with everolimus.

In certain embodiments, Compound 1 is administered to renal cell carcinoma patients in combination with everolimus. In certain embodiments, a formulation of Compound 1 provided herein is administered to renal cell carcinoma patients in combination with everolimus.

Also encompassed herein is a method of increasing the dosage of an anti-cancer drug or agent that can be safely and effectively administered to a patient, which comprises administering to the patient *(e.g.,* a human) Compound 1, for example, a formulation of Compound 1 provided herein in combination with the second anti-cancer drug. Patients that can benefit by this method are those likely to suffer from an adverse effect associated with anti-cancer drugs for treating a specific cancer of the skin, subcutaneous tissue, lymph nodes, brain, lung, liver, bone, intestine, colon, heart, pancreas, adrenal, kidney, prostate, breast, colorectal, or combinations thereof. The administration of Compound 1, for example, a formulation of Compound 1 provided herein, alleviates or reduces adverse effects which are of such severity that it would otherwise limit the amount of anti-cancer drug.

Also encompassed herein is a method of decreasing the dosage of an anti-cancer drug or agent that can be safely and effectively administered to a patient, which comprises administering to the patient *(e.g.,* a human) Compound 1, for example, a formulation of Compound 1 provided herein in combination with the second anti-cancer drug. Patients that can benefit by this method are those likely to suffer from an adverse effect associated with anti-cancer drugs for treating a specific cancer of the skin, subcutaneous tissue, lymph nodes, brain, lung, liver, bone, intestine, colon, heart, pancreas, adrenal, kidney, prostate, breast, colorectal, or combinations thereof. The administration of Compound 1, for example, a formulation of Compound 1 provided herein, potentiates the activity of the anti-cancer drug, which allows for a reduction in dose of the anti-cancer drug while maintaining efficacy, which in turn can alleviate or reduce the adverse effects which are of such severity that it limited the amount of anti-cancer drug.

In one embodiment, Compound 1 is administered daily in an amount ranging from about 0.1 to about 20 mg, from about 1 to about 15 mg, from about 1 to about 10 mg, or from about 1 to about 15 mg prior to, during, or after the occurrence of the adverse effect associated with the administration of an anti-cancer drug to a patient. In certain embodiments, Compound 1 is administered in combination with specific agents such as heparin, aspirin, coumadin, or G-CSF to avoid adverse effects that are associated with anti-cancer drugs such as but not limited to neutropenia or thrombocytopenia.

In one embodiment, Compound 1, for example, a formulation of Compound 1 provided herein, is administered to patients with diseases and disorders associated with or characterized by, undesired angiogenesis in combination with

additional active ingredients, including, but not limited to, anti-cancer drugs, anti-inflammatories, antihistamines, antibiotics, and steroids.

In another embodiment, encompassed herein is a method of treating, preventing and/or managing cancer, which comprises administering Compound 1, for example, a formulation of Compound 1 provided herein, in conjunction with (e.g. before, during, or after) at least one anti-cancer therapy including, but not limited to, surgery, immunotherapy, biological therapy, radiation therapy, or other non-drug based therapy presently used to treat, prevent and/or manage cancer. The combined use of the compound provided herein and other anti-cancer therapy may provide a unique treatment regimen that is unexpectedly effective in certain patients. Without being limited by theory, it is believed that Compound 1 may provide additive or synergistic effects when given concurrently with at least one anti-cancer therapy.

As discussed elsewhere herein, encompassed herein is a method of reducing, treating and/or preventing adverse or undesired effects associated with other anti-cancer therapy including, but not limited to, surgery, chemotherapy, radiation therapy, hormonal therapy, biological therapy and immunotherapy. Compound 1, for example, a formulation of Compound 1 provided herein, and other active ingredient can be administered to a patient prior to, during, or after the occurrence of the adverse effect associated with other anti-cancer therapy.

In certain embodiments, the methods provided herein comprise administration of one or more of calcium, calcitriol, or vitamin D supplementation with Compound 1. In certain embodiments, the methods provided herein comprise administration of calcium, calcitriol, and vitamin D supplementation prior to the treatment with Compound 1. In certain embodiments, the methods provided herein comprise administration of calcium, calcitriol, and vitamin D supplementation prior to the administration of first dose of Compound 1 in each cycle. In certain embodiments, the methods provided herein comprise administration of calcium, calcitriol, and vitamin D supplementation at least up to 3 days prior to the treatment with Compound 1. In certain embodiments, the methods provided herein comprise administration of calcium, calcitriol, and vitamin D supplementation prior to the administration of first dose of Compound 1 in each cycle. In certain embodiments, the methods provided herein comprise administration of calcium, calcitriol, and vitamin D supplementation at least up to 3 days prior to the administration of first dose of Compound 1 in each cycle. In certain embodiments, the methods provided herein comprise administration of calcium, calcitriol, and vitamin D supplementation prior to administration of first dose of Compound 1 in each cycle and continues after administration of the last dose of Compound 1 in each cycle. In certain embodiments, the methods provided herein comprise administration of calcium, calcitriol, and vitamin D supplementation at least up to 3 days prior to administration of first dose of Compound 1 in each cycle and continues until at least up to 3 days after administration of the last dose of Compound 1 in each cycle *(e.g.,* at least up to day 8 when Compound 1 is administered on Days 1-5). In one embodiment, the methods provided herein comprise administration of calcium, calcitriol, and vitamin D supplementation at least up to 3 days prior to administration of day 1 of each cycle and continue until ≥ 3 days after the last dose of Compound 1 in each cycle (eg, ≥ Day 8 when Compound 1 is administered on Days 1-5, ≥ Day 13 when Compound 1 is administered on Days 1-3 and Days 8-10).

In certain embodiments, calcium supplementation is administered to deliver at least 1200 mg of elemental calcium per day given in divided doses. In certain embodiments, calcium supplementation is administered as calcium carbonate in a dose of 500 mg administered three times a day per orally (PO).

In certain embodiments, calcitriol supplementation is administered to deliver 0.25 $\mu$g calcitriol (PO) once daily.

In certain embodiments, vitamin D supplementation is administered to deliver about 500 IU to about 50,000 IU vitamin D once daily. In certain embodiments, vitamin D supplementation is administered to deliver about 1000 IU vitamin D once daily. In certain embodiments, vitamin D supplementation is administered to deliver about 50,000 IU vitamin D weekly. In certain embodiments, vitamin D supplementation is administered to deliver about 1000 IU vitamin D2 or D3 once daily. In certain embodiments, vitamin D supplementation is administered to deliver about 500 IU vitamin D once daily. In certain embodiments, vitamin D supplementation is administered to deliver about 50,000 IU vitamin D weekly. In certain embodiments, vitamin D supplementation is administered to deliver about 20,000 IU vitamin D weekly. In certain embodiments, vitamin D supplementation is administered to deliver about 1000 IU vitamin D2 or D3 once daily. In certain embodiments, vitamin D supplementation is administered to deliver about 50,000 IU vitamin D2 or D3 weekly. In certain embodiments, vitamin D supplementation is administered to deliver about 20,000 IU vitamin D2 or D3 weekly.

In certain embodiments, a formulation of Compound 1 provided herein and doxetaxol are administered to patients with non-small cell lung cancer who were previously treated with carbo/VP 16 and radiotherapy.

### Use **With Transplantation Therapy**

Compound 1, for example, a formulation of Compound 1 provided herein, can be used to reduce the risk of Graft Versus Host Disease (GVHD). Therefore, encompassed herein is a method of treating, preventing and/or managing cancer, which comprises administering Compound 1, for example, a formulation of Compound 1 provided herein, in conjunction with transplantation therapy.

As those of ordinary skill in the art are aware, the treatment of cancer is often based on the stages and mechanism of the disease. For example, as inevitable leukemic transformation develops in certain stages of cancer, transplantation

of peripheral blood stem cells, hematopoietic stem cell preparation or bone marrow may be necessary. The combined use of Compound 1, for example, a formulation of Compound 1 provided herein, and transplantation therapy provides a unique and unexpected synergism. In particular, a formulation of Compound 1 provided herein exhibits immunomodulatory activity that may provide additive or synergistic effects when given concurrently with transplantation therapy in patients with cancer.

Compound 1, for example, a formulation of Compound 1 provided herein, can work in combination with transplantation therapy reducing complications associated with the invasive procedure of transplantation and risk of GVHD. Encompassed herein is a method of treating, preventing and/or managing cancer which comprises administering to a patient *(e.g.,* a human) formulation of Compound 1 provided herein before, during, or after the transplantation of umbilical cord blood, placental blood, peripheral blood stem cell, hematopoietic stem cell preparation, or bone marrow. Some examples of stem cells suitable for use in the methods provided herein are disclosed in U.S. patent no. 7,498,171.

In one embodiment, Compound 1, for example, a formulation of Compound 1 provided herein, is administered to patients with acute myeloid leukemia before, during, or after transplantation.

In one embodiment, Compound 1, for example, a formulation of Compound 1 provided herein, is administered to patients with multiple myeloma before, during, or after the transplantation of autologous peripheral blood progenitor cells.

In one embodiment, Compound 1, for example, a formulation of Compound 1 provided herein, is administered to patients with NHL *(e.g.,* DLBCL) before, during, or after the transplantation of autologous peripheral blood progenitor cells.

**Cycling Therapy**

In certain embodiments, Compound 1, for example, a formulation of Compound 1 provided herein, are cyclically administered to a patient independent of the cancer treated. Cycling therapy involves the administration of an active agent for a period of time, followed by a rest for a period of time, and repeating this sequential administration. Cycling therapy can reduce the development of resistance to one or more of the therapies, avoid or reduce the side effects of one of the therapies, and/or improve the efficacy of the treatment.

In certain embodiments, Compound 1, for example, a formulation of Compound 1 provided herein, is administered daily in a single or divided dose in a four to six week cycle with a rest period of about a week or two weeks. In certain embodiments, Compound 1, for example, a formulation of Compound 1 provided herein, is administered daily in a single or divided doses for one to ten consecutive days of a 28 day cycle, then a rest period with no administration for rest of the 28 day cycle. The cycling method further allows the frequency, number, and length of dosing cycles to be increased. Thus, encompassed herein in certain embodiments is the administration of Compound 1, for example, a formulation of Compound 1 provided herein, for more cycles than are typical when it is administered alone. In certain embodiments, Compound 1, for example, a formulation of Compound 1 provided herein, is administered for a greater number of cycles that would typically cause dose-limiting toxicity in a patient to whom a second active ingredient is not also being administered.

In one embodiment, Compound 1, for example, a formulation of Compound 1 provided herein, is administered daily and continuously for three or four weeks to administer a dose of Compound 1 from about 0.1 to about 20 mg/d followed by a break of one or two weeks.

In another embodiment, Compound 1, for example, a formulation of Compound 1 provided herein, is administered intravenously and a second active ingredient is administered orally, with administration of Compound 1, for example, a formulation of Compound 1 provided herein, occurring 30 to 60 minutes prior to a second active ingredient, during a cycle of four to six weeks. In certain embodiments, the combination of Compound 1, for example, a formulation of Compound 1 provided herein, and a second active ingredient is administered by intravenous infusion over about 90 minutes every cycle. In certain embodiments, one cycle comprises the administration from about 0.1 to about 150 mg/day of Compound 1, for example, a formulation of Compound 1 provided herein, and from about 50 to about 200 $mg/m^2$/day of a second active ingredient daily for three to four weeks and then one or two weeks of rest. In certain embodiments, the number of cycles during which the combinatorial treatment is administered to a patient is ranging from about one to about 24 cycles, from about two to about 16 cycles, or from about four to about three cycles.

In one embodiment, a cycling therapy provided herein comprises administering Compound 1, for example, a formulation of Compound 1 provided herein, in a treatment cycle which includes an administration period of up to 5 days followed by a rest period. In one embodiment, the treatment cycle includes an administration period of 5 days followed by a rest period. In one embodiment, the treatment cycle includes an administration period of up to 10 days followed by a rest period. In one embodiment, the rest period is from about 10 days up to about 40 days. In one embodiment, the treatment cycle includes an administration period of up to 10 days followed by a rest period from about 10 days up to about 40 days. In one embodiment, the treatment cycle includes an administration period of up to 10 days followed by a rest period from about 23 days up to about 37 days. In one embodiment, the rest period is from about 23 days up to

about 37 days. In one embodiment, the rest period is 23 days. In one embodiment, the treatment cycle includes an administration period of up to 10 days followed by a rest period of 23 days. In one embodiment, the rest period is 37 days. In one embodiment, the treatment cycle includes an administration period of up to 10 days followed by a rest period of 37 days.

In one embodiment, the treatment cycle includes an administration of Compound 1, for example, a formulation of Compound 1 provided herein, on days 1 to 5 of a 28 day cycle. In another embodiment, the treatment cycle includes an administration of Compound 1, for example, a formulation of Compound 1 provided herein, on days 1- 10 of a 28 day cycle. In one embodiment, the treatment cycle includes an administration on days 1 to 5 of a 42 day cycle. In another embodiment, the treatment cycle includes an administration on days 1-10 of a 42 day cycle. In another embodiment, the treatment cycle includes an administration on days 1 - 5 and 15 - 19 of a 28 day cycle. In another embodiment, the treatment cycle includes an administration on days 1 - 3 and 8 - 10 of a 28 day cycle.

In one embodiment, the treatment cycle includes an administration of Compound 1, for example, a formulation of Compound 1 provided herein, on days 1 to 21 of a 28 day cycle. In another embodiment, the treatment cycle includes an administration on days 1 to 5 of a 7 day cycle. In another embodiment, the treatment cycle includes an administration on days 1 to 7 of a 7 day cycle.

Any treatment cycle described herein can be repeated for at least 2, 3, 4, 5, 6, 7, 8, or more cycles. In certain instances, the treatment cycle as described herein includes from 1 to about 24 cycles, from about 2 to about 16 cycles, or from about 2 to about 4 cycles. In certain instances a treatment cycle as described herein includes from 1 to about 4 cycles. In certain embodiments, cycle 1 to 4 are all 28 day cycles. In certain embodiments, cycle 1 is a 42 day cycle and cycles 2 to 4 are 28 day cycles. In some embodiments, Compound 1, for example, a formulation of Compound 1 provided herein, is administered for 1 to 13 cycles of 28 days (e.g. about 1 year). In certain instances, the cycling therapy is not limited to the number of cycles, and the therapy is continued until disease progression. Cycles, can in certain instances, include varying the duration of administration periods and/or rest periods described herein.

In one embodiment the treatment cycle includes administering Compound 1 at a dosage amount of about 0.3 mg/day, 0.6 mg/day, 1.2 mg/day, 1.8 mg/day, 2.4 mg/day, 3.6 mg/day, 5.4 mg/day, 7.2 mg/day, 8.1 mg/day, 9.0 mg/day, 10.0 mg/day, 10.8 mg/day, or 12.2 mg/day administered once per day. In one embodiment the treatment cycle includes administering Compound 1 at a dosage amount of about 0.3 mg/day, 0.6 mg/day, 1.2 mg/day, 1.8 mg/day, 2.4 mg/day, 3.6 mg/day, 5.4 mg/day, 7.2 mg/day, 8.1 mg/day, 9.0 mg/day, 10.0 mg/day, 10.8 mg/day, 12.2 mg/day, or 20 mg/day administered once per day. In one embodiment the treatment cycle includes administering Compound 1 at a dosage amount of about 0.6 mg/day, 1.2 mg/day, 1.8 mg/day, 2.4 mg/day, or 3.6 mg/day, administered once per day. In some such embodiments, the treatment cycle includes administering Compound 1 at a dosage amount of about 0.6 mg, 1.2 mg, 1.8 mg, 2.4 mg, or 3.6 mg on days 1 to 3 of a 28 day cycle. In other embodiments, the treatment cycle includes administering Compound 1 at a dosage amount of about 0.6 mg, 1.2 mg, 1.8 mg, 2.4 mg, or 3.6 mg on days 1 to 5 and 15 to 19 of a 28 day cycle. In other embodiments, the treatment cycle includes administering Compound 1 at a dosage amount of about 0.6 mg, 1.2 mg, 1.8 mg, 2.4 mg, 3.6 mg, 5.4 mg/day, 7.2 mg/day, 8.1 mg/day, 9.0 mg/day, or 10.0 mg/day, on days 1 to 5 and 15 to 19 of a 28 day cycle.

Compound 1, for example, a formulation of Compound 1 provided herein, can be administered at the same amount for all administration periods in a treatment cycle. Alternatively, in one embodiment, the compound is administered at different doses in the administration periods.

In one embodiment, a formulation of Compound 1 provided herein is administered to a subject in a cycle, wherein the cycle comprises administering the formulation for at least 5 days in a 28 day cycle. In one embodiment, a formulation of Compound 1 provided herein is administered to a subject in a cycle, wherein the cycle comprises administering the formulation on days 1 to 5 of a 28 day cycle. In one embodiment, the formulation is administered to deliver Compound 1 in a dose of about 0.1 mg to about 20 mg on days 1 to 5 of a 28 day cycle. In one embodiment, the formulation is administered to deliver Compound 1 in a dose of about 0.5 mg to about 5 mg on days 1 to 5 of a 28 day cycle. In one embodiment, the formulation is administered to deliver Compound 1 in a dose of about 0.5 mg to about 10 mg on days 1 to 5 of a 28 day cycle. In one embodiment, a formulation of Compound 1 provided herein is administered to a subject in a cycle, wherein the cycle comprises administering the formulation on days 1 to 5 and 15 to 19 of a 28 day cycle. In one embodiment, the formulation is administered to deliver Compound 1 in a dose of about 0.1 mg to about 20 mg on days 1 to 5 and 15 to 19 of a 28 day cycle. In one embodiment, the formulation is administered to deliver Compound 1 in a dose of about 0.5 mg to about 5 mg on days 1 to 5 and 15 to 19 of a 28 day cycle. In one embodiment, the formulation is administered to deliver Compound 1 in a dose of about 0.5 mg to about 10 mg on days 1 to 5 and 15 to 19 of a 28 day cycle.

In one embodiment, provided herein is a method of treating of AML by administering to a subject a formulation of Compound 1 provided herein in a cycle, wherein the cycle comprises administering the formulation to deliver Compound 1 in a dose of about 0.1 mg to about 20 mg for at least 5 days in a 28 day cycle. In one embodiment, provided herein is a method of treating of AML by administering to a subject a formulation of Compound 1 provided herein in a cycle, wherein the cycle comprises administering the formulation to deliver Compound 1 in a dose of about 0.1 mg to about

20 mg on days 1 to 5 of a 28 day cycle. In one embodiment, provided herein is a method of treating of AML by administering to a subject a formulation of Compound 1 provided herein in a cycle, wherein the cycle comprises administering the formulation to deliver Compound 1 in a dose of about 0.1 mg to about 5 mg on days 1 to 5 of a 28 day cycle. In one embodiment, provided herein is a method of treating of AML by administering to a subject a formulation of Compound 1 provided herein in a cycle, wherein the cycle comprises administering the formulation to deliver Compound 1 in a dose of about 0.5 mg to about 5 mg on days 1 to 5 of a 28 day cycle. In another embodiment, provided herein is a method of treating of AML by administering to a subject a formulation of Compound 1 provided herein in a cycle, wherein the cycle comprises administering the formulation to deliver Compound 1 in a dose of about 0.1 mg to about 20 mg on days 1 to 5 and 15 to 19 of a 28 day cycle. In one embodiment, provided herein is a method of treating of AML by administering to a subject a formulation of Compound 1 provided herein in a cycle, wherein the cycle comprises administering the formulation to deliver Compound 1 in a dose of about 0.1 mg to about 5 mg on days 1 to 5 and 15 to 19 of a 28 day cycle. In one embodiment, provided herein is a method of treating of AML by administering to a subject a formulation of Compound 1 provided herein in a cycle, wherein the cycle comprises administering the formulation to deliver Compound 1 in a dose of about 0.5 mg to about 5 mg on days 1 to 5 and 15 to 19 of a 28 day cycle.

In one embodiment, provided herein is a method of treating of MDS by administering to a subject a formulation of Compound 1 provided herein in a cycle, wherein the cycle comprises administering the formulation to deliver Compound 1 in a dose of about 0.1 mg to about 20 mg for at least 5 days in a 28 day cycle. In one embodiment, provided herein is a method of treating of MDS by administering to a subject a formulation of Compound 1 provided herein in a cycle, wherein the cycle comprises administering the formulation to deliver Compound 1 in a dose of about 0.1 mg to about 20 mg on days 1 to 5 of a 28 day cycle. In one embodiment, provided herein is a method of treating of MDS by administering to a subject a formulation of Compound 1 provided herein in a cycle, wherein the cycle comprises administering the formulation to deliver Compound 1 in a dose of about 0.1 mg to about 5 mg on days 1 to 5 of a 28 day cycle. In one embodiment, provided herein is a method of treating of MDS by administering to a subject a formulation of Compound 1 provided herein in a cycle, wherein the cycle comprises administering the formulation to deliver Compound 1 in a dose of about 0.5 mg to about 5 mg on days 1 to 5 of a 28 day cycle. In another embodiment, provided herein is a method of treating of MDS by administering to a subject a formulation of Compound 1 provided herein in a cycle, wherein the cycle comprises administering the formulation to deliver Compound 1 in a dose of about 0.1 mg to about 20 mg on days 1 to 5 and 15 to 19 of a 28 day cycle. In one embodiment, provided herein is a method of treating of MDS by administering to a subject a formulation of Compound 1 provided herein in a cycle, wherein the cycle comprises administering the formulation to deliver Compound 1 in a dose of about 0.1 mg to about 5 mg on days 1 to 5 and 15 to 19 of a 28 day cycle. In one embodiment, provided herein is a method of treating of MDS by administering to a subject a formulation of Compound 1 provided herein in a cycle, wherein the cycle comprises administering the formulation to deliver Compound 1 in a dose of about 0.5 mg to about 5 mg on days 1 to 5 and 15 to 19 of a 28 day cycle.

**Patient Population**

In certain embodiments of the methods provided herein, the subject is an animal, preferably a mammal, more preferably a non-human primate. In particular embodiments, the subject is a human. The subject can be a male or female subject.

Particularly useful subjects for the methods provided herein include human cancer patients, for example, those who have been diagnosed with leukemia, including acute myeloid leukemia, acute lymphocytic leukemia, chronic myelogenous leukemia, and chronic myelogenous leukemia. In certain embodiments, the subject has not been diagnosed with acute promyelocytic leukemia.

In some embodiments, the subject has a higher than normal blast population. In some embodiments, the subject has a blast population of at least 10%. In some embodiments, the subject has a blast population of between 10 and 15%. In some embodiments, the subject has a blast population of at least 15%. In some embodiments, the subject has a blast population of between 15 and 20%. In some embodiments, the subject has a blast population of at least 20%. In some embodiments, the subject has a blast population of about 10-15%, about 15-20%, or about 20-25%. In other embodiments, the subject has a blast population of less than 10%. In the context of the methods described herein, useful subjects having a blast population of less than 10% includes those subjects that, for any reason according to the judgment of the skilled practitioner in the art, are in need of treatment with a compound provided herein, alone or in combination with a second active agent.

In some embodiments, the subject is treated based on the Eastern Cooperative Oncology Group (ECOG) performance status score of the subject for leukemia. ECOG performance status can be scored on a scale of 0 to 5, with 0 denoting asymptomatic; 1 denoting symptomatic but completely ambulant; 2 denoting symptomatic and <50% in bed during the day; 3 denoting symptomatic and >50% in bed, but not bed bound; 4 denoting bed bound; and 5 denoting death. In some embodiments, the subject has an ECOG performance status score of 0 or 1. In some embodiments, the subject has an ECOG performance status score of 0. In some embodiments, the subject has an ECOG performance

status score of 1. In other embodiments, the subject has an ECOG performance status score of 2.

In certain embodiments, the methods provided herein encompass the treatment of subjects who have not been previously treated for leukemia. In some embodiments, the subject has not undergone allogeneic bone marrow transplantation. In some embodiments, the subject has not undergone a stem cell transplantation. In some embodiments, the subject has not received hydroxyurea treatment. In some embodiments, the subject has not been treated with any investigational products for leukemia. In some embodiments, the subject has not been treated with systemic glucocorticoids.

In other embodiments, the methods encompass treating subjects who have been previously treated or are currently being treated for leukemia. For example, the subject may have been previously treated or are currently being treated with a standard treatment regimen for leukemia. The subject may have been treated with any standard leukemia treatment regimen known to the practitioner of skill in the art. In certain embodiments, the subject has been previously treated with at least one induction/reinduction or consolidation AML regimen. In some embodiments, the subject has undergone autologous bone marrow transplantation or stem cell transplantation as part of a consolidation regimen. In some embodiments, the bone marrow or stem cell transplantation occurred at least 3 months prior to treatment according to the methods provided herein. In some embodiments, the subject has undergone hydroxyurea treatment. In some embodiments, the hydroxyurea treatment occurred no later than 24 hours prior to treatment according to the methods provided herein. In some embodiments, the subject has undergone prior induction or consolidation therapy with cytarabine (Ara-C). In some embodiments, the subject has undergone treatment with systemic glucocorticosteroids. In some embodiments, the glucocorticosteroid treatment occurred no later 24 hours prior to treatment according to the methods described herein. In other embodiments, the methods encompass treating subjects who have been previously treated for cancer, but are non-responsive to standard therapies.

Also encompassed are methods of treating subjects having relapsed or refractory leukemia. In some embodiments, the subject has been diagnosed with a relapsed or refractory AML subtype, as defined by the World Health Organization (WHO). Relapsed or refractory disease may be de novo AML or secondary AML, e.g., therapy-related AML (t-AML).

In some embodiments, the methods provided herein are used to treat leukemia, characterized by presence of a mutant allele of IDH2. In one embodiment, the mutant allele of IDH2 is IDH2 R140Q or R172K.

In some embodiments, the methods provided herein are used to treat AML, characterized by presence of a mutant allele of IDH2. In one embodiment, the mutant allele of IDH2 is IDH2 R140Q or R172K.

Thus, treatment with a compound provided herein could provide an alternative for patients who do not respond to other methods of treatment. In some embodiments, such other methods of treatment encompass treatment with Gleevec® (imatinib mesylate). In some embodiments, provided herein are methods of treatment of Philadelphia chromosome positive chronic myelogenous leukemia (Ph+CML). In some embodiments, provided herein are methods of treatment of Gleevec® (imatinib mesylate) resistant Philadelphia chromosome positive chronic myelogenous leukemia (Ph+CML).

In some embodiments, the methods provided herein are used to treat drug resistant leukemias, such as CML. Thus, treatment with a compound provided herein could provide an alternative for patients who do not respond to other methods of treatment. In some embodiments, such other methods of treatment encompass treatment with Gleevec® (imatinib mesylate). In some embodiments, provided herein are methods of treatment of Ph+CML. In some embodiments, provided herein are methods of treatment of Gleevec® (imatinib mesylate) resistant Ph+CML.

Also encompassed are methods of treating a subject regardless of the subject's age, although some diseases or disorders are more common in certain age groups. In some embodiments, the subject is at least 18 years old. In some embodiments, the subject is more than 18, 25, 35, 40, 45, 50, 55, 60, 65, or 70 years old. In other embodiments, the subject is less than 65 years old. In some embodiments, the subject is less than 18 years old. In some embodiments, the subject is less than 18, 15, 12, 10, 9, 8 or 7 years old.

In some embodiments, the methods may find use in subjects at least 50 years of age, although younger subjects could benefit from the method as well. In other embodiments, the subjects are at least 55, at least 60, at least 65, and at least 70 years of age. In another embodiment, the subject has a cancer with adverse cytogenetics. "Adverse cytogenetics" is defined as any nondiploid karyotype, or greater than or equal to 3 chromosomal abnormalities. In another embodiment, the subjects are at least 60 years of age and have a cancer with adverse cytogenetics. In another embodiment, the subjects are 60-65 years of age and have a cancer with adverse cytogenetics. In another embodiment, the subjects are 65-70 years of age and have a cancer with adverse cytogenetics.

In certain embodiments, the subject treated has no history of myocardial infarction within three months of treatment according to the methods provided herein. In some embodiments, the subject has no history of cerebrovascular accident or transient ischemic attack within three months of treatment according to the methods provided herein. In some embodiments, the subject has no suffered no thromboembelic event, including deep vein thrombosis or pulmonary embolus, within 28 days of treatment according to the methods provided herein. In other embodiments, the subject has not experienced or is not experiencing uncontrolled disseminated intravascular coagulation.

Because subjects with cancer have heterogeneous clinical manifestations and varying clinical outcomes, the treatment given to a patient may vary, depending on his/her prognosis. The skilled clinician will be able to readily determine without undue experimentation specific secondary agents, types of surgery, and types of non-drug based standard therapy that can be effectively used to treat an individual subject with cancer.

It will be appreciated that every suitable combination of the compounds provided herein with one or more of the aforementioned compounds and optionally one or more further pharmacologically active substances is contemplated herein.

## Evaluation of Activity

Standard physiological, pharmacological and biochemical procedures are available for testing the compounds to identify those that possess the desired activity.

Such assays include, for example, cell based assays, including the assay described in the Example section.

Embodiments provided herein may be more fully understood by reference to the following examples. These examples are meant to be illustrative of pharmaceutical compositions and dosage forms provided herein, but are not in any way limiting.

## EXAMPLES

The following Examples are presented by way of illustration, not limitation. Examples of formulations of Compound 1 and HPBCD or SBEβCD in concentration ranges defined in the claims are according to the invention. Any other example is to be understood as being for reference only.

The following abbreviations are used in descriptions and examples.

SWFI - Sterile Water for Injection
WFI - Water for injection
D5W - Dextrose 5% in Water
HPβCD or HPBCD - Hydroxypropyl-beta-cyclodextrin
SBEβCD - Sulfobutylether-β-cyclodextrin sodium salt
CD - cyclodextrin
DMSO - Dimethylsulfoxide
FDM - Freeze-drying microscope
SEM - Scanning electron microscope
LT-DSC - Low temperature differential scanning calorimetry
DSC - Differential scanning calorimetry
DVS Dynamic vapor sorption
TGA - Thermogravimetic analysis
GC - Gas chromatography
KF - Karl Fisher

"Compound 1, Form C" or "Form C" or "API" " in the Examples herein refers to polymorph Form C of 2-(4-chlorophenyl)-N-((2-(2,6-dioxopiperidin-3-yl)-1-oxoisoindolin-5-yl)methyl)-2,2-difluoroacetamide. "Compound 1, Form A" or "Form A" in the Examples herein refers to polymorph Form A of 2-(4-chlorophenyl)-N-((2-(2,6-dioxopiperidin-3-yl)-1-oxoisoindolin-5-yl)methyl)-2,2-difluoroacetamide. The physical and chemical properties of 2-(4-chlorophenyl)-N-((2-(2,6-dioxopiperidin-3-yl)-1-oxoisoindolin-5-yl)methyl)-2,2-difluoroacetamide are summarized in Table 1.

**Table 1: Summary of physical and chemical properties of 2-(4-Chlorophenyl)-N-((2-(2,6-dioxopiperidin-3-yl)-1-oxoisoindolin-5-yl)methyl)-2,2-difluoroacetamide**

| | |
|---|---|
| **Structure** | 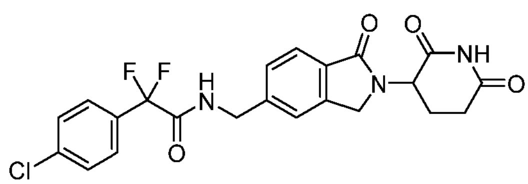 |
| **Molecular Formula** | $C_{22}H_{18}ClF_2N_3O_4$ |
| **Molecular Weight** | 461.85 |

(continued)

| Structure | 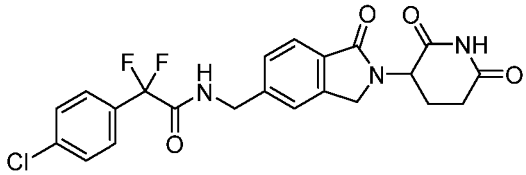 |
|---|---|
| **Log D** | cLogP = 2.18 (Log D not measured due to solubility) |
| **pKa** | cpKa = 10.66 (Not measured due to low stability above pH 7) |
| **Melting Point** | 234°C (Form C) |
| **Appearance** | White powder |
| **Solubility** | Practically insoluble in water (≤1 μg/ml across pH range of 1-8) |
| **Solid State Stability** | DS is physically stable under all storage conditions. |
| **Solution Stability** | DS is not stable in solution at pH of 5.0 or above. Hydrolysis is the major degradation pathway. |
| **Hygroscopicity** | Not hygroscopic |
| **Pharmaceutical Form** | Crystalline; Anhydrous; five polymorph forms |

**Example 1: Solvent Selection**

A solvent screen was conducted to identify suitable Class 3 solvents listed in the ICH (International Conference on Harmonisation) Q3C Guidance as the Class 3 solvents have a higher permitted daily exposure (PDE) level of 50 mg/day. A good solvent candidate was considered to have 1) good miscibility with water and 2) sufficiently low boiling point so that it can be easily removed during the lyophilization process. Compound 1 solubility was tested in a series of solvent mixtures with 20 mM pH 4.2 citrate buffer; the results are shown in Table 2.

**Table 2: Solubility of Compound 1 in solvent and buffer mixtures**

| Vehicle | t=24 Hr Solubility (mg/mL) |
|---|---|
| 20% Acetone: 80% Citrate Buffer | 0.015 |
| 40% Acetone: 60% Citrate Buffer | 0.19 |
| 60% Acetone: 40% Citrate Buffer | 1.48 |
| 80% Acetone: 20% Citrate Buffer | 4.82 |
| 100% Acetone | 1.69 |
| 20% Acetic acid: 80% Citrate Buffer | 0.020 |
| 40% Acetic acid: 60% Citrate Buffer | 0.25 |
| 60% Acetic acid : 40% Citrate Buffer | 1.67 |
| 80% Acetic acid : 20% Citrate Buffer | 6.13 |
| 100% Acetic acid | 4.09 |
| 20% 2-propanol: 80% Citrate Buffer | 0.018 |
| 40% 2-propanol : 60% Citrate Buffer | 0.25 |
| 60% 2-propanol: 40% Citrate Buffer | 0.61 |
| 80% 2-propanol : 20% Citrate Buffer | 0.79 |
| 100% 2-propanol | 0.13 |
| 20% 1-propanol : 80% Citrate Buffer | 0.016 |

(continued)

| Vehicle | t=24 Hr Solubility (mg/mL) |
|---|---|
| 40% 1-propanol: 60% Citrate Buffer | 0.24 |
| 60% 1-propanol : 40% Citrate Buffer | 0.61 |
| 80% 1-propanol: 20% Citrate Buffer | 0.82 |
| 100% 1-propanol | 0.13 |
| 20% Formic acid : 80% Citrate Buffer | 0.024 |
| 40% Formic acid : 60% Citrate Buffer | 0.22 |
| 60% Formic acid: 40% Citrate Buffer | 2.10 |
| 80% Formic acid : 20% Citrate Buffer | 19.18 |
| 100% Formic acid | 165 |
| 100% DMSO | 330 |

It was found that the drug solubility in most of the tested solvent systems is lower than 10 mg/mL except for that in 100% formic acid or 100% DMSO, which provide a drug solubility above 100 mg/mL. The objective was to achieve a high solubility in the solvent so that a small quantity of solvent is used during processing (and thus limited solvent removal is needed). Therefore, formic acid and DMSO were identified as two lead solvents for further formulation development.

A subsequent solubility test was done on the formic acid/DMSO mixtures of different ratios to evaluate if there was any synergy through using two solvents.

**Table 3: Solubility of Compound 1 in formic acid and DMSO mixtures**

| Vehicle | t= 1 Hr Solubility (mg/mL) | t= 1 day Solubility (mg/mL) | t= 2 day Solubility (mg/mL) |
|---|---|---|---|
| 100% FA | 143.5 | 158.6 | 156.3 |
| 80:20 FA:DMSO | 48.6 | 39.4 | 43.9 |
| 60:40 FA:DMSO | 26.1 | 14.5 | 14.2 |
| 40:60 FA:DMSO | 21.6 | 11.6 | 10.8 |
| 20:80 FA:DMSO | 92.8 | 75.6 | 71.2 |
| 100% DMSO | 330.0 | 330.0 | 330.0 |

As shown in Table 3, the solubilities of all the formic acid/DMSO mixtures were much lower than that of either the solvent alone. Therefore, it was decided that the API premix should be prepared in either formic acid or DMSO alone.

**Example 2: Solubilizer Selection**

Cyclodextrins (CD) are the most commonly-used complexing agents to increase the aqueous solubility of poorly water-soluble drug compounds. Among all cyclodextrin derivatives, only Hydroxypropyl-β-Cyclodextrin (HPβCD) and sulfobutyl ether beta-cyclodextrin (SBEβCD) have been used in approved parenteral products. Consequently, only these two cyclodextrin types were evaluated in this study.

As a first step, the drug solubility in aqueous solutions as a function of the concentration of either HPβCD (Kleptose® by Roquette) or SBEβCD (Dexolve® by Cyclolab) were measured at ambient temperature for the time point of 1, 2 and 6 days. The results are shown in Table 4.

**Table 4: Solubility of Compound 1 in aqueous solution containing Dexolve or Kleptose**

| CD Conc. (%w/w) | t=1 Day Solubility (μg/mL) | | t=2 Day Solubility (μg/mL) | | t=6 Day Solubility (μg/mL) | |
|---|---|---|---|---|---|---|
| | Dexolve | Kleptose | Dexolve | Kleptose | Dexolve | Kleptose |
| 1.0 | 2.0 | 3.6 | 1.8 | 2.0 | 0.6 | 0.9 |

(continued)

| CD Conc. (%w/w) | t=1 Day Solubility (μg/mL) | | t=2 Day Solubility (μg/mL) | | t=6 Day Solubility (μg/mL) | |
|---|---|---|---|---|---|---|
| | Dexolve | Kleptose | Dexolve | Kleptose | Dexolve | Kleptose |
| 2.5 | 7.7 | 9.6 | 7.8 | 7.6 | 3.8 | 5.3 |
| 5.0 | 17.6 | 28.5 | 17.2 | 17.1 | 11.9 | 11.9 |
| 10.0 | 43.0 | 46.6 | 41.2 | 40.6 | 33.9 | 32.9 |
| 20.0 | 90.0 | 102.1 | 87.9 | 96.4 | 91.6 | 110.0 |
| 40.0 | 283.0 | 324.1 | 327.2 | 351.0 | 303.7 | 362.5 |
| 60.0 | 397.0 | 418.5 | 401.9 | 448.7 | 526.1 | 719.8 |

In general, the solubility of Compound 1 in both types of CDs was comparable. The solubility of Compound 1 increased as the concentration of CD increased. When the CD concentration of 40% or higher was used, the solution became very viscous which likely impeded the kinetic solubility of the drug in the solution. It is generally difficult to lyophilize a solution containing > 25% CD. Therefore, the subsequent solubility studies were conducted at four CD concentration levels: 3, 9, 15 and 25% w/w. In addition to Kleptose® and Dexolve®, two other commercially available CD brands were evaluated, namely Captisol® by Ligand (SBEβCD) and a generic version of HP® CD provided by Acros Organics. The impact of solvent type and level on the drug solubility were also evaluated in this study. Formic acid or DMSO were added in various solvent to CD molar ratios to the solution (0.4:1, 1:1, 2.5:1, 5:1 and 10:1). Solubility data at 24 hr and 48 hr were collected; longer periods were not considered due to drug chemical stability risk. Accordingly, only 48 hr data are presented here. FIG. 30 shows the solubility of Compound 1 with different cyclodextrin brands and concentrations, different solvent types, and varied solvent to cyclodextrin ratios. It was found that the solubility of Compound 1 is affected predominantly by the concentration of CD in the solution, whereas the type and brand of CD had minimal impact on the solubility. With respect to the solvent type, Compound 1 showed a slightly higher solubility when formic acid, rather than DMSO, was added to the cyclodextrin solution. For both formic acid and DMSO, there was slight increase in the drug solubility with the increase of the solvent to cyclodextrin ratio. The effect of solvent on the drug solubility was minimal as opposed to the effect of CD concentration.

**Example 3: Buffer Selection**

The pH of the drug solution upon IV dosing is generally preferred to be in a range of 4 - 7, and or in a range of 5 - 7. A variety of pharmaceutically acceptable buffer systems including acetate, benzoate, citrate, lactate and tartrate were further considered with respect to their buffer capacity and their impact on the drug solubility. As acetate buffers can be sublimed during the lyophilization process and result in a pH shift, this buffer was subsequently removed from evaluation.

To evaluate alternate buffers, a series of drug solutions were prepared with Kleptose at 3% or 20%. Formic acid or DMSO were added to the solution at approximately 0.1% w/w corresponding to their API premix concentration. As a buffer strength of greater than 50 mM can cause irritation to the patient, benzoate, citrate, lactate and tartrate were evaluated at various buffer strengths ranging from 2 to 20 mM. 20 mM citrate, tartrate, benzoate or lactate buffer with an initial pH of 4.2 or 4.7 was also added to the solution. The final pH and the solubility of each solution were measured subsequently. The results are shown in Table 5 and Table 6.

**Table 5: pH values of solutions containing Kleptose, solvent, and different buffers**

| Strength (mM) | Buffer | pH | 3% Kleptose, FA | 3% Kleptose, DMSO | 20% Kleptose, FA | 20% Kleptose, DMSO |
|---|---|---|---|---|---|---|
| 20 | Citrate | 4.2 | 3.6 | 4.3 | 3.7 | 4.5 |
| 20 | Citrate | 4.7 | 4.0 | 4.8 | 4.1 | 5.0 |
| 10 | Tartrate | 4.2 | 3.8 | 4.1 | 3.9 | 4.3 |
| 20 | Benzoate | 4.2 | 3.9 | 4.6 | 3.8 | 5.4 |
| 20 | Benzoate | 4.7 | 4.0 | 5.5 | 3.9 | 5.9 |
| 20 | Lactate | 4.2 | 3.5 | 4.2 | 3.6 | 4.4 |

(continued)

| Strength (mM) | Buffer | pH | 3% Kleptose, FA | 3% Kleptose, DMSO | 20% Kleptose, FA | 20% Kleptose, DMSO |
|---|---|---|---|---|---|---|
| 20 | Lactate | 4.7 | 3.6 | 4.8 | 3.8 | 4.9 |
| Water | | | 2.7 | 7.4 | 2.8 | 7.3 |

**Table 6: Solubility of Compound 1 in solutions containing Kleptose, solvent, and different buffers**

| Strength (mM) | Buffer | pH | 3% Kleptose, FA (μg/mL) | 3% Kleptose, DMSO (μg/mL) | 20% Kleptose, FA (μg/mL) | 20% Kleptose, DMSO (μg/mL) |
|---|---|---|---|---|---|---|
| 20 | Citrate | 4.2 | 8.4 | 8.9 | 84.9 | 83.4 |
| 20 | Citrate | 4.7 | 9.5 | 9.1 | 77.0 | 90.0 |
| 10 | Tartrate | 4.2 | 10.9 | 9.7 | 79.8 | 82.5 |
| 20 | Benzoate | 4.2 | 2.1 | 2.6 | 58.9 | 62.0 |
| 20 | Benzoate | 4.7 | 3.8 | 6.7 | 72.2 | 67.4 |
| 20 | Lactate | 4.2 | 9.6 | 9.8 | 75.9 | 89.9 |
| 20 | Lactate | 4.7 | 9.7 | 10.0 | 87.3 | 88.1 |
| Water | | | 10.8 | 8.5 | 99.0 | 76.6 |

It was observed that at the same Kleptose level, regardless of the solvent type, all the buffered solutions had comparable solubility except for the benzoate solution which exhibited a much lower solubility. In addition, in the presence of formic acid, the drug solubility in the buffered solutions were slightly lower than that of the unbuffered solution. In contrast, the solubility of the buffered solutions in the presence of DMSO were slightly higher than that of the unbuffered. This implies that different types of buffer salt may have different molecular interactions with the solvent molecules which may consequently impact the drug complexation into CD cavities. The solutions containing DMSO exhibited a well maintained pH at 4.2 or 4.7 with all the tested buffers except for benzoate. However, all the tested solutions containing formic acid showed a pH below 4 regardless of the buffer type and initial pH. In order to maintain the final pH of bulk solution above 4 in the presence of formic acid, it is likely that a buffer pH higher than 4.7 is required to withstand the change in pH. As a buffer offers the best buffering capacity when the pH is close or equals to its pKa and since benzoate, lactate and tartrate have pKa's of 4.2, 3.86 and 4.4, respectively, these were considered to be very unlikely to be able to support pHs greater than 4. As a result, benzoate, lactate and tartrate buffers were removed from consideration. Citrate has three pKa's, 3.13, 4.76 and 6.39, yielding a good buffering capacity at high pH values. Therefore, citrate buffer was selected in the further formulation development.

In the subsequent study, 10% Dexolve was dissolved in citrate buffers of various pHs and strengths, along with an amount of formic acid equating to an API premix concentration of 150 mg/mL. FIG. 31 shows the final solution pH as a function of the citrate buffer strength ranging from 2 to 20 mM and initial buffer pH ranging from 4.2 to 5.3. The pH of the solution increased with buffered solution pH and strength as expected. When a 20mM citrate buffer at pH 5.3 was used, the solution pH was able to maintain a final pH of 4.3 after addition of the formic acid. As a result, when formic acid was used as the solvent, a 20 mM citrate buffer at pH 5.3 was recommended to be added to the formulation to maintain the reconstituted solution pH above 4.

Example 4: **Thermal Analysis of Lyophilization Formulations**

In this study, a series of thermal analysis were conducted with the freeze drying microscope (FDM) and the low temperature differential scanning calorimetry (LT-DSC) to determine the collapse temperature and the Tg' of the various bulk solution formulations containing different CD types, CD concentrations, solvent types, solvent concentrations, and buffer strengths. In general, lyophilization cycle time is reduced and good cake stability during lyophilization is obtained as the Tg' and collapse temperature of the formulation system increase.

Table 7 summarizes the thermal characterization results obtained as part of this study, along with exemplary formulations disclosed in US Publication No. 2017-0196847.

**Table 7: Collapse temperature of cyclodextrin-based formulations**

| CD Type | % CD | Solvent Type | % Solvent (V/V) () | Citrate Buffer strength (mM) | Buffer target pH | Collapse Temp (°C) | Tg' (°C) | API Premix Concentration |
|---|---|---|---|---|---|---|---|---|
| Kleptose | 25 | | 0 | 2 | 4.2 | -8.4 | -8.9 | |
| Kleptose | 15 | | 0 | 2 | 4.2 | -8.6 | -9 | |
| Kleptose | 9 | | 0 | 2 | 4.2 | -10.2 | 10.6 | |
| Kleptose | 3 | | 0 | 2 | 4.2 | -14.7 | 15.2 | |
| Kleptose | 10 | | 0 | 2 | 4.2 | -7.7 | -8.2 | |
| Kleptose | 10 | | 0 | 5 | 4.2 | -8.7 | -9.4 | |
| Kleptose | 10 | | 0 | 10 | 4.2 | -9.6 | 10.1 | |
| Kleptose | 10 | | 0 | 20 | 4.2 | -10.7 | 11.5 | |
| Kleptose | 10 | DMSO | 0.06 | 20 | 4.2 | -10.7 | 12.1 | 220 mg/mL |
| Kleptose | 10 | DMSO | 0.07 | 20 | 4.2 | -8.4 | -9 | 170 mg/mL |
| Dexolve | 10 | DMSO | 0.06 | 20 | 4.2 | -23.2 | 24.1 | 220 mg/mL |
| **CD Type** | **% CD** | **Solvent Type** | **% Solvent** | **Citrate Buffer strength (mM)** | **Buffer target pH** | **Collapse Temp (°C)** | **Tg' (°C)** | **API Premix Concentration** |
| Kleptose | 10 | Formic Acid | 0.08 | 20 | 4.7 | -8.2 | -8.9 | 150 mg/mL |
| Kleptose | 10 | Formic Acid | 0.125 | 20 | 4.7 | -11.1 | 12.2 | 100 mg/mL |
| Dexolve | 10 | Formic Acid | 0.08 | 20 | 4.7 | -22.9 | 23.4 | 150 mg/mL |
| Captisol | 3 | DMA | 0.17 | 20 | 4.2 | -30 | 36.6 | 75 mg/mL |
| Kleptose | 3 | DMA | 0.1 | 20 | 4.2 | -18 | 20.4 | 120 mg/mL |
| Captisol | 3 | | 0 | 0 | | -26.5 | 25.5 | |
| Kleptose | 3 | | 0 | 0 | | -6.6 | -9.6 | |

The last four formulations disclosed in Table 7 are described in US Publication No. US 2017-0196847.

As shown in Table 7, Tg's of all the tested formulations measured by LT-DSC were very close to their collapse temperatures determined by FDM. In general, Tg's were 1-2°C lower than their corresponding collapse temperatures. The collapse temperature was increased from -14.7°C to -8.6°C with the increase of Kleptose concentration from 3% to 15% and then remained unchanged when the Kleptose concentration was increased from 15% to 25%. At the same Kleptose level of 10%, the collapse temperature was decreased from -7.7°C to -10.7°C with the increase of buffer strength from 2 mM to 20 mM. Solvent type and solvent level showed minimal impact on the collapse temperature with no obvious trends observed. The type of cyclodextrin turned out to be the variable that had the most significant impact on the collapse temperature, with SBEβCD (Captisol® or Dexolve®) formulations exhibiting the collapse temperatures lower than their HPβCD (Kleptose®) counterparts. In comparison with the formulation disclosed in US Publication No. 2017-0196847, which has a collapse temperature of -18°C, the formulations disclosed herein, which contained 10% Kleptose, presented a higher collapse temperature of -8.2°C to -11.1°C. This indicated that the lyophilization cycle which has a primary drying shelf temperature of -16°C is very conservative for the formulations disclosed herein. Nevertheless, the same lyophilization cycle was used as a starting point to minimize processing-related risks, with an option of modifying it further as needed.

**Example 5: Formulation and Process Evaluation of Formulation Ia**

A lab-scale batch was prepared at 3-kg batch size having the formulation composition shown in Table 8.

**Table 8: Formulation composition of 3-kg trial batch**

| Material | Composition (mg/mL) | Composition (mg/vial)[b] |
|---|---|---|
| Compound 1 | 0.125 | 1.05 |
| Kleptose HPB | 30 | 252.00 |
| Citric Acid Anhydrous | 2.21 | 18.56 |
| Sodium Citrate anhydrous | 2.19 | 18.40 |
| DMSO | 1.02[a] | 8.56[a] |
| Purified Water | q.s. to 1000 | Removed upon drying |
| Comment | Equivalent to 220 mg/mL API premix | |
| a: removed upon drying; b: with 5% overfill (8.4 mL/vial fill volume) | | |

Compound 1 was prepared in DMSO premix first at 220 mg/mL and then added drop-wise to the buffered Kleptose solution. Upon completion of the compounding, the solution was filtered through a 0.22 μm PVDF filter and filled into 20 cc Type I glass vials with a fill weight of 8.4 g/vial, and then loaded to the lyophilizer under the lyophilization cycle cycle parameters shown in Table 9.

Samples were pulled after primary drying and during the secondary drying to understand solvent level as a function of drying time. The analytical results of the finished drug product are shown in Table 10. The residual DMSO level was about 6 mg/vial after 24 hours of secondary drying at 67°C, amounting to roughly 29% removal (initial level: ~ 8.6 mg/mL). It was noted that bulk of the solvent removal was during primary drying, with a minimal reduction in solvent level during secondary drying. The appearance of the lyophilized cakes and the reconstituted solutions were noted, and found to be acceptable.

Table 9: Lyophilization cycle parameters

| Step | Shelf Temp. Setpoint (°C) | SoakTime (hours) | Ramping Rate (°C/ hour) | Pressure Setpoint |
|---|---|---|---|---|
| Product Loading/F reezing | 5 | 2 | | Evac. To 12 psia to ensure chamber is airtight |
| | | | 30 | |
| Freezing | -50 | 3 | | |
| | | | 30 | |
| Primary Drying | -16 | 70 | | 140 microns |
| | | | 30 | 140 microns |
| Secondary Drying | 67 | 24 | | 140 microns |
| Stoppering | 25 | | | 14.7 PSIA |

**Table 10: Characterization of drug products of trial batch**

| Assay (% LC) | Related Impurities | Residual DMSO | Moistu re content | Recon time | Recon appearance | pH |
|---|---|---|---|---|---|---|
| 96.6 | ND | 6.36 mg/vial (upon completion of PD); 6.44 mg/vial (after 6 hr of SD); 6.05 mg/vial (after 24 hr of SD) | 0.15% | <1 min (with 2 mL WFI) | Clear and colorless solution | 4.1 |
| Note: PD=primary drying; SD=secondary drying | | | | | | |

The assay of the lyophilized cake was noted to be at the low end, with the actual assay value of 91.9%, taking into account 5% overfill. With a residual solvent of ~ 6 mg/mL, this formulation could potentially support a dose up to 8

mg/day (as described earlier, PDE level for DMSO is 50 mg/day).

Based on the above study results, a few additional trial batches of the same formulation were prepared at 1-kg lab scale for compounding only to evaluate the potential risks of drug precipitation. The formulation information and the assay results are shown in Table 11. The first three trial batches were not successful with the low assay at the beginning and the decreasing assay over time within 8 hours of storage at ambient condition. The precipitated drug particles were visually observed at the bottom of the container after 4 hours of storage. In the subsequent three trial batches, the compounding process was modified with the reduced amount of initial water charge, the reduced dispensing volume of API premix, and the moderate mixing during API premix addition. As a result, the initial assays of the last three batches were greatly improved. However, significant assay drop was observed after 19 hours of storage at ambient condition for all the three batches. Collectively, the data indicated that the formulation is not physically stable and the risk of drug precipitation is quite high. Therefore, the 'solvent swap' formulation using 3% CD level was not considered for further evaluation.

**Table 11: Bulk solution stability evaluation of the 'solvent swap' formulation (3% CD and 135 mg/mL API in DMSO)**

| Sample | Compounding Process | t=0 Assay (% of target) | t= 4 hr Assay (% of target) | t=8 hr Assay (% of target) |
|---|---|---|---|---|
| 1-A | 96% initial water charge; 50 μL API premix addition; 500 rpm mixing | 84.3 | 78.4 | 68.6 |
| 2-A1 | 96% initial water charge; 25μL API premix addition; 900 rpm mixing | Top: 88.3<br>Bottom: 89.7 | Top: 81.6<br>Bottom: 84.7 | Top: 72.3<br>Bottom: 73.9 |
| 2-A2 | 96% initial water charge; 25μL API premix addition; 900 rpm mixing | Top: 89.2<br>Bottom: 89.8 | Top: 85.0<br>Bottom: 82.3 | Top: 75.2<br>Bottom: 71.0 |
| 3-A1 | 50% initial water charge; 25μL API premix addition; 500 rpm mixing | Top: 98.6<br>Bottom: 98.9 | Not Tested | Top*: 70.8<br>Bottom*: 72.5 |
| 3-A2 | 80% initial water charge; 25μL API premix addition; 750 rpm mixing | Top: 99.8<br>Bottom: 99.9 | Not Tested | Top*: 75.0<br>Bottom*: 64.7 |
| 3-A3 | 96% initial water charge; 25μL API premix addition; 600 rpm mixing | Top: 99.4<br>Bottom: 98.3 | Not Tested | Top*: 64.0<br>Bottom*: 73.6 |
| *: the data was received for T=19 hr time point | | | | |

The following three 1-kg trial batches (1-B, 1-C and 1-D) increased the Kleptose level to 10-20% based on the previous solubility study. The formulation compositions of each bulk solution are described in Table 12. With the drug concentration and buffer compositions being constant, the only variables of the three formulations were the Kleptose level and the initial DMSO charge corresponding to the API premix concentration. The lyophilization cycle parameters are shown in Table 13; a lower secondary drying temperature of 50°C was used.

**Table 12: Formulation compositions of 1-kg trial batches**

| Lot No. | 1-B (mg/vial)[a] | 1-C (mg/vial)[a] | 1-D (mg/vial)[a] |
|---|---|---|---|
| Compound 1 | 1.05 | 1.05 | 1.05 |
| Kleptose HPB | 840 | 840 | 1680 |
| Citric Acid Anhydrous | 18.56 | 18.56 | 18.56 |
| Sodium Citrate anhydrous | 18.40 | 18.40 | 18.40 |
| DMSO | 6.80 mg/vial[b] | 5.25 mg/vial[b] | 5.25 mg/vial[b] |
| Purified Water | Removed upon drying | Removed upon drying | Removed upon drying |

(continued)

| Lot No. | 1-B (mg/vial)[a] | 1-C (mg/vial)[a] | 1-D (mg/vial)[a] |
|---|---|---|---|
| Comment | Equivalent to 170 mg/mL premix | Equivalent to 220 mg/mL premix | Equivalent to 220 mg/mL premix |
| a: 5% overfill; b: Removed upon drying. The numbers in the table represent the initial solvent content. | | | |

**Table 13: Lyophilization cycle parameters of 1-kg trial batch**

| Step | Shelf Temp. Setpoint (°C) | Soak Time (hours) | Ramping Rate (°C/hour) | Pressure Setpoint |
|---|---|---|---|---|
| Product Loading/Freezing | 5 | 2 | | Evac. To 12 psia to ensure chamber is airtight |
| | | | 30 | |
| Freezing | -50 | 4 | | |
| | | | 30 | |
| Primary Drying | -16 | 70 | | 140 microns |
| | | | 30 | 140 microns |
| Secondary Drying | 50* | 12 | | 140 microns |
| Stoppering | 25 | | | 14.7 PSIA |
| *50°C was used in LTI batches; 60°C was used in BSP batches | | | | |

The post-filtration bulk solutions of the three batches were stored at ambient condition for up to 8 hours. The assay of each batch was measured at t=0, 4, and 8 hours. The results are shown in Table 14. The bulk solutions of the three batches appeared to be clear upon visual inspection and the assay remained stable for the duration of 8 hours. The lyophilized cakes of the three batches were analyzed and the results are shown in Table 15. The assays of the three batches were all ~ 100% and the individual degradants/impurities in each formulation were all below 0.1%. The residual DMSO level of these three batches went down to about 4 mg/vial, showing ~ 25 - 40% solvent removal.

Table 14: Bulk solution stability evaluation of the trial batch

| | Assay (% of target) | | |
|---|---|---|---|
| **Lot No.** | **t=0** | **t=4 hr** | **t=8 hr** |
| **1-B** | 97.8 | 97.5 | 97.8 |
| **1-C** | 99.0 | 98.7 | 99.2 |
| **1-D** | 104.3 | 103.9 | 104.5 |

**Table 15: Characterization of the DMSO-based formulations**

| Test | 1-B | 1-C | 1-D |
|---|---|---|---|
| Appearance | cake | Cake | cake |
| Color | white | white | white |
| Foreign Matter | conform | conform | conform |
| Recon Time | 59 seconds | 73 seconds | Cannot be reconstituted |
| Recon appearance | Clear Solution | Clear Solution | Cloudy solution |
| Assay (% LC) | 100.5 | 106.2 | 110.0 |

(continued)

| Test | 1-B | 1-C | 1-D |
|---|---|---|---|
| Related Impurities (%) | RRT 0.46 <0.05<br>RRT 0.47 <0.05<br>RRT1.28: 0.06 | RRT 0.46 <0.05<br>RRT 0.47 <0.05<br>RRT1.28: 0.05 | RRT 0.46 <0.05<br>RRT 0.47 <0.05<br>RRT1.28: 0.09 |
| Residual DMSO | 4.0 mg/vial | 3.8 mg/vial | 3.9 mg/vial |
| pH | 4.4 | 4.4 | N/A |

In addition to the above batches, two replicate trial batches at 1-kg batch size, 3-B 1 and 3-B2, were also prepared with the same formulation as Batch 1-B (10% Kleptose, 170 mg/mL DMSO premix) for compounding only to evaluate the hold time of the bulk solutions. The post-filtration bulk solutions were stored at ambient condition for the duration of 19 hours. The solution remained clear with no visible undissolved particles and the assay remained stable as shown in Table 16. Similarly, two other replicated trial batches at 1-kg batch size, 4-C1 and 4-C2, were prepared with the same formulation as Batch 1-C (10% Kleptose, 220 mg/mL DMSO premix) for compounding only to evaluate the hold time of the bulk solutions. The post-filtration bulk solutions were stored at ambient condition for the duration of 8 hours. The solution remained clear with no visible undissolved particles and the assay remained stable as shown in Table 17. The compounding studies confirmed that the bulk solutions with 10% Kleptose in the formulation can provide sufficient stability during manufacturing timeframe. Consequently, 1-C was selected as the final formulation, with 10% CD level.

Table 16: **Bulk solution stability evaluation of the trial** batch

| | Assav (% of Target) | |
|---|---|---|
| | t=0 | t=19 hr |
| 3-B1 Top | 100.1 | 104.0 |
| 3-B1 Bottom | 100.8 | 104.3 |
| 3-B2 Top | 101.3 | 104.7 |
| 3-B2 Bottom | 101.7 | 104.8 |

Table 17: Bulk solution stability evaluation of the trial batch

| | Assay (% of Target) | | |
|---|---|---|---|
| | t=0 | t=4 hr | t=8 hr |
| 4-C1 Top | 102.0 | 102.2 | 102.2 |
| 4-C1 Bottom | 101.9 | 102.2 | 102.2 |
| 4-C2 Top | 102.5 | 102.9 | 102.9 |
| 4-C2 Bottom | 103.1 | 102.9 | 102.9 |

With above results on solution stability and residual solvent levels, more efforts were taken to reduce the residual DMSO level further by decreasing the initial DMSO amount in the formulation and using different types of cyclodextrin. Two 10-L trial batches were prepared at with the increased API-DMSO premix concentration from 220 mg/mL to 270 mg/mL (with 1 mL rinse using DMSO). Both batches had the same formulation compositions (10% CD and 20 mM pH 5.3 citrate buffer) but used different type of cyclodextrin, as shown in Table 18. The lyophilization cycle parameters and the lyophilized drug product properties are exhibited in Table 19 and Table 20, respectively. Regardless of the CD type, both batches obtained the same residual DMSO level of 4.6 mg/vial, corresponding to 11% removal of initial DMSO charge of 5.2 mg/vial. As no major improvement in residual solvent reduction was achieved from these two trial runs compared to previous trials, this approach was not pursued anymore and the API-DMSO premix concentration was fixed as 220 mg/mL in the later process development work.

**Table 18: Formulation compositions of DMSO-based trial batches**

| Ingredient | 5-F1 (mg/vial)[a] | 6-F1 (mg/vial)[a] |
|---|---|---|
| Compound 1 | 1.05 | 1.05 |
| Citric acid | 9.41 | 9.41 |
| Sodium citrate | 30.74 | 30.74 |
| Captisol | 840 | / |
| Kleptose | / | 840 |
| DMSO | 5.20[b] | 5.20[b] |
| WFI | Removed upon drying | Removed upon drying |
| Comments | API Premix prepared at 270 mg/mL DMSO | API Premix prepared at 270 mg/mL DMSO |
| a: with 5% overfill (8.4 mL/vial fill volume); b: Partially removed upon drying. The numbers in the table represent the initial solvent content. | | |

**Table 19: Lyophilization cycle parameters of trial batches**

| Step | Shelf Temp. Setpoint (°C) | Soak Time (hours) | Ramping Rate (°C/ hour) | Pressure Setpoint |
|---|---|---|---|---|
| Product Loading/Freezing | 5 | 2 | | Evac. To 12 psia to ensure chamber is airtight |
| | | | 30 | |
| Freezing | -50 | 4 | | |
| | | | 30 | |
| Primary Drying | -16 | 70 | | 140 microns |
| | | | 30 | 140 microns |
| Secondary Drying | 60 | 12 | | 140 microns |
| Stoppering | 25 | | | 14.7 PSIA |

**Table 20: Characterization of the DMSO-based lyophilized trial batches**

| Test | 5-F1 | 6-F1 |
|---|---|---|
| Appearance | cake | cake |
| Color | white | white |
| Foreign Matter | conform | conform |
| Assay (% LC) | 100.5 | Not Tested |
| Related Impurities | <0.05% | Not Tested |
| Residual DMSO (mg/vial) | 4.6 | 4.6 |
| Recon Solution pH | 5.2 | 5.2 |

An additional trial batch, 7-Dex1, was made with 10% Dexolve and 220 mg/mL DMSO premix at the 500-mL lab scale, using the same formulation (except CD) and process as the Batch 1-C. The bulk solution was stored at ambient conditions for up to 24 hours and no drug precipitation was observed by visual inspection. The test result of the finished drug product was included in Table 21 to give a head-to-head comparison with that of Batch 1-C. The comparable drug product characteristics between the two batches suggested that the current lyophilization cycle may be applicable to the SBEβCD-based formulation as well although the SBEβCD-based lyophilie had a much lower collapse temperature

than its Kleptose-based counterpart. Even though comparable results were obtained for both Dexolve and Kleptose-based formulations, Kleptose was selected as the cyclodextrin of choice due to more experience with this type of formulation and having better collapse temperature profile than Dexolve.

**Table 21: Characterization of the Dexolve and DMSO-based formulation**

| Test | 7-Dex1 |
|---|---|
| Appearance | cake |
| Color | white |
| Foreign Matter | conform |
| Recon Time | 82 seconds |
| Recon appearance | clear Solution |
| Assay (% LC) | 112.4 |
| Related Impurities (%) | Total impurities <0.07 |
| Residual DMSO (mg/vial) | 4.7 |
| pH | 4.0 |

**Example 6: Reconstitution Scheme of Formulation Ia**

As the composition of DMSO-based formulation Ia was finalized, the reconstitution scheme was evaluated to ensure that the reconstituted solution presented acceptable pH and osmolality for IV administration. Preferably, the pH should stay in the range of 4-7 and the osmolality should stay as close to the human plasma osmolality of 285-295 mOsm/kg as possible. Three most commonly used commercially available IV fluids including water for injection (WFI), normal saline (NS), and 5% Dextrose (D5W) were evaluated. The osmolality and pH values of the reconstituted solutions with varied reconstitution schemes are shown in Table 22. It was found that either NS or D5W alone with a volume of 5-15 mL provided an osmolality value higher than 370 mOsm/kg. Among all the tested vehicles, only 4 mL WFI, or a mix of 4 mL WFI with the equal volume of NS or D5W, was able to provide a reconstituted solution with an osmolality value of 260-280 mOsm/kg. The pHs of all the reconstituted solutions were in the range of 4.2-4.5. To make the reconstitution scheme as simple as possible, 4 mL WFI was considered to be the most favorable option. In the end, the reconstitution vehicle volume was adjusted to 3.8 mL so that the concentration of the reconstituted solution is at a round number of 0.25 mg/mL with the actual drug loading of 1.05 mg/vial (with 5% overfill) and the final reconstituted solution volume of 4.2 mL/vial.

**Table 22: Osmolality and pH measurement of reconstituted solutions of formulation Ia**

| Diluent 1 | Diluent 1 Vol (mL) | Diluent 2 | Diluent 2 Vol (mL) | Osmolality (mOsm/kg) | pH |
|---|---|---|---|---|---|
| WFI | 3 | / | / | 357 | 4.4 |
| WFI | 4 | / | / | 260 | 4.4 |
| WFI | 5 | / | / | 205 | 4.4 |
| D5W | 5 | / | / | 530 | 4.5 |
| D5W | 15 | / | / | 369 | 4.3 |
| NS | 5 | / | / | 512 | NT |
| NS | 10 | / | / | 404 | NT |
| NS | 15 | / | / | 367 | 4.0 |
| WFI | 3 | NS | 3 | 327 | 4.3 |
| WFI | 4 | NS | 4 | 282 | 4.2 |
| WFI | 5 | NS | 5 | 252 | 4.2 |
| WFI | 4 | D5W | 4 | 281 | 4.4 |

(continued)

| Diluent 1 | Diluent 1 Vol (mL) | Diluent 2 | Diluent 2 Vol (mL) | Osmolality (mOsm/kg) | pH |
|---|---|---|---|---|---|
| WFI | 5 | D5W | 5 | 246 | 4.4 |

**Example 7: Process Scale up of Formulation Ia**

Once the formulation Ia feasibility was demonstrated in the lab trials, an engineering batch was manufactured at the scale of 8.5 L (Batch A). The same lyophilization cycle as exhibited in Table 13 as used. To assure a successful scale up of the Formulation Ia, a thorough risk assessment was carried out on critical operational procedures of the process disclosed in US Publication No. 2017-0196847. The identified risks and the implemented process improvements are summarized in Table 23 below.

**Table 23: Process improvements implemented in formulation Ia scale-up batches**

| | process disclosed in US 2017-0196847 | **Risks** | **Changes/Mitigation plan** |
|---|---|---|---|
| **API premix preparation** | Mixing speed not specified | Lot-to-lot variability | Mixing speed set at 300±25 rpm |
| | Mixing time NLT 5 min | Insufficient mixing | Mixing time NLT 20 min |
| **API premix addition** | Manual dropper, drop wise | Operator errors / variability | Electronic pipette, 50 μL per addition, to the center of vortex |
| | No rinse after premix addition | Potential drug loss due to API residual | Rinse premix container with 1 mL DMSO after premix addition |
| **Q.S. step** | QS step (~1440g water addition with no mixing) | Local instability => Drug crystallization | Increase initial water charge to minimize QS; QS expected < 50g |
| **After Q.S.** | Mixing speed not specified | Lot-to-lot variability | Mixing speed set at 250-320 rpm |
| | Sampling for final pH check | Extra time spent prior to filtration | pH check step removed |
| **Filtration** | Millipak 100, 0.45 μm filter used in prefiltration | Potential drug loss due to filter absorption | Millipak 40, 0.45 μm filter used in prefiltration |
| **Filling** | Purge one vial if filling is halted for extended period of time | Potential drug precipitation on filling needle | Purge one vial if filling is halted for 5 minutes or longer, and at the beginning of each tray |
| **Lyophilization** | Freezing hold time 3 hrs | Incomplete freezing | Freezing hold time 4 hrs |
| | Thermocouple inserted during lyophilization cycle | Potential sterility issue | Thermocouple removed during lyophilization cycle |
| | Condenser atypical spike during freezing | Poor freezing | Maintenance check and temp mapping beforehand |
| **Sampling & Labeling** | Beg (Tray 1), Mid (Tray 2), End (Tray 4), no sample pulled from Tray 3 | Incomplete product information from various tray locations | Sampling and labeling from each of the four trays |

The release testing results of the batch are shown in Table 24.

**Table 24: Batch analysis results of formulation Ia from scale-up batches**

| Test | Formulation Ia scale-up batches |
|---|---|
| In Process Assay Preparation Tank (% target) | 100.5 |
| In Process Assay Receiving Tank (% target) | 101.1 |
| Appearance | cake |

(continued)

| Test | Formulation Ia scale-up batches |
|---|---|
| Color | white |
| Foreign Matter | conform |
| Water Content | 0.04% |
| Recon Time | 32 sec |
| Particulate Matter | 2 particles per vial ≥ 10 micron, 0 particles per vial ≥ 25 micron |
| Assay (% LC) | 104.2 |
| Related Impurities | Individual: 0.06% Total:0.06% |
| Content Uniformity | Yes (AV=1.2) |
| Osmolality | 294 mOsm/kg |
| Bacterial Endotoxin | pass |
| Sterility | pass |
| Residual Solvents | 5.6 mg/vial |
| pH | 4.4 |

Following process improvements summarized in Table 25 below were implemented in further scale up batches:

**Table 25: Process improvements implemented in Formulation Ia scale-up batches**

| | **Batch A** | **Risks** | **Proposed changes/ mitigation plan** |
|---|---|---|---|
| **Compounding** | Use a 6 cm stir bar for mixing | Insufficient mixing | Use a 12 cm stir bar for mixing |
| | Use a 15 L narrow neck jacketed glass vessel | Heat fluid made visual check of solution clarity difficult; Narrow neck made API premix addition difficult | Use a 20 L broad neck unj acketed glass vessel |
| **API premix addition** | Electronic pipette, 100 µL dispensing volume, to the center of vortex | Drug precipitation | Electronic pipette, 50 µL dispensing volume, to the center of vortex |
| | No rinse after premix addition | Potential drug loss due to high concentrated API residual | Rinse premix container with 1 mL DMSO after premix addition |
| **Q.S. step** | QS step (~1440g water addition with no mixing) | Local instability => Drug crystallization | Increase initial water charge to minimize QS; QS expected < 50g |
| **Filtration** | Three Millipak 100, 0.22 µm filters used in prefiltration | Potential drug loss due to filter absorption | One Millipak 40, 0.45 µm and two Millipak 20, 0.22 µm filters |
| **Lyophilization** | Freezing hold time 3 hrs | Incomplete freezing | Freezing hold time 4 hrs |
| | Secondary drying at 50°C for 12 hours | Higher residual solvent | Secondary drying at 60°C for 12 hours |

The batch analysis results of exemplary batches are shown in Table 26.

Table 26: Batch analysis results of formulation **Ia**

| Test | Batch 1 | Batch 2 | Batch 3 |
|---|---|---|---|
| In Process Assay_ Preparation Tank (% target) | 98.6 | 99.6 (top); 99.8 (bottom) | 100.1 (top); 100.2 (bottom) |
| In Process Assay Receiving Tank (% target) | 98.8 | 99.8 (top); 99.8 (bottom) | 99.4 (top); 99.8 (bottom) |
| Appearance | Cake and powder (broken cake) | Cake and powder (broken cake) | Cake and powder (broken cake) |
| Color | white | white | white |
| Foreign Matter | conform | conform | conform |
| Water Content | 0.1% | NT | 0.1% |
| Recon Time | 29 sec | 36 sec | 58 sec |
| Particulate Matter | 14 particles per vial ≥ 10 micron, 1 particles per vial ≥ 25 micron | NT | 0 particles per vial ≥ 10 micron, 0 particles per vial ≥ 25 micron |
| Assay | 105.5 | NT | 104.2 |
| Related Impurities | Individual: <0.05% Total: <0.05% | NT | Individual: <0.05%; Total: <0.05% |
| Content Uniformity | Yes (AV= 4.8) | NT | Yes (AV=0.8) |
| Osmolality | 303 mOsm/Kg | NT | 274 mOsm/kg |
| Bacterial Endotoxin | NT | NT | pass |
| Sterility | NT | NT | pass |
| Residual Solvents | 6.7 mg/vial | NT | 5.7 mg/vial |
| pH | 4.4 | 4.3 | 4.4 |
| NT = Not Tested | | | |

Example 8: **Formulation and Process Evaluation of Formulation Ib**

Two trial batches, 1-Dex2 and 1-Dex3, were prepared at a 500-mL lab scale on formulations containing formic acid as the co-solvent and 10% Dexolve as the cyclodextrin. Dexolve was used in both batches as it had demonstrated comparable formulation performance with Kleptose in the previous trials. As the drug solubility in formic acid is about 170 mg/mL, the API premix in formic acid was prepared at a concentration of 150 mg/mL (equivalent to 8.54 mg/vial) and 100 mg/mL (equivalent to 12.81 mg/vial) respectively in the two batches. The citrate buffer pH was increased from 4.2 to 5.3 in order to render the reconstituted solution with a pH above 4.0. The lyophilization cycle stayed the same as used in the formulation disclosed in US Publication No. 2017-0196847. The formulation composition of the two batches are shown in Table 27. The analytical results of the finished drug products are s hown in Table 28.

Table 27: **Formulation compositions of the lab scale formic acid based trial** batches

| Ingredient | 1-Dex2 (mg/vial)[a] | 1-Dex3 (mg/vial)[a] |
|---|---|---|
| Compound 1 | 1.05 | 1.05 |
| Dexolve-7 | 840 | 840 |
| Citric Acid Anhydrous | 9.41 | 9.41 |
| Sodium Citrate anhydrous | 30.74 | 30.74 |
| Formic Acid | 8.54[b] | 12.81[b] |

(continued)

| Ingredient | 1-Dex2 (mg/vial)[a] | 1-Dex3 (mg/vial)[a] |
|---|---|---|
| Purified Water | Removed upon drying | Removed upon drying |
| Comment | Equivalent to 150 mg/mL premix | Equivalent to 100 mg/mL premix |
| a: 5% overfill<br>b: Partially removed upon drying. The numbers in the table represent the initial solvent content, including 1 mL rinse | | |

Table 28: Characterization of the lyophilized drug products of formic acid based trial batches

| Test | 1-Dex2 | 1-Dex3 |
|---|---|---|
| Appearance | cake | Cake |
| Color | white | white |
| Foreign Matter | conform | conform |
| Recon Time | 85 seconds | 96 seconds |
| Recon appearance | Clear Solution | Clear Solution |
| Assay | 109.4 | 107.4 |
| Related Impurities (%) | 0.05% | 0.09% |
| Residual Formic Acid | 5.6 mg/vial (34% removal) | 7.9 mg/vial (38% removal) |
| pH | 4.5 | 4.4 |

As formic acid has a boiling point of 100.8°C, much lower than that of DMA (165°C) or DMSO (189°C), it was supposed to be more easily removed during freeze drying. The reduction rate of formic acid was found to be only approximately 35%. A further analysis indicated that this was likely due to sodium formate formation. Since formic acid has a pKa of 3.75, which is close to the bulk solution pH, formic acid can disassociate into its ionic form. Now, in the presence of buffers, these ions can react with the free metal ions to form a more stable product. In this case, sodium citrate is used a part of the buffer system, and thus it can lead to the formation of sodium formate. Once sodium formate is formed, it can be very hard to remove by sublimation as it has a very high melting point (253°C). In order to remove the formic acid efficiently, the key is to avoid formation of sodium formate and have formic acid in its native form. This can be either achieved by keeping pH of the bulk solution low (at least 1 to 2 units below its pKa) or rather avoid a presence of any counterions such as sodium in the system (e.g., by removing buffers from the system). After considering both options, it was decided to first evaluate removing of the buffers from the system.

The subsequent feasibility studies were conducted to investigate the effect of solution pH on the solvent removal. In the first trial batch 1-F2, 10% Captisol was used and the buffer was removed from the formulation. The second trial batch 2-F2 had the same formulation compositions as Batch 1-F2 except that 10% Captisol was replaced with 10% Kleptose. The formulation compositions are shown in Table 29. The solvent composition was calculated with the addition of 1-mL solvent rinse per 10 L batch. The lyophilization cycle is shown in Table 19. The analytical results of the finished drug products are shown in Table 30. It was observed that the absence of buffer in Batch 1-F2, which resulted in a bulk solution pH below 3, significantly reduced the residual formic acid level from previous 5.6 mg/vial down to 2.3 mg/vial. An even more encouraging result was seen in 2-F2 formulation, which showed a residual formic acid level as low as 0.4 mg/vial, or 95% reduction of solvent.

Without wishing to be bound by any theory, the difference in the residual formic acid level between the two trial runs was attributed to the chemical structure differences between Captisol and Kleptose. Captisol is a mixture of beta cyclodextrin derivatives of a sodium sulfonate salt tethered to the lipophilic cavity by a butyl ether group. The presence of sodium ions in Captisol molecules may lead to the formation of sodium formate to some extent even at low pH condition. In contrast, Kleptose is a partially substituted poly-hydroxypropyl ether of beta cyclodextrin with no association with any sodium ions. Based on the residual solvent result from the second trial batch, the formulation 2-F2 was selected as the final formulation Ib.

Table 29: Formulation compositions of 10-kg formic acid-based trial runs

| **Ingredient** | **1-F2 (mg/vial)[a]** | **2-F2 (mg/vial)[a]** |
|---|---|---|
| Compound 1 | 1.0 | 1.0 |
| Captisol | 100 | / |
| Kleptose | / | 100 |
| Formic acid | 9.12[b] | 9.12[b] |
| WFI | Removed upon drying | Removed upon drying |
| Comments | API Premix prepared at 150 mg/mL Formic acid | API Premix prepared at 150 mg/mL Formic acid |
| a: without 5% overfill (8.0 mL fill volume per vial)<br>b: Partially removed upon drying. The numbers in the table represent the initial solvent content. | | |

Table 30: **Characterization of the lyophilized drug products of formic acid-based trial** batches

| **Test** | **1-F2** | **2-F2** |
|---|---|---|
| Appearance | cake | cake |
| Color | white | white |
| Foreign Matter | conform | conform |
| Assay (% LC) | 101.6 | 99.4 |
| Related Impurities | <0.02% | <0.05% |
| Residual Formic Acid | 2.3 mg/vial | 0.4 mg/vial |
| Recon Solution pH | 3.0 | 3.4 |

The bulk solutions of both trial batches were stored at ambient condition for a duration of seven days and their physical and chemical stabilities were evaluated. Table 31 shows the assay and impurity results of each formulation at initial time point and after sever days. Both formulations remained physically and chemically stable for a minimum of 7 days at ambient condition.

Table 31: Bulk solution stability evaluation of the trial batches

| | **1-F2** | **2-F2** |
|---|---|---|
| t=0 Pre-filtration assay (%) | 100.8 | 99.2 |
| t=0 Post-filtration assay (%) | 100.8 | 99.2 |
| t=7 day Post-filtration assay (%) | 100.0 | 100.0 |
| t=7 day Hydrolysis 1 (%) | 0.13% | 0.13% |
| t=7 day Hydrolysis 2 (%) | 0.04% | 0.04% |

Aside from formic acid-based formulation, the feasibility of a ’dual solvents’ formulation was also evaluated during the development. Here the assumption was that each solvent is allowed for a PDE level of 50 mg/day. Four ’dual solvent’ formulations were prepared at 10-L scale, with half of the API dissolved in 270 mg/mL DMSO solution and the other half dissolved in 150 mg/mL formic acid solution. The two API premix solutions were then added to the bulk solution in sequence. Table 32 shows the compositions of the four formulations. Batch 1-F3 and 1-F4 both used 10% Captisol. The only difference between them was that F3 was buffered at pH 5.3 while F4 was not buffered. Batch 2-F3 and 2-F4 used 10% Kleptose, with F3 buffered at pH 5.3 and F4 not buffered.

**Table 32: Compositions of 'dual solvents' formulations**

| Ingredient | 1-F3 (mg/vial)[a] | 1-F4 (mg/vial)[a] | 2-F3 (mg/vial)[a] | 2-F4 (mg/vial)[a] |
|---|---|---|---|---|
| Compound 1 | 1.0 | 1.0 | 1.0 | 1.0 |
| Citric acid | 8.96 | / | 8.96 | / |
| Sodium citrate | 29.28 | / | 29.28 | / |
| Captisol | 800 | 800 | / | / |
| Kleptose | / | / | 800 | 800 |
| DMSO | 3.06[b] | 3.06[b] | 3.06[b] | 3.06[b] |
| Formic acid | 4.27[b] | 4.27[b] | 4.27[b] | 4.27[b] |
| WFI | Removed upon drying | Removed upon drying | Removed upon drying | Removed upon drying |
| Comments | 50% of API in 270 mg/mL DMSO; 50% of API in 150 mg/mL Formic acid | 50% of API in 270 mg/mL DMSO; 50% of API in 150 mg/mL Formic acid | 50% of API in 270 mg/mL DMSO; 50% of API in 150 mg/mL Formic acid | 50% of API in 270 mg/mL DMSO; 50% of API in 150 mg/mL Formic acid |
| a: without 5% overfill (8.0 mL fill volume per vial)<br>b: Partially removed upon drying. The numbers in the table represent the initial solvent content. | | | | |

The lyophilized drug product properties of the four formulations are shown in Table 33. Although the initial amount of either DMSO or formic acid was reduced as opposed to the formulations using the single solvent alone, the residual DMSO levels in all the four formulations were barely around 2.5 mg/vial, whereas the residual formic acid level was very close to that of formulation Ib. Based on this result, the 'dual solvents' formulations were not taken into further consideration as they did not offer any advantage over formulation Ib.

**Table 33: Characterization of the lyophilized 'dual solvents' formulations**

| Test | 1-F3 | 1-F4 | 2-F3 | 2-F4 |
|---|---|---|---|---|
| Appearance | cake | cake | cake | cake |
| Color | white | white | white | white |
| Foreign Matter | conform | conform | conform | conform |
| Assay | 102.6 | 100.6 | NT | NT |
| Related Impurities (%) | <0.02% | <0.02% | NT | NT |
| Residual DMSO (mg/vial) | 2.6 | 2.6 | 2.4 | 2.4 |
| Residual formic acid (mg/vial) | 2.9 | 1.1 | 3.8 | 0.3 |
| Recon time (sec) | 25 | 26 | 26 | 26 |
| Recon solution pH | 4.6 | 3.2 | 4.8 | 3.5 |

**Example** 9: **Reconstitution Scheme of Formulation Ib**

A reconstitution study was carried out using commercially available IV fluids including WFI, NS, D5W and Lactate Ringers (LR). The osmolality of the reconstituted solutions with and without NS dilution were measured. The results are shown in Table 34. WFI of 3 mL or above provided an osmolality below 220 mOsm/kg while D5W or LR of 4-20 mL provided an osmolality above 400 mOsm/kg. NS alone with a volume less than 13 mL also gave an osmolality above 300 mOsm/kg. When 13-19 mL NS was used for reconstitution, the osmolality was close to 290 mOsm/kg. However, it would result in a total dosing volume close to 400 mL for a dose up to 20 mg, which would require a long infusion time. As a lower reconstitution volume was desired, another commercially available IV fluid, ½ normal saline

(0.45% sodium chloride), was also evaluated. It was found that 4-5 mL ½ normal saline was able to achieve an osmolality of 290 mOsm/kg. The final reconstitution scheme for Formulation Ib was determined to be 4.5 mL ½ normal saline in order to deliver a reconstituted solution at the exact concentration of 0.2 mg/mL, given the final solution volume of 5.0 mL in the vial upon reconstitution and the drug loading of 1.0 mg/vial (without overfill).

Table 34: **Osmolality and pH measurement of reconstituted solutions of formulation Ib**

| Diluent | Volume (mL) | pH | **No dilution (mOsm/kg)** | **Diluted 2x with NS (mOsm/kg)** | **Diluted** 4x **with** NS (mOsm/kg) |
|---|---|---|---|---|---|
| Water | 2 | Not determined | Could not freeze | | |
| Water | 3 | Not determined | 218 | 262 | 276 |
| Water | 4 | 3.39 | 157 | 234 | 262 |
| NS | 4 | 3.32 | 465 | Not determined | Not determined |
| NS | 8 | Not determined | 376 | Not determined | Not determined |
| NS | 9.7 | Not determined | 356 | Not determined | Not determined |
| NS | 13.4 | Not determined | 291 | Not determined | Not determined |
| NS | 17.1 | Not determined | 293 | 290 | 289 |
| Lactate ringer | 4 | Not determined | 735 | Not determined | Not determined |
| Lactate ringer | 15 | Not determined | 582 | Not determined | Not determined |
| Lactate ringer | 19.7 | Not determined | 567 | Not determined | Not determined |
| D5W | 4 | Not determined | DNF | Not determined | Not determined |
| D5W | 8.7 | Not determined | 479 | Not determined | Not determined |
| D5W | 15 | Not determined | 443 | Not determined | Not determined |
| D5W | 19.7 | Not determined | 429 | Not determined | Not determined |
| ½ NS | 9.6 | Not determined | 215 | 254 | 266 |
| ½ NS | 4.8 | Not determined | 294 | 290 | 290 |
| ½ NS | 4.6 | Not determined | 289 | Not determined | Not determined |
| ½ NS | 5.6 | Not determined | 263 | Not determined | Not determined |
| ½ NS | 4.6 | Not determined | 289 | 292 | 287 |
| ½ NS | 4.4 | Not determined | 294 | 294 | 293 |

(continued)

| Diluent | Volume (mL) | pH | **No dilution (mOsm/kg)** | **Diluted 2x with NS (mOsm/kg)** | **Diluted** 4x **with** NS (mOsm/kg) |
|---|---|---|---|---|---|
| ½ NS | 4.4 | 3.37 | 295 | 294 | 292 |

The pH of the reconstituted solution of Formulation Ib was noted to be on a lower side (< 3.6), but this was considered acceptable.

Example 10: Process Scale up of Formulation **Ib**

Three development lyophilization runs, with multiple batches in each lyophilization batch, were manufactured to scale up the batch size from 10 L to the clinical batch size of 30 L.

*I$^{st}$ Scale-Up Batch:*

In the first scale-up batch, one 30-L and two 10-L compounding batches were prepared. The formulation compositions are shown in Table 35. Batch 3-F2-1 was prepared with 150 mg/mL API premix in formic acid at a 30-L batch size. The premix was added to the compounding vessel through a peristaltic pump and a 0.6mm silicone tubing, at a rate of 50 μL per addition every 10 seconds. Batch 3-F2-2 and 3-F2-3 were both prepared with 100 mg/mL API premix in formic acid at 10-L batch size. The only difference between F2-2 and F2-3 was the API premix addition method. In F2-2, API premix addition was very fast (premix was instanteously poured directly to the compounding vessel at one time to test as a worst-case scenario) while F2-3 followed the same API premix addition procedure as the batch F2-1. All the three batches were lyophilized in a lab lyophilizer using the same lyophilization cycle as shown in Table 19. For the scale-up batch F2-1, bulk solution samples were taken from the compounding vessel as well as from the beginning, middle, and end of the receiving vessel for in process assay testing. The pre- and post- filtration bulk solution assay of the batch F2-2 and F2-3 were also tested. The filtered bulk solutions of F2-1 and F2-2 were stored in the receiving vessel at ambient condition for seven days. The physical and the chemical stability of both batches were inspected.

Table 35: Formulation compositions of **1$^{st}$** scale up run

| **Ingredient** | 3-F2-1 **(mg/vial)$^a$** | 3-F2-2 **(mg/vial)$^a$** | 3-F2-3 **(mg/vial)$^a$** |
|---|---|---|---|
| Compound 1 | 1.0 | 1.0 | 1.0 |
| Kleptose | 800 | 800 | 800 |
| Formic acid | 9.1$^b$ | 13.2$^b$ | 13.2$^b$ |
| WFI | Removed upon drying | Removed upon drying | Removed upon drying |
| Comments | API Premix prepared at 150 mg/mL formic acid. 30 L batch size. API premix added at 50 μL per 10 sec. | API Premix prepared at 100 mg/mL formic acid. 10 L batch size. API premix poured at one time. | API Premix prepared at 100 mg/mL formic acid. 10 L batch size. API premix added at 50 μL per 10 sec. |
| a: without 5% overfill (8.0 mL fill volume per vial)<br>b: Partially removed upon drying. The number in the table corresponds to the initial solvent content. | | | |

Table 36 and Table 37 present bulk stability and finished drug products results, respectively. In general, all the three batches exhibited acceptable quality attributes. Batch F2-2, despite its extreme rate of API premix addition, achieved good in-process assay and the bulk solution stability comparable to the other two batches. This indicates that the compounding process is robust and is not sensitive to the API premix addition rate. All three batches had residual formic acid level < 2.5 mg/vial as targeted regardless of the initial amount of formic acid charge. It was speculated that the residual formic acid removal kinetics may have reached a plateau determined by the freeze drying process condition rather than the initial solvent level. In addition, the scale-up to a 30-L compounding batch size was done successfully (3-F2-1).

It was noticed that the residual formic acid level of these batches were higher than observed for the formulation development batch, 2-F2. There were minor differences in these scale-batches to the formulation development batch,

however 3-F2-3 and 2-F2 had the same formulation and the same process, yet the residual solvent levels were very different (1.4 mg/vial vs. 0.4 mg/vial). As part of finding a potential cause of this, the lyophilization data profile, as illustrated in FIG. 32 was evaluated. The profile showed that the pirani pressure was not completely converged with the chamber pressure (measured by capacitance manometer) at the end of primary drying, implying that sublimation was not complete. Therefore, in the next scale-up batch, more efforts were made to understand the residual solvent variability.

Table 36: Bulk solution stability of **1st** scale up batch

| | 3-F2-1 | 3-F2-2 | 3-F2-3 |
|---|---|---|---|
| t=0 Pre-filtration assay (%) | 98.4 | 100.0 | 99.2 |
| t=0 Post-filtration assay (%) | 98.4 (beg); 98.4 (mid); 98.4 (end) | 99.2 | 98.4 |
| t=7 day Post-filtration assay (%) | 99.7 (beg); 99.7 (mid); 99.7 (end) | 99.7 | Not Tested |
| t=7 day Hydrolysis 1 (%) | 0.06 (beg) 0.05 (mid) 0.05 (end) | 0.05 | Not Tested |
| t=7 day Hydrolysis 2 (%) | 0.14 (beg) 0.14 (mid) 0.13 (end) | 0.14 | Not Tested |

Table 37: Drug product characterization of **1st** scale up batch

| Test | 3-F2-1 | 3-F2-2 | 3-F2-3 |
|---|---|---|---|
| Appearance | cake | cake | cake |
| Color | white | white | white |
| Foreign Matter | conform | conform | conform |
| Assay | 98.5 | 99.5 | 99.9 |
| Related Impurities (%) | Total <0.05 | Total <0.05 | Total <0.05 |
| Water Content | 0.10% | 0.10% | 0.06% |
| Residual Solvent | 1.3 mg/vial | 1.5 mg/vial | 1.4 mg/vial |
| Recon time (sec) | 34 | 31 | 35 |
| Recon solution pH | 3.0 | 3.0 | 3.0 |
| *2nd Scale-Up Batch:* | | | |

Compound 1 concentration of 100 mg/mL and 50 mg/mL were evaluated with two compounding batches, 4-F1 and 4-F2. The formulation compositions of these two batches are shown in Table 38.

Table 38: Formulation compositions of **2nd** scale up run

| Ingredient | **4-F1 (mg/vial)[a]** | 4-F2 **(mg/vial)[a]** |
|---|---|---|
| Compound 1 | 1.0 | 1.0 |
| Kleptose | 800 | 800 |
| Formic acid | 13.2[b] | 25.4[b] |

(continued)

| Ingredient | **4-F1 (mg/vial)[a]** | 4-F2 **(mg/vial)[a]** |
|---|---|---|
| WFI | Removed upon drying | Removed upon drying |
| Comments | Compound 1premix prepared at 100 mg/mL formic acid. 30 L batch size. | Compound 1 premix prepared at 50 mg/mL formic acid. 30 L batch size. |
| a: without 5% overfill (8.0 mL fill volume per vial)<br>b: Partially removed upon drying. The number in the table corresponds to the initial solvent content. | | |

A more conservative lyophilization cycle was evaluated (to minimize processing risk, and thus enabling a direct manufacture of a clinical batch in the GMP suite). As shown in Table 40 three major changes were made in the lyophilization process: 1) the freezing hold time was extended from 4 hours to 6 hours; 2) the primary drying hold time was extended from 70 hours to 90 hours; 3) the secondary drying time was extended from 12 hours to 18 hours.

**Table 39: Lyophilization cycle parameters of 2nd trial batches**

| Step | Shelf Temp. Setpoint (°C) | Soak Time (hours) | Ramping Rate (°C/ hour) | Pressure Setpoint |
|---|---|---|---|---|
| Product Loading/F reezing | 5 | 2 | | Evac. To 12 psia to ensure chamber is airtight |
| | | | 30 | |
| Freezing | -50 | 6 | | |
| | | | 30 | |
| Primary Drying | -16 | 90 | | 140 microns |
| | | | 30 | 140 microns |
| Secondary Drying | 60 | 18 | | 140 microns |
| Stoppering | 25 | | | 14.7 PSIA |

Samples were withdrawn from various locations and time points from the lyophilizer chamber. More specifically, three vials were taken from the front edge of the middle shelf by a thief at 65-hour, 75-hour, and 90-hour of primary drying hold, and at each time point of 6-hour, 12-hour and 18-hour of secondary drying hold.

Batch was prepared in a controlled bioburden condition to enable bioburden method development.

The batch was processed successfully. The results of the finished drug products are shown in Table 40. All the quality attributes were found to be acceptable. It was noted that the two compounding batches had the same residual solvent level of 0.6 mg/vial, despite different initial solvent levels. It was also found that the residual solvent level was lower than the 1st scale-up batch, likely due to additional 6 hr of secondary drying time. As described above, this batch also investigated change in the residual solvent level with time; the results are shown in FIG. 33.

**Table 40: Drug product characterization of 2nd scale up batch**

| Test | **4-F1** | 4-F2 |
|---|---|---|
| Appearance | cake | cake |
| Color | white | white |
| Foreign Matter | conform | conform |
| Assay | 100.5 | 101.6 |
| Related Impurities (%) | Total < 0.05% | Total < 0.05% |
| Water Content | 0.07% | 0.07% |
| Residual Solvent | 0.6 mg/vial | 0.6 mg/vial |
| Recon time (sec) | 36 | 39 |
| Recon solution pH | 3.2 | 3.2 |

With the initial solvent level of 13.2 mg/vial, the residual solvent in the lyophilized vials was reduced to 4.5 mg/vial after 65 hours of primary drying hold, with minimal change after that during the primary drying. With subsequent secondary drying, the residual solvent decreased continuously, achieving ~ 0.6 mg/vial after 18 hr of drying. This finding supports that increasing the secondary drying hold time to 18 hours can be helpful in reducing the residual solvent level. The lyophilization data profile, as illustrated in FIG. 34 showed that the pirani pressure was completed converged with the chamber pressure as measured by a capacitance manometer at the end of primary drying, indicating a complete sublimation. Therefore, a primary drying hold time of 90 hours was recommended for the clinical batch to be on a conservative side (as mentioned earlier, lyophilizer for the clinical batch is ~ 3X of the development batch).

In this batch, the residual solvent level from different tray locations (center vs. edge) and different shelf locations (middle and bottom shelf only, top three shelves were collapsed due to the thief positioning) was also investigated. The center vials contained slightly higher level of residual solvent than the edge vials because the edge vials generally receive more heat radiation from the door and the walls of the lyophilizer. Overall, the residual solvent level was not varied much with the vial locations in the lyophilization chamber. This showed that the sampling location was not the likely cause of variable residual solvent levels observed for earlier batches.

*3rd Scale-Up Batch:*

The third and the last scale-up run was a confirmation batch at 30-L batch scale with the same formulation compositions and process parameters as Batch 4-F1. The batch was made at controlled bioburden condition and was used to support ICH stability study later on. The batch release testing results are shown in Table 41.

**Table 41: Drug product characterization of scale up batch**

| Test | **B-3** |
|---|---|
| Appearance | cake |
| Color | white |
| Foreign Matter | conform |
| Assay (% LC) | 101.3 |
| Related Impurities | Total < 0.05% |
| Water Content | 0.08% |
| Residual Solvent | 0.9 mg/vial |
| Recon time | 39 sec |
| Recon solution pH | 3.1 |
| Osmolality | 297 mOsm/kg |
| Particulate Matter | 11 particles per vial ≥ 10 μm ; 0 particles per vial ≥ 25 μm |

Additional formulations were screened by varying drug loading, vial size, drug concentration and Kleptose concentration in several lyophilization trial runs. All the tested formulations showed acceptable product quality attributes as shown in Table 42.

Table 42

| **Dose strength and container** | | **Lyophilizer Formulation Composition** | | | **Test Results** | |
|---|---|---|---|---|---|---|
| **Drug Loading (mg/vial)** | **Vial size (mL)** | **Compound 1 (% w/w)** | **Kleptose (% w/w)** | **Residual Formic Acid (% w/w)** | **Assay (%)** | **Residual formic acid amount (mg/vial)** |
| 2 | 30 | 0.12 | 99.68 | 0.20 | 97.65 | 2.92 |
| 2 | 50 | 0.04 | 99.77 | 0.19 | 98.25 | 9.45 |
| 4 | 100 | 0.05 | 99.76 | 0.19 | 101.01 | 15.42 |
| 5 | 100 | 0.05 | 99.75 | 0.20 | 100.29 | 21.15 |

(continued)

| Dose strength and container | | Lyophilizer Formulation Composition | | | Test Results | |
|---|---|---|---|---|---|---|
| Drug Loading (mg/vial) | Vial size (mL) | Compound 1 (% w/w) | Kleptose (% w/w) | Residual Formic Acid (% w/w) | Assay (%) | Residual formic acid amount (mg/vial) |
| 1 | 30 | 0.12 | 99.75 | 0.13 | 99.96 | 0.98 |
| 1.5 | 30 | 0.12 | 99.72 | 0.16 | 100.00 | 1.76 |
| 1.5 | 50 | 0.12 | 99.75 | 0.13 | 99.89 | 1.61 |
| 1.5 | 100 | 0.12 | 99.74 | 0.14 | 100.00 | 1.59 |
| 3 | 50 | 0.12 | 99.74 | 0.13 | 99.93 | 3.56 |
| 3 | 100 | 0.12 | 99.74 | 0.13 | 99.69 | 3.56 |
| 5 | 100 | 0.12 | 99.72 | 0.16 | 99.90 | 6.48 |
| 6.25 | 100 | 0.12 | 99.66 | 0.21 | 99.86 | 10.23 |
| 1.8 | 50 | 0.05 | 99.79 | 0.16 | 99.49 | 5.50 |
| 1.8 | 100 | 0.05 | 99.82 | 0.13 | 99.48 | 5.08 |
| 3 | 100 | 0.05 | 99.81 | 0.14 | 99.87 | 8.26 |

**Example** 11: Evaluation of Formulation Stability

The selected formulations (Ia and Ib) are provided in Table 43 below:

Table 43: Compositions of formulations **Ia** and **Ib**

| | Formulation Ia* | Formulation Ib |
|---|---|---|
| Compound 1 | 1.05 mg/vial | 1.0 mg/vial |
| Citric acid anhydrous, USP | 18.6 mg/vial | - |
| Sodium citrate anhydrous, USP | 18.4 mg/vial | - |
| Kleptose® HPB (HP-β-CD), parenteral grade | 840 mg/vial | 800 mg/vial |
| Dimethyl sulfoxide (processing aid) | Partially removed upon drying | - |
| Formic acid (processing aid) | - | Partially removed upon drying |
| Water for injection (processing aid) | Removed upon drying | Removed upon drying |
| * with 5% overfill | | |

Formulations Ia and Ib were further evaluated for 1) physical and chemical stability of finished drug products, 2) in-use stability of the drug product upon reconstitution, and 3) material compatibility studies during administration. Here the goal was to have a minimum of 12-month shelf-life for the drug product. For the drug solution upon reconstitution, a minimum of 8-hour stability was desired to allow clinicians for sufficient preparation time before dosing to patients. In addition, this reconstituted solution upon dilution (if needed) is expected to have acceptable chemical and physical stability with material of contact such as syringes, IV tubing, infusion bags, heparin, in-line filters, etc. during the administration timeframe.

**Stability of Finished Drug Products**

The drug product stability of formulation Ia was evaluated and 1-month and 3-month stability results are shown in Table 44. The drug products remained stable with no major changes in appearance, assay and impurities, and reconstitution after 3 months of storage at accelerated 40°C/75%RH condition. This result is considered to support a shelf-life of up to 12 months at ambient condition for formulation Ia.

Table 44: Drug product stability of formulation **Ia**

| Test | Acceptance Criteria | t=0 | t= 1 month 40°C/75%RH | t= 3 month 40°C/75%RH |
|---|---|---|---|---|
| Appearance | Cake | Cake | No change from Initial | No change from Initial |
| Color | White to off white | White | White | White |
| Water Content | Report results | 0.04% | 0.20% | 0.20% |
| Assay (% LC) | 90-110 | 104.2 | 104.3 | 104.5 |
| Related Impurities | Total impurities < 3% Individual impurities <1 % | Individual: 0.06% Total: 0.06% | <QL | <QL |
| Recon Time | Report results | 32 | 29 | 31 |
| pH | Report results | 4.4 | 4.4 | 4.4 |

Similarly, the drug product stability of Formulation Ib was evaluated. As shown in Table 45, no major changes in appearance, assay and impurities, and reconstitution were observed after one month of storage at accelerated 40°C/75%RH condition.

Table 45: **Drug product stability of Formulation Ib**

| Test | Acceptance Criterion | t=0 | t=1 Month 40°C/75%RH |
|---|---|---|---|
| Appearance | Cake or powder | Cake | Cake |
| Color | White to off-white | White | White |
| Water Content | Report results | 0.05% | 0.21% |
| Recon Time[1] | Report results | 28 sec | 30 sec |
| Assay (% LC) | 90-110 | 99.4 | 100.5 |
| Related Impurities | Individual: NMT 1.0% Total: NMT 3.0% | Total <0.05 | Total <0.05 |
| pH | Report results | 3.4 | 3.3 |

As the drug product stability of formulation Ia was established earlier than formulation Ib, an Accelerated Stability Assessment Program (ASAP) was set up to compare the formulation Ib with formulation Ia. The study was run intentionally on open vials in several high temperature/low humidity conditions for a short period of time in order to forecast the degradation potential and the temperature/humidity sensitivity of each formulation. The assay and impurity results are shown in Table 46. The percentage of impurities increased with the increase of temperature, humidity and storage time as anticipated. Overall, formulations Ia and Ib showed very similar degradation profile for all the tested conditions. Only at the most stressful condition of 80°C/21%RH, formulation Ib showed slightly higher degradation (5%) than formulation Ia (3%) at 7-day time point. Later on it was confirmed by Mass Spectrometer that all the degradants were resulted from the hydrolysis degradation and no unknown degradation mechanism was involved. Based on the ASAP result, it was concluded that formulation Ib had comparable drug product stability with formulation Ia. Therefore, formulation Ib is expected to have the same shelf life and the same storage condition as formulation Ia.

**Table 46: ASAP stability results of formulations Ia and Ib**

| Sample | Condition | Time Pull (day) | % Assay | % Total Impurity |
|---|---|---|---|---|
| Ia | Initial | 0 | 104.2 | <0.05% |
| Ib | | 0 | 99.4 | <0.05% |

(continued)

| Sample | Condition | Time Pull (day) | % Assay | % Total Impurity |
|---|---|---|---|---|
| Ia | 50°C / 11% RH | 3 | 105.0 | ND |
| | | 7 | 105.2 | 0.11 |
| Ib | | 3 | 99.6 | ND |
| | | 7 | 99.7 | 0.09 |
| Ia | 50°C / 21% RH | 3 | 105.4 | ND |
| | | 7 | 105.2 | 0.20 |
| Ib | | 3 | 99.5 | ND |
| | | 7 | 100.2 | 0.15 |
| Ia | 80°C / 11% RH | 3 | 104.9 | 1.40 |
| | | 7 | 103.5 | 2.80 |
| Ib | | 3 | 99.4 | 1.20 |
| | | 7 | 100.5 | 2.80 |
| Ia | 80°C / 21% RH | 3 | 103.7 | 1.30 |
| | | 7 | 102.1 | 3.00 |
| Ib | | 3 | 98.2 | 0.80 |
| | | 7 | 92.5 | 5.00 |
| ND =Not Detected | | | | |

**In-use Stability of Reconstituted Solutions**

The in-use stabilities of the reconstituted solutions in product vials as well as in syringes, IV lines, and IV bags were evaluated extensively. For both formulations Ia and Ib, the reconstituted solution was found to be physically and chemically stable in product vials for up to 8 hours at ambient condition and at 5°C. In addition, the reconstituted solution demonstrated acceptable chemical and physical stability in syringes, IV tubing and infusion bags with no compatibility issues with heparin, diluent or in-line filters during simulated dose administration timeframe.

An additional stability study was conducted on the reconstituted solution in product vials for both formulations Ia and Ib to understand risk of physical stability of the solution. The vials of both formulations were stored at 5°C for and the assay/related impurities were tested at different time points. The 3-month data show no major change in assay for all the tested samples (Table 47). The total degradation remained below 1% for both formulations Ia and Ib after 55 days of storage for the refrigerated solutions. With the two hydrolysis degradants growing over time, formulation Ia showed the total impurity > 1% beyond 62 days, while formulation Ib, due to the low pH of its reconstituted solution, still kept its total impurity lower than 1% up to 96 days. These results show a low risk of drug precipitation in the reconstituted solutions of formulations Ia and Ib based on the data.

**Table 47: Reconstituted solution stability in vials at 5°C (formulations Ia and Ib)**

| Day | Formulation | Assay % | Related Impurities (RRT) | | | | |
|---|---|---|---|---|---|---|---|
| | | | 1.668 | 1.829 | Compound 1 3.825 | 7.619 | Total |
| 0 | Ia | 104.2 | 0.00 | 0.00 | 100.00 | 0.00 | 0.00 |
| | Ib | 99.4 | 0.00 | 0.00 | 100.00 | 0.00 | 0.00 |
| 23 | Ia | 106.4 | 0.17 | 0.23 | 99.55 | 0.05 | 0.45 |
| | Ib | 98.2 | 0.13 | 0.00 | 99.87 | 0.00 | 0.13 |

(continued)

| Day | Formulation | Assay % | Related Impurities (RRT) | | | | |
|---|---|---|---|---|---|---|---|
| | | | 1.668 | 1.829 | Compound 1 3.825 | 7.619 | Total |
| 27 | Ia | 104.8 | 0.19 | 0.25 | 99.46 | 0.10 | 0.54 |
| | Ib | 98.3 | 0.23 | 0.23 | 99.48 | 0.06 | 0.52 |
| 55 | Ia | 106.6 | 0.28 | 0.09 | 99.63 | 0.00 | 0.37 |
| | Ib | 101.2 | 0.38 | 0.49 | 99.13 | 0.00 | 0.87 |
| 62 | Ia | 105.9 | 0.42 | 0.55 | 98.71 | 0.00 | 1.28 |
| | Ib | 101.6 | 0.33 | 0.10 | 99.51 | 0.00 | 0.49 |
| 96 | Ia | 103.6 | 0.66 | 0.86 | 98.48 | 0.00 | 1.52 |
| | Ib | 100.2 | 0.50 | 0.15 | 99.35 | 0.00 | 0.65 |

**Example 12: Process procedures of formulations Ia and Ib**

A detailed description of the process procedure of formulation Ia is as follows:
Compounding: Citric acid, sodium citrate, and hydroxypropyl-beta-cyclodextrin (Kleptose®) are dissolved in Water for Injection (WFI) in an appropriate-sized vessel (Vessel 1). Compound 1 is dissolved in dimethyl sulfoxide (DMSO) in a separate vessel (Vessel 2). This Compound 1-DMSO premix solution is then added to Vessel 1 through an electronic pipette or a peristaltic pump at a constant rate (~ 50 μL per addition every 10 seconds) while the solution in Vessel 1 is being mixed with good vortex. Solution is visually inspected to make sure that no undissolved particles are present in Vessel 1. Following mixing, the batch weight is adjusted to the target weight with WFI.

Filtration: The bulk solution is then filtered using two 0.2 μm sterile filters in series. Prior to this step, the bulk solution may be optionally pre-filtered using one 0.45 μm or 0.2 μm sterile filter.

Aseptic filling, lyophilization, and vial capping: Aseptic fill is performed in a 20-ml vial with a target fill volume of 8.40 mL (with 5% overfill). Lyophilization stoppers are then partially placed (to the first notch) on each filled vial. The lyophilizer is then loaded and the freeze-drying cycle (Table 13) is executed. After lyophilization is completed, vials are stoppered under reduced pressure in an atmosphere of nitrogen and sealed.

The process flow diagram of Formulation Ia is illustrated in FIG. 35 and the lyophilization process parameters of formulation Ia is shown in Table 48.

**Table 48: Lyophilization cycle parameters of formulation Ia**

| Step | Shelf Temp. Setpoint (°C) | Soak Time (hours) | Ramping Rate (°C/hour) | Pressure Setpoint |
|---|---|---|---|---|
| Product Loading/ Freezing | 5 | 2 | | Evac. To 12 psia to ensure chamber is airtight |
| | | | 30 | |
| Freezing | -50 | 4 | | |
| | | | 30 | |
| Primary Drying | -16 | 70 | | 140 microns |
| | | | 30 | 140 microns |
| Secondary Drying | 50* | 12 | | 140 microns |
| Stoppering | 25 | | | 14.7 PSIA |
| *: 50°C was used in LTI batches; 60°C was used in BSP batches | | | | |

A detailed description of the process procedures of formulation Ib is as follows:

Compounding: Hydroxypropyl-beta-cyclodextrin (Kleptose®) is dissolved in Water for Injection (WFI) in an

appropriate-sized vessel (Vessel 1). Compound 1 is dissolved in formic acid (FA) in a separate vessel (Vessel 2). This Compound 1-FA premix solution is then added to Vessel 1 through an electronic pipette or a peristaltic pump at a constant rate (~ 50 μL per addition every 10 seconds) while the solution in Vessel 1 is being mixed with good vortex. Solution is visually inspected to make sure that no undissolved particles are present in Vessel 1. Following mixing, the batch weight is adjusted to the target weight with WFI.

Filtration: The bulk solution is then filtered using two 0.2 μm sterile filters in series. Prior to this step, the bulk solution may be optionally pre-filtered using one 0.45 μm or 0.2 μm sterile filter.

Aseptic filling, lyophilization, and vial capping: Aseptic fill is performed in a 20-ml vial with a target fill weight of 8.0 mL (without overfill). Lyophilization stoppers are then partially placed (to the first notch) on each filled vial. The lyophilizer is then loaded and the freeze-drying cycle is executed. After lyophilization is completed, vials are stoppered under reduced pressure in an atmosphere of nitrogen and sealed.

The process flow diagram of Formulation Ib is illustrated in FIG. 36 and the lyophilization process parameters of formulation Ib is shown in Table 49.

**Table 49: Lyophilization cycle parameters of formulation Ib**

| Step | Shelf Temp. Setpoint (°C) | Soak Time (hours) | Ramping Rate (°C/ hour) | Pressure Setpoint |
|---|---|---|---|---|
| Product Loading/ Freezing | 5 | 2 | | Evac. To 12 psia to ensure chamber is airtight |
| | | | 30 | |
| Freezing | -50 | 6 | | |
| | | | 30 | |
| Primary Drying | -16 | 90 | | 140 microns |
| | | | 30 | 140 microns |
| Secondary Drying | 60 | 18 | | 140 microns |
| Stoppering | 25 | | | 14.7 PSIA |

**Example 13: Supersaturation Risk Assessment of Formulation Ib**

In formulation Ib described in Example 11, the bulk solution and the reconstituted solution are about 2-fold supersaturated above their equilibrium solubility. Two studies were performed to evaluate the supersaturation risks of Kleptose based formulations. The objective of the first study is to determine the equilibrium solubility of Compound 1 in Kleptose solutions of different concentrations using both 'top-down' and 'bottom-up' approaches. The second study is intended to evaluate the kinetics of drug precipitation in Formulation Ib.

**Determination of Equilibrium Solubility of Compound 1 in Kleptose solutions**

A 'bottom-up' approach was employed to measure the equilibrium solubility of Compound 1 in Kleptose solutions. In this experiment, ten solutions were prepared by dissolving Kleptose and formic acid in WFI. The Kleptose concentration in all the ten solutions varied from 30 mg/mL to 250 mg/mL, while the formic acid concentration was kept constant at 1.65 mg/mL as used in the bulk solution of formulation Ib. Excess Compound 1 of approximately 25 mg was added to each of the prepared 100-mL solution and mixed for 48 or 72 hours at 25°C. Then the sample of each solution was taken and centrifuged for assay testing. The solubility of Compound 1 in each vehicle is listed in Table 50.

**Table 50: Solubility of Compound 1 in Kleptose solutions**

| Kleptose (mg/mL) | Formic acid (mg/mL) | 48-hr Solubility at 25°C (mg/mL) |
|---|---|---|
| 30 | 1.65 | 0.021 |
| 60 | | 0.037 |
| 70 | | 0.047 |
| 80 | | 0.050 |
| 90 | | 0.059 |
| 100 | | 0.065 |
| 130 | | 0.086[a] |
| 170 | | 0.115[a] |
| 200 | | 0.131[a] |
| 250 | | 0.171[a] |
| [a]: solubility measured after 72 hours at 25°C | | |

It was found that the solubility of Compound 1 increased linearly with the Kleptose concentration, as shown in FIG. 37.

Another solubility experiment was carried out using a 'top-down' approach, i.e. adding the crystalline Compound 1 to a supersaturated solution to induce Compound 1 precipitation until the solution reached the equilibrium solubility. In the experiment, six solution formulations were prepared by adding the 100 mg/mL Compound 1 in formic acid premix to a Kleptose solution to make the final drug concentration at approximately 125 μg/mL. The Kleptose concentration varied from 30 to 100 mg/mL. Then 1 mL Compound 1 slurry in water containing 6.25 mg Compound 1 (accounted for about 10% of total Compound 1 in solution) was added to 500 mL Compound 1/Kleptose solution and mixed continuously for 9 days at 25°C. The assay of the solution was tested at the end of 9 days and the results are shown in Table 51. In contrast, the assay of the same solution without addition of Compound 1 slurry was also monitored for up to 4 weeks at 25°C. The assay results at the end of 4 weeks are shown in Table 51.

**Table 51 Solubility of Compound 1 in 'top-down' approach**

| Kleptose (mg/mL) | Formic acid (mg/mL) | Assay at t=0 (mg/mL) | Assay at t=9 days with Compound 1 crystal at 25°C(mg/mL) | Assay at t= 4 wks without Compound 1 crystal at 25°C (mg/mL) |
|---|---|---|---|---|
| 30 | 1.65 | 0.114 | 0.036 | 0.036 |
| 60 | | 0.115 | 0.065 | 0.114 |
| 70 | | 0.115 | 0.077 | 0.114 |
| 80 | | 0.116 | 0.103 | 0.114 |
| 90 | | 0.116 | 0.114 | 0.115 |
| 100 | | 0.117 | 0.117 | 0.115 |

The results showed that all the six solutions except for the one with 30 mg/mL Kleptose remained relatively stable at 25°C with no assay change after 4 weeks without the addition of Compound 1 crystals into the solutions. When the crystalline Compound 1 material was added into the solution, the solutions with 30-80 mg/mL Kleptose were observed obvious drug precipitation. The lower the Kleptose concentration in the solution, the more assay reduction occurred after 9 days of mixing. There was no assay change in the solutions with 90-100 mg/mL Kleptose even in the presence of Compound 1 crystals. The precipitated Compound 1 was confirmed to be Form B instead of Form C of the starting material by XRPD (data not shown). It suggests that the solubility measured from the 'top-down' approach was that of Form B. Therefore, the values were higher than the solubility of Form C obtained from the 'bottom-up' approach. It may require longer time for the 'top-down' approach to reach the equilibrium solubility of the most thermodynamically stable Compound 1 form (Form C).

**Kinetics of drug precipitation in supersaturated Kleptose solutions**

In the first precipitation study, four solution formulations were prepared at a Kleptose concentration of 100 mg/mL. Compound 1 drug concentration varied from 50 μg/mL to 480 μg/mL in each formulation. The same amount of crystalline Compound 1 material was added into each formulation (1.25 mg Compound 1 to 500 g solution) upon the completion of compounding. Then the solutions were stored in three conditions: (1) at ambient condition with mild mixing; (2) at ambient condition without mixing; (3) at 2-8°C without mixing. The assay of each solution at t=48 hours was tested. The results are shown in Table 52.

**Table 52: Drug precipitation of Compound 1 in 100 mg/mL Kleptose solutions**

| | F1 | F2 | F3 | F4 |
|---|---|---|---|---|
| t=0 assay (μg/mL) | 57.9 | 152.5 | 303.4 | 482.0 |
| Added API crystal/total API in solution | 5% | 2.5% | 1.25% | 0.6% |
| t=48-hr assay (μg/mL), no mixing at RT | 57.9 | 149.6 | 278.7 | 357.1 |
| t=48-hr assay (μg/mL), mild mixing at RT | 57.9 | 149.8 | 234.7 | 135.6 |
| t=48-hr assay (μg/mL), no mixing at 5°C | 57.9 | 148.4 | NT | NT |
| **Assay change from t=0** | | | | |
| 48-hr no mixing at RT | 0.0% | -1.9% | -8.1% | -25.9% |
| 48-hr mixing at RT | 0.0% | -1.8% | -22.7% | -71.9% |
| 48-hr no mixing at 5°C | 0.0% | -2.7% | NT | NT |

Formulation F1 remained stable at all the three storage conditions with no assay change within 48 hours. Formulation F2 ended up with a concentration of about 148 μg/mL, with 2-3% assay reduction from t=0 after 48 hours storage for all the three storage conditions. Formulation F3 and F4, despite a lower ratio of Compound 1 seeding, showed significant assay reduction from initial. Mixing expedited the drug precipitation kinetics in both formulations. It was concluded that the drug precipitation risks in 100 mg/mL Kleptose solutions increased at a higher supersaturation ratio and when mixing was applied to the solution.

In the second precipitation study, three solution formulations were prepared with the Kleptose concentration of 10%, 15%, and 20% in each formulation, respectively. The corresponding drug concentration of each formulation was 300, 400, and 500 μg/mL so that the supersaturation ratio was about 5x for all the three formulations. After the addition of 2% of Compound 1 crystal seeds, each solution formulation was stored at two different conditions: (1) at ambient condition with mild mixing; (2) at 2-8°C without mixing. The assay of all the solutions were monitored for up to 34 days. In the study, the same experiments were repeated twice to evaluate the reproducibility of the results. The assay results are shown in FIG. 38 and FIG. 39.

As shown in FIG. 38, the drug precipitation became slower with the increase of Kleptose concentration. For Formulation F1 with 10% Kleptose, the drug concentration reached plateau at approximately 98 μg/mL after 7 days at room temperature. Formulation F2 with 15% Kleptose appeared to reach a plateau concentration of 184 μg/mL after 34 days, while the concentration of Formulation F3 with 20% Kleptose went down to 290 μg/mL and was still in a declining trend. When the supersaturated solution was stored at 2-8°C without mixing, the drug precipitation rate was even slower, as shown in FIG. 39. The assay values of the three formulations after 34 days became 97 μg/mL, 133 μg/mL, and 385 μg/mL, respectively.

Based on these two precipitation studies plus the one conducted in the 'top-down' solubility study, the apparent solubility of Compound 1 in a 10% Kleptose solution at room temperature was 135 μg/mL after 2 days, 115 μg/mL after 9 days, and 98 μg/mL after 34 days. It suggested that formulation Ib which consists of 10% Kleptose and a drug concentration of 125 μg/mL, although in a supersaturated state, may undergo a very slow precipitation kinetics to reach its equilibrium solubility within days to weeks, due to the stabilization effect of Kleptose as a complexing agent.

**Physical stability of Formulation Ib bulk solution and reconstituted solution**

In this study, bulk solution (10% Kleptose, 125 μg/mL Compound 1) and reconstituted solution (16% Kleptose, 200 μg/mL Compound 1) for Formulation Ib were prepared and then challenged with 0.5% Compound 1 seeds. Both solutions were stored at room temperature with continuous mixing for 48 hours. The assays of the solutions were monitored at different time points. Subsequently, the experiment was repeated with 2.5% Compound 1 seeds. The

results of the two experiments are shown in Table 53.

**Table 53: Assay of bulk solution and reconstituted solution for Formulation Ib with Compound 1 seeds**

| | Time Point (hr) | RT, mixing with 0.5% Compound 1 seeds | | RT, mixing with 2% Compound 1 seeds | |
|---|---|---|---|---|---|
| | | Concentration (μg/mL) | Assay change from T0 | Concentration (μg/mL) | Assay change from T0 |
| F1 (10% CD, 125 μg/mL) | 0 | 133.6 | 0.0% | 128.3 | 0.0 |
| | 1 | 137.9 | 3.2% | 126.7 | -1.2 |
| | 2 | 129.3 | -3.2% | 128.2 | -0.1 |
| | 4 | 138.1 | 3.3% | 127.1 | -0.9 |
| | 24 | 134.6 | 0.7% | 128.5 | 0.1 |
| | 48 | 134.4 | 0.6% | 127.8 | -0.4 |
| F2 (16% CD, 200 μg/mL) | 0 | 204.2 | 0.0% | 206.6 | 0.0 |
| | 1 | 203.8 | -0.2% | 202.3 | -2.1 |
| | 2 | 200.3 | -1.9% | 203.7 | -1.4 |
| | 4 | 200.8 | -1.7% | 201.7 | -2.4 |
| | 24 | 205.4 | 0.6% | 204.4 | -1.1 |
| | 48 | 202.2 | -1.0% | 203.2 | -1.6 |

It was shown that even in the presence of Compound 1 seeds, both the bulk solution and reconstituted solution remained relatively stable at room temperature within 48 hours, with no obvious trend of assay reduction over time, regardless of the amounts of Compond 1 seeds (0.5% or 2.5%) added to the formulation. The assay change from TO is 3% or less, well within the assay specification of 90-110%. It implied that the drug precipitation risks of formulation Ib during manufacturing and patient in-use administration were quite low, especially when the solutions were essentially free of foreign participates in a well-controlled aseptic condition.

**Example 14: Formulation Development of Formulation Ic**

**Definition of formulation design space**

There are two primary constraints in the design of ICP formulation: (1) the supersaturation ratio of the formulation, which affects the stability of the drug product; (2) the total amount of Kleptose intake in the final dose, which affects the safety profile of the drug product. The supersaturation ratio and the total amount of Kleptose intake can be expressed as a function of drug concentration, Kleptose concentration, and the total drug dose in the following equations:

$$Supersaturation\ Ratio = \frac{Drug\ Concentration}{Equilibrium\ Solubility}$$

$$Total\ Kleptose\ Intake = \frac{Total\ Drug\ Dose}{Drug\ Concentration} \times Kleptose\ Concentration$$

Based on the solubility data shown in FIG. 40, the equilibrium solubility is linearly correlated with the Kleptose concentration and can be calculated by:

$$Equilibrium\ Solubility\ \left(\frac{mg}{mL}\right) = 0.0007 \times Kleptose\ Concentration\left(\frac{mg}{mL}\right) + 0.0014$$

Provided that the maximum fill volume is 50 mL in a 100cc vial and a maximum of 2 vials can be dosed to a patient, the interdependence between the supersaturation ratio, the total Kleptose intake, and the maximum dose to be delivered was plotted in FIG. 40.

Preferably, the supersaturation ratio of the formulation should be below 1 (i.e. non-supersaturated). The Kleptose intake should be less than its permitted daily exposure (PDE) threshold of 300 mg/kg/day. A toxicology assessment based on commercial IV drug product suggested a Kleptose daily dose below 8 g/day. The maximum dose that can be delivered within the formulation design space was less than 6 mg. As the top dose of the intended commercial product (ICP) formulation was set at 3.6 mg/day, a Kleptose-based formulation can be identified within the design space to balance between the supersaturation level and the total Kleptose intake.

**Evaluation of Prototype Formulations**

A series of feasibility batches were manufactured at the lab scale (3-15 L) with varied drug concentration, Kleptose concentration, fill volume, and vial size. The same lyophilization cycle as used in formulation Ib were adopted for all the feasibility batches. The study has two objectives: (1) to demonstrate the feasibility of delivering a dose ranging from 1 mg up to 6 mg in a Kleptose-based formulation; (2) to evaluate the impact of formulation presentation (fill volume and vial size) on the critical quality attributes of the drug product such as the cake appearance, reconstitution time, and the residual formic acid level. The formulation compositions are shown in Table 54.

**Table 54: Prototype formulations evaluated in the feasibility batches**

| | Kleptose conc. (mg/mL) | Drug Loading (mg/ vial) | Drug conc. (mg/mL) | Fill volume (mL/ vial) | Vial size (mL) |
|---|---|---|---|---|---|
| 1 | 100 | 2 | 0.075 | 26.7 | 50 |
| 2 | | 1 | 0.125 | 8 | 30 |
| 3 | | 1 | 0.125 | 8 | 20 |
| 4 | | 1.5 | 0.125 | 12 | 30 |
| 5 | | 1.5 | 0.125 | 12 | 50 |
| 6 | | 1.5 | 0.125 | 12 | 100 |
| 7 | | 2 | 0.125 | 16 | 30 |
| 8 | | 2 | 0.125 | 16 | 30 |
| 9 | | 2 | 0.125 | 16 | 30 |
| 10 | | 3 | 0.125 | 24 | 50 |
| 11 | | 3 | 0.125 | 24 | 100 |
| 12 | | 5 | 0.125 | 40 | 100 |
| 13 | | 6.25 | 0.125 | 50 | 100 |
| 14 | 150 | 1.8 | 0.075 | 24 | 50 |
| 15 | | 1.8 | 0.075 | 24 | 100 |
| 16 | | 3 | 0.075 | 40 | 100 |
| 17 | | 2 | 0.08 | 25 | 50 |
| 18 | | 1 | 0.125 | 8 | 20 |
| 19 | | 2 | 0.125 | 16 | 30 |

(continued)

| | Kleptose conc. (mg/mL) | Drug Loading (mg/vial) | Drug conc. (mg/mL) | Fill volume (mL/vial) | Vial size (mL) |
|---|---|---|---|---|---|
| 20 | 200 | 2 | 0.08 | 25 | 50 |
| 21 | | 2 | 0.08 | 25 | 50 |
| 22 | | 4 | 0.1 | 40 | 100 |
| 23 | | 4 | 0.1 | 40 | 100 |
| 24 | | 5 | 0.1 | 50 | 100 |
| 25 | | 5 | 0.1 | 50 | 100 |

The lyophilized samples of each formulation were tested on the cake appearance, the assay and impurities, the residual solvent, and the reconstitution time. The test results are summarized in Table 55. As the formulation components and the manufacturing process of those prototype formulations are very similar to those of formulation Ib, the stability profiles of the lyophilized drug products were expected to be comparable to that of formulation Ib. Therefore, stability evaluation was not performed in these feasibility batches.

**Table 55 Test results of prototype formulations in the feasibility batches**

| | FP Assay[a] (% LC) | Total Impurities (%) | Residual FA[a] (mg/vial) | Recon volume (mL) | Recon Time[b] (sec) |
|---|---|---|---|---|---|
| 1 | 100 | 1.47 | 5.99 | 16.6 | 31.5 |
| 2 | 102 | 0.04 | 0.98 | 5 | 42.5 |
| 3 | 100 | 0.88 | 1.23 | 4.5 | 50.5 |
| 4 | 99.3 | ND | 1.76 | 7.5 | 40 |
| 5 | 100.7 | 0.11 | 1.61 | 7.5 | 45 |
| 6 | 100.7 | ND | 1.59 | 7.5 | 42 |
| 7 | 100.73 | NT | 3.17 | 9 | 135 |
| 8 | 98 | ND | 2.92 | 9 | 36.5 |
| 9 | 100 | 0.65 | 3.54 | 10 | 40 |
| 10 | 99.7 | 0.13 | 3.56 | 15 | 48.5 |
| 11 | 100 | 0.2 | 3.57 | 15 | 51.5 |
| 12 | 97.4 | 0.04 | 6.48 | 25 | 44 |
| 13 | 96 | 0.07 | 10.23 | 31.25 | 53.5 |
| 14 | 98.3 | 0.51 | 5.5 | 22.5 | 74 |
| 15 | 98.9 | 0.5 | 5.08 | 22.5 | 91.5 |
| 16 | 99 | 0.09 | 8.26 | 37.5 | 85 |
| 17 | 100 | 1.64 | 7.54 | 23.4 | 73.5 |
| 18 | 100 | 1.24 | 2.13 | 7.5 | 60 |
| 19 | 100 | 1.06 | 6.38 | 15 | 63 |
| 20 | 92.00 | NT | 9.74 | 25 | 237 |
| 21 | 99 | ND | 9.45 | 25 | 120 |
| 22 | 94 | NT | 16.93 | 40 | 310 |
| 23 | 101 | ND | 15.42 | 40 | 133 |
| 24 | 94 | NT | 22.96 | 50 | 261 |

(continued)

| | FP Assay[a] (% LC) | Total Impurities (%) | Residual FA[a] (mg/vial) | Recon volume (mL) | Recon Time[b] (sec) |
|---|---|---|---|---|---|
| 25 | 100.3 | ND | 21.15 | 50 | 120 |

The twenty-five prototype formulations covered a wide range of drug load of 1-6.25 mg/vial. The Kleptose concentration varied from 10% to 20%, the drug concentration varied from 0.075 mg/mL to 0.125 mg/mL, and the fill volume varied from 8 mL to 50 mL in different vial sizes. All the lyophilized vials showed elegant cake appearances and acceptable assay/impurity values. The reconstitution time was less than 5 minutes for all the prototype formulations. The formulations with 20% Kleptose tended to have longer reconstitution time than those with 10% or 15% Kleptose, which may be attributed to the increased viscosity of the reconstituted solution at a higher Kleptose concentration. Overall, the Kleptose-based formulation demonstrated its robustness despite of the differences in Kleptose concentration, drug concentration, fill volume and vial size.

The residual formic acid level varied in a wide range across the different prototype formulations. It was found that the efficiency of residual formic acid removal was negatively correlated with the total amount of Kleptose in the vial. As shown in FIG. 41, the percent of formic acid removed by the same lyophilization cycle decreased with the increase of Kleptose amount. It can be explained by the propensity of Kleptose entrapping solvent in its complex cavity.

The impact of cake thickness on the residual formic acid level was also investigated. As shown in FIG. 42, the residual formic acid level was independent of the fill height given the same Kleptose amount in the vial. It suggests that the increased mass transfer resistance in a thicker lyophilized cake may have minimal impact on the residual formic acid level. Rather, the residual formic acid level increased with the increase of Kleptose amount, which is consistent with the trend observed in FIG. 41.

It is known that the residual formic acid level in formulation Ib is about 0.9 mg/vial for 1 mg dose in 20cc vial presentation and the release specification of residual formic acid is NMT 2.5 mg per mg of API so that the total daily intake of formic acid can be kept below ICH limit of 50 mg/day for a top dose of 20mg. As a comparison with formulation Ib, the residual formic acid per mg of Compound 1 of all the 25 prototype formulations are plotted as a function of Kleptose concentration in FIG. 43. Taking account of various drug loadings and vial size presentations, the residual formic acid level is 1.0-1.8 mg/mg Compound 1 at a Kleptose concentration of 100 mg/ml (same as formulation Ib), 2.1-3.8 mg/mg Compound 1 at a Kleptose concentration of 150 mg/ml, and 3.9-4.9 mg/mg Compound 1 at a Kleptose concentrating of 200 mg/ml. Therefore, the residual formic acid of all the prototype formulations, regardless of the Kleptose concentration, should be well below the ICH threshold for a top dose of 3.6 mg.

## Final formulation selection

As all the prototype formulations were deemed feasible with acceptable assay and impurity, residual solvent level, and reconstitution time, the DPD team selected a formulation in which 0.08 mg/mL Compound 1 was dissolved in 150 mg/mL Kleptose with the aid of formic acid. With reduced drug concentration and increased Kleptose concentration, this new formulation rendered undersaturated bulk solution and reconstituted solution. As a result, it eliminates potential drug precipitation risks posed in formulation Ib. The formulation is referred as formulation Ic. Alternatively, a formulation in which 0.08 mg/mL API was dissolved in 100 mg/mL Kleptose with the aid of formic acid can also be considered. This formulation is referred as formulation Ibm, reduced the supersaturation ratio of formulation Ib from 2x to 1x, thus alleviating the potential drug precipitation risks. The formulation compositions of formulation Ic is listed in Table 56 in comparison with formulation Ibm.

**Table 56: Composition of formulation Ic in comparison with formulation Ibm**

| | Formulation Ibm | Formulation Ic |
|---|---|---|
| Compound 1 | 1.0 mg/vial | 1.0 mg/vial |
| Kleptose® HPB (HP-β-CD), parenteral grade | 1275 mg/vial | 1875 mg/vial |
| Formic acid (in process solvent) | Partially removed upon drying | Partially removed upon drying |
| Water for injection (in process media) | Removed upon drying | Removed upon drying |

**Example 15: Evaluation of Formulation Stability**

Formulation stability evaluation contains three parts: (1) the physical and chemical stability of bulk solution, which determines the maximum hold time in the manufacturing process; (2) the ICH stability testing of lyophilized cake, which determines the shelf life of finished drug product; (3) the physical and chemical stability of reconstituted drug solution in the vial and in the IV bag, which determines the in-use time for the drug administration to a patient.

**Stability of Bulk Solutions**

The bulk solution of formulation Ic consisted of 150 mg/ml Kleptose, 0.08 mg/ml Compound 1, and 0.98 mg/ml formic acid. The bulk solution had the pH of 2.8, and the drug concentration of the bulk solution was below its equilibrium solubility so that the supersaturation risks embedded in formulation Ib was eliminated in formulation Ic. Therefore, the physical and chemical stability of bulk solution Ic were expected to be superior to that of formulation Ib. The 'hold-time' study of the bulk solution was performed in the engineering batch later and confirmed an up to 24-hour solution stability at ambient condition.

**Stability of Finished Drug Products**

The drug product stability of formulation Ic was evaluated using the vials from a development batch. The stability results are shown in Table 57. The drug products remained stable with no major changes in appearance, assay and impurities, and reconstitution time after 6 months of storage at both 25°C/60%RH and 40°C/75%RH condition. This result is used to support a shelf-life of up to 18 months at ambient condition for formulation Ic.

**Table 57: Drug product stability of formulation Ic**

| Test | Acceptance Criterion | t=0 | 25°C/60%R H | 40°C/75%RH | | |
|---|---|---|---|---|---|---|
| | | | 6 Month | 1 Month | 3 Month | 6 Month |
| Appearance | Cake or powder | Cake | Cake | Cake | Cake | Cake |
| Color | White to off-white | White | White | White | White | White |
| Water Content | Report results | 0.06% | 0.08% | 0.09% | 0.11% | 0.17% |
| Recon Time[1] | Report results | 118 seconds | 106 seconds | 116 seconds | 101 seconds | 150 seconds |
| Recon pH | NLT 2.7 | 3.2 | 3.2 | 3.1 | 3.2 | 3.2 |
| Assay | 90.0 to 110.0% of LC | 100.0% | 98.6% | 100.0% | 98.9% | 98.6% |
| Related Impurities | Individual: NMT 1.0% Total: NMT | Individual: 0.08% Total: | Individual: <0.05% Total | Individual <0.05% Total<0.0 | Individual: <0.05% Total | Individual: <0.05% Total |

**Example 16: Lyophilization process optimization of Formulation Ic**

**I. Thermal Characterization of Ic Formulation**

The thermal analysis was conducted with the freeze-drying microscope (FDM) and the low temperature differential scanning calorimetry (LT-DSC) on Ib and Ic formulations. Formulation Ic had a drug concentration of 0.125 mg/ml. A summary of the results is shown in Table 58. Formulation Ic exhibited similar collapse temperature and Tg' as Formulation Ib. Based on this result, the recommended product temperature during the primary drying of the lyophilization process is -11°C to -12°C, 2-3 degrees below the collapse temperature.

**Table 58: Collapse temperature and Tg' of Formulations Ib and Ic:**

| | Formulation Ib | Formulation Ic |
|---|---|---|
| Tg' (DSC) | -11.4°C | -11.0° |

(continued)

| | Formulation Ib | Formulation Ic |
|---|---|---|
| Collapse Onset Temperature (FDM) | -9.3°C | -9.0° |

**II. Lyophilization modeling**

Instead of running trial and error lyophilization experiments, a lyophilization model developed by Professor Michael Pikal was utilized in the first place to predict the product temperature profiles at different drying conditions. The existing thermocouple data collected from three Formulation Ib development batches were used as the model input to back calculate the mass transfer resistance coefficient ($R_p$) as a function of the dry layer thickness ($L_{dry}$) as well as the heat transfer coefficient ($k_v$). Then the coefficient R0, A1, and A2 were derived from the curve fitting based on the equation below. The calculated parameters are shown in Table 59.

$$Rp = R0 + \frac{A1 \times Ldry}{1 + (A2 \times Ldry)}$$

**Table 59: Calculated heat transfer coefficient and product resistance**

| Lot# | Thermocouple # | Product resistance ($R_p$) | | | Heat transfer coefficient ($K_v$) |
|---|---|---|---|---|---|
| | | R0 | A1 | A2 | |
| A | TP1 | 0 | 64.32 | 2.9 | 4.49E-04 |
| | TP2 | 0 | 61.69 | 3.7 | 4.04E-04 |
| | TP3 | 1.72 | 100 | 3.4 | 7.60E-04 |
| | TP4 | 0 | 54.19 | 2.5 | 4.78E-04 |
| B | TP1 | 0 | 60.5 | 2.3 | 5.45E-04 |
| | TP2 | 0 | 58.8 | 3.6 | 5.26E-04 |
| | TP3 | 0 | 74.6 | 3.0 | 5.58E-04 |
| C | TP1 | 1.68 | 100 | 4.0 | 9.49E-04 |
| | TP2 | 0 | 56.5 | 3.1 | 5.50E-04 |
| | TP3 | 0 | 87.8 | 3.6 | 5.43E-04 |
| | TP4 | 0 | 75.4 | 2.9 | 4.84E-04 |
| Median | | 0 | 64.32 | 3.1 | N/A |

Due to the similar Tg' and collapse temperature between Formulations Ib and Ic, the calculated $R_p$ and $K_v$ values from Table 59 were applied in the model prediction of Gen2c formulation, along with other known parameters as listed in Table 60.

As center vials have lower $K_v$ than edge vials ($4.04\times10^{-4}$ vs. $9.49\times10^{-4}$), center vials require longer drying time than edge vials, whereas edge vials tend to have higher product temperature than center vials, resulting in a higher risk of cake collapse. Therefore, $K_v$ of center vials were used to estimate the drying time and $K_v$ of edge vials were used to estimate product temperature at different shelf temperature (Ts) conditions as the worst case of scenario in the modeling. As shown in Table 61, at a shelf temperature of 10°C, the predicted product temperature is approximately -12°C, close to the recommended product temperature from FDM study above. At the same shelf condition, the predicted primary drying time is approximately 20 hours for 1.0 mg dose in 20cc vial, as opposed to 70 hours in formulation Ib lyophilization cycle.

**Table 60: Key input parameters used in lyophilization modeling**

| Model parameter | Value | Comments |
|---|---|---|
| R0 | 0.1 | From model built; avoid value of 'R0 = 0' which results in computational error |
| A1 | 64.32 | |
| A2 | 3.1 | |
| Kv, $10^{\wedge 4}$ cal.$s^{-1}$.$cm^{-2}$.$K^{-1}$ | 4.04 (center) 9.49 (edge) | From model built - |
| Fill volume, mL | 8 | Product presentation |
| Solids content, g/g | 0.0972 | Formulation parameter |
| Inner CSA (Ap), cm2 | 5.87 | Vial dimensions |
| Outer CSA/Inner CSA (Av/Ap) | 1.18 | |
| Shelf Temp (Ts), °C | -16 °C | Operating conditions |
| Chamber pressure, mtorr | 140 | |

**Table 61: Predicted primary drying time and product temperature**

| Formulation Ic | Predicted Primary Drying Time (Center vials) | | | |
|---|---|---|---|---|
| | Ts=-5°C | Ts=0°C | Ts=5°C | Ts=10°C |
| 20cc vial (1 mg dose, 12.5 mL fill) | 34.8 hr | 28.3 hr | 23.7 hr | 20.4 hr |
| 50cc vial (2 mg dose, 25 mL fill) | 68.9 hr | 56.0 hr | 47.0 hr | 40.3 hr |
| | **Predicted Product Temperature (edge vials)** | | | |
| | Ts=-5°C | Ts=0°C | Ts=5°C | Ts=10°C |
| 20cc vial (1 mg dose, 12.5 mL fill) | -16.8°C | -14.9°C | -13.2°C | -11.6°C |
| 50cc vial (2 mg dose, 25 mL fill) | -16.9°C | -15.0°C | -13.3°C | -11.8°C |

**Example 17: Lyophilization cycle development for 3-L bach**

Based on the modeling results, one 3-L lyophilization batch of Formulation Ic was manufactured using a lab scale lyophilizer (Model SP Virtis Genesis 25 EL). The drug products were 2 mg/vial drug loading, or 25 ml fill in a 50cc vial. The lyophilization cycle parameters are shown in Table 62. The shelf temperature and chamber pressure in the primary drying stage were set at 10°C and 250 microns, respectively.

**Table 62: Lyophilization cycle parameters for lab scale batch 1**

| Step | Shelf Temp. Setpoint (°C) | Soak Time (hours) | Ramping Rate (°C/ hour) | Pressure Setpoint |
|---|---|---|---|---|
| Product Loading/ Freezing | 25 | 0.5 | | Evac. To 12 psia to ensure chamber is airtight |
| | | | 30 | |
| Freezing | -40 | 6 | | |
| | | | 30 | |
| Primary Drying | 10 | 90 | | 250 microns |
| | | | 30 | 250 microns |
| Secondary Drying | 60 | 18 | | 250 microns |
| Stoppering | 25 | | | 14.7 PSIA |

Four thermocouples were put in two center vials and two edge vials respectively. The product temperature profile for lab scale batch 1 is shown in FIG. 45. The two edge vials displayed a product temperature of ∼-11°C as opposed to ∼-14°C from the two center vials during the primary drying. The 'break point', or the time point when the product temperature started to approach the shelf temperature, was 24 hours for edge vials and 35 hours for center vials. No cake collapse was observed at the end of the lyophilization cycle. This experimental result was in good agreement with the predictive modeling results shown in Table 61.

Subsequently, to have a better understanding of the product temperature change as a function of the shelf temperature, the same filled vials underwent another lyophilization cycle, in which the shelf temperature of primary drying was increased stepwise from 5°C up to 14°C. The hold time of each step was 2-6 hours and the temperature increased 3°C in every step. Then the shelf temperature was reduced to 10°C and held for 30 hours. The lyophilization cycle parameters are shown in Table 63.

**Table 63: Lyophilization cycle parameters lab scale batch 2**

| Step | Shelf Temp. Setpoint (°C) | Soak Time (hours) | Ramping Rate (°C/ hour) | Pressure Setpoint |
|---|---|---|---|---|
| Product Loading/ Freezing | 25 | 0.5 | | Evac. To 12 psia to ensure chamber is airtight |
| | | | 30 | |
| Freezing | -40 | 6 | | |
| | | | 30 | |
| Primary Drying | 5 | 6 | 30 | 250 microns |
| | 8 | 3 | 30 | 250 microns |
| | 11 | 3 | 30 | 250 microns |
| | 14 | 2 | 30 | |
| | 10 | 30 | 30 | 250 microns |
| Secondary Drying | 60 | 18 | | 250 microns |
| Stoppering | 25 | | | 14.7 PSIA |

Three thermocouples were placed in one edge vial, one center vial, and one vial between edge and center, respectively. The product temperature profile for lab scale batch 2 is shown in FIG. 46. The product temperatures measured by each thermocouple under each shelf temperature condition were summarized in Table 64**Error! Reference source not found.**. It was found that the product temperature increased 1°C for every incremental increase of 3°C on the shelf temperature. When the shelf temperature reached 11°C, the vials in the middle and center still had product temperature below -11°C whereas the edge vial showed a product temperature of -9.6°C, exceeding the collapse temperature. Nonetheless, no signs of cake collapse were observed in any of the lyophilized vials including the edge vials. When the shelf temperature was increased to 14°C, the product temperature already passed the 'break point', implying that the primary drying is close to completion. Therefore, the elevated temperature did not cause any cake collapse.

**Table 64: Lyophilization cycle parameters lab scale batch 2**

| | Product Temperature (°C) | | |
|---|---|---|---|
| **Shelf Temperature (°C)** | **TC #5 (edge vial)** | **Tc #7 (vial in the middle row)** | **TC #8 (center vial)** |
| 5 | -12.1 | -13.7 | -15.3 |
| 8 | -10.7 | -12.3 | -13.6 |
| 11 | -9.6 | -11.3 | -12.5 |

Combining the results from the two lyophilization runs, the recommended shelf temperature and chamber pressure of primary drying were determined to be 10°C and 250 microns, respectively.

**Example 18: Lyophilization cycle development for scale-up batches**

Following the two trial runs, three 15-L batches (Ic-1, Ic-2 and Ic-3) of formulation Ic were manufactured using a lab lyophilizer (Model: Millrock Magnum®). Each batch included two sublots, one for 1 mg/vial dose and the other for 4 mg/vial dose. Batch Ic-1 and Ic-2 used 20cc vial for 1.0 mg dose and 100cc vial for 4 mg dose. As a small percentage of vial breakage in 1.0 mg dose sublot was observed in both batches and was attributed to the relatively high fill volume in the 20cc vial, a larger vial size of 50cc was used for 1.0 mg dose in Batch Ic-3 instead. The formulation composition and presentation of each sublot are shown in Table 65.

**Table 65: Formulation presentations of development batches**

| Batch No. | Drug Loading (mg/vial) | Kleptose (mg/vial) | Fill volume (mL/vial) | Vial size (mL) |
|---|---|---|---|---|
| Ic-1-F1 | 1 | 1875 | 12.5 | 20 |
| Ic-1-F2 | 4 | 7500 | 50 | 100 |
| Ic-2-F1 | 1 | 1875 | 12.5 | 20 |
| Ic-2-F2 | 4 | 7500 | 50 | 100 |
| Ic-3-F1 | 1 | 1875 | 12.5 | 50 |
| Ic-3-F2 | 4 | 7500 | 50 | 100 |

The main variables being evaluated in the three development batches are: shelf temperature and chamber pressure of primary drying, as well as shelf temperature and hold time of secondary drying. The lyophilization cycle conditions of each batch are summarized in Table 66.

**Table 66: Lyophilization cycle parameters of development batches**

| | Ic-1-F1 | Ic-2-F1 | Ic-3-F1 |
|---|---|---|---|
| Loading temp (°C) | 20 | | |
| Freezing temp (°C) | -50 | | |
| Freezing rate (°C/min) | 0.5 | | |
| Freezing hold time (minutes) | 360 | | |
| Drying ramp rate (°C/min) | 0.5 | | |
| PD shelf temp (°C) | 14 | 8 | 10 |
| PD chamber pressure (microns) | 275 | 225 | 250 |
| PD hold time (minutes) | 6060 | 7840 | 3300 |
| SD shelf temp (°C) | 60 | 40 | 60 |
| SD chamber pressure (microns) | 275 | 225 | 250 |
| SD hold time (minutes) | 1080 | 1080 | 2160 |
| Stoppering temp (°C) | 20 | | |

**I. Impact of primary drying on product temperature and cycle time**

The product temperature profiles of the three development batches are shown in FIG. 47- FIG. 49. The key characteristics including the product temperature during primary drying and the time to reach the end point of primary drying are summarized in Table 67. The product temperature was -10°C, -11°C, and -13°C respectively in correspondence to a shelf temperature of 14°C, 10°C, and 8°C, which were consistent with the findings from the previous batches described in Example 37. No cake collapse was observed in any of the three batches including the one with a shelf temperature of 14°C which has a corresponding product temperature of -10°C. This is an evidence showing the process robustness of formulation Ic.

**Table 67 Characterization of lyophilization development batches**

| Batch No. | PD Product Temp (°C) | Time to reach cross-over temp (hours) | Time for pirani gauge to converge with CM (hours) |
|---|---|---|---|
| Ic-1-F1 | -10 | 27 | 83 |
| Ic-1-F2 | | 45 | |
| Ic-2-F 1 | -13 | 38 | 83 |
| Ic-2-F2 | | 54 | |
| Ic-3-F1 | -11 | 17 | NA |
| Ic-3-F2 | | 53 | |

There are different approaches to determine the end point of primary drying. The most conservative way is to look at the time point when the pirani gauge pressure curve is fully converged with the capacitance manometer pressure curve, which indicates that no water vapor is sublimated from the dried cake any more. An alternative way is to look at the time point when the product temperature is crossing over with the shelf temperature, which also indicates the completion of sublimation. The only caveats of the latter approach are that the thermocouple must be placed to the bottom of the vial and the vials with the thermocouple tends to have slightly higher product temperature than the vials without. Therefore, an extra 10-30% of drying time is always added after the cross-over temperature is reached as a cushion at the end of primary drying. In this study, as each batch contained two sublots with different fill volume and vial size, the first approach was unable to reflect the end point of primary drying for each sublot, the second approach was employed to determine the primary drying time of each individual sublot. As shown in Table 67, in the first two batches, sublots Ic-1-F2 and Ic-2-F2 (4 mg dose) required approximately 1.5 folds of drying time of sublots Ic-1-F1 and Ic-2-F1 (1.0 mg dose) due to increases cake thickness. Batch Ic-2 showed an additional 20-40% drying time than Batch Ic-1 due to a lower shelf temperature. In Batch Ic-3, the drying time of sublot Ic-3-F1 was shortened to 17 hours as opposed to 27 hours in Batch Ic-1-F1 despite of a lower shelf temperature at 10°C, mainly because of the reduced cake thickness of 10 mm in a larger 50cc vial size as opposed to a cake thickness of 18 mm in 20cc vial. Based on this result, the recommended primary drying condition is a shelf temperature at 10°C, a chamber pressure at 250 microns, and a hold time of 20 hours for a 1.0 mg dose filled in a 50cc vial.

**II. Impact of cycle condition on residual water**

Two critical quality attributes of the final lyophilized drug product are water content and residual solvent level. To evaluate the removal rate of water and formic acid during lyophilization, sample vials were pulled at different time points of drying step in Batch Ic-1 and Ic-2. The residual water content of each sample was measured by Karl Fisher method and the results are shown in FIG. 50. The first four samples were pulled in primary drying step between the time point when cross-over temperature was reached and the time point when primary drying ended. The last two or three samples were pulled in secondary drying step between the time point when secondary drying lasted for four to six hours and the end of secondary drying. The data showed that by the time when the cross-over temperature was reached, the residual moisture in the lyophilized cake was already below 0.2%. Additional primary drying time allowed the residual moisture to reduce to 0.06%. This suggested that majority of water removal took place during primary drying step. In the secondary drying step, the residual moisture level was further decreased to 0.04% in the first four to six hours and then became plateaued. The residual moisture of the finished drug products from both batches ended up being at the same level of 0.04% regardless of the cake size or drying temperature difference. Likewise, in Batch Ic-3, the residual moisture level of the finished drug products was 0.03% for both sublots despite of a prolonged secondary drying time of 36 hours. In conclusion, a secondary drying time of 18 hours and a drying temperature of 40°C-60°C were sufficient for residual moisture removal of formulation Ic.

**III. Impact of cycle condition on residual formic acid**

The change of residual formic acid with drying time was also evaluated by pulling sample vials at different time points from the end of primary drying to the end of secondary drying. The results are shown in FIG. 51. Batch Ic-1 and Ic-2 both had a secondary drying time of 18 hours. However, Batch Ic-1 had a higher secondary drying temperature of 60°C than 40°C of Batch Ic-2. As a result, Batch Ic-1 exhibited a faster residual formic acid removal rate than Batch Ic-2. The final residual formic acid in Ic-1 was only 60% of that in Batch Ic-2 for both sublots. Therefore, secondary drying temperature was a critical process parameter with significant impact on residual solvent level of finished drug product.

The residual formic acid level also varied between the two sublots in the same lyophilization batch. In both batches, sublot F1 (1.0 mg dose) showed only 80% of the residual formic acid level as in sublot F2 (4mg dose). It was not surprising because residual formic acid level was found to increase with the increased Kleptose amount in the lyophilization vial.

It was also observed from the first two batches that the residual formic acid level did not reach a plateau at the end of the 18-hr secondary drying. Therefore, the secondary drying was extended to 36 hours at 60°C in Batch Ic-3. As shown in FIG. 51, the residual formic acid level was reduced from 0.15% to 0.08% in sublot F1 when the drying time was doubled to 36 hours. sublot F2 followed the same trend. After the vials were unloaded from the lyophilizer upon the completion of the lyophilization cycle, a few F1 and F2 vials were placed into 60°C vacuum oven for an additional 24 hours. The residual formic acid started to show a plateau at 16-hr time point in the vacuum oven and reached the lowest level at 0.01% and 0.03% for sublot F1 and F2, respectively at the end of 24-hour oven drying.

According to ICH Q3 guidance, PDE of formic acid is 50 mg/day as a Class 3 solvent. Given a top dose of 4 mg/day, the maximum allowable residual formic acid level is 12.5 mg/mg of API, or 0.6%w/w of the cake weight. Based on the data shown in FIG. 51, the residual formic acid level was less than 0.2% after 18 hours of secondary drying, way below the ICH threshold. Double the drying time to 36 hours only provides limited residual formic acid reduction from 0.2% to approximately 0.1%. Therefore, the team recommended a secondary drying time of 18-24 hours at 60°C for formulation Ic of different vial presentations to keep the residual formic acid level below 0.4%w/w. This residual formic acid level of formulation Ic was comparable to the release specification of NMT 0.3%w/w in formulation Ib.

**IV. Impact of freezing rate on cycle time and residual formic acid**

It was reported in some literatures that the freezing rate may have impact on the primary and/or secondary drying efficiency due to the changes in the ice crystal morphologies. Therefore, a study was performed to evaluate the effect of freezing rate on the drying time and the residual solvent level of finished drug products. Two 10-L lyophilization batches of formulation Ic were manufactured. Each lyophilization batch contained three sublots, namely 1.0 mg in 50cc vial, 2 mg in 50cc vial, and 4 mg in 100cc vial. The freezing rates used in the two lyophilization batches were 0.1°C/min and 1.0°C/min, respectively, as opposed to the freezing rate of 0.5°C/min used in previous lyophilization batches. The lyophilization cycle parameters of the two batches are described in Table 68.

**Table 68: Lyophilization cycle parameters**

| | Batch C1 | Batch C2 |
|---|---|---|
| Loading temp (°C) | 20 | |
| Freezing temp (°C) | -50 | |
| Freezing rate (°C/min) | 0.1 | 1.0* |
| Freezing hold time (minutes) | 360 | |
| Drying ramp rate (°C/min) | 0.5 | |
| PD shelf temp (°C) | 10 | |
| PD chamber pressure (microns) | 250 | |
| PD hold time (minutes) | 4170 | 4068 |
| SD shelf temp (°C) | 60 | |
| SD chamber pressure (microns) | 250 | |
| SD hold time (minutes) | 1080 | |
| Stoppering temp (°C) | 25 | |
| *: the actual freezing rate achieved is 0.85°C/min. | | |

As shown in FIG. 52 and FIG. 53, the product temperature profiles of the two batches were comparable to each other despite of the freezing rate difference. The product temperatures and the primary drying time of all the sublots at the 'break' point are summarized in Table 69. The 'break' product temperatures were similar across different sublots. As expected, the 'break' time increased with the increase of dose or cake size, and the center vials displayed longer sublimation time than the edge vials in both batches. Batch C1, with a higher freezing rate, showed slightly shorter primary drying time than Batch C2.

**Table 69: 'Break point' product temperature and drying time**

| Thermocouple location | Batch C1 | | Batch C2 | |
|---|---|---|---|---|
| | Break Temperature (°C) | Break Time (hours) | Break Temperature (°C) | Break Time (hours) |
| 1 mg Center | -19.0 | 9.6 | -16.8 | 7.2 |
| 1 mg Front Closest | -18.4 | 8.5 | -16.3 | 6.8 |
| 1 mg Front Furthest | -17.3 | 8.8 | -12.9 | 7.3 |
| 2 mg Center | -16.7 | 25.3 | -16.1 | 25.5 |
| 2 mg Front Closest | -15.2 | 19.8 | -16.6 | 16.6 |
| 2 mg Front Furthest | -16.2 | 22.4 | -16.4 | 17.3 |
| 4 mg Center | -17.6 | 40.2 | -16.5 | 20.8 |
| 4 mg Center | -16.8 | 40.8 | -16.6 | 37.3 |
| 4 mg Front Closest | -17.8 | 33.3 | -16.4 | 26.1 |
| 4 mg Front Furthest | -18.6 | 28.8 | -16.6 | 25.4 |

The finished lyophilization vials from the two batches showed similar cake appearance, as shown in FIG. 54. The residual formic acid, residual moisture, and reconstitution time of the finished lyophilization vials from the two batches were also tested. The testing results are shown in Table 70. The reconstitution time was comparable between the two batches. The residual formic acid levels of each sublot in Batch C2 were slightly lower than their counterparts from Batch C1. Interestingly, the residual moisture levels demonstrated an opposite trend, showing a higher level in Batch C2. It is known that faster freezing rate leads to smaller ice crystals due to shorter nucleation time. As a result, the mass transfer resistance during primary drying may be increased due to smaller pore sizes of dried cake. On the other side, the desorption efficiency of secondary drying may be increased due to increased surface area of dried cake. It is speculated that removal of water relies mainly on the primary drying and removal of formic acid relies more on the secondary drying. That may explain the differences in residual formic acid and residual moisture levels between the two batches. Nonetheless, the difference between the two batches were deemed not substantial, suggesting that the freezing rate had no significant impact on the critical quality attributes of finished drug product. As a result, the team decided to keep the freezing rate at 0.5°C/min for future batches

**Table 70: Finished drug product testing of Batches C1 and C2**

| Sample Name | Location | Batch C1 | | | Batch C2 | | |
|---|---|---|---|---|---|---|---|
| | | Residual FA (mg/vial) | Moisture (%) | Recon Time (sec) | Residual FA (mg/vial) | Moisture (%) | Recon Time (sec) |
| 1.0 mg dose | Front Edge | 2.31 | 0.05 | 57 | 1.53 | 0.15 | 68 |
| | Edge | 2.13 | 0.04 | | 1.72 | 0.16 | |
| | Center | 2.05 | / | | 1.26 | / | |
| | Average | 2.16 | 0.04 | | 1.50 | 0.15 | |
| 2.0 mg dose | Front Edge | 4.78 | 0.04 | 48 | 4.27 | 0.11 | 48 |
| | Edge | 4.70 | 0.03 | | 3.65 | 0.09 | |
| | Center | 3.83 | / | | 3.52 | / | |
| | Average | 4.44 | 0.04 | | 3.81 | 0.10 | |

(continued)

| Sample Name | Location | Batch C1 | | | Batch C2 | | |
|---|---|---|---|---|---|---|---|
| | | Residual FA (mg/vial) | Moisture (%) | Recon Time (sec) | Residual FA (mg/vial) | Moisture (%) | Recon Time (sec) |
| 4.0 mg dose | Front Edge | 11.04 | 0.04 | 51 | 9.37 | 0.08 | 71 |
| | Edge | 8.85 | 0.03 | | 9.42 | 0.09 | |
| | Center | 8.58 | / | | 7.38 | / | |
| | Back Edge | 10.72 | / | | 9.82 | / | |
| | Average | 9.80 | 0.04 | | 9.00 | 0.09 | |

**Example 19: Final process for formulation Ic**

A detailed description of the process procedures of formulation Ic is as follows:
Compounding: Kleptose® is dissolved in Water for Injection (WFI) in an appropriate-sized vessel (Vessel 1). Compound 1 is dissolved in formic acid (FA) in a separate vessel (Vessel 2). This Compound 1-FA premix solution is then added to Vessel 1 through an electronic pipette or a peristaltic pump at a constant rate (~ 50 μL per addition every 10 seconds) while the solution in Vessel 1 is being mixed with good vortex. Solution is visually inspected to make sure that no undissolved particles are present in Vessel 1. Following mixing, the batch weight is adjusted to the target weight with WFI.

Filtration: The bulk solution is then filtered using two 0.2 μm sterile filters in series. Prior to this step, the bulk solution will be pre-filtered using one 0.45 μm or 0.2 μm sterile filter.

Aseptic filling, lyophilization, and vial capping: Aseptic fill is performed in a 50cc vial with a target fill weight of 12.5 mL for a 1.0 mg dose strength. Lyophilization stoppers are then partially placed (to the first notch) on each filled vial. The lyophilizer is then loaded and the freeze-drying cycle is executed. After lyophilization is completed, vials are stoppered under reduced pressure in an atmosphere of nitrogen and sealed.

The lyophilization process parameters of formulation Ic (1.0 mg dose strength) are shown in Table 71. The total lyophilization cycle time of formulation Ic (1.0 mg dose strength) is about 2.6 days, only half of formulation Ib lyophilization cycle time.

**Table 71: Lyophilization cycle parameters of formulation Ic**

| Step | Shelf Temp. Setpoint (°C) | Soak Time (minutes) | Ramping Time (minutes) | Pressure Setpoint |
|---|---|---|---|---|
| Product Loading/ Freezing | 20 | 60 | | Evac. To 12 psia to ensure chamber is airtight |
| | | | 140 | |
| Freezing | -50 | 360 | | |
| | | | 120 | |
| Primary Drying | 10 | 1500 | | 250 microns |
| | | | 100 | 250 microns |
| Secondary Drying | 60 | 1440 | | 250 microns |
| Stoppering | 25 | | | 14.7 PSIA |

The process flow diagramsof formulation Ic is illustrated in FIG. 55.

**Example 20: Osmolality of reconstituted formulations with normal saline**

Formulation 1b (1mg/vial) was used in the study. Each vial was reconstituted with 4.5 mL normal saline (NS) to render a drug solution with a concentration of 0.2 mg/mL (the final solution volume upon reconstitution was 5 mL).

The osmolality of the reconstituted solution in the vial was measured by an osmometer (Advanced® Model 3250, Advanced Instruments Inc.) and an osmolality value of 440 mOsm/kg was obtained. Then 12 mL reconstituted solution was withdrawn from three vials and diluted with 38 mL normal saline to a final volume of 50 mL to obtain a dosing solution representative of clinical dose of 2.4 mg. The osmolality of the diluted dosing solution was measured to be 317 mOsm/kg. Subsequently, 30 mL reconstituted solution was withdrawn from six vials and diluted with 20 mL normal saline to a final volume of 50 mL to obtain a dosing solution representative of clinical dose of 6.0 mg. The osmolality of the diluted dosing solution was measured to be 371 mOsm/kg. The osmolality measurement results are summarized in Table 72 below.

Additionally, the drug product vial was reconstituted with 10 mL normal saline. The osmolality of the reconstituted solution was measured to be 352 mOsm/kg (Sample 4 in Table 72). It resulted in approximately 50 mL of the reconstituted solution for a dose of 4.8 mg.

**Table 72: Osmolality of formulation Ib reconstituted solutions**

| Sample No. | Dose | Dilution volume with NS | Osmolality of final dosing solution (mOsm/kg) |
|---|---|---|---|
| 1 | 1.0 mg | 4.5 mL NS recon, no dilution | 440 |
| 2 | 2.4 mg | 12 mL NS recon + 38 mL NS dilution | 318 |
| 3 | 6.0 mg | 30 mL NS recon + 20 mL NS dilution | 371 |
| 4 | 1.0 mg | 10 mL NS recon, no dilution | 352 |

The osmolality of the pure normal saline was measured to be 285 mOsm/kg. The osmolality of the reconstituted solution was found to be linearly correlated to the Kleptose concentration in the solution, as shown in FIG. 44.

Thus, formulation Ib, when reconstituted with 4.5 mL normal saline, renders a reconstituted solution of 440 mOsm/kg. For a dose range of 2.4-6 mg, the final dosing solution in the 50-mL normal saline bag has an osmolality range of 318-371 mOsm/kg. When the volume of the reconstituted NS or diluted NS is changed for a specific dose, the osmolality of the resulting solution can be interpolated from FIG. 44 based on the calculated HPBCD concentration.

Based on this data, provided that the drug concentration has little contribution to the osmolality, the osmolality of reconstituted solution of Formulation Ic in normal saline can be calculated. For formulation Ic, each vial contains 1 mg drug and 1875 mg Kleptose. When one vial is reconstituted with 12.5 ml normal saline, the reconstituted solution has an osmolality of about 416 mOsm/kg with 150 mg/ml Kleptose in the product vial. When the 45-mL reconstituted solution of 3.6 mg drug was diluted with normal saline to 50 ml, the dosing solution in the IV bag has an osmolality of about 383 mOsm/kg with 112.5 mg/ml Kleptose. This osmolality range, although higher than the plasma osmolality, was considered acceptable for IV infusion.

The reconstituted solution of formulation Ic consisted of 0.08 mg/ml Compound 1 and 150 mg/ml Kleptose. Comparing with reconstituted solution for formulation Ib, it has similar solution pH and higher Kleptose: drug ratio. In certain embodiments, reconstituted solution for formulation Ic has comparable chemical stability profile and improved physical stability than reconstituted solution of formulation Ib.

**Example 21: Solvent free combination**

Two 1-kg trial batches of solvent free Compound 1 formulations were prepared as shown in Tables 73 and 74. Batch B-1 was prepared with 10%w/w Kleptose at a target drug concentration of 40μg/mL. Batch B-2 was prepared with 20%w/w Kleptose at a target drug concentration of 80μg/mL. The target drug concentrations of both batches are ~60% of their saturation solubility at room temperature. Citrate buffer was used in the formulation to adjust the solution pH to 4.2 as Compound 1 is known to be chemically instable in solution above pH 5.

**Table 73: Formulation Compositions of Solvent-free Feasibility Batches**

| Batch No. | B-1 | B-2 |
|---|---|---|
| Compound 1 (mg/g) | 0.040 | 0.080 |
| Kleptose (mg/g) | 10 | 20 |
| Citric acid anhydrous (mg/g) | 2.21 | 2.19 |
| Sodium citrate anhydrous (mg/g) | 2.21 | 2.19 |
| WFI | q.s. to 1000 mg/g | q.s. to 1000 mg/g |

In the solvent-free compounding process, Kleptose was dissolved in water first. Then Compound 1 powder was added directly into Kleptose solution and mixed with a benchtop overhead homogenizer (POLYTRON PT 3100, Kinematica AG, Switzerland) at 6800 rpm. A jacketed compounding vessel was used to maintain the solution temperature at 20-25°C during the mixing process. For Batch B-1, mixing was continued for 24 hours. Then the solution was kept at room temperature without mixing for another 24 hours. For Batch B-2, mixing was continued for 48 hours. The solution samples were taken at t=4, 24, and 48 hours for in-process assay testing. The assay results are shown in Table 74.

**Table 74: In-process Assay Results of Solvent-free Feasibility Batches**

| Batch No. | | t=4 hr | t=24 hr | t=48 hr |
|---|---|---|---|---|
| B-1 | Concentration (mg/mL) | 0.016 | 0.022 | 0.024[c] |
| | Assay (% of target concentration[a]) | 39% | 53% | 58%[c] |
| B-2 | Concentration (mg/mL) | 0.052 | 0.062 | 0.069 |
| | Assay (% of target concentration[b]) | 61% | 72% | 81% |
| a: target concentration = 0.040 mg/g, or 0.041 mg/mL, with a solution density of 1.033 g/mL<br>b: target concentration = 0.080 mg/g, or 0.086 mg/mL, with a solution density of 1.069 g/mL<br>c: mixing stopped at t=24 hr. | | | | |

**Example 22: *In vitro* combination studies**

*In vitro* combination studies to assess the activity of Compound 1 were conducted in the following AML and solid tumor cell lines:

AML cell lines: MOLM-13, MV-4-11, OCI-AML-2, F-36-P, OCT-AML-3, NOMO-1, ML-2, KG-1, HNT-34 and HL-60.
Breast cancer cell lines: AU565, ZR-75-30, SK-BR-3, MCF-7 (E545K), BT-474 (K111N) AND CAL-51 (E542K).
Neuroendocrine tumor (NET) cell lines: COLO320DM, NCI-H727 and QGP-1; and
Renal cell carcinoma (RCC) cell lines: 786-O, A-498, ACHN and CAKI-1.

Cells were seeded into 384-well tissue culture plates at optimized seeding densities (50 microliters per well). Cells were grown at 37 °C with 5 % $CO_2$ for 1 day, then treated with compounds by adding 5 microliters of concentrated compound solutions to each well, and incubating at 37 °C with 5% carbon dioxide for 3 days. Cell viability was then measured by adding CellTitre-Glo® Luminescent Cell Viability reagent to the compound treated cells (20 microliters per well), incubating at room temperature for at least 20 minutes, then quantifying luminescent signal with a luminometer.

For the preliminary synergy analysis, the Bliss Independence Combination Index was used to calculate synergy for each Compound 1 combination treatment using the following equation (Foucquier, Pharma Res Per, 3(3), 2015):

$$CI = \frac{E_A + E_B - E_A E_B}{E_{AB}}$$

where $E_A$ and $E_B$ represent the effect of each single agent and $E_{AB}$ represents the effect of the combination at a given dose.

Subsequent synergy analysis was done using the Loewe additivity, Bliss independence, highest single agent (HSA), and cooperative effect synergy (CES) models as previously described (Veroli G., Bioinformatics, 32(18), 2016; Geary N., Am J Physiol Endocrinol Metab, 2012).

All data was analyzed using the CES equation:

$$CES = E_{AB} - \max (E_A, E_B)$$

In cases where single agent dose response fit a Hill slope, the CES analysis was compared to Loewe, Bliss, and HSA models.

FIG. 56A provides the effect of combinations of Compound 1 with 1) everolimus, 2) fedratinib, 3) midostaurin, and 4) pladienolide B. As seen in FIG. 56A, the dose response curves show $EC_{50}$ shifts with the tested compounds. For example, at all the tested dose levels, combinations with everolimus had lower $EC_{50}$ than single agent Compound 1.

FIG. 56B provides the effect of combinations of Compound 1 with pladienolide B in AML cell lines.

FIG. 56C provides the effect of combinations of Compound 1 with Compound A in AML cell lines.

FIG. 56D provides the effect of combinations of Compound 1 with Compound B in AML cell lines.

Table 75 provides a summary of synergy analysis for combinations of Compound 1 with exemplary second agents, including mTOR, JAK2, FLT3, spliceosome, BET, and LSD-1 inhibitors in AML cell lines.

**Table 75: Synergy analysis for combinations with Compound 1**

| Compound | Cell Lines | | | | | | | | | |
|---|---|---|---|---|---|---|---|---|---|---|
| | MOLM-13 | MV-4-11 | OCI-AML-2 | F-36-P | OCI-AML-3 | NOMO-1 | ML-2 | KG-1 | HNT-34 | HL-60 |
| Triptolide | Yes | No | No | No | No | No | No | No | No | No |
| Thapsigargin | No | No | No | No | No | No | No | No | No | No |
| Tanespimycin | No | No | No | No | No | No | No | No | No | No |
| Silvestrol | No | No | No | No | No | No | No | No | No | No |
| Salubrinol | No | No | No | No | No | No | No | No | No | No |
| Retaspimycin | Yes | Yes | Yes | No | No | No | No | No | No | No |
| Alvespimycin | No | Yes | Yes | No | No | No | No | No | No | No |
| CC-223 | Yes | Yes | Yes | Yes | Yes | Yes | Yes | No | No | No |
| CC-115 | Yes | Yes | Yes | Yes | Yes | Yes | Yes | No | Yes | No |
| Rapamycin | Yes | Yes | Yes | Yes | Yes | No | Yes | No | No | No |
| MLN-0128 | Yes | No | Yes | Yes | No | No | No | No | No | No |
| Everolimus | Yes | Yes | Yes | Yes | Yes | No | Yes | No | No | No |
| AZD8055 | Yes | No | Yes | Yes | Yes | Yes | No | No | No | No |
| STAT5i | No | No | No | No | No | No | No | No | No | No |
| Pladienolide B | Yes | Yes | Yes | Yes | No | Yes | No | No | No | No |
| Topotecan | Yes | Yes | No | No | No | No | No | No | No | No |
| Thioguanine | Yes | No | No | No | No | No | No | No | No | No |
| Mitoxantrone | Yes | Yes | No | No | No | No | No | No | No | No |
| Methotrexate | No | No | No | No | No | No | No | No | No | No |
| Idarubicin HCl | No | No | No | No | No | No | No | No | No | No |
| Hydroxyurea | No | No | No | No | No | No | No | No | No | No |
| Fludarabine | No | No | No | No | No | No | No | No | No | No |
| Etoposide | Yes | Yes | No | No | No | No | No | No | No | No |
| Dexamethason | No | No | No | No | No | Yes | No | No | No | No |
| Decitabine | No | No | No | No | No | No | No | No | No | No |
| Daunorubicin | Yes | No | No | No | No | No | No | No | No | No |
| Cytarabine | No | No | No | No | No | No | No | No | No | No |
| Clofarabine | Yes | Yes | No | No | No | No | No | No | No | No |
| Cladribine | Yes | No | No | No | No | No | No | No | No | No |
| Azacitidine | No | No | No | No | No | No | No | No | No | No |
| 6-Mercaptopurine | Yes | No | No | No | No | No | No | No | No | No |
| Compound Ii | Yes | Yes | Yes | Yes | Yes | Yes | No | No | No | No |

(continued)

| Compound | Cell Lines | | | | | | | | | |
|---|---|---|---|---|---|---|---|---|---|---|
| | MOLM-13 | MV-4-11 | OCI-AML-2 | F-36-P | OCI-AML-3 | NOMO-1 | ML-2 | KG-1 | HNT-34 | HL-60 |
| Omacetaxine | No | No | No | No | No | No | No | No | No | No |
| NPI-0052 | No | No | No | No | No | No | No | No | No | No |
| Ixazomib | No | No | No | No | No | No | No | No | No | No |
| CEP-18770 | No | No | No | No | No | No | No | No | No | No |
| Carfilzomib | No | No | No | No | No | No | No | No | No | No |
| Bortezomib | No | No | No | No | No | No | No | No | No | No |
| YO-01027 | No | No | No | No | No | No | No | No | No | No |
| Fedratinib | Yes | No | Yes | Yes | Yes | Yes | No | No | No | No |
| Metformin | No | No | No | No | No | No | No | No | No | No |
| Sunitinib | Yes | Yes | No | No | No | No | No | No | No | No |
| Sorafenib | No | No | No | No | No | No | No | No | No | No |
| Pexidartinib | Yes | Yes | No | No | No | No | No | No | No | No |
| Midostaurin | Yes | Yes | Yes | Yes | Yes | No | No | No | No | No |
| Lestaurtinib | Yes | Yes | No | No | No | No | No | No | No | No |
| Crenolanib | Yes | Yes | No | No | No | No | No | No | No | No |
| Venetoclax | No | No | No | No | No | No | No | Yes | No | No |
| Compound A | Yes | No | Yes | Yes | Yes | Yes | Yes | No | No | No |
| Compound B | Yes | No | No | Yes | No | No | No | No | No | No |

Table 76 provides a list of FLT3 and JAK inhibitors that were tested in combination with Compound 1 in MOLM-13 and NOMO-1 cell lines.

**Table 76**

| Compound | Synergy |
|---|---|
| Sorafenib | No |
| Sunitinib | Yes |
| Midostaurin | Yes |
| Pexidartinib | Yes |
| Lestaurtinib | Yes |
| Tandutinib | Yes |
| Quizartinib | Yes |
| Crenolanib | Yes |
| Filgotinib | Yes |
| Decernotinib | Yes |
| Baricitinib | Yes |
| Ruxolitinib | Yes |
| Fedratinib | Yes |

(continued)

| Compound | Synergy |
|---|---|
| NS-018 | Yes |
| pacritinib | Yes |
| Momelotinib | Yes |

As seen above, most of the tested combinations demonstrated synergy in AML cell lines MOLM-13 and NOMO-1. FIG. 57 illustrates synergy for combinations of Compounds 1 with midostaurin (FLT3 inhibitor) and ruxolitinib (JAK inhibitor) in FLT3-activated cell lines, such as cell lines carrying a FLT3 internal tandem duplication (ITD) mutation.

The combination treatments of Compound 1 and TOR inhibitors, everolimus, temsirolimus, 1-ethyl-7-(2-methyl-6-(1H-1,2,4-triazol-3-yl)pyridin-3-yl)-3,4-dihydropyrazino[2,3-b]pyrazin-2(1H)-one (CC-115) and 7-(6-(2-hydroxypropan-2-yl)pyridin-3-yl)-1-((trans)-4-methoxycyclohexyl)-3,4-dihydropyrazino[2,3-b]pyrazin-2(1H)-one (CC-223) showed synergy in BrCa (5/6), RCC (4/4) and NET (2/3) cell lines as illustrated in FIGs. 58 and 59.

**Example 23: *In vitro* studies in BON cells**

*In vitro* studies to assess the activity of Compound 1 on signaling and proliferation of BON cell lines, administered as a single agent and in combination with everolimus (referred as RAD in the FIGs. 60-74), were conducted using a CellTiter-Glo® assay.

The effect of Compound 1 alone and in combination with everolimus on signaling and proliferation was studied at 24 hours post treatment on 2D plates, 120 hours post-treatment on 2D plates, 120 hours post-treatment on 3D plates and 96 hours post-treatment on 2D plates.

FIG. 60 provides relative luminescence units (RLU) values for Compound 1 alone, and in combinations with everolimus at 2 nM, 20 nM and 200 nM.

FIG. 61 provides relative luminescence units (RLU) values for Compound 1 alone, and in combinations with everolimus at 2 nM, 20 nM and 200 nM.

FIG. 62 provides relative luminescence units (RLU) values for Compound 1 alone, and in combinations with everolimus at 2 nM, 20 nM and 200 nM.

FIG. 63 provides relative luminescence units (RLU) values for Compound 1 alone, and in combinations with everolimus at 2 nM, 20 nM and 200 nM.

FIGs. 64A and 64B provide plots for two runs depicting relative luminescence units (RLU) values for Compound 1 alone, and in combinations with everolimus at 2 nM, 20 nM and 200 nM.

The data demonstrate that Compound 1 has no standalone activity on BON1. Combination treatment of Compound 1 with everolimus results in synergy in growth inhibition, as seen by growth arrest to the pre-drug level, with no apoptosis.

**Example 24: Effect of Compound 1 and everolimus in a GA0087 PDX model *-Ex vivo* assay**

The combination effect of Compound 1 and everolimus on cell viability of GA0087 PDX model was investigated, using an ex-vivo 3D assay. The 50% inhibition concentration ($IC_{50}$) of the two compounds using an *ex vivo* 3D methylcellulose assay was determined, followed by determination of the synergy effect in matrix combination using the Combination Index.

Study design: Each cell line was seeded and treated with Compound 1 alone, matrix combination with Compound 1 and everolimus, and one reference control compound at required doses.

Materials and Methods: The stomach cancer cell line GA0087 was used in this study. The growth medium containing DMEM/F12 + 10% FBS + Pen/Strep + supplemental growth factors was used to culture the cells at the temperature of 37 °C, 5% $CO_2$ and 95% humidity. Culture media was purchased from GIBCO or Sigma, USA.

The following materials and reagents were used:

- CellTiter-Glo® Luminescent Cell Viability Assay (Cat. No.: G7572, Promega. Store at - 20 °C)
- 96-well polystyrene Microplates (Cat# 655096, Greiner bio-one)
- Lid for micro plates (Cat# 656171, Greiner bio-one)
- Methylcellulose (Cat# M0512, Sigma)
- Backseal black adhesive bottom seal (Cat# 6005189, Perkin Elmer)
- Collagennases (Cat#: 17100-017, Invitrogen)

- Falcon cell strainer (Cat# 352340, BD Falcon)

Compound 1 was reconstituted in DMSO to make a 5 mM solution, for 15μl aliquot per use. Everolimus was obtained from Selleck. Cisplatin was used as a reference drug, and was obtained from Hospira Australia pty Ltd.

**Methods**:

1% Methylcellulose preparation: 1 g methylcellulose was measured into a glass container with lid. The container was autoclaved at standard sterilization conditions. The container was cooled, and 100 mL appropriate cell culture media was added. Any methylcellulose remaining was removed from the bottom with a sterile cell scraper and the contents were mixed vigorously. The container was stored on a rocker platform at 4 °C to complete the dissolution for up to 48 hours, to achieve complete dissolution. The methylcellulose solution was stored at 4 °C.

**1. Single cell isolation**

1. PDX tumor models were maintained at Crownbio HuPrime animal facility. Tumor growth was monitored weekly. Tumor volumes were measured in two dimensions using a caliper, and the volume was expressed in $mm^3$ using the formula: $V = 0.5\ a \times b^2$ where a and b are the long and short diameters of the tumor, respectively.
2. Mouse xenografts at tumor volume around 800 $mm^3$ were housed with sterile surgical tools and tumor tissue was minced into tiny pieces with scissors in small amount of PBS on a new tissue culture plate.
3. The cell suspension was filtered through Falcon cell strainer. The nylon mesh was washed 3-5 times with PBS.
4. The cell suspension was centrifuged at 1000 rpm for 5 minutes and the pellet was washed with PBS and centrifuged again.
5. Red blood cells were removed using red blood cell lysis buffer.
6. The cell suspension was centrifuged at 1000 rpm for 5 minutes and the pellet was washed with PBS and centrifuged again.
7. The cell pallet was re-suspended with appropriate cell culture media for different cells.
8. Cells were counted with Countstar by trypan blue exclusion.

**2. CellTiter-Glo® cell viability assay (3D methylcellulose format)**

**Day -1: Cell plating**

1. The cells were harvested during the logarithmic growth period. The harvested cells were cultured with appropriate cell media and centrifuged at 1000 rpm for 3 minutes. The cells were re-suspended and counted using CountStar (The cell viability should meet the standard of > 90 % by trypan blue exclusion assay.).
2. The cell concentrations were adjusted to $2\times10^5$ cells/ml with respective medium. (Cell concentration was adjusted according to the data base or density optimization assay).
3. 3.5 mL of cell suspension was mixed with 6.5 mL of 1% methylcellulose. This step yielded 10 ml of cell suspension in 0.65% methylcellulose solution.
4. 90 μL cell suspensions were added to 96-well plates according to plate map with final cell density. Two duplicate plates were set up: one for day 0 reading (T0) and the other was cultured in incubator for reading at the end point.

The plates were incubated for overnight in humidified incubator at 37 °C with 5% $CO_2$.

**Day 0: T0 plate reading and compound treatment**

5.10 μL culture medium was added to TO plate in each well for TO reading.
6.100 μl CellTiter-Glo® Reagent was added to each well.
7. The contents were mixed for 2 minutes on an orbital shaker to facilitate cell lysis.
8. The plates were allowed to incubate at room temperature for 10 minutes to stabilize luminescent signal.
9. A backseal black sticker was placed to the bottom of each plate.

10.The luminescence was read using EnVision Multi Label Reader.
11. Compound 1, everolimus and reference drug solutions were diluted at the concentration indicated below.

Compound 1 and everolimus were dissolved in DMSO to make 10 mM stock solutions and aliquots. The solutions were stored at -20 °C. Nine concentrations of Compound 1 (in 3x dilution) were combined with 6 concentrations of everolimus. The response score was calculated by normalizing to DMSO control. The combination index (CI) was calculated using Chou and Talalay method, wherein a CI of less than 1 indicates synergy. Cisplatin was used as control in the single compound dose response, but was not included in the combination test.

**Day 7: Plate reading of 7 days' compound treatment.**

12. After drug incubation, pictures for representative wells were taken using a phase contrast microscope.
13. 100 μL CellTiter-Glo® Reagent was added to each well.
14. Contents were mixed for 2 minutes on an orbital shaker to facilitate cell lysis.
15. The plates were allowed to incubate at room temperature for 10 minutes to stabilize luminescent signal.
16. Backseal black sticker was placed to the bottom of each plate.
17. Luminescence was recorded using EnVision Multi Label Reader.

**Data analysis**

The data was displayed graphically using GraphPad Prism 5.0. In order to calculate ICso, a dose-response curve was fitted using nonlinear regression model with a sigmoidal dose response. The formula of surviving rate is shown below, and the ICso was automatically produced by GraphPad Prism 5.0.

$$\text{The surviving rate (\%)} = (\text{Lum}_{\text{Test article}} - \text{Lum}_{\text{Medium control}}) / (\text{Lum}_{\text{None treated}} - \text{Lum}_{\text{Medium control}}) \times 100\%.$$

**Synergism Determination**

Compound interactions were calculated by multiple drug effect analysis and were performed by the median equation principle according to the methodology described by Chou and Talalay. Fa is the fraction affected by the dose. The combination index (CI) was calculated by the Chou *et al.* equation which takes into account both the potency ($D_m$ or $IC_{50}$) and the shape of the dose-effect curve (the m value).

The general equation for the CI of the two compounds is given by:

$$CI = \frac{(D)_1}{(D_x)_1} + \frac{(D)_2}{(D_x)_2} + \frac{(D)_1(D)_2}{(D_x)_1(D_x)_2}$$

Where: $(D_x)_1$ and $(D_x)_2$ in the denominators are the doses (or concentrations) for Compound 1 and Compound 2 alone which demonstrate x% of inhibition. Whereas $(D)_1$ and $(D)_2$ in the numerators are doses of both compounds (1 and 2) in combination that also inhibit x% (iso-effective). CI<1, =1, and >1 indicate synergism, additive effect and antagonism, respectively.

The $(D_x)_1$ and $(D_x)_2$ can be calculated from the median-effect equation of Chou *et al.* :

$$D_x = D_m\left(\frac{f_a}{(1-f_a)}\right)^{1/m}$$

Where: $D_m$ is the median-effect dose that is obtained from the anti-log of x-intercept of the median-effect plot, x = log(D) *versus* y = $\log\{f_a/(1-f_a)\}$, or $D_m = 10^{-(\text{-intercept})/m}$; and m is the slope of the median-effect plot and $f_a$ is the fraction of cells affected by the treatment.

Each CI was calculated with CalcuSyn software from the mean affected fraction at each drug ratio concentration.

For fixed ratio combination of 2 compounds at 7 concentrations, 7 CI values were obtained.

**Table 77**

| *Range of CI* | *Description* |
|---|---|
| *0.1* | *Very strong synergism* |
| *0.1-0.3* | *Strong synergism* |
| *0.3-0.7* | *Synergism* |
| *0.7-0.85* | *Moderate synergism* |
| *0.85-0.90* | *Slight synergism* |
| *0.90-1.10* | *Nearly additive* |
| *1.10-1.20* | *Slight antagonism* |
| *1.20-1.45* | *Moderate antagonism* |
| *1.45-3.3* | *Antagonism* |
| *3.3-10* | *Strong antagonism* |
| *>10* | *Very strong antagonism* |

The DRI is a measure of how many-fold the dose of each drug in a synergistic combination may be reduced at a given effect level compared with the doses of each drug alone. For two-drug combinations

$$CI = \frac{(D)_1}{(D_x)_1} + \frac{(D)_2}{(D_x)_2} = \frac{1}{(DRI)_1} + \frac{1}{(DRI)_2}$$

and for n-drug combinations

$$CI = \sum_{j=1}^{n} \frac{(D)_j}{(D_x)_j} = \sum_{j=1}^{n} \frac{1}{(DRI)_j}$$

Therfore,

$$(DRI)_1 = \frac{(D_x)_1}{(D)_1}, (DRI)_2 = \frac{(D_x)_2}{(D)_2}, \ldots, etc$$

0r

$$(DRI)_1 = \frac{(D_m)_1[f_a/(1-f_a)]^{1/m_1}}{(D)_1},$$

$(DRI)_2$ Each DRI was calculated with CalcuSyn software from the mean affected fraction at each drug *ratio* concentration of each drugs. For fixed ratio combination of 2 drugs at 7 concentrations, 7 × 2 = 14 DRI values were obtained.

**Results**: Table 78 below provides a summary of $IC_{50}$ and maximal inhibition for the GA0087 model.

**Table 78**

| Model | Compound 1 | | | Everolimus | | |
|---|---|---|---|---|---|---|
| | Relative $IC_{50}$ ($\mu$M) | Absolute $IC_{50}$ ($\mu$M) | Maximal Inhibition (%) | Relative $IC_{50}$ ($\mu$M) | Absolute $IC_{50}$ ($\mu$M) | Maximal Inhibition (%) |
| **GA0087-1st** | 0.0163 | 0.0203 | 90.71% | NA | 0.0061 | 73.15% |
| **GA0087-2nd** | 0.0152 | 0.0164 | 92.41% | NA | 0.0075 | 72.80% |

**Table 79**

| Model | Cisplatin | | |
|---|---|---|---|
| | Relative $IC_{50}$ ($\mu$M) | Absolute $IC_{50}$ ($\mu$M) | Maximal Inhibition (%) |
| **GA0087-1st** | 3.1730 | 3.3498 | 98.73% |
| **GA0087-2nd** | 3.2990 | 3.5251 | 98.48% |

FIGs. 65A and 65B provide dose-response curves (3D *ex vivo* clonogenic assay) depicting the maximal inhibition of Compound 1 and everolimus in the GA0087 model for 2 experiments. FIG. 66 provides $IC_{50}$ and maximal inhibition of the reference compound in the GA0087 model, in duplicate.

FIGs. 67A and 67B provide inhibition effect of the matrix combination assay for the two experiments, and FIGs. 68A and 68B provide the combination index for the two experiments.

As seen from the data, Compound 1 shows activity in the GA0087 model, and shows synergy with everolimus at the majority of the concentrations tested. In particular, Compound 1 shows synergy with everolimus at multiple concentrations of intermediate doses of Compound 1 (0.5 -111.1 nM).

**Example 25: Effect of Compound 1 and everolimus in GA0087 PDX model- *In vivo* assay**

The combination treatment of Compound 1 and everolimus was tested in a HuPrime® gastric cancer xenograft GA-0087 (a neuroendocrine tumor) model in female BALB/c nude mice. Tumor fragments from stock mice were harvested and used for inoculation into mice. Each mouse was inoculated subcutaneously at the right flank with primary human tumor xenograft model GA0087 tumor fragment (P7,2-3 mm in diameter) for tumor development. For the efficacy study, when the mean tumor volume reached approximately 196 $mm^3$, mice were randomly allocated into 9 groups (10 mice in each group, 1 vehicle control group, 2 everolimus and 3 Compound 1 single agent treatment groups, and 3 combination treatment groups) based on tumor volume and body weight with dosing starting on the same day (Study Day 0). Dosing and schedules are shown in FIG. 69. After tumor inoculation, the animals were checked daily for morbidity and mortality. At the time of routine monitoring, the animals were checked for any effects of tumor growth and treatments on normal behavior such as mobility, food and water consumption, body weight gain/loss, eye/hair matting and any other abnormal effect. Death of euthanized mouse was recorded. All groups were terminated on Day 40. Three tumors in each group, with tumor volume near median of each group were collected. Plasma of the same mice were collected. For comparison among three or more groups, one-way ANOVA was performed followed by multiple comparison procedures. All data were analyzed using SPSS 18.0. P values < 0.05 is considered to be statistically significant.

FIG. 69 provides mean tumor volume for Compound 1 and everolimus, alone and in combination. As seen from the data, Compound 1 showed tumor growth inhibition. A combination of Compound 1 with 1.25 mg/kg everolimus significantly increased tumor growth inhibition compared to either agent alone. A transient tumor regression was achieved with 5 mg/kg Compound 1 and everolimus 1.25 mg/kg combination.

In summary, single-agent treatments with everolimus(1.25 mg/kg and 5 mg/kg) and Compound 1 (1.25 mg/kg and 2.5 mg/kg) produced moderate anti-tumor activity, while single-agent treatment with Compound 1 (5 mg/kg) and with the combination treatments (everolimus, 1.25 mg/kg and Compound 1, 1.25 mg/kg, 2.5 mg/kg, and 5 mg/kg) produced prominent anti-tumor activity against the HuPrime® gastric cancer xenograft model GA0087.

**Example 26: Effect of Compound 1 on myelofibrosis progenitors**

The effect of Compound 1 was studied in a colony forming assay using myelofibrosis patient samples. Peripheral blood mononuclear cells (PBMCs) from myelofibrosis patients were seeded in methocult media with and without Compound 1 for 14 days, after which the number of colony forming cells were determined.

The following samples were used:

MF13: De novo MF, characterized by JAK2wt, FLT3wt, NPM1 wt, CEBPA wt
MF14: De novo MF, characterized by JAK2wt, CALR exon9 mut, ASXL1 wt, MPLwt

The data provided in FIG. 70 demonstrate that the number of colony forming cells was reduced by Compound 1 in a dose dependent manner in samples from myelofibrosis patients. $IC_{50S}$ achieved in these samples were lower than $IC_{50S}$ observed in samples from healthy volunteers suggesting the potential of Compound 1 as a treatment for myelofibrosis.

**Example 27: Combination studies in myeloproliferative neoplasms**

*In vitro* combination studies to assess the activity of Compound 1 in combination with the following JAK2 inhibitors were conducted in BaF3 cells engineered to stably express exogenous proteins. The following cell lines were used: hCRBN is a human CRBN expressing BaF3 cell line; EF1a-GFP-P2A-Nluc-P2A-JAK2 is a hCRBN, and JAK2 wt expressing BaF3 cell line; EF1a-GFP-P2A-Nluc-P2A-JAK2-V617F newly transduced IL3 dependent, is a hCRBN and mutant JAK2V617F that is still dependent on IL3 expressing BaF3 cell line; and EF1a-GFP-P2A-Nluc-P2A-JAK2-V617F; IL3 independent clone is a hCRBN and mutant JAK2V617F that has been adapted to become independent of IL3 BaF3 cell line. The JAK2 inhibitors used in the assay were NS-018, INCB018424 (Ruxolitinib; Jakafi), CYT387 (Momelotinib), TG101348 (Fedratinib), and pacritinib.

The cells were treated with compounds by adding a serial dilution of Compound 1 (starting at 50 nM) in combination with a serial dilution of JAK2 inhibitors (starting concentration 10 $\mu$M for all JAK2 inhibitors). Everolimus was included as a control. The cell viability was then measured by CellTitre-Glo® after 3 days.

As demonstrated by data in FIGs. 71-77, no difference in Compound 1 or JAK2 inhibitor when used as single agents in IL3 dependent BaF3 lines was observed. The IL3 independent JAK2 V617F cells were more sensitive to most JAK2 inhibitors, Compound 1 and everolimus.

As demonstrated in FIG. 78, a combination of Compound 1 and NS-018 showed an $EC_{50}$ shift in parental, JAK2 wt and JAK2V617F cells. No apparent difference in synergy profiles between hCRBN, wt and JAK2 V617F newly transduced lines was observed. The IL3 independent JAK2 V617F cells were more sensitive to NS-018 single agent compared to all three IL3 dependent lines. Synergy was observed at clinical $C_{max}$ for NS-018 (2.57 $\mu$M, as described in a phase I, open-label, dose-escalation, multicenter study of the JAK2 inhibitor NS-018 in patients with myelofibrosis, Leukemia (2017) 31, 393-402).

As shown in FIG. 79, at less than 100 nM of NS-018, no apparent $EC_{50}$ shift was observed for all 4 cell lines.

As demonstrated in FIG. 80, a combination of Compound 1 and ruxolitinib showed a strong $EC_{50}$ shift in parental, JAK2 wt and JAK2V617F cells. No apparent difference in synergy profiles between hCRBN, wt and JAK2 V617F newly transduced lines was observed. The IL3 independent JAK2 V617F cells were more sensitive to ruxolitinib single agent compared to all three IL3 dependent lines. Synergy was observed at clinical $C_{max}$ for ruxolitinib (~0.5-1 $\mu$M, as described in Blood (2011) 118:5162).

As shown in FIG. 81, at less than 100 nM of ruxolitinib, no apparent $EC_{50}$ shift was observed for all 4 cell lines.

As demonstrated in FIG. 82, a combination of Compound 1 and momelotinib showed a strong $EC_{50}$ shift in parental, JAK2 wt and JAK2V617F cells. No apparent difference in synergy profiles between hCRBN, wt and JAK2 V617F newly transduced lines was observed. The IL3 independent JAK2 V617F cells were more sensitive to momelotinib single agent compared to all three IL3 dependent lines.

As demonstrated in FIG. 83, a combination of Compound 1 and pacritinib showed strong $EC_{50}$ shift in parental, JAK2 wt and JAK2V617F cells. No apparent difference in synergy profiles between hCRBN, wt and JAK2 V617F newly transduced lines was observed.

As demonstrated in FIG. 84, a combination of Compound 1 and fedratinib showed a strong $EC_{50}$ shift in parental, JAK2 wt and JAK2V617F cells. No apparent difference in synergy profiles between hCRBN, wt and JAK2 V617F newly transduced lines was observed.

As demonstrated in FIG. 85, a combination of Compound 1 and everolimus showed a slight $EC_{50}$ shift in parental, JAK2 wt and JAK2V617F cells. A stronger $EC_{50}$ shift in IL3 independent JAK2 V617F cells was observed. The extent of $EC_{50}$ shift with everolimus was weaker than combinations with JAK2 inhibitors in all BaF3 lines, suggesting mTOR activity is not the major downstream mechanism responsible for synergy between JAK2 inhibitors and Compound 1.

Conclusion: These data demonstrate synergy between Compound 1 and JAK2 inhibitors in BaF3 cells engineered to express hCRBN with either WT JAK2 or JAK2V617F. Since combination with the mTOR inhibitor everolimus showed reduced synergy compared to combination with the JAK2 inhibitors, these results suggest JAK2 mediated synergy with Compound 1 is likely through other mechanisms besides or in addition to mTOR activity.

**Example 28: Combination studies with Compound 1 and JAK2 inhibitors in AML cell lines**

*In vitro* combination studies to assess the activity of Compound 1 in combination with the following JAK2 inhibitors NS-018, INCB018424 (Ruxolitinib; Jakafi), CYT387 (Momelotinib), TG101348 (Fedratinib), and pacritinib were conducted in 12 AML cell lines.
The following AML cell lines were used:

JAK2V617F: HEL, SET-2, MUTZ-8
JAK Wild Type (WT): HL-60, HNT-34, KG-1, ML-2, NOMO-1, MOLM-13, MV4-11, F36P, OCI-AML2

The cell viability was measured by CellTitre-Glo® after 3 days as described elsewhere herein.
As demonstrated by the data in FIG. 86, NS-018 and ruxolitinib inhibit cell viability of JAK2V617F AML cell lines as single agents.
As demonstrated in FIG. 87, a combination of Compound 1 and NS-018 showed synergy in the inhibition of the viability of JAK2 V617F in HEL, SET-2 and MUTZ-8 cell lines.
As demonstrated in FIG. 88, a combination of Compound 1 and ruxolitinib showed synergy in the inhibition of the viability of JAK2 V617F in HEL, SET-2 and MUTZ-8 cell lines. Synergy was more apparent on cell lines (HEL) that were less sensitive to Compound 1 single agent.
As demonstrated in FIG. 89, a combination of Compound 1 and everolimus showed synergy in the inhibition of the viability of HEL cells, but no apparent ICso shift was observed on SET-2 or MUTZ-8 cells.
As demonstrated in FIG. 90, all five JAK2 inhibitors showed synergy with Compound 1 in the inhibition of the viability of JAK2 V617F cells.
Conclusion: Strong synergy with Compound 1 was demonstrated with all 5 clinical stage JAK2 inhibitors.

**Example 29: Combination studies with Compound 1 and IDH2 inhibitors**

*In vitro* combination studies to assess the activity of Compound 1 in combination with the IDH2 inhibitor Enasidenib (AG-221) were conducted in the IDH2 mutant cell line TF1-R140Q.
Cells were plated at a density of $0.2e^6$ cells/ml of 2 ml culture in 6 well plates. Enasidenib was tested at 0, 200 nM, and 1000 nM concentration, while Compound 1 was tested at a concentration of 0, 10 nM, 30 nM, 100 nM.
FIG. 91 provides the dosing schedules used in this study.
TF1 cells were cultured in the following media: RPMI containing HEPES and L-glutamine, 10% FBS, Pen/Strep, G418 (final conc 500 μg/ml), and GM-CSF (final conc 5 ng/ml). The cells were cultered for 7 days, with or without compound treatment as described in FIG. 91.
For the hemoglobinization assay, TF1 cells were washed three times with PBS to remove residual GM-CSF then plated at (100,000 cells/ml). Cells were then induced to differentiate using EPO (2 unit/ml). Induction continued for 7 days with changing media on 4$^{th}$ day and adding media with fresh EPO. The cell pellets were collected and imaged for hemoglobinization content (as a surrogate for differentiation into blood lineage). Cells were pelleted, washed, stained with a panel of antibodies and analyzed by flow cytometry, using the following antibodies:

| Antibodv | Clone | Fluorophore |
|---|---|---|
| CD34 | 8G12 | PE-Cy7 |
| CD38 | HIT2 | BV421 |
| CD235a | GA-R2 (HIR2) | BV711 |
| Vimentin | RV202 | FITC |
| cCaspase 3 | C92-605 | PE |
| GSPT1 | | AF647 (APC) |

In the hemoglobinization assay, Enasidenib and Compound 2 yielded expected effect of increase in hemoglobin expression, as seen in FIG. 92. Additionally, combination treatment with Compound 1 and enasidenib showed augmented hemoglobinization and cell differentiation, as shown by reduced HSC (CD34+/CD38-) and progenitor cells CD34+/CD38+, and increased CD34-/CD38- and CD34-/CD235a+ erythyoblast cells shown in FIGs. 93-97.
The results from EPO induced differentiation assay provided in FIGs. 93-94 demonstrate the enhanced effect of Compound 1 and enasidenib in reducing TF-1:IDH2R140Q progenitor cells (CD34+/CD38+) and hematopoietic stem

cells (HSC) (CD34+/CD38-), and increasing differentiated CD34-/CD38- cells and erythroblasts. FIG. 95 shows the increase in CD235a+ (Glycophorins A), a marker for erythrocytes population. The enhancement was predominant in schedules A and C.

FIGs. 96 and 97 show the preferential GSPT1 degradation in CD34+ cells over differentiated cells in the TF1 assay. Schedule A (Compound 1 added 24 hours prior to enasidenib) resulted in the lowest absolute count of CD34+ due to increased differentiation (also seen in schedule C) and the lasting effect of preferential killing of CD34+ cells.

FIG. 98 demonstrates the inhibition of proliferation of total cell count and HSC count (CD34+/CD38-) and progenitor count (CD34+/CD38+) by Compound 1. As seen, schedule A has a strong and long lasting inhibition on cell proliferation, and schedule A results in lowest cell number of un-differentiated CD34+ cells. FIG. 98 shows that enasidenib has a mild growth promoting effect, but combination with Compound 1 resulted in a clear reduction of cell count for total (left) and stem and progenitor groups (right two panels).

Conclusion: The data demonstrate that the combination of enasidenib and Compound 1 resulted in a lower levels of GSPT1 in stem and progenitor cells compared to the more differentiated subset of CD34-/CD38- and erythroblasts. Additionally, it is shown that enasidenib has a mild growth promoting effect, but combination with Compound 1 resulted in a reduction of cell count for total and stem and progenitor groups. The net loss of cell counts of stem and progenitors may be due to the death of these cells, or to differentiation of CD34+ cells into CD34- cells.

**Example 30: Effect of Compound 1 in solid tumor cell lines**

Compound 1 was tested for its activity in 563 solid tumor cell lines by a Luminex assay. The AUC values provided in Table 80 below were calculated based on the Median Fluorescent Intensity values, the primary read out of the Luminex assay in PRISM. The following parameters were used in this assay:

- 8 point dose was used in triplicate
- max concentration of 10 μM was used with serial 4-fold dilutions (low of - InM)
- 563 adherent cell lines in 23 pools.
- treatment duration was 5 days.

**Table 80:**

| Cell line | AUC |
|---|---|
| A101D_SKIN | 0.821 |
| A172_CENTRAL_NERVOUS_SYSTEM | 0.314 |
| A204_SOFT_TISSUE | 0.156 |
| A2058_SKIN | 0.818 |
| A253_SALIVARY_GLAND | 0.864 |
| A2780_OVARY | 0.485 |
| A375_SKIN | 0.795 |
| A498_KIDNEY | 0.845 |
| A549_LUNG | 0.857 |
| A673_BONE | 0.885 |
| A673STAG2NT23_ENGINEERED | NA |
| A704_KIDNEY | 0.823 |
| ABC1_LUNG | 0.591 |
| ACCMESO1_PLEURA | 0.631 |
| ACHN_KIDNEY | 0.912 |
| AGS_STOMACH | 0.865 |
| AM38_CENTRAL_NERVOUS_SYSTEM | 0.543 |
| AN3CA_ENDOMETRIUM | 0.861 |
| ASPC1_PANCREAS | NA |
| BC3C_URINARY_TRACT | NA |
| BCPAP_THYROID | 0.782 |
| BECKER_CENTRAL_NERVOUS_SYSTEM | 0.874 |
| BEN_LUNG | 0.826 |
| BFTC909_KIDNEY | 0.843 |
| BHT101_THYROID | 0.858 |

| Cell line | AUC |
|---|---|
| BHY_UPPER_AERODIGESTIVE_TRACT | 0.405 |
| BICR16_UPPER_AERODIGESTIVE_TRACT | 0.585 |
| BICR18_UPPER_AERODIGESTIVE_TRACT | 0.845 |
| BICR22_UPPER_AERODIGESTIVE_TRACT | NA |
| BICR31_UPPER_AERODIGESTIVE_TRACT | NA |
| BICR56_UPPER_AERODIGESTIVE_TRACT | 0.984 |
| BICR6_UPPER_AERODIGESTIVE_TRACT | 0.801 |
| BT474_BREAST | 0.801 |
| BT549_BREAST | 0.242 |
| BXPC3_PANCREAS | 0.916 |
| C32_SKIN | 0.881 |
| CADOES1_BONE | 0.566 |
| CAKI1_KIDNEY | NA |
| CAKI2_KIDNEY | 0.867 |
| CAL120_BREAST | 0.812 |
| CAL12T_LUNG | 0.366 |
| CAL27_UPPER_AERODIGESTIVE_TRACT | 0.812 |
| CAL29_URINARY_TRACT | 0.831 |
| CAL51_BREAST | 0.589 |
| CAL54_KIDNEY | 0.679 |
| CAL62_THYROID | 0.083 |

| Cell line | AUC |
|---|---|
| CAL78_BONE | 0.798 |
| CALU6_LUNG | 0.794 |
| CAMA1_BREAST | 0.589 |
| CAOV3_OVARY | NA |
| CAPAN2_PANCREAS | 0.835 |
| CAS1_CENTRAL_NERVOUS_SYSTEM | NA |
| CBAGPN_BONE | 0.236 |
| CCFSTTG1_CENTRAL_NERVOUS_SYSTEM | NA |
| CCK81_LARGE_INTESTINE | 0.021 |
| CFPAC1_PANCREAS | 0.872 |
| CHL1_SKIN | 0.330 |
| CHLA10_BONE | 0.933 |
| CJM_SKIN | 0.215 |
| CL11_LARGE_INTESTINE | 0.849 |
| CL34_LARGE_INTESTINE | 0.604 |
| COLO678_LARGE_INTESTINE | 0.811 |
| COLO679_SKIN | 0.830 |
| COLO680N_OESOPHAGUS | 0.863 |
| COLO741_SKIN | 0.411 |
| COLO783_SKIN | NA |
| COLO792_SKIN | 0.629 |
| COLO800_SKIN | 0.810 |
| COLO829_SKIN | 0.748 |
| CORL105_LUNG | 0.824 |
| CORL23_LUNG | 0.879 |
| COV318_OVARY | 0.883 |

| Cell line | AUC |
|---|---|
| COV362_OVARY | 0.824 |
| COV644_OVARY | 0.994 |
| CW2_LARGE_INTESTINE | NA |
| DANG_PANCREAS | 0.793 |
| DAOY_CENTRAL_NERVOUS_SYSTEM | 0.928 |
| DBTRG05MG_CENTRAL_NERVOUS_SYSTEM | 0.795 |
| DETROIT562_UPPER_AERODIGESTIVE_TRACT | 0.855 |
| DKMG_CENTRAL_NERVOUS_SYSTEM | NA |
| DMS273_LUNG | 0.771 |
| DMS53_LUNG | 0.903 |
| DU145_PROSTATE | 0.878 |
| DV90_LUNG | 0.174 |
| EBC1_LUNG | 0.960 |
| ECGI10_OESOPHAGUS | 0.877 |
| EFE184_ENDOMETRIUM | 0.679 |
| EFM19_BREAST | 0.917 |
| EFM192A_BREAST | 0.766 |
| EFO21_OVARY | 0.843 |
| EFO27_OVARY | 0.534 |
| EKVX_LUNG | 0.837 |
| EN_ENDOMETRIUM | 0.831 |
| ES2_OVARY | 0.801 |
| ESS1_ENDOMETRIUM | 0.512 |
| EW8_BONE | 0.985 |
| EWS502_BONE | 0.875 |

| Cell line | AUC |
|---|---|
| FADU_UPPER_AERODIGESTIVE_TRACT | 0.859 |
| FTC133_THYROID | 0.833 |
| FTC238_THYROID | 0.842363862 |
| G292CLONEA141B1_BONE | 0.274597509 |
| G402_SOFT_TISSUE | 0.829684634 |
| GAMG_CENTRAL_NERVOUS_SYSTEM | 0.365029338 |
| GB1_CENTRAL_NERVOUS_SYSTEM | 0.835864779 |
| GCIY_STOMACH | 0.866232166 |
| GI1_CENTRAL_NERVOUS_SYSTEM | 0.587405141 |
| GMS10_CENTRAL_NERVOUS_SYSTEM | 0.404794442 |
| GOS3_CENTRAL_NERVOUS_SYSTEM | 0.504650731 |
| GP2D_LARGE_INTESTINE | 0.796 |
| HARA_LUNG | 0.796371228 |
| HCC1143_BREAST | 0.813308373 |
| HCC1195_LUNG | 0.90039631 |

| Cell line | AUC |
|---|---|
| HCC1359_LUNG | 0.875025083 |
| HCC1395_BREAST | 0.950788588 |
| HCC1419_BREAST | 0.848671829 |
| HCC1428_BREAST | 0.916722078 |
| HCC1438_LUNG | 0.970081471 |
| HCC15_LUNG | 0.95828541 |
| HCC1806_BREAST | 0.877471248 |
| HCC1937_BREAST | 0.579049524 |
| HCC1954_BREAST | 0.810654202 |
| HCC366_LUNG | 0.318310589 |
| HCC38_BREAST | 0.487250949 |
| HCC4006_LUNG | 0.840292379 |
| HCC44_LUNG | 0.906 |
| HCC56_LARGE_INTESTINE | 0.849 |
| HCC78_LUNG | 0.793 |
| HCC827_LUNG | 0.516 |
| HCT116_LARGE_INTESTINE | 0.826 |

| Cell line | AUC |
|---|---|
| HCT15_LARGE_INTESTINE | 0.554 |
| HDQP1_BREAST | 0.825 |
| HEC108_ENDOMETRIUM | 0.812 |
| HEC151_ENDOMETRIUM | 0.903 |
| HEC1A_ENDOMETRIUM | 0.832 |
| HEC1B_ENDOMETRIUM | NA |
| HEC251_ENDOMETRIUM | 0.698 |
| HEC265_ENDOMETRIUM | 0.831 |
| HEC59_ENDOMETRIUM | 0.794 |
| HEC6_ENDOMETRIUM | 0.858 |
| HEP3B217_LIVER | 0.596 |
| HEPG2_LIVER | 0.511 |
| HGC27_STOMACH | 0.846 |
| HLF_LIVER | 0.952 |
| HMC18_BREAST | 0.847 |
| HOS_BONE | 0.854 |
| HS294T_SKIN | 0.883 |
| HS729_SOFT_TISSUE | 0.598 |
| HS746T_STOMACH | 0.428 |
| HS766T_PANCREAS | 0.830 |
| HS852T_SKIN | 0.710 |
| HS939T_SKIN | 0.836 |
| HS944T_SKIN | 0.719 |
| HSC2_UPPER_AERODIGESTIVE_TRACT | 0.597 |
| HSC3_UPPER_AERODIGESTIVE_TRACT | 0.834 |
| HT115_LARGE_INTESTINE | NA |
| HT1376_URINARY_TRACT | 0.900 |

| Cell line | AUC |
|---|---|
| HT144_SKIN | 0.641 |
| HT29_LARGE_INTESTINE | 0.546 |
| HT55_LARGE_INTESTINE | 0.571 |
| HUCCT1_BILIARY_TRACT | 0.533 |
| HUH1_LIVER | 0.508 |
| HUH28_BILIARY_TRACT | 0.267 |
| HUH6_LIVER | 0.867 |
| HUPT3_PANCREAS | 0.876 |
| HUPT4_PANCREAS | 0.594820709 |
| IALM_LUNG | 0.24806342 |
| IGR1_SKIN | NA |
| IGR37_SKIN | 0.849536662 |
| IGROV1_OVARY | 0.697842767 |
| IM95_STOMACH | 0.110972619 |
| IPC298_SKIN | 0.894032519 |
| ISHIKAWAHERAKLIO02ER_ENDOMETRIUM | 0.840171102 |
| ISTMES1_PLEURA | 0.138148777 |
| ISTMES2_PLEURA | 0.819231515 |
| J82_URINARY_TRACT | 0.826340546 |

| Cell line | AUC |
|---|---|
| JHH1_LIVER | 0.469 |
| JHH4_LIVER | 0.680 |
| JHH5_LIVER | 0.806 |
| JHH6_LIVER | 0.449 |
| JHH7_LIVER | 0.778 |
| JHOC5_OVARY | 0.865 |
| JHOM1_OVARY | 0.833 |
| JHOS2_OVARY | 0.566 |
| JHUEM2_ENDOMETRIUM | 0.761 |
| JIMT1_BREAST | 0.816 |
| JL1_PLEURA | 0.591 |
| JMSU1_URINARY_TRACT | 0.842 |
| K029AX_SKIN | 0.834 |
| KALS1_CENTRAL_NERVOUS_SYSTEM | 0.838 |
| KE39_STOMACH | 0.521 |
| KELLY_AUTONOMIC_GANGLIA | 0.591 |
| KMBC2_URINARY_TRACT | 0.913 |
| KMRC1_KIDNEY | 0.842 |
| KMRC20_KIDNEY | 0.951 |
| KMRC3_KIDNEY | 0.990 |
| KNS42_CENTRAL_NERVOUS_SYSTEM | 0.826 |
| KNS60_CENTRAL_NERVOUS_SYSTEM | 0.795 |
| KNS62_LUNG | 0.842 |
| KNS81_CENTRAL_NERVOUS_SYSTEM | 0.808 |

| Cell line | AUC |
|---|---|
| KP2_PANCREAS | 0.767 |
| KP3_PANCREAS | 0.592 |
| KP4_PANCREAS | 0.447 |
| KPL1_BREAST | 0.890 |
| KPNYN_AUTONOMIC_GANGLIA | 0.648 |
| KU1919_URINARY_TRACT | 0.926 |
| KURAMOCHI_OVARY | 0.857 |
| KYSE140_OESOPHAGUS | 0.883 |
| KYSE150_OESOPHAGUS | 0.863 |
| KYSE180_OESOPHAGUS | 0.865 |
| KYSE270_OESOPHAGUS | 0.835 |
| KYSE510_OESOPHAGUS | 0.870 |
| KYSE520_OESOPHAGUS | 0.887 |
| KYSE70_OESOPHAGUS | 0.788 |
| LC1SQSF_LUNG | 0.936 |
| LCLC103H_LUNG | NA |
| LI7_LIVER | 0.811 |
| LK2_LUNG | 0.768 |
| LN229_CENTRAL_NERVOUS_SYSTEM | 0.758 |
| LNCAPCLONEFGC_PROSTATE | 0.672 |
| LOVO_LARGE_INTESTINE | NA |
| LOXIMVI_SKIN | 0.905 |
| LS1034_LARGE_INTESTINE | 0.223 |
| LS180_LARGE_INTESTINE | 0.142 |
| LS411N_LARGE_INTESTINE | 0.811 |
| LS513_LARGE_INTESTINE | 0.235 |

| Cell line | AUC |
|---|---|
| LU99_LUNG | 0.421 |
| LUDLU1_LUNG | NA |
| LXF289_LUNG | 0.858 |
| MALME3M_SKIN | 0.180 |
| MCAS_OVARY | 0.845 |
| MCF7_BREAST | 0.831 |
| MDAMB175VII_BREAST | 0.524 |
| MDAMB231_BREAST | 0.278 |
| MDAMB361_BREAST | NA |
| MDAMB435S_SKIN | 0.845 |
| MDAMB436_BREAST | NA |
| MELHO_SKIN | 0.907 |
| MELJUSO_SKIN | 0.845 |
| MESSA_SOFT_TISSUE | NA |
| MEWO_SKIN | 0.267 |
| MFE280_ENDOMETRIUM | 0.881 |
| MFE296_ENDOMETRIUM | 0.824 |
| MFE319_ENDOMETRIUM | 0.751 |
| MG63_BONE | 0.968 |
| MHHES1_BONE | 0.984 |
| MIAPACA2_PANCREAS | 0.866 |
| MKN1_STOMACH | 0.871 |
| MKN45_STOMACH | 0.338 |
| MKN7_STOMACH | 0.841 |
| MKN74_STOMACH | 0.883 |
| MON_SOFT_TISSUE | 0.789 |
| MPP89_PLEURA | 0.822 |
| MSTO211H_PLEURA | NA |

| Cell line | AUC |
|---|---|
| NB1_AUTONOMIC_GANGLIA | 0.096 |
| NCIH1048_LUNG | 0.116 |
| NCIH1299_LUNG | 0.806 |
| NCIH1339_LUNG | 0.815 |
| NCIH1355_LUNG | 0.878 |
| NCIH1373_LUNG | 0.959 |
| NCIH1435_LUNG | 0.862 |
| NCIH1437_LUNG | 0.882 |
| NCIH1563_LUNG | 0.140 |
| NCIH1568_LUNG | 0.790 |
| NCIH1573_LUNG | 0.995 |
| NCIH1581_LUNG | 0.552 |
| NCIH1623_LUNG | 0.997 |
| NCIH1648_LUNG | 0.828 |
| NCIH1650_LUNG | 0.835 |
| NCIH1651_LUNG | NA |
| NCIH1693_LUNG | 0.949 |
| NCIH1703_LUNG | 0.879 |
| NCIH1792_LUNG | NA |
| NCIH1793_LUNG | 0.290 |
| NCIH1838_LUNG | NA |
| NCIH1915_LUNG | 0.876 |
| NCIH1944_LUNG | 0.911 |
| NCIH1975_LUNG | 0.866 |
| NCIH2009_LUNG | 0.760 |
| NCIH2023_LUNG | NA |
| NCIH2030_LUNG | 0.839 |
| NCIH2052_PLEURA | NA |

| Cell line | AUC |
|---|---|
| NCIH2073_LUNG | 0.878 |
| NCIH2077_LUNG | 0.855 |
| NCIH2087_LUNG | 0.866 |
| NCIH2110_LUNG | 0.837 |
| NCIH2126_LUNG | 0.820 |
| NCIH2170_LUNG | 0.887 |
| NCIH2172_LUNG | 0.794 |
| NCIH2196_LUNG | 0.870 |
| NCIH2228_LUNG | 0.851 |
| NCIH226_LUNG | 0.637 |
| NCIH23_LUNG | 0.951 |
| NCIH2347_LUNG | 0.324 |
| NCIH2444_LUNG | 0.620 |
| NCIH2452_PLEURA | 0.843 |
| NCIH28_PLEURA | 0.936 |
| NCIH292_LUNG | NA |
| NCIH322_LUNG | 0.714 |
| NCIH358_LUNG | NA |
| NCIH441_LUNG | 0.755 |
| NCIH446_LUNG | 0.820 |
| NCIH460_LUNG | 0.917 |
| NCIH520_LUNG | 0.562 |
| NCIH522_LUNG | 0.974 |
| NCIH596_LUNG | 0.893 |
| NCIH647_LUNG | 0.957 |
| NCIH650_LUNG | 0.854 |
| NCIH661_LUNG | 0.819 |
| NCIH727_LUNG | NA |

| Cell line | AUC |
|---|---|
| NCIH747_LARGE_INTESTINE | 0.836 |
| NCIH838_LUNG | 0.834 |
| NCIH841_LUNG | 0.848 |
| NCIN87_STOMACH | 0.881 |
| NIHOVCAR3_OVARY | NA |
| NMCG1_CENTRAL_NERVOUS_SYSTEM | NA |
| NUGC3_STOMACH | 0.261 |
| NUGC4_STOMACH | 0.839 |
| OAW28_OVARY | 0.814 |
| OAW42_OVARY | NA |
| OC314_OVARY | 0.915 |
| OC316_OVARY | 0.872 |
| OE19_OESOPHAGUS | 0.808 |
| OE21_OESOPHAGUS | 0.893 |
| OE33_OESOPHAGUS | 0.847 |
| ONCODG1_OVARY | NA |
| ONS76_CENTRAL_NERVOUS_SYSTEM | 0.811 |
| OSRC2_KIDNEY | 0.842 |
| OV56_OVARY | 0.874 |
| OV7_OVARY | 0.597 |
| OV90_OVARY | 0.939 |
| OVCAR8_OVARY | 0.844 |
| OVISE_OVARY | 0.630 |
| OVKATE_OVARY | 0.851 |
| OVSAHO_OVARY | 0.617 |
| OVTOKO_OVARY | 0.907 |

| Cell line | AUC |
|---|---|
| PANC0203_PANCREAS | 0.927 |
| PANC0327_PANCREAS | 0.813 |
| PANC0403_PANCREAS | 0.877 |
| PANC0504_PANCREAS | 0.245 |
| PANC0813_PANCREAS | NA |
| PANC1_PANCREAS | 0.781 |
| PANC1005_PANCREAS | 0.540 |
| PATU8902_PANCREAS | 0.145 |
| PATU8988S_PANCREAS | NA |
| PATU8988T_PANCREAS | 0.075 |
| PC14_LUNG | 0.500 |
| PC3_PROSTATE | 0.735 |
| PECAPJ15_UPPER_AERODIGESTIVE_TRACT | 0.877 |
| PECAPJ41CLONED2_UPPER_AERODIGESTIVE_TRACT | 0.719 |
| PECAPJ49_UPPER_AERODIGESTIVE_TRACT | 0.843 |
| PEDS005TADH_KIDNEY | 0.448 |
| PEDS015T_SOFT_TISSUE | 0.814 |
| PK1_PANCREAS | 0.859 |
| PK45H_PANCREAS | 0.843 |
| PK59_PANCREAS | 0.778 |
| PLCPRF5_LIVER | 0.833 |
| PSN1_PANCREAS | 0.820 |
| QGP1_PANCREAS | 0.976 |
| RCC10RGB_KIDNEY | 0.801 |
| RCM1_LARGE_INTESTINE | NA |
| RD_SOFT_TISSUE | 0.769 |

| Cell line | AUC |
|---|---|
| RDES_BONE | 0.571 |
| RERFLCAD1_LUNG | 0.782 |
| RERFLCAD2_LUNG | 0.874 |
| RERFLCAI_LUNG | 0.859 |
| RERFLCKJ_LUNG | 0.824 |
| RKN_SOFT_TISSUE | 0.988 |
| RKO_LARGE_INTESTINE | 0.847 |
| RL952_ENDOMETRIUM | 0.403 |
| RMGI_OVARY | NA |
| RMUGS_OVARY | 0.857 |
| RPMI7951_SKIN | 0.786 |
| RT112_URINARY_TRACT | 0.766 |
| RT4_URINARY_TRACT | NA |
| RVH421_SKIN | 0.839 |
| S117_SOFT_TISSUE | 0.866 |
| SBC5_LUNG | 0.948 |
| SCC25_UPPER_AERODIGESTIVE_TRACT | 0.782 |
| SF126_CENTRAL_NERVOUS_SYSTEM | 0.862 |
| SF295_CENTRAL_NERVOUS_SYSTEM | 0.917 |
| SF539_CENTRAL_NERVOUS_SYSTEM | 0.888 |
| SH10TC_STOMACH | 0.886 |
| SH4_SKIN | 0.537 |
| SIMA_AUTONOMIC_GANGLIA | 0.764 |
| SJSA1_BONE | 0.808 |

| Cell line | AUC |
|---|---|
| SKES1_BONE | 0.984 |
| SKHEP1_LIVER | 0.817 |
| SKLU1_LUNG | 0.644 |
| SKMEL2_SKIN | 0.910 |
| SKMEL24_SKIN | 0.862 |
| SKMEL3_SKIN | 0.848 |
| SKMEL30_SKIN | 0.795 |
| SKMEL5_SKIN | 0.831 |
| SKMES1_LUNG | 0.902 |
| SKNAS_AUTONOMIC_GANGLIA | 0.832 |
| SKNBE2_AUTONOMIC_GANGLIA | 0.879 |
| SKNEP1_BONE | 0.523 |
| SKNFI_AUTONOMIC_GANGLIA | 0.852 |
| SKOV3_OVARY | 0.850 |
| SKPNDW_BONE | 0.861 |
| SKUT1_SOFT_TISSUE | 0.845 |
| SNB75_CENTRAL_NERVOUS_SYSTEM | 0.768 |
| SNGM_ENDOMETRIUM | 0.794 |
| SNU1041_UPPER_AERODIGESTIVE_TRACT | 0.806 |
| SNU1066_UPPER_AERODIGESTIVE_TRACT | 0.860 |
| SNU1076_UPPER_AERODIGESTIVE_TRACT | NA |
| SNU1077_ENDOMETRIUM | 0.759 |

| Cell line | AUC |
|---|---|
| SNU1079_BILIARY_TRACT | NA |
| SNU1105_CENTRAL_NERVOUS_SYSTEM | 0.620 |
| SNU1196_BILIARY_TRACT | 0.653 |
| SNU1214_UPPER_AERODIGESTIVE_TRACT | 0.826 |
| SNU182_LIVER | 0.179 |
| SNU213_PANCREAS | NA |
| SNU216_STOMACH | 0.521 |
| SNU245_BILIARY_TRACT | 0.853 |
| SNU308_BILIARY_TRACT | 0.873 |
| SNU398_LIVER | 0.951 |
| SNU407_LARGE_INTESTINE | 0.284 |
| SNU410_PANCREAS | 0.994 |
| SNU423_LIVER | 0.697 |
| SNU449_LIVER | 0.971 |
| SNU46_UPPER_AERODIGESTIVE_TRACT | 0.925 |
| SNU601_STOMACH | NA |
| SNU61_LARGE_INTESTINE | 0.881 |
| SNU668_STOMACH | 0.423 |
| SNU685_ENDOMETRIUM | 0.812 |
| SNU719_STOMACH | 0.869 |
| SNU738_CENTRAL_NERVOUS_SYSTEM | 0.826 |
| SNU761_LIVER | NA |
| SNU8_OVARY | NA |
| SNU81_LARGE_INTESTINE | 0.400 |
| SNU840_OVARY | 0.848 |

| Cell line | AUC |
|---|---|
| SNU869_BILIARY_TRACT | 0.782 |
| SNU878_LIVER | 0.890 |
| SNU886_LIVER | 0.656 |
| SNU899_UPPER_AERODIGESTIVE_TRACT | NA |
| SNUC2A_LARGE_INTESTINE | 0.608 |
| SNUC4_LARGE_INTESTINE | 0.290 |
| SNUC5_LARGE_INTESTINE | NA |
| SQ1_LUNG | 0.838 |
| SU8686_PANCREAS | 0.848 |
| SUIT2_PANCREAS | 0.856 |
| SW1088_CENTRAL_NERVOUS_SYSTEM | 0.835 |
| SW1116_LARGE_INTESTINE | 0.879 |
| SW1271_LUNG | 0.718 |
| SW1573_LUNG | 0.894 |
| SW1710_URINARY_TRACT | 0.835 |
| SW1783_CENTRAL_NERVOUS_SYSTEM | NA |
| SW1990_PANCREAS | 0.874 |
| SW780_URINARY_TRACT | 0.986 |
| SW837_LARGE_INTESTINE | 0.995 |
| SW948_LARGE_INTESTINE | 0.766 |
| T24_URINARY_TRACT | 0.906 |
| T3M10_LUNG | 0.951 |
| T47D_BREAST | 0.698 |
| T98G_CENTRAL_NERVOUS_SYSTEM | 0.830 |
| TC32_BONE | 0.681 |

| Cell line | AUC |
|---|---|
| TCCPAN2_PANCREAS | 0.905 |
| TCCSUP_URINARY_TRACT | NA |
| TE1_OESOPHAGUS | 0.875 |
| TE10_OESOPHAGUS | 0.872 |
| TE11_OESOPHAGUS | 0.870 |
| TE14_OESOPHAGUS | 0.898 |
| TE4_OESOPHAGUS | NA |
| TE5_OESOPHAGUS | 0.797 |
| TE6_OESOPHAGUS | 0.864 |
| TE8_OESOPHAGUS | 0.802 |
| TE9_OESOPHAGUS | 0.911 |
| TEN_ENDOMETRIUM | 0.815 |
| TM31_CENTRAL_NERVOUS_SYSTEM | 0.424 |
| TOV112D_OVARY | 0.871 |
| TOV21G_OVARY | 0.864 |
| TT_OESOPHAGUS | 0.859 |
| TT_THYROID | 0.179 |
| TT2609C02_THYROID | 0.251 |
| TTC549_SOFT_TISSUE | NA |
| TTC709_SOFT_TISSUE | 0.520 |
| TUHR4TKB_KIDNEY | 0.265 |
| U118MG_CENTRAL_NERVOUS_SYSTEM | 0.871 |
| U251MG_CENTRAL_NERVOUS_SYSTEM | 0.806 |
| U2OS_BONE | 0.862 |
| U87MG_CENTRAL_NERVOUS_SYSTEM | 0.724 |

| Cell line | AUC |
|---|---|
| UACC257_SKIN | 0.863 |
| UACC62_SKIN | 0.837 |
| UBLC1_URINARY_TRACT | 0.875 |
| UO31_KIDNEY | 0.710 |
| VMRCRCW_KIDNEY | 0.345 |
| VMRCRCZ_KIDNEY | 0.969 |
| WM1799_SKIN | 0.101 |
| WM2664_SKIN | 0.578 |
| WM793_SKIN | 0.866 |
| WM88_SKIN | 0.802 |
| WM983B_SKIN | 0.861 |
| X2313287_STOMACH | 0.254 |
| X253J_URINARY_TRACT | 0.905 |
| X253JBV_URINARY_TRACT | 0.966 |
| X42MGBA_CENTRAL_NERVOUS_SYSTEM | 0.845 |
| X5637_URINARY_TRACT | 0.624 |
| X639V_URINARY_TRACT | NA |
| X647V_URINARY_TRACT | 0.284 |
| X769P_KIDNEY | 0.463 |
| X786O_KIDNEY | 0.853 |
| X8305C_THYROID | 0.849 |
| X8505C_THYROID | 0.558 |
| X8MGBA_CENTRAL_NERVOUS_SYSTEM | 0.805 |
| YAPC_PANCREAS | 0.719 |
| YD10B_UPPER_AERODIGESTIVE_TRACT | 0.903 |
| YD15_SALIVARY_GLAND | NA |
| YD38_UPPER_AERODIGESTIVE_TRACT | 0.877 |
| YD8_UPPER_AERODIGESTIVE_TRACT | 0.501 |
| YKG1_CENTRAL_NERVOUS_SYSTEM | 0.936 |
| ZR751_BREAST | 0.853 |

Conclusion: These data demonstrated the activity of Compound 1 on solid tumor cell lines, with the highest activity on lung, intestine, stomach, liver, pancreatic and kidney cell lines.

**Example 31: Effect of Compound 1 and everolimus in QGP1 model- In vivo assay**

The combination treatment of Compound 1 and everolimus was tested in human QGP1 pancreatic somatostatinoma xenografts in female NOD/SCID mice. Treatment began on Day 1 in nine groups of mice (n = 10) with established subcutaneous QGP1 tumors. Everolimus was dosed at 3 or 10 mg/kg, intraperitoneally (i.p.) daily for 21 days (qd $\times$ 21). Vehicle (5% NMP/45% PEG400/50% saline) and Compound 1 (2.5, 5 or 10 mg/kg) were dosed intraperitoneally (i.p.) twice a day (bid $\times$ 21), three hours apart. Group 1 received vehicle; Groups 2 and 3 received 3 and 10 mg/kg everolimus, respectively; Groups 4, 5 and 6 received 2.5, 5 and 10 mg/kg Compound 1, respectively; Groups 7, 8, and 9 received 3 mg/kg everolimus plus 2.5, 5 and 10 mg/kg Compound 1, respectively. Tumors were measured using calipers twice each week to the end of the study on Day 52. Three tumors at or near the group median tumor volume 2000 $mm^3$ endpoint were sampled from each group.

Treatment efficacy was based on tumor growth inhibition (TGI), defined as the difference between the final (Day 52) median tumor volumes (MTVs) of treated and control mice. The results were analyzed utilizing the Mann-Whitney U-test, and were deemed statistically significant at $P \leq 0.05$. Drug tolerability was assessed by body weight measurements and by frequent observation of treated animals.

The Day 52 median tumor volume (MTV) of Group 1 vehicle-treated control mice was 2181 $mm^3$, with individual tumor volumes ranging from 650 to 5000 $mm^3$. Everolimus monotherapy Groups 2 and 3 resulted in tumor growth inhibition of 41 and 39%, respectively. Further, Group 2 was more efficacious than Compound 1 monotherapy Groups 4, 5 and 6 ($P \leq 0.01$ v. Groups 4 and 5, $P \leq 0.001$), even though Groups 2 and 3 did not reach statistical significance compared to controls. The only treatment regimen to reach statistically significant efficacy was Group 9 (3 mg/kg everolimus and 10 mg/kg Compound 1), which achieved two partial tumor regressions (PR) and tumor growth inhibition (TGI) of 70% compared to controls, with a Day 52 MTV of 650 $mm^3$ and individual tumor volumes ranging from 446 to 1688 $mm^3$. All regimens were well-tolerated with moderate weight loss (1.6 to 6.8%) between Days 14 and 17 in all groups receiving Compound 1.

Conclusion: under the conditions of this study groups dosed with everolimus demonstrated some tumor growth inhibition that was not statistically significant. Furthermore, the combination of everolimus (3 mg/kg) and Compound 1 (10 mg/kg) significantly delayed the growth of human QGP1 pancreatic somatostatinoma xenografts in female NOD/SCID over the corresponding monotherapies.

**Example 32: Reduction of GSPT1 levels in BON cells upon treatment with combinations of Compound 1 and Everolimus**

**Methods:** BON cells were cultured in DMEM: F12K media containing 10% fetal bovine serum and penicillin/streptomycin antibiotics. Cells were plated at 3e6 cells in 10 cm dish and treated daily with vehicle, RAD (everolimus) and/or increasing concentrations of Compound 1 alone over 120 h. Cells were harvested and probed with antibodies for western blots and imaged using a Li-Cor Odyssey imaging system. All total and phosphorylated antibodies were obtained from Cell Signaling Technology.

**Results:** Compound 1 caused dose dependent GSPT1 degradation and has no direct effect on mTORC1 effectors such as p4EBP1 or S6K1 mediated phosphorylation of ribosomal protein S6. In contrast, RAD dramatically decreased phosphorylation of S6 and phosphorylation of 4EBP1 through inhibition of mTORC1, and increased pAKT through negative feedback FIG. 99. Compared to single compound alone, a combination of RAD and Compound 1 significantly increased GSPT1 degradation, increased inhibition of cell growth (pS6) and cap-dependent translation (decrease in p4EBP1, p-elF2, pAKT, elFG1 & cyclin D1) and activated AMPK pathway (increase in pAMPK).

**Conclusion:** Treatment of pancreatic neuroendocrine tumor cells with a combination of RAD and Compound 1 resulted in increased degradation of GSPT1 compared to treatment with RAD or Compound 1 alone, which may lead to augmented effect of RAD in cell growth, cap-dependent translation and metabolic activity.

**Example 33: Effect on AML cells of combination treatment with everolimus or kinase inhibitors and Compound 1**

To assess whether inhibition of mTOR, FLT3, JAK2, or JAK3could enhance the response of AML cells to Compound 1, U937 AML cells were treated with Compound 1 alone or in combination with a panel of kinase inhibitors. Cells were treated with DMSO vehicle control or Compound 1 in the presence or absence of various inhibitors at specified concentrations (see FIGs. 101A-101E) for 5 days. Cell proliferation was measured by Cell-Titer Glo, and the percentage of cell proliferation was normalized to parental cells treated with DMSO control.

Additionally, U937 AML cells were treated with 100 nM Compound 1 alone or in combination with everolimus, quizartinib, ruxolitinib, AZD1480, and tofacitinib at specified concentrations (see FIG. 102) for 16 hours. Whole cell extracts were collected and subjected to immunoblot analysis.

**Results:** The 5 kinase inhibitors and everolimus augmented the anti-proliferative effect of Compound 1, as

shown by a leftward shift of the proliferation ICso value (FIG. 100A to FIG. 100E). The growth inhibitory effect of Compound 1 was enhanced by combination treatment with everolimus, ruxolitinib, AZD 1480, and tofacitinib and was linked to increased GSPT1 depletion and Caspase-3 cleavage (FIG. 101).

**Conclusion:** Treatment of AML cells with a combination of Compound 1 and mTOR, FLT3, JAK2, or JAK3 inhibitors resulted in increased degradation of GSPT1 and increased apoptosis compared to treatment with Compound 1 or the inhibitors alone.

**Example 34: Treatment of AML cell lines with Compound 1 as single agent or in combination with Venetoclax.**

**Methods:** *Cell TitreGlo*: 5000 cells were plated in 50 μl per well in 384 well plates containing compound combinations in indicated contentrations. After 48 h treatment, relative ATP levels were measured by adding Cell TitreGlo reagent, incubating in the dark for 30 min, and then measuring luminescence on an Envision Plate Reader.

**Results:** Synergy was shown for combination treatment of multiple AML cell lines with Compound 1 and Venetoclax.

**Conclusion:** Treatment with Venetoclax sensitized AML cells to Compound 1 treatment. This synergistic effect was shown by the leftward shift of Compound 1 dose response curves in the presence of sub-lethal doses of Venetoclax. FIGs. 102A-102G show the effect on AML cell line proliferation when incubated for 48 h with increased concentrations of Compound 1 with and without Venetoclax at the indicated concentrations.

**Example 35: Combination treatment of AML cells with Compound 1 and Venetoclax.**

**Methods:** *Cell TitreGlo:* KG-1 cells were seeded (200,000 cells per ml, 0.05 mL per well) in 384-well tissue culture plates (Corning, 3764), and then grown for 1 day at 37 °C, 5% $CO_2$. Cells were then treated with indicated concentrations of Venetoclax and/or Compound 1 for 3 days at 37 °C, 5% $CO_2$. After treatment, relative ATP levels were measured by adding Cell TitreGlo reagent (0.025 mL per well), incubating in the dark for 20 min, and then measuring luminescence on an Envision Plate Reader.

*Western Blots:* KG-1 cells were seeded in 6 well tissue culture plates at 500,000 cells per mL, 4 mL per well. Cells were treated with indicated doses of Venetoclax and/or Compound 1 for 16 h at 37 °C, 5% $CO_2$. Cells were then washed twice with cold PBS and lysed with M-PER lysis buffer (Thermo) supplemented with 150 mM NaCl and Halt protease and phosphatase inhibitor cocktail (Thermo) on ice for 30 min. Crude lysates were clarified by centrifugation (14000 rpm, 10 min, 4 °C) and total protein quantified by BCA assay. GSPT1 (Abcam, ab49878), Mcl-1 (CST, 4572), Bcl-2 (CST, 4223), cleaved caspase 3 (CST, 9664), and GAPDH (Santa Cruz, sc-47724) levels were probed by western and imaged on a LiCor infrared imager.

*Caspase 3/7 and Confluency Live-cell Analysis:* KG-1 cells were seeded (200,000 cells per ml, 0.05 mL per well) in 384-well tissue culture plates (Corning, 3764) previously coated with fibronectin (Sigma, F1141), and grown for 1 day at 37 °C, 5% $CO_2$. Cells were then treated with indicated concentrations of Venetoclax and/or Compound 1 and Caspase-3/7 Green Apoptosis Assay Reagent (Incucyte, 4440). Cells were imaged every 4 h with an IncuCyte Live Cell Analysis Imaging System over the course of 3 days while incubated at 37 °C, 5% $CO_2$. FIG. 103 shows the relative ATP levels as a measure of viability in response to several dose combinations of Compound 1 and Venetoclax. FIG. 104 shows western blot analysis measuring GSPT1, Mcl-1, Bcl-2, cleaved caspase 3, and GAPDH protein levels in KG-1 cells 16 hours after treatment with a set of doses of Compound 1, Venetoclax or the combination of Compound 1 and Venetoclax. FIGs. 105A and 105B shows the live cell analyses of KG-1 confluency (FIG. 106A) and apoptotic event counts (FIG. 106B) showing that Compound 1 and Venetoclax combination treatment enhances apoptosis compared to treatment with single agent.

**Results:** Synergy was shown for combination treatment of the FLT3-ITD AML cell line (KG-1) with Compound 1 and Venetoclax. Compound 1 alone, and in combination with Venetoclax, was shown to reduce levels of Mcl-1 in KG-1 cells. Additionally, Compound 1 and Venetoclax combination treatment of KG-1 cells enhanced apoptosis compared to treatment with the single agents.

**Conclusion:** Treatment of AML cells with Compound 1 decreased the levels of MCL-1. Combination treatment with Venetoclax and Compound 1 was synergistic, and resulted in enhanced apoptosis, compared to treatment with Venetoclax or Compound 1 alone.

**Example 36: Effect of combination treatment with everolimus and Compound 1 on AML cell lines**

To determine whether inhibition of mTOR could promote Compound 1-induced GSPT1 depletion and induce apoptosis, AML cells were treated with Compound 1 alone, everolimus alone or with the combination of everolimus and Compound 1. Whole cell extracts were collected and subjected to immunoblot analysis.

**Results:** Although everolimus treatment significantly blocked the activation of the mTOR pathway, shown by

reduced phosphorylation levels of S6K1 and 4E-BP1, it had minimal effect on GPST1 expression and Caspase-3 cleavage (See FIG. 106). However, everolimus dramatically enhanced the effect of Compound 1 on GSPT1 degradation, Mcl-1 loss, and induction of apoptosis, as compared to treatment with Compound 1 alone.

The sensitization to Compound 1 conferred by everolimus was observed in multiple AML cell lines (MOLM-13, MV-4-11, NB-4, U937, UT-7, OCI-AML2 and F-36P). In AML cell lines HNT-34 and KG1, Compound 1 treatment alone was sufficient to induce robust GSPT1 degradation, Caspase-3 cleavage, and Mcl-1 loss.

**Conclusion:** Treatment of AML cells with a combination of Compound 1 and everolimus resulted in increased degradation of GSPT1, increased apoptosis, and loss of Mcl-1, compared to treatment with Compound 1 alone.

### Example 37: Effect of Compound 1 on cells from MDS patients

**Methods.** Bone marrow mononuclear cells (BMMCs) from MDS patients or matching $CD34^+$ cells isolated from BMMCs from same patient samples, were cultured ex vivo to assess their sensitivity to Compound 1. BMMCs were seeded in complete media (CellGenix media supplemented with LDL, FLT3L, IL-6, IL-3 and TPO) at $10^6$ cells/mL and $CD34^+$ cells were seeded in complete stem media (Serum Free StemSpan media supplemented with CC100 cytokine cocktail) at $5\times10^5$ cells/mL. All cultures were exposed to Compound 1 at 0, 37 and 111 nM during up to 10 days and cells were collected twice a week for cell media replacement and cell count assessment.

**Results.** As shown in FIG. 107, exposure to Compound 1 reduced cell numbers in total BMMCs but also in $CD34^+$ blast cells in samples from 2 different MDS patients. Kinetics and concentrations required were different across patients and cell types.

**Conclusion.** Compound 1 produced a decrease in cell number in MDS samples showing a different spectrum of sensitivity between bulk BMMCs and isolated $CD34^+$ blast cells

### Example 38: Effect of treatment with Compound 1 and everolimus on cells from MDS patients

**Methods.** BMMCs from MDS patients were cultured ex vivo to assess their sensitivity to treatment with Compound 1 in combination with everolimus. In order to evaluate the bulk effect on MDS bone marrow cells, total BMMCs were seeded in complete media (CellGenix media supplemented with LDL, FLT3L, IL-6, IL-3 and TPO) media at $10^6$ cells/mL and exposed to Compound 1 (0, 37, 111 and 333 nM) alone or in combination with everolimus at a fixed concentration of 111 nM. Cultures were maintained for 1 week and cell numbers were measured by viable cell count on days 1, 3 and 7 of culture. Effect on apoptosis and GSPT1 degradation was measured by flow cytometry 24 hours after exposure.

To better define Compound 1 effect on MDS progenitors/stem cells, these BMMCs were seeded in Methocult media and exposed to Compound 1 (0, 37, 111, 333 and 1000 nM) alone or in combination with everolimus at 111 nM to assess their clonogenic potential. Colony numbers were scored after 14 days of culture using Stem Vision.

**Results.** FIG. 108A shows the effect of treatment with Compound 1 on BMMCs from a MDS patient sample. Treatment with 37 nM Compound 1 reduced cell numbers by ~70% after 3 days of culture and this effect was enhanced to up to 95% cell number decrease at higher concentrations. No effect of single agent was observed at 37 nM. Interestingly, when combined with everolimus, this effect was significantly increased and treatment at this low dose of 37 nM Compound 1 combined with everolimus achieved similar inhibitory effects as observed for treatment with 111 nM Compound 1 single agent. FIG. 108B shows the same trends in MDS progenitor/stem cells. GSPT1 was degraded after 24 hours exposure to Compound 1 and this effect was slightly increased at lower doses by combination treatment with everolimus (FIG. 109). Caspase 3 was activated upon Compound 1 exposure and this effect was increased by combination with everolimus.

**Conclusion.** In summary, combination treatment with everolimus increased sensitivity to low doses of Compound 1 in MDS patient samples and may improve therapeutic index. This effect was mediated by induction of apoptosis as a consequence of GSPT1 degradation.

### Example 39: A Phase 1, Open-Label, Dose-Finding Study Of Compound 1, A Novel Cereblon E3 Ligase Modulating Drug, In Subjects With Relapsed Or Refractory Acute Myeloid Leukemia

**Primary Objectives:** To determine the safety and tolerability of Compound 1. To define the non-tolerated dose (NTD), the maximum tolerated dose (MTD) and/or the recommended Phase 2 dose (RP2D) of Compound 1.

**Secondary Objectives:** To provide information on the preliminary efficacy of Compound 1. To characterize the pharmacokinetics (PK) of Compound 1.

**Study Design:** This study is an open-label, Phase 1, dose escalation and expansion, first-in-human clinical study of Compound 1 in subjects with relapsed or refractory AML. The dose escalation part (Part A) of the study will evaluate the safety and tolerability of escalating doses of Compound 1, administered intravenously, and determine the

MTD of Compound 1. In Part A, two formulations will be tested. The expansion part (Part B) will further evaluate the safety and efficacy of Compound 1 administered at or below the MTD in selected expansion cohorts of up to approximately 20 evaluable subjects each in order to determine the RP2D. One or more dosing regimens may be selected for cohort expansion. Parts A and B will consist of 3 periods: Screening, Treatment, and Follow-up. Leukemia response will be determined by the Investigator. Disease assessment will be based on the International Working Group Response Criteria in AML (Cheson, et al, J Clin Oncol 2003; 21(24):4642-9).

**Screening Period:** The Screening Period starts 28 days prior to first dose of Compound 1. The informed consent document must be signed and dated by the subject and the administering staff prior to the start of any other study procedures. All screening tests and procedures must be completed within the 28 days prior to the first dose of Compound 1.

**Treatment Period:** In the Treatment Period, Compound 1 will be administered intravenously on Days 1-5 of each 28 day cycle for up to 4 cycles in the absence of disease progression, relapse, unacceptable toxicity, or subject/physician decision to withdraw. (Disease progression is defined as: a > 50% increase in bone marrow blast count percentage from the baseline (screening) bone marrow blast count that persists for at least 2 bone marrow assessments separated by at least 1 month, unless the baseline bone marrow blast count is > 70%, in which case, a finding of > 70% blasts that persists for 2 postbaseline bone marrow assessments separated by at least 1 month would be considered progression, or a doubling of the baseline absolute peripheral blood blast count that persists for ≥ 7 days and the final absolute peripheral blood blast count is > 10 × 109/L.) During Part A, 2 additional cycles of treatment beyond Cycle 4 may be allowed if the subject is demonstrating clinical benefit (SD or PR) and tolerating the study drug without unacceptable toxicity. Modified dosing schedules (eg, increasing from 5 days to up to 10 days of dosing or increasing infusion length) may be evaluated in additional cohorts, if necessary, based on toxicity, PK profiles, and PD findings. All subjects will be required to start calcium, calcitriol, and vitamin D supplementation at least 3 days prior to Day 1 of each cycle and continue until ≥ 3 days after the last dose of Compound 1 in each cycle (eg, ≥ Day 8 when Compound 1 is administered on Days 1-5).

In Cycle 1, a bone marrow evaluation will be performed on Day 28 (± 3 days). Based on the Day 28 bone marrow evaluation, subjects with hypoplastic bone marrow, without evidence of persistent leukemia, who have Grade ≥ 3 neutropenia will be followed for an additional 2 weeks for safety monitoring in Cycle 1 (total duration of 42 days). An additional bone marrow assessment will be performed at the time of hematologic recovery or Day 42 (± 3 days). Thus, in Part A, the window for evaluation of dose-limiting toxicity (DLT) during Cycle 1 will be up to 42 days (28 or 42 days). Cycles ≥ 2 will be 28 days in length. Subsequent cycles should start ≤ 7 days following the last day of the previous cycle.

**Follow-up Period:** In the Follow-up Period, all subjects will be followed for 28 days (± 3 days) after the last dose of Compound 1 for safety. Subjects without documented progression of disease (or relapse) will have efficacy evaluations of complete blood counts and peripheral blood smears performed every subsequent 8 weeks (± 1 week) for the 1st year and every 12 weeks (± 2 weeks) for the 2nd year or until progression of disease (or relapse), initiation of a new anticancer therapy, withdrawal of consent from the study, death, or the End of Trial, whichever comes first. A bone marrow evaluation will be completed at the end of the 1st year and as clinically indicated during the Follow-up Period.

All subjects will be followed for survival follow-up according to the schedule for the efficacy long term follow-up for up to 2 years or until death, lost to follow-up, or the End of Trial, whichever occurs first. Survival follow-up may be conducted by record review (including public records) and/or telephone contact with the subject, family, or the subject's treating physician.

**Part A-Dose Escalation.** During the escalation phase (Part A), a modified accelerated titration design (Simon et al, J Natl Cancer Inst 1997; 89(15): 1138-47) will be used to establish initial toxicity. Cohorts of one or more subjects each will be administered Compound 1 at doses that will increase in 100% increments per cohort until ≥ 2 subjects experience a Compound 1-related Grade ≥ 2 adverse event in the DLT window (may be different cohorts), or ≥ 1 subject experiences a DLT within the DLT window. At that time the current cohort and all subsequent cohorts will be expanded enrolling 3 to 6 subjects. A dose escalation schedule with dose increments not to exceed 50% will concurrently be initiated in order to establish the NTD and MTD. The initial dose will be 0.3 mg. At the study start an initial formulation of Compound 1 (Formulation A) will be utilized, and during dose escalation a second formulation of Compound 1 (Formulation Ib described in Table 43) will be introduced to replace Formulation A. Formulation A has the following composition (*see* US Publication No. 2017/0196847-A1).

| | Formulation A |
|---|---|
| Compound 1 | 1.05 mg/vial |
| Citric acid anhydrous, USP | 18.6 mg/vial |
| Sodium citrate anhydrous, USP | 18.4 mg/vial |

(continued)

| | Formulation A |
|---|---|
| Kleptose® HPB (HP-β-CD), parenteral grade | 840 mg/vial |
| Dimethyl sulfoxide (in process media) | Partially removed upon drying |
| Formic acid | - |
| Water for injection (in process media) | Removed upon drying |

The DMA residual solvent in Formulation A must not exceed the permitted daily exposure (PDE) limits set in the ICH *Q3C Impurities: Residual Solvents* in order to proceed with dose escalation cohorts above a daily Compound 1 dose of 2.4 mg. The residual solvent (formic acid) level in Formulation Ib allows daily doses of Compound 1 of up to 20 mg without exceeding its PDE set in the ICH Q3C guidance.

The Formulation Ib will be introduced at a new dose cohort (in accordance with study dose escalation guidelines) after review of observed toxicities seen in the initial dose levels of Formulation A.

It may be decided to evaluate a higher dose cohort, additional subjects within a dose cohort, intermediate dose cohorts, smaller dose increments, alternate dosing schedules (eg, increasing from 5 to up to 10 days of Compound 1 administration or longer infusion times), and/or declare an MTD based on their review of available clinical and laboratory safety data, PK profiles, and PD findings. In the event that an alternate dosing schedule is evaluated, the starting dose and schedule will not exceed the dose intensity of a dose cohort that has previously met the criteria for dose escalation.

After the first dose is administered in any cohort during dose escalation, subjects in each cohort are observed for at least 28 days and up to 42 days (Cycle 1, DLT window) before the next higher, dose cohort can begin. No more than one subject per day will be enrolled in a given dose escalation cohort. A subject evaluable for DLT is defined as one that: has received at least 80% of the total planned Cycle 1 dose (eg, 4 complete Compound 1 doses for a 5-day dose schedule; in case of a missed dose, ≥ 4 doses to be completed on or before Day 7) of Compound 1 during Cycle 1 without experiencing a DLT, or experienced a DLT after receiving at least one dose (or fraction thereof) of Compound 1.

In the event that an alternate dose schedule (eg, increasing from 5 days to up to 10 days of dosing) is evaluated in Part A, the same criteria for determining DLT-evaluable subjects will be applied. Subjects non-evaluable for DLT will be replaced.

A dose will be considered intolerable if > 33% of evaluable subjects in a dose cohort experience DLT during Cycle 1. The MTD will be defined as the last dose below the NTD, at which ≤ 33% of evaluable subjects experienced DLT during Cycle 1. If 2 or more of 6 evaluable subjects experience DLTs in the first dose cohort, a lower dose cohort may be explored (ie, 0.1 mg Compound 1). An intermediate dose of Compound 1 (one between the NTD and the last dose level before the NTD) may be evaluated to accurately determine the MTD.

Intra-subject dose escalation will not be allowed during the DLT assessment period; however, in Cycles ≥ 2, subjects without evidence of disease progression who are tolerating their assigned dose of Compound 1 may escalate to the highest dose level shown to be adequately tolerated by at least one cohort of subjects in this study (ie, ≤ 33% of evaluable subjects having experienced a DLT at that dose level). If the highest tolerated dose level is Formulation Ib, a subject currently enrolled on Formulation A is permitted to switch to the newer formulation.

**Part B-Cohort Expansion:** Following completion of dose escalation (Part A), additional subjects may be enrolled into an expansion phase (Part B) with up to approximately 20 evaluable subjects in each cohort. Expansion may occur at the MTD and schedule established in the dose escalation phase, and/or at an alternative tolerable dose and schedule, based on review of safety, PK, and PD data from Part A. One or more dosing regimens (dose, schedules) may be selected for cohort expansion.

**Study Population:** Men and women, 18 years or older, with relapsed or refractory AML as defined by World Health Organization (WHO) criteria, who are not suitable for other established therapies, will be enrolled in the study.

**Length of Study.** Enrollment is expected to take approximately 18 to 24 months to complete (12 to 15 months for dose escalation, and 6 to 9 months for expansion). Completion of active treatment and post-treatment follow-up is expected to take an additional 6 to 24 months. The entire study is expected to last up to approximately 3 to 4 years. The End of Trial is defined as either the date of the last visit of the last subject to complete the post-treatment follow-up, or the date of receipt of the last data point from the last subject that is required for primary, secondary and/or exploratory analysis, as prespecified in the protocol, whichever is the later date.

**Study Treatments.** The investigational product, Compound 1 for IV injection, labeled appropriately for investigational use as per the regulations of the relevant country health authority will be provided. Study drug will be administered as outlined in the Treatment Period section above.

Study treatment may be discontinued if there is evidence of clinically significant disease progression (or relapse), unacceptable toxicity or subject/physician decision to withdraw. Subjects may continue to receive study drugs beyond

disease progression at the discretion of the Investigator in consultation with the Celgene Medical Monitor.

**Overview of Key Efficacy Assessments.** The primary efficacy variable is leukemia response rate. All treated subjects will be included in the efficacy analyses. Leukemia response will be determined by the Investigator. Assessment will be based on the International Working Group Response Criteria in AML (Cheson, et al, J Clin Oncol 2003;21(24):4642-9).

A descriptive analysis of evidence of antileukemic activity will be provided based on clinical, laboratory, molecular, and cytogenetic assessments by Investigator, which includes assessment of bone marrow blast percentage, bone marrow cytogenetics, molecular genetic studies to evaluate molecular responses, bone marrow flow cytometry, platelet count, and absolute neutrophil count. Response criteria will be summarized by best overall response categories: complete remission rate (CRR), and objective response rate (ORR). The ORR includes all responses of complete remission (CR) (ie, morphologic leukemia-free state, morphologic CR, cytogenetic CR, molecular CR, and morphologic CR with incomplete blood recovery), and partial remission.

The efficacy variable of focus will be ORR and CRR. Other measures of clinical activity including overall survival (OS), relapse-free survival (RFS), progression-free survival (PFS), event-free survival, duration of remission, duration of response, and time to remission/response will be summarized.

**Overview of Key Safety Assessments.** The safety variables for this study include adverse events, safety clinical laboratory variables, 12-lead electrocardiograms, Eastern Cooperative Oncology Group Performance Status, left ventricular ejection fraction assessments, physical examinations, vital signs, exposure to study treatment, assessment of concomitant medications, and pregnancy testing for females of childbearing potential.

**Overview of Key Pharmacokinetic Assessments.** The plasma PK parameters determined for Compound 1 will be maximum observed plasma concentration ($C_{max}$), area under the plasma concentration-time curve from time 0 to 24 hours postdose ($AUC_{24}$), terminal-phase elimination half-life ($t_{1/2}$), total plasma clearance (CL), time to peak (maximum) plasma concentration (tmax), volume of distribution at the steady state (Vss). Selected PK parameters (eg, $C_{max}$, $AUC_{24}$, $t_{1/2}$) will be estimated for R- and S-enantiomers of Compound 1 as appropriate.

**Statistical Methods.** Statistical analyses will be performed by dose level (Part A) and cohort (Part B) as needed or applicable. All analyses will be descriptive in nature. All summaries of safety data will be conducted using subjects receiving any Compound 1 (the Treated Population).

The efficacy variables of primary interest are the ORR and CRR. Other preliminary efficacy variables including OS, RFS, PFS, event-free survival, duration of remission, duration of response, and time to remission/response will be summarized. Efficacy analysis will be repeated for the Treated Population and Efficacy Evaluable Population (received a baseline leukemia assessment evaluation, at least one cycle of study treatment or at least 80% of scheduled doses in Cycle 1, and one on-study leukemia assessment evaluation), with the result using the Treated Population considered primary.

All biomarker-related data presentations will be based on treated subjects with at least one biomarker assessment, unless specified otherwise. Descriptive statistics will be presented for baseline and change from baseline of continuous biomarker endpoints, by dosing regimens and/or disease subsets, and overall.

The study will be conducted in compliance with International Council for Harmonisation of Technical Requirements for Pharmaceuticals for Human Use (ICH)/Good Clinical Practice and applicable regulatory requirements.

**Inclusion Criteria.** Subjects must satisfy the criteria below to be enrolled in dose escalation (Part A) or dose expansion (Part B) of this study.

1. Men and women ≥ 18 years of age, at the time of signing the informed consent document (ICD).
2. Subject must understand and voluntarily sign an ICD prior to any study-related assessments/procedures being conducted.
3. Subject is willing and able to adhere to the study visit schedule and other protocol requirements.
4. Relapsed or refractory AML as defined by World Health Organization (WHO) criteria who are not suitable for other established therapies.
5. Eastern Cooperative Oncology Group Performance Status (ECOG PS) of 0 to 2.
6. At least 4 weeks (from first dose) has elapsed from donor lymphocyte infusion (DLI) without conditioning.
7. Subjects must have the following screening laboratory values:

- Corrected serum Ca or free (ionized) serum Ca within normal limits (WNL).

  ○

  ○ Corrected Ca (mg/dL) = Total Ca (mg/dL) – 0.8 (albumin [g/dL] – 4)

- Total White Blood Cell count (WBC) < 25 × $10^9$/L prior to first infusion. Prior or concurrent treatment with hydroxyurea to achieve this level is allowed.
- Potassium and magnesium within normal limits or correctable with supplements.
- Aspartate aminotransferase/serum glutamic oxaloacetic transaminase (AST/SGOT) or alanine aminotransferase/serum glutamate pyruvic transaminase (ALT/SGPT) ≤ 2.5 × Upper Limit of Normal (ULN).
- Uric acid ≤ 7.5 mg/dL (446 μmol/L). Prior and/or concurrent treatment with hypouricemic agents (eg, allopurinol, rasburicase) are allowed.
- Serum bilirubin ≤ 1.5 × ULN.
- Estimated serum creatinine clearance of ≥ 60 mL/min using the Cockcroft-Gault equation.
- INR < 1.5 × ULN and PTT < 1.5 × ULN.

8. Per the Compound 1 Pregnancy Prevention Plan (PPP):

- Females of childbearing potential (FCBP) must undergo pregnancy testing based on the frequency outlined in PPP and pregnancy results must be negative.
- Unless practicing complete abstinence from heterosexual intercourse, sexually active FCBP must agree to use adequate contraceptive methods as specified in PPP.

    - FCBP must agree to use two reliable forms of contraception simultaneously (or to practice complete abstinence), without interruption, for 28 days before starting Compound 1, throughout the entire duration of Compound 1 treatment, during dose interruptions and for at least 28 days after the last dose of Compound 1.
    - Complete abstinence is only acceptable in cases where this is the preferred and usual lifestyle of the subject.
    - Periodic abstinence (calendar ovulation, symptothermal, post-ovulation methods) and withdrawal are not acceptable.

- Unless practicing complete abstinence from heterosexual intercourse, sexually active males (including those who have had a vasectomy) must use barrier contraception (condoms) when engaging in sexual activity with FCBP as specified in PPP.

    - Complete abstinence is only acceptable in cases where this is the preferred and usual lifestyle of the subject.

- Females must agree to abstain from breastfeeding or providing breast milk for the duration specified in the PPP.
- Males must agree not to donate semen or sperm for the duration specified in the PPP.
- All subjects must:

    - Understand that Compound 1 could have a potential teratogenic risk.
    - Agree to abstain from donating blood for the duration specified in the PPP.
    - Be counseled about pregnancy precautions and risks of fetal exposure (refer to PPP).

**Exclusion Criteria.** The presence of any of the following will exclude a subject from enrollment:

1. Subjects with acute promyelocytic leukemia (APL)
2. Subjects with clinical symptoms suggesting active central nervous system (CNS) leukemia or known CNS leukemia. Evaluation of cerebrospinal fluid is only required if there is clinical suspicion of CNS involvement by leukemia during screening.
3. Subjects with immediately life-threatening, severe complications of leukemia such as disseminated/uncontrolled infection, uncontrolled bleeding, and/or uncontrolled disseminated intravascular coagulation.
4. Disorders or conditions disrupting normal calcium homeostasis or preventing calcium supplementation including:

- Any known condition disrupting calcium absorption.
- Clinical evidence of hypo- or hyperparathyroidism.
- Bisphosphonate or denosumab therapy within last 4 weeks prior to starting Compound 1.
- Active or recent kidney stones (≤ 1 year prior to starting Compound 1).
- Serum 25-hydroxyvitamin D level < 12 ng/mL (30 nmol/L).

5. Impaired cardiac function or clinically significant cardiac diseases, including any of the following:

- Left ventricular ejection fraction (LVEF) < 45% as determined by multiple gated acquisition (MUGA) scan or echocardiogram (ECHO).
- Complete left bundle branch or bifascicular block.
- Congenital long QT syndrome.
- Persistent or clinically meaningful ventricular arrhythmias.
- QTcF ≥ 470 msec on Screening electrocardiogram (ECG) (mean of triplicate recordings performed ≥ 72 hours prior to Day 1).
- Unstable angina pectoris or myocardial infarction ≤ 3 months prior to starting Compound 1.

6. Patients with prior autologous hematopoietic stem cell transplant who, in the investigator's judgment, have not fully recovered from the effects of the last transplant (eg, transplant related side effects).
7. Prior allogeneic hematopoietic stem cell transplant (HSCT) with either standard or reduced intensity conditioning ≤ 6 months prior to starting Compound 1.
8. Subjects on systemic immunosuppressive therapy post HSCT at the time of screening, or with clinically significant graft-versus-host disease (GVHD). The use of topical steroids for ongoing skin or ocular GVHD is permitted.
9. Prior systemic cancer-directed treatments or investigational modalities ≤ 5 half lives or 4 weeks prior to starting Compound 1, whichever is shorter. Hydroxyurea is allowed to control peripheral leukemia blasts.
10. Leukapheresis ≤ 2 weeks prior to starting Compound 1.
11. Major surgery ≤ 2 weeks prior to starting Compound 1. Subjects must have recovered from any clinically significant effects of recent surgery.
12. Pregnant or nursing females.
13. Known human immunodeficiency virus (HIV) infection.
14. Known chronic, active hepatitis B or C (HBV/HCV) infection.
15. Ongoing treatment with chronic, therapeutic dosing of anti-coagulants (eg, warfarin, low molecular weight heparin, Factor Xa inhibitors).
16. History of concurrent second cancers requiring active, ongoing systemic treatment.
17. Subject has a known allergy/hypersensitivity to calcium, calcitriol, and/or vitamin D supplements or any of their ingredients.
18. Subject has any significant medical condition, laboratory abnormality, or psychiatric illness that would prevent the subject from participating in the study.
19. Subject has any condition including the presence of laboratory abnormalities, which places the subject at unacceptable risk if he/she were to participate in the study.
20. Subject has any condition that confounds the ability to interpret data from the study.

**Example 40: A Phase 1, Open-Label, Dose-Finding Study Of Compound 1, A Novel Cereblon E3 Ligase Modulating Drug, In Subjects With Relapsed Or Refractory Acute Myeloid Leukemia Relapsed or Refractory Higher-Risk Myelodysplastic Syndromes**

**Primary Objectives:** To determine the safety and tolerability of Compound 1. To define the non-tolerated dose (NTD), the maximum tolerated dose (MTD) and/or the recommended Phase 2 dose (RP2D) of Compound 1.
**Secondary Objectives:** To provide information on the preliminary efficacy of Compound 1 in R/R AML and R/R HR-MDS. To characterize the pharmacokinetics (PK) of Compound 1 in plasma and urine.
**Study Design:** This study is an open-label, Phase 1, dose escalation and expansion, first in human clinical study of Compound 1 in subjects with relapsed or refractory AML or in subjects with relapsed or refractory higher-risk MDS. The dose escalation part (Part A) of the study will evaluate the safety and tolerability of escalating doses of Compound 1, administered intravenously, and determine the MTD of Compound 1. In Part A, two formulations will be tested. The expansion part (Part B) will further evaluate the safety and efficacy of Compound 1 administered at or below the MTD in selected expansion cohorts of up to approximately 20 evaluable subjects each in order to determine the RP2D. One or more dosing regimens may be selected for cohort expansion (at a minimum, one in R/R AML and one in R/R HR-MDS). MDS subjects will only be enrolled during Part B. Parts A and B will consist of 3 periods: Screening, Treatment, and Follow-up. Leukemia response will be determined by the Investigator. Disease assessment will be based on the International Working Group (IWG) Response Criteria in AML (Cheson, et al, J Clin Oncol 2003;21(24):4642-9). MDS response will be based on the IWG Response Criteria for Myelodysplasia (Cheson BD, et al., Blood. 2006; 108(2):419-25).
**Screening Period:** The Screening Period starts 28 days prior to first dose of Compound 1. The informed consent document must be signed and dated by the subject and the administering staff prior to the start of any other study procedures. All screening tests and procedures must be completed within the 28 days prior to the first dose of Compound 1. For Part B of the study, subjects will be assigned to either the R/R AML or the R/R HR-MDS cohort based

on a central laboratory diagnosis confirmation.

**Treatment Period:** In the Treatment Period, Compound 1 will be administered intravenously on Days 1-5 of each 28 day cycle for up to 4 cycles in the absence of disease progression, relapse, unacceptable toxicity, or subject/physician decision to withdraw. (Disease progression is defined as: a > 50% increase in bone marrow blast count percentage from the baseline (screening) bone marrow blast count that persists for at least 2 bone marrow assessments separated by at least 1 month, unless the baseline bone marrow blast count is > 70%, in which case, a finding of > 70% blasts that persists for 2 postbaseline bone marrow assessments separated by at least 1 month would be considered progression, or a doubling of the baseline absolute peripheral blood blast count that persists for ≥ 7 days and the final absolute peripheral blood blast count is > 10 × 109/L.) Modified dosing schedules (eg, increasing from 5 days to up to 10 days of dosing or increasing infusion length) may be evaluated in additional cohorts, if necessary, based on toxicity, PK profiles, and PD findings. An additional schedule of Compound 1 administered once daily on Days 1-3 and Days 8 10 of each 28 day cycle may be explored. Those who demonstrate benefit from treatment without unacceptable toxicity (complete remission [CR], partial remission [PR], or stable disease with discussion with Medical Monitor) may continue treatment beyond Cycle 4 until loss of that benefit, unacceptable toxicity, or subject/physician decision to withdraw. All subjects will be required to start calcium, calcitriol, and vitamin D supplementation at least 3 days prior to Day 1 of each cycle and continue until ≥ 3 days after the last dose of Compound 1 in each cycle (eg, ≥ Day 8 when Compound 1 is administered on Days 1-5, ≥ Day 13 when Compound 1 is administered on Days 1-3/Days 8-10).

In Cycle 1 of Part A, a bone marrow evaluation will be performed on Day 28 (± 3 days). Based on the Day 28 bone marrow evaluation, subjects with hypoplastic bone marrow, without evidence of persistent leukemia, who have Grade ≥ 3 neutropenia will be followed for an additional 2 weeks for safety monitoring in Cycle 1 (total duration of 42 days). An additional bone marrow assessment will be performed at the time of hematologic recovery or Day 42 (± 3 days). Thus, in Part A, the window for evaluation of dose-limiting toxicity (DLT) during Cycle 1 will be up to 42 days (28 or 42 days). In Part B, Cycle 1 will be 28 days in length. Cycles ≥ 2 will be 28 days in length. Subsequent cycles should start ≤ 7 days following the last day of the previous cycle.

**Follow-up Period:** In the Follow-up Period, all subjects will be followed for 28 days (± 3 days) after the last dose of Compound 1 for safety. Subjects without documented progression of disease (or relapse) will have efficacy evaluations of complete blood counts and peripheral blood smears performed every subsequent 8 weeks (± 1 week) for the 1st year and every 12 weeks (± 2 weeks) for the 2nd year or until progression of disease (or relapse), initiation of a new anticancer therapy, withdrawal of consent from the study, death, or the End of Trial, whichever comes first. A bone marrow evaluation will be completed at the end of the 1st year and as clinically indicated during the Follow-up Period.

All subjects will be followed for survival follow-up according to the schedule for the efficacy long term follow-up for up to 2 years or until death, lost to follow-up, or the End of Trial, whichever occurs first. Survival follow-up may be conducted by record review (including public records) and/or telephone contact with the subject, family, or the subject's treating physician.

**Part A-Dose Escalation.** During the escalation phase (Part A), a modified accelerated titration design (Simon et al, J Natl Cancer Inst 1997; 89(15):1138-47) will be used to establish initial toxicity. Cohorts of one or more subjects each will be administered Compound 1 at doses that will increase in 100% increments per cohort until ≥ 2 subjects experience a Compound 1-related Grade ≥ 2 adverse event in the DLT window (may be different cohorts), or ≥ 1 subject experiences a DLT within the DLT window. At that time the current cohort and all subsequent cohorts will be expanded enrolling 3 to 6 subjects. A dose escalation schedule with dose increments not to exceed 50% will concurrently be initiated in order to establish the NTD and MTD. The initial dose will be 0.3 mg. At the study start an initial formulation (Formulation A) will be utilized, and during dose escalation a second formulation (Formulation Ib described in Table 43) will be introduced to replace Formulation A. Formulation A has the following composition (*see* US Publication No. 2017/0196847-A1).

| | **Formulation A** |
|---|---|
| Compound 1 | 1.05 mg/vial |
| Citric acid anhydrous, USP | 18.6 mg/vial |
| Sodium citrate anhydrous, USP | 18.4 mg/vial |
| Kleptose® HPB (HP-β-CD), parenteral grade | 840 mg/vial |
| Dimethyl sulfoxide (in process media) | Partially removed upon drying |
| Formic acid | - |
| Water for injection (in process media) | Removed upon drying |

The DMA residual solvent in Formulation A must not exceed the permitted daily exposure (PDE) limits set in the ICH *Q3C Impurities: Residual Solvents* in order to proceed with dose escalation cohorts above a daily Compound 1 dose of 2.4 mg. The residual solvent (formic acid) level in Formulation Ib allows daily doses of Compound 1 of up to 20 mg without exceeding its PDE set in the ICH Q3C guidance.

The Formulation Ib will be introduced at a new dose cohort (in accordance with study dose escalation guidelines) after review of observed toxicities seen in the initial dose levels of Formulation A.

It may be decided to evaluate a higher dose cohort, additional subjects within a dose cohort, intermediate dose cohorts, smaller dose increments, alternate dosing schedules (eg, increasing from 5 to up to 10 days of Compound 1 administration or longer infusion times), and/or declare an MTD based on their review of available clinical and laboratory safety data, PK profiles, and PD findings. In the event that an alternate dosing schedule is evaluated, the starting dose and schedule will not exceed the dose intensity of a dose cohort that has previously met the criteria for dose escalation. An additional schedule of Compound 1 administered once daily on Days 1-3 and Days 8-10 of each 28 day schedule may be explored.

After the first dose is administered in any cohort during dose escalation, subjects in each cohort are observed for at least 28 days and up to 42 days (Cycle 1, DLT window) before the next higher, dose cohort can begin. No more than one subject per day will be enrolled in a given dose escalation cohort. A subject evaluable for DLT is defined as one that: has received at least 80% of the total planned Cycle 1 dose (eg, ≥ 4 Compound 1 doses for a 5-day dose schedule; in case of a missed dose, ≥ 4 doses to be completed on or before Day 10 or ≥ 5 doses by Day 14 for the D1-3/D8-10 schedule) of Compound 1 during Cycle 1 without experiencing a DLT, or experienced a DLT after receiving at least one dose (or fraction thereof) of Compound 1.

In the event that an alternate dose schedule (eg, increasing from 5 days to up to 10 days of dosing) is evaluated in Part A, the same criteria for determining DLT-evaluable subjects will be applied. Subjects non evaluable for DLT will be replaced.

A dose level (dose/schedule) will be considered intolerable if > 33% of evaluable subjects in a dose cohort experience DLT during Cycle 1. The MTD will be defined as the last dose below the NTD, at which ≤ 33% of evaluable subjects experienced DLT during Cycle 1. If 2 or more of 6 evaluable subjects experience DLTs in the first dose cohort, a lower dose cohort may be explored (ie, 0.1 mg Compound 1). An intermediate dose of Compound 1 (one between the NTD and the last dose level before the NTD) may be evaluated to accurately determine the MTD.

Intra-subject dose escalation will not be allowed during the DLT assessment period; however, in Cycles ≥ 2, subjects without evidence of disease progression who are tolerating their assigned dose of Compound 1 may escalate to the highest dose level shown to be adequately tolerated by at least one cohort of subjects in this study (ie, ≤ 33% of evaluable subjects having experienced a DLT at that dose level). If the highest tolerated dose level is Formulation Ib, a subject currently enrolled on Formulation A is permitted to switch to the newer formulation.

**Part B-Cohort Expansion:** Following completion of dose escalation (Part A), additional subjects may be enrolled into an expansion phase (Part B) with up to approximately 20 evaluable subjects in each cohort. Expansion may occur at the MTD and schedule established in the dose escalation phase, and/or at an alternative tolerable dose and schedule, based on review of safety, PK, and PD data from Part A. One or more dosing regimens (dose, schedules) may be selected for cohort expansion.

**Study Population:** Men and women, 18 years or older, with relapsed or refractory AML or relapsed or refractory higher-risk MDS as defined by World Health Organization (WHO) criteria, who are not suitable for other established therapies, will be enrolled in the study.

In Part A, only R/R AML subjects will be enrolled. In Part B, at least one cohort will include R/R AML subjects, including subjects who relapse after allogeneic HSCT, who are in second or later relapse, who are refractory to initial induction or re-induction treatment, who are refractory to or relapse after hypomethylating agent (HMA failure defined as primary progression or lack of clinical benefit after a minimum of 6 cycles or unable to tolerate HMA due to toxicity), or who relapse within 1 year of initial treatment (excluding those with favorable-risk status).

In Part B, at least one cohort of R/R HR-MDS subjects will be treated including subjects who score > 3.5 points in the Revised International Prognostic Scoring System (IPSS-R) [eg, IPSS-R intermediate risk (in combination with more than 10% bone marrow blasts or poor or very poor IPSS-R cytogenetic risk), IPSS-R high and IPSS-R very high risk] and are not suitable for other established therapies (eg, transplant or hypomethylating agent).

**Length of Study.** Enrollment is expected to take approximately 27 to 36 months to complete (18 to 24 months for dose escalation, and 9 to 12 months for expansion). Completion of active treatment and post treatment follow-up is expected to take an additional 6 to 24 months. The entire study is expected to last up to approximately 3 to 5 years. The End of Trial is defined as either the date of the last visit of the last subject to complete the post-treatment follow-up, or the date of receipt of the last data point from the last subject that is required for primary, secondary and/or exploratory analysis, as prespecified in the protocol, whichever is the later date.

**Study Treatments.** The investigational product, Compound 1 for IV injection, labeled appropriately for investigational use as per the regulations of the relevant country health authority will be provided. Study drug will be administered as outlined in the Treatment Period section above.

Study treatment may be discontinued if there is evidence of clinically significant disease progression (or relapse), unacceptable toxicity or subject/physician decision to withdraw. Subjects may continue to receive study drugs beyond disease progression at the discretion of the Investigator in consultation with the Celgene Medical Monitor.

**Overview of Key Efficacy Assessments.** The primary efficacy variable is response rate. All treated subjects will be included in the efficacy analyses. Leukemia response will be determined by the Investigator. Disease assessment will be based on the International Working Group (IWG) Response Criteria in AML (Cheson, et al, J Clin Oncol 2003;21(24):4642-9). Overall response will be determined using the IWG Response Criteria for Myelodysplasia for the HR-MDS cohort (Cheson BD, et al., Blood. 2006; 108(2):419-25).

A descriptive analysis of evidence of antileukemic activity will be provided based on clinical, laboratory, molecular, and cytogenetic assessments by Investigator, which includes assessment of bone marrow blast percentage, bone marrow cytogenetics, molecular genetic studies to evaluate molecular responses, bone marrow flow cytometry, platelet count, and absolute neutrophil count. AML response criteria will be summarized by best overall response categories: complete remission rate (CRR), and objective response rate (ORR). The ORR includes all responses of complete remission (CR) (ie, morphologic leukemia-free state, morphologic CR, cytogenetic CR, molecular CR, and morphologic CR with incomplete blood recovery), and partial remission. For MDS, the ORR includes all responses (CR, marrow complete remission mCR and PR).

The efficacy variable of focus will be ORR and CRR. Other measures of clinical activity including overall survival (OS), relapse free survival (RFS), progression-free survival (PFS), event-free survival, duration of remission, duration of response, time to transformation to AML (HR-MDS subjects only) and time to remission/response will be summarized.

**Overview of Key Safety Assessments.** The safety variables for this study include adverse events, safety clinical laboratory variables, 12-lead electrocardiograms, Eastern Cooperative Oncology Group Performance Status, left ventricular ejection fraction assessments, physical examinations, vital signs, exposure to study treatment, assessment of concomitant medications, and pregnancy testing for females of childbearing potential.

**Overview of Key Pharmacokinetic Assessments.** Key plasma PK parameters determined for Compound 1 will include maximum observed concentration (Cmax), area under the plasma concentration-time curve from time 0 to 24 hours postdose (AUC24), terminal-phase elimination half-life (t1/2), total plasma clearance (CL), time to peak (maximum) plasma concentration (tmax), volume of distribution at the steady state (Vss), percent dose excreted in urine as unchanged (Fe) and renal clearance (CLR). Selected PK parameters (eg, Cmax, AUC24, t1/2) will be estimated for R- and S-enantiomers of Compound 1 as appropriate. Key plasma and urine PK parameters as described above will also be estimated for HPBCD.

**Statistical Methods.** Statistical analyses will be performed by dose level (Part A) and cohort (Part B) as needed or applicable. All analyses will be descriptive in nature. All summaries of safety data will be conducted using subjects receiving any Compound 1 (the Treated Population).

The efficacy variables of primary interest are the ORR and CRR. Other preliminary efficacy variables including OS, RFS, PFS, event-free survival, duration of remission, duration of response, and time to remission/response will be summarized. Efficacy analysis will be repeated for the Treated Population and Efficacy Evaluable Population (received a baseline leukemia assessment evaluation, at least one cycle of study treatment or at least 80% of scheduled doses in Cycle 1, and one on-study leukemia assessment evaluation), with the result using the Treated Population considered primary.

All biomarker-related data presentations will be based on treated subjects with at least one biomarker assessment, unless specified otherwise. Descriptive statistics will be presented for baseline and change from baseline of continuous biomarker endpoints, by dosing regimens and/or disease subsets, and overall.

The study will be conducted in compliance with International Council for Harmonisation of Technical Requirements for Pharmaceuticals for Human Use (ICH)/Good Clinical Practice and applicable regulatory requirements.

**Inclusion Criteria.** Subjects must satisfy the criteria below to be enrolled in dose escalation (Part A) or dose expansion (Part B) of this study.

1. Men and women ≥ 18 years of age, at the time of signing the ICD.
2. Subject must understand and voluntarily sign an ICD prior to any study-related assessments/procedures being conducted.
3. Subject is willing and able to adhere to the study visit schedule and other protocol requirements.
4. Relapsed or refractory AML (Parts A and B) or R/R HR-MDS (Part B only) as defined by World Health Organization (WHO) criteria who are not suitable for other established therapies.

    a. In Part A, R/R AML
    b. In Part B, R/R AML including

- Relapsed after allogeneic HSCT or
- In second or later relapse or
- Refractory to initial induction or re-induction treatment or
- Refractory or relapse after HMA treatment (HMA failure defined as primary progression or lack of clinical benefit after a minimum of 6 cycles or unable to tolerate HMA due to toxicity) or
- Relapsed within 1 year of initial treatment (excluding those with favorable risk based on cytogenetics)

c. In Part B, R/R HR-MDS (IPSS-R > 3.5 points):

- IPSS-R intermediate risk (in combination with more than 10% bone marrow blasts or poor or very poor IPSS-R cytogenetic risk) or
- IPSS-R high or
- IPSS-R very high risk.

5. Eastern Cooperative Oncology Group Performance Status (ECOG PS) of 0 to 2.
6. At least 4 weeks (from first dose) has elapsed from donor lymphocyte infusion (DLI) without conditioning.
7. Subjects must have the following screening laboratory values:

- Corrected serum Ca or free (ionized) serum Ca within normal limits (WNL).

    ◦

    ◦ Corrected Ca (mg/dL) = Total Ca (mg/dL) – 0.8 (albumin [g/dL] – 4)

- Total White Blood Cell count (WBC) < 25 × $10^9$/L prior to first infusion. Prior or concurrent treatment with hydroxyurea to achieve this level is allowed.
- Potassium and magnesium within normal limits or correctable with supplements.
- Aspartate aminotransferase/serum glutamic oxaloacetic transaminase (AST/SGOT) or alanine aminotransferase/serum glutamate pyruvic transaminase (ALT/SGPT) ≤ 2.5 × Upper Limit of Normal (ULN).
- Uric acid ≤ 7.5 mg/dL (446 μmol/L). Prior and/or concurrent treatment with hypouricemic agents (eg, allopurinol, rasburicase) are allowed.
- Serum bilirubin ≤ 1.5 × ULN.
- Estimated serum creatinine clearance of ≥ 60 mL/min using the Cockcroft-Gault equation. Measured creatinine clearance from a 24-hour urine collection is acceptable if clinically indicated.
- INR < 1.5 × ULN and PTT < 1.5 × ULN.

8. Per the Compound 1 Pregnancy Prevention Plan (PPP):

- Females of childbearing potential (FCBP) must undergo pregnancy testing based on the frequency outlined in PPP and pregnancy results must be negative.
- Unless practicing complete abstinence from heterosexual intercourse, sexually active FCBP must agree to use adequate contraceptive methods as specified in PPP.

    ◦ FCBP must agree to use two reliable forms of contraception simultaneously (or to practice complete abstinence), without interruption, for 28 days before starting Compound 1, throughout the entire duration of Compound 1 treatment, during dose interruptions and for at least 28 days after the last dose of Compound 1.
    ◦ Complete abstinence is only acceptable in cases where this is the preferred and usual lifestyle of the subject.
    ◦ Periodic abstinence (calendar ovulation, symptothermal, post-ovulation methods) and withdrawal are not acceptable.

- Unless practicing complete abstinence from heterosexual intercourse, sexually active males (including those who have had a vasectomy) must use barrier contraception (condoms) when engaging in sexual activity with FCBP as specified in PPP.

    ◦ Complete abstinence is only acceptable in cases where this is the preferred and usual lifestyle of the subject.
    ◦ Male patients must inform their partners who are females of childbearing potential to use two methods of

reliable contraception throughout the entire duration of treatment, during dose interruptions and for at least 90 days after the last dose of Compound 1, as specified in PPP.

- Females must agree to abstain from breastfeeding or providing breast milk for the duration specified in the PPP.
- Males must agree not to donate semen or sperm while receiving Compound 1, during dose interruptions or for at least 90 days following the last dose of Compound 1, as specified in the PPP.
- All subjects must:

    - Understand that Compound 1 could have a potential teratogenic risk.
    - Agree to abstain from donating blood for the duration specified in the PPP.
    - Be counseled about pregnancy precautions and risks of fetal exposure (refer to PPP).

**Exclusion Criteria.**
The presence of any of the following will exclude a subject from enrollment:

1. Subjects with acute promyelocytic leukemia (APL)
2. Subjects with clinical symptoms suggesting active central nervous system (CNS) leukemia or known CNS leukemia. Evaluation of cerebrospinal fluid is only required if there is clinical suspicion of CNS involvement by leukemia during screening.
3. Subjects with immediately life-threatening, severe complications of leukemia such as disseminated/uncontrolled infection, uncontrolled bleeding, and/or uncontrolled disseminated intravascular coagulation.
4. Disorders or conditions disrupting normal calcium homeostasis or preventing calcium supplementation including:

- Any known condition disrupting calcium absorption.
- Clinical evidence of hypo- or hyperparathyroidism.
- Bisphosphonate or denosumab therapy within last 4 weeks prior to starting Compound 1.
- Active or recent kidney stones (≤ 1 year prior to starting Compound 1).
- Serum 25-hydroxyvitamin D level < 12 ng/mL (30 nmol/L).

5. Impaired cardiac function or clinically significant cardiac diseases, including any of the following:

- Left ventricular ejection fraction (LVEF) < 45% as determined by multiple gated acquisition (MUGA) scan or echocardiogram (ECHO).
- Complete left bundle branch or bifascicular block.
- Congenital long QT syndrome.
- Persistent or clinically meaningful ventricular arrhythmias.
- QTcF ≥ 470 msec on Screening electrocardiogram (ECG) (mean of triplicate recordings performed ≥ 72 hours prior to Day 1).
- Unstable angina pectoris or myocardial infarction ≤ 3 months prior to starting Compound 1.

6. Patients with prior autologous hematopoietic stem cell transplant who, in the investigator's judgment, have not fully recovered from the effects of the last transplant (eg, transplant related side effects).
7. Prior allogeneic hematopoietic stem cell transplant (HSCT) with either standard or reduced intensity conditioning ≤ 6 months prior to starting Compound 1.
8. Subjects on systemic immunosuppressive therapy post HSCT at the time of screening, or with clinically significant graft-versus-host disease (GVHD). The use of topical steroids for ongoing skin or ocular GVHD is permitted.
9. Prior systemic cancer-directed treatments or investigational modalities ≤ 5 half lives or 4 weeks prior to starting Compound 1, whichever is shorter. Hydroxyurea is allowed to control peripheral leukemia blasts.
10. Leukapheresis ≤ 2 weeks prior to starting Compound 1.
11. Major surgery ≤ 2 weeks prior to starting Compound 1. Subjects must have recovered from any clinically significant effects of recent surgery.
12. Pregnant or nursing females.
13. Known human immunodeficiency virus (HIV) infection.
14. Known chronic, active hepatitis B or C (HBV/HCV) infection.
15. Ongoing treatment with chronic, therapeutic dosing of anti-coagulants (eg, warfarin, low molecular weight heparin, Factor Xa inhibitors).
16. History of concurrent second cancers requiring active, ongoing systemic treatment.
17. Subject has a known allergy/hypersensitivity to calcium, calcitriol, and/or vitamin D supplements or any of their

ingredients.
18. Subject has any significant medical condition, laboratory abnormality, or psychiatric illness that would prevent the subject from participating in the study.
19. Subject has any condition including the presence of laboratory abnormalities, which places the subject at unacceptable risk if he/she were to participate in the study.
20. Subject has any condition that confounds the ability to interpret data from the study.

## Claims

1. A formulation comprising: (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof in an amount of about 0.01 to about 0.15%, and hydroxypropyl β-cyclodextrin or sulfobutyl ether-beta-cyclodextrin in an amount of about 99.1 to about 99.99%, based on the total weight of the formulation, wherein the term "about" contemplates weight percent within 10%, or 5% of the weight percent which is encompassed.

2. The formulation of claim 1 comprising: (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof in an amount of about 0.08 to about 0.15%, and hydroxypropyl β-cyclodextrin or sulfobutyl ether-beta-cyclodextrin in an amount of about 99.1 to about 99.9%, based on the total weight of the formulation.

3. The formulation of claim 1, comprising (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof in the amount from about 0.1 to about 0.13%, or wherein (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof is present in the amount of about 0.12% based on the total weight of the formulation.

4. The formulation of claim 1 comprising: (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof in an amount of about 0.01 to about 0.08%, and hydroxypropyl β-cyclodextrin in an amount of about 99.40 to about 99.99%.

5. The formulation of claim 1, comprising (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof from about 0.01 to about 0.08%, hydroxypropyl β-cyclodextrin from about 99.40% to about 99.99%, and formic acid from about 0.1 to about 0.3% based on total weight of the formulation.

6. The formulation of any one of claims 1 to 5 further comprising formic acid in an amount of no more than about 0.5% based on the total weight of the composition

7. The formulation of any one of claims 1 to 5, comprising a solid form of (2-(4-chlorophenyl)-N-((2-(2,6-dioxopiperidin-3-yl)-1-oxoisoindolin-5-yl)methyl)-2,2-difluoroacetamide) or an amorphous form of (2-(4-chlorophenyl)-N-((2-(2,6-dioxopiperidin-3-yl)-1-oxoisoindolin-5-yl)methyl)-2,2-difluoroacetamide).

8. An aqueous formulation comprising the formulation of any one of claims 1 to 5 and a diluent, optionally wherein the diluent is water or ½ normal saline, or wherein the diluent is normal saline.

9. The aqueous formulation of claim 8, comprising (2-(4-chlorophenyl)-N-((2-(2,6-dioxopiperidin-3-yl)-1-oxoisoindolin-5-yl)methyl)-2,2-difluoroacetamide), or a stereoisomer or mixture of stereoisomers, pharmaceutically acceptable salt, tautomer, solvate, hydrate, co-crystal, clathrate, or polymorph thereof in an amount of about 0.1 to 0.3 mg/mL.

10. The aqueous formulation of claim 8, wherein the aqueous solution has a pH in a range from about 3.0 to about 3.6, or wherein the aqueous solution has a pH in a range from about 4.2 to about 4.4.

11. The aqueous formulation of claim 8, wherein the aqueous solution has an osmolality of about 260-280 mOsm/kg or wherein the aqueous solution has an osmolality of about 310-380 mOsm/kg.

12. The formulation of any one of claims 1 to 5 or the aqueous formulation of claim 8 for use in a method of treating a cancer in a mammal.

13. The aqueous formulation for use of claim 12, wherein the method comprises administering the aqueous formulation intravenously.

14. The formulation for use of claim 12 or the aqueous formulation for use of claim 12 or 13, wherein the cancer is leukemia, optionally the leukemia is chronic lymphocytic leukemia, chronic myelocytic leukemia, acute lymphoblastic leukemia or acute myeloid leukemia.

15. The formulation for use of claim 12, or the aqueous formulation for use of claim 12 or 13, further comprising administering a therapeutically effective amount of another second active agent or a supportive care therapy, optionally wherein the other second active agent is a therapeutic antibody that specifically binds to a cancer antigen, hematopoietic growth factor, cytokine, anti-cancer agent, antibiotic, cox-2 inhibitor, immunomodulatory agent, immunosuppressive agent, corticosteroid or a pharmacologically active mutant or derivative thereof.

16. A process for preparing the formulation of any one of claims 1 to 5 comprising: dissolving (2-(4-chlorophenyl)-N-((2-(2,6-dioxopiperidin-3-yl)-1-oxoisoindolin-5-yl)methyl)-2,2-difluoroacetamide) in formic acid to obtain a premix, dissolving hydroxypropyl β-cyclodextrin in water to obtain a solution, adding the premix to the solution to obtain a drug solution.

17. The process of claim 16 further comprising lyophilizing the solution to produce a lyophilized formulation.

## Patentansprüche

1. Formulierung, umfassend: (2-(4-Chlorphenyl)-N-((2-(2,6-dioxopiperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in einer Menge von etwa 0,01 bis etwa 0,15% und Hydroxypropyl-β-cyclodextrin oder Sulfobutylether-beta-cyclodextrin in einer Menge von etwa 99,1 bis etwa 99,99%, bezogen auf das Gesamtgewicht der Formulierung, wobei der Begriff "etwa" eine Gewichtsprozentangabe innerhalb von 10% oder 5% der Gewichtsprozentangabe, die umfasst ist, betrachtet.

2. Formulierung nach Anspruch 1, umfassend: (2-(4-Chlorphenyl)-N-((2-(2,6-dioxo-piperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in einer Menge von etwa 0,08 bis etwa 0,15% und Hydroxypropyl-β-cyclodextrin oder Sulfobutylether-beta-cyclodextrin in einer Menge von etwa 99,1 bis etwa 99,9%, bezogen auf das Gesamtgewicht der Formulierung.

3. Formulierung nach Anspruch 1, umfassend (2-(4-Chlorphenyl)-N-((2-(2,6-dioxo-piperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in der Menge von etwa 0,1 bis etwa 0,13%, oder wobei (2-(4-Chlorphenyl)-N-((2-(2,6-dioxo-piperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in der Menge von etwa 0,12% bezogen auf das Gesamtgewicht der Formulierung vorliegt.

4. Formulierung nach Anspruch 1, umfassend: (2-(4-Chlorhenyl)-N-((2-(2,6-dioxopiperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in einer Menge von etwa 0,01 bis etwa 0,08% und Hydroxypropyl-β-cyclodextrin in einer Menge von etwa 99,40 bis etwa 99,99%.

5. Formulierung nach Anspruch 1, umfassend: (2-(4-Chlorphenyl)-N-((2-(2,6-dioxopiperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon von etwa 0,01 bis etwa 0,08%, Hydroxypropyl-β-cyclodextrin von etwa 99,40 bis etwa 99,99% und Ameisensäure von etwa 0,1 bis etwa 0,3% bezogen auf Gesamtgewicht der Formulierung.

**6.** Formulierung nach einem der Ansprüche 1 bis 5, ferner umfassend Ameisensäure in einer Menge von nicht mehr als etwa 0,5% bezogen auf das Gesamtgewicht der Zusammensetzung.

**7.** Formulierung nach einem der Ansprüche 1 bis 5, umfassend eine Feststoffform von (2-(4-Chlorphenyl)-N-((2-(2,6-dioxopiperidin-3-yl)-1-oxoisoindolin-5-yl)methyl)-2,2-difluor-acetamid) oder eine amorphe Form von (2-(4-Chlorphenyl)-N-((2-(2,6-dioxopiperidin-3-yl)-1-oxoisoindolin-5-yl)methyl)-2,2-difluoracetamid).

**8.** Wässrige Formulierung, umfassend die Formulierung nach einem der Ansprüche 1 bis 5 und ein Verdünnungsmittel, optional wobei es sich bei dem Verdünnungsmittel um Wasser oder ½-normale Salzlösung handelt oder wobei es sich bei dem Verdünnungsmittel um normale Salzlösung handelt.

**9.** Wässrige Formulierung nach Anspruch 8, umfassend (2-(4-Chlorphenyl)-N-((2-(2,6-dioxopiperidin-3-yl)-1-oxoisoindolin-5-yl)methyl)-2,2-difluoracetamid) oder ein Stereoisomer oder Gemisch von Stereoisomeren, ein pharmazeutisch verträgliches Salz, ein Tautomer, ein Solvat, ein Hydrat, einen Co-Kristall, ein Clathrat oder ein Polymorph davon in einer Menge von etwa 0,1 bis 0,3 mg/ml.

**10.** Wässrige Formulierung nach Anspruch 8, wobei die wässrige Lösung einen pH in einem Bereich von etwa 3,0 bis etwa 3,6 aufweist oder wobei die wässrige Lösung einen pH in einem Bereich von etwa 4,2 bis etwa 4,4 aufweist.

**11.** Wässrige Formulierung nach Anspruch 8, wobei die wässrige Lösung eine Osmolalität von etwa 260-280 mOsm/kg aufweist oder wobei die wässrige Lösung eine Osmolalität von etwa 310-380 mOsm/kg aufweist.

**12.** Formulierung nach einem der Ansprüche 1 bis 5 oder wässrige Formulierung nach Anspruch 8 zur Verwendung bei einem Verfahren zur Behandlung einer Krebserkrankung bei einem Säuger.

**13.** Wässrige Formulierung zur Verwendung nach Anspruch 12, wobei das Verfahren intravenöses Verabreichen der wässrigen Formulierung umfasst.

**14.** Formulierung zur Verwendung nach Anspruch 12 oder wässrige Formulierung zur Verwendung nach Anspruch 12 oder 13, wobei es sich bei der Krebserkrankung um Leukämie, optional bei der Leukämie um chronische lymphatische Leukämie, chronische myeloische Leukämie, akute lymphatische Leukämie oder akute myeloische Leukämie handelt.

**15.** Formulierung zur Verwendung nach Anspruch 12 oder wässrige Formulierung zur Verwendung nach Anspruch 12 oder 13, ferner umfassend Verabreichen einer therapeutisch wirksamen Menge eines weiteren, zweiten Wirkstoffs oder einer unterstützenden Pflegetherapie, optional wobei es sich bei dem weiteren, zweiten Wirkstoff um einen therapeutischen Antikörper, der an ein Krebsantigen spezifisch bindet, einen hämatopoetischen Wachstumsfaktor, ein Cytokin, ein krebshemmendes Mittel, ein Antibiotikum, einen Cox-2-Inhibitor, einen Immunmodulator, ein Immunsuppressum, ein Corticosteroid oder eine pharmakologisch aktive Mutante oder ein Derivat davon handelt.

**16.** Verfahren zur Herstellung der Formulierung nach einem der Ansprüche 1 bis 5, umfassend: Lösen von (2-(4-Chlorphenyl)-N-((2-(2,6-dioxopiperidin-3-yl)-1-oxoisoindolin-5-yl)methyl)-2,2-difluoracetamid) in Ameisensäure unter Erhalt eines Vorgemischs, Lösen von Hydroxypropyl-β-cyclodextrin in Wasser unter Erhalt einer Lösung, Zugeben des Vorgemischs zur Lösung unter Erhalt einer Arzneistofflösung.

**17.** Verfahren nach Anspruch 16, ferner umfassend Lyophilisieren der Lösung zur Erzeugung einer lyophilisierten Formulierung.

## Revendications

**1.** Formulation comprenant du (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant en une quantité d'environ 0,01 à environ 0,15 %, et de l'hydroxypropyl-β-cyclodextrine ou de l'éther de sulfobutyle de bêtacyclodextrine en une quantité d'environ 99,1 à environ 99,99 %, sur la base du poids total de la formulation, le terme « environ » se rapportant à un pourcentage en poids dans les 10 %, ou 5 % du pourcentage en poids qui est englobé.

**2.** Formulation selon la revendication 1, comprenant : du (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant en une quantité d'environ 0,08 à environ 0,15 %, et de l'hydroxypropyl-β-cyclodextrine ou de l'éther de sulfobutyle de bêta-cyclodextrine en une quantité d'environ 99,1 à environ 99,9 %, sur la base du poids total de la formulation.

**3.** Formulation selon la revendication 1, comprenant du (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant en la quantité d'environ 0,1 à environ 0,13 %, ou le (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant étant présent en la quantité d'environ 0,12 % sur la base du poids total de la formulation.

**4.** Formulation selon la revendication 1, comprenant : du (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant en une quantité d'environ 0,01 à environ 0,08 %, et de l'hydroxypropyl-β-cyclodextrine en une quantité d'environ 99,40 à environ 99,99 %.

**5.** Formulation selon la revendication 1, comprenant du (2-(4-chlorophényl)-N-((2-(2,6-dioxopipéridin-3-yl)-1-oxoisoindolin-5-yl)méthyl)-2,2-difluoroacétamide), ou un sel, une forme tautomère, un solvate, un hydrate, un cocristal, un clathrate ou un polymorphe pharmaceutiquement acceptable correspondant(e), ou un stéréoisomère ou mélange de stéréoisomères correspondant d'environ 0,01 à environ 0,08 %, de l'hydroxypropyl-β-cyclodextrine d'environ 99,40 % à environ 99,99 %, et de l'acide formique d'environ 0,1 à environ 0,3 % sur la base du poids total de la formulation.

**6.** Formulation selon l'une quelconque des revendications 1 à 5, comprenant en outre de l'acide formique en une quantité non supérieure à environ 0,5 % sur la base du poids total de la composition.

**7.** Formulation selon l'une quelconque des revendications 1 à 5, comprenant une forme solide du (2-(4-chlorophényl)-N-((2-(2,6-dioxopipéridin-3-yl)-1-oxoisoindolin-5-yl)méthyl)-2,2-difluoroacétamide) ou une forme amorphe du (2-(4-chlorophényl)-N-((2-(2,6-dioxopipéridin-3-yl)-1-oxoisoindolin-5-yl)méthyl)-2,2-difluoroacétamide).

**8.** Formulation aqueuse comprenant la formulation selon l'une quelconque des revendications 1 à 5 et un diluant, éventuellement, le diluant étant de l'eau ou une solution saline ½ normale, ou le diluant étant une solution saline normale.

**9.** Formulation aqueuse selon la revendication 8, comprenant du (2-(4-chlorophényl)-N-((2-(2,6-dioxopipéridin-3-yl)-1-oxoisoindolin-5-yl)méthyl)-2,2-difluoroacétamide) ou un sel, une forme tautomère, un solvate, un hydrate, un cocristal, un clathrate ou un polymorphe pharmaceutiquement acceptable correspondant(e) ou un stéréoisomère ou mélange de stéréoisomères correspondant en une quantité d'environ 0,1 à 0,3 mg/mL.

**10.** Formulation aqueuse selon la revendication 8, la solution aqueuse possédant un pH dans une plage d'environ 3,0 à environ 3,6, ou la solution aqueuse possédant un pH dans une plage d'environ 4,2 à environ 4,4.

**11.** Formulation aqueuse selon la revendication 8, la solution aqueuse possédant une osmolalité d'environ 260 à 280 mOsm/kg ou la solution aqueuse possédant une osmolalité d'environ 310 à 380 mOsm/kg.

**12.** Formulation selon l'une quelconque des revendications 1 à 5 ou formulation aqueuse selon la revendication 8 pour une utilisation dans une méthode de traitement d'un cancer chez un mammifère.

**13.** Formulation aqueuse pour une utilisation selon la revendication 12, une méthode comprenant l'administration de la formulation aqueuse de manière intraveineuse.

**14.** Formulation pour une utilisation selon la revendication 12 ou formulation aqueuse pour une utilisation selon la revendication 12 ou 13, le cancer étant une leucémie, éventuellement la leucémie étant une leucémie lymphocytaire chronique, une leucémie myélocytaire chronique, une leucémie lymphoblastique aiguë ou une leucémie myéloïde aiguë.

**15.** Formulation pour une utilisation selon la revendication 12 ou formulation aqueuse pour une utilisation selon la revendication 12 ou 13, comprenant en outre l'administration d'une quantité thérapeutiquement efficace d'un autre deuxième agent actif ou d'une thérapie de soins de soutien, éventuellement, l'autre deuxième agent actif étant un anticorps thérapeutique qui se lie spécifiquement à un antigène de cancer, à un facteur de croissance hématopoïétique, à une cytokine, à un agent anticancéreux, à un antibiotique, à un inhibiteur de cox-2, à un agent immunomodulateur, à un agent immunosuppresseur, à un corticostéroïde ou à un mutant ou dérivé pharmacologiquement actif correspondant.

**16.** Procédé pour la préparation de la formulation selon l'une quelconque des revendications 1 à 5 comprenant : la dissolution de (2-(4-chlorophényl)-N-((2-(2,6-dioxopipéridin-3-yl)-1-oxoisoindolin-5-yl)méthyl)-2,2-difluoroacétamide) dans de l'acide formique pour obtenir un prémélange, la dissolution d'hydroxypropyl-β-cyclodextrine dans de l'eau pour obtenir une solution, l'ajout du prémélange à la solution pour obtenir une solution de médicament.

**17.** Procédé selon la revendication 16 comprenant en outre la lyophilisation de la solution pour produire une formulation lyophilisée.

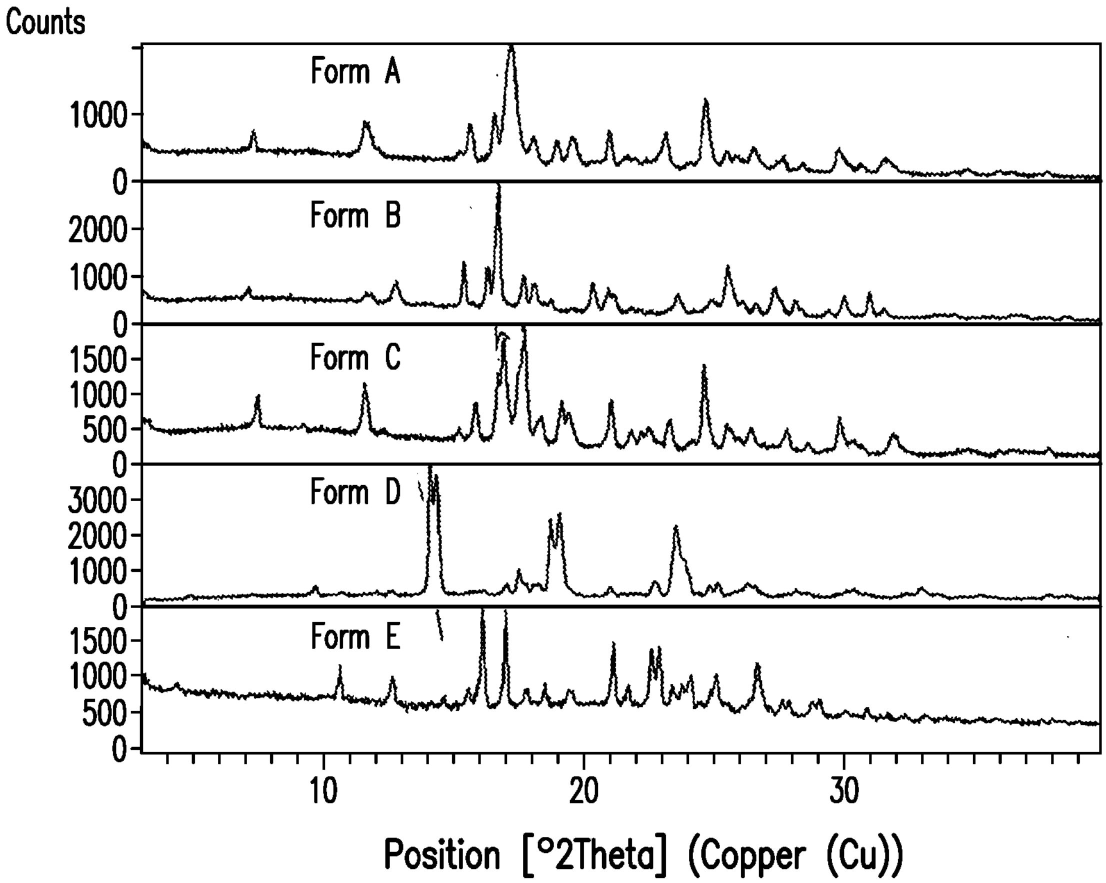
Counts
Form A
Form B
Form C
Form D
Form E
1000
0
2000
1000
0
1500
1000
500
0
3000
2000
1000
0
1500
1000
500
0
10
20
30
Position [°2Theta] (Copper (Cu))

FIG. 1

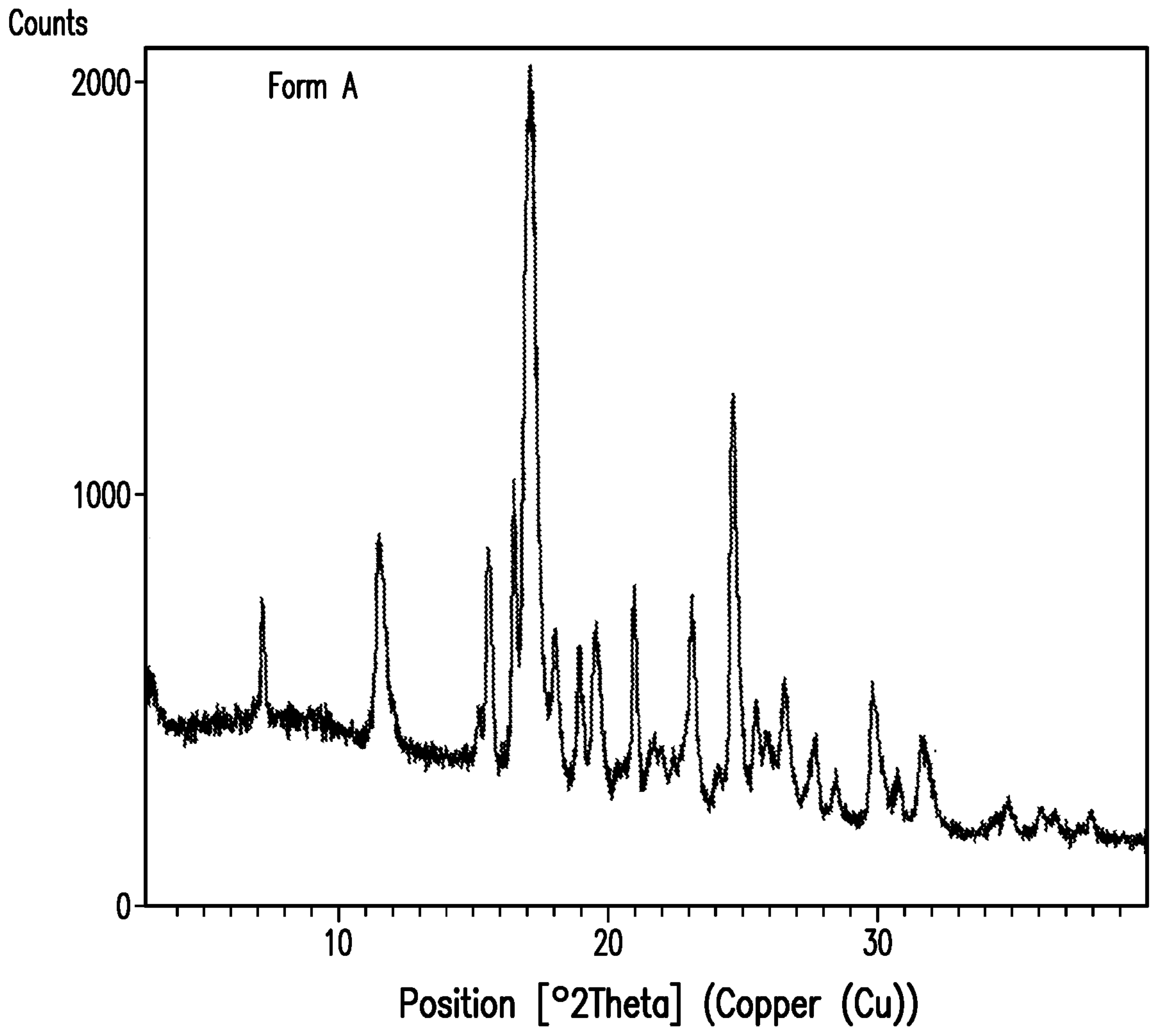


FIG. 2

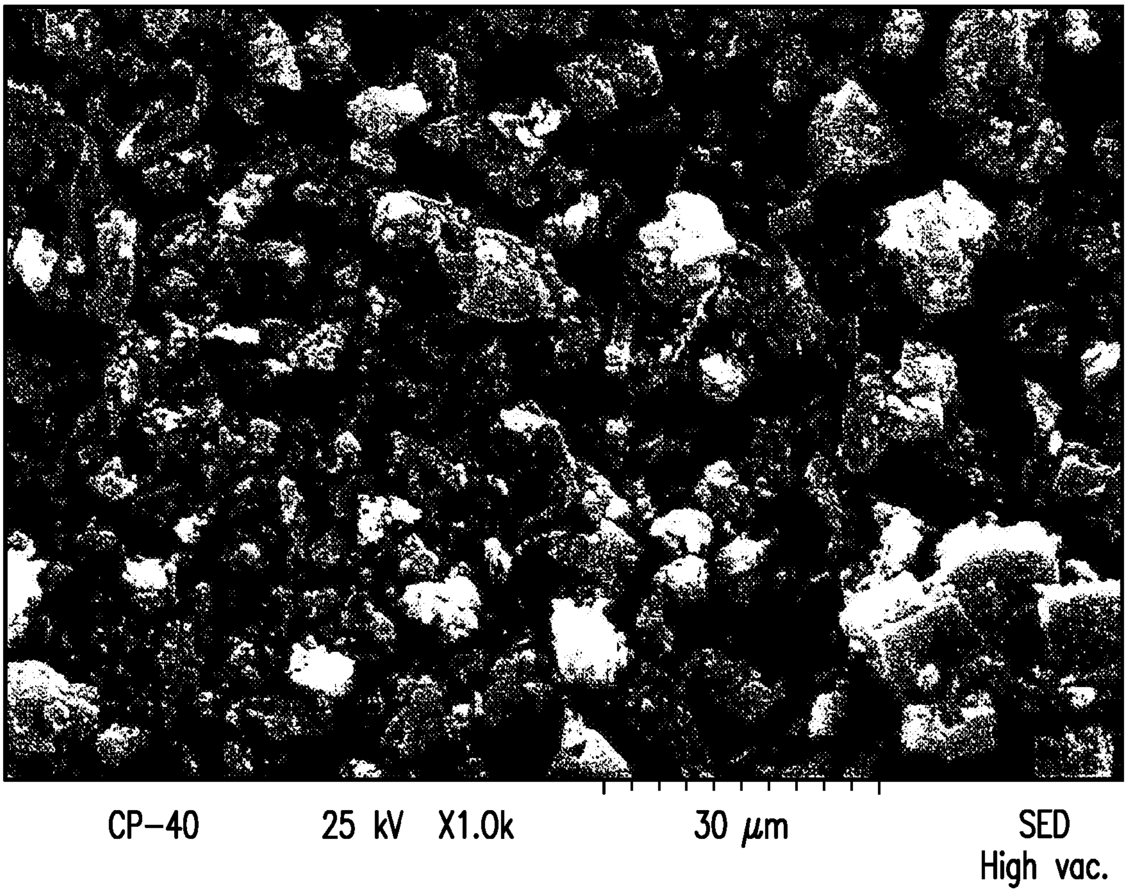


FIG. 3

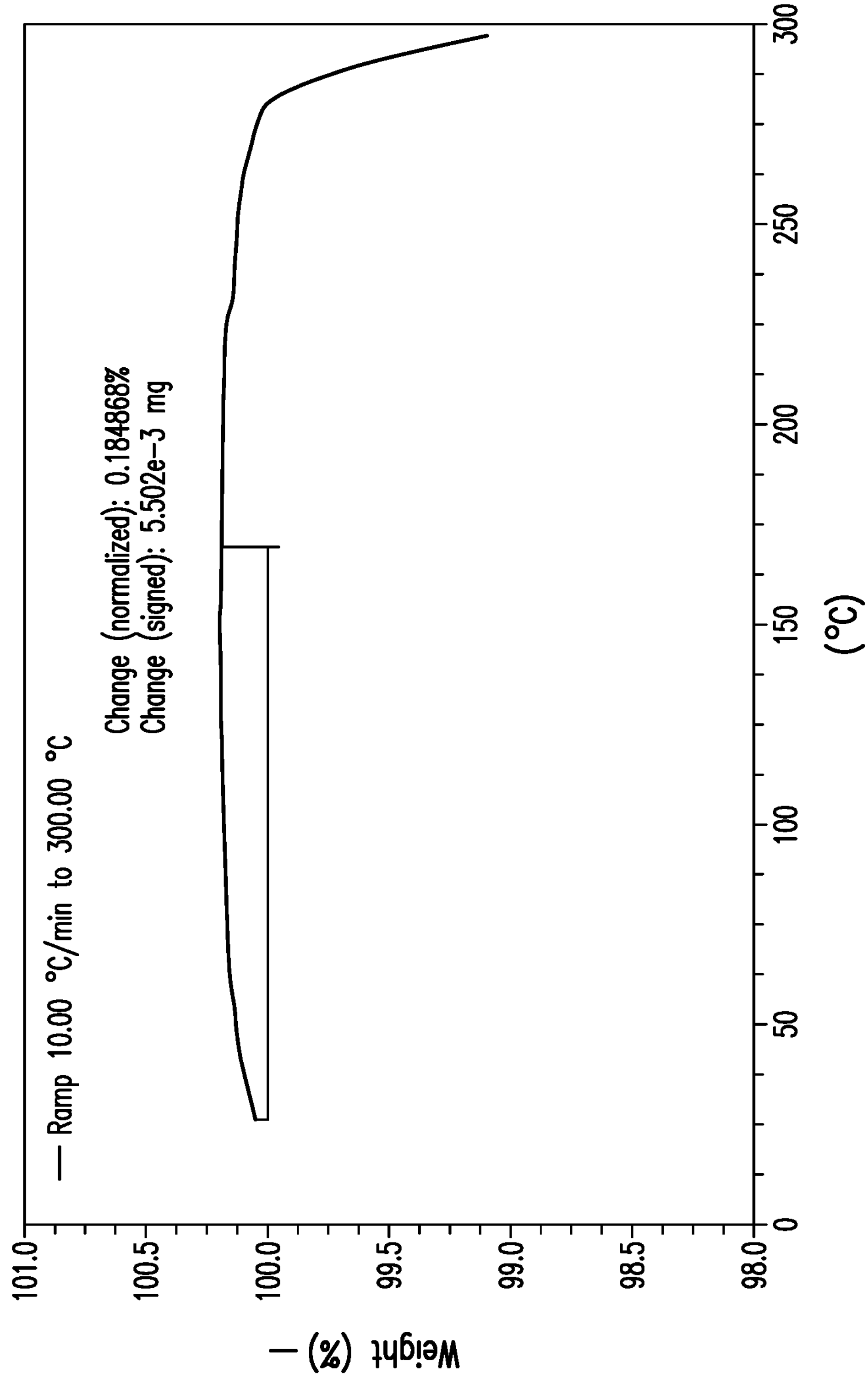
Ramp 10.00 °C/min to 300.00 °C
Change (normalized): 0.184868%
Change (signed): 5.502e−3 mg
Weight (%)
101.0
100.5
100.0
99.5
99.0
98.5
98.0
0
50
100
150
200
250
300
(°C)

FIG. 4

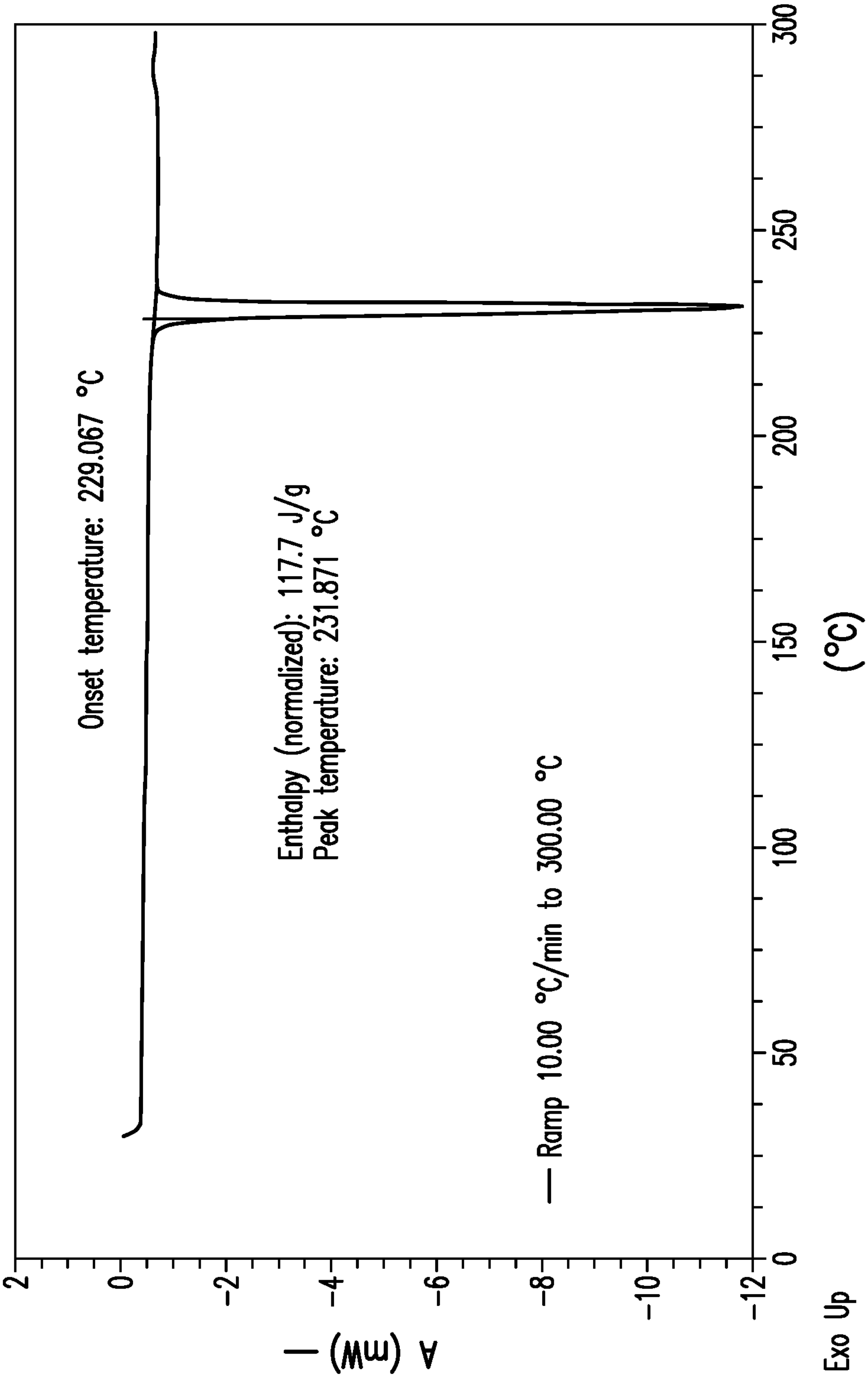
Onset temperature: 229.067 °C
Enthalpy (normalized): 117.7 J/g
Peak temperature: 231.871 °C
— Ramp 10.00 °C/min to 300.00 °C
(mW)
Exo Up
(°C)
0
50
100
150
200
250
300
2
0
−2
−4
−6
−8
−10
−12

FIG. 5

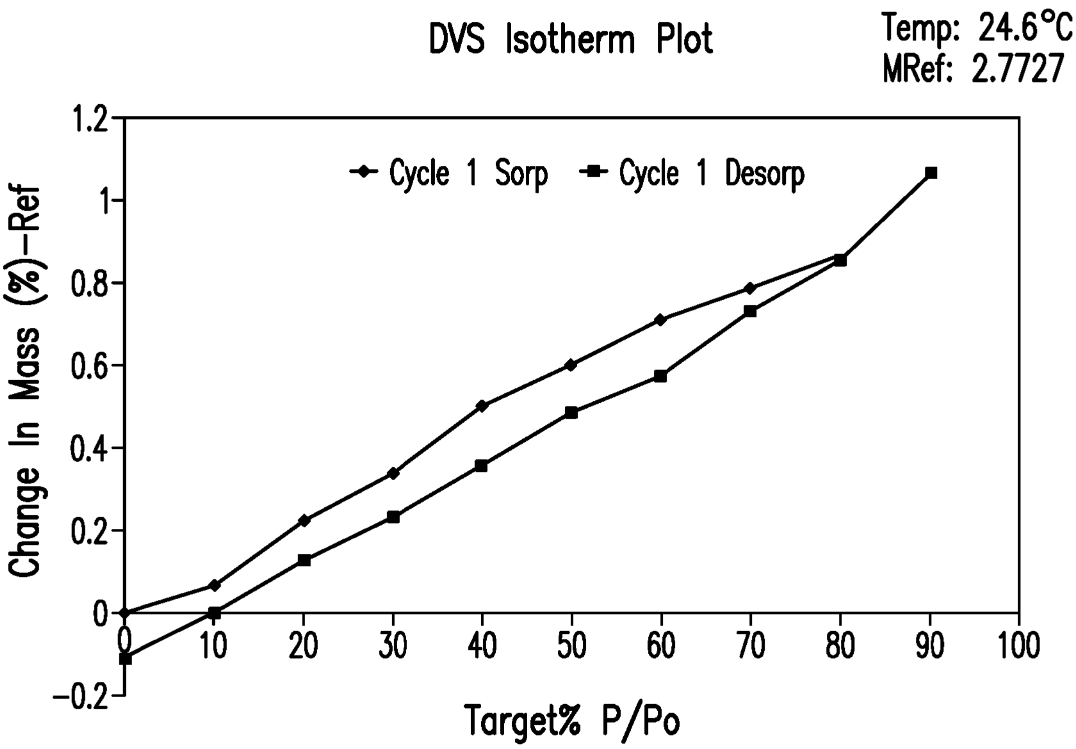


| | Target % P/Po | Change In Mass (%)−ref Sorption | Desorption | Hysteresis |
|---|---|---|---|---|
| Cycle 1 | 0.0 | −0.004 | −0.111 | |
| | 10.0 | 0.063 | −0.002 | −0.066 |
| | 20.0 | 0.221 | 0.126 | −0.094 |
| | 30.0 | 0.339 | 0.231 | −0.108 |
| | 40.0 | 0.509 | 0.360 | −0.149 |
| | 50.0 | 0.614 | 0.496 | −0.118 |
| | 60.0 | 0.726 | 0.586 | −0.139 |
| | 70.0 | 0.808 | 0.751 | −0.057 |
| | 80.0 | 0.887 | 0.880 | −0.007 |
| | 90.0 | 1.099 | 1.099 | |

FIG. 6

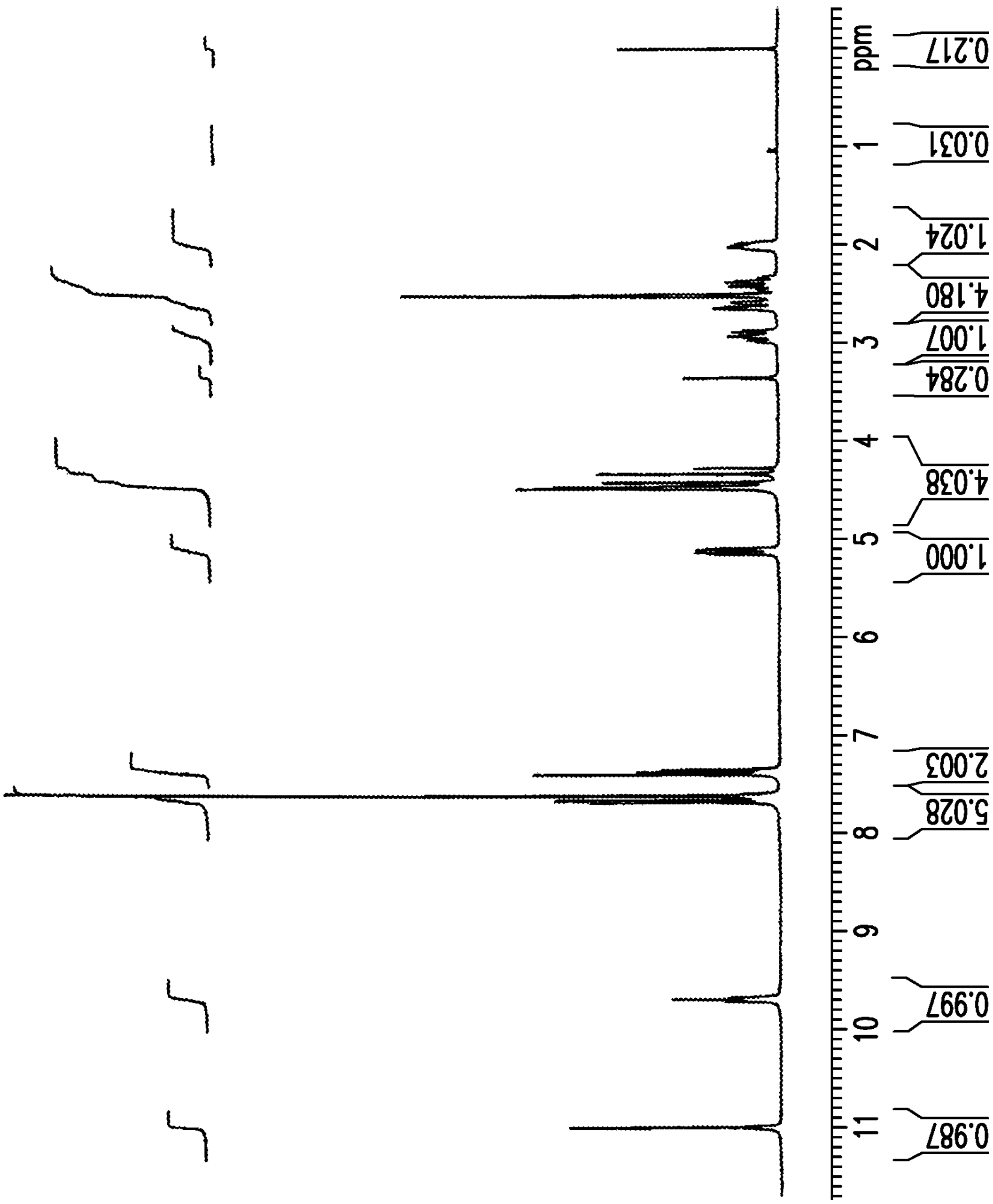
ppm
0.217
0.031
1.024
4.180
1.007
0.284
4.038
1.000
2.003
5.028
0.997
0.987
1
2
3
4
5
6
7
8
9
10
11

FIG. 7

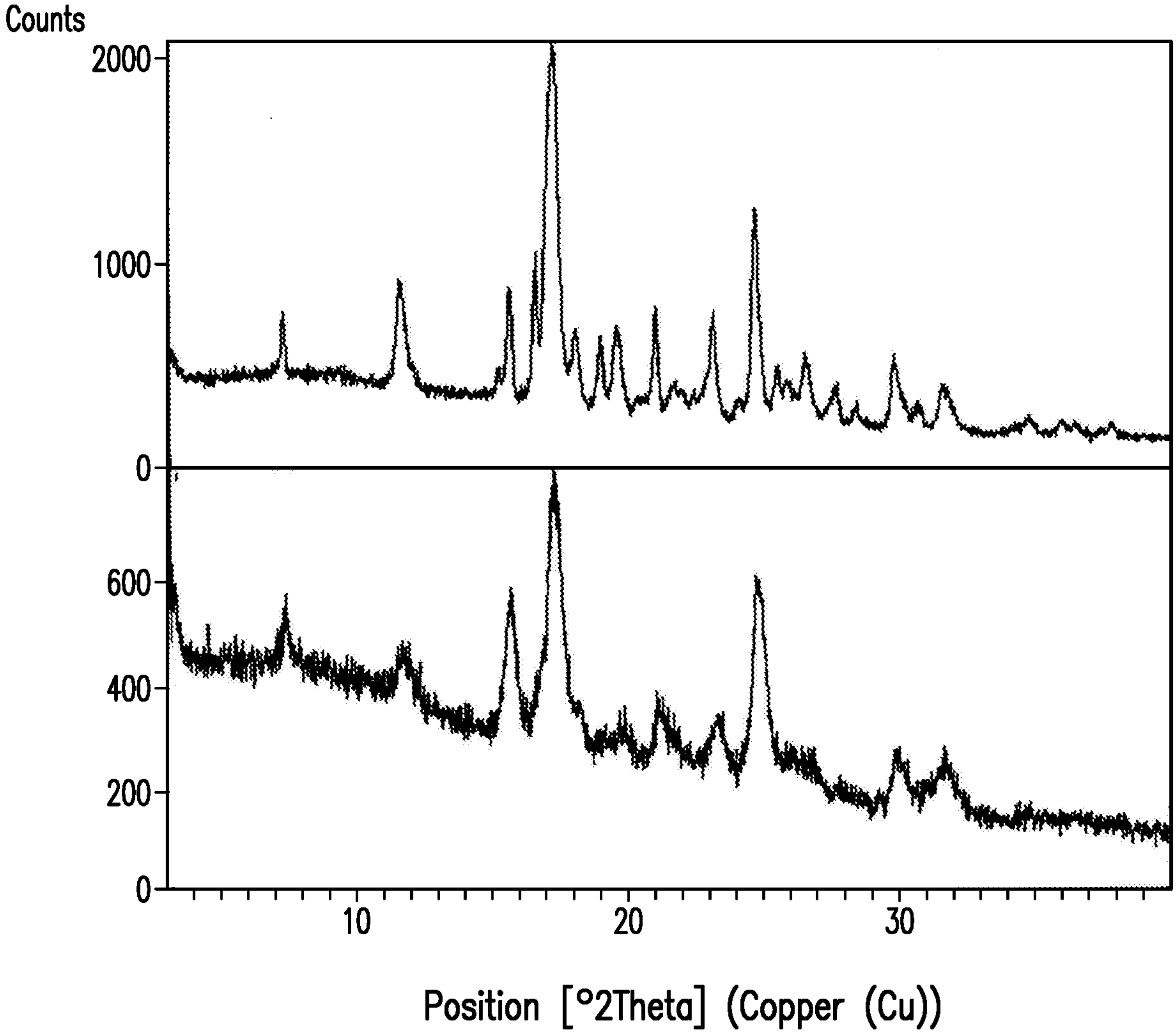
Counts
2000
1000
0
600
400
200
0
10
20
30
Position [°2Theta] (Copper (Cu))

FIG. 8

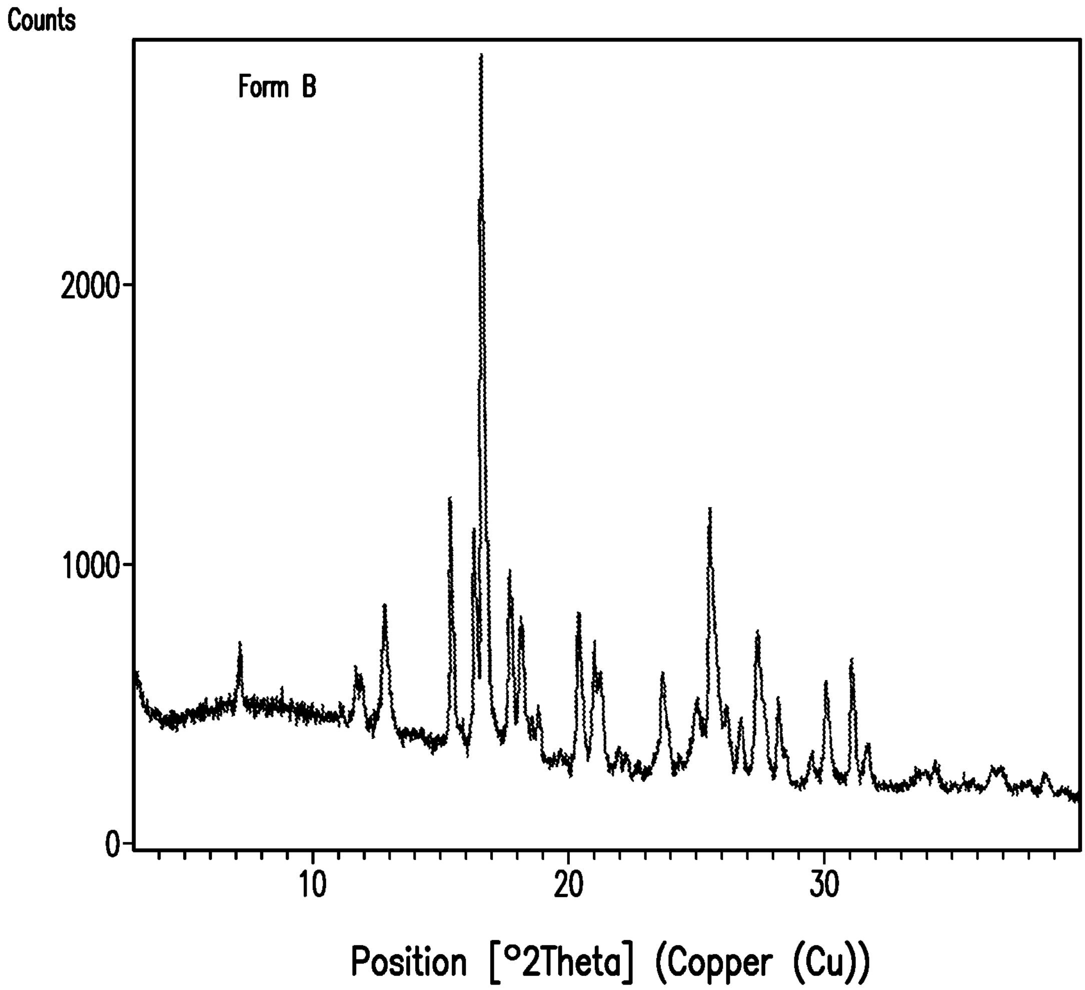


FIG. 9

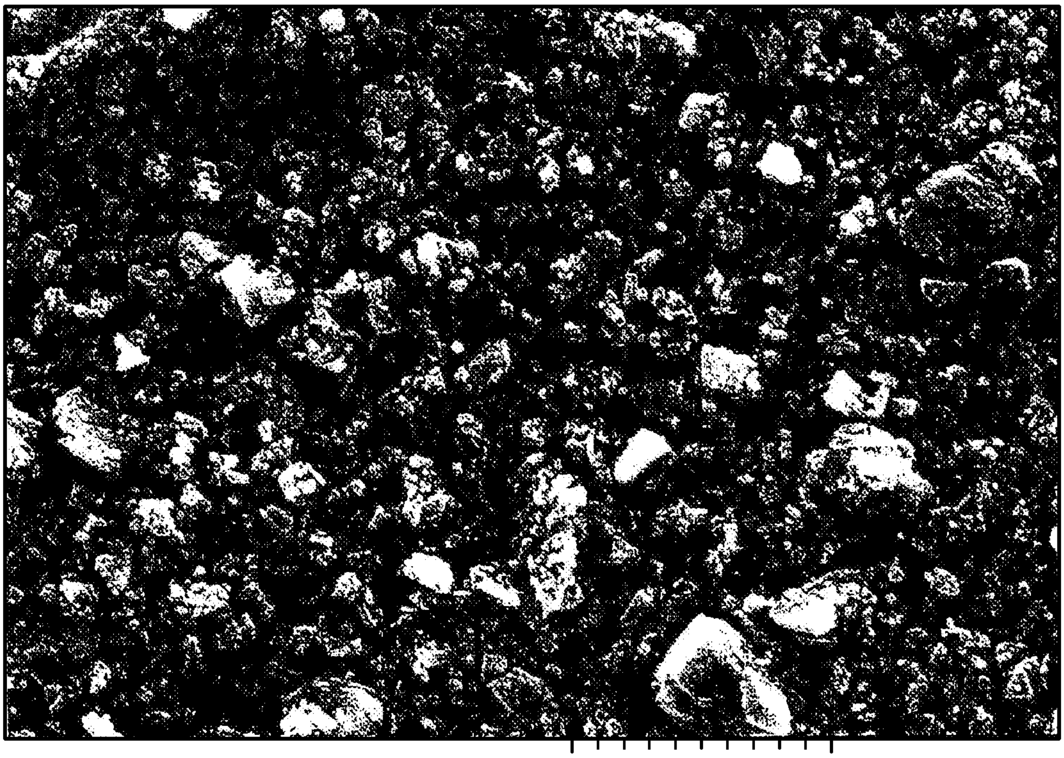


FIG. 10

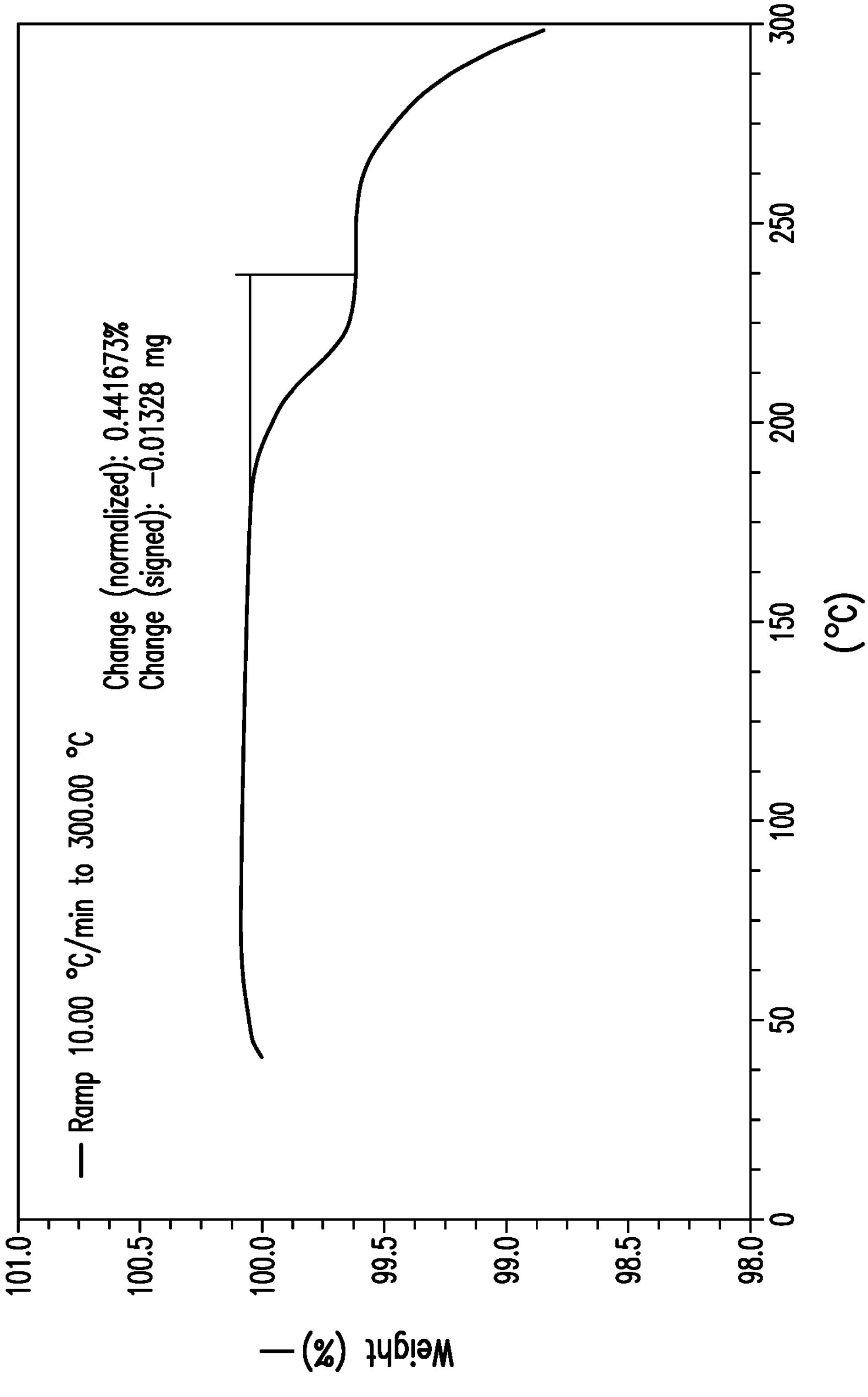
— Ramp 10.00 °C/min to 300.00 °C
Change (normalized): 0.441673%
Change (signed): −0.01328 mg
Weight (%) —
101.0
100.5
100.0
99.5
99.0
98.5
98.0
0
50
100
150
200
250
300
(°C)

FIG. 11

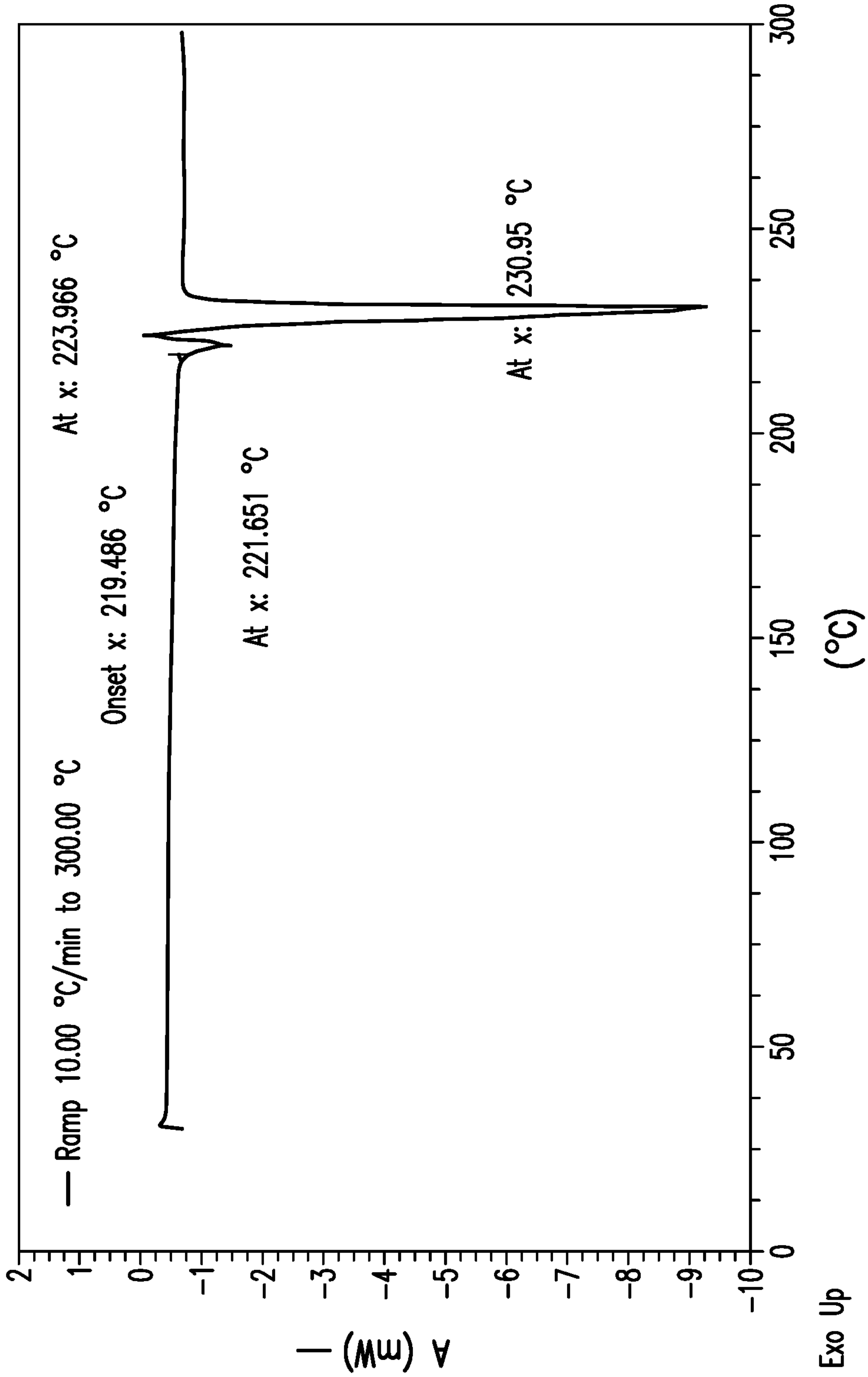

Ramp 10.00 °C/min to 300.00 °C
Onset x: 219.486 °C
At x: 221.651 °C
At x: 223.966 °C
At x: 230.95 °C
^ (mW)
Exo Up
(°C)
2
1
0
−1
−2
−3
−4
−5
−6
−7
−8
−9
−10
0
50
100
150
200
250
300


FIG. 12

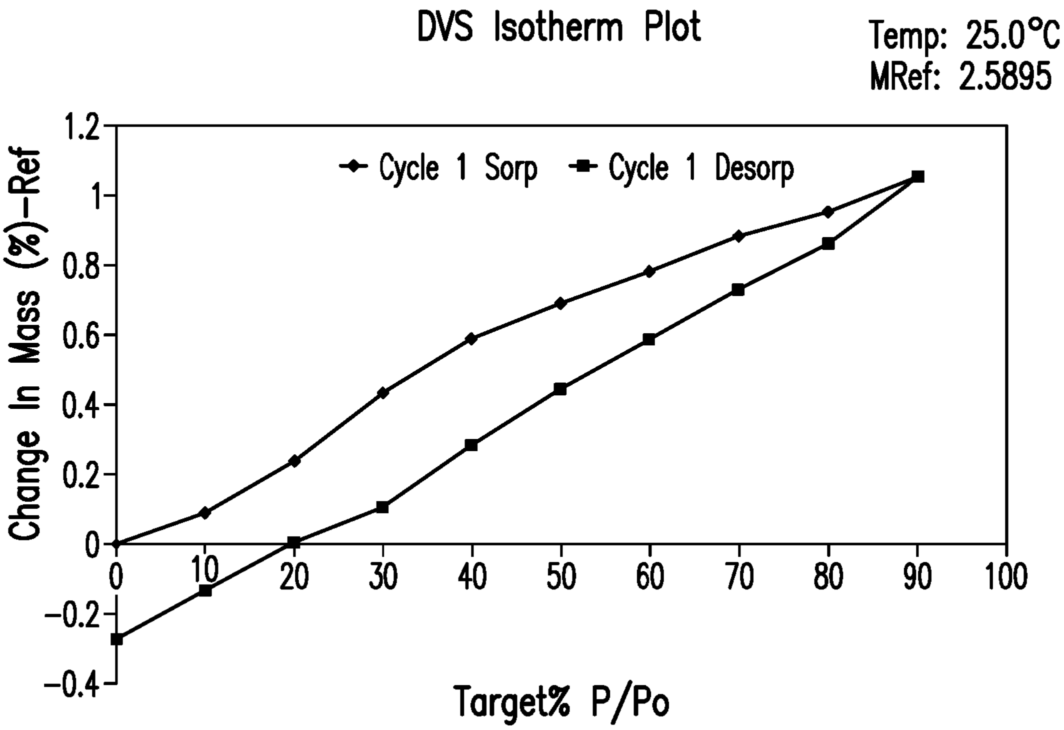


| | Target % P/Po | Change In Mass (%)−ref Sorption | Desorption | Hysteresis |
|---|---|---|---|---|
| Cycle 1 | 0.0 | 0.000 | −0.270 | |
| | 10.0 | 0.093 | −0.120 | −0.213 |
| | 20.0 | 0.241 | 0.009 | −0.232 |
| | 30.0 | 0.439 | 0.112 | −0.327 |
| | 40.0 | 0.595 | 0.292 | −0.303 |
| | 50.0 | 0.700 | 0.456 | −0.244 |
| | 60.0 | 0.798 | 0.599 | −0.198 |
| | 70.0 | 0.903 | 0.748 | −0.154 |
| | 80.0 | 0.977 | 0.886 | −0.091 |
| | 90.0 | 1.085 | 1.085 | |

FIG. 13

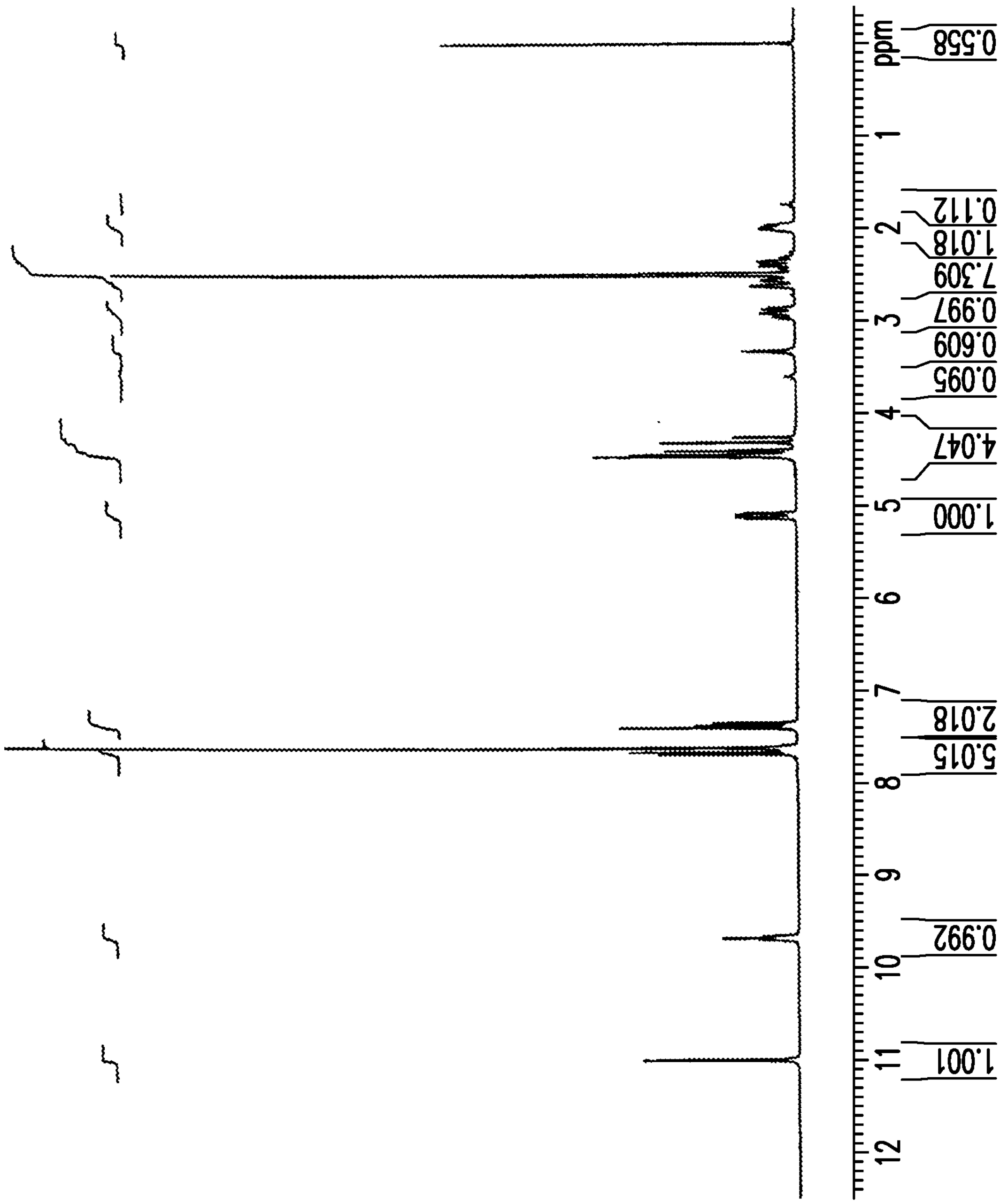
ppm
1
2
3
4
5
6
7
8
9
10
11
12
0.558
0.112
1.018
7.309
0.997
0.609
0.095
4.047
1.000
2.018
5.015
0.992
1.001

FIG. 14

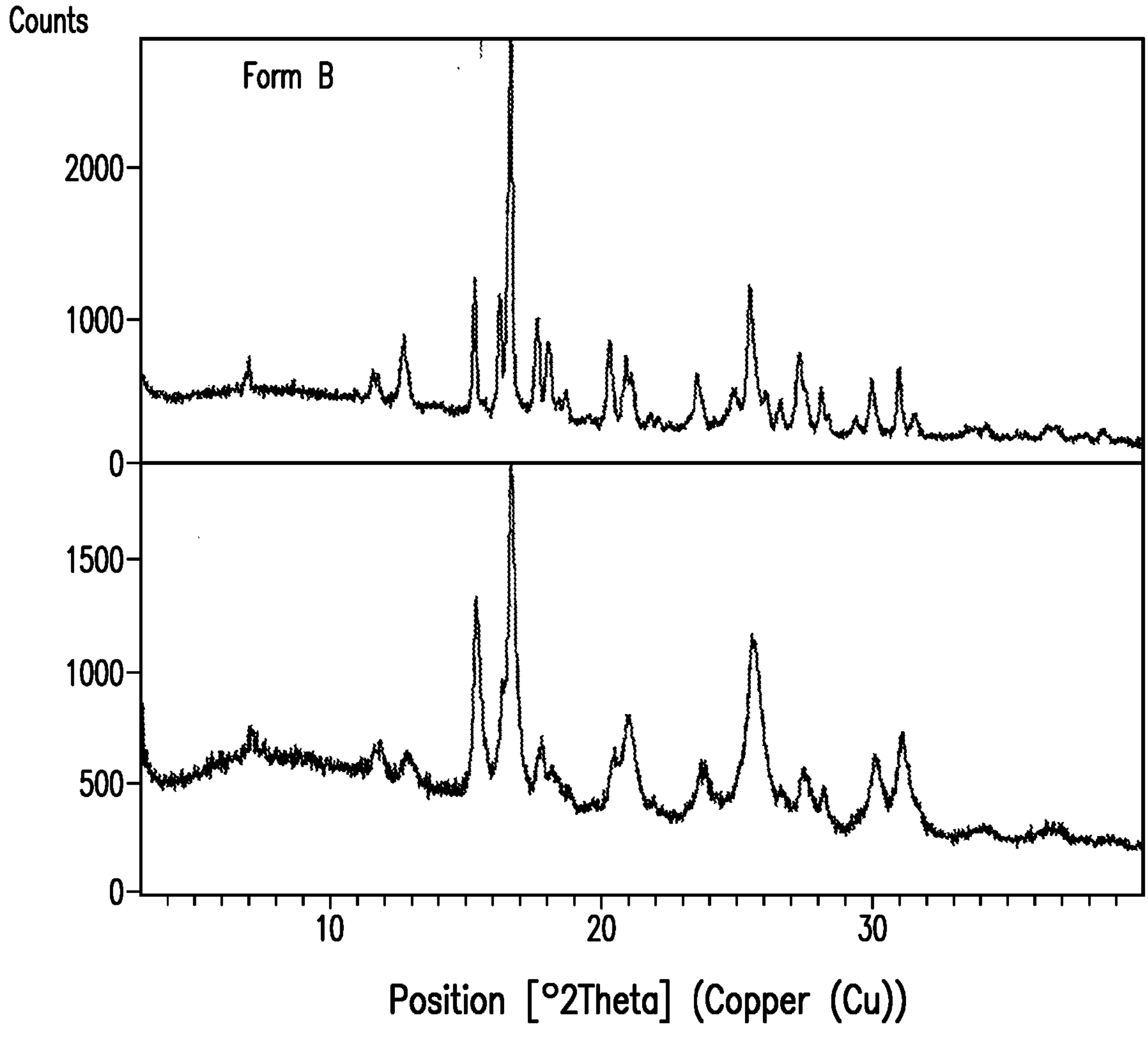
Counts
Form B
2000
1000
0
1500
1000
500
0
10
20
30
Position [°2Theta] (Copper (Cu))

FIG. 15

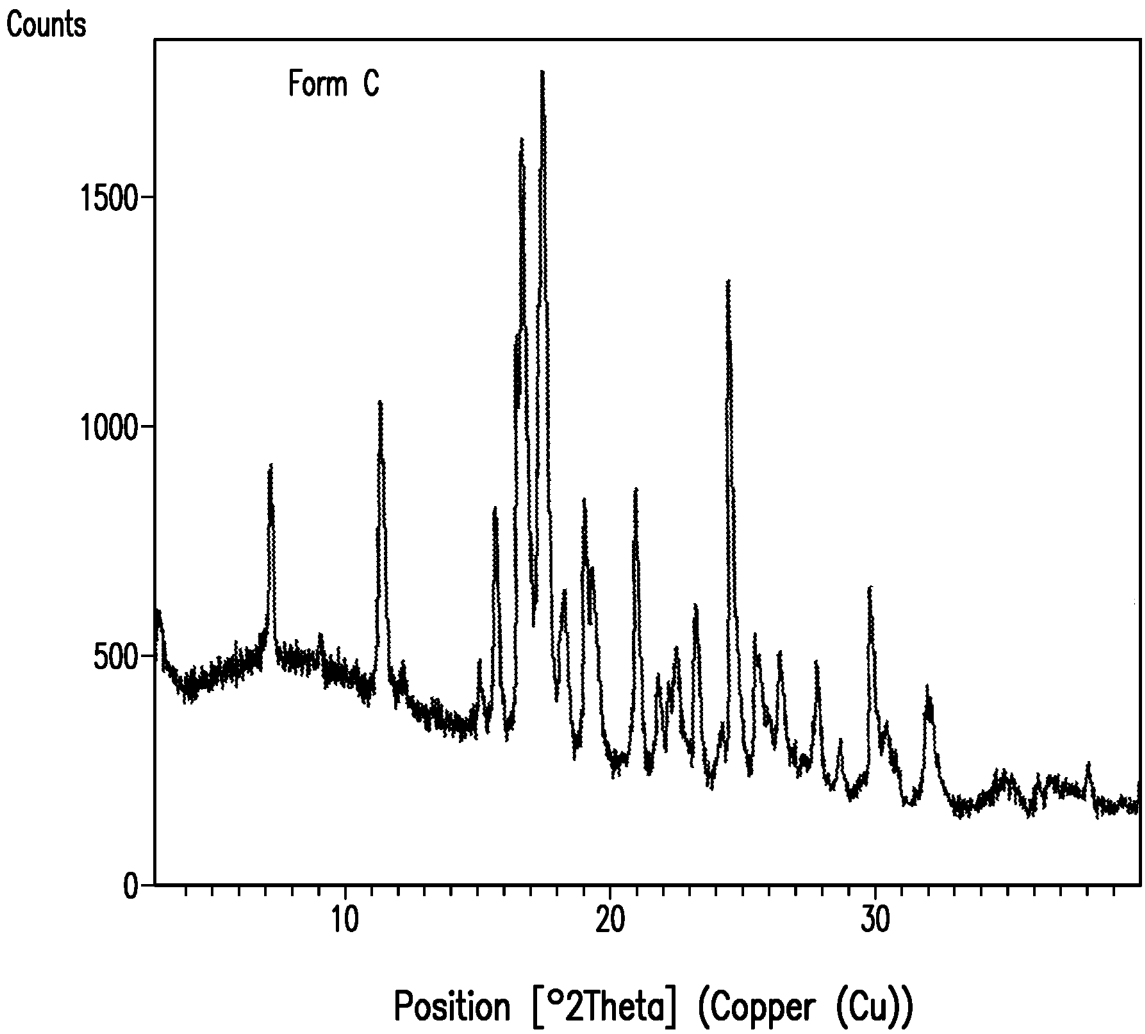
Counts
Form C
1500
1000
500
0
10
20
30
Position [°2Theta] (Copper (Cu))

FIG. 16

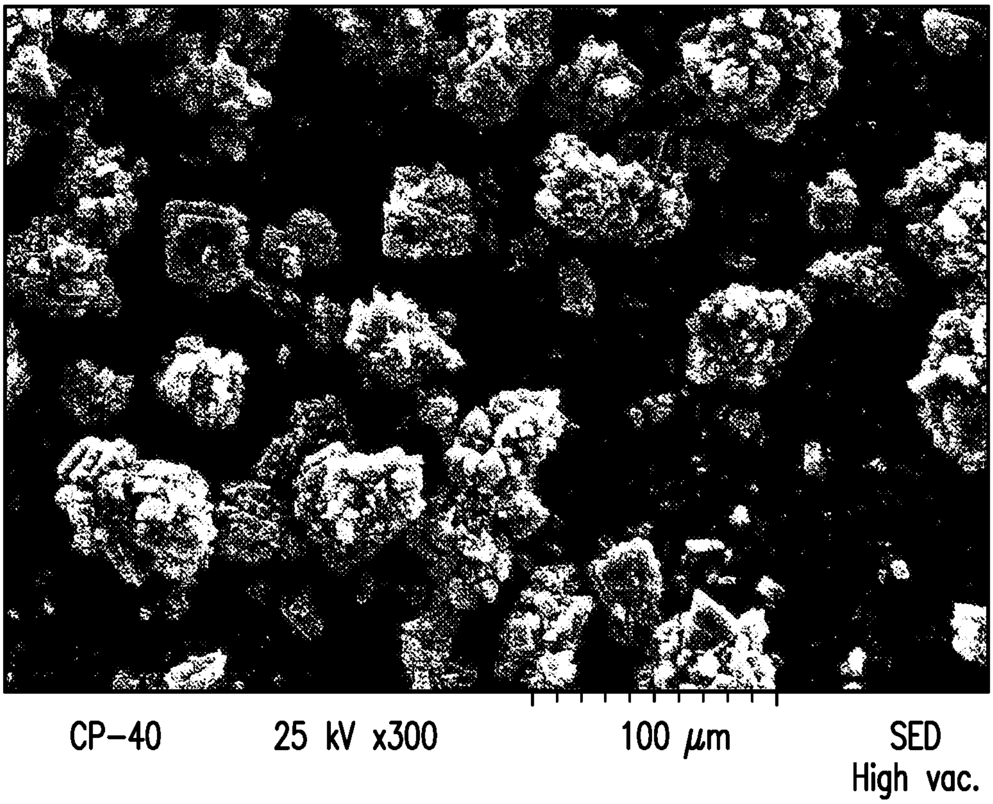


FIG. 17

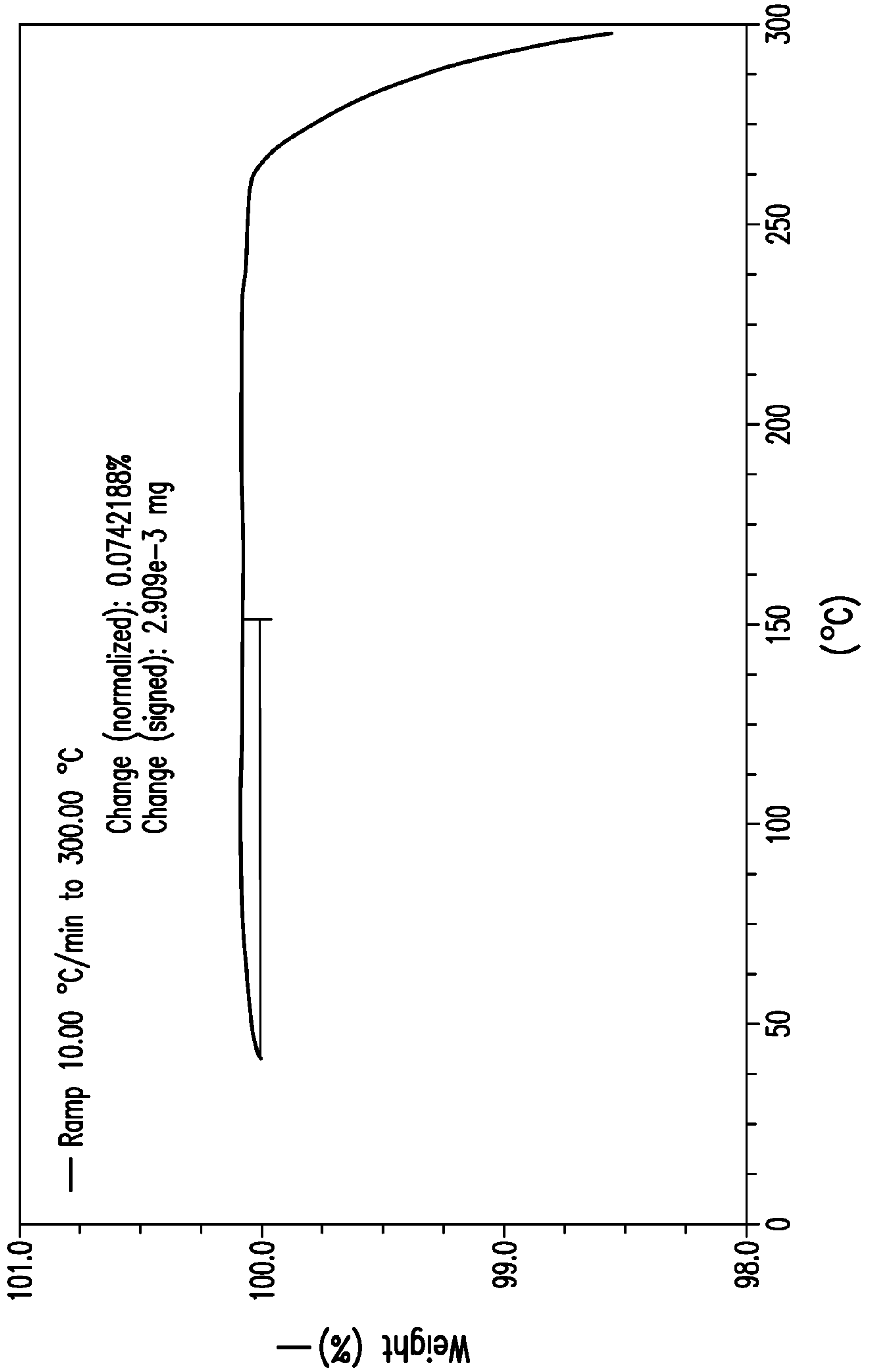

Ramp 10.00 °C/min to 300.00 °C
Change (normalized): 0.0742188%
Change (signed): 2.909e-3 mg
Weight (%)
101.0
100.0
99.0
98.0
0
50
100
150
200
250
300
(°C)


FIG. 18

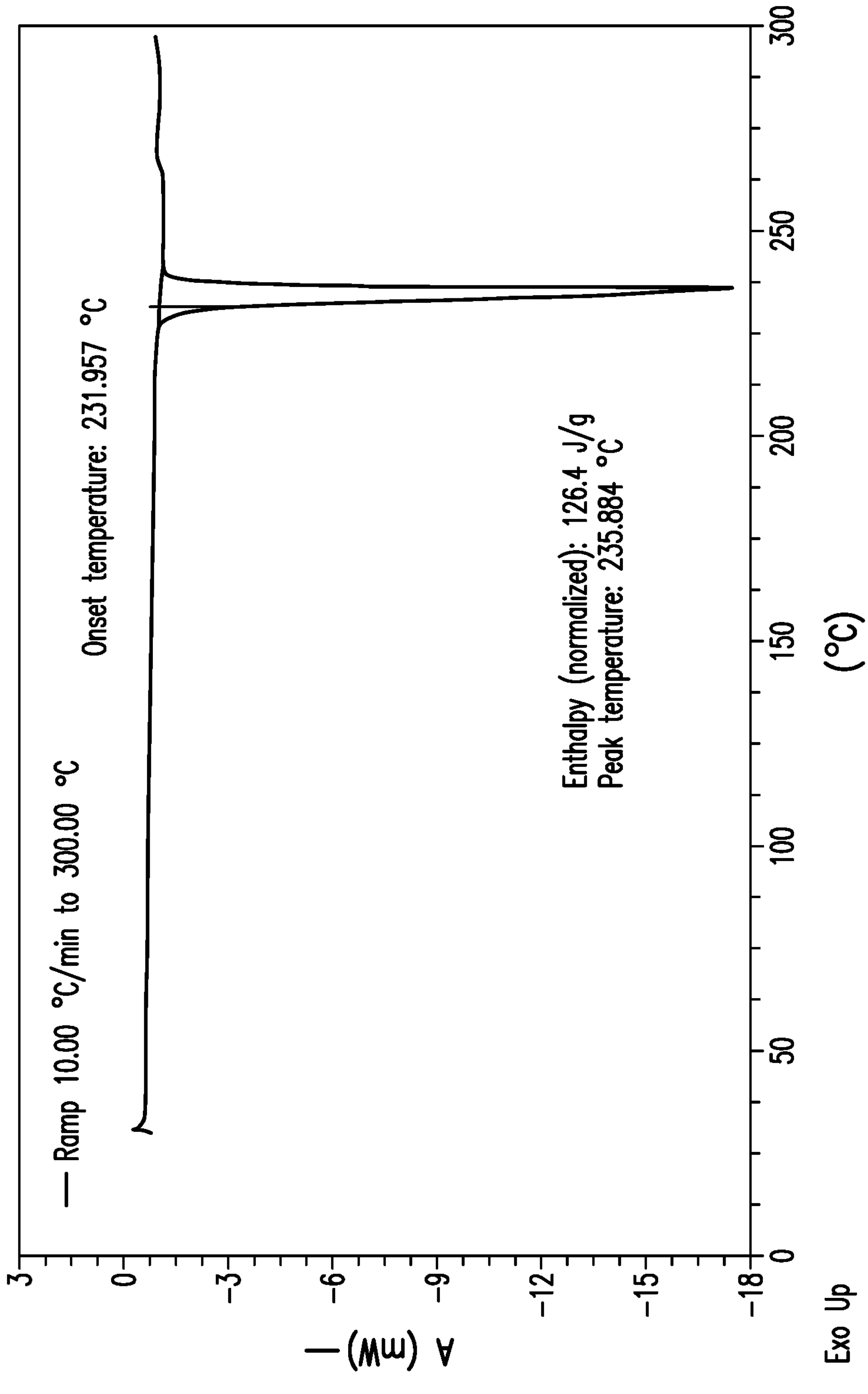

Ramp 10.00 °C/min to 300.00 °C
Onset temperature: 231.957 °C
Enthalpy (normalized): 126.4 J/g
Peak temperature: 235.884 °C
A (mW)
Exo Up
(°C)
0
50
100
150
200
250
300
3
0
−3
−6
−9
−12
−15
−18


FIG. 19

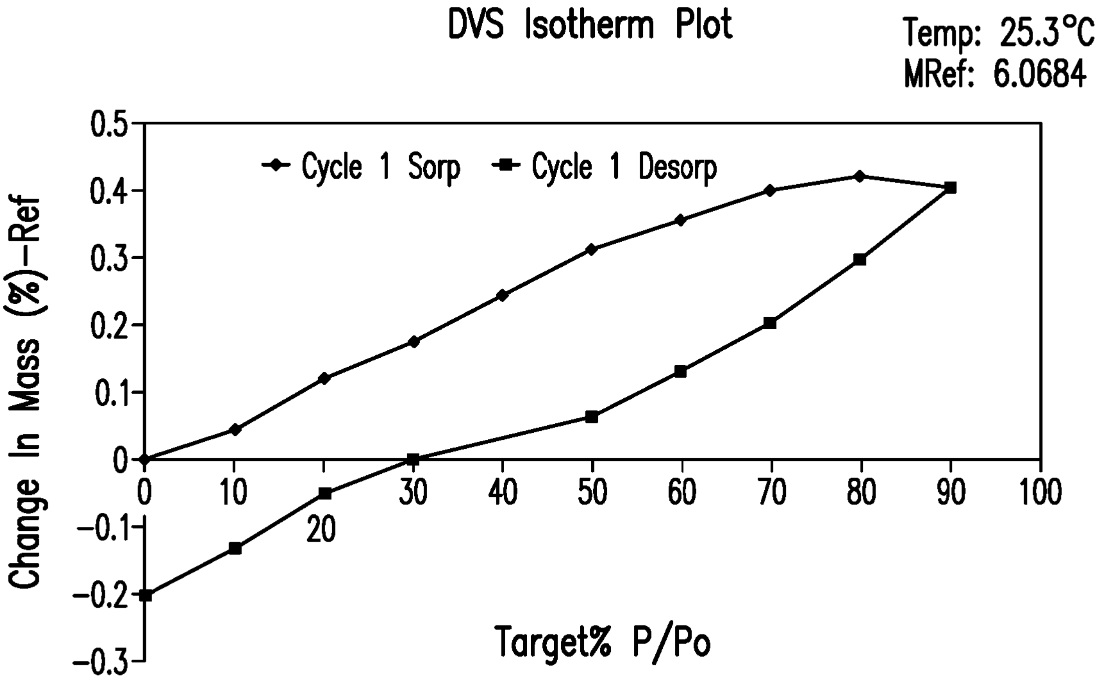


| | Target % P/Po | Change In Mass (%)−ref Sorption | Desorption | Hysteresis |
|---|---|---|---|---|
| Cycle 1 | 0.0 | −0.0020 | −0.2070 | |
| | 10.0 | 0.0409 | −0.1368 | −0.1776 |
| | 20.0 | 0.1186 | 0.0547 | −0.1734 |
| | 30.0 | 0.1730 | 0.0049 | −0.1780 |
| | 40.0 | 0.2409 | 0.0395 | −0.2805 |
| | 50.0 | 0.3114 | 0.0606 | −0.2508 |
| | 60.0 | 0.3559 | 0.1305 | −0.2254 |
| | 70.0 | 0.4014 | 0.2027 | −0.1987 |
| | 80.0 | 0.4235 | 0.2983 | −0.1252 |
| | 90.0 | 0.4087 | 0.4087 | |

FIG. 20

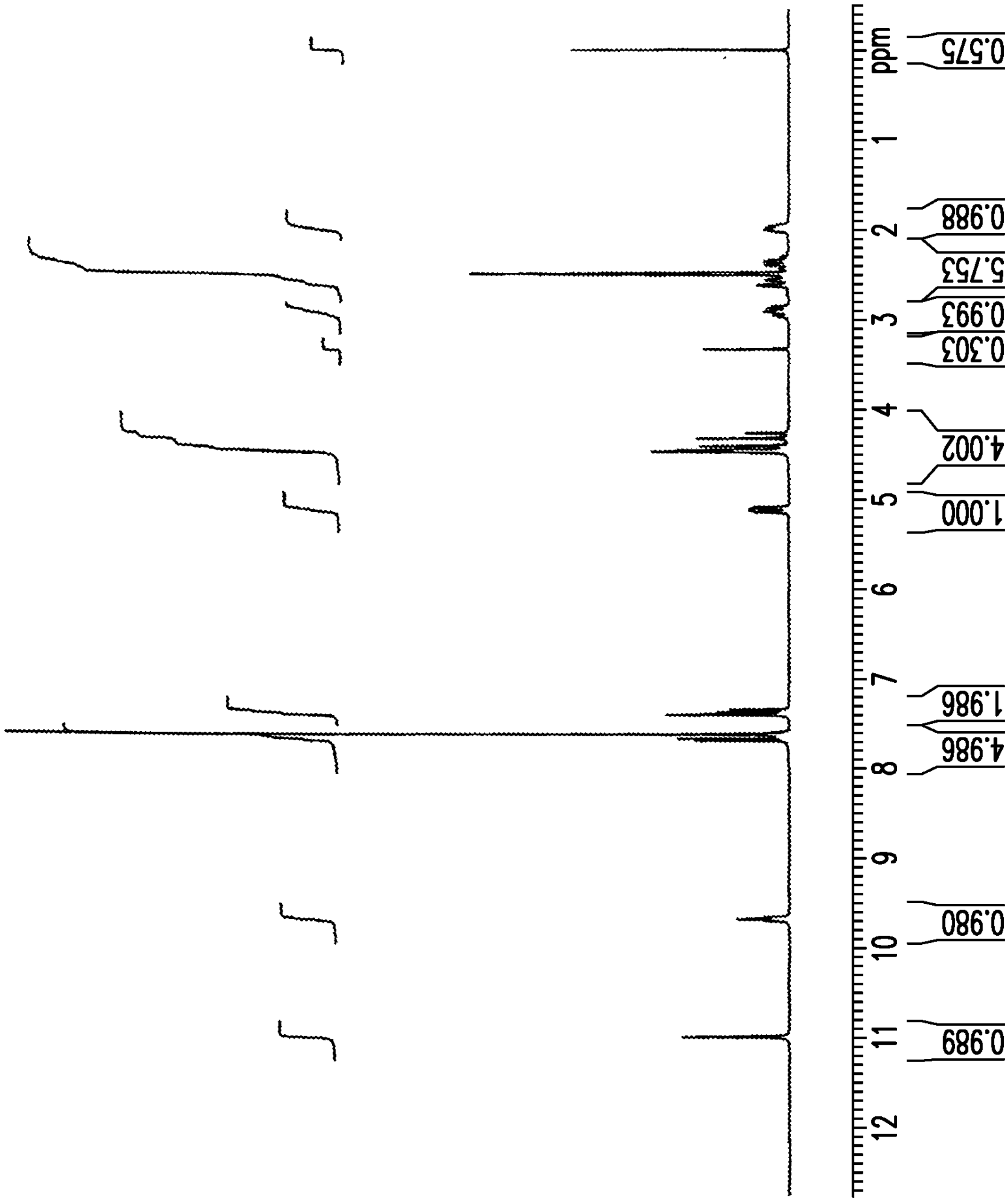
ppm
1
2
3
4
5
6
7
8
9
10
11
12
0.575
0.988
5.753
0.993
0.303
4.002
1.000
1.986
4.986
0.980
0.989

FIG. 21

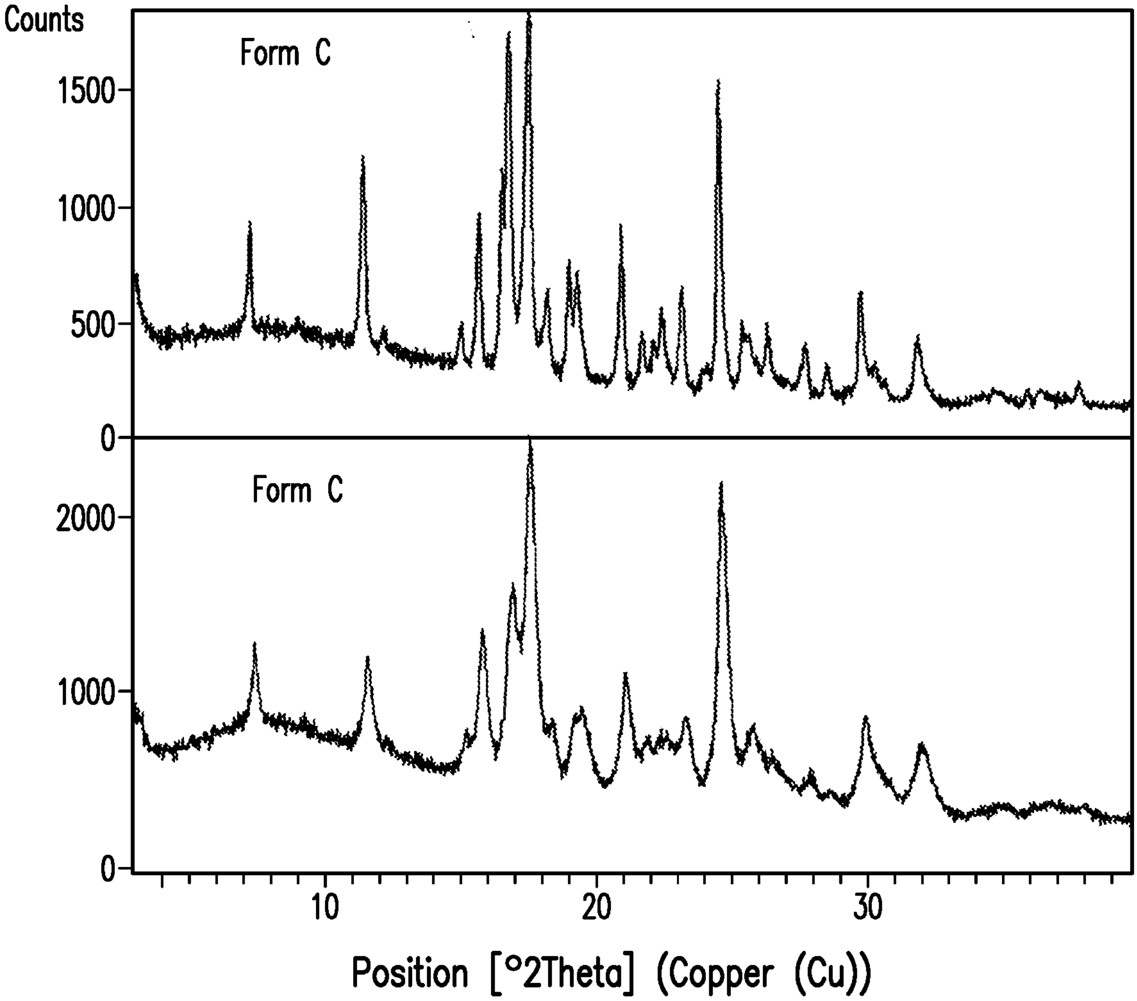
Counts
Form C
1500
1000
500
0
Form C
2000
1000
0
10
20
30
Position [°2Theta] (Copper (Cu))

FIG. 22

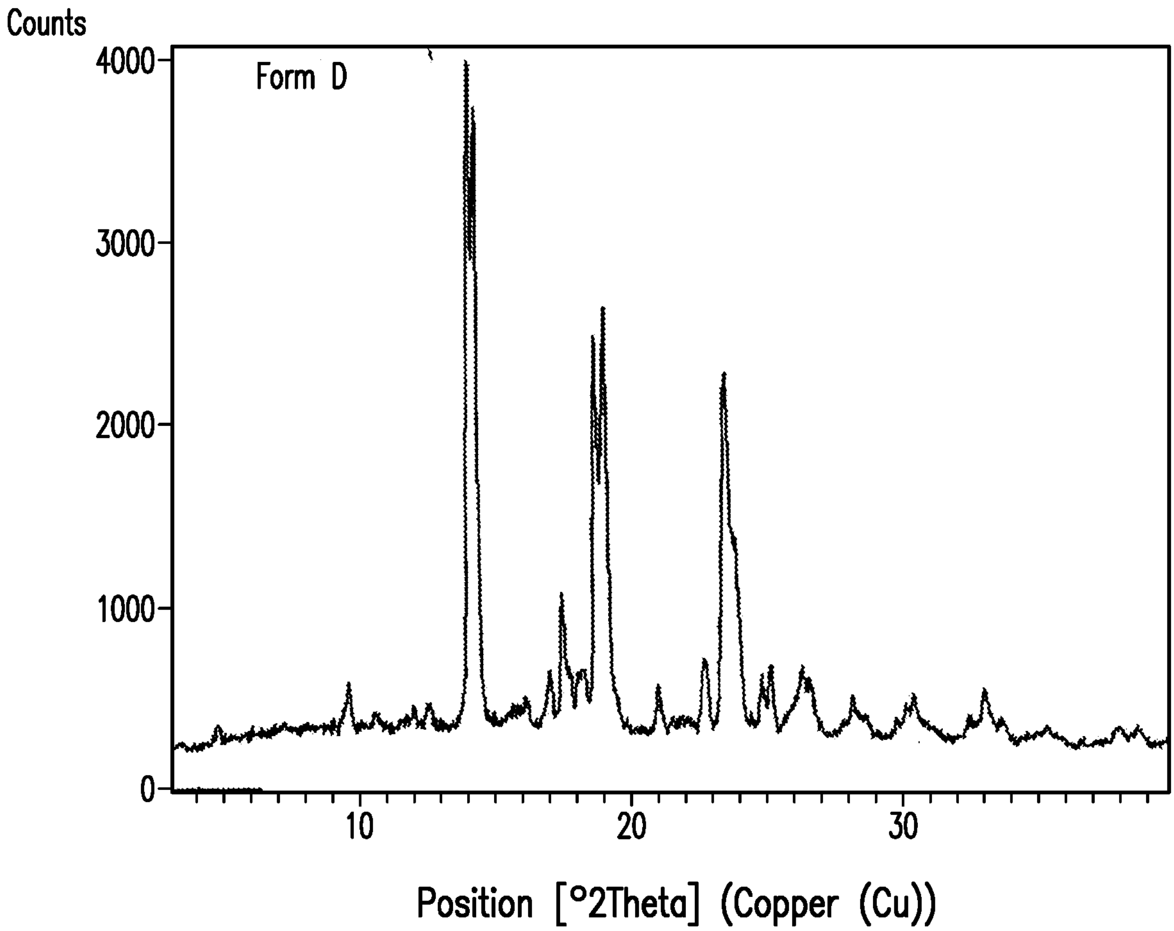
Counts
4000
3000
2000
1000
0
Form D
10
20
30
Position [°2Theta] (Copper (Cu))

FIG. 23

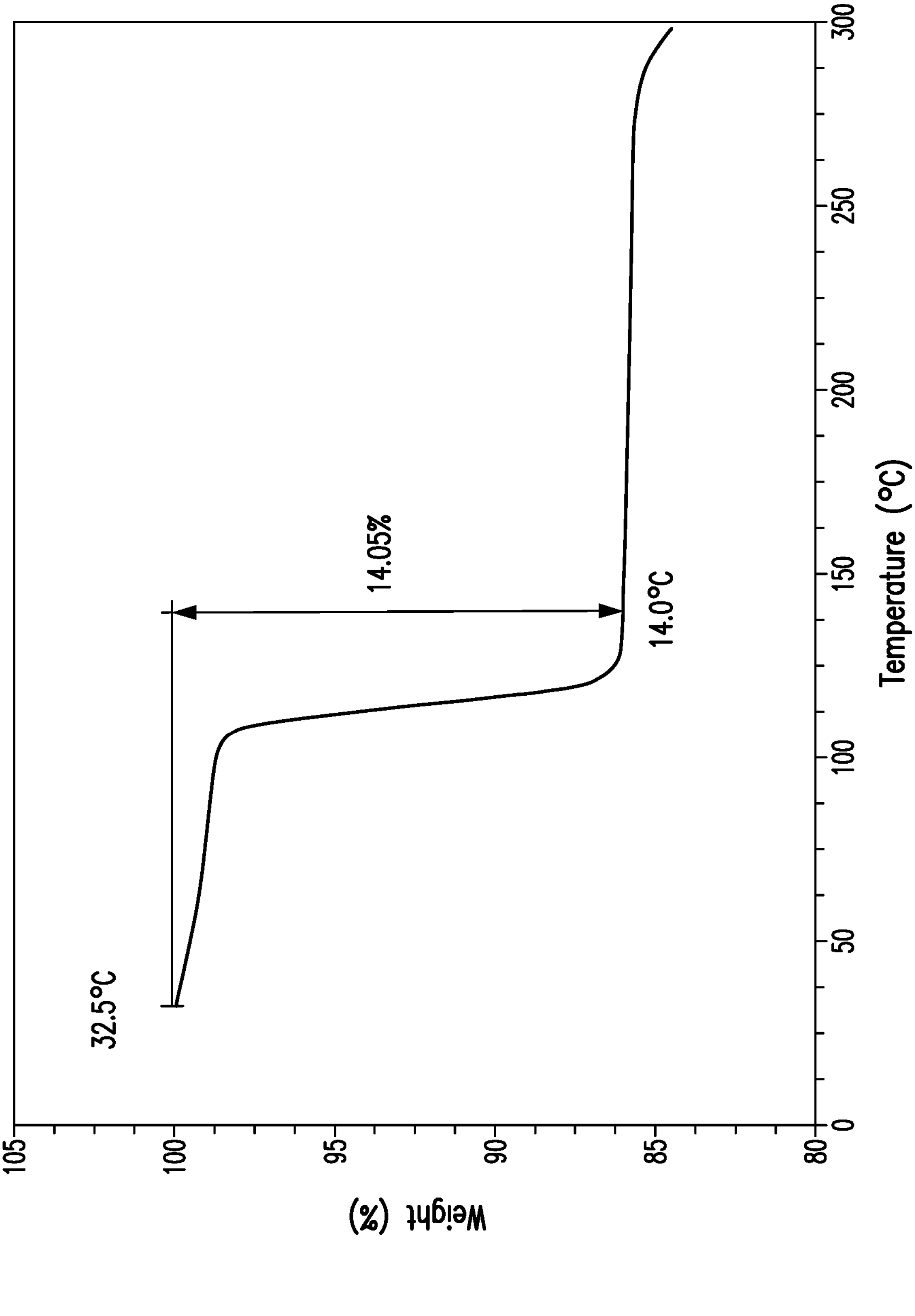
Weight (%)
105
100
95
90
85
80
Temperature (°C)
0
50
100
150
200
250
300
32.5°C
14.05%
14.0°C

FIG. 24

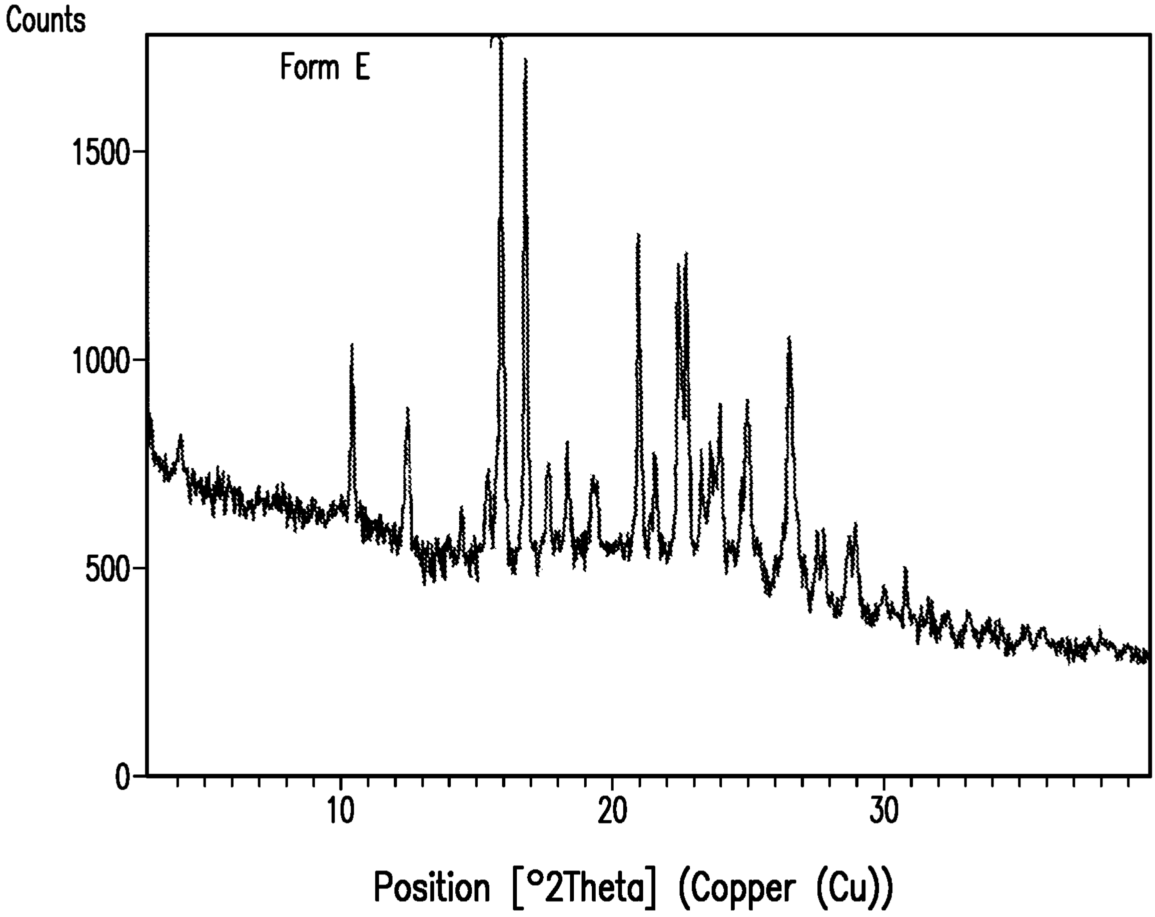
Counts
Form E
1500
1000
500
0
10
20
30
Position [°2Theta] (Copper (Cu))

FIG. 25

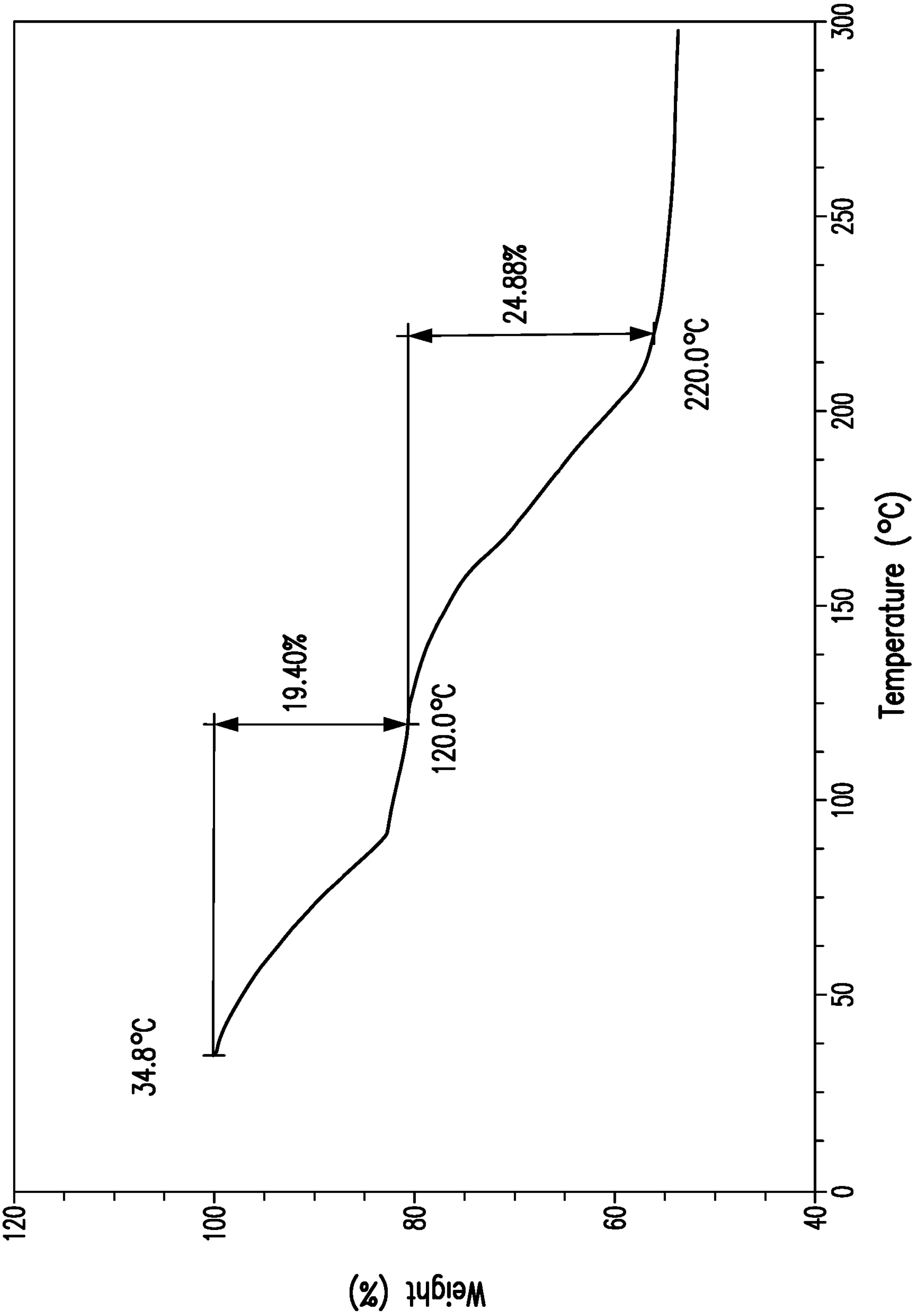
Weight (%)
120
100
80
60
40
Temperature (°C)
0
50
100
150
200
250
300
34.8°C
19.40%
120.0°C
24.88%
220.0°C

FIG. 26

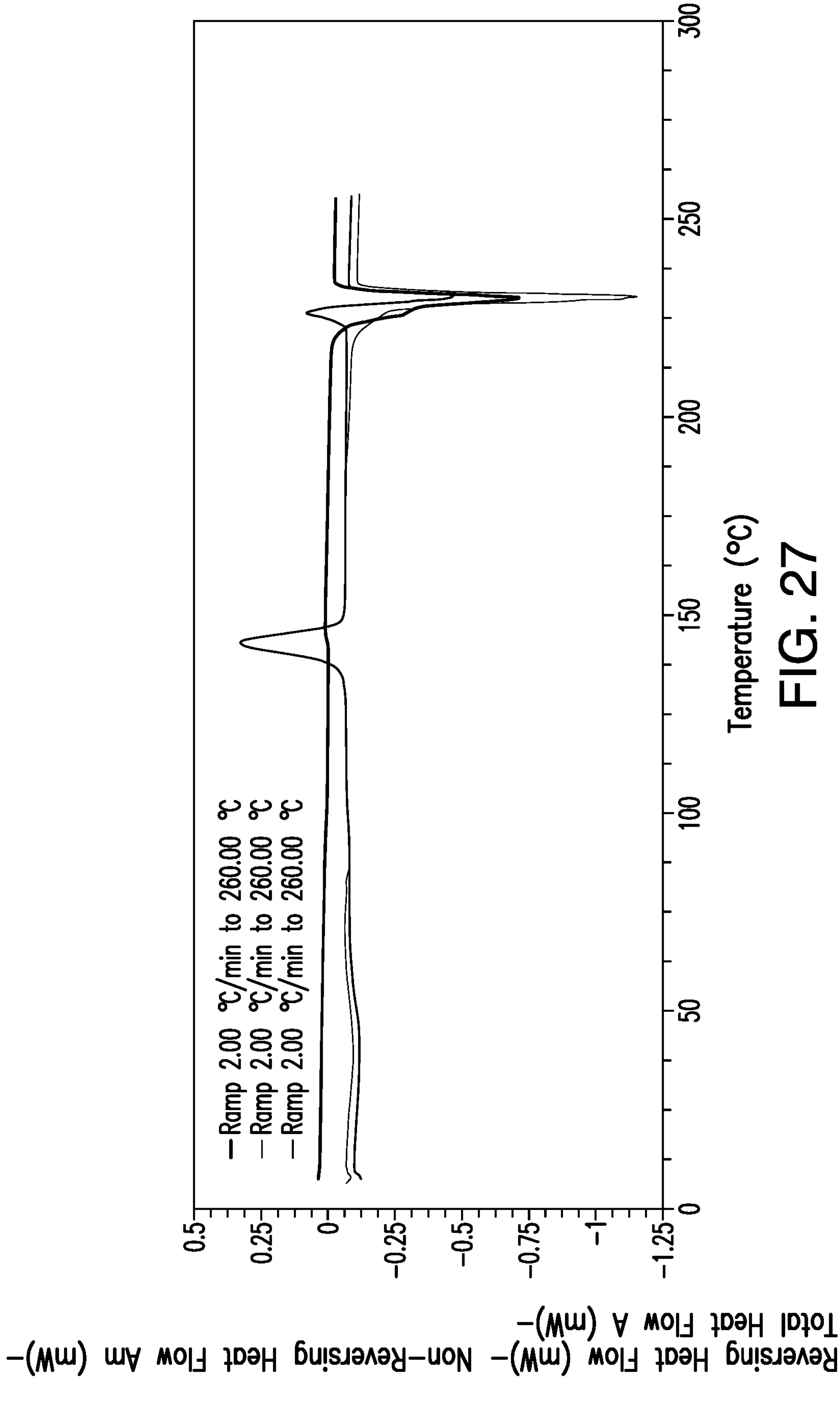

—Ramp 2.00 °C/min to 260.00 °C
—Ramp 2.00 °C/min to 260.00 °C
—Ramp 2.00 °C/min to 260.00 °C
Reversing Heat Flow (mW)– Non–Reversing Heat Flow Am (mW)–
Total Heat Flow A (mW)–
0.5
0.25
0
–0.25
–0.5
–0.75
–1
–1.25
0
50
100
150
200
250
300
Temperature (°C)


FIG. 27

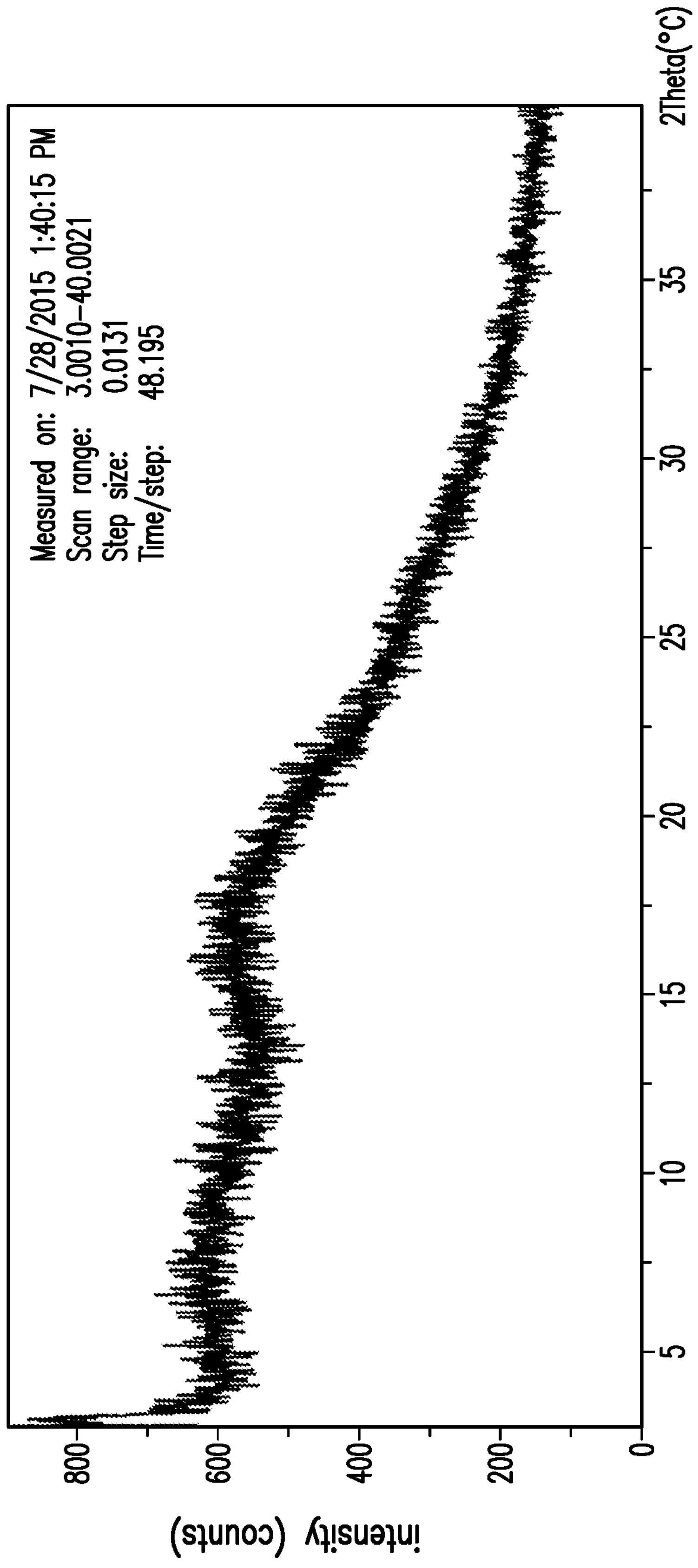
Measured on: 7/28/2015 1:40:15 PM
Scan range: 3.0010–40.0021
Step size: 0.0131
Time/step: 48.195
intensity (counts)
800
600
400
200
0
5
10
15
20
25
30
35
2Theta(°C)

FIG. 28

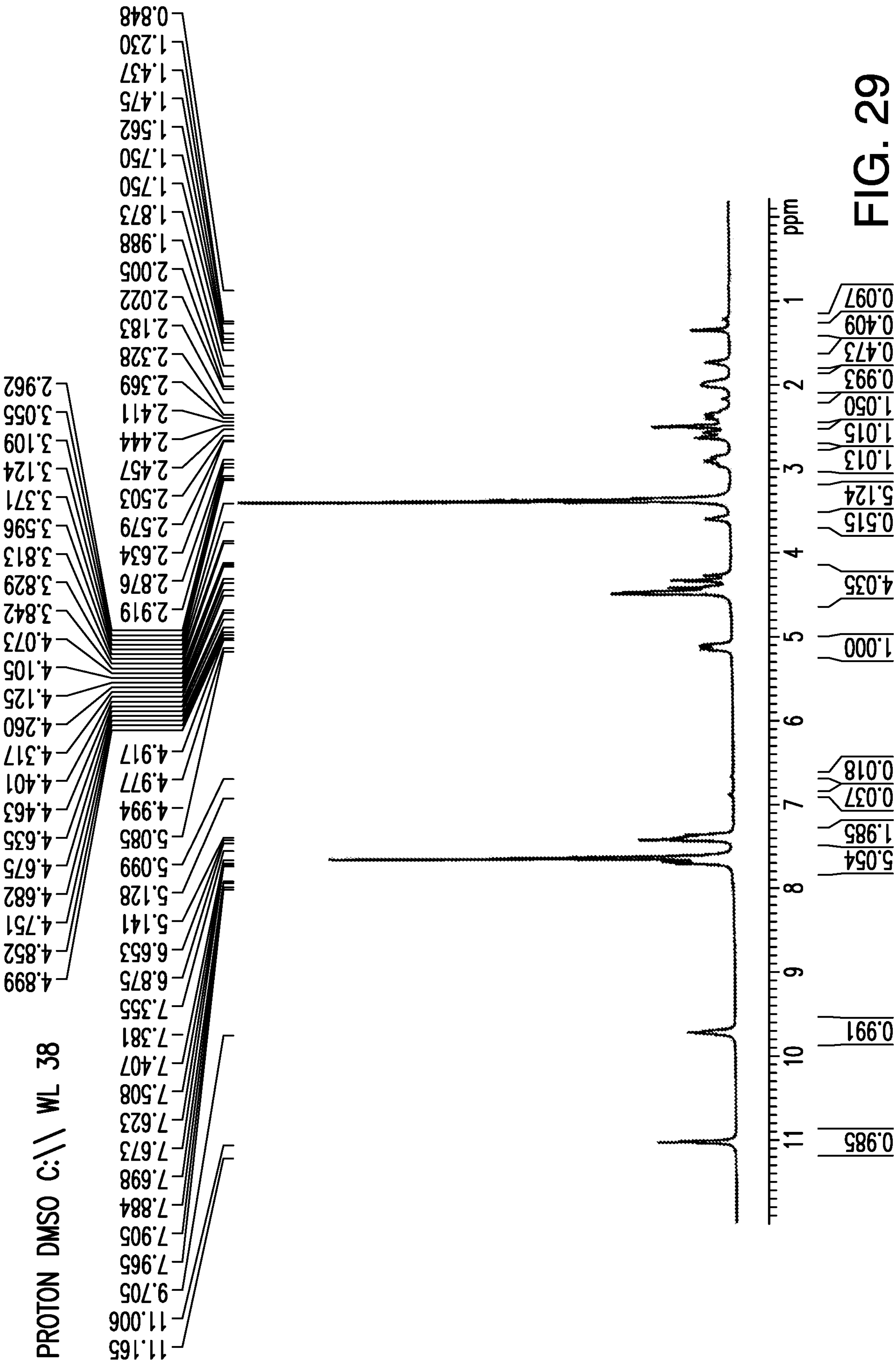
PROTON DMSO C:\\ WL 38
0.848
1.230
1.437
1.475
1.562
1.750
1.750
1.873
1.988
2.005
2.022
2.183
2.328
2.369
2.411
2.444
2.457
2.503
2.579
2.634
2.876
2.919
2.962
3.055
3.109
3.124
3.371
3.596
3.813
3.829
3.842
4.073
4.105
4.125
4.260
4.317
4.401
4.463
4.635
4.675
4.682
4.751
4.852
4.899
4.917
4.977
4.994
5.085
5.099
5.128
5.141
6.653
6.875
7.355
7.381
7.407
7.508
7.623
7.673
7.698
7.884
7.905
7.965
9.705
11.006
11.165
ppm
1
2
3
4
5
6
7
8
9
10
11
0.097
0.409
0.473
0.993
1.050
1.015
1.013
5.124
0.515
4.035
1.000
0.018
0.037
1.985
5.054
0.991
0.985

FIG. 29

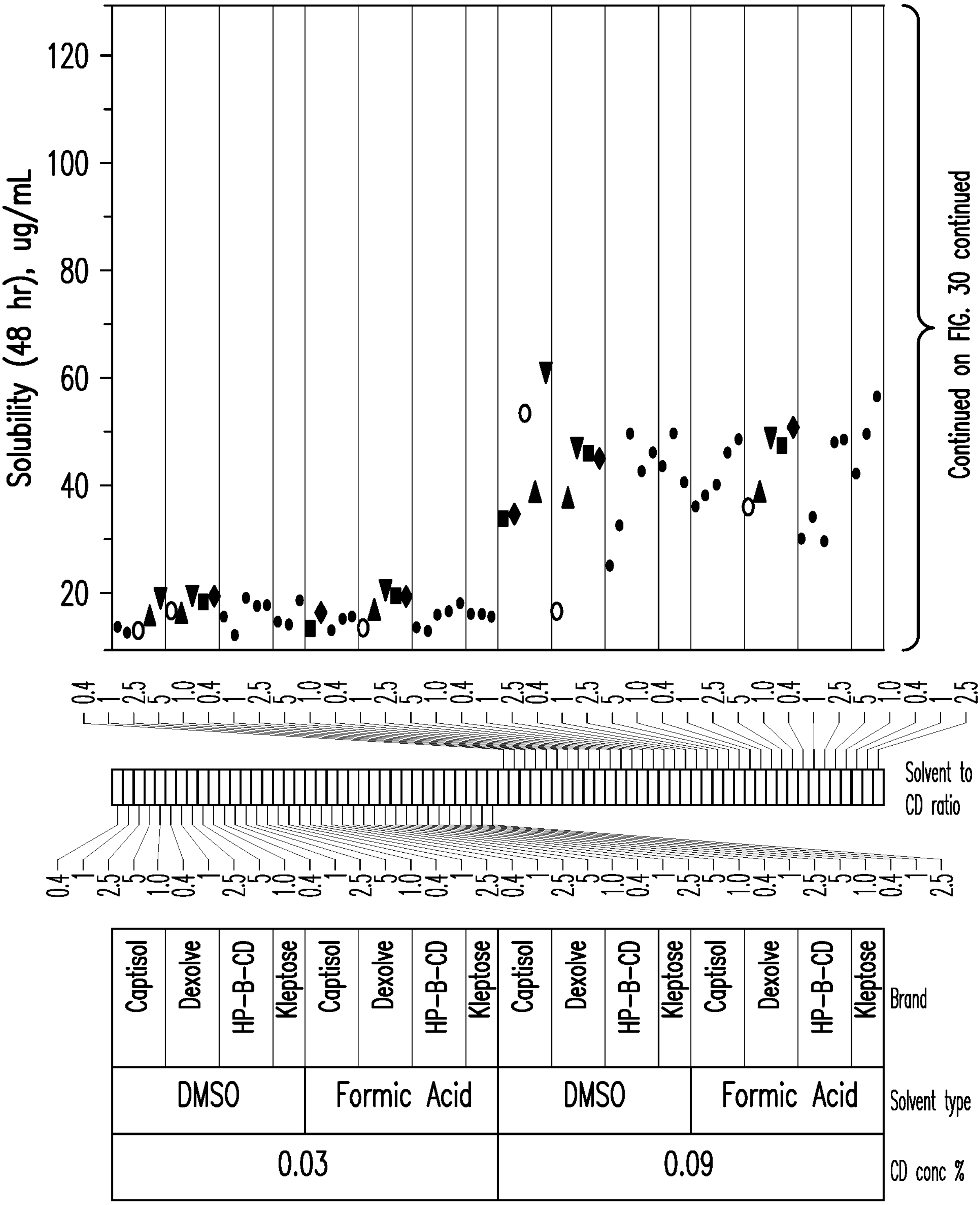

Solubility (48 hr), ug/mL
120
100
80
60
40
20
Continued on FIG. 30 continued
Solvent to CD ratio
Captisol
Dexolve
HP-B-CD
Kleptose
Captisol
Dexolve
HP-B-CD
Kleptose
Captisol
Dexolve
HP-B-CD
Kleptose
Captisol
Dexolve
HP-B-CD
Kleptose
Brand
DMSO
Formic Acid
DMSO
Formic Acid
Solvent type
0.03
0.09
CD conc %


FIG. 30

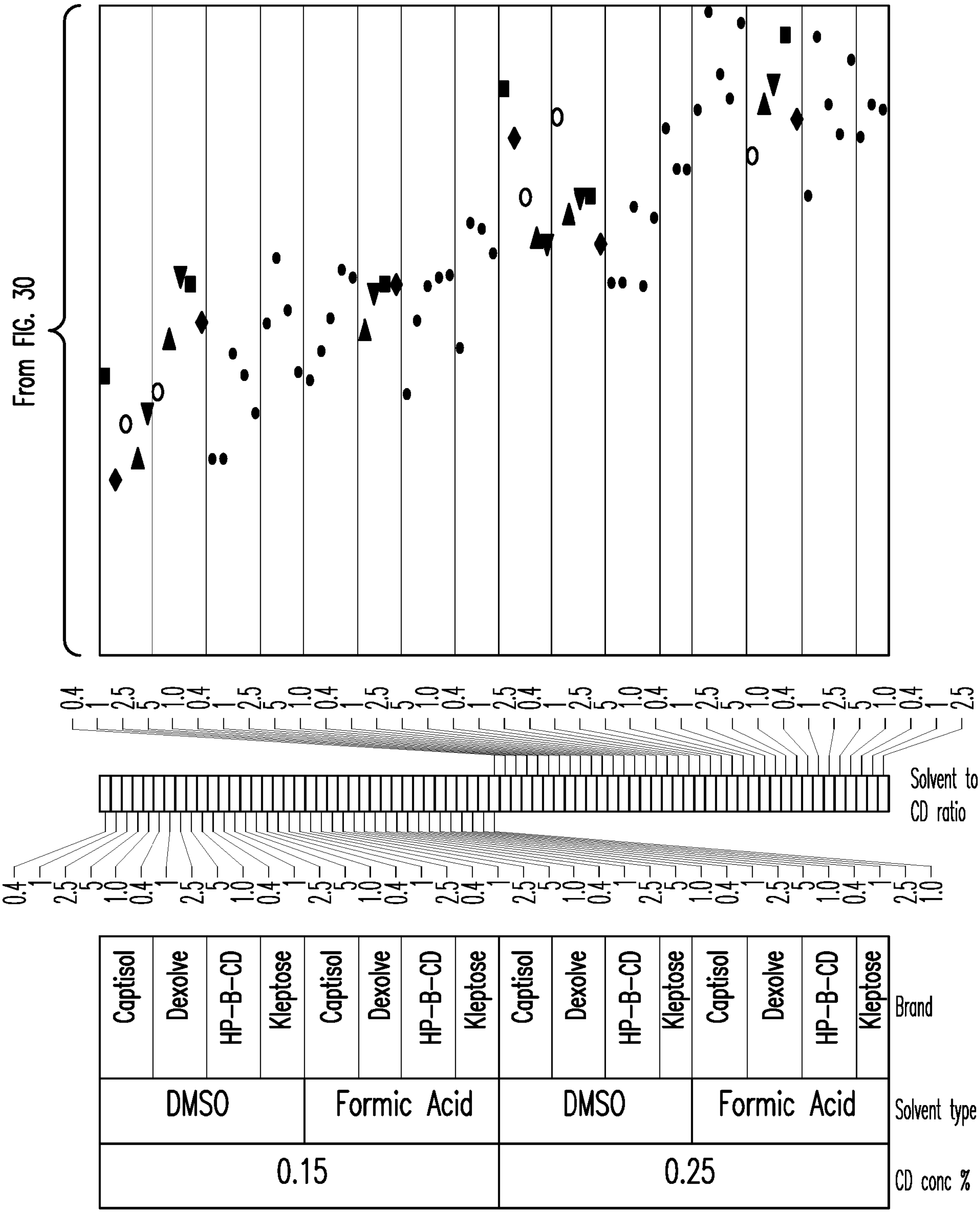


FIG. 30 continued

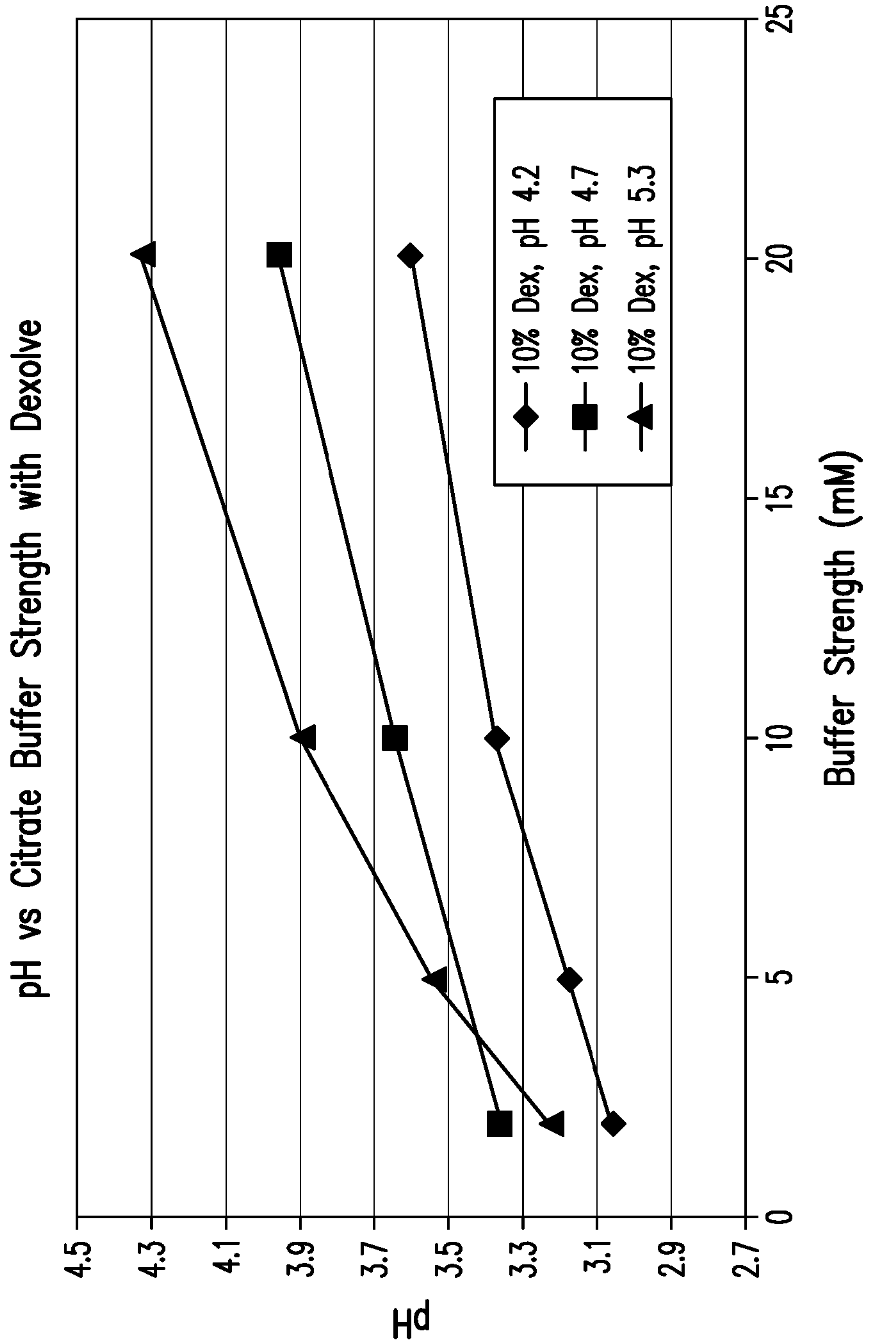
pH vs Citrate Buffer Strength with Dexolve
pH
4.5
4.3
4.1
3.9
3.7
3.5
3.3
3.1
2.9
2.7
0
5
10
15
20
25
Buffer Strength (mM)
10% Dex, pH 4.2
10% Dex, pH 4.7
10% Dex, pH 5.3

FIG. 31

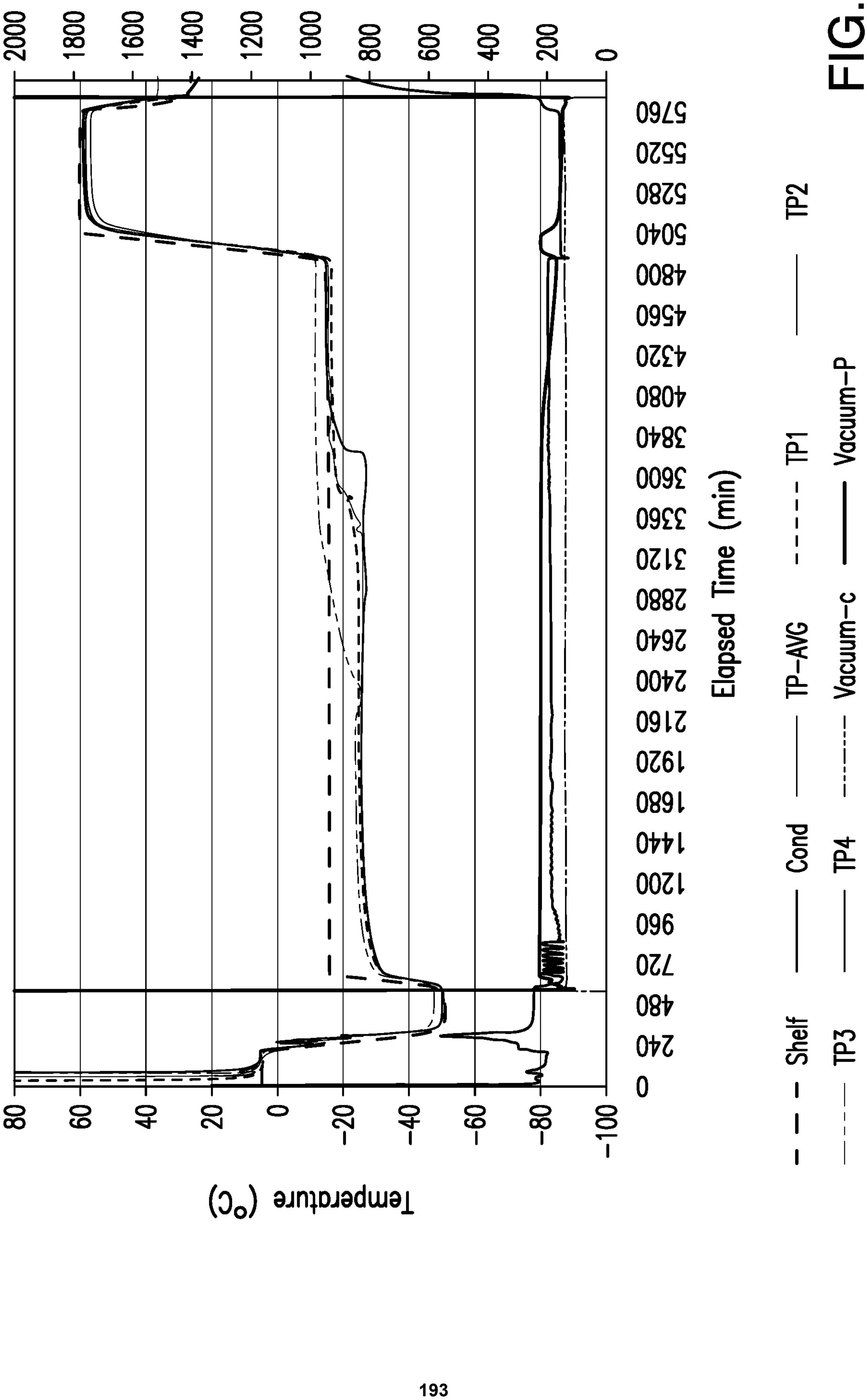
Temperature (°C)
80
60
40
20
0
-20
-40
-60
-80
-100
2000
1800
1600
1400
1200
1000
800
600
400
200
0
0
240
480
720
960
1200
1440
1680
1920
2160
2400
2640
2880
3120
3360
3600
3840
4080
4320
4560
4800
5040
5280
5520
5760
Elapsed Time (min)
Shelf
Cond
TP-AVG
TP1
TP2
TP3
TP4
Vacuum-c
Vacuum-P

FIG. 32

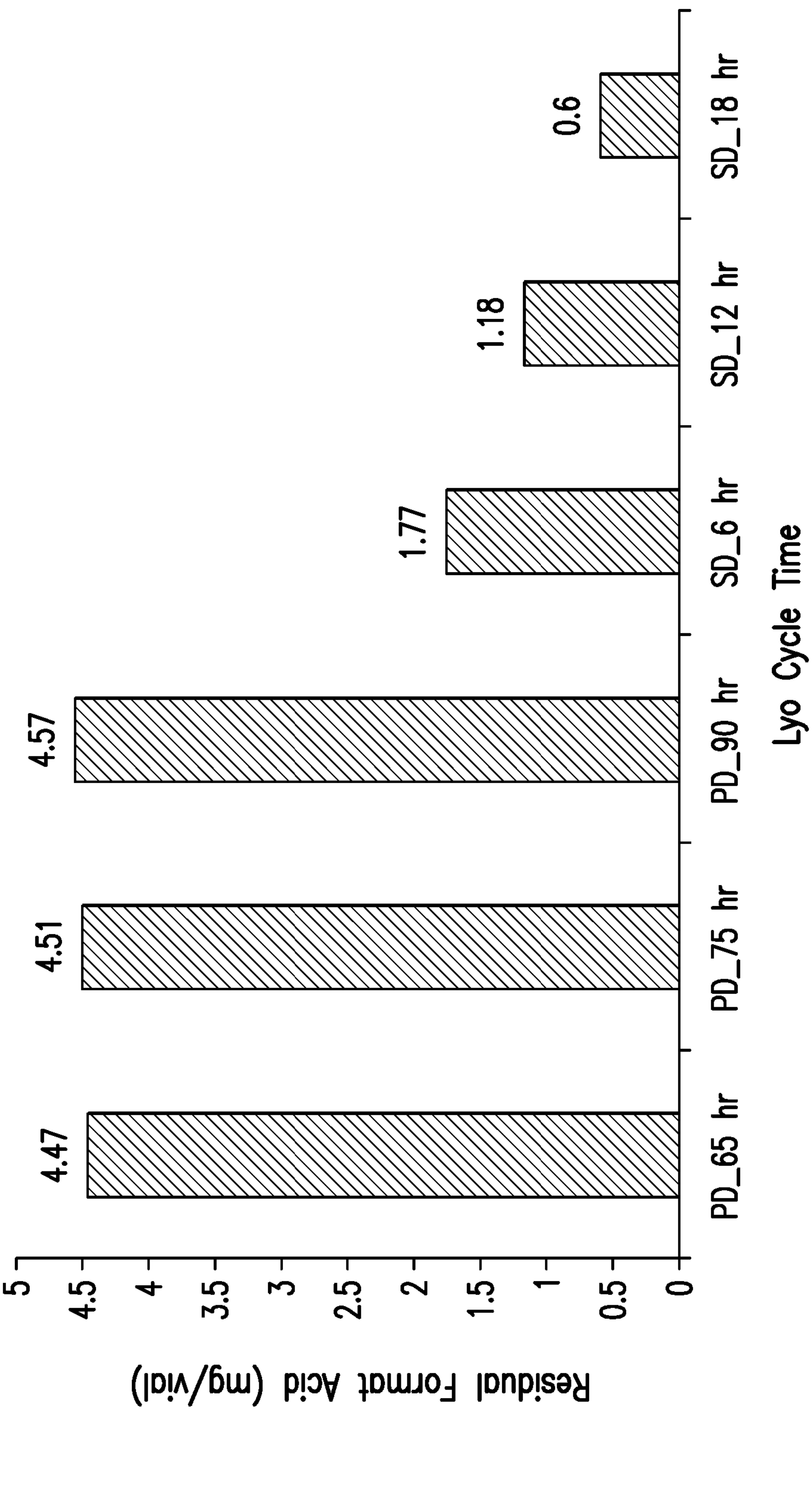
Residual Format Acid (mg/vial)
0
0.5
1
1.5
2
2.5
3
3.5
4
4.5
5
4.47
4.51
4.57
1.77
1.18
0.6
PD_65 hr
PD_75 hr
PD_90 hr
SD_6 hr
SD_12 hr
SD_18 hr
Lyo Cycle Time

FIG. 33

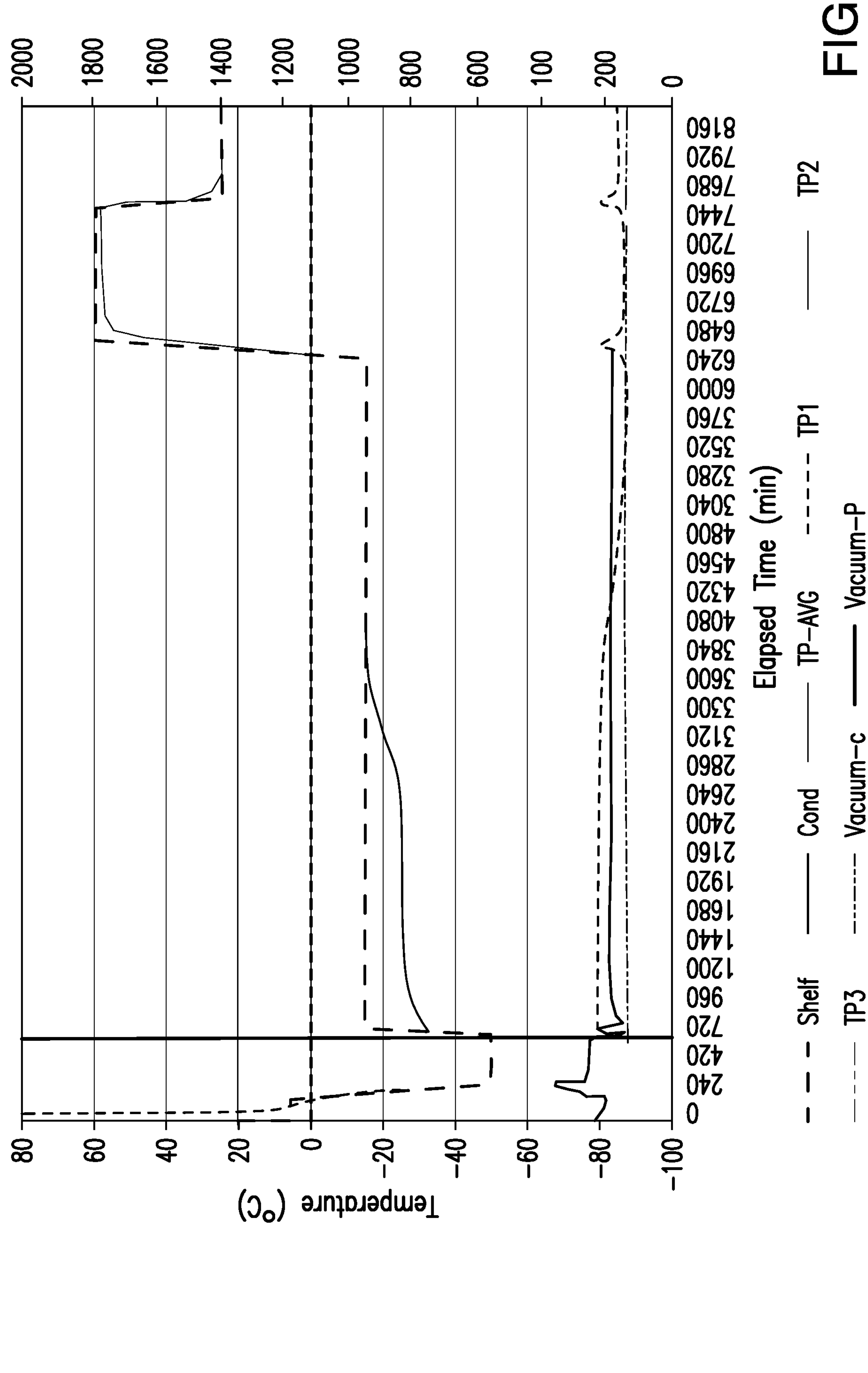
Temperature (°C)
80
60
40
20
0
-20
-40
-60
-80
-100
2000
1800
1600
1400
1200
1000
800
600
100
200
0
0
240
420
720
960
1200
1440
1680
1920
2160
2400
2640
2860
3120
3300
3600
3840
4080
4320
4560
4800
3040
3280
3520
3760
6000
6240
6480
6720
6960
7200
7440
7680
7920
8160
Elapsed Time (min)
Shelf
Cond
TP-AVG
TP1
TP2
TP3
Vacuum-c
Vacuum-P

FIG. 34

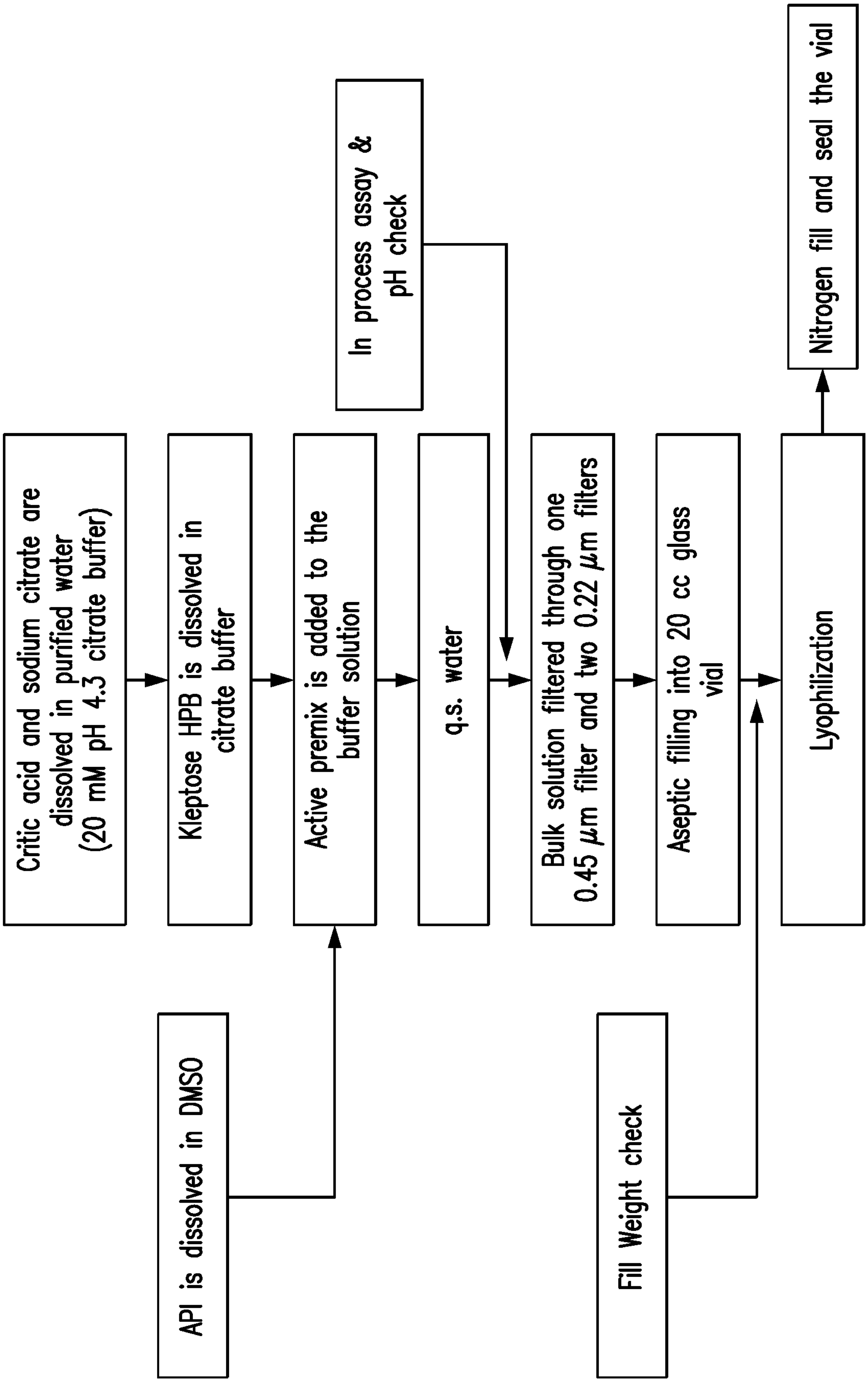
Critic acid and sodium citrate are dissolved in purified water (20 mM pH 4.3 citrate buffer)
Kleptose HPB is dissolved in citrate buffer
API is dissolved in DMSO
Active premix is added to the buffer solution
q.s. water
In process assay & pH check
Bulk solution filtered through one 0.45 μm filter and two 0.22 μm filters
Aseptic filling into 20 cc glass vial
Fill Weight check
Lyophilization
Nitrogen fill and seal the vial

FIG. 35

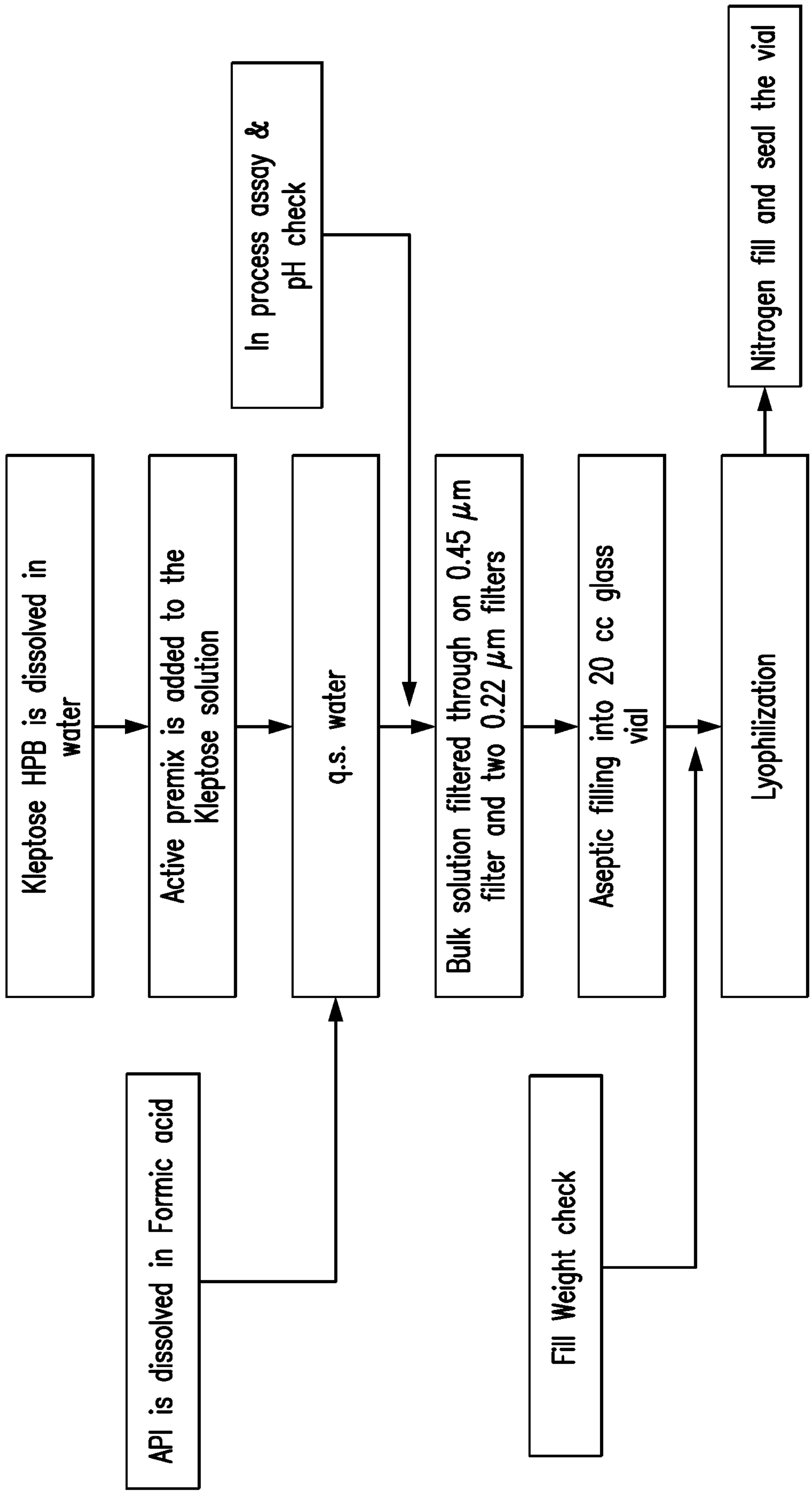
Kleptose HPB is dissolved in water
Active premix is added to the Kleptose solution
API is dissolved in Formic acid
q.s. water
In process assay & pH check
Bulk solution filtered through on 0.45 μm filter and two 0.22 μm filters
Aseptic filling into 20 cc glass vial
Fill Weight check
Lyophilization
Nitrogen fill and seal the vial

FIG. 36

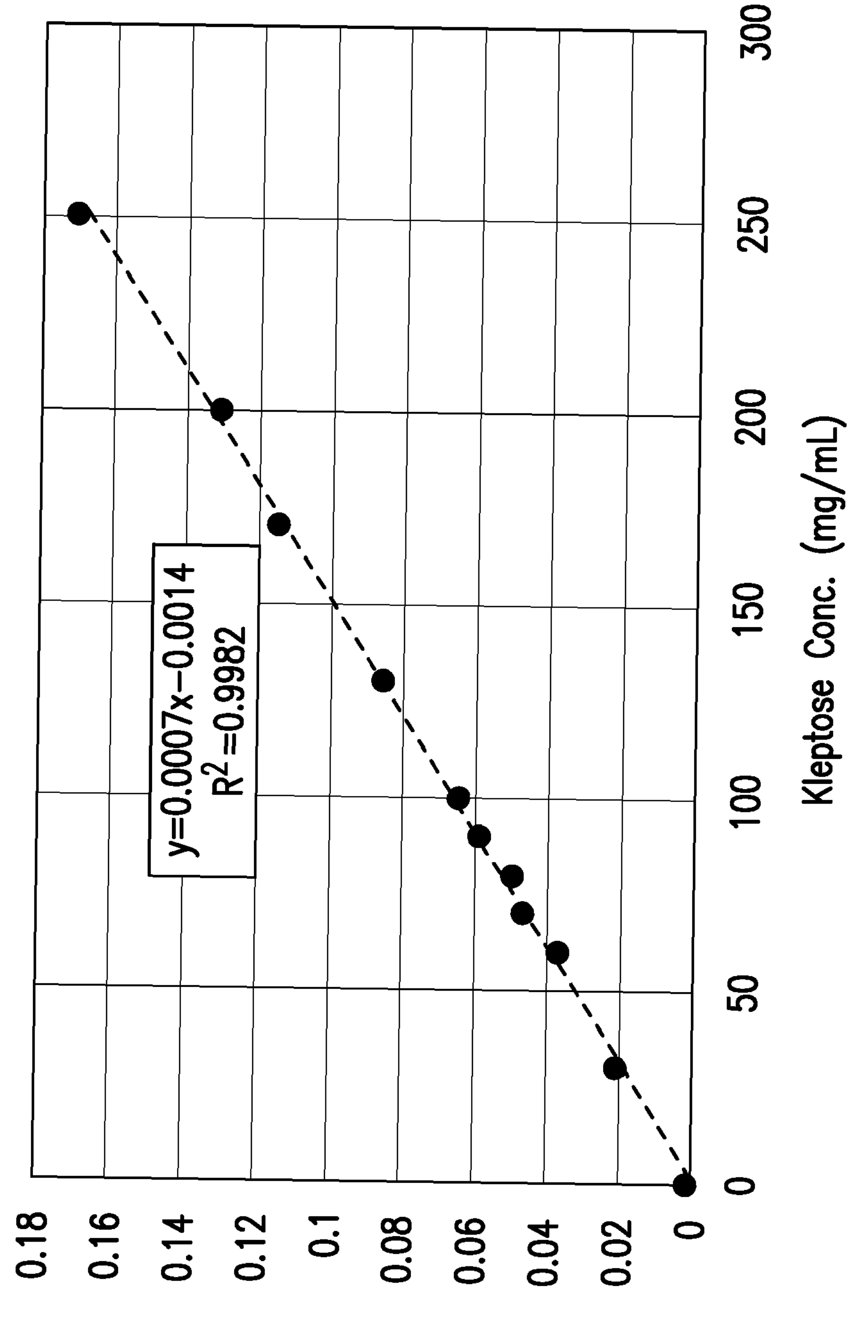

y=0.0007x−0.0014
$R^2$=0.9982
Compound 1 solubility (mg/mL)
0
0.02
0.04
0.06
0.08
0.1
0.12
0.14
0.16
0.18
Kleptose Conc. (mg/mL)
0
50
100
150
200
250
300


FIG. 37

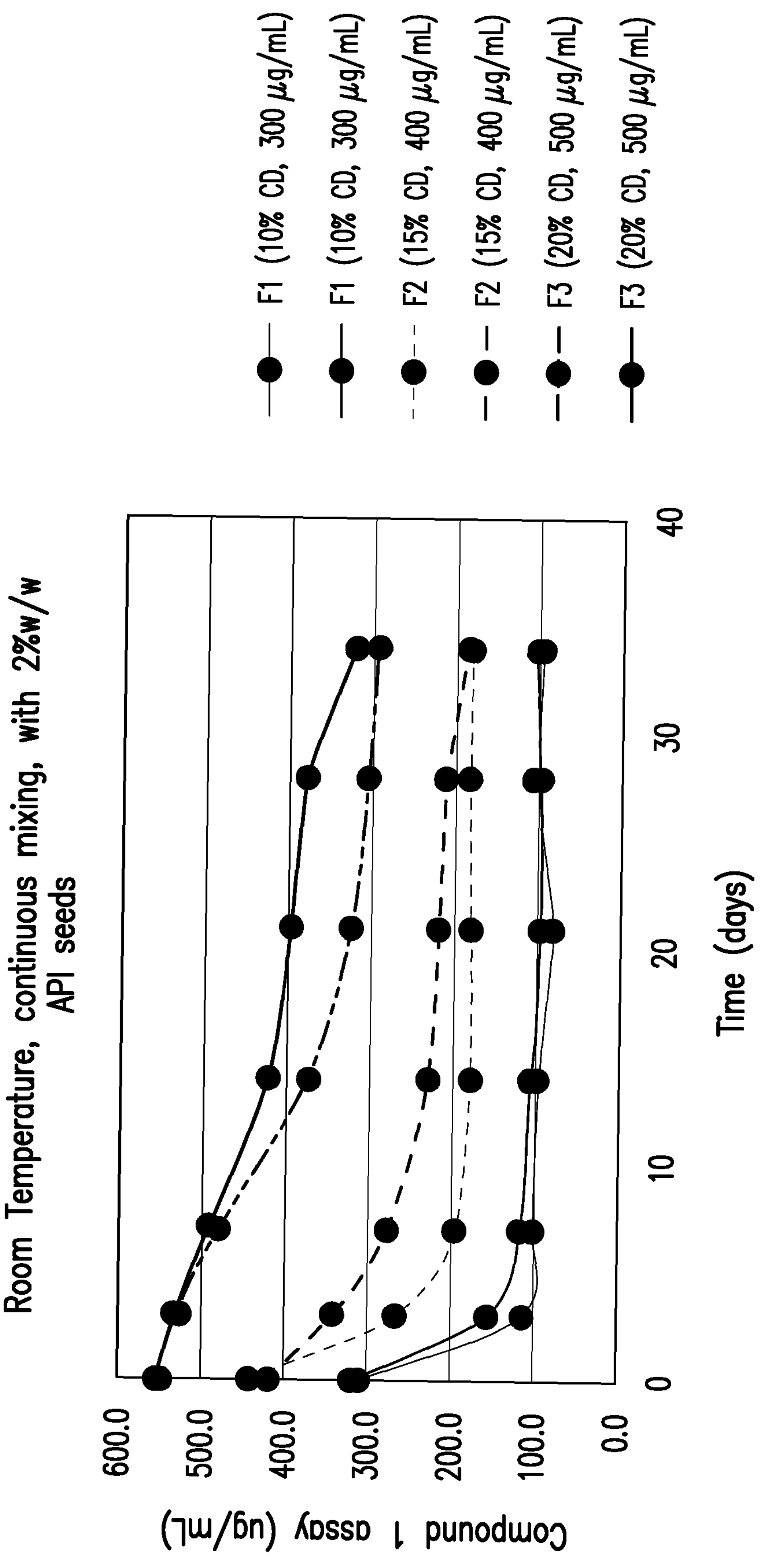
Room Temperature, continuous mixing, with 2%w/w
API seeds
Compound 1 assay (ug/mL)
600.0
500.0
400.0
300.0
200.0
100.0
0.0
0
10
20
30
40
Time (days)
F1 (10% CD, 300 μg/mL)
F1 (10% CD, 300 μg/mL)
F2 (15% CD, 400 μg/mL)
F2 (15% CD, 400 μg/mL)
F3 (20% CD, 500 μg/mL)
F3 (20% CD, 500 μg/mL)

FIG. 38

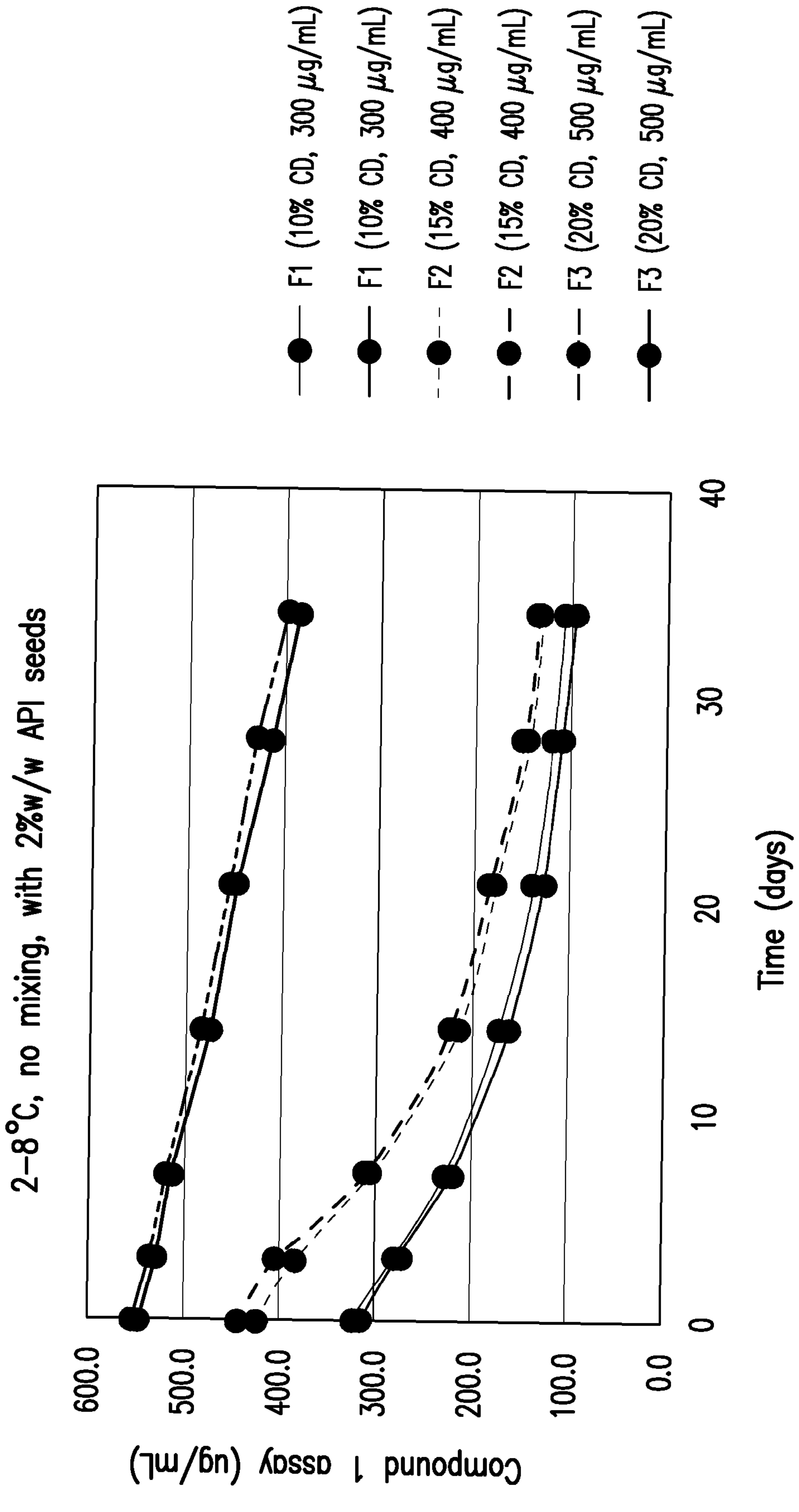
2–8°C, no mixing, with 2%w/w API seeds
Compound 1 assay (ug/mL)
600.0
500.0
400.0
300.0
200.0
100.0
0.0
0
10
20
30
40
Time (days)
F1 (10% CD, 300 μg/mL)
F1 (10% CD, 300 μg/mL)
F2 (15% CD, 400 μg/mL)
F2 (15% CD, 400 μg/mL)
F3 (20% CD, 500 μg/mL)
F3 (20% CD, 500 μg/mL)

FIG. 39

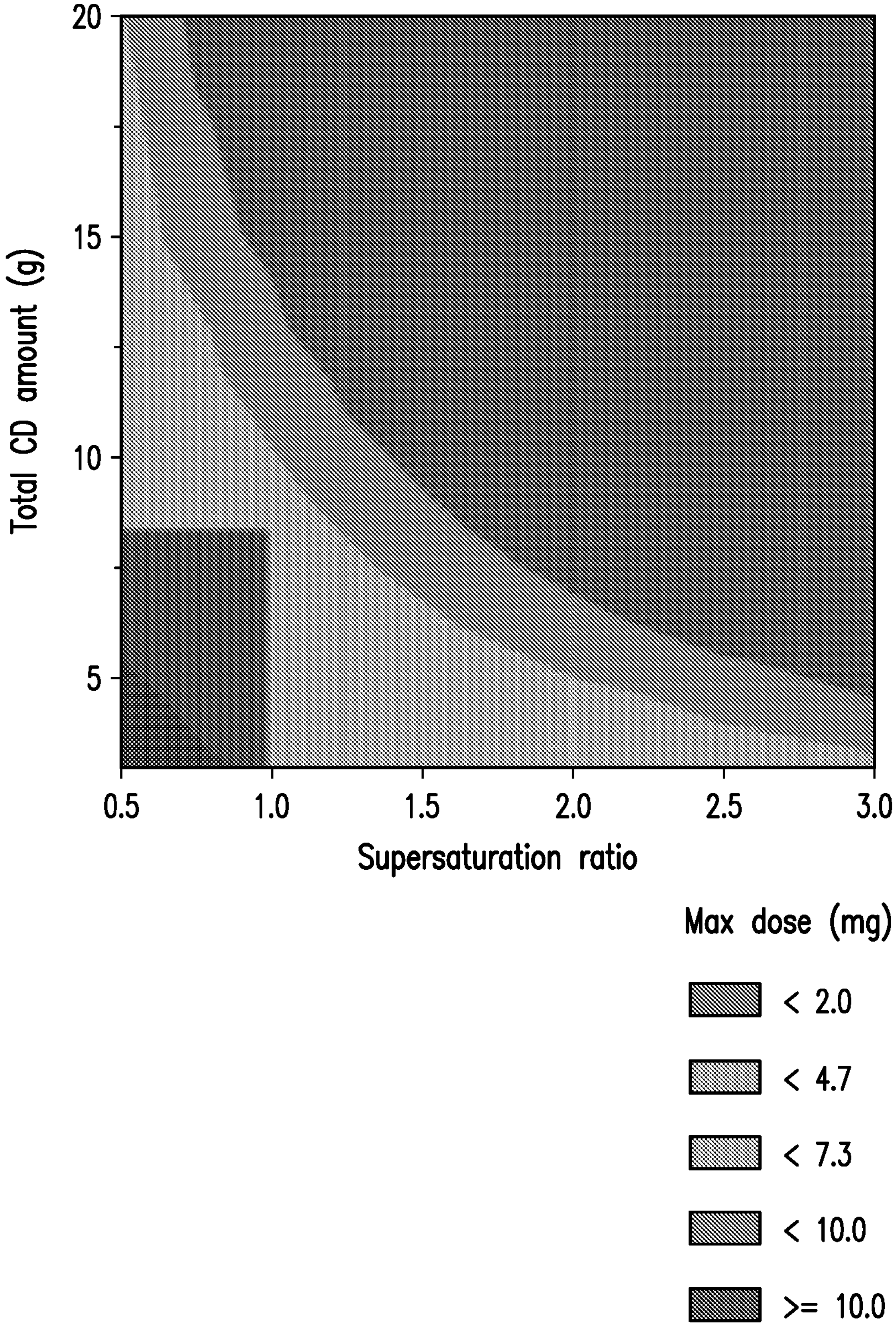
20
15
10
5
Total CD amount (g)
0.5
1.0
1.5
2.0
2.5
3.0
Supersaturation ratio
Max dose (mg)
< 2.0
< 4.7
< 7.3
< 10.0
>= 10.0

FIG. 40

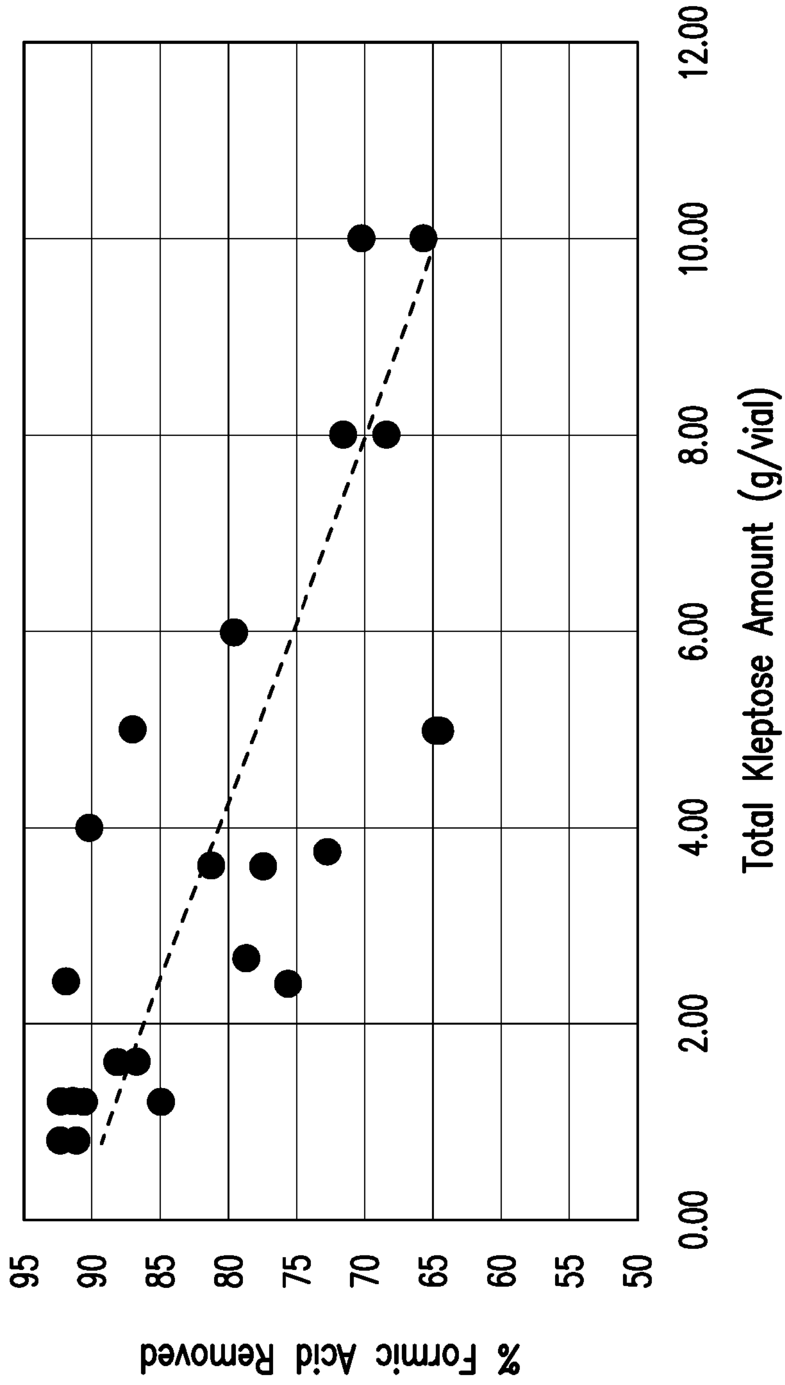
% Formic Acid Removed
95
90
85
80
75
70
65
60
55
50
0.00
2.00
4.00
6.00
8.00
10.00
12.00
Total Kleptose Amount (g/vial)

FIG. 41

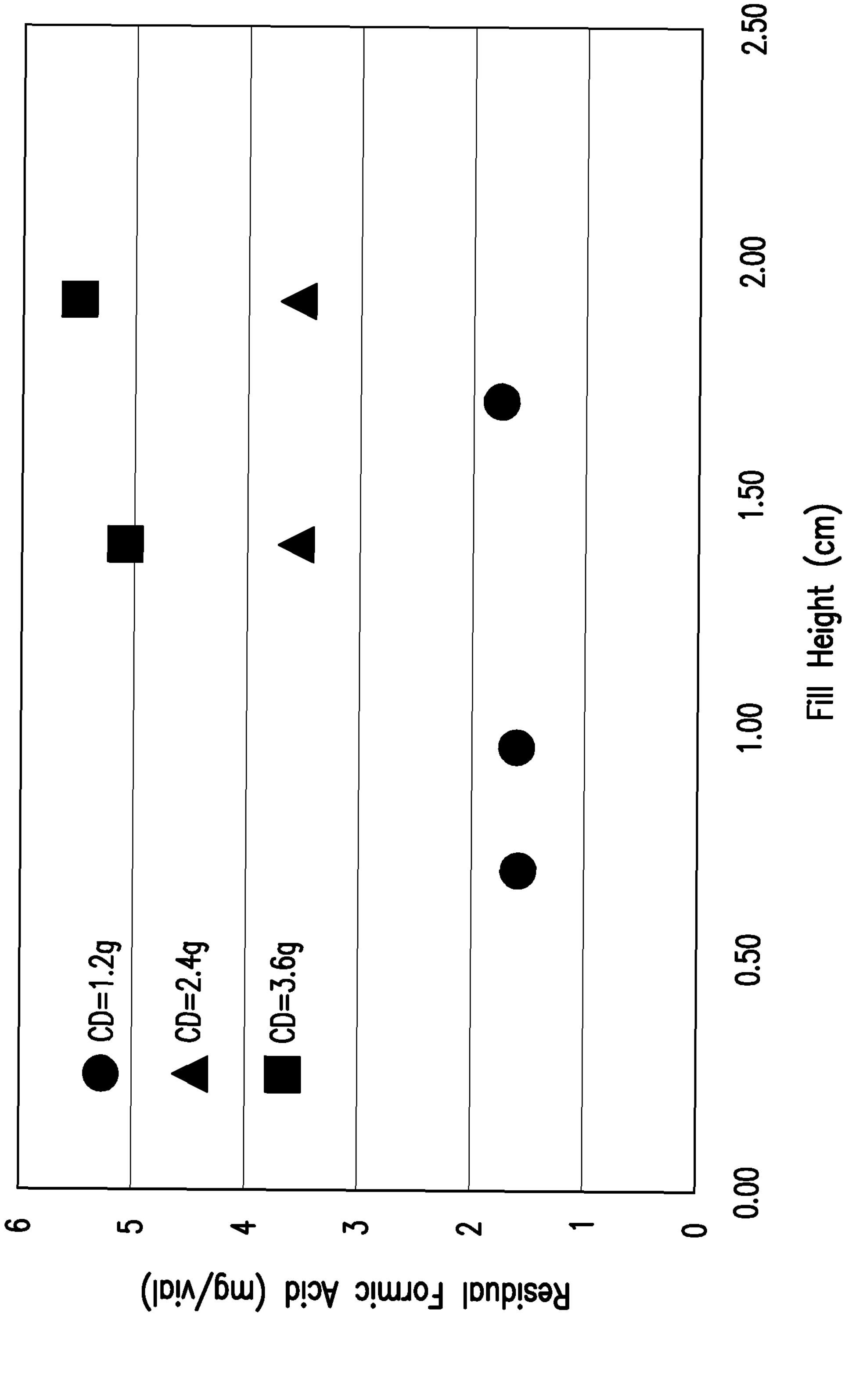
Residual Formic Acid (mg/vial)
0
1
2
3
4
5
6
Fill Height (cm)
0.00
0.50
1.00
1.50
2.00
2.50
CD=1.2g
CD=2.4g
CD=3.6g

FIG. 42

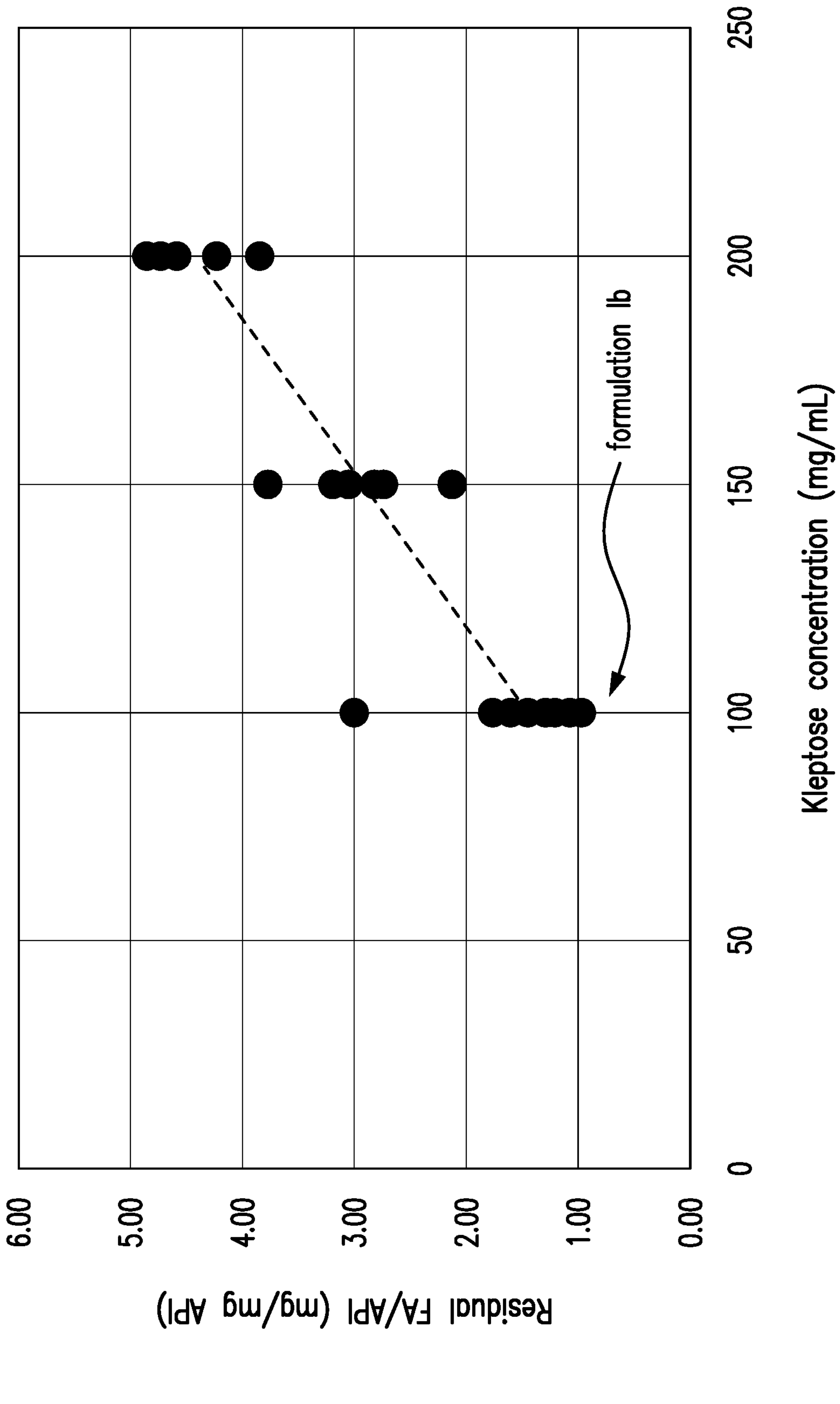
Residual FA/API (mg/mg API)
6.00
5.00
4.00
3.00
2.00
1.00
0.00
0
50
100
150
200
250
Kleptose concentration (mg/mL)
formulation Ib

FIG. 43

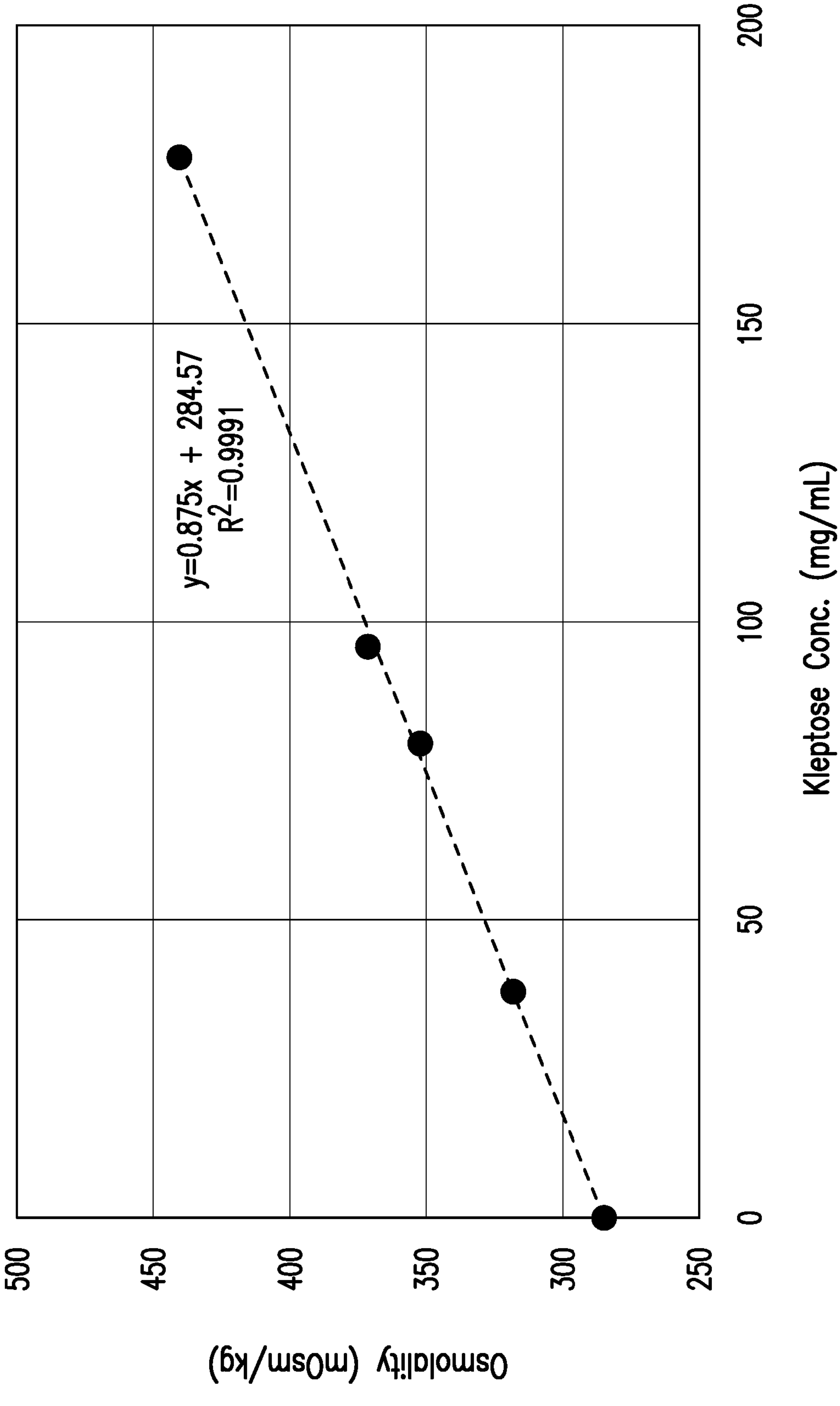
y=0.875x + 284.57
$R^2$=0.9991
Osmolality (mOsm/kg)
500
450
400
350
300
250
Kleptose Conc. (mg/mL)
0
50
100
150
200

FIG. 44

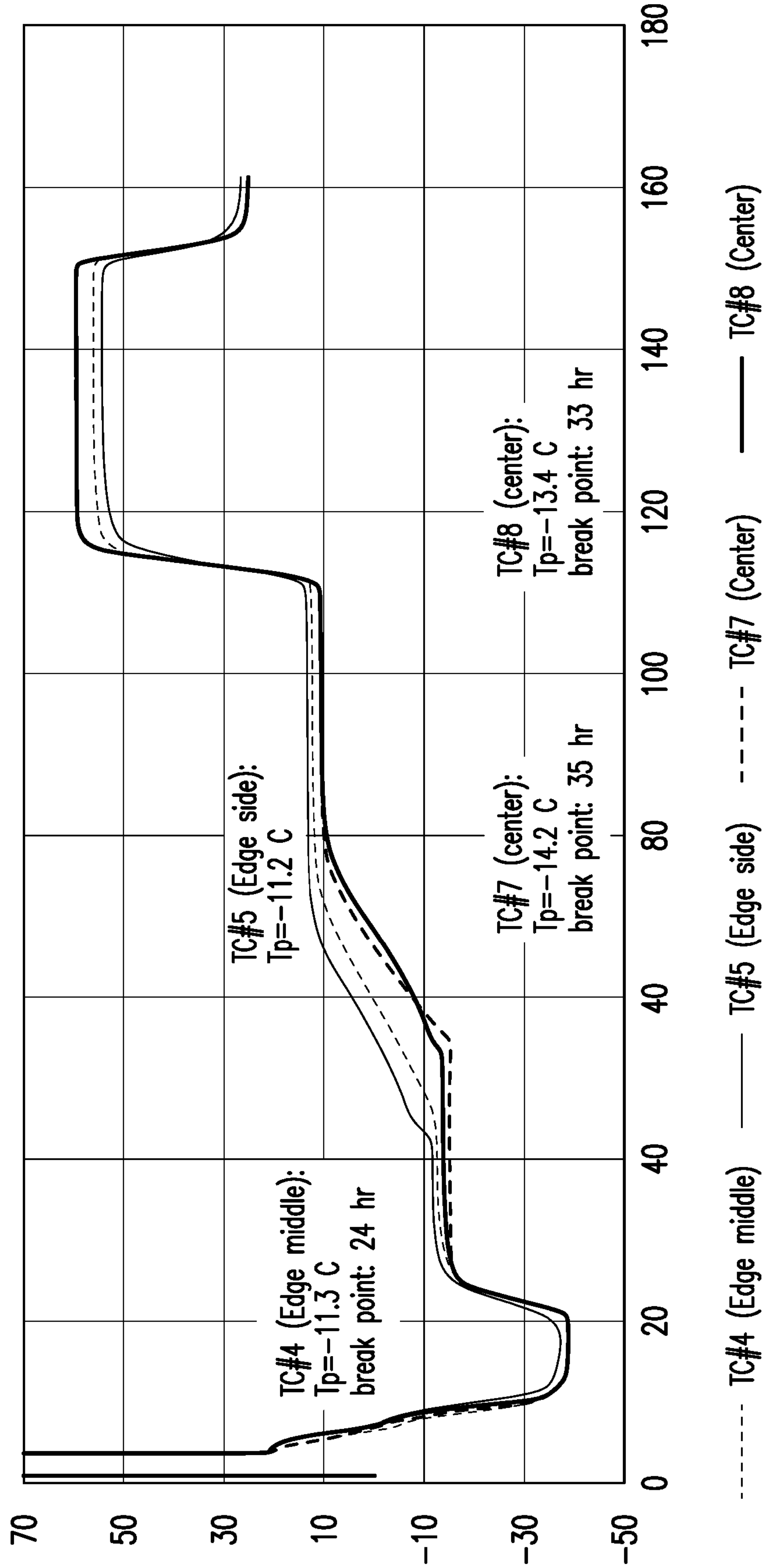
70
50
30
10
−10
−30
−50
0
20
40
40
80
100
120
140
160
180
TC#4 (Edge middle):
Tp=−11.3 C
break point: 24 hr
TC#5 (Edge side):
Tp=−11.2 C
TC#7 (center):
Tp=−14.2 C
break point: 35 hr
TC#8 (center):
Tp=−13.4 C
break point: 33 hr
TC#4 (Edge middle)
TC#5 (Edge side)
TC#7 (Center)
TC#8 (Center)

FIG. 45

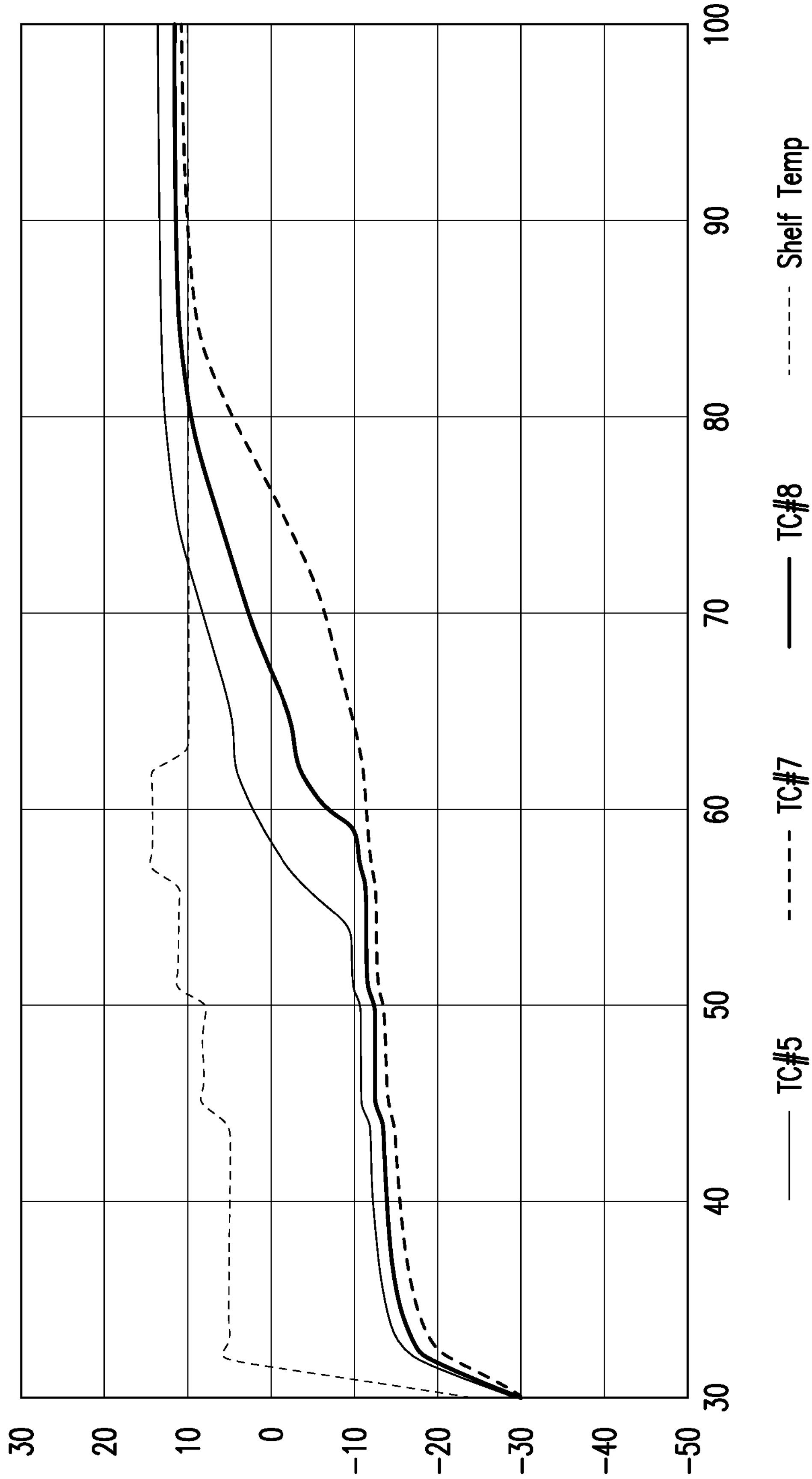
30
20
10
0
−10
−20
−30
−40
−50
30
40
50
60
70
80
90
100
TC#5
TC#7
TC#8
Shelf Temp

FIG. 46

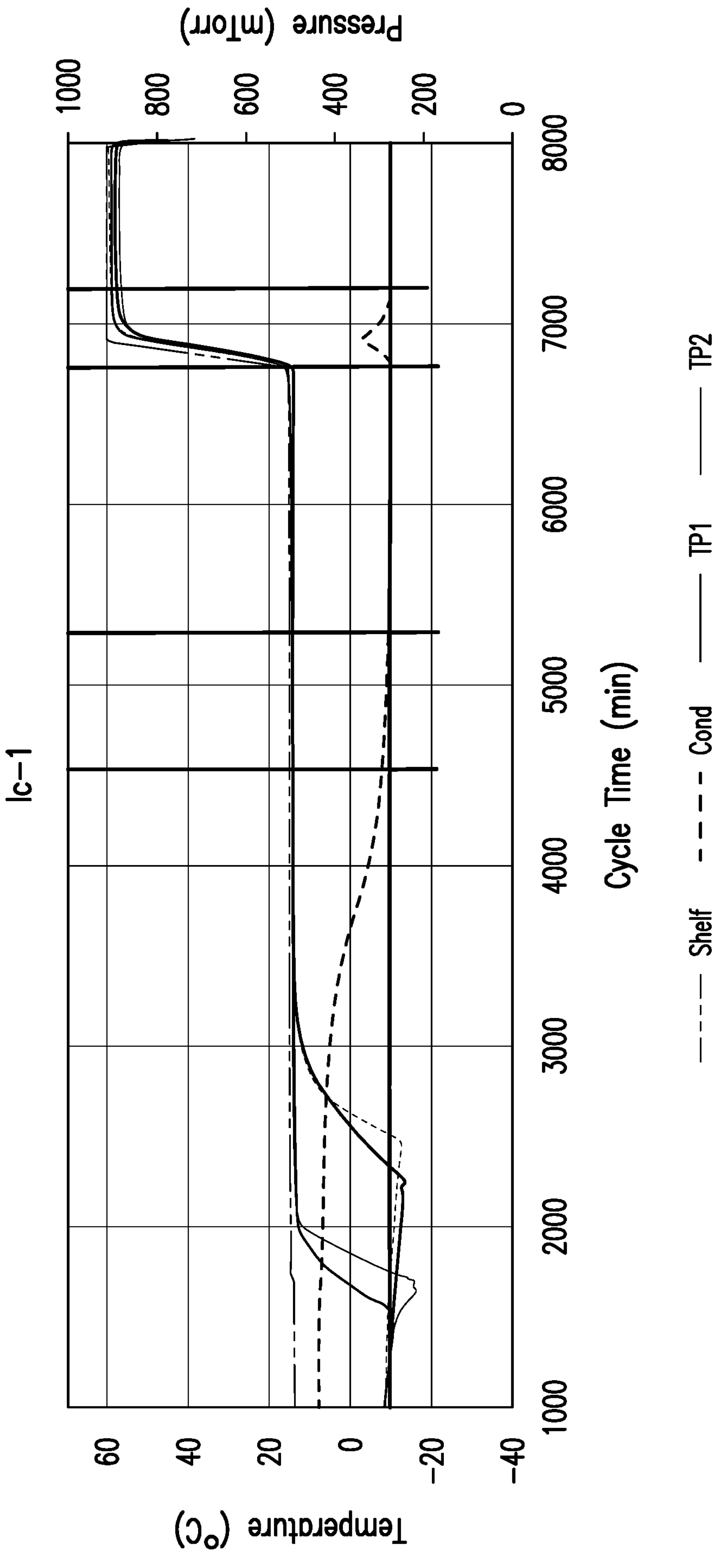
Ic–1
Pressure (mTorr)
1000
800
600
400
200
0
Temperature (°C)
60
40
20
0
–20
–40
1000
2000
3000
4000
5000
6000
7000
8000
Cycle Time (min)
Shelf
Cond
TP1
TP2

FIG. 47

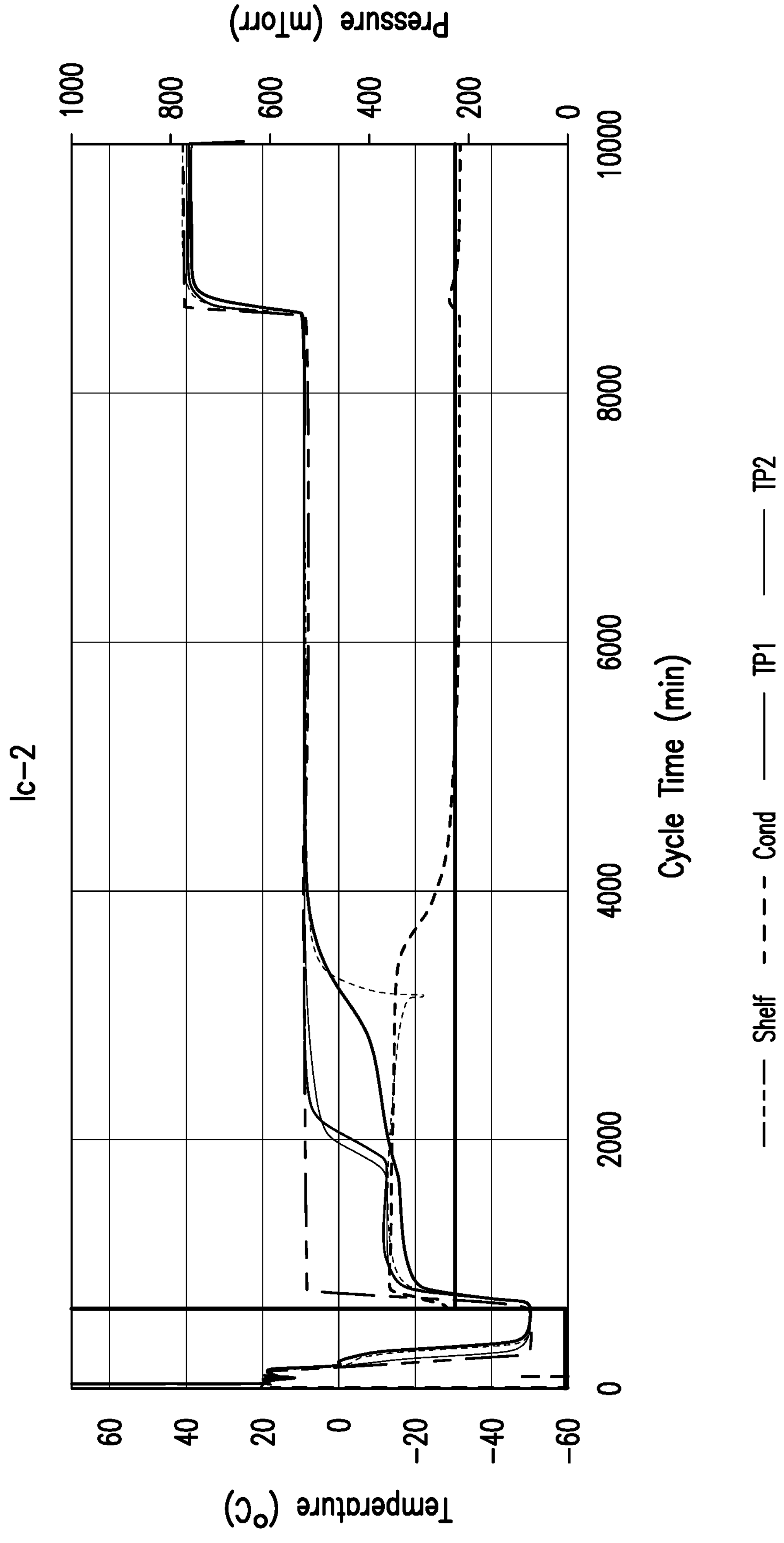
Ic–2
Pressure (mTorr)
1000
800
600
400
200
0
Temperature (°C)
60
40
20
0
–20
–40
–60
0
2000
4000
6000
8000
10000
Cycle Time (min)
Shelf
Cond
TP1
TP2

FIG. 48

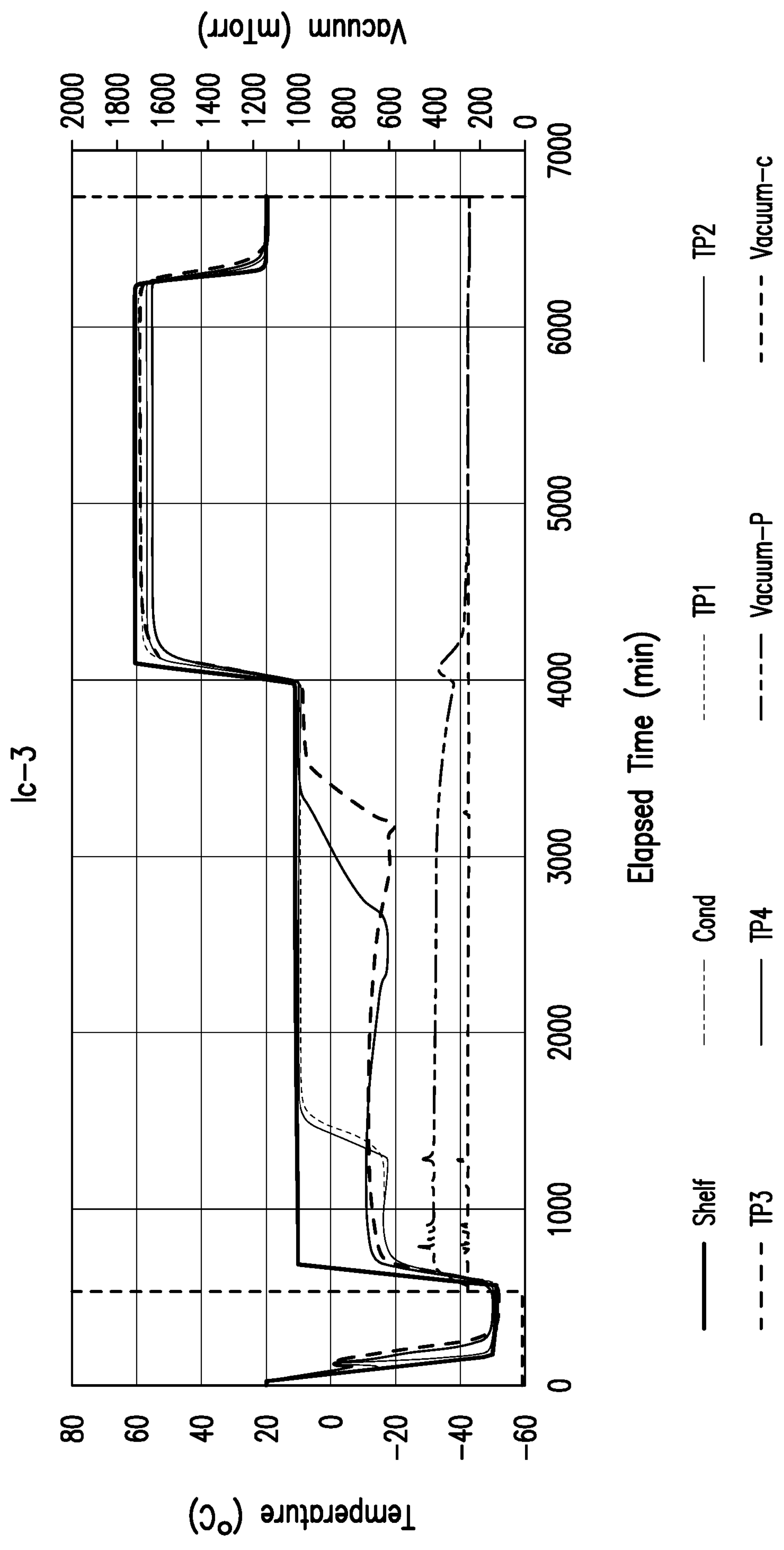
Ic–3
Vacuum (mTorr)
2000
1800
1600
1400
1200
1000
800
600
400
200
0
Temperature (°C)
80
60
40
20
0
–20
–40
–60
0
1000
2000
3000
4000
5000
6000
7000
Elapsed Time (min)
Shelf
Cond
TP1
TP2
TP3
TP4
Vacuum–P
Vacuum–c

FIG. 49

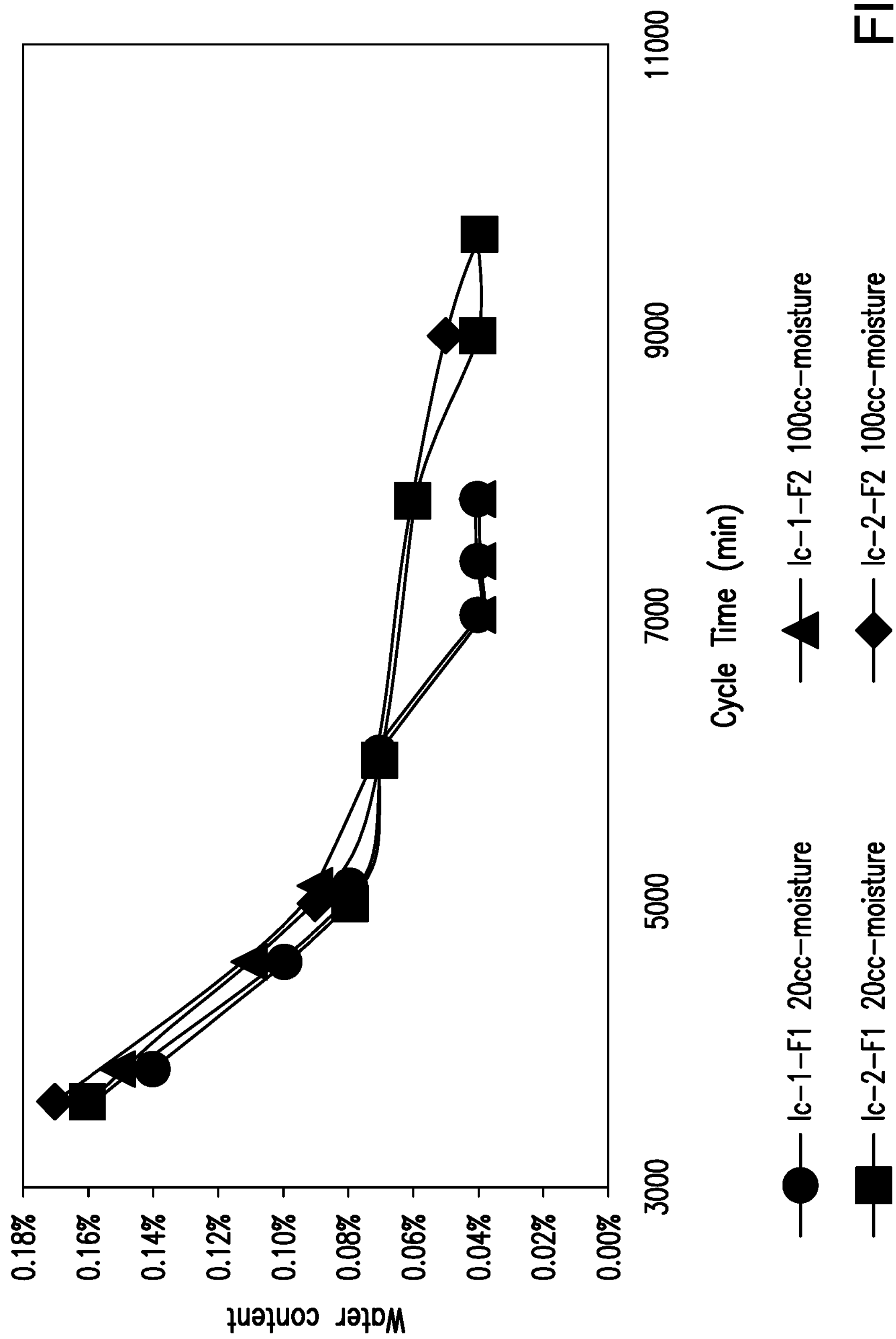
Water content
0.18%
0.16%
0.14%
0.12%
0.10%
0.08%
0.06%
0.04%
0.02%
0.00%
3000
5000
7000
9000
11000
Cycle Time (min)
Ic−1−F1 20cc−moisture
Ic−1−F2 100cc−moisture
Ic−2−F1 20cc−moisture
Ic−2−F2 100cc−moisture

FIG. 50

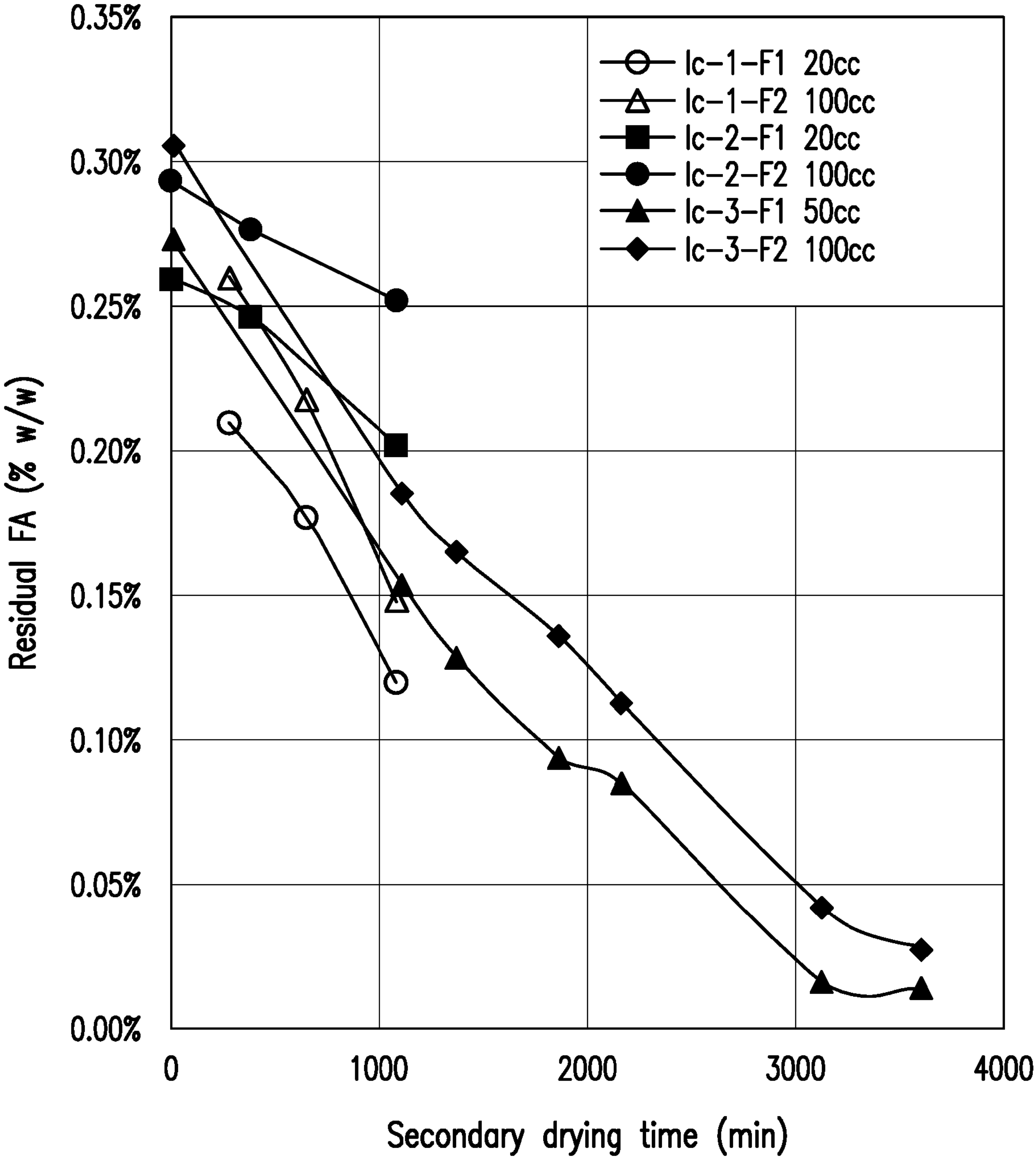
0.35%
0.30%
0.25%
0.20%
0.15%
0.10%
0.05%
0.00%
0
1000
2000
3000
4000
Residual FA (% w/w)
Secondary drying time (min)
Ic-1-F1 20cc
Ic-1-F2 100cc
Ic-2-F1 20cc
Ic-2-F2 100cc
Ic-3-F1 50cc
Ic-3-F2 100cc

FIG. 51

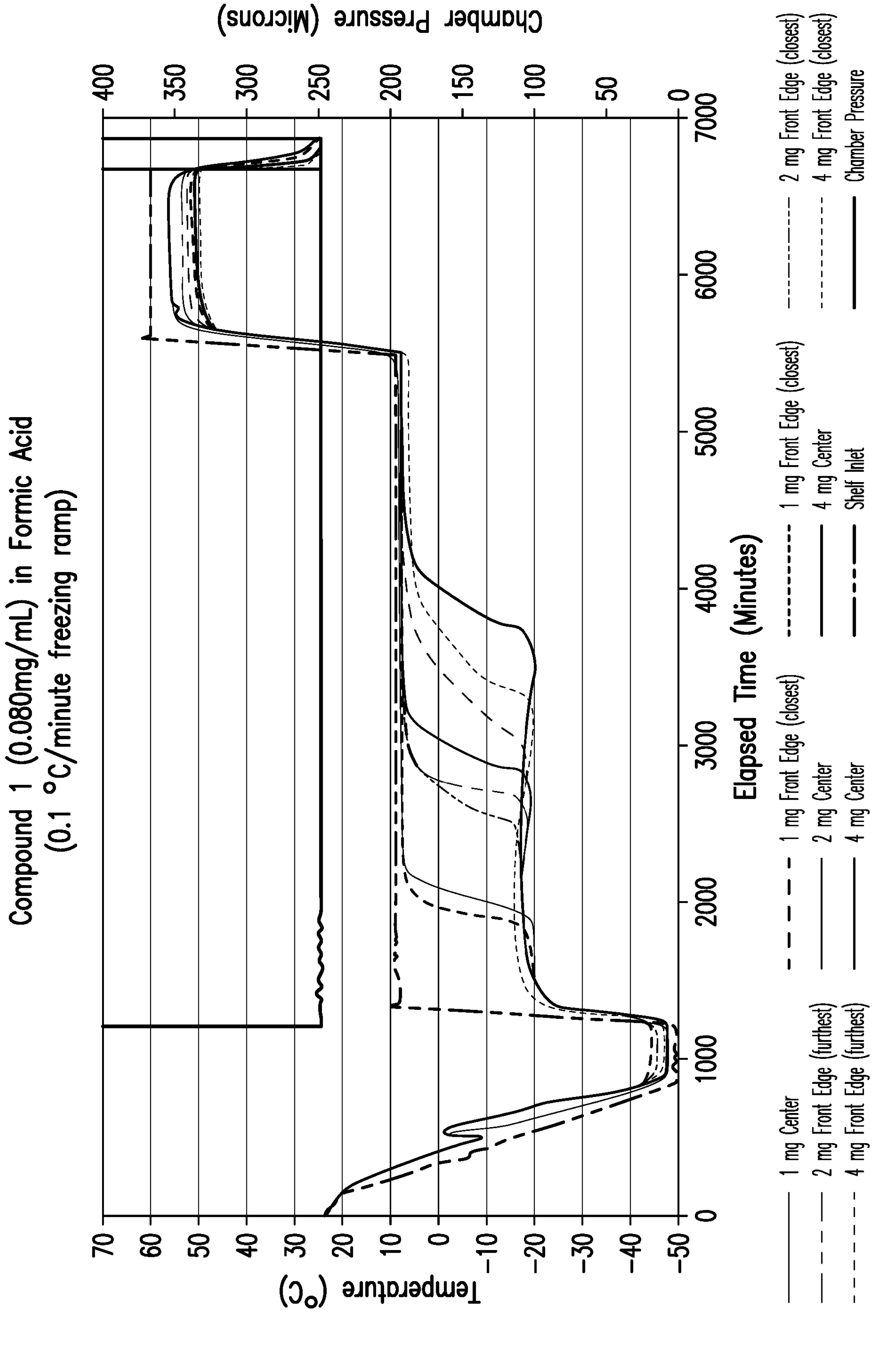
Compound 1 (0.080mg/mL) in Formic Acid
(0.1 °C/minute freezing ramp)
Temperature (°C)
70
60
50
40
30
20
10
0
-10
-20
-30
-40
-50
Chamber Pressure (Microns)
400
350
300
250
200
150
100
50
0
Elapsed Time (Minutes)
0
1000
2000
3000
4000
5000
6000
7000
1 mg Center
2 mg Front Edge (furthest)
4 mg Front Edge (furthest)
1 mg Front Edge (closest)
2 mg Center
4 mg Center
1 mg Front Edge (closest)
4 mg Center
Shelf Inlet
2 mg Front Edge (closest)
4 mg Front Edge (closest)
Chamber Pressure

FIG. 52

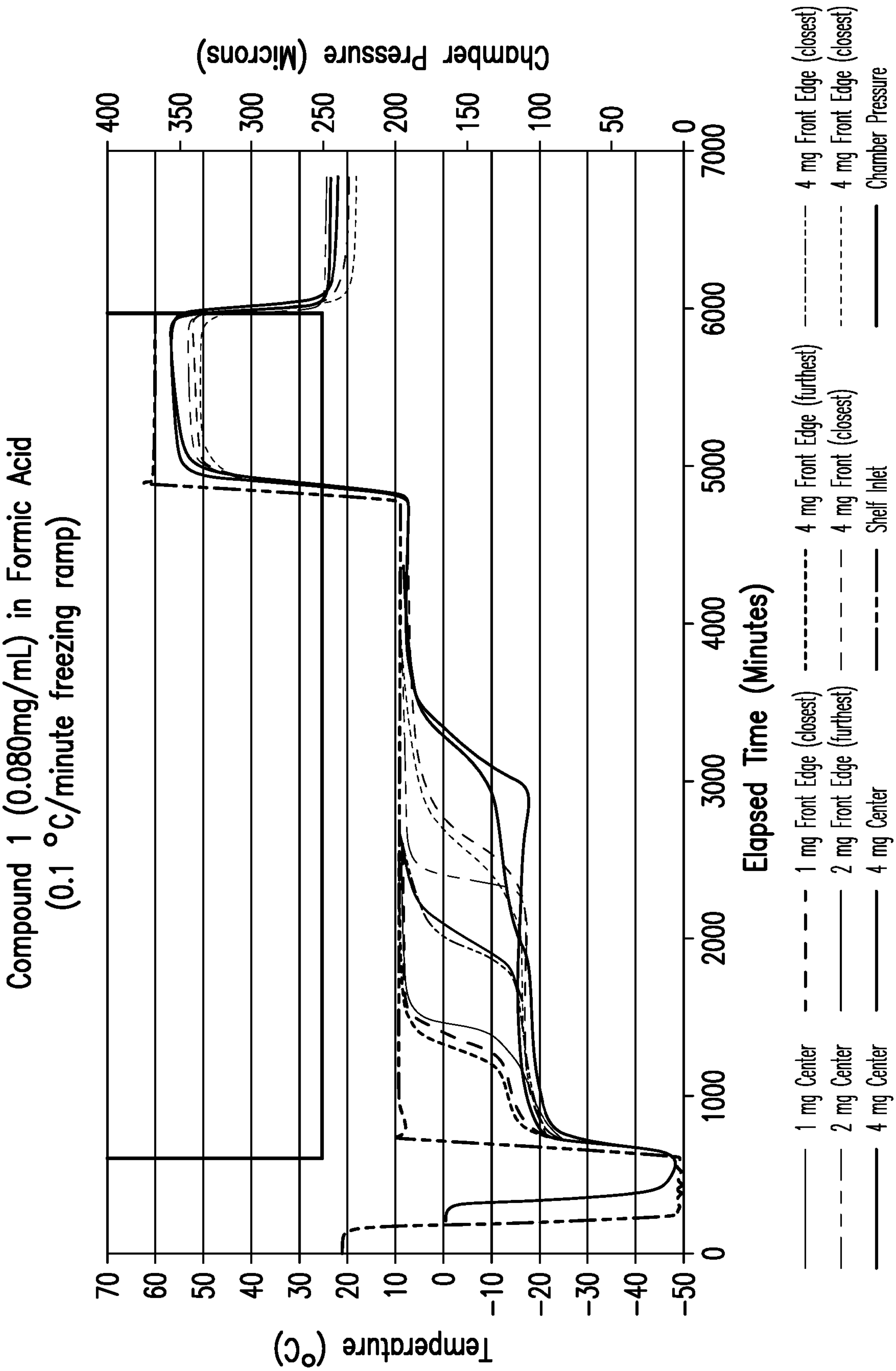
Compound 1 (0.080mg/mL) in Formic Acid
(0.1 °C/minute freezing ramp)
Temperature (°C)
70
60
50
40
30
20
10
0
-10
-20
-30
-40
-50
Chamber Pressure (Microns)
400
350
300
250
200
150
100
50
0
Elapsed Time (Minutes)
0
1000
2000
3000
4000
5000
6000
7000
1 mg Center
2 mg Center
4 mg Center
1 mg Front Edge (closest)
2 mg Front Edge (furthest)
4 mg Center
4 mg Front Edge (furthest)
4 mg Front (closest)
Shelf Inlet
4 mg Front Edge (closest)
4 mg Front Edge (closest)
Chamber Pressure

FIG. 53

| | | | |
|---|---|---|---|
| Picture (Batch C1) | 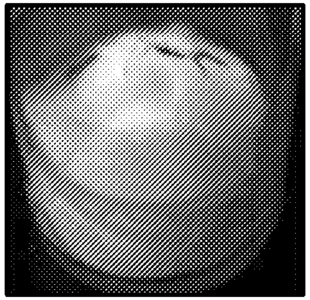 1 mg/vial | 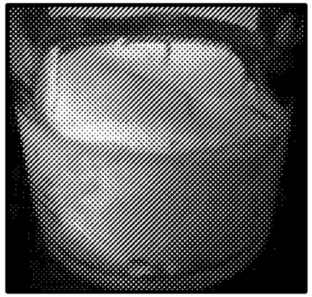 2 mg/vial | 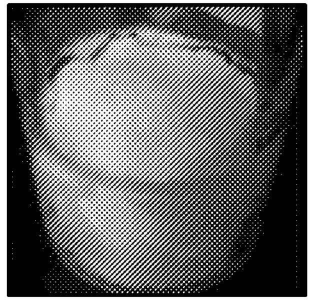 4 mg/vial |
| Picture (Batch C2) | 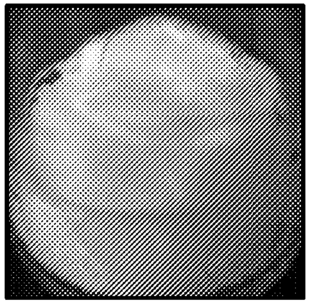 1 mg/vial | 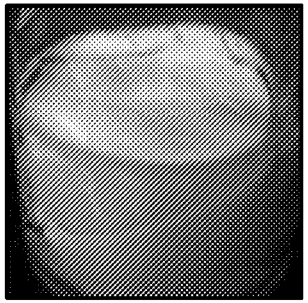 2 mg/vial | 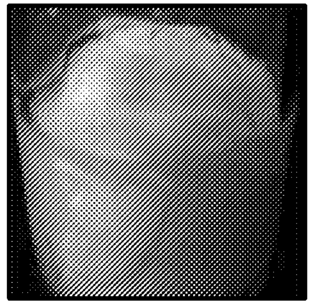 4 mg/vial |

FIG. 54

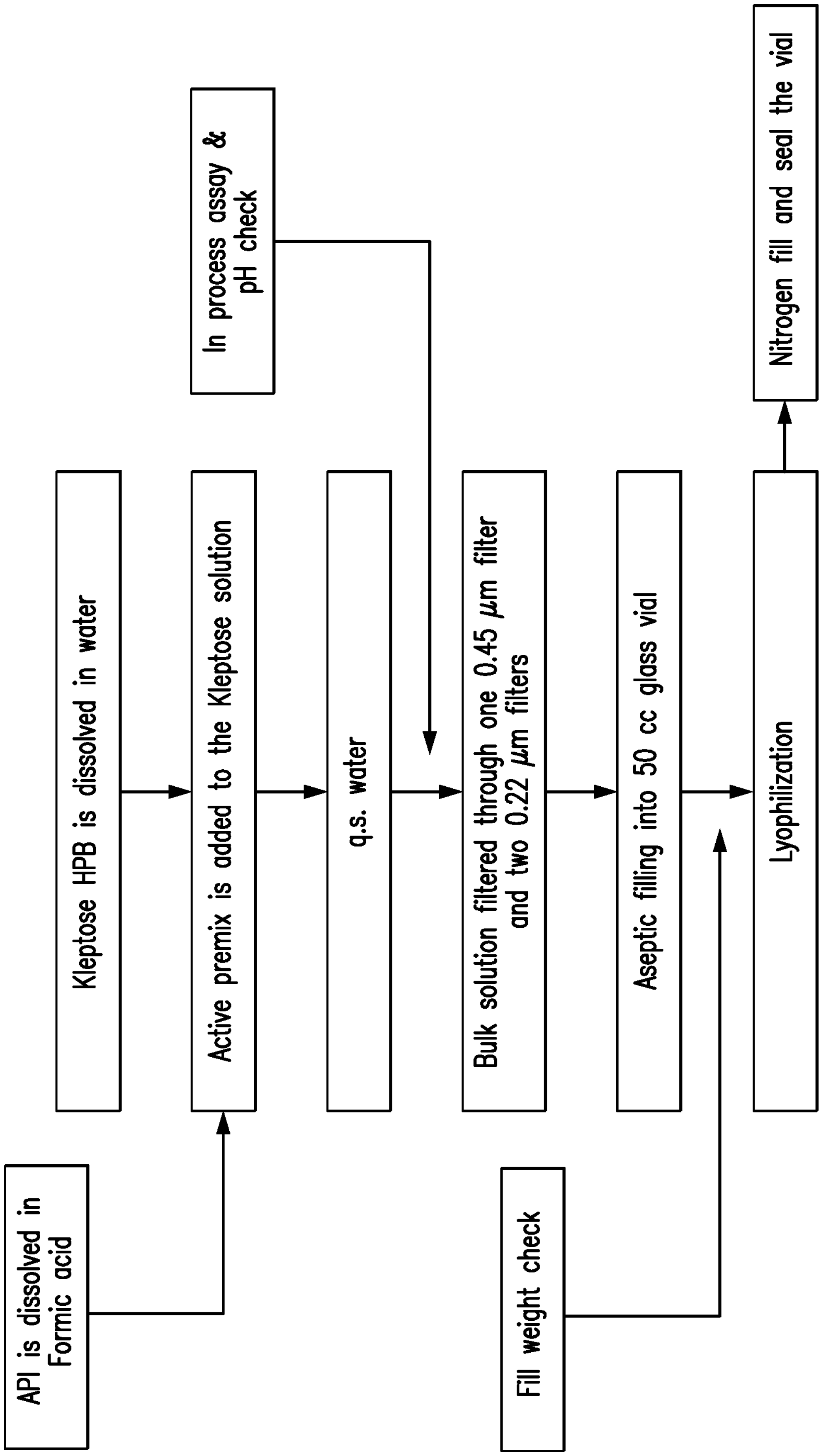

Kleptose HPB is dissolved in water
API is dissolved in Formic acid
Active premix is added to the Kleptose solution
q.s. water
In process assay & pH check
Bulk solution filtered through one 0.45 μm filter and two 0.22 μm filters
Aseptic filling into 50 cc glass vial
Fill weight check
Lyophilization
Nitrogen fill and seal the vial


FIG. 55

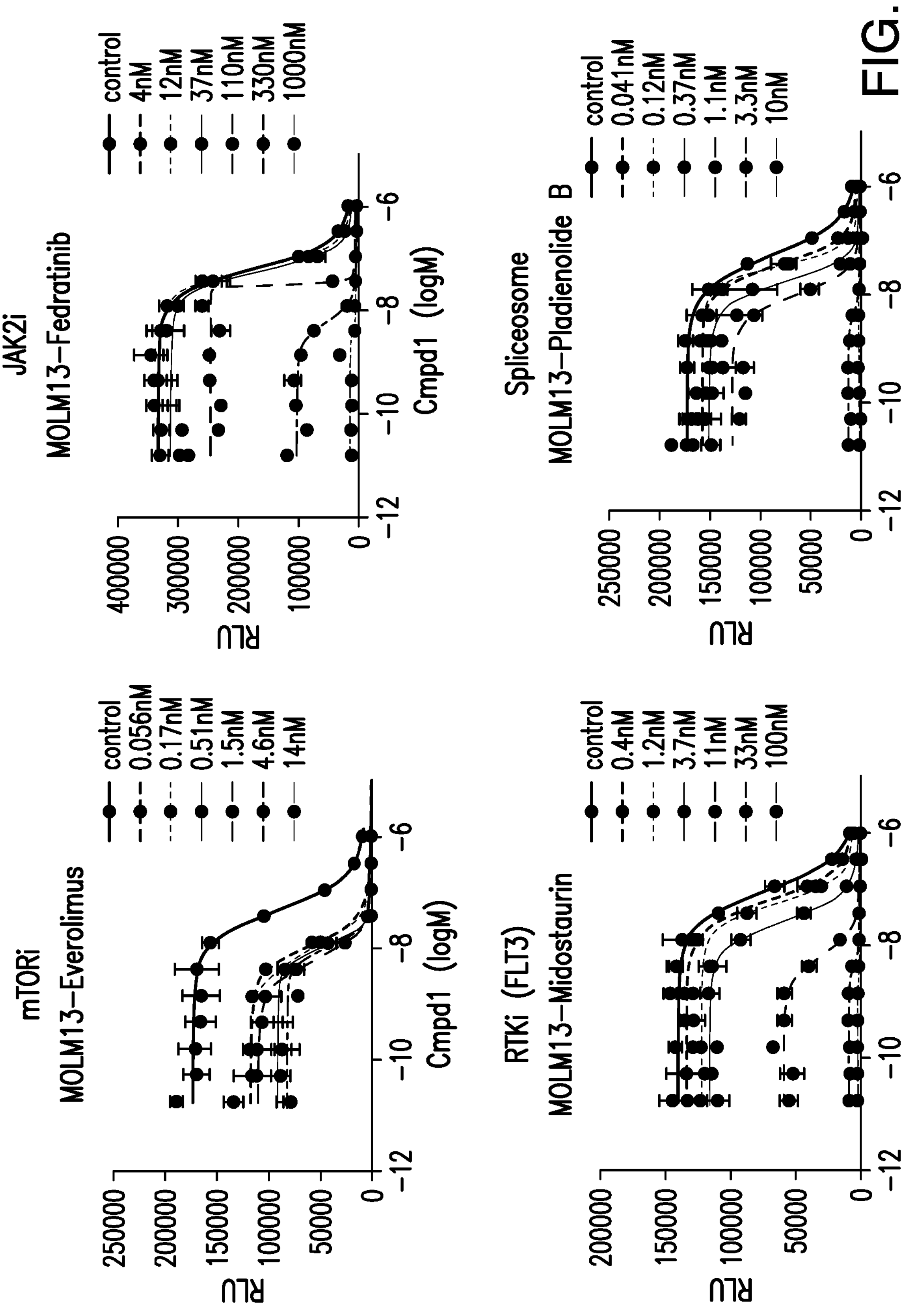
mTORi
MOLM13–Everolimus
RLU
250000
200000
150000
100000
50000
0
−12
−10
−8
−6
Cmpd1 (logM)
control
0.056nM
0.17nM
0.51nM
1.5nM
4.6nM
14nM
JAK2i
MOLM13–Fedratinib
RLU
400000
300000
200000
100000
0
−12
−10
−8
−6
Cmpd1 (logM)
control
4nM
12nM
37nM
110nM
330nM
1000nM
RTKi (FLT3)
MOLM13–Midostaurin
RLU
200000
150000
100000
50000
0
−12
−10
−8
−6
Cmpd1 (logM)
control
0.4nM
1.2nM
3.7nM
11nM
33nM
100nM
Spliceosome
MOLM13–Pladienolide B
RLU
250000
200000
150000
100000
50000
0
−12
−10
−8
−6
Cmpd1 (logM)
control
0.041nM
0.12nM
0.37nM
1.1nM
3.3nM
10nM

FIG. 56A

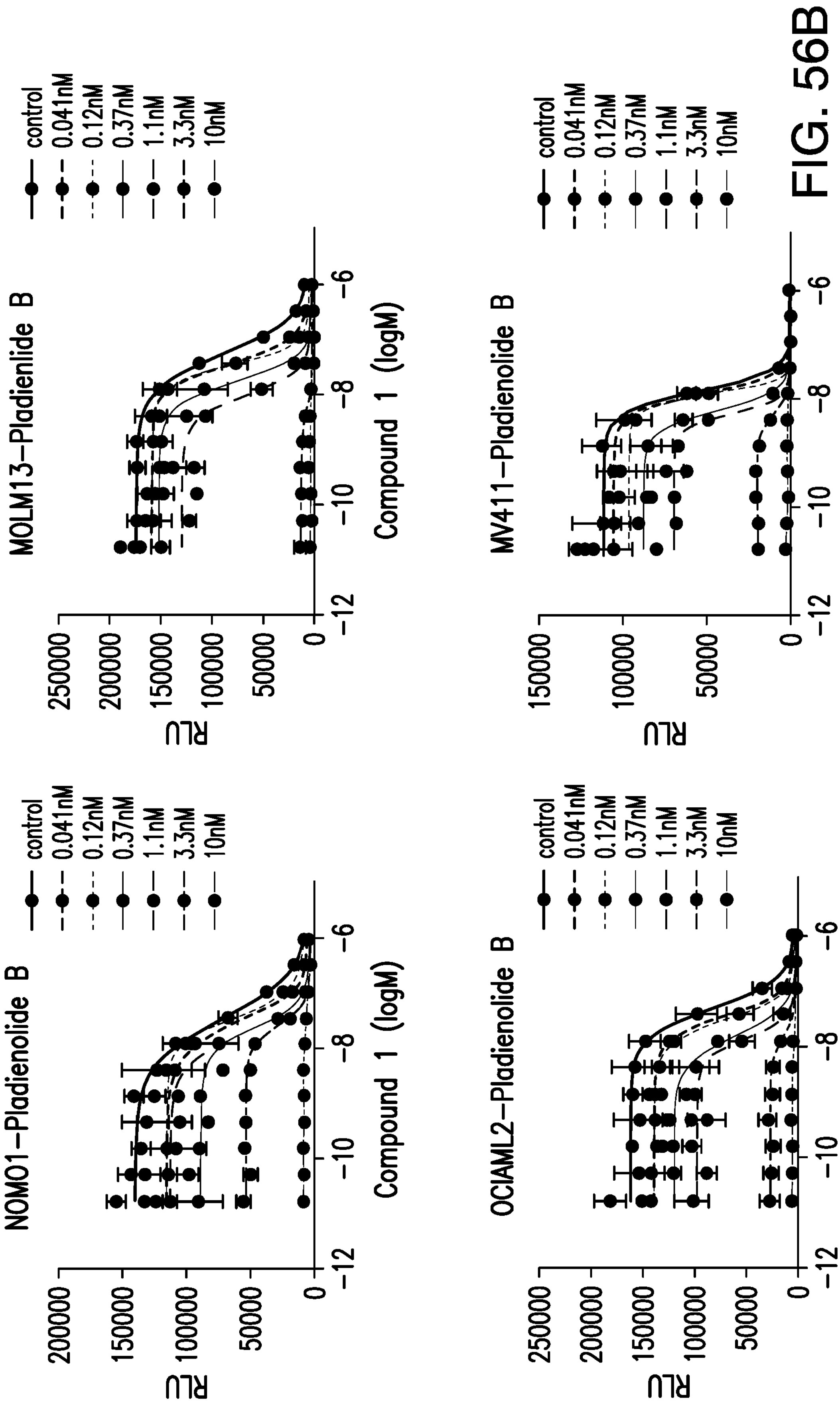
NOMO1–Pladienolide B
OCIAML2–Pladienolide B
MOLM13–Pladienolide B
MV411–Pladienolide B
RLU
Compound 1 (logM)
−12
−10
−8
−6
200000
150000
100000
50000
0
250000
control
0.041nM
0.12nM
0.37nM
1.1nM
3.3nM
10nM

FIG. 56B

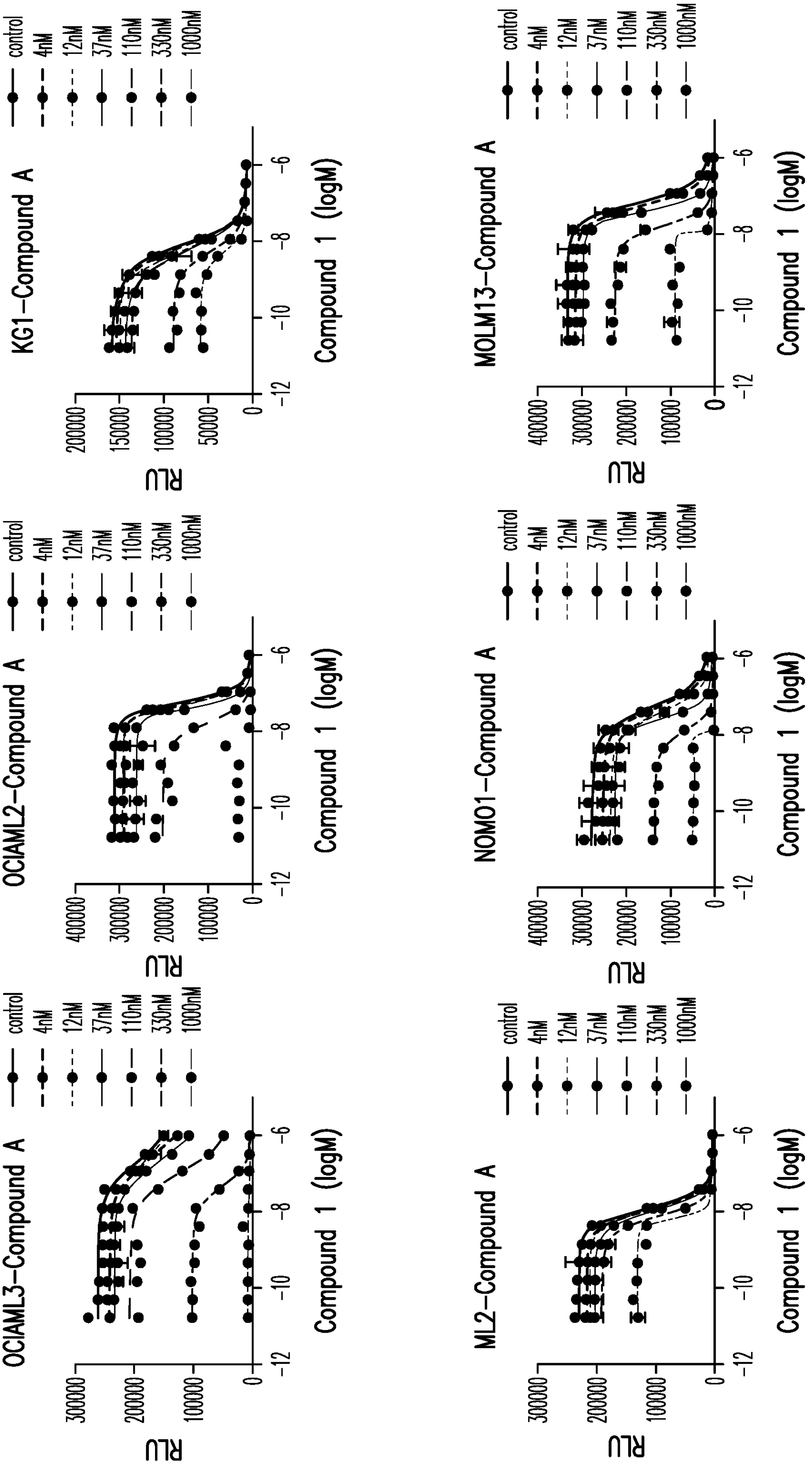
OCIAML3–Compound A
OCIAML2–Compound A
KG1–Compound A
ML2–Compound A
NOMO1–Compound A
MOLM13–Compound A
RLU
Compound 1 (logM)
control
4nM
12nM
37nM
110nM
330nM
1000nM

FIG. 56C

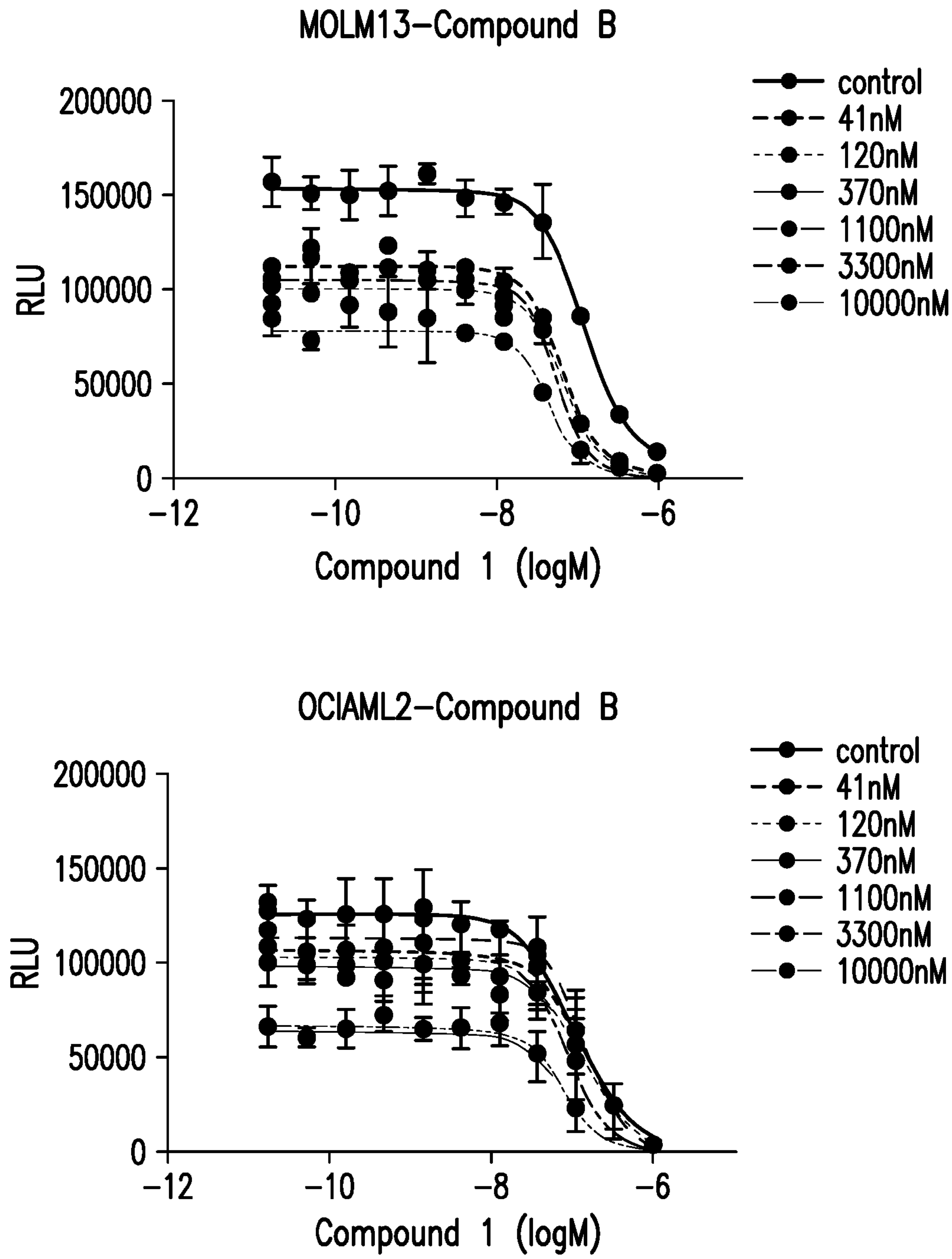
MOLM13-Compound B
control
41nM
120nM
370nM
1100nM
3300nM
10000nM
RLU
200000
150000
100000
50000
0
-12
-10
-8
-6
Compound 1 (logM)
OCIAML2-Compound B
control
41nM
120nM
370nM
1100nM
3300nM
10000nM
RLU
200000
150000
100000
50000
0
-12
-10
-8
-6
Compound 1 (logM)

FIG. 56D

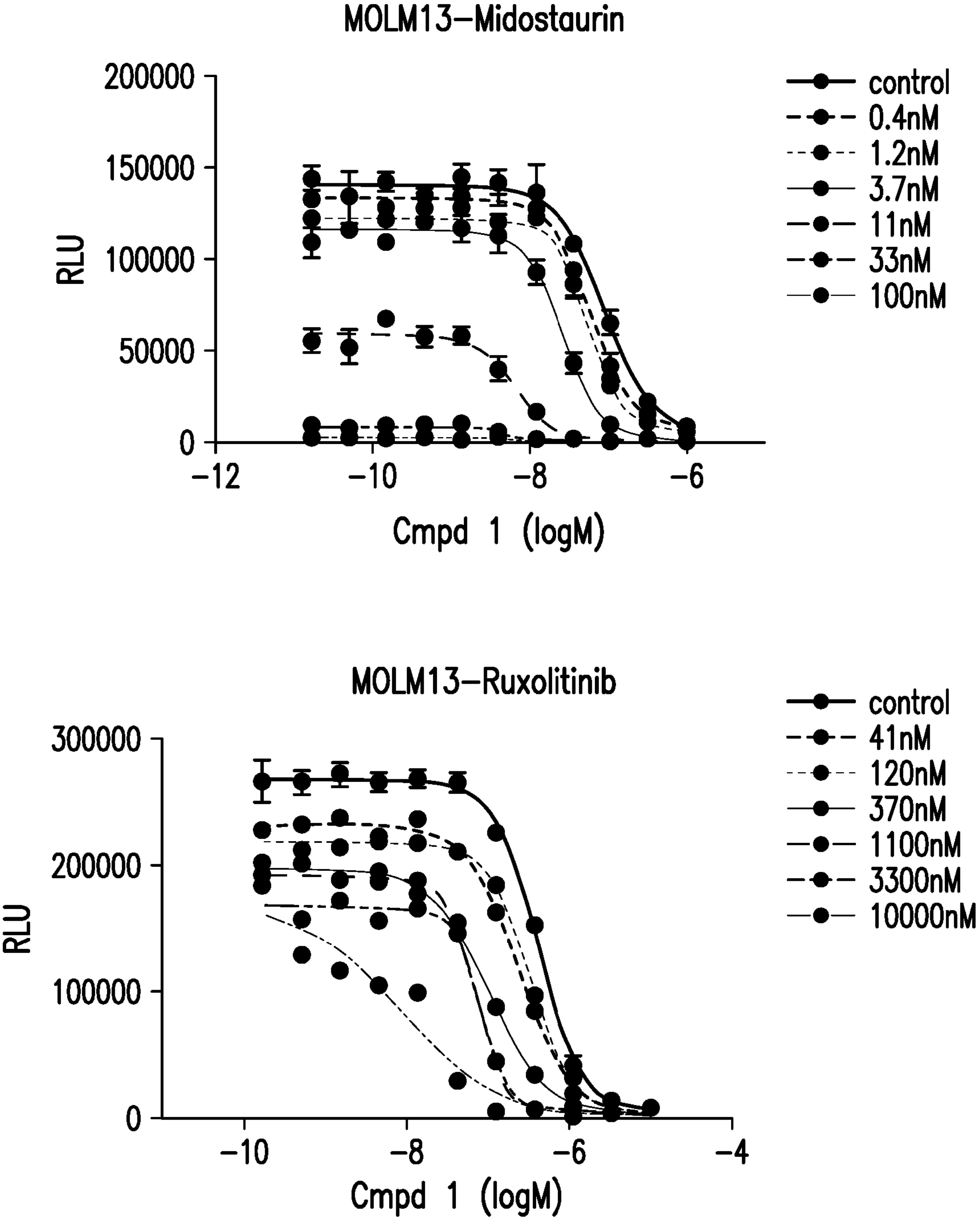
MOLM13–Midostaurin
200000
150000
100000
50000
0
RLU
–12
–10
–8
–6
Cmpd 1 (logM)
control
0.4nM
1.2nM
3.7nM
11nM
33nM
100nM
MOLM13–Ruxolitinib
300000
200000
100000
0
RLU
–10
–8
–6
–4
Cmpd 1 (logM)
control
41nM
120nM
370nM
1100nM
3300nM
10000nM

FIG. 57

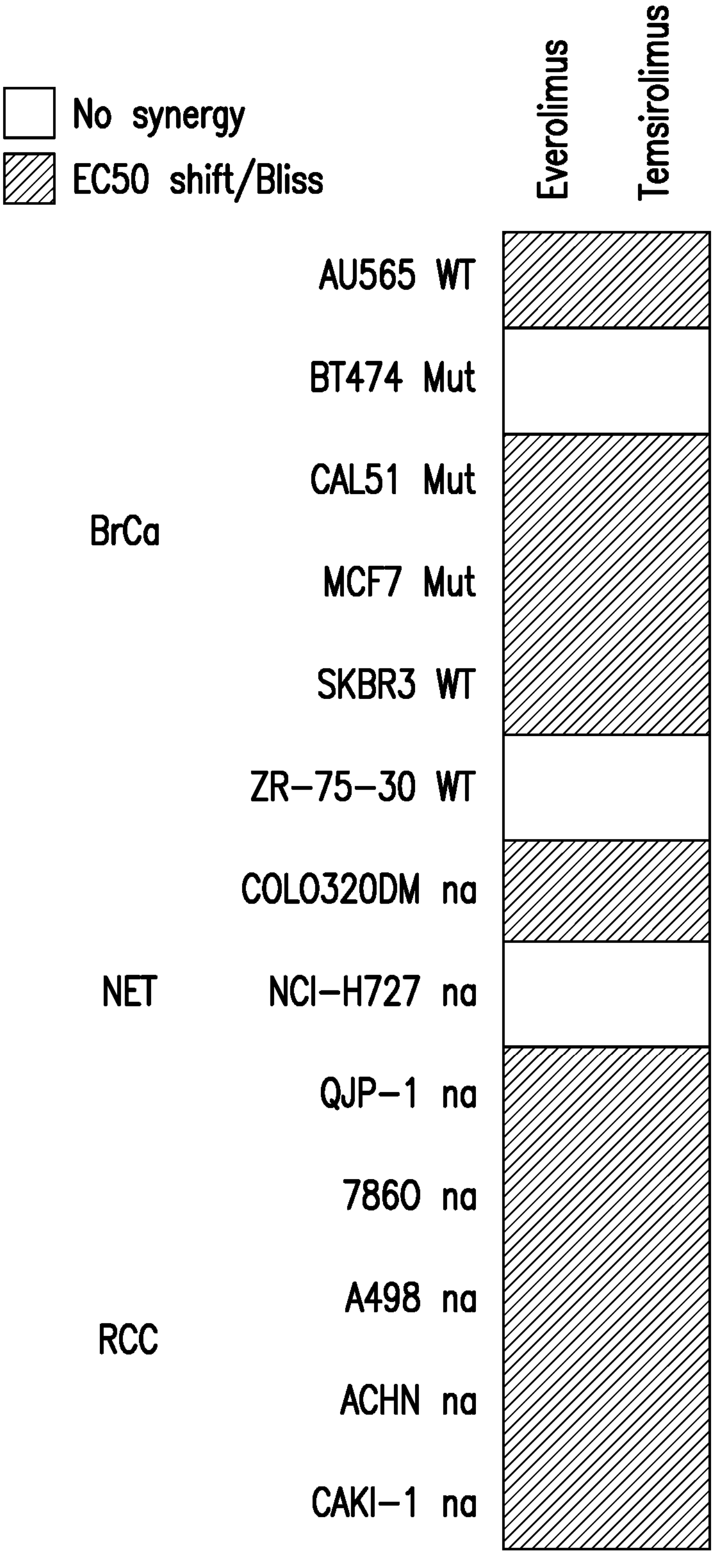
No synergy
EC50 shift/Bliss
Everolimus
Temsirolimus
AU565 WT
BT474 Mut
CAL51 Mut
BrCa
MCF7 Mut
SKBR3 WT
ZR-75-30 WT
COLO320DM na
NET
NCI-H727 na
QJP-1 na
7860 na
A498 na
RCC
ACHN na
CAKI-1 na

FIG. 58

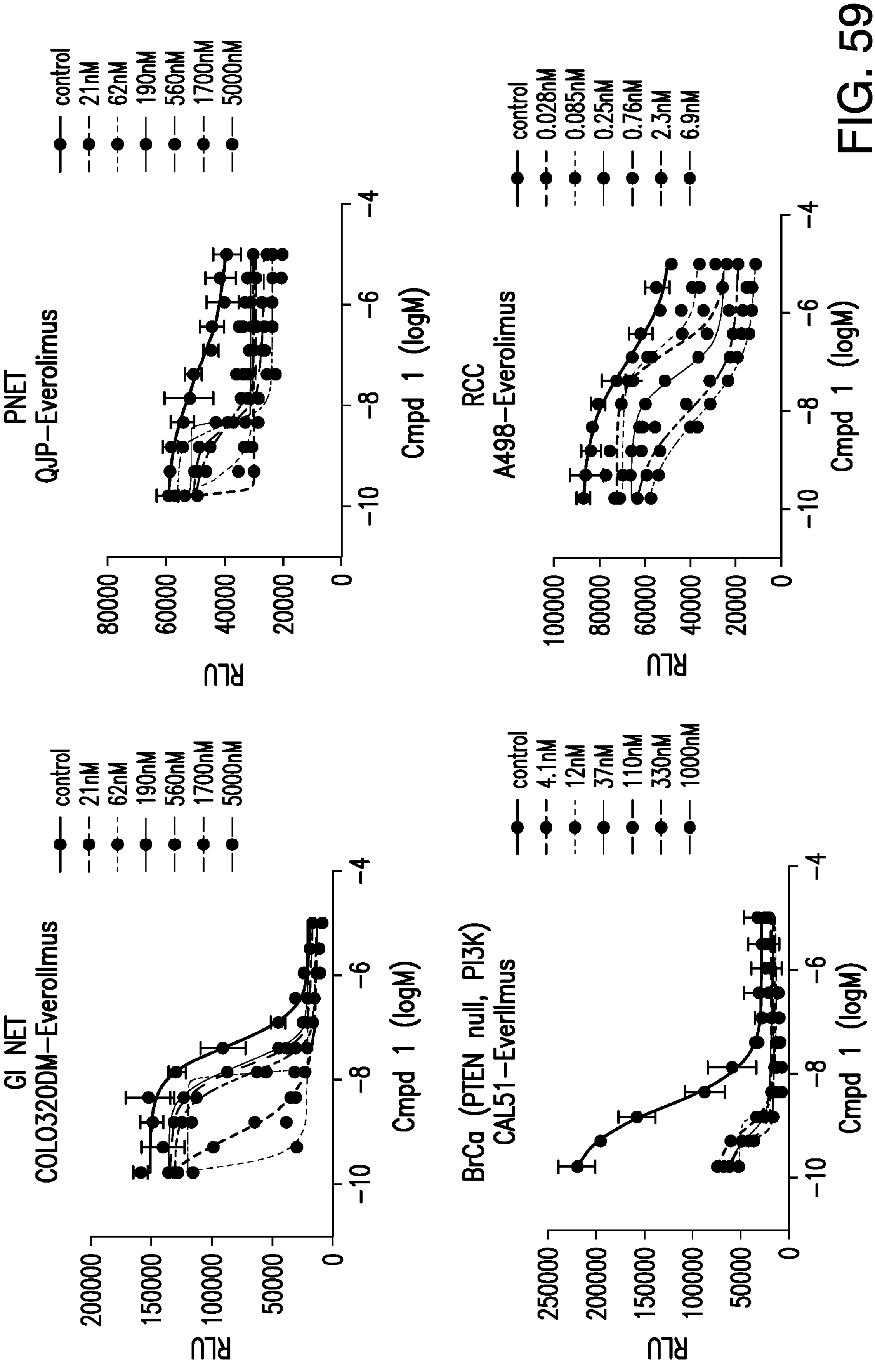
GI NET
COLO320DM-Everolimus
RLU
200000
150000
100000
50000
0
-10
-8
-6
-4
Cmpd 1 (logM)
control
21nM
62nM
190nM
560nM
1700nM
5000nM
PNET
QJP-Everolimus
80000
60000
40000
20000
BrCa (PTEN null, PI3K)
CAL51-Everolimus
250000
4.1nM
12nM
37nM
110nM
330nM
1000nM
RCC
A498-Everolimus
100000
0.028nM
0.085nM
0.25nM
0.76nM
2.3nM
6.9nM

FIG. 59

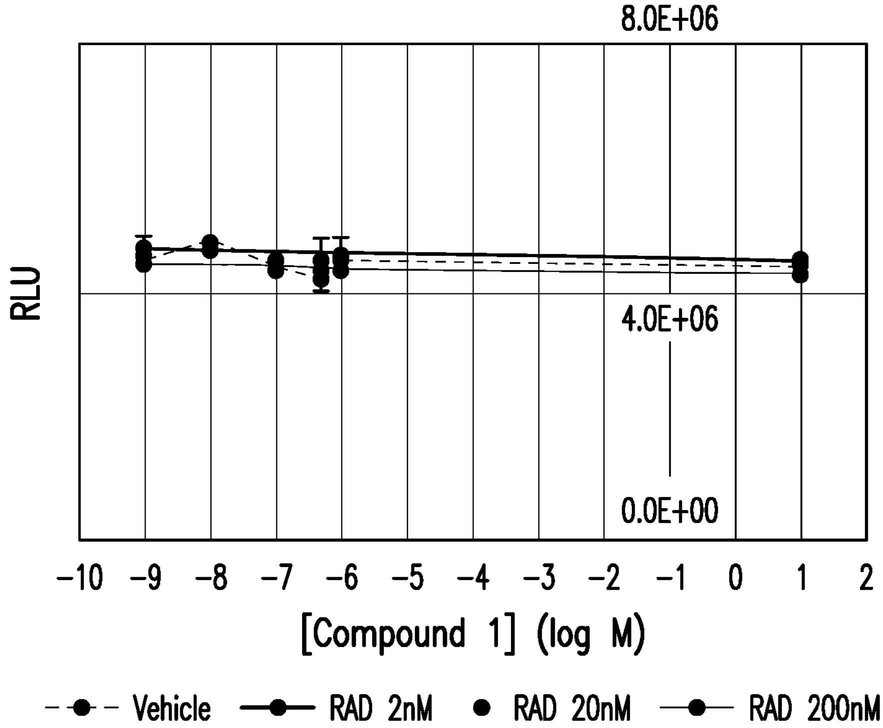
8.0E+06
4.0E+06
0.0E+00
RLU
-10 -9 -8 -7 -6 -5 -4 -3 -2 -1 0 1 2
[Compound 1] (log M)
Vehicle
RAD 2nM
RAD 20nM
RAD 200nM

FIG. 60

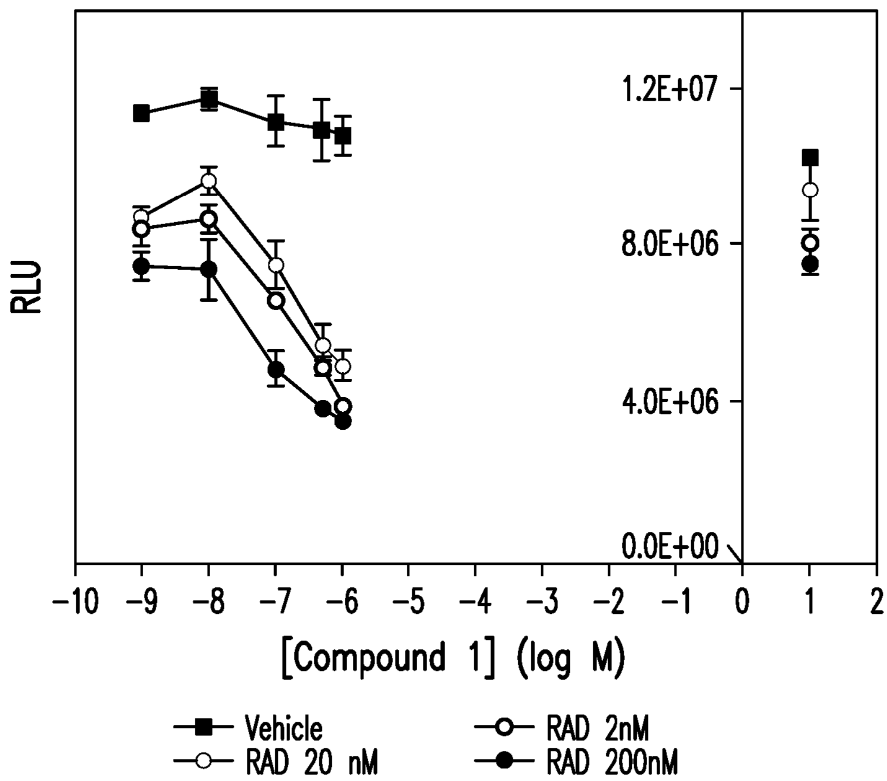
1.2E+07
8.0E+06
4.0E+06
0.0E+00
RLU
-10 -9 -8 -7 -6 -5 -4 -3 -2 -1 0 1 2
[Compound 1] (log M)
Vehicle
RAD 2nM
RAD 20 nM
RAD 200nM

FIG. 61

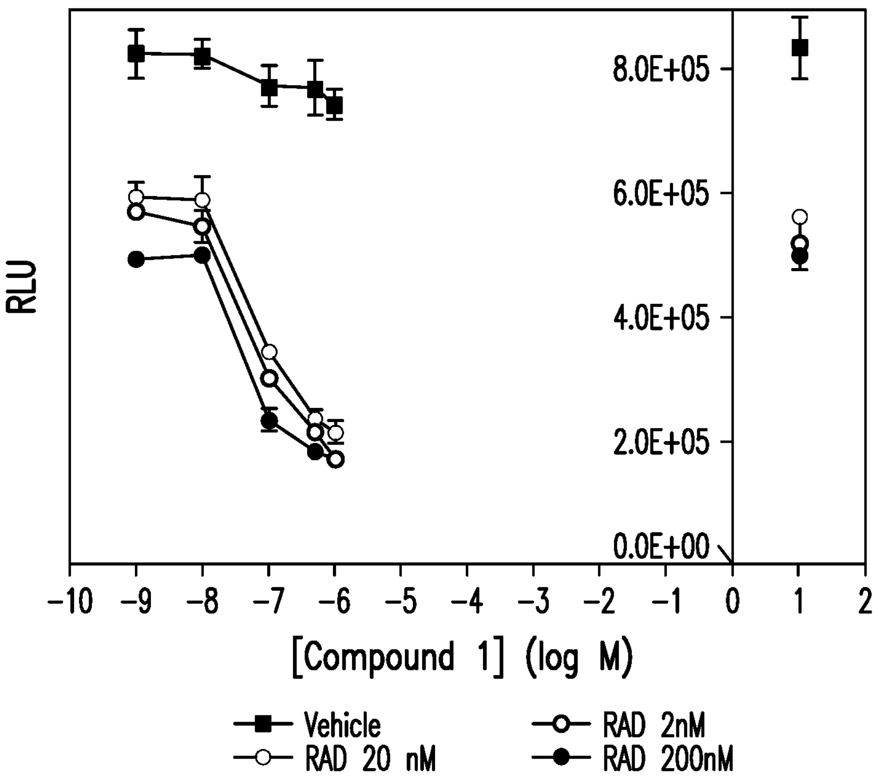
RLU
8.0E+05
6.0E+05
4.0E+05
2.0E+05
0.0E+00
−10 −9 −8 −7 −6 −5 −4 −3 −2 −1 0 1 2
[Compound 1] (log M)
Vehicle
RAD 20 nM
RAD 2nM
RAD 200nM

FIG. 62

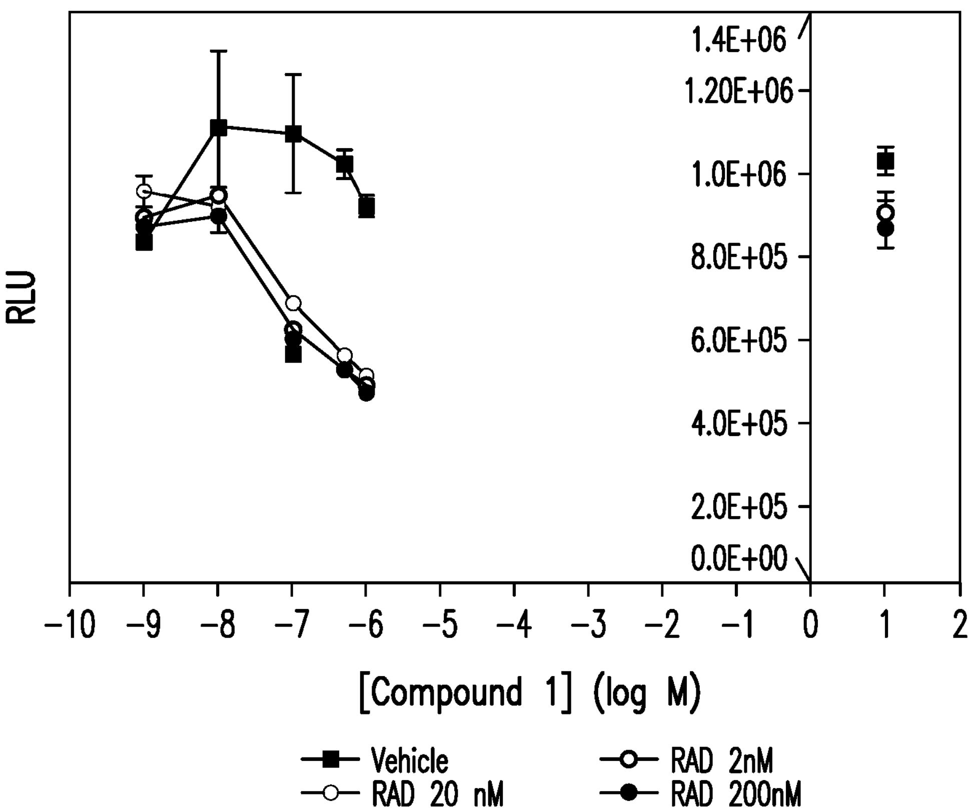
RLU
1.4E+06
1.20E+06
1.0E+06
8.0E+05
6.0E+05
4.0E+05
2.0E+05
0.0E+00
−10 −9 −8 −7 −6 −5 −4 −3 −2 −1 0 1 2
[Compound 1] (log M)
Vehicle
RAD 20 nM
RAD 2nM
RAD 200nM

FIG. 63

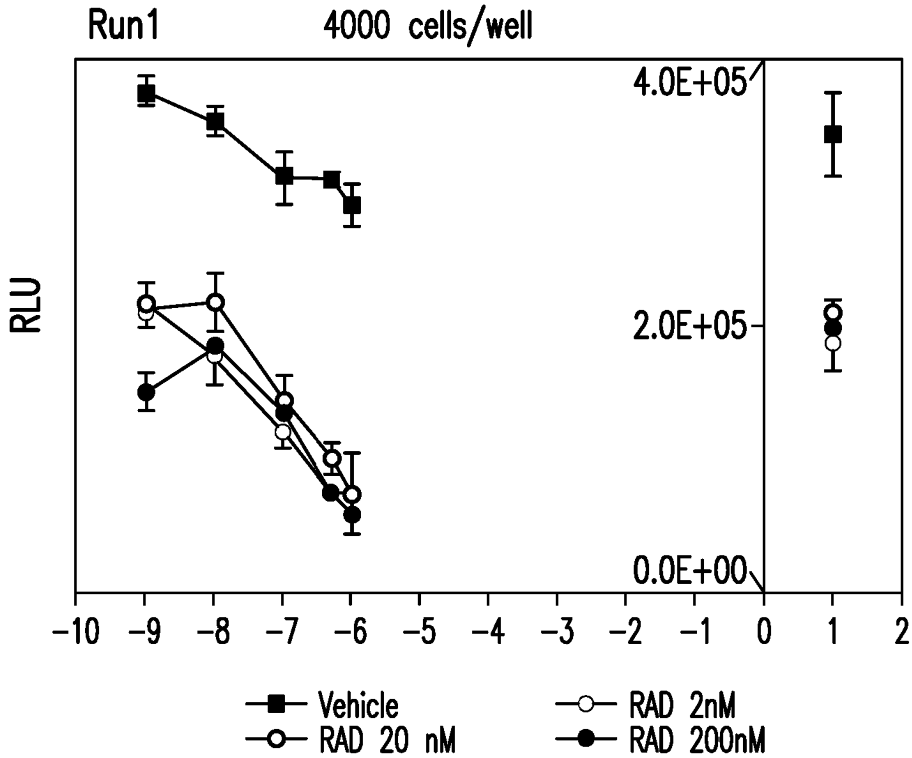


FIG. 64A

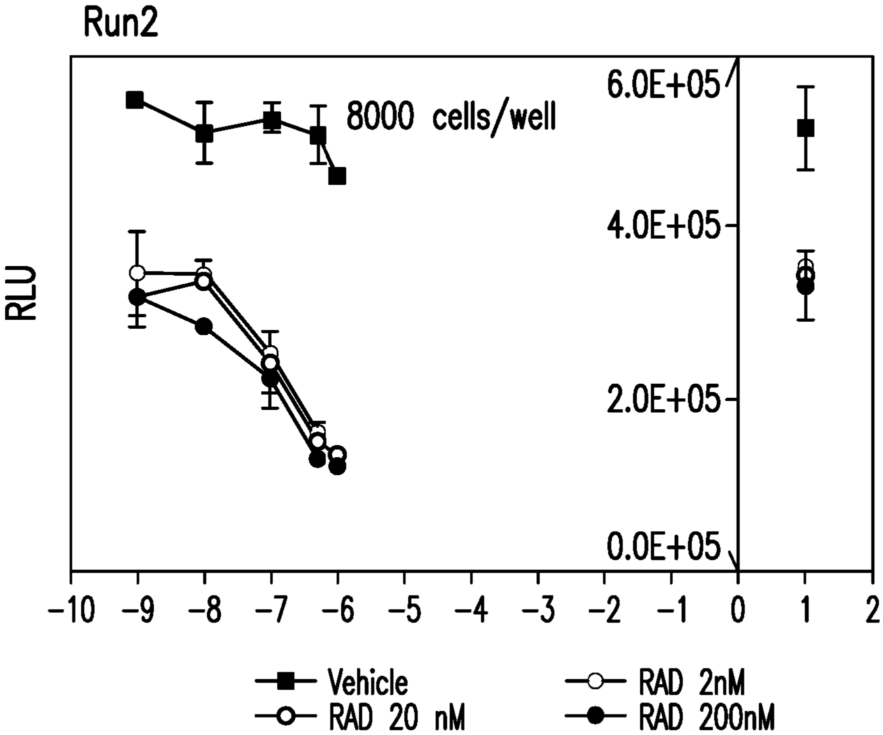


FIG. 64B

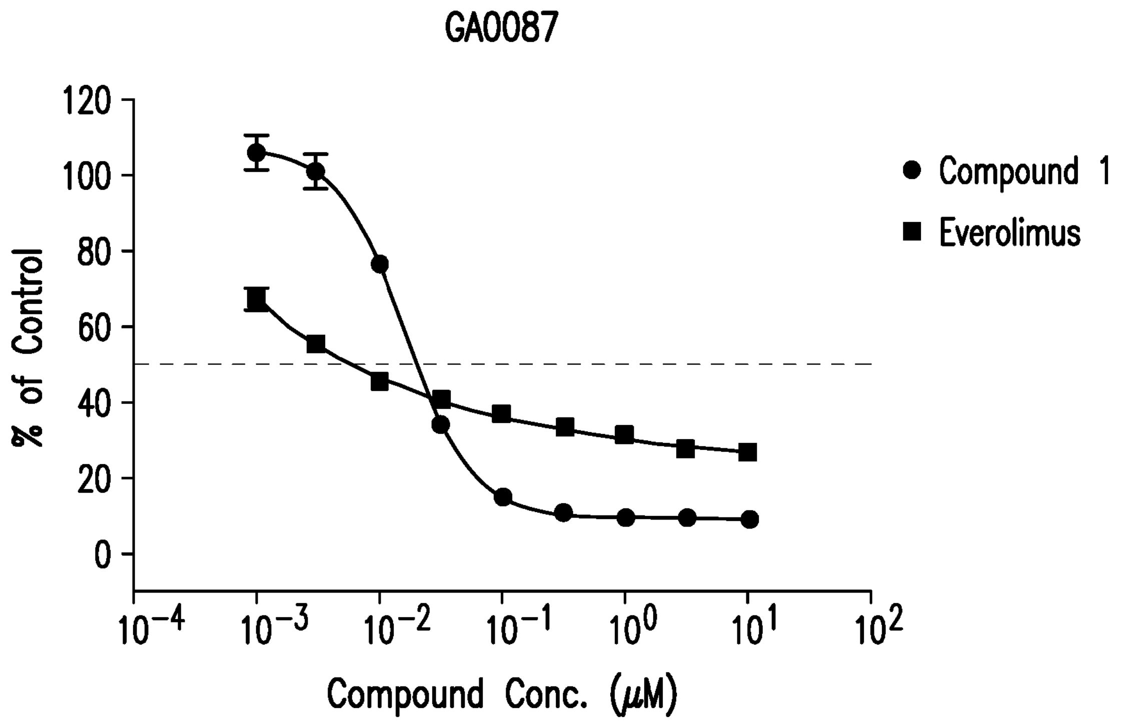


FIG. 65A

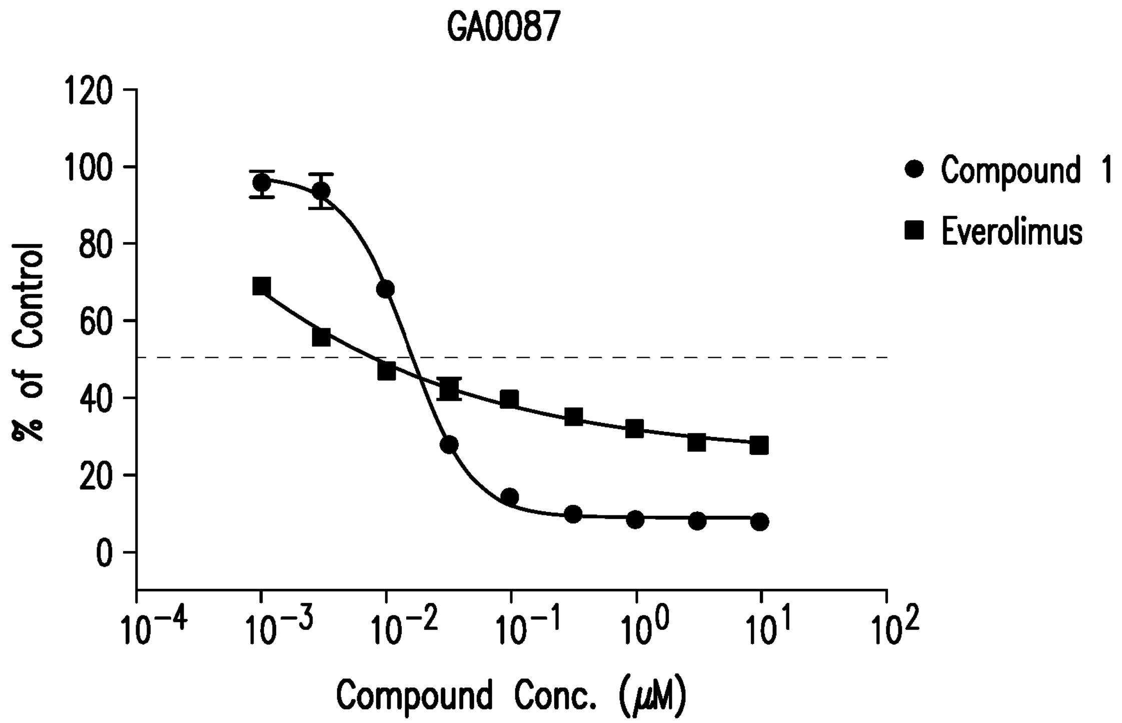


FIG. 65B

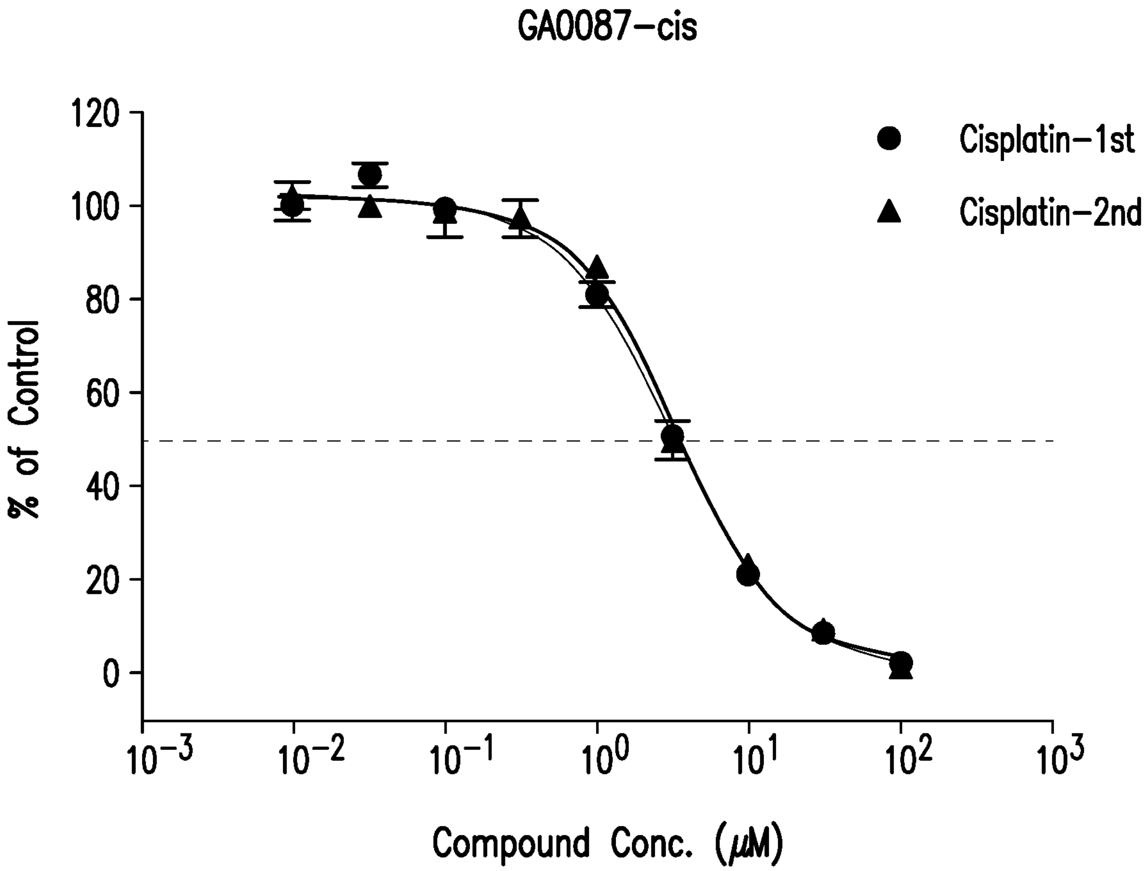
GA0087-cis
120
100
80
60
40
20
0
% of Control
Cisplatin-1st
Cisplatin-2nd
10−3
10−2
10−1
100
101
102
103
Compound Conc. (μM)

FIG. 66

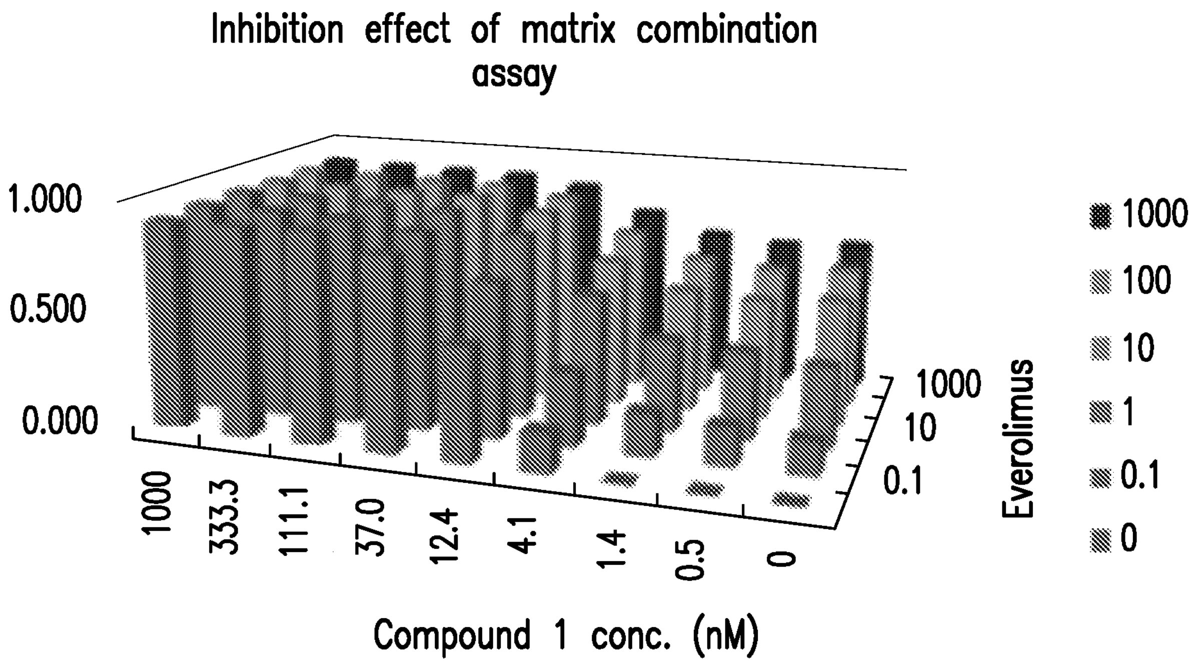


FIG. 67A

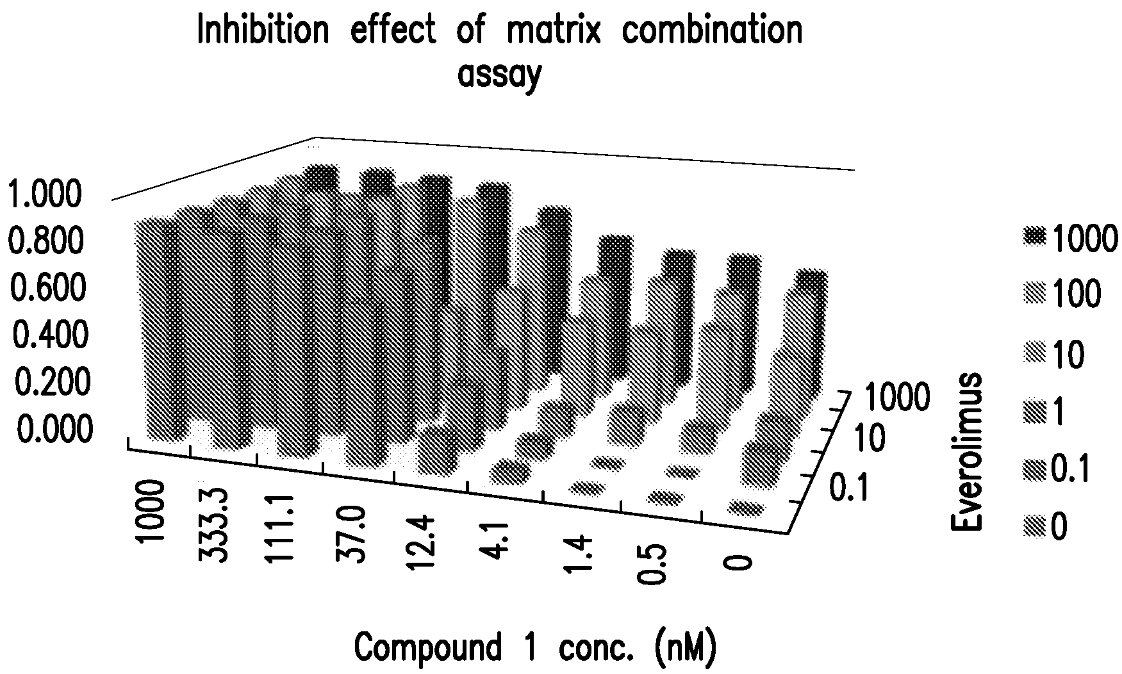


FIG. 67B

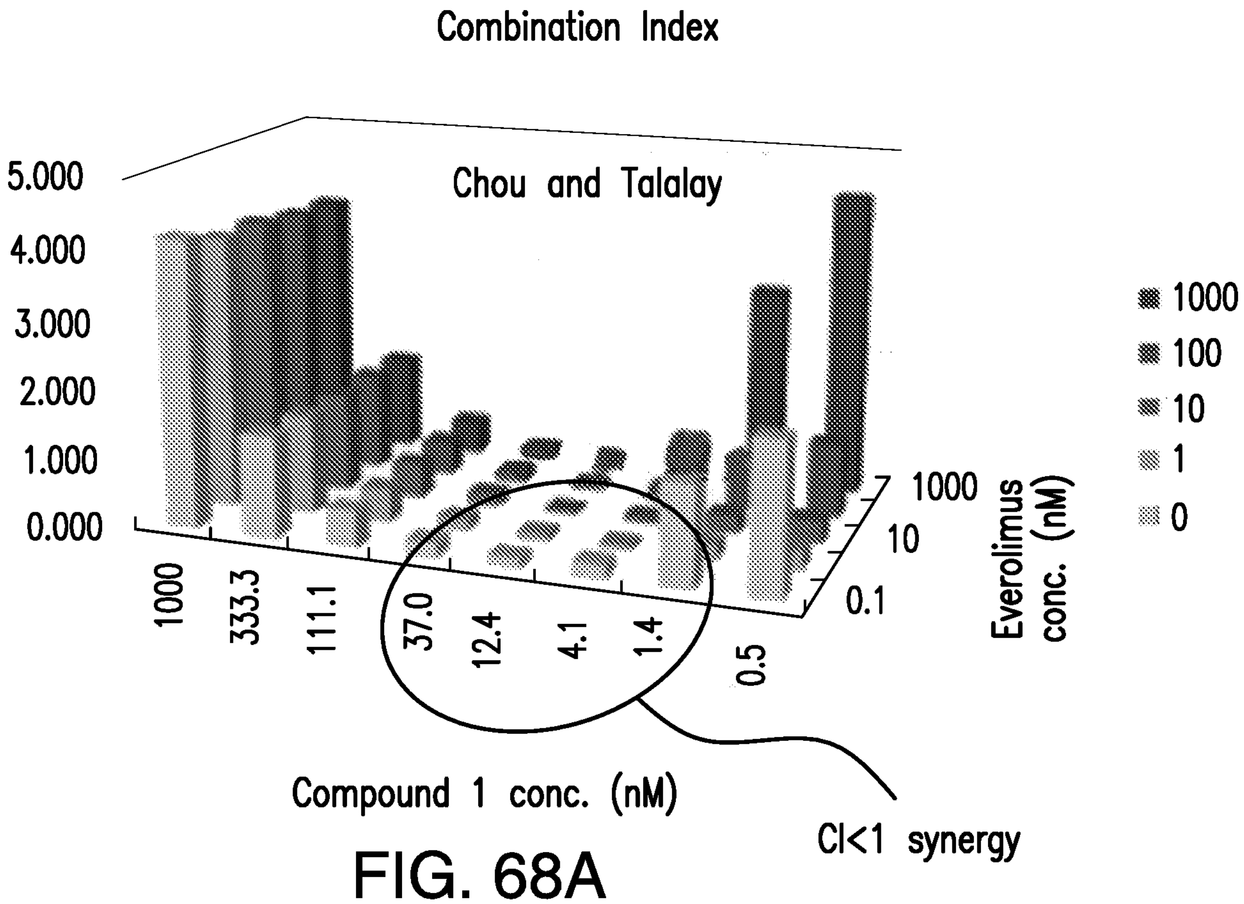


FIG. 68A

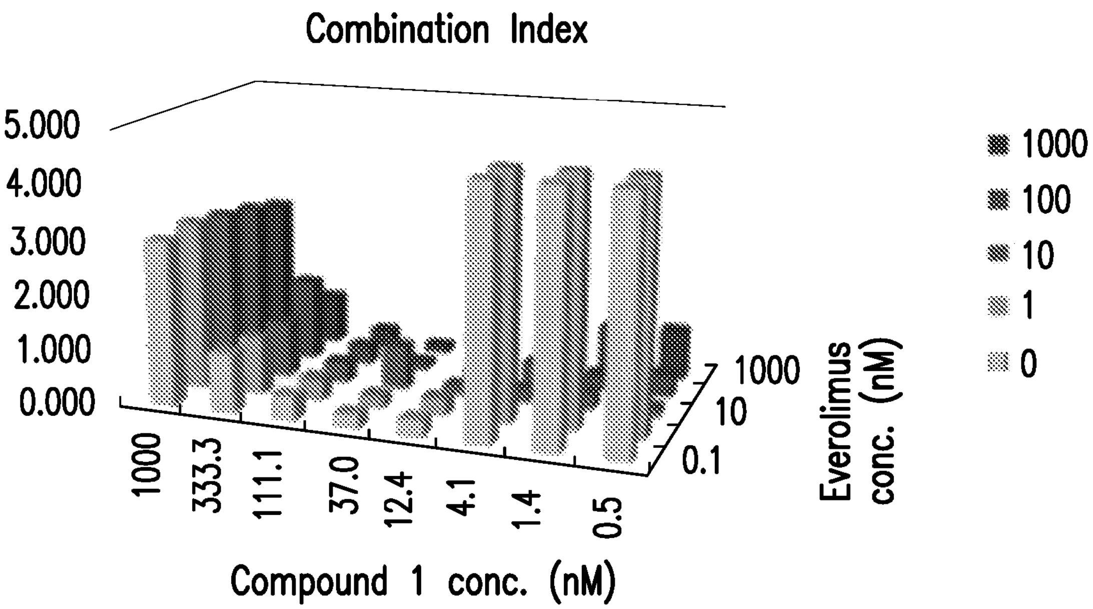


FIG. 68B

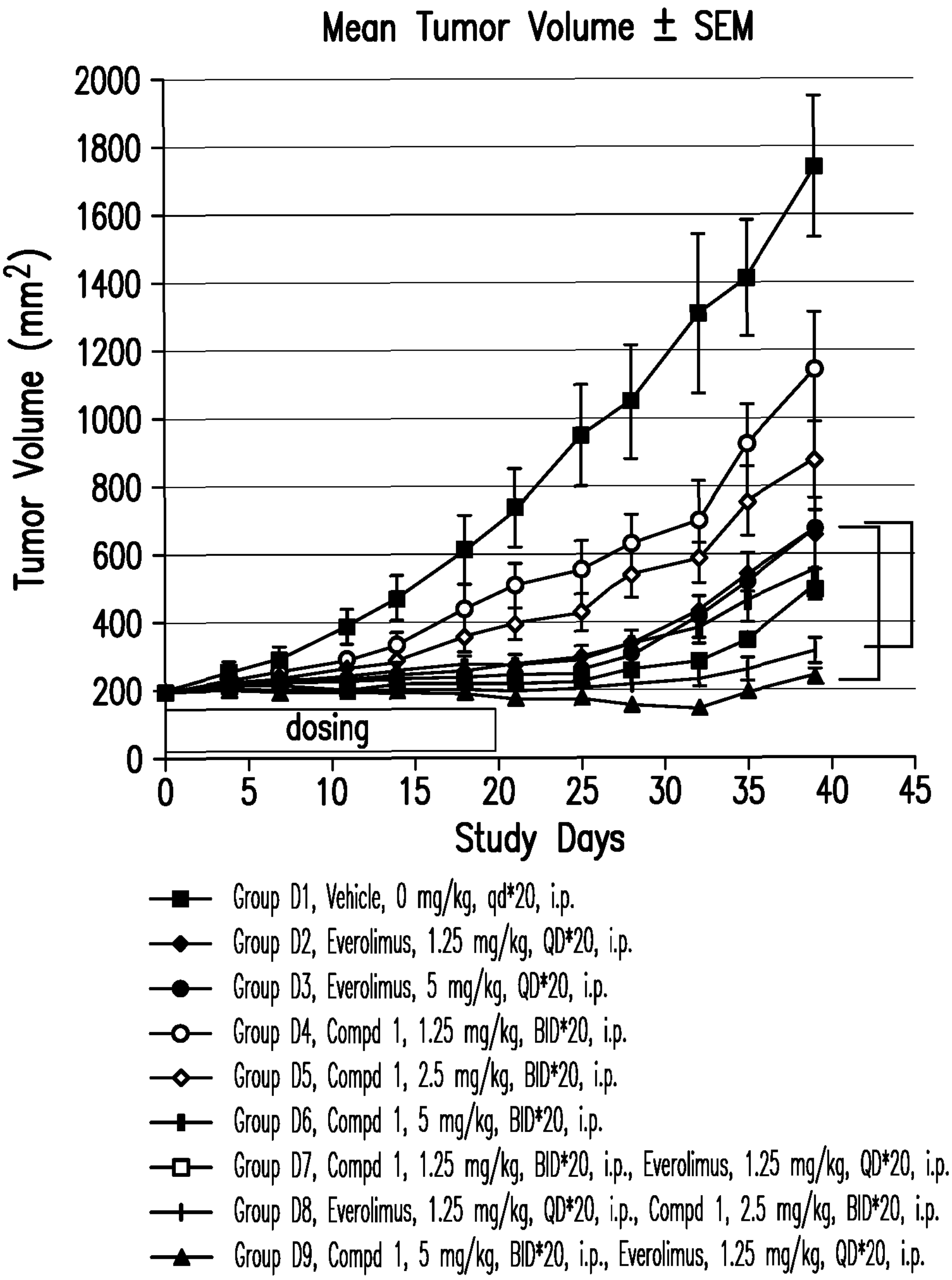


P=0.0006 (group 8 vs group 2)

P=0.0007 (group 8 vs group 5)

P=0.0001 (group 9 vs group 2)

P=0.00056 (group 9 vs group 6)

P=0.059 (group 7 vs group 2)

P value from 2 tailed student T test

FIG. 69

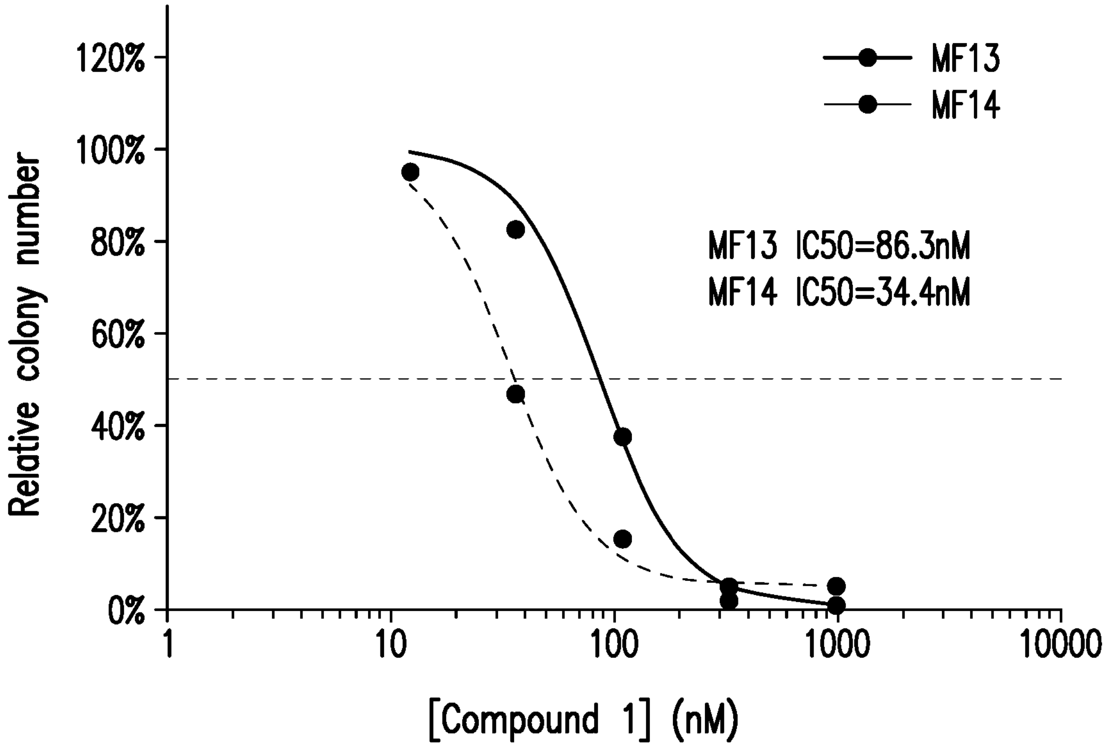
120%
100%
80%
60%
40%
20%
0%
Relative colony number
1
10
100
1000
10000
[Compound 1] (nM)
MF13
MF14
MF13 IC50=86.3nM
MF14 IC50=34.4nM

FIG. 70

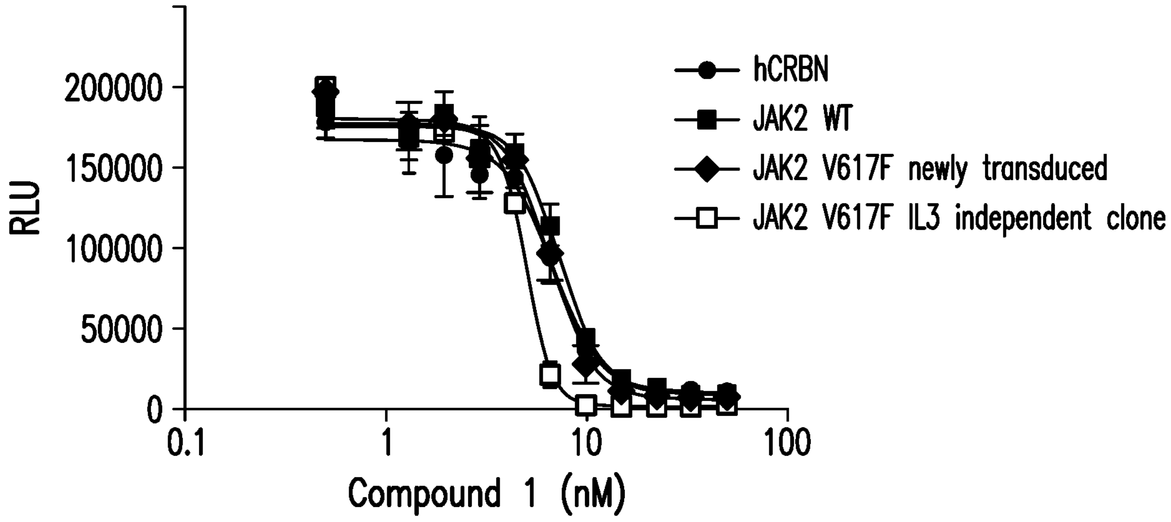


| | hCRBN | JAK2 WT | JAK2 V617F newly transduced | JAK2 V617F IL3 independent clone |
|---|---|---|---|---|
| HillSlope | −3.616 | −4.08 | −4.001 | −6.716 |
| IC50 | 6.724 | 7.312 | 6.553 | 4.992 |

FIG. 71

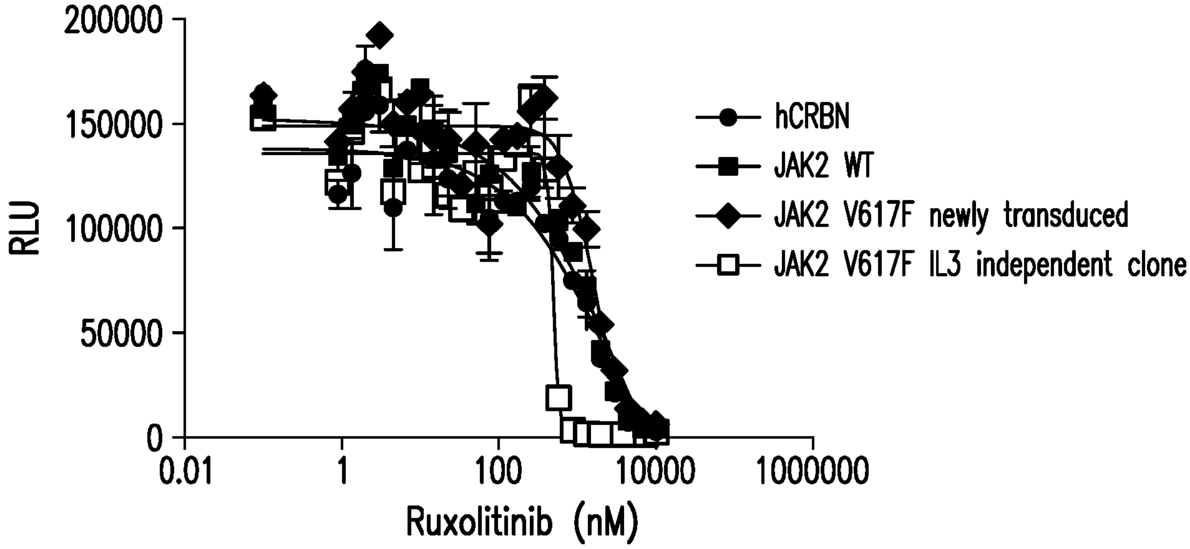


| | hCRBN | JAK2 WT | JAK2 V617F newly transduced | JAK2 V617F IL3 independent clone |
|---|---|---|---|---|
| HillSlope | −0.691 | −0.6379 | −2.347 | −11.79 |
| IC50 | 2526 | 3469 | 1578 | 496.8 |

FIG. 72

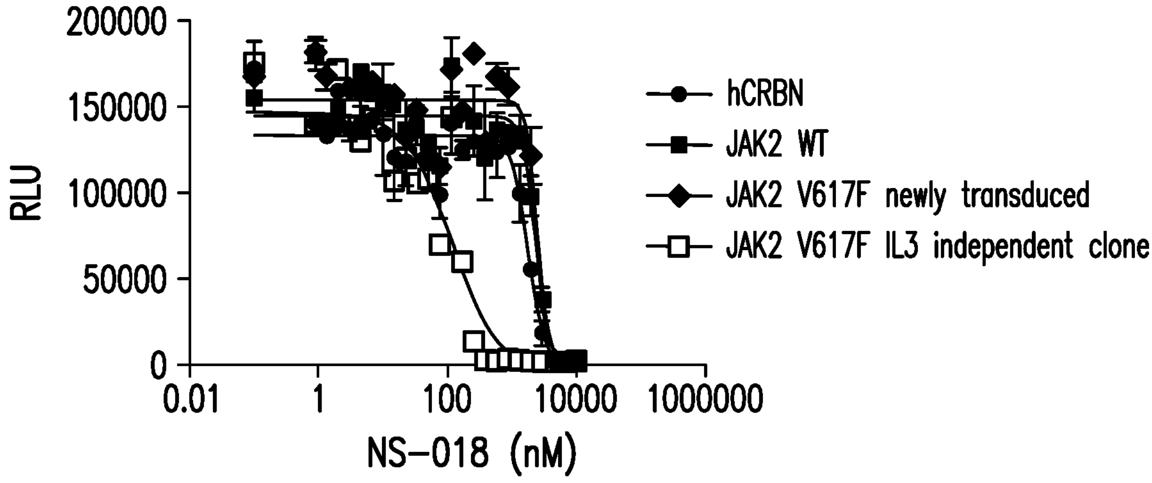


| | hCRBN | JAK2 WT | JAK2 V617F newly transduced | JAK2 V617F IL3 independent clone |
|---|---|---|---|---|
| HillSlope | -3.561 | -4.313 | -5.53 | -1.211 |
| IC50 | 1780 | 2321 | 2443 | 121.8 |

FIG. 73

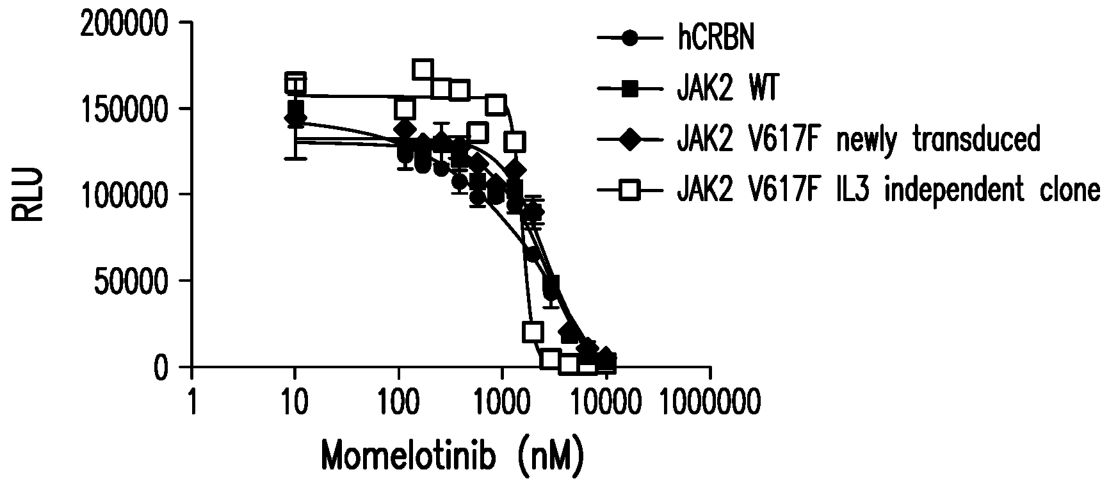


| | hCRBN | JAK2 WT | JAK2 V617F newly transduced | JAK2 V617F IL3 independent clone |
|---|---|---|---|---|
| HillSlope | -0.6718 | -1.593 | -2.053 | -8.822 |
| IC50 | 6238 | 2798 | 2638 | 1573 |

FIG. 74

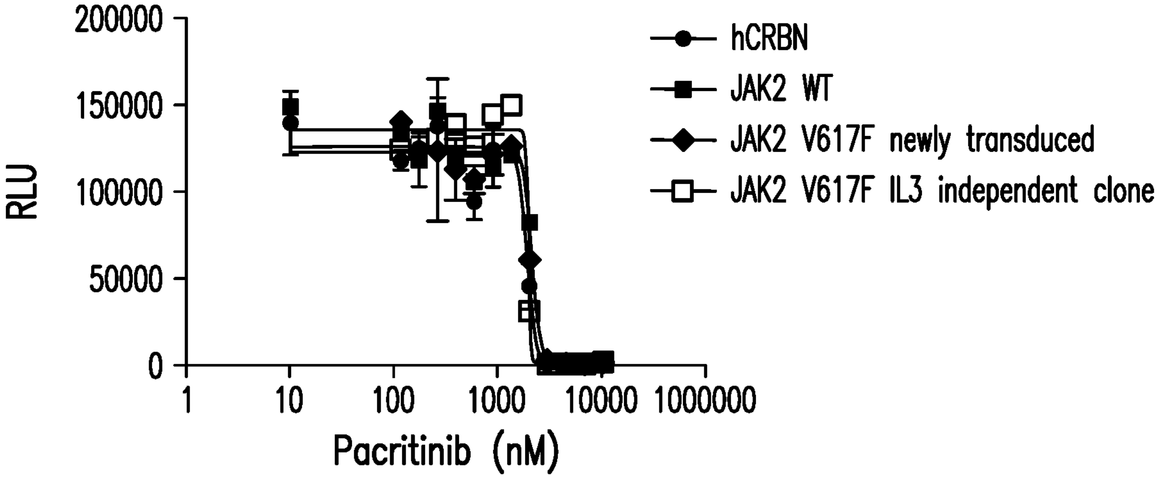


| | hCRBN | JAK2 WT | JAK2 V617F newly transduced | JAK2 V617F IL3 independent clone |
|---|---|---|---|---|
| HillSlope | −11.27 | −11.62 | −14.02 | ~ −39.85 |
| IC50 | 1882 | 2084 | 1961 | ~ 1914 |

FIG. 75

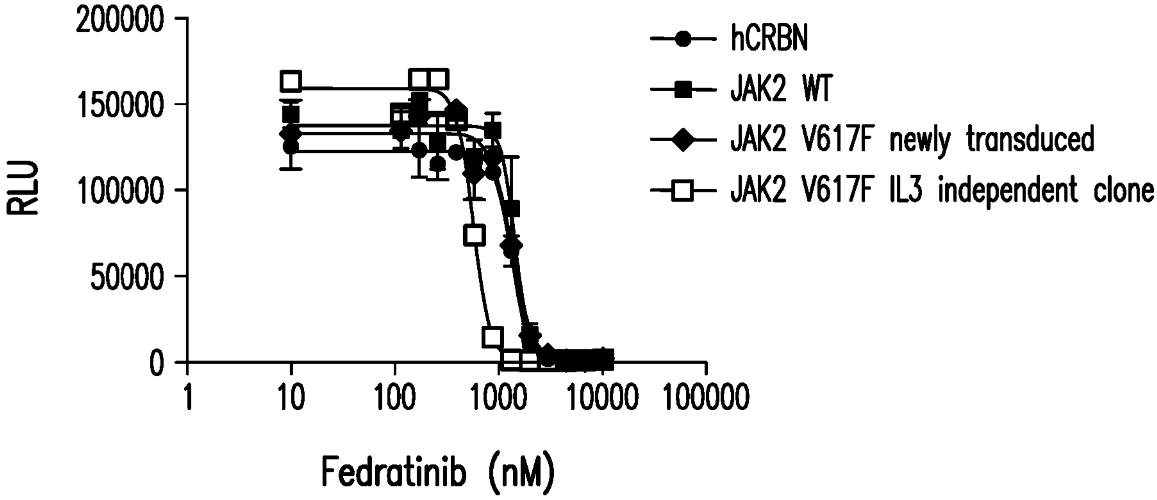


| | hCRBN | JAK2 WT | JAK2 V617F newly transduced | JAK2 V617F IL3 independent clone |
|---|---|---|---|---|
| HillSlope | −5.401 | −6.66 | −4.52 | −5.578 |
| IC50 | 1330 | 1443 | 1313 | 567.9 |

FIG. 76

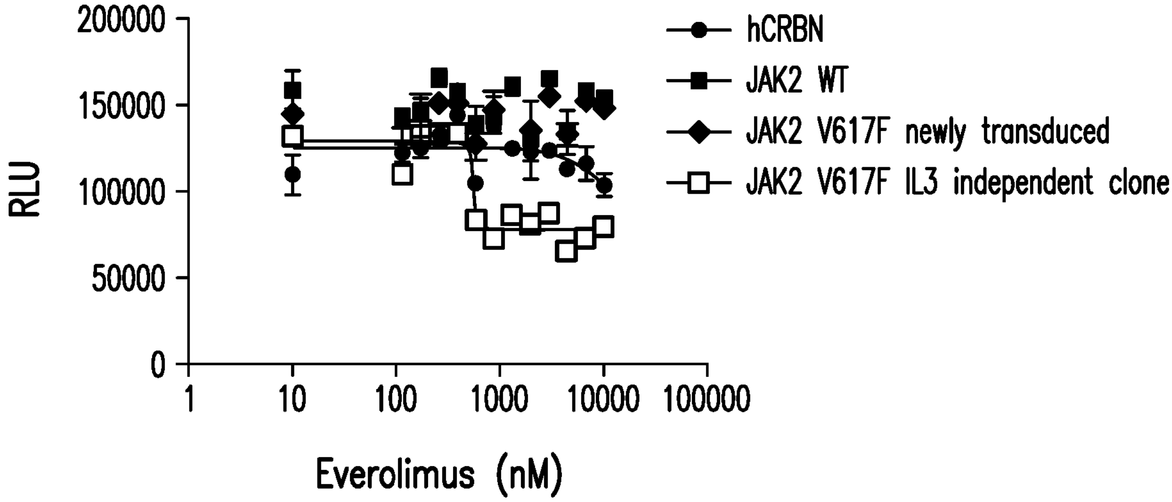


| | hCRBN | JAK2 WT | JAK2 V617F newly transduced | JAK2 V617F IL3 independent clone |
|---|---|---|---|---|
| HillSlope | -1.959 | | | ~ -35.61 |
| IC50 | 12190 | | | ~ 551.7 |

FIG. 77

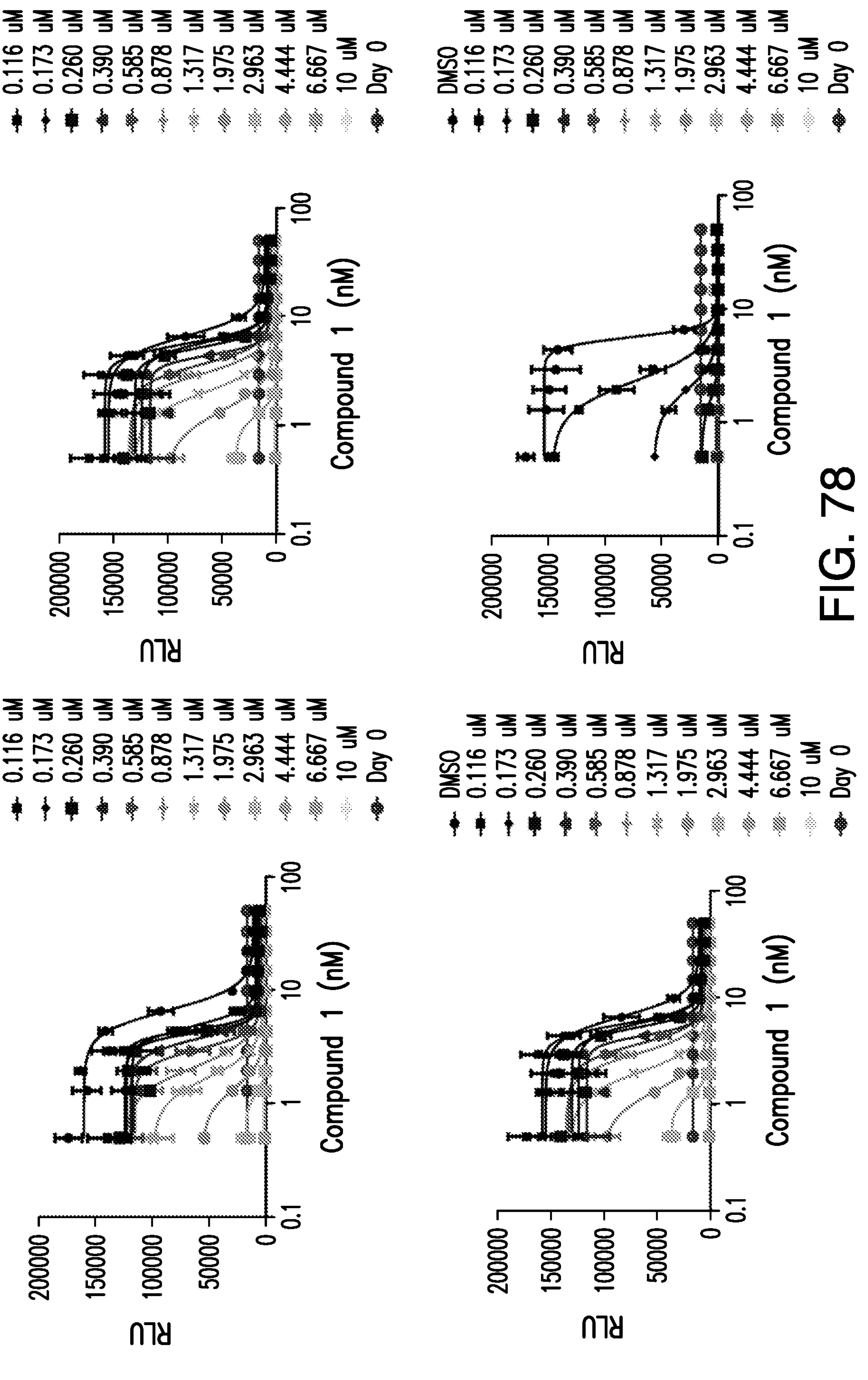
RLU
200000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 78

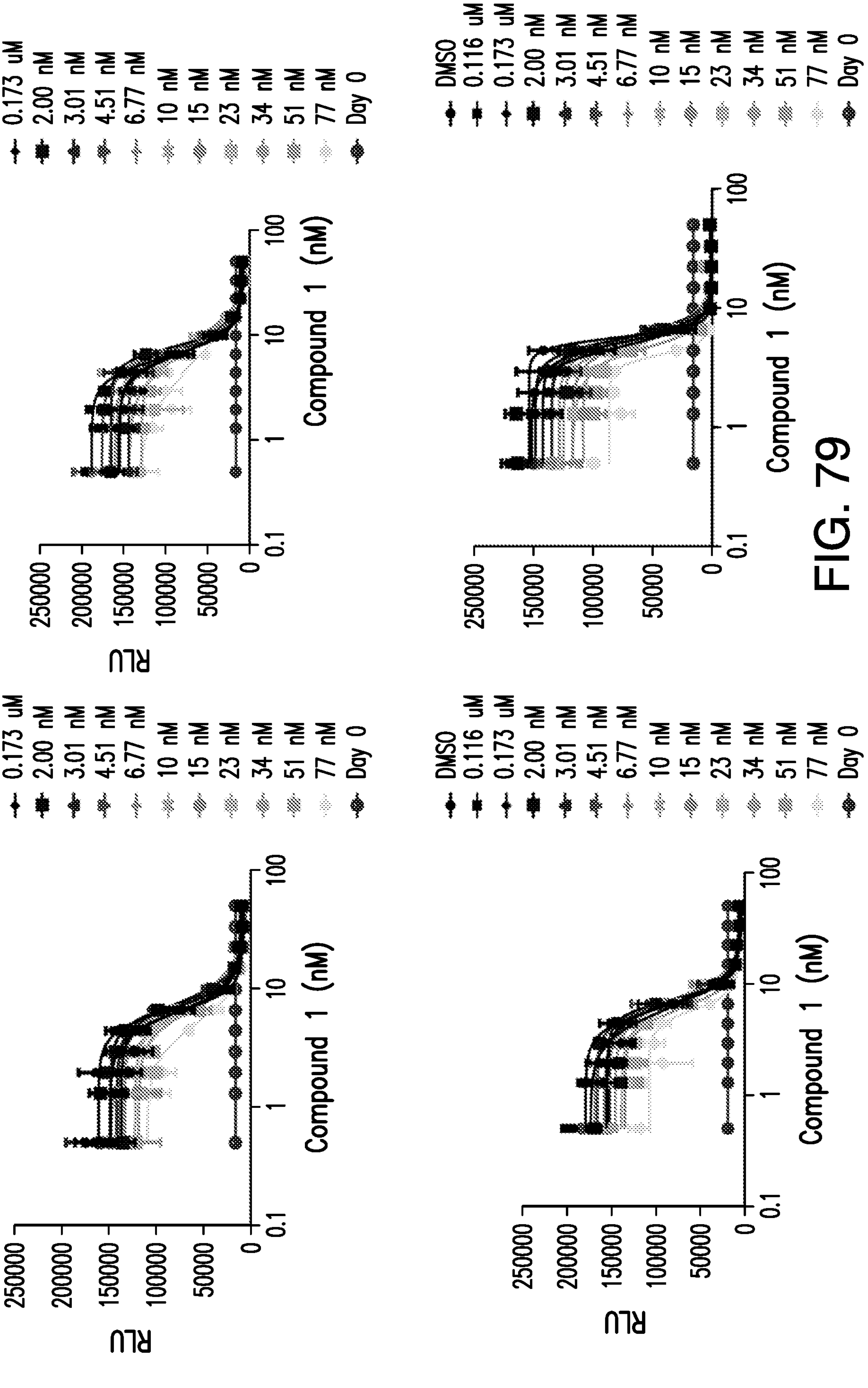
RLU
250000
200000
150000
100000
50000
0
0.1
1
10
100
Compound 1 (nM)
DMSO
0.116 uM
0.173 uM
2.00 nM
3.01 nM
4.51 nM
6.77 nM
10 nM
15 nM
23 nM
34 nM
51 nM
77 nM
Day 0

FIG. 79

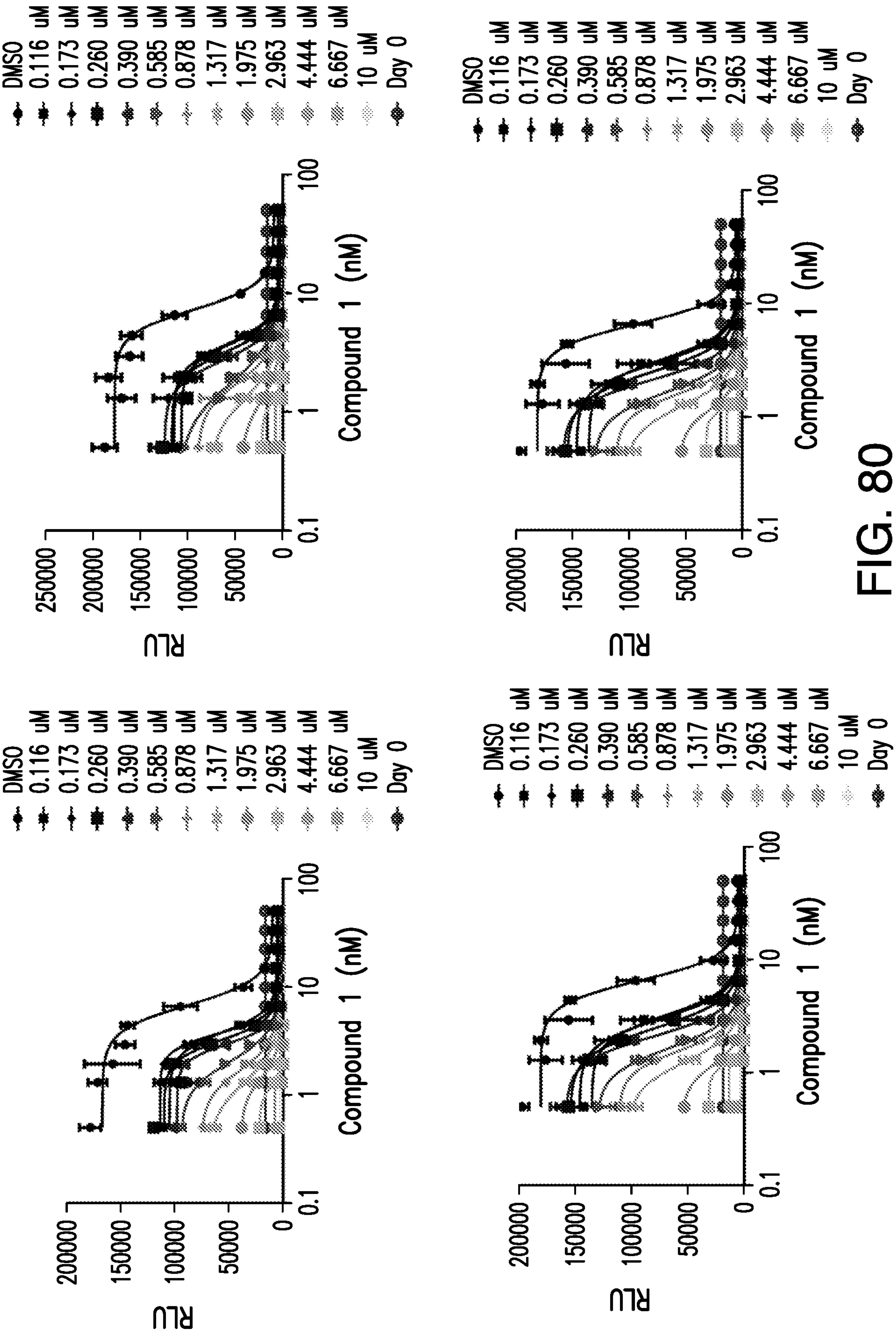
RLU
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 80

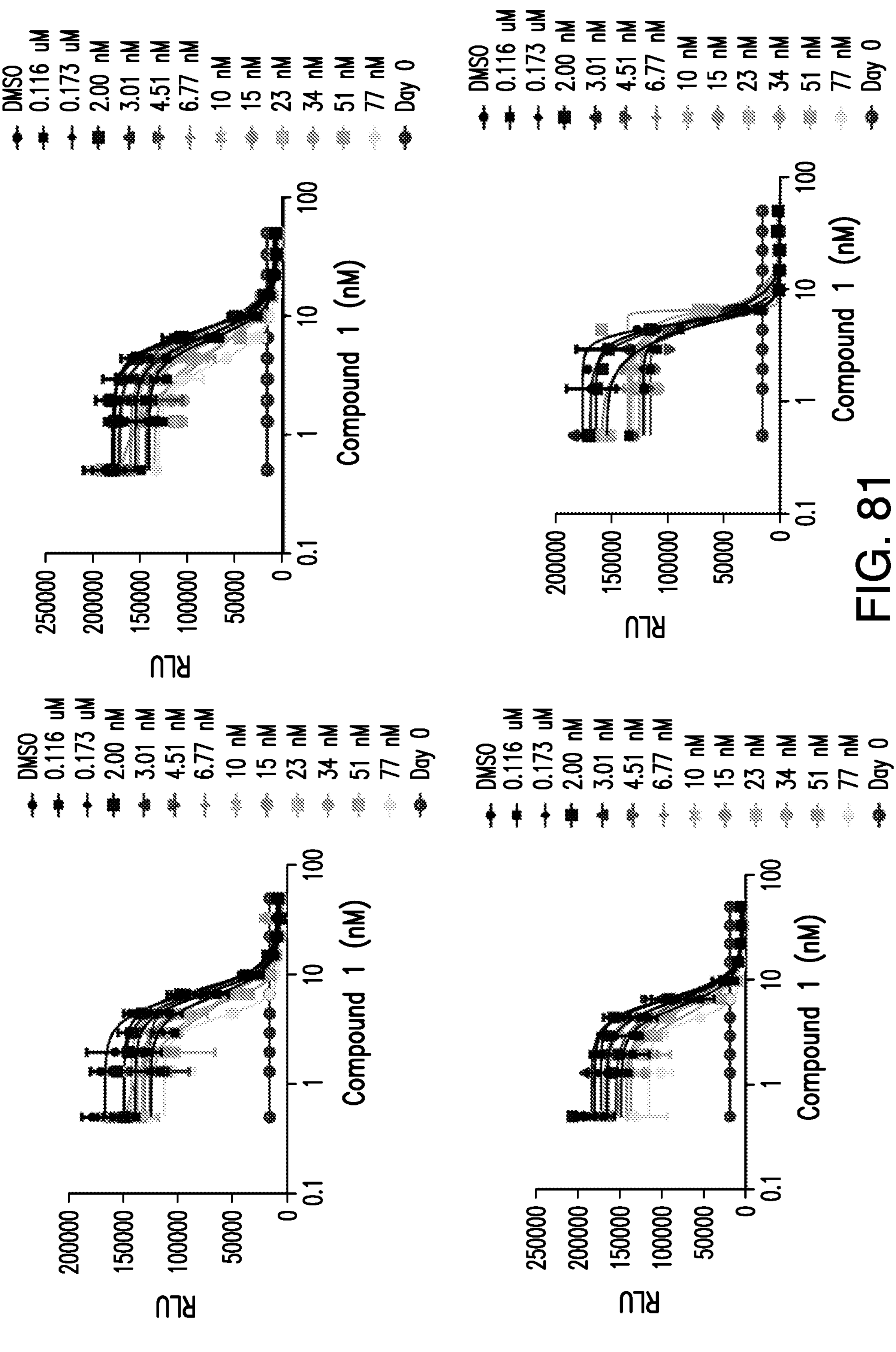
RLU
200000
150000
100000
50000
0
0.1
1
10
100
Compound 1 (nM)
DMSO
0.116 uM
0.173 uM
2.00 nM
3.01 nM
4.51 nM
6.77 nM
10 nM
15 nM
23 nM
34 nM
51 nM
77 nM
Day 0
RLU
250000
200000
150000
100000
50000
0
0.1
1
10
100
Compound 1 (nM)
DMSO
0.116 uM
0.173 uM
2.00 nM
3.01 nM
4.51 nM
6.77 nM
10 nM
15 nM
23 nM
34 nM
51 nM
77 nM
Day 0
RLU
250000
200000
150000
100000
50000
0
0.1
1
10
100
Compound 1 (nM)
DMSO
0.116 uM
0.173 uM
2.00 nM
3.01 nM
4.51 nM
6.77 nM
10 nM
15 nM
23 nM
34 nM
51 nM
77 nM
Day 0
RLU
200000
150000
100000
50000
0
0.1
1
10
100
Compound 1 (nM)
DMSO
0.116 uM
0.173 uM
2.00 nM
3.01 nM
4.51 nM
6.77 nM
10 nM
15 nM
23 nM
34 nM
51 nM
77 nM
Day 0

FIG. 81

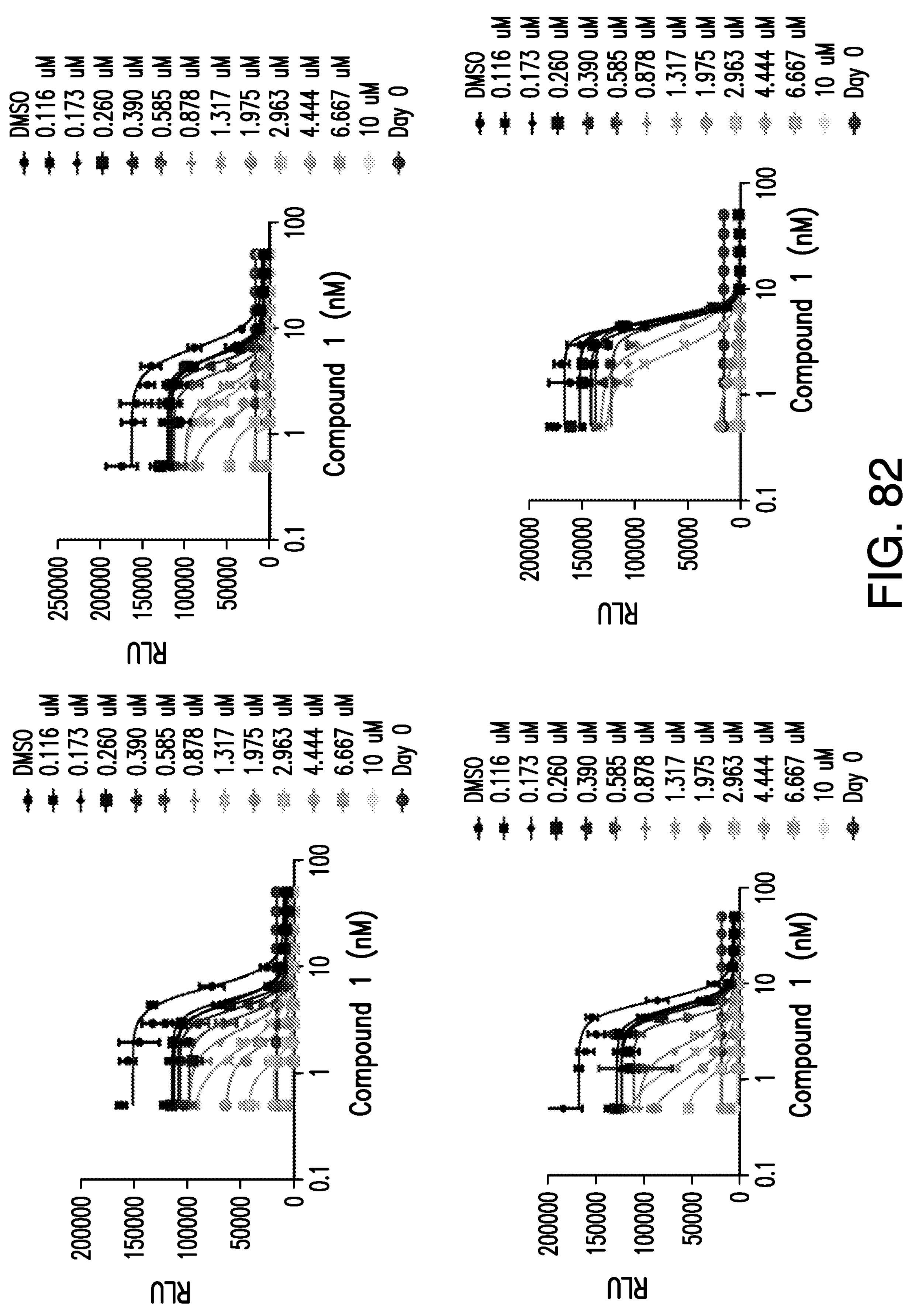
RLU
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 82

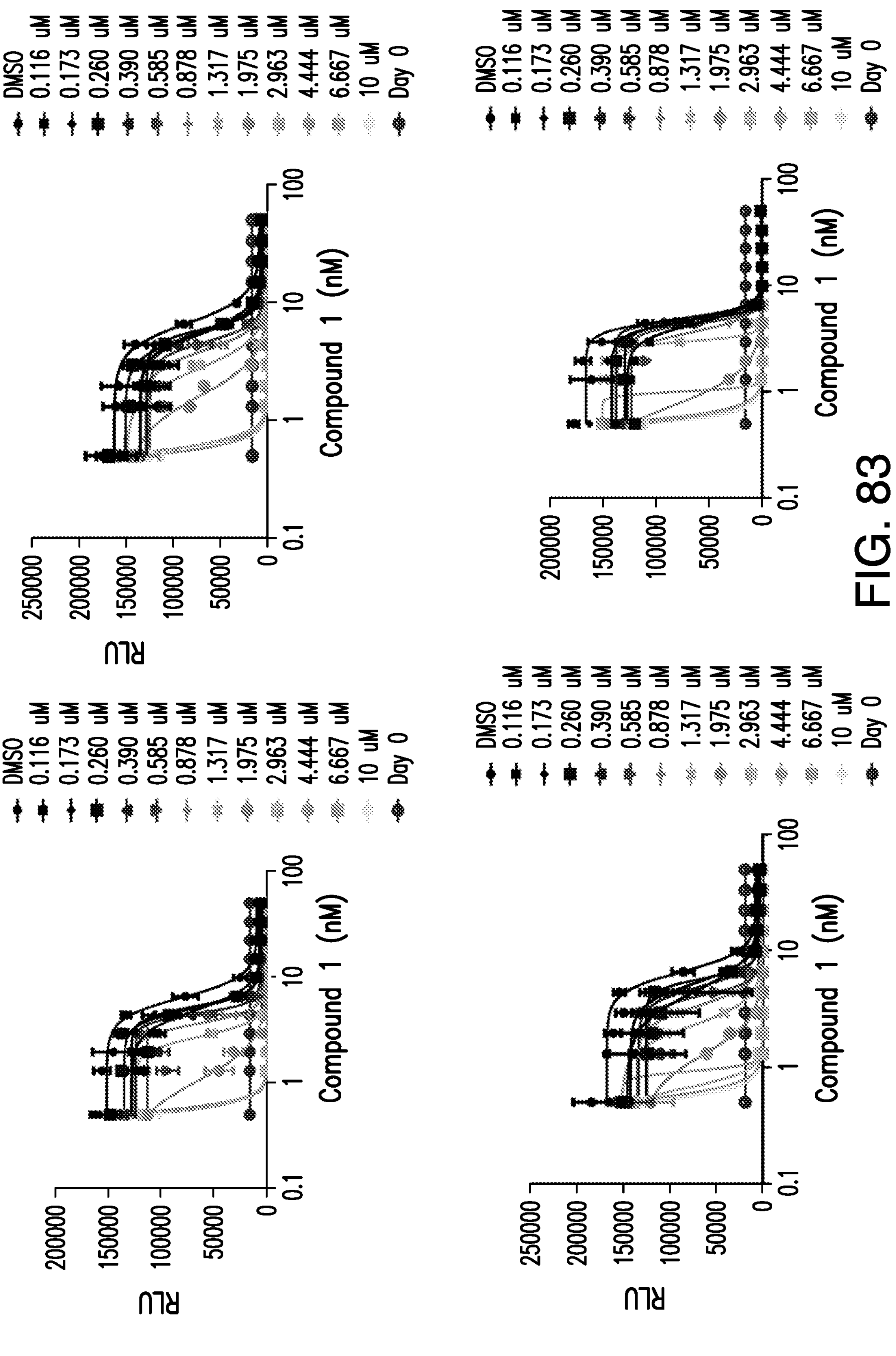
RLU
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 83

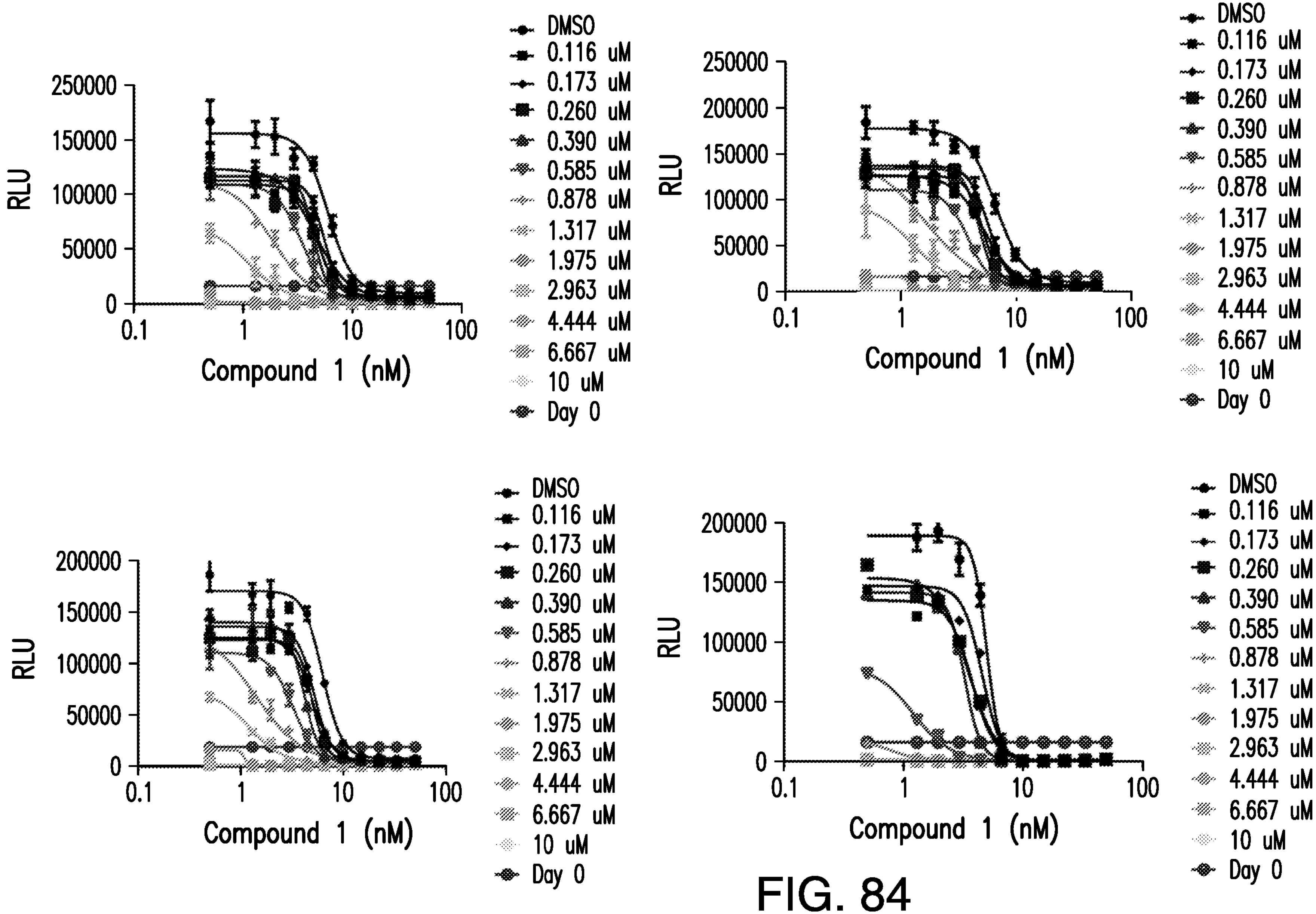
RLU
250000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0
RLU
250000
200000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0
RLU
200000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0
RLU
200000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 84

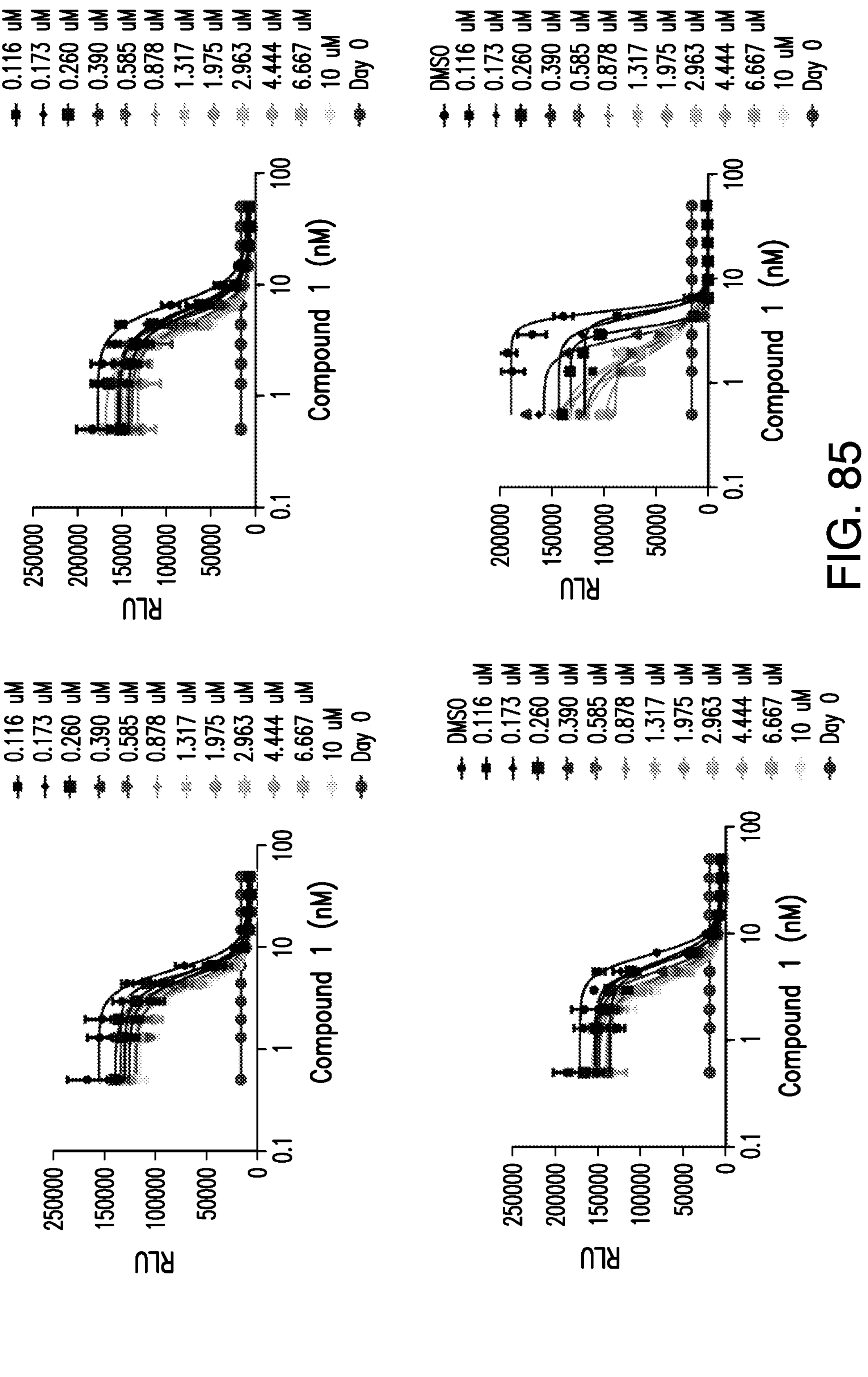
RLU
250000
200000
150000
100000
50000
0
0.1
1
10
100
Compound 1 (nM)
DMSO
0.116 uM
0.173 uM
0.260 uM
0.390 uM
0.585 uM
0.878 uM
1.317 uM
1.975 uM
2.963 uM
4.444 uM
6.667 uM
10 uM
Day 0

FIG. 85

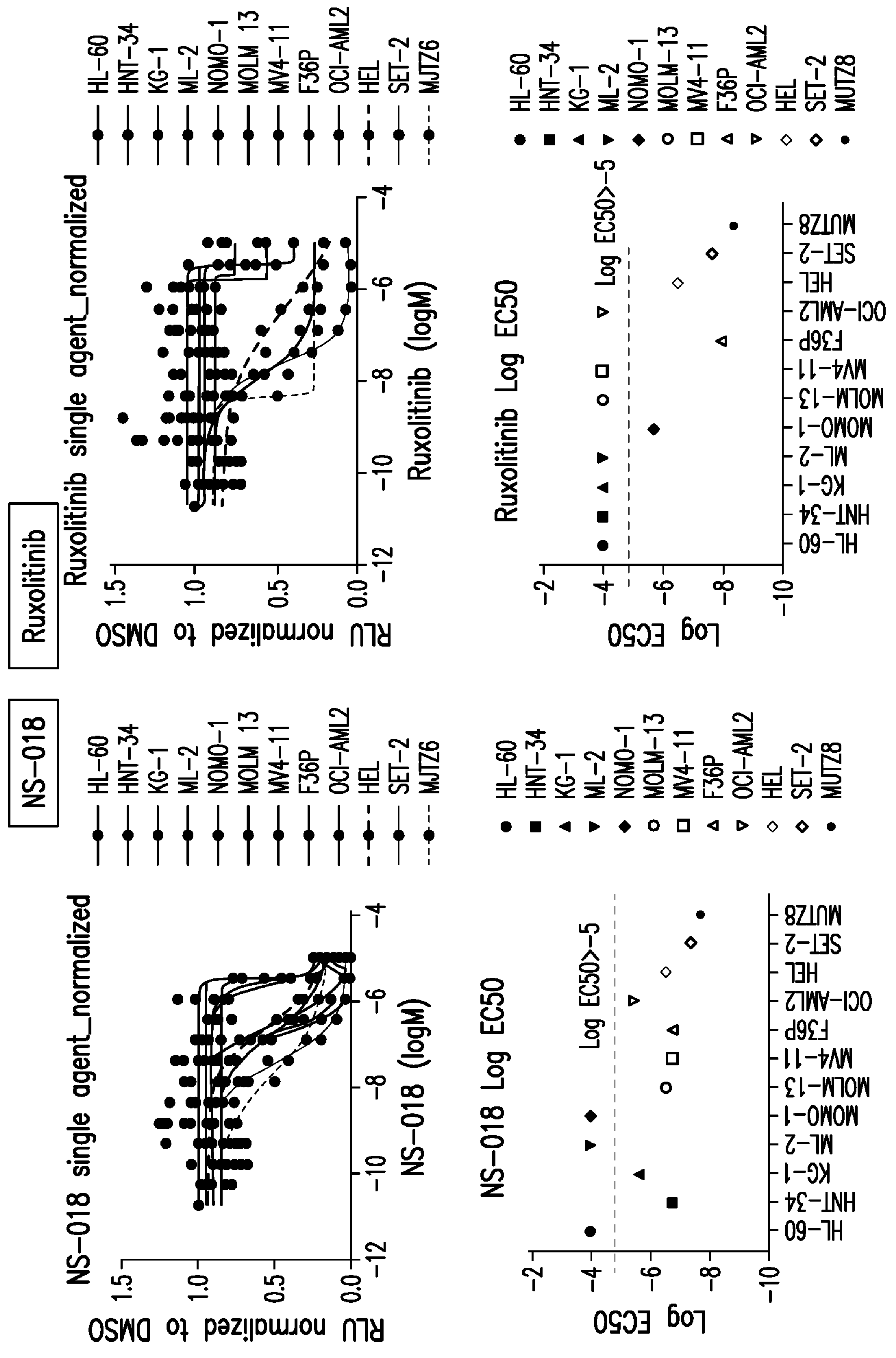

NS-018
NS-018 single agent_normalized
RLU normalized to DMSO
NS-018 (logM)
NS-018 Log EC50
Log EC50
Log EC50>-5
Ruxolitinib
Ruxolitinib single agent_normalized
Ruxolitinib (logM)
Ruxolitinib Log EC50
HL-60
HNT-34
KG-1
ML-2
NOMO-1
MOLM 13
MV4-11
F36P
OCI-AML2
HEL
SET-2
MUTZ6
MOLM-13
MUTZ8


FIG. 86

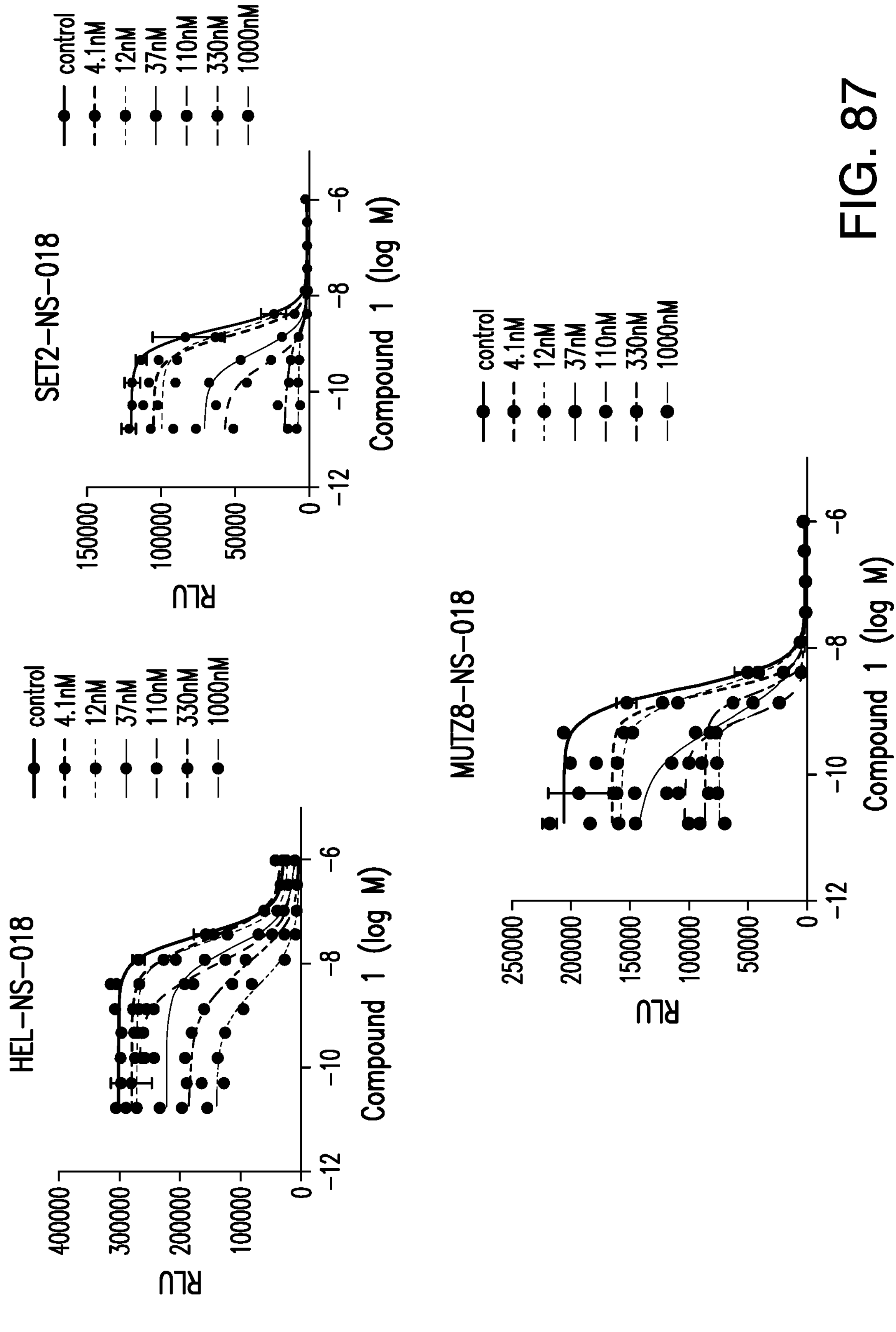
HEL-NS-018
RLU
400000
300000
200000
100000
0
-12
-10
-8
-6
Compound 1 (log M)
control
4.1nM
12nM
37nM
110nM
330nM
1000nM
SET2-NS-018
RLU
150000
100000
50000
0
-12
-10
-8
-6
Compound 1 (log M)
control
4.1nM
12nM
37nM
110nM
330nM
1000nM
MUTZ8-NS-018
RLU
250000
200000
150000
100000
50000
0
-12
-10
-8
-6
Compound 1 (log M)
control
4.1nM
12nM
37nM
110nM
330nM
1000nM

FIG. 87

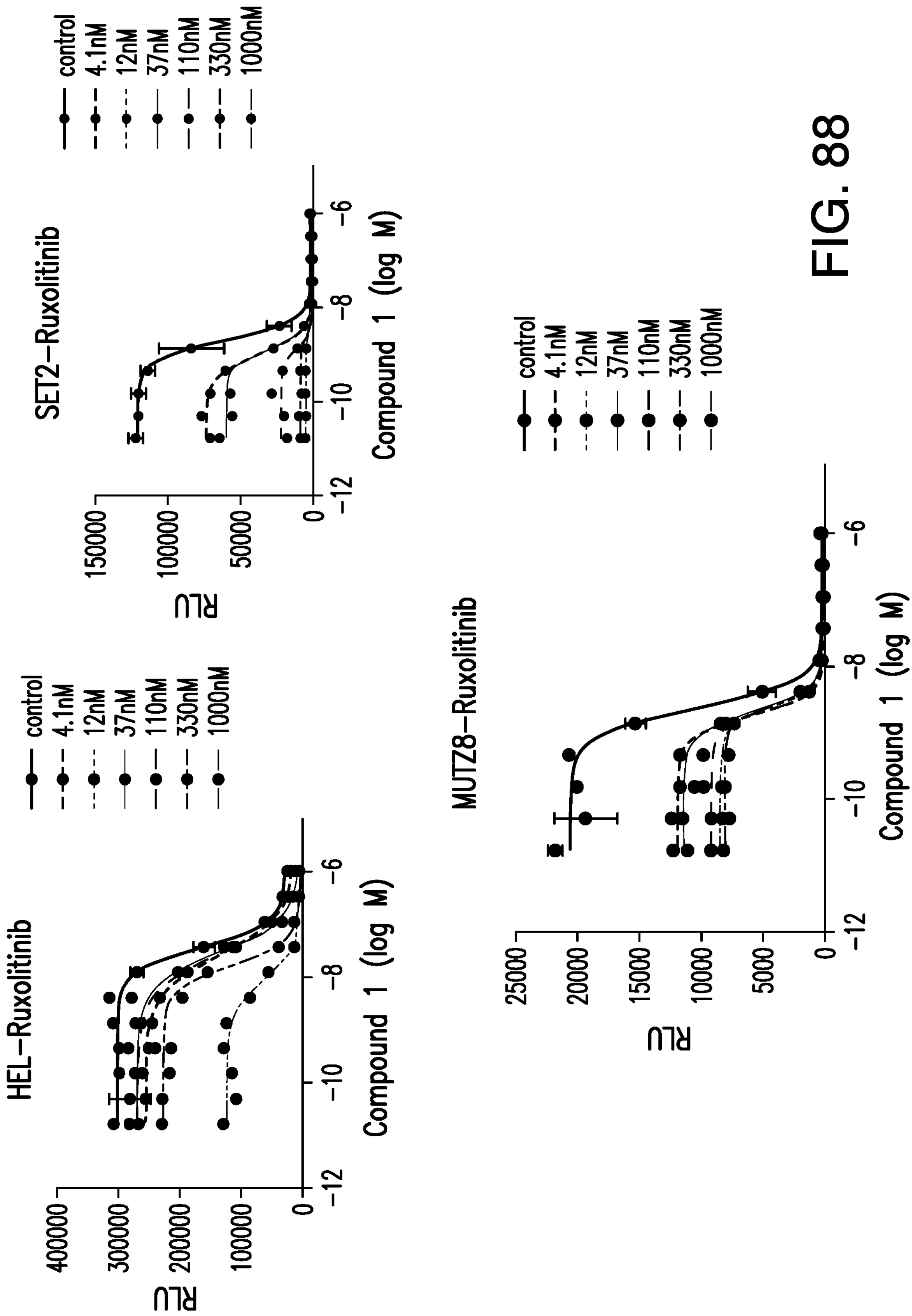
HEL-Ruxolitinib
RLU
400000
300000
200000
100000
0
-12
-10
-8
-6
Compound 1 (log M)
control
4.1nM
12nM
37nM
110nM
330nM
1000nM
SET2-Ruxolitinib
RLU
150000
100000
50000
0
-12
-10
-8
-6
Compound 1 (log M)
control
4.1nM
12nM
37nM
110nM
330nM
1000nM
MUTZ8-Ruxolitinib
RLU
25000
20000
15000
10000
5000
0
-12
-10
-8
-6
Compound 1 (log M)
control
4.1nM
12nM
37nM
110nM
330nM
1000nM

FIG. 88

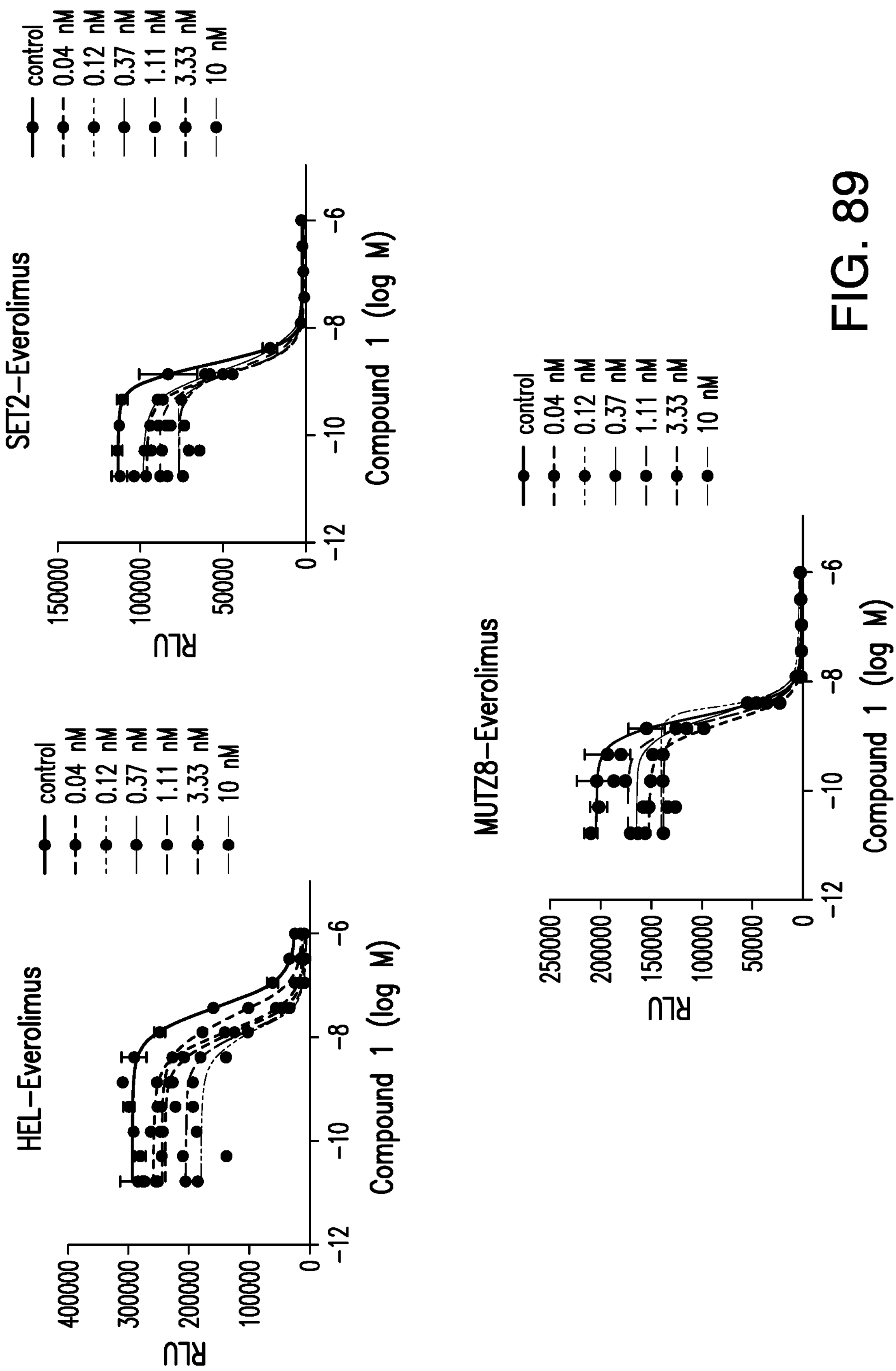
HEL–Everolimus
SET2–Everolimus
MUTZ8–Everolimus
RLU
Compound 1 (log M)
control
0.04 nM
0.12 nM
0.37 nM
1.11 nM
3.33 nM
10 nM
400000
300000
200000
100000
250000
150000
100000
50000
0
−12
−10
−8
−6

FIG. 89

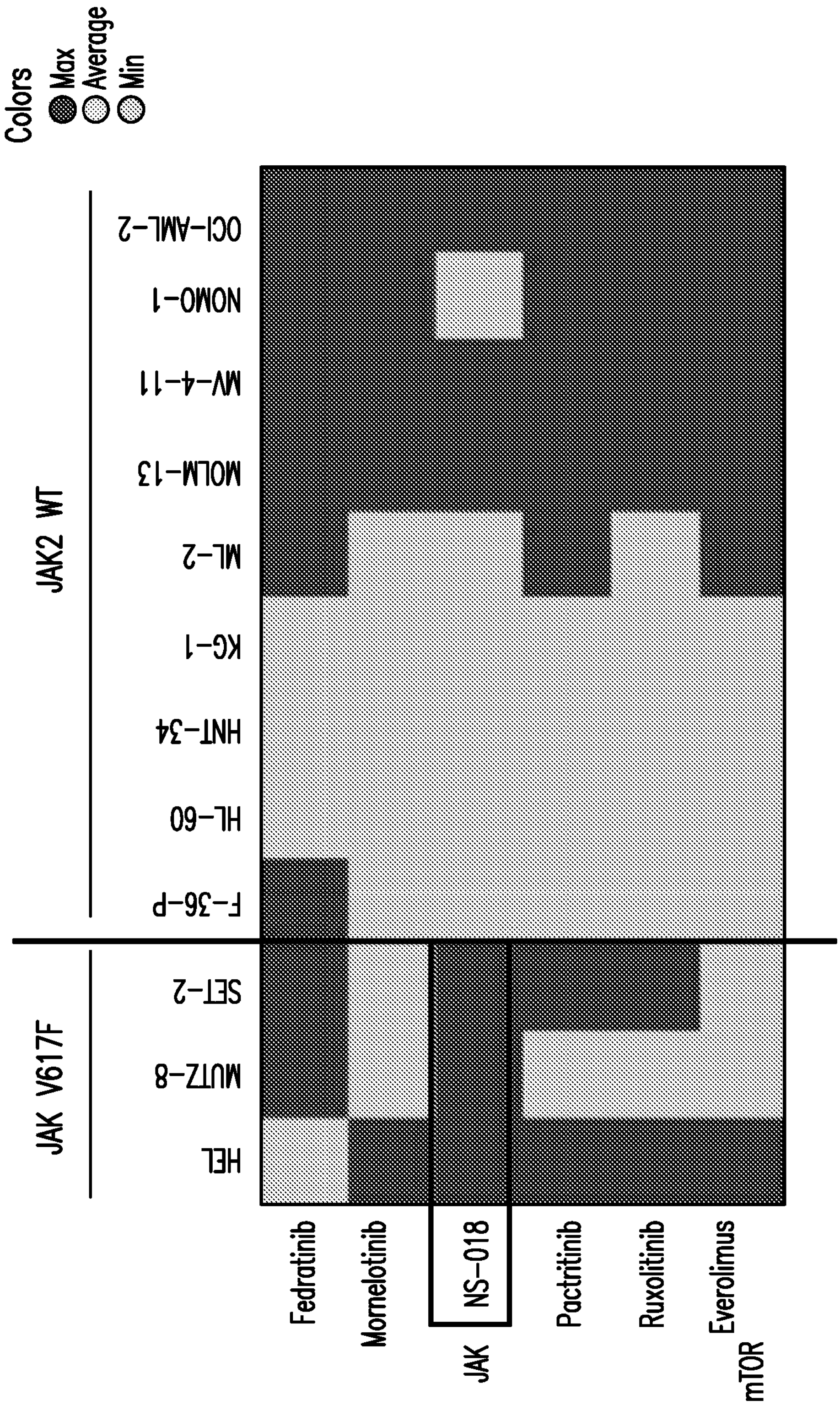
Colors
Max
Average
Min
JAK V617F
JAK2 WT
HEL
MUTZ-8
SET-2
F-36-P
HL-60
HNT-34
KG-1
ML-2
MOLM-13
MV-4-11
NOMO-1
OCI-AML-2
Fedratinib
Momelotinib
JAK
NS-018
Pacritinib
Ruxolitinib
mTOR
Everolimus

FIG. 90

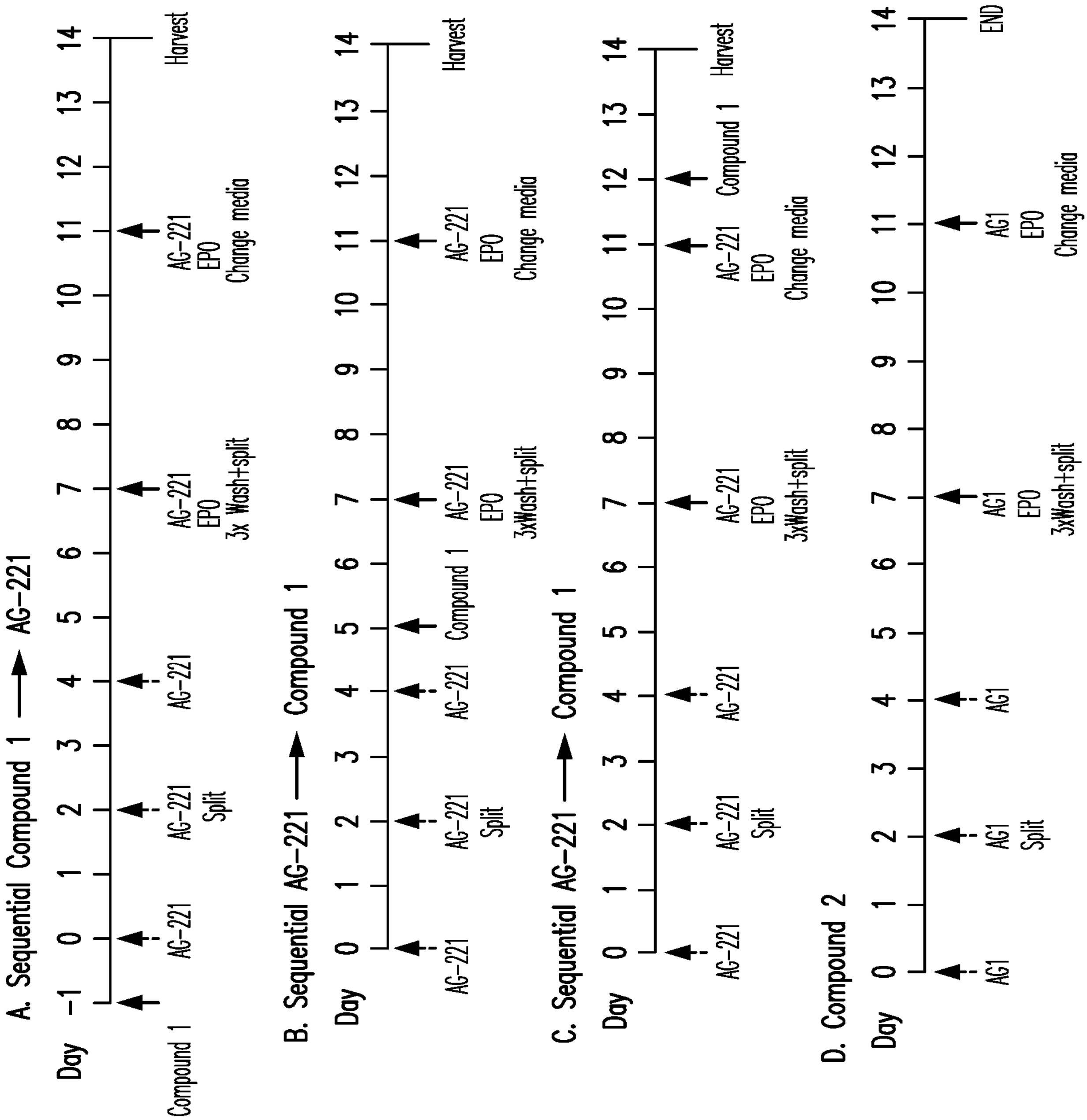
A. Sequential Compound 1 → AG-221
Day -1 0 1 2 3 4 5 6 7 8 9 10 11 12 13 14
Compound 1
AG-221
AG-221 Split
AG-221
AG-221 EPO 3x Wash+split
AG-221 EPO Change media
Harvest
B. Sequential AG-221 → Compound 1
Day 0 1 2 3 4 5 6 7 8 9 10 11 12 13 14
AG-221
AG-221 Split
AG-221
Compound 1
AG-221 EPO 3xWash+split
AG-221 EPO Change media
Harvest
C. Sequential AG-221 → Compound 1
Day 0 1 2 3 4 5 6 7 8 9 10 11 12 13 14
AG-221
AG-221 Split
AG-221
AG-221 EPO 3xWash+split
AG-221 EPO Change media
Compound 1
Harvest
D. Compound 2
Day 0 1 2 3 4 5 6 7 8 9 10 11 12 13 14
AG1
AG1 Split
AG1
AG1 EPO 3xWash+split
AG1 EPO Change media
END

FIG. 91

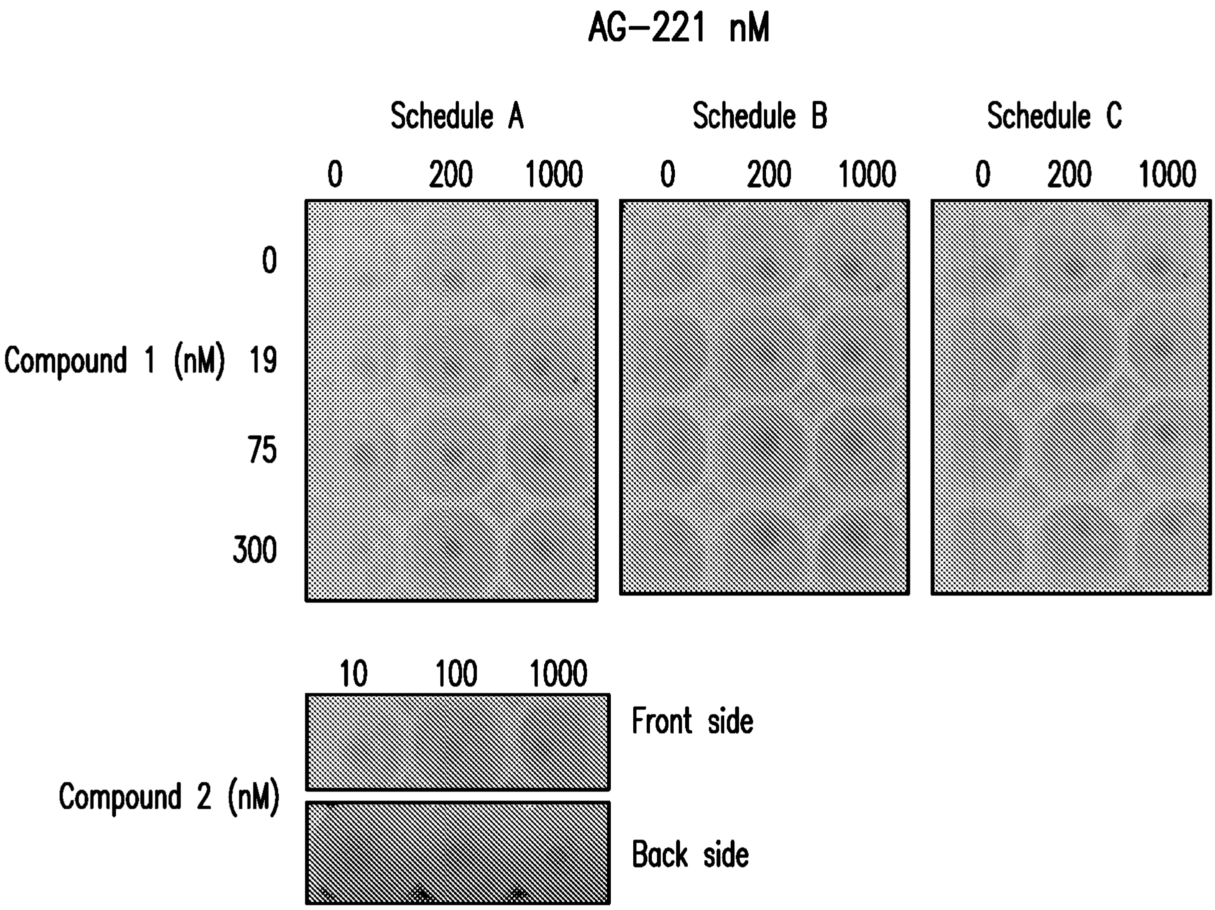
AG-221 nM
Schedule A
Schedule B
Schedule C
0
200
1000
0
200
1000
0
200
1000
Compound 1 (nM)
0
19
75
300
10
100
1000
Front side
Compound 2 (nM)
Back side

FIG. 92

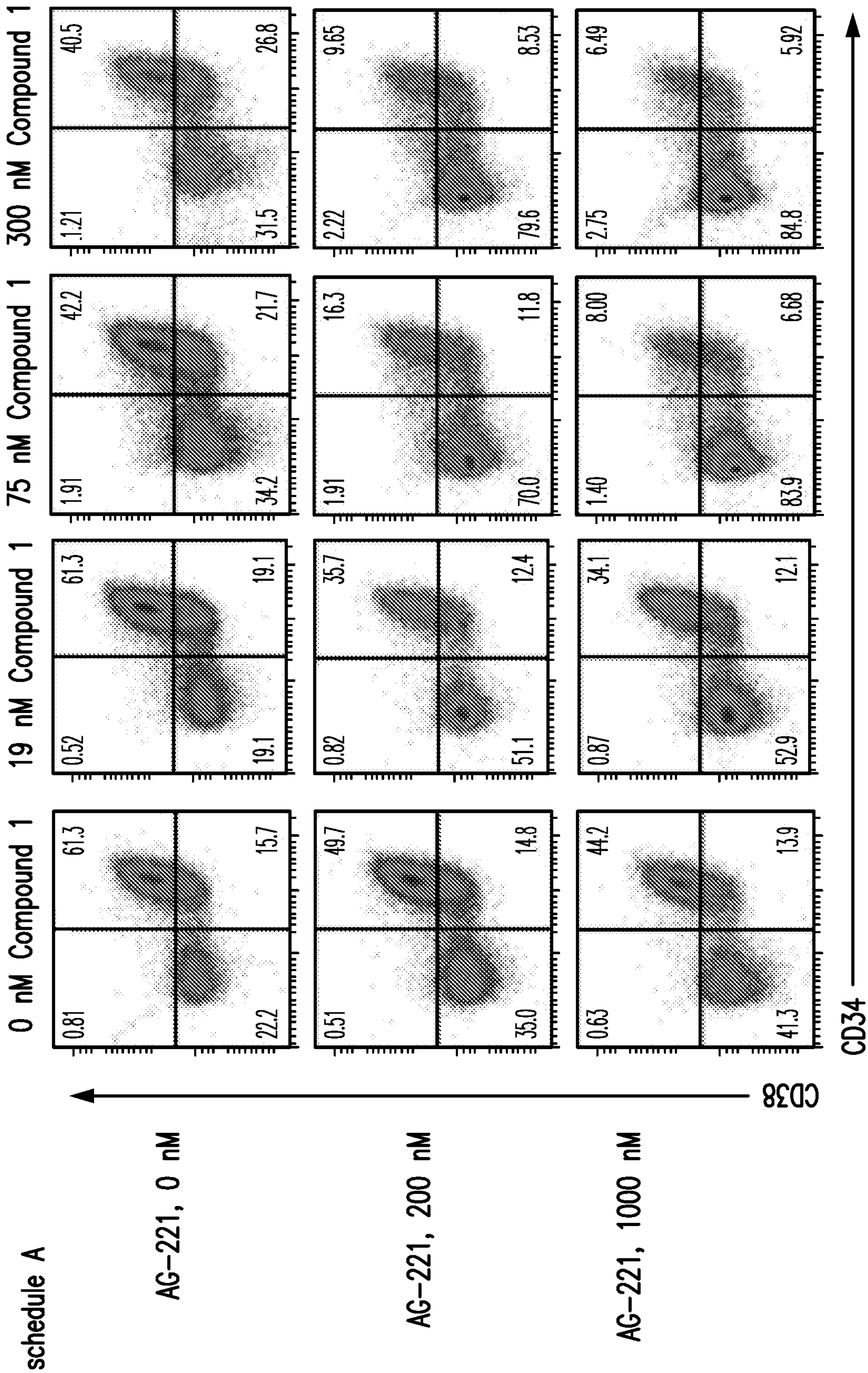
schedule A
0 nM Compound 1
19 nM Compound 1
75 nM Compound 1
300 nM Compound 1
AG-221, 0 nM
0.81
61.3
22.2
15.7
0.52
61.3
19.1
19.1
1.91
42.2
34.2
21.7
1.21
40.5
31.5
26.8
AG-221, 200 nM
0.51
49.7
35.0
14.8
0.82
35.7
51.1
12.4
1.91
16.3
70.0
11.8
2.22
9.65
79.6
8.53
AG-221, 1000 nM
0.63
44.2
41.3
13.9
0.87
34.1
52.9
12.1
1.40
8.00
83.9
6.68
2.75
6.49
84.8
5.92
CD38
CD34

FIG. 93

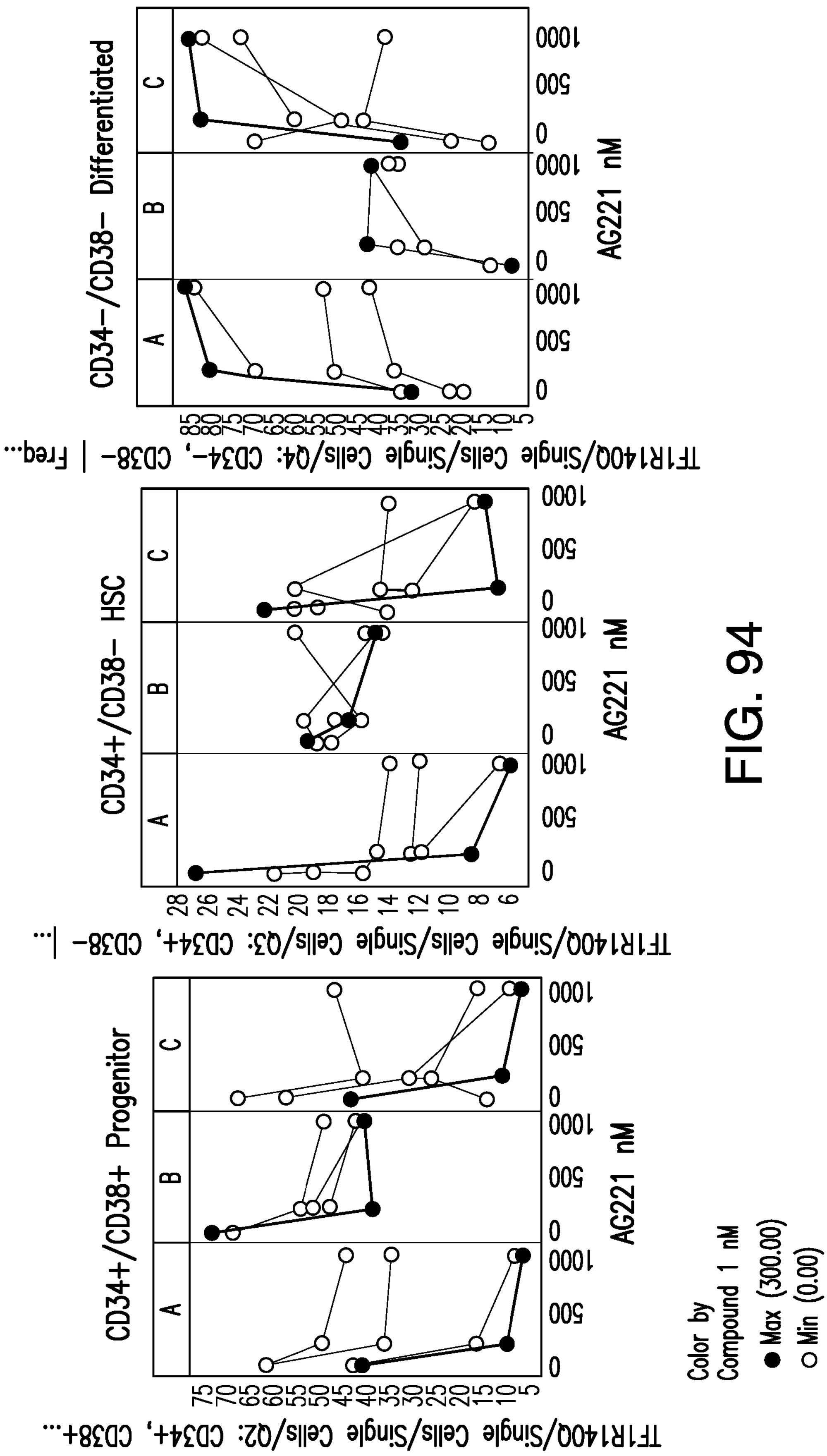
CD34+/CD38+ Progenitor
CD34+/CD38− HSC
CD34−/CD38− Differentiated
A
B
C
AG221 nM
TF1R140Q/Single Cells/Single Cells/Q2: CD34+, CD38+...
TF1R140Q/Single Cells/Single Cells/Q3: CD34+, CD38− |...
TF1R140Q/Single Cells/Single Cells/Q4: CD34−, CD38− | Freq...
Color by
Compound 1 nM
Max (300.00)
Min (0.00)

FIG. 94

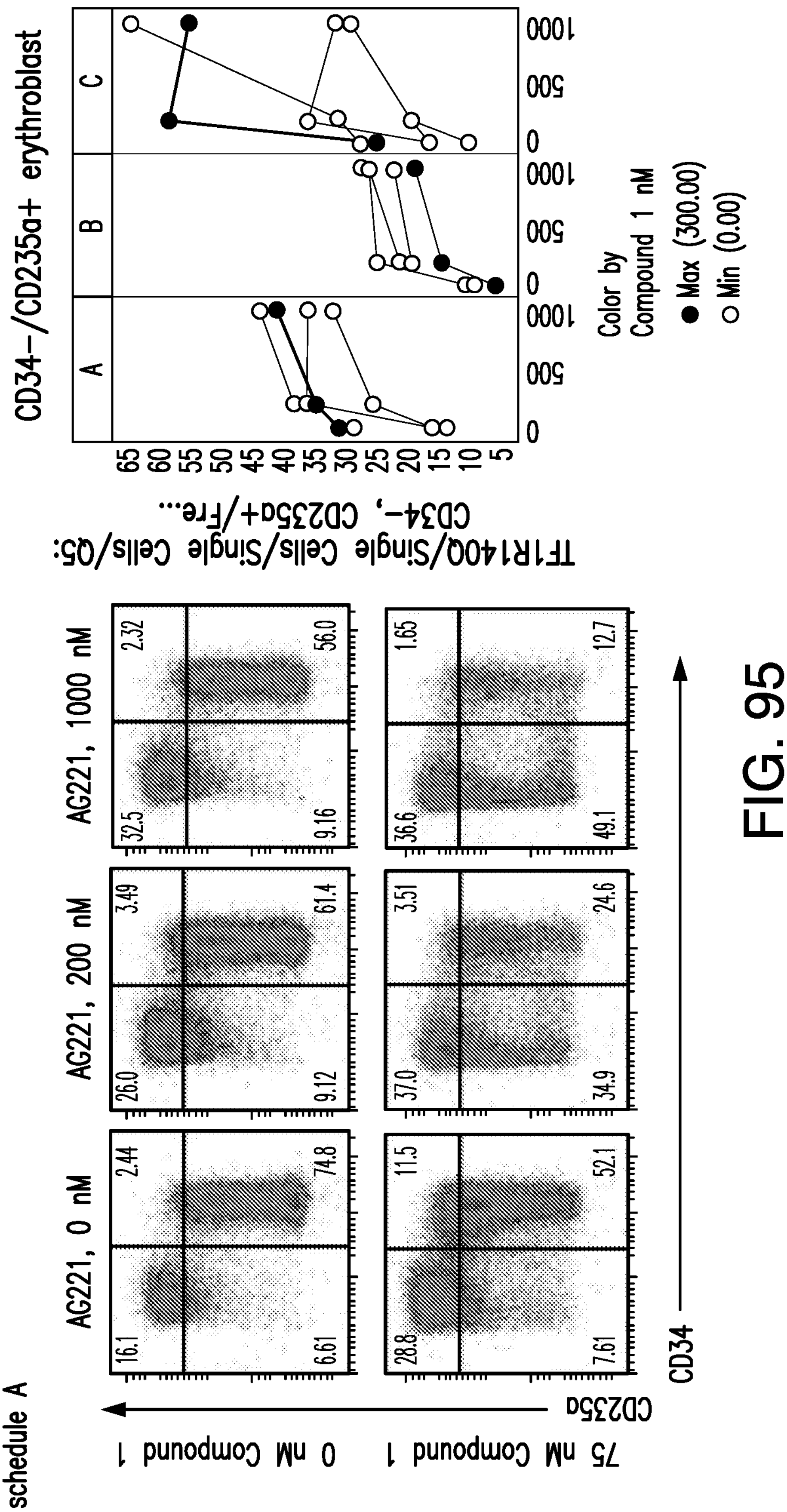
CD34−/CD235a+ erythroblast
A
B
C
TF1R140Q/Single Cells/Single Cells/Q5: CD34−, CD235a+/Fre...
0
500
1000
65
60
55
50
45
40
35
30
25
20
15
10
5
Color by Compound 1 nM
Max (300.00)
Min (0.00)
schedule A
AG221, 0 nM
AG221, 200 nM
AG221, 1000 nM
0 nM Compound 1
75 nM Compound 1
CD235a
CD34
16.1
2.44
6.61
74.8
26.0
3.49
9.12
61.4
32.5
2.32
9.16
56.0
28.8
11.5
7.61
52.1
37.0
3.51
34.9
24.6
36.6
1.65
49.1
12.7

FIG. 95

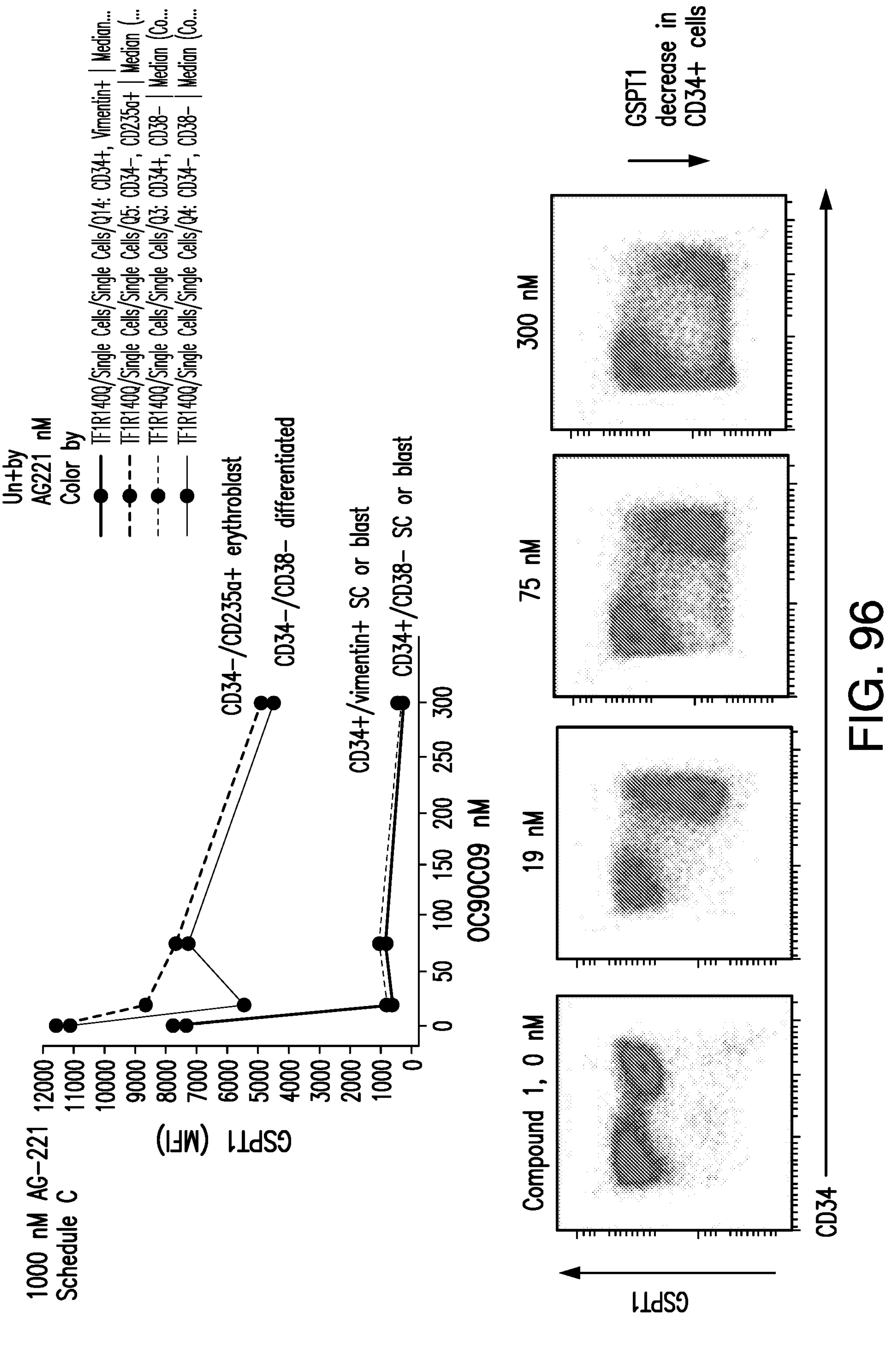
1000 nM AG-221
Schedule C
GSPT1 (MFI)
12000
11000
10000
9000
8000
7000
6000
5000
4000
3000
2000
1000
0
0
50
100
150
200
250
300
OC90C09 nM
Un+by
AG221 nM
Color by
TF1R140Q/Single Cells/Single Cells/Q14: CD34+, Vimentin+ | Median...
TF1R140Q/Single Cells/Single Cells/Q5: CD34-, CD235a+ | Median (...
TF1R140Q/Single Cells/Single Cells/Q3: CD34+, CD38- | Median (Co...
TF1R140Q/Single Cells/Single Cells/Q4: CD34-, CD38- | Median (Co...
CD34-/CD235a+ erythroblast
CD34-/CD38- differentiated
CD34+/vimentin+ SC or blast
CD34+/CD38- SC or blast
Compound 1, 0 nM
19 nM
75 nM
300 nM
GSPT1
CD34
GSPT1
decrease in
CD34+ cells

FIG. 96

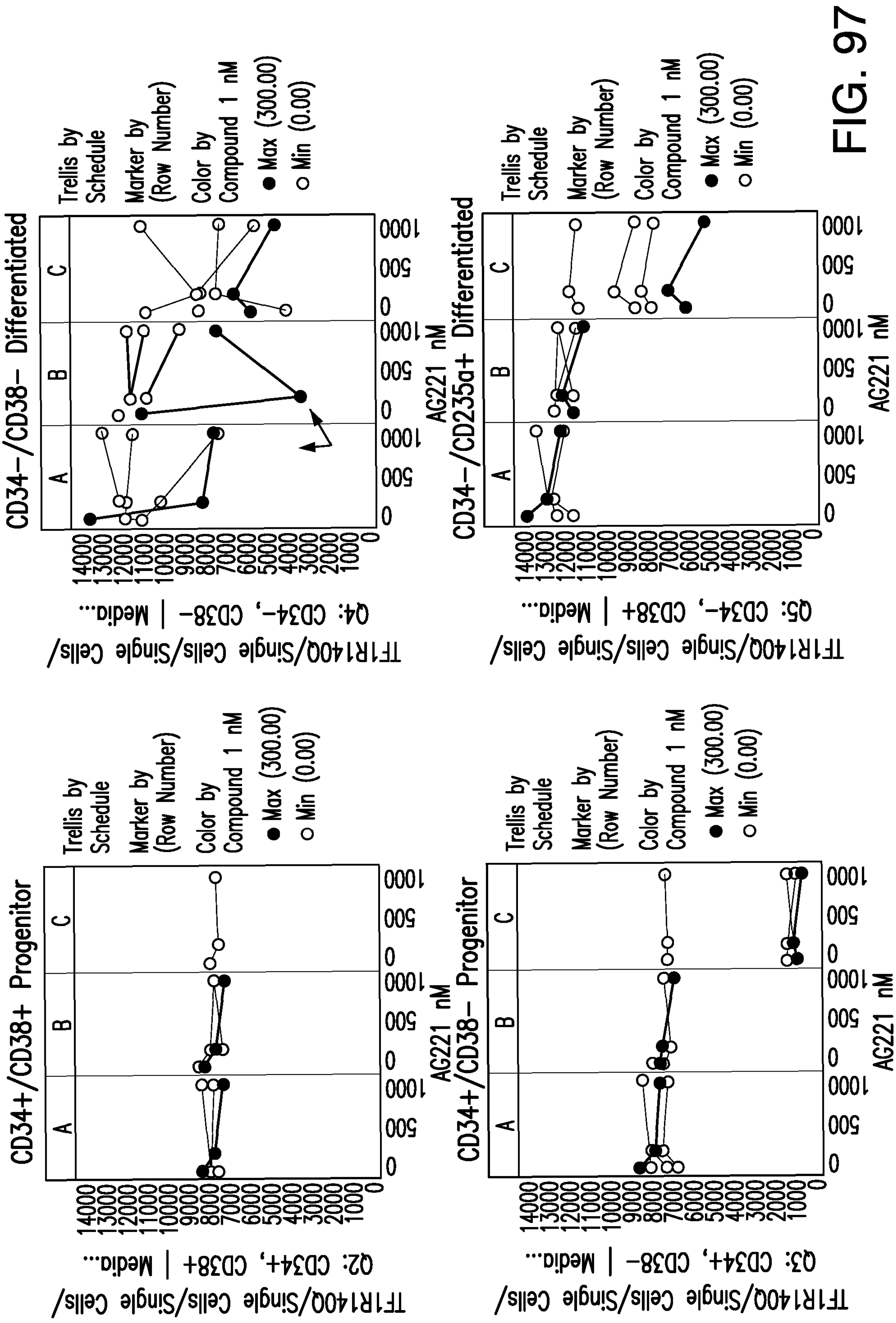
CD34-/CD38- Differentiated
A
B
C
TF1R140Q/Single Cells/Single Cells/
Q4: CD34-, CD38- | Media...
14000
13000
12000
11000
10000
9000
8000
7000
6000
5000
4000
3000
2000
1000
0
0
500
1000
AG221 nM
Trellis by
Schedule
Marker by
(Row Number)
Color by
Compound 1 nM
Max (300.00)
Min (0.00)
CD34-/CD235a+ Differentiated
Q5: CD34-, CD38+ | Media...
CD34+/CD38+ Progenitor
Q2: CD34+, CD38+ | Media...
CD34+/CD38- Progenitor
Q3: CD34+, CD38- | Media...

FIG. 97

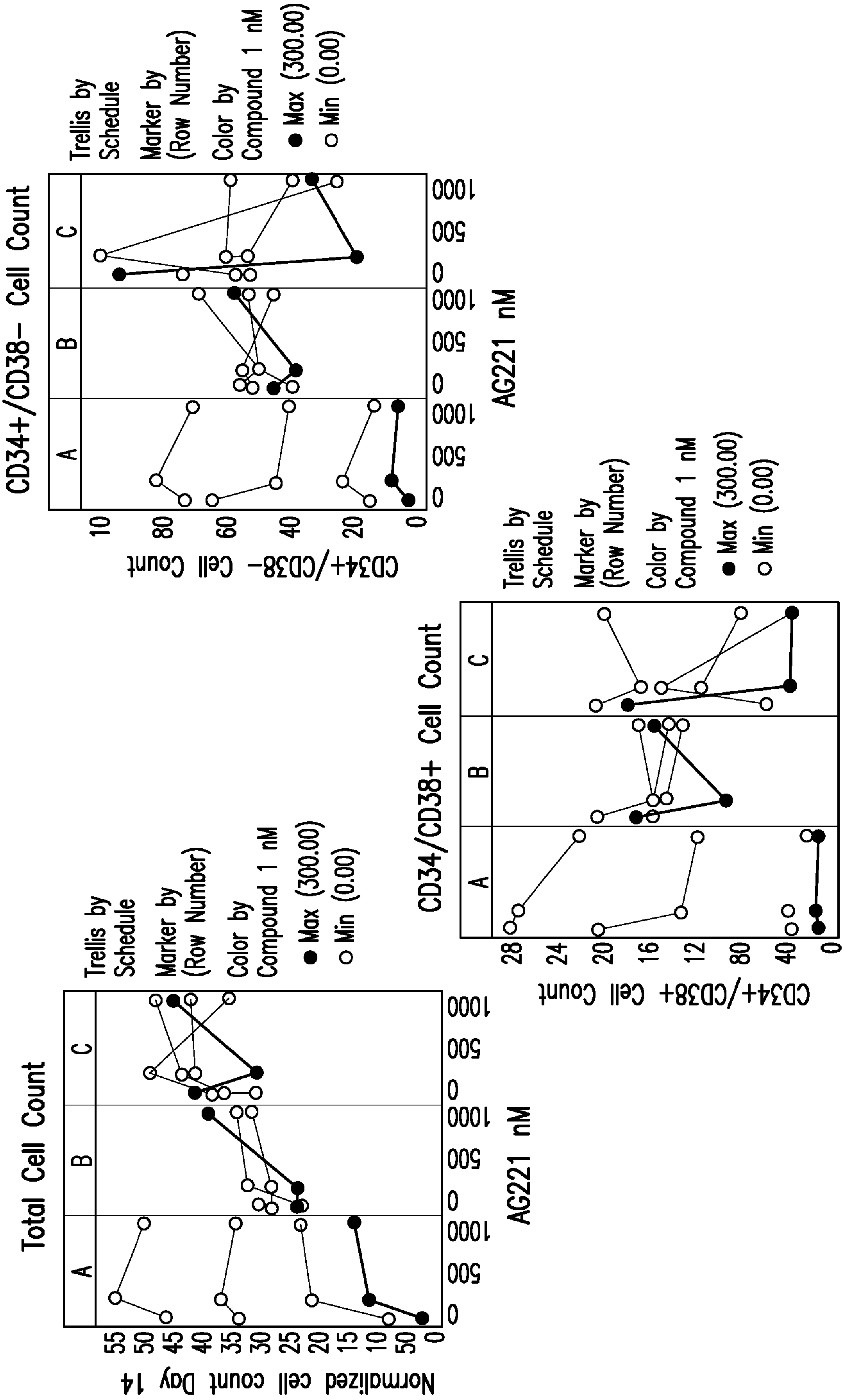
CD34+/CD38- Cell Count
CD34+/CD38- Cell Count
A
B
C
AG221 nM
Trellis by Schedule
Marker by (Row Number)
Color by Compound 1 nM
Max (300.00)
Min (0.00)
CD34/CD38+ Cell Count
CD34+/CD38+ Cell Count
A
B
C
AG221 nM
Trellis by Schedule
Marker by (Row Number)
Color by Compound 1 nM
Max (300.00)
Min (0.00)
Total Cell Count
Normalized cell count Day 14
A
B
C
AG221 nM
Trellis by Schedule
Marker by (Row Number)
Color by Compound 1 nM
Max (300.00)
Min (0.00)

FIG. 98

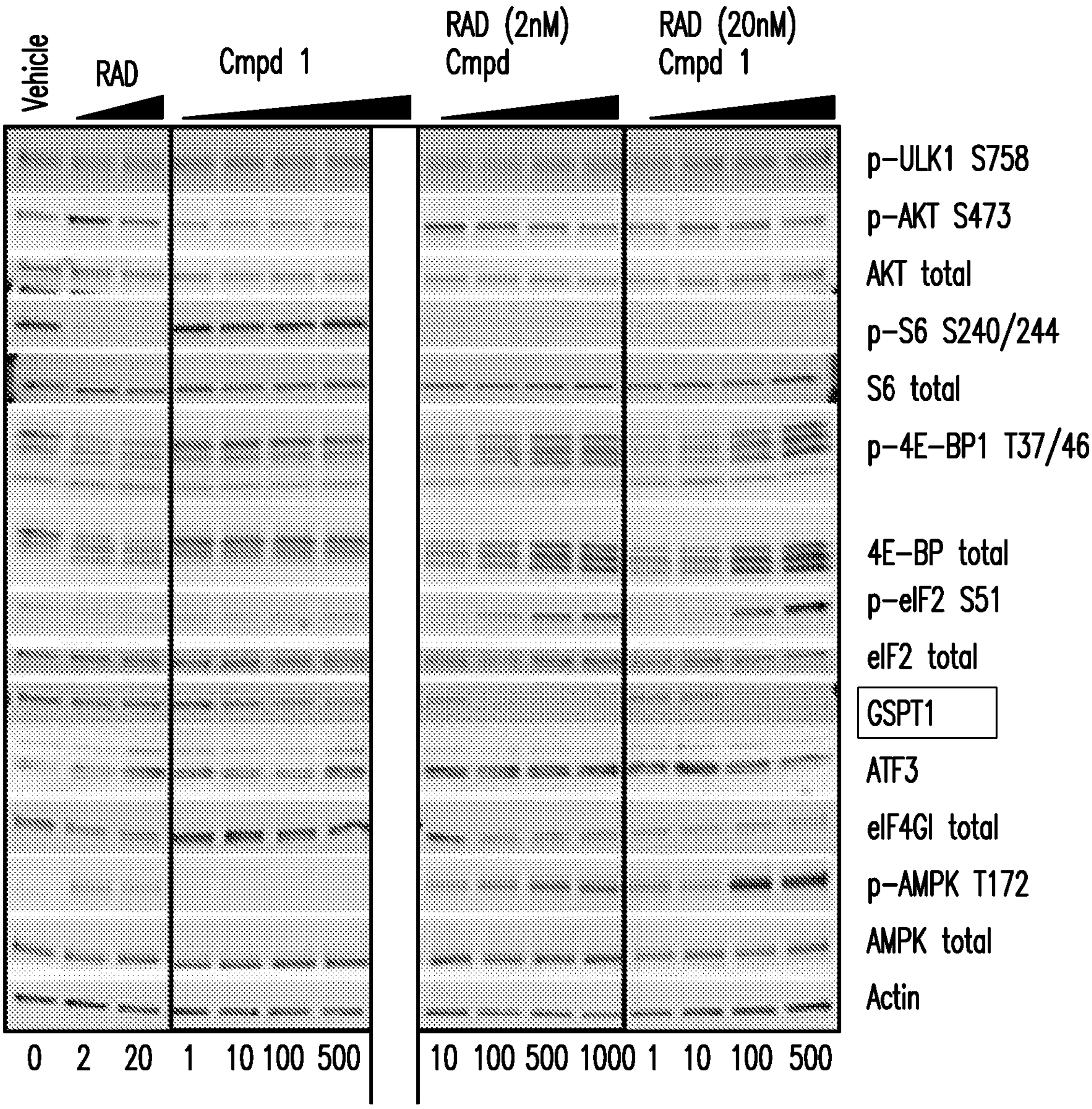
Vehicle
RAD
Cmpd 1
RAD (2nM)
Cmpd
RAD (20nM)
Cmpd 1
p-ULK1 S758
p-AKT S473
AKT total
p-S6 S240/244
S6 total
p-4E-BP1 T37/46
4E-BP total
p-eIF2 S51
eIF2 total
GSPT1
ATF3
eIF4GI total
p-AMPK T172
AMPK total
Actin
0 2 20 1 10 100 500
10 100 500 1000 1 10 100 500

FIG. 99

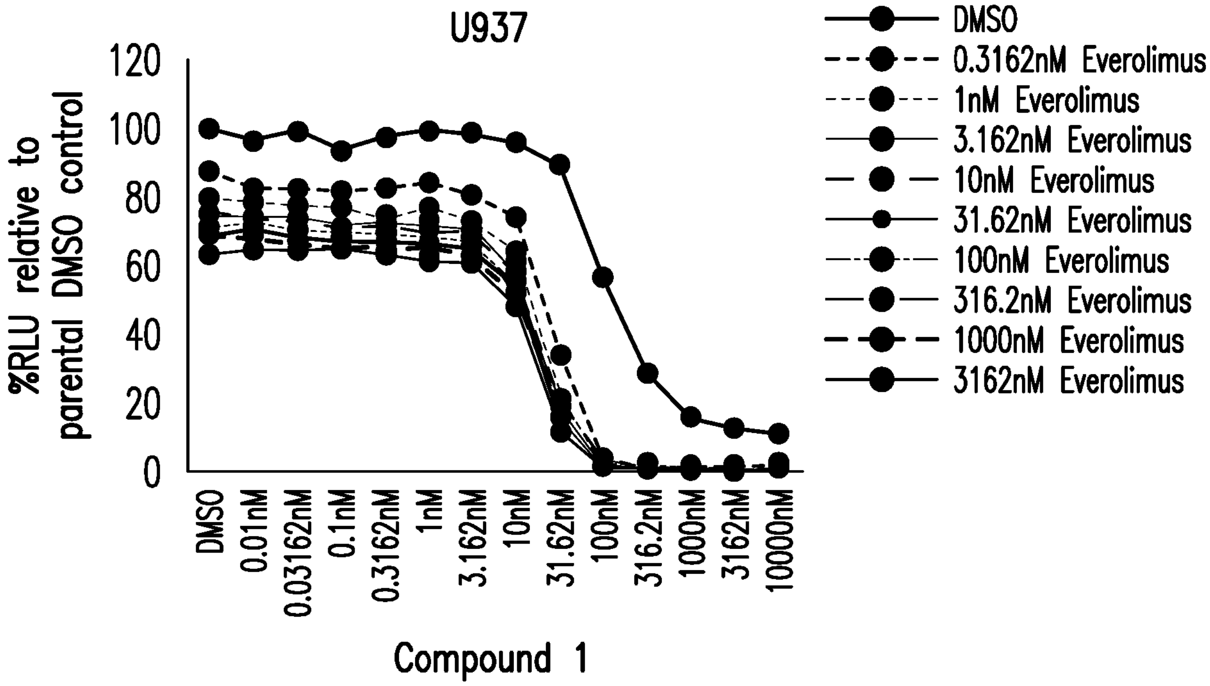


FIG. 100A

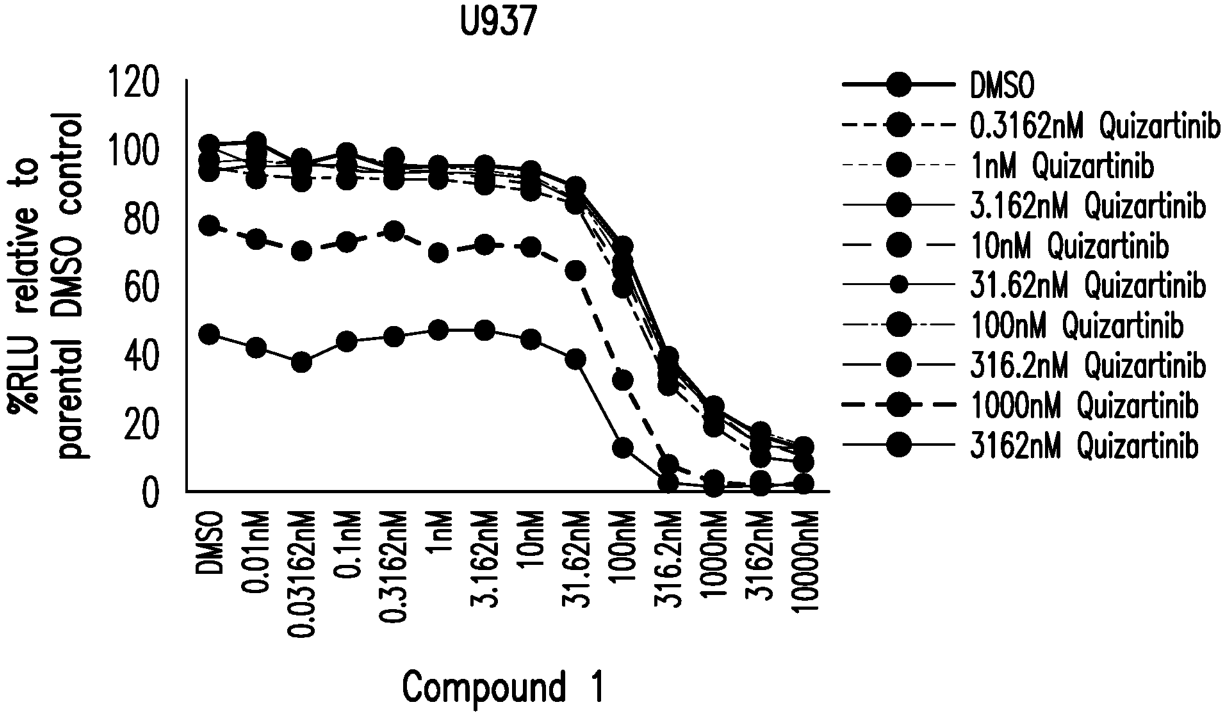


FIG. 100B

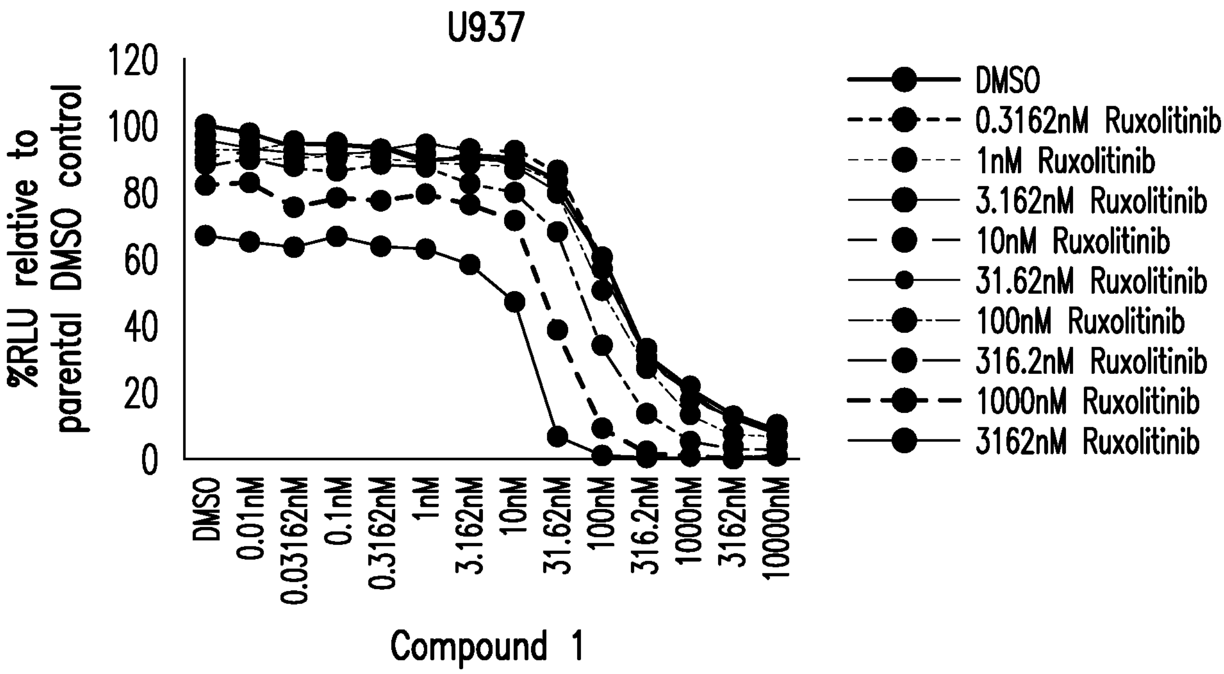


FIG. 100C

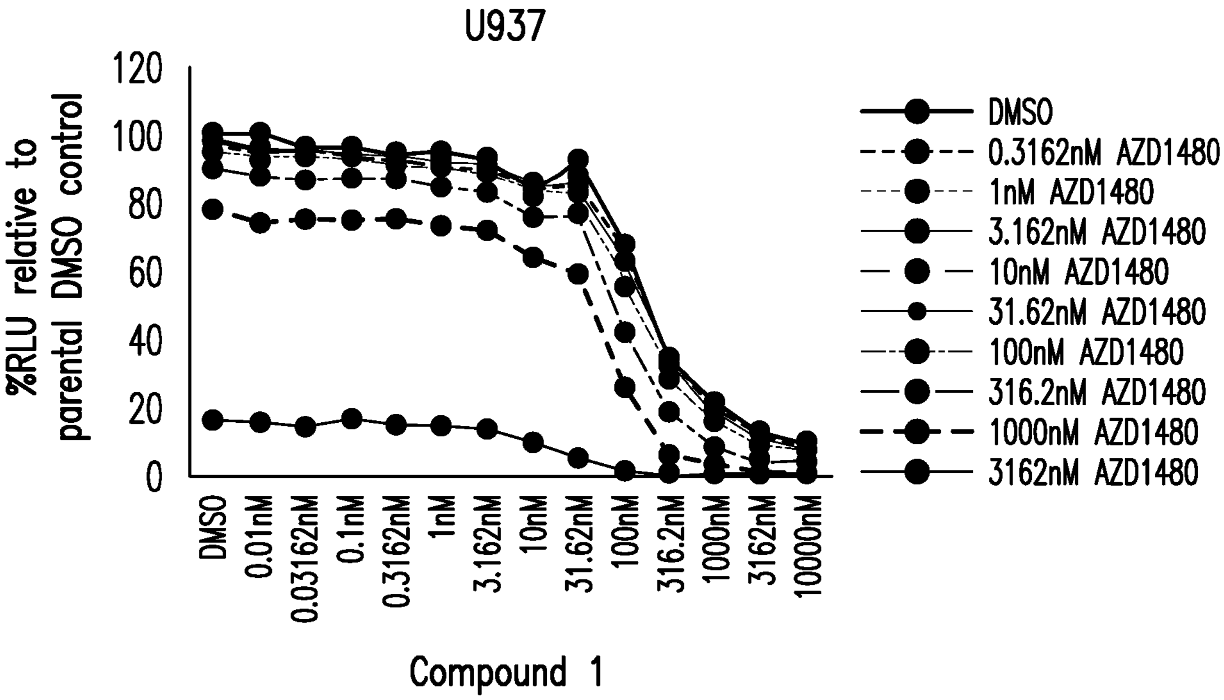


FIG. 100D

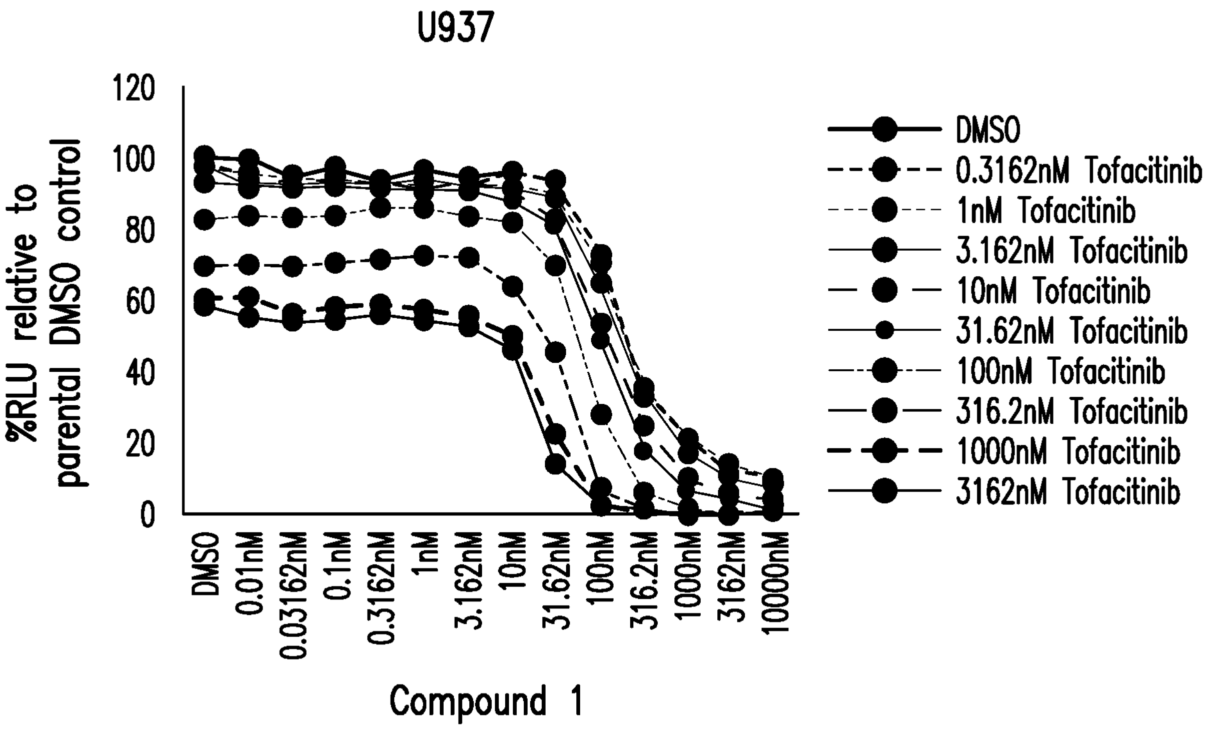
U937
%RLU relative to parental DMSO control
120
100
80
60
40
20
0
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Tofacitinib
1nM Tofacitinib
3.162nM Tofacitinib
10nM Tofacitinib
31.62nM Tofacitinib
100nM Tofacitinib
316.2nM Tofacitinib
1000nM Tofacitinib
3162nM Tofacitinib

FIG. 100E

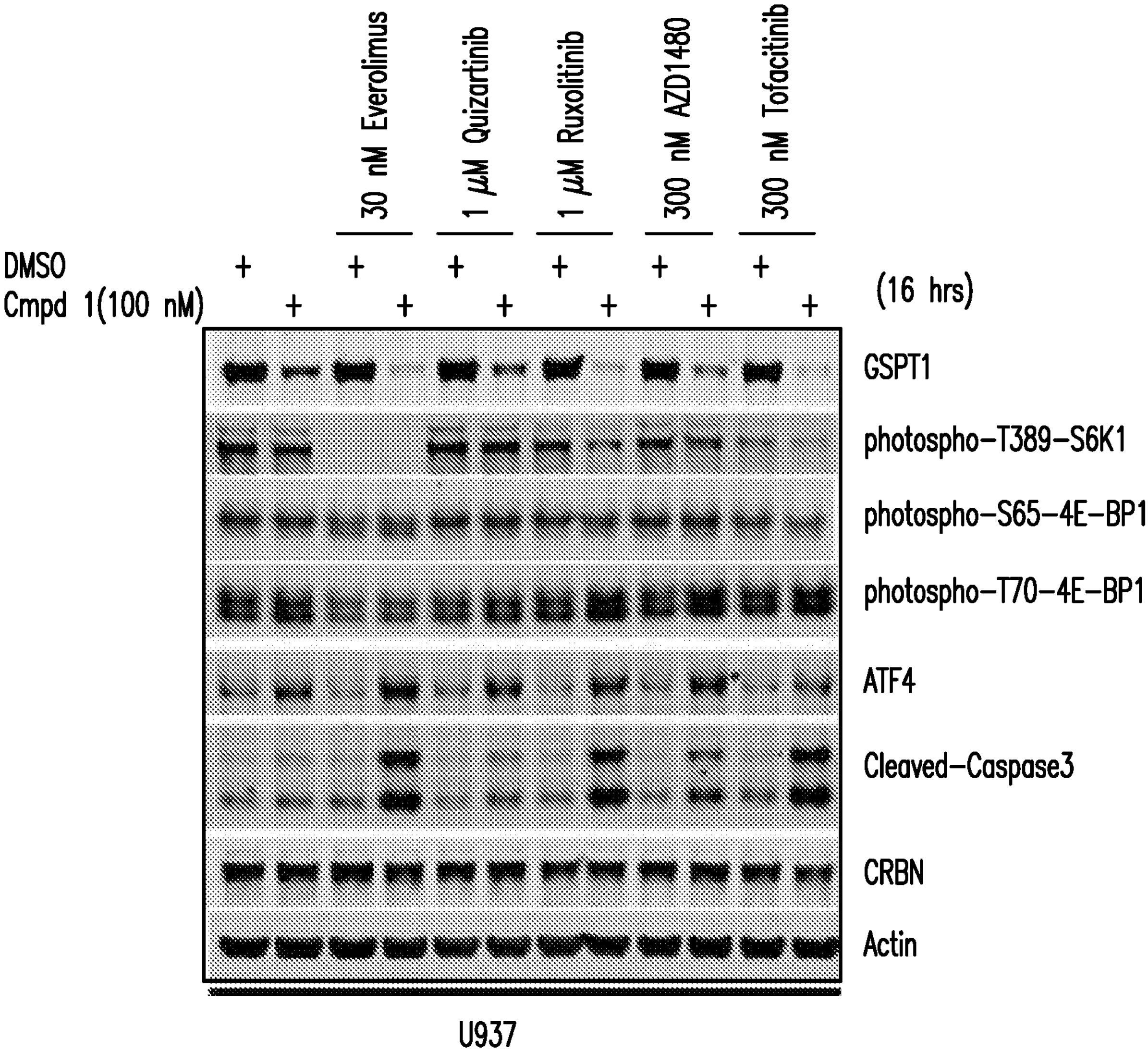
30 nM Everolimus
1 μM Quizartinib
1 μM Ruxolitinib
300 nM AZD1480
300 nM Tofacitinib
DMSO
Cmpd 1(100 nM)
(16 hrs)
GSPT1
photospho-T389-S6K1
photospho-S65-4E-BP1
photospho-T70-4E-BP1
ATF4
Cleaved-Caspase3
CRBN
Actin
U937

FIG. 101

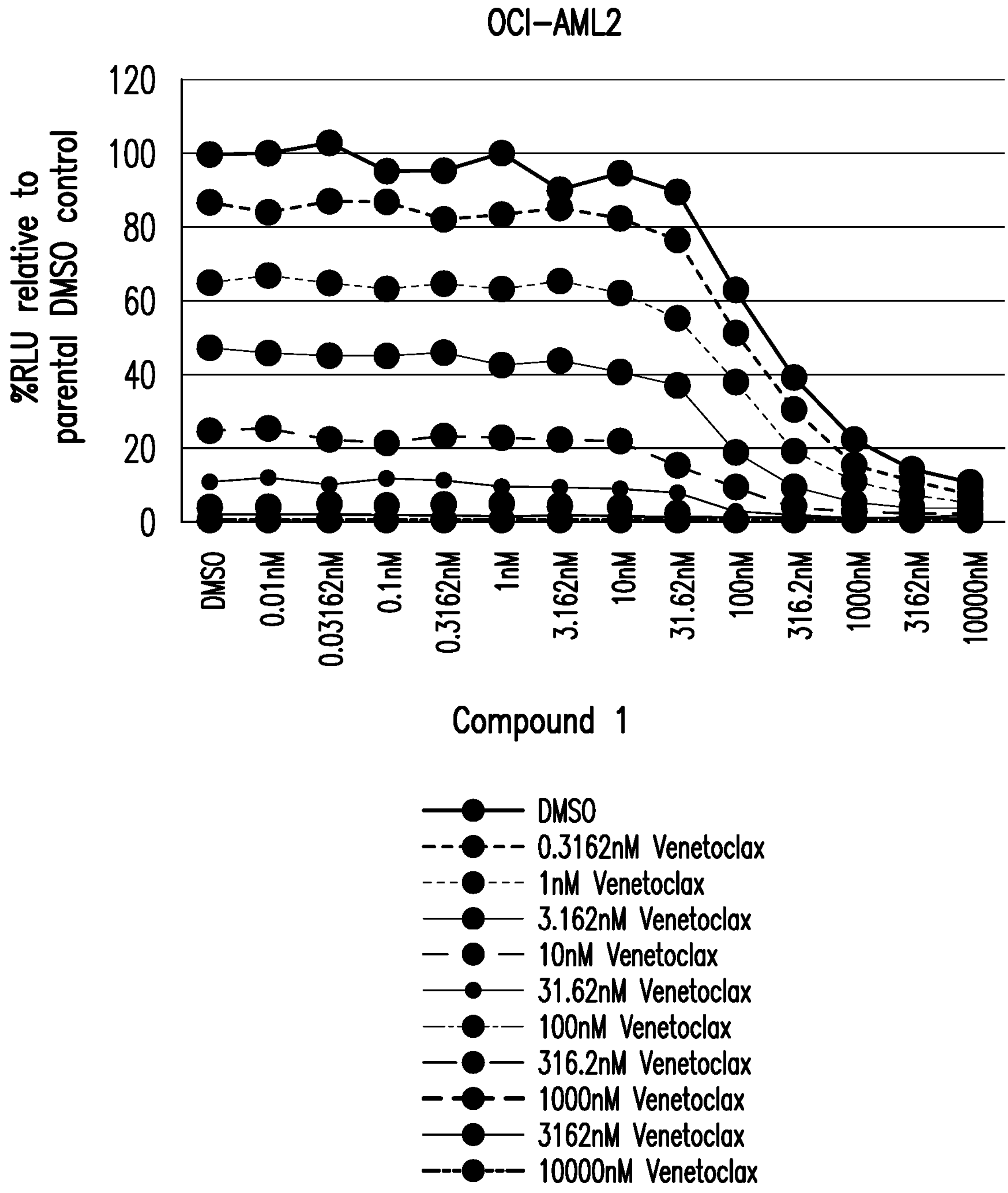
OCI-AML2
%RLU relative to parental DMSO control
120
100
80
60
40
20
0
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102A

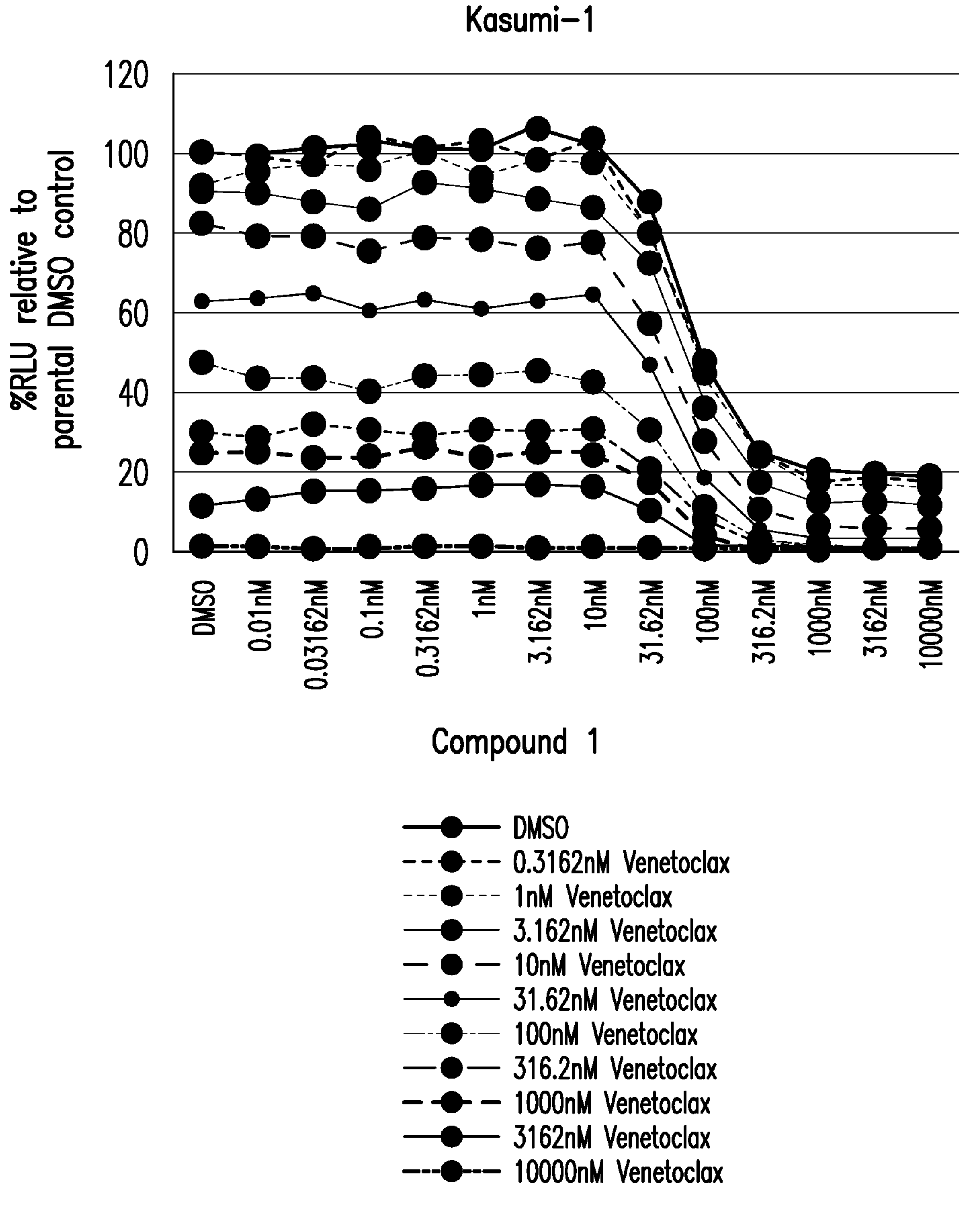
Kasumi-1
120
100
80
60
40
20
0
%RLU relative to parental DMSO control
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102B

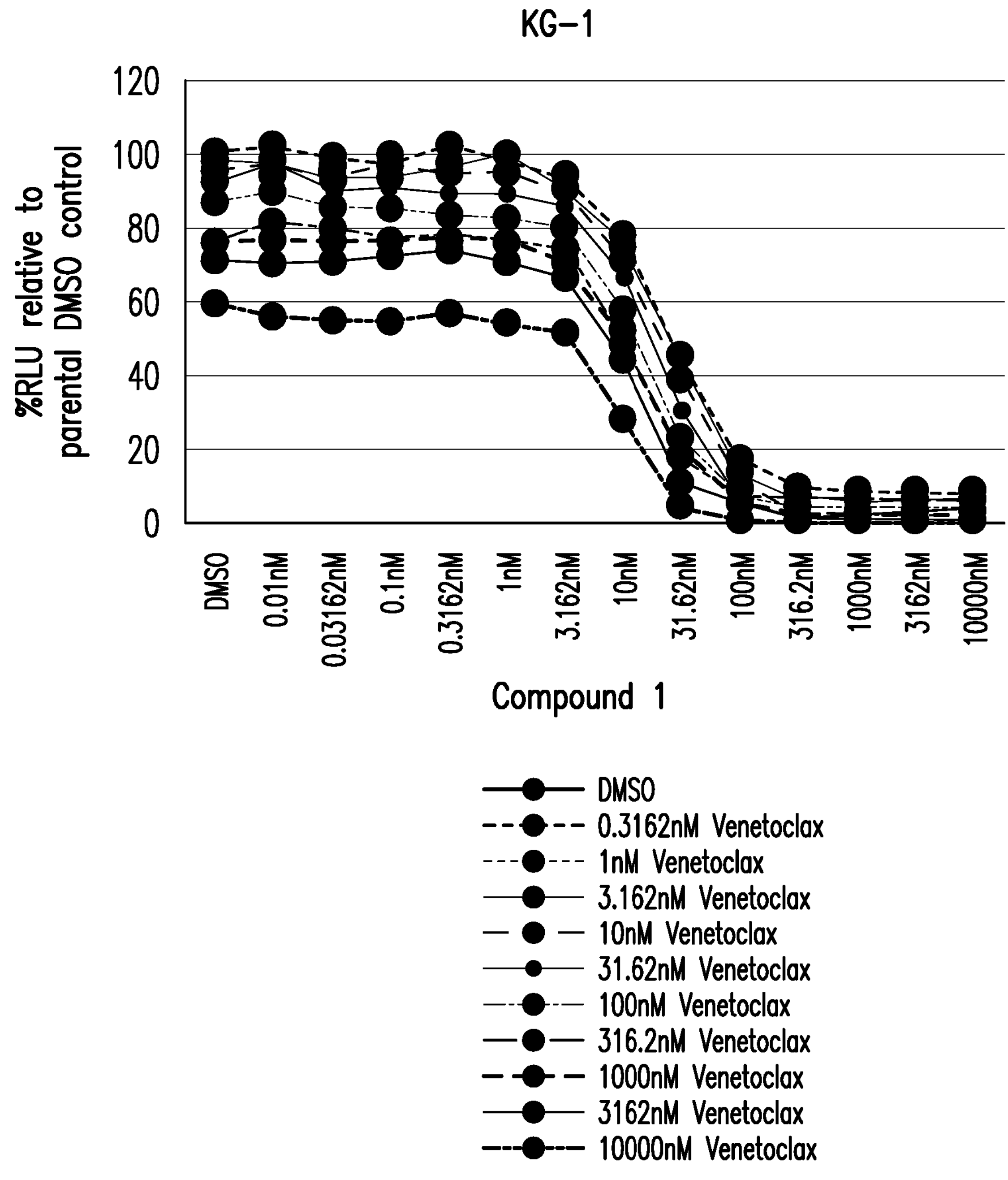
KG-1
120
100
80
60
40
20
0
%RLU relative to parental DMSO control
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102C

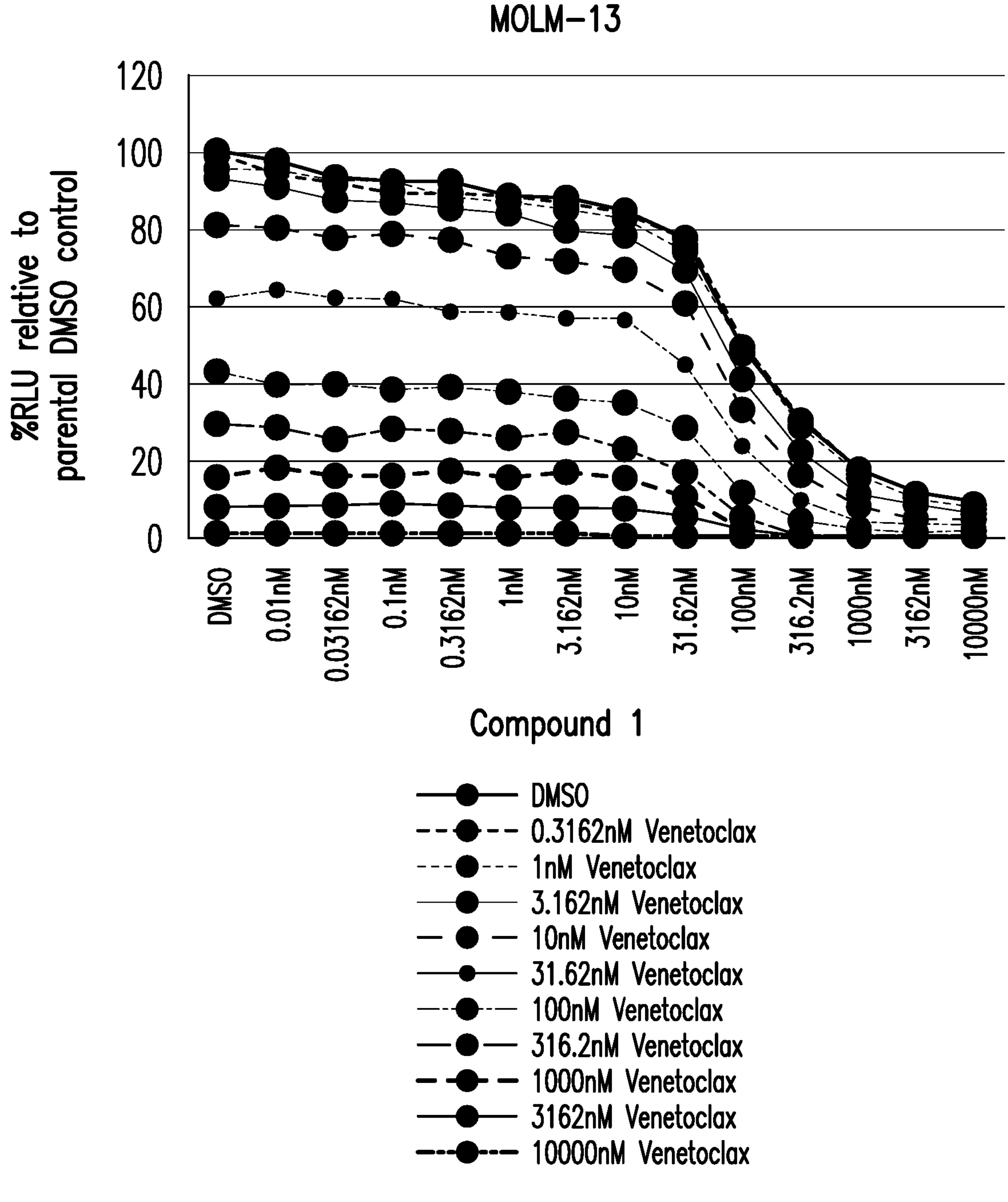
MOLM-13
120
100
80
60
40
20
0
%RLU relative to parental DMSO control
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102D

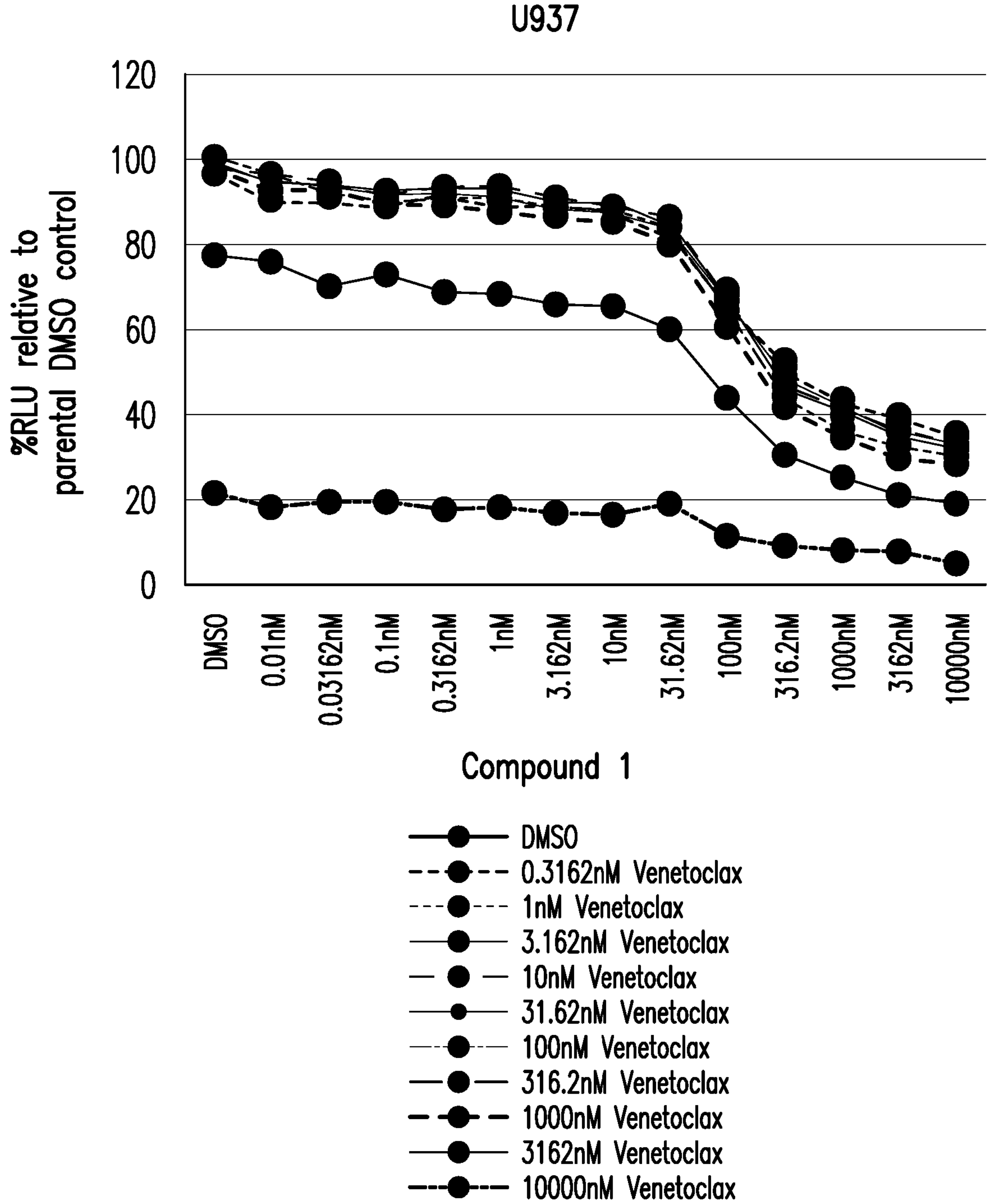
U937
%RLU relative to parental DMSO control
120
100
80
60
40
20
0
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102E

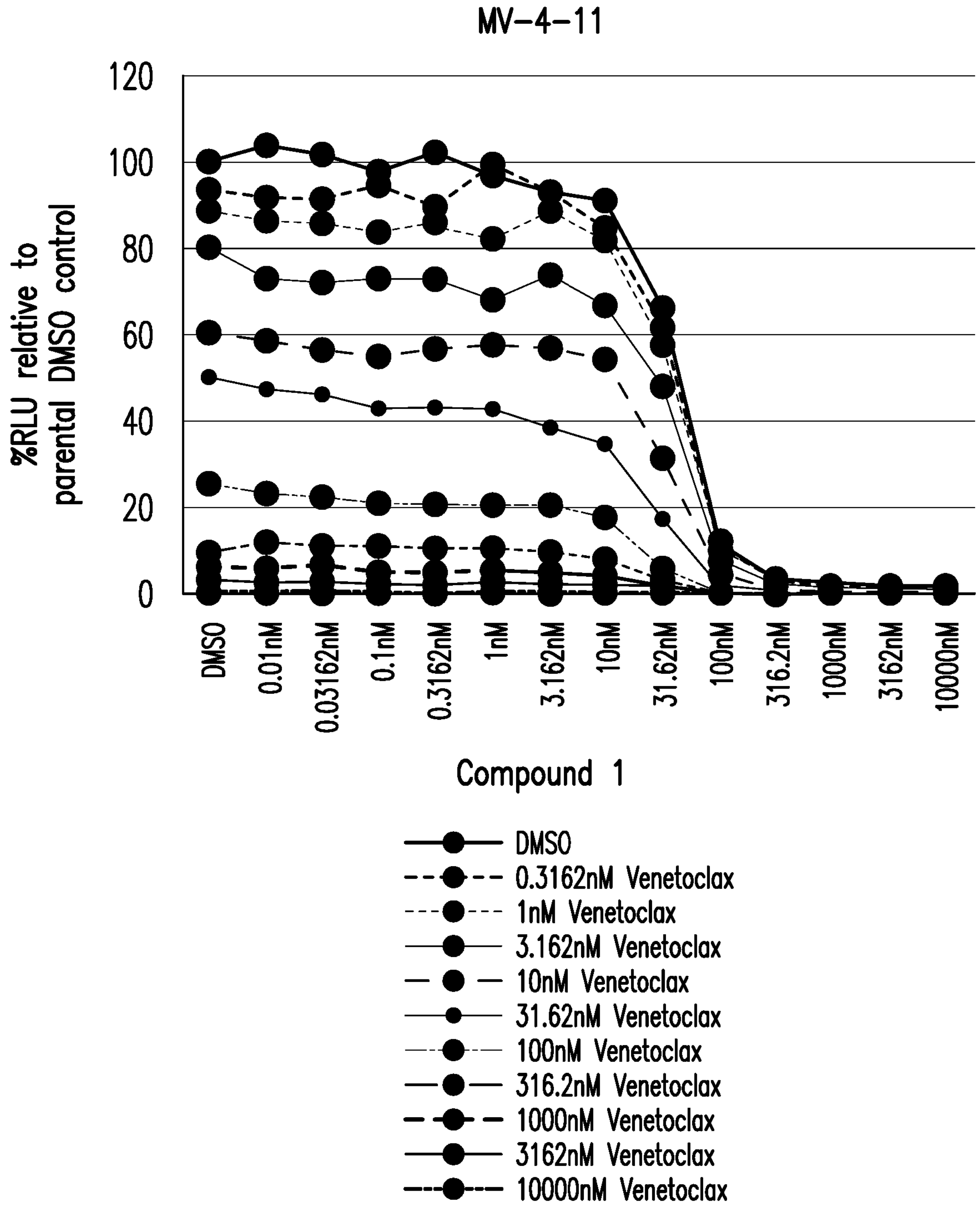
MV-4-11
%RLU relative to parental DMSO control
120
100
80
60
40
20
0
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102F

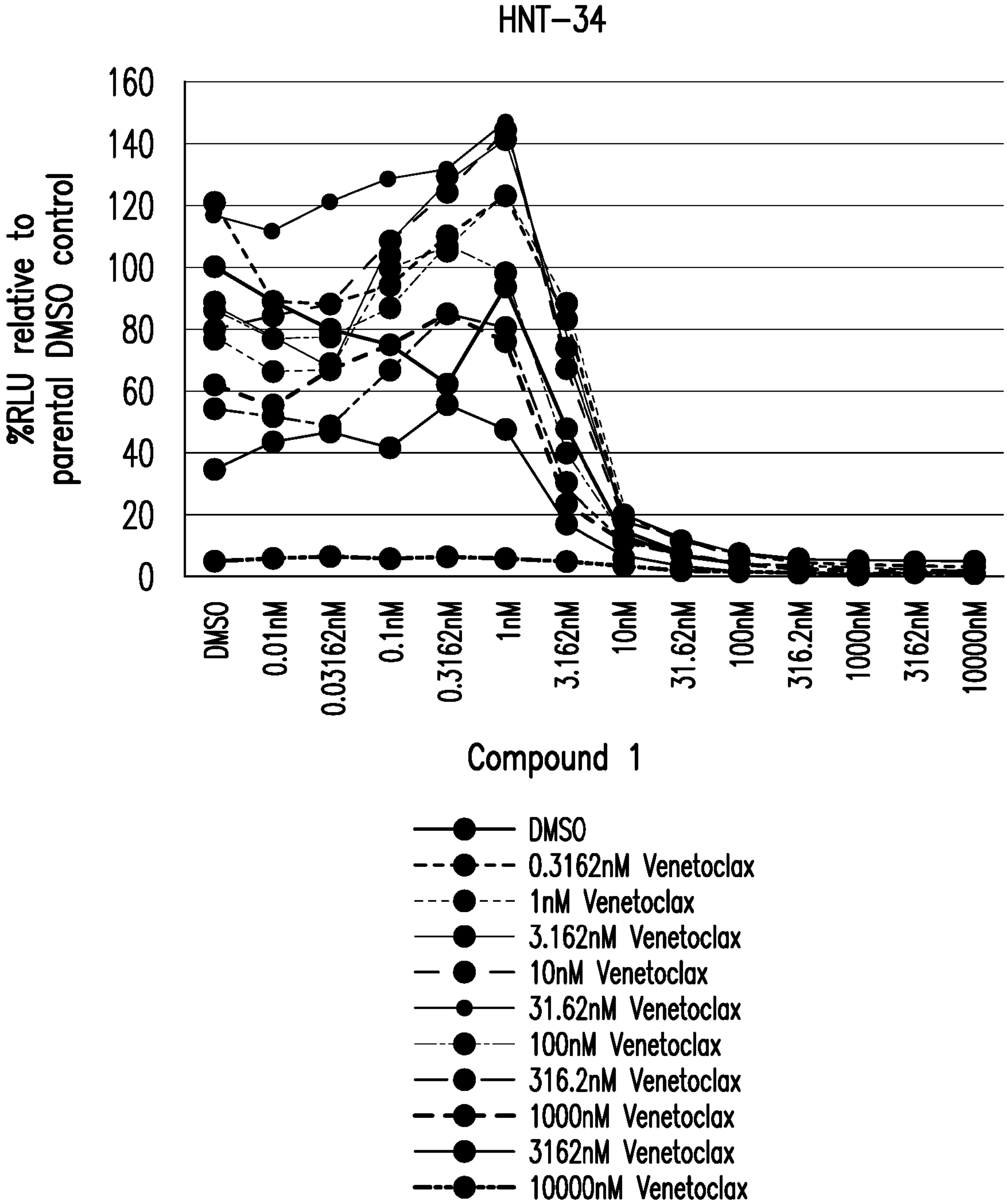
HNT-34
160
140
120
100
80
60
40
20
0
%RLU relative to parental DMSO control
DMSO
0.01nM
0.03162nM
0.1nM
0.3162nM
1nM
3.162nM
10nM
31.62nM
100nM
316.2nM
1000nM
3162nM
10000nM
Compound 1
DMSO
0.3162nM Venetoclax
1nM Venetoclax
3.162nM Venetoclax
10nM Venetoclax
31.62nM Venetoclax
100nM Venetoclax
316.2nM Venetoclax
1000nM Venetoclax
3162nM Venetoclax
10000nM Venetoclax

FIG. 102G

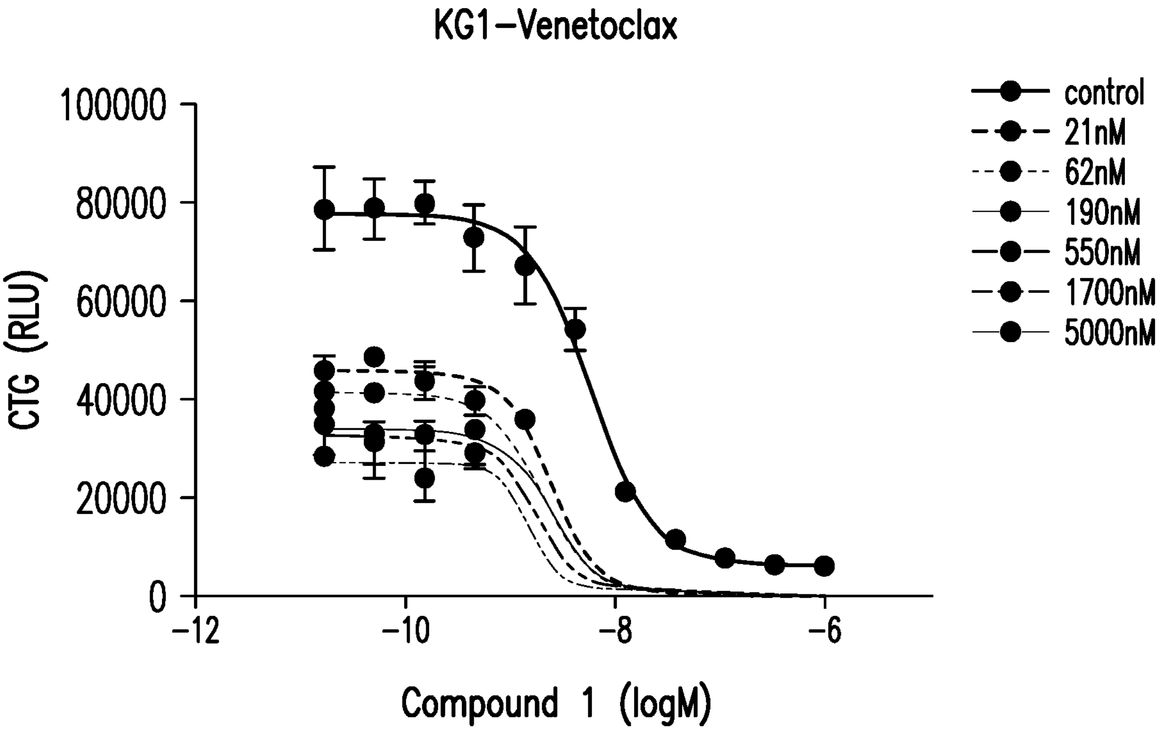
KG1-Venetoclax
CTG (RLU)
100000
80000
60000
40000
20000
0
-12
-10
-8
-6
Compound 1 (logM)
control
21nM
62nM
190nM
550nM
1700nM
5000nM

FIG. 103

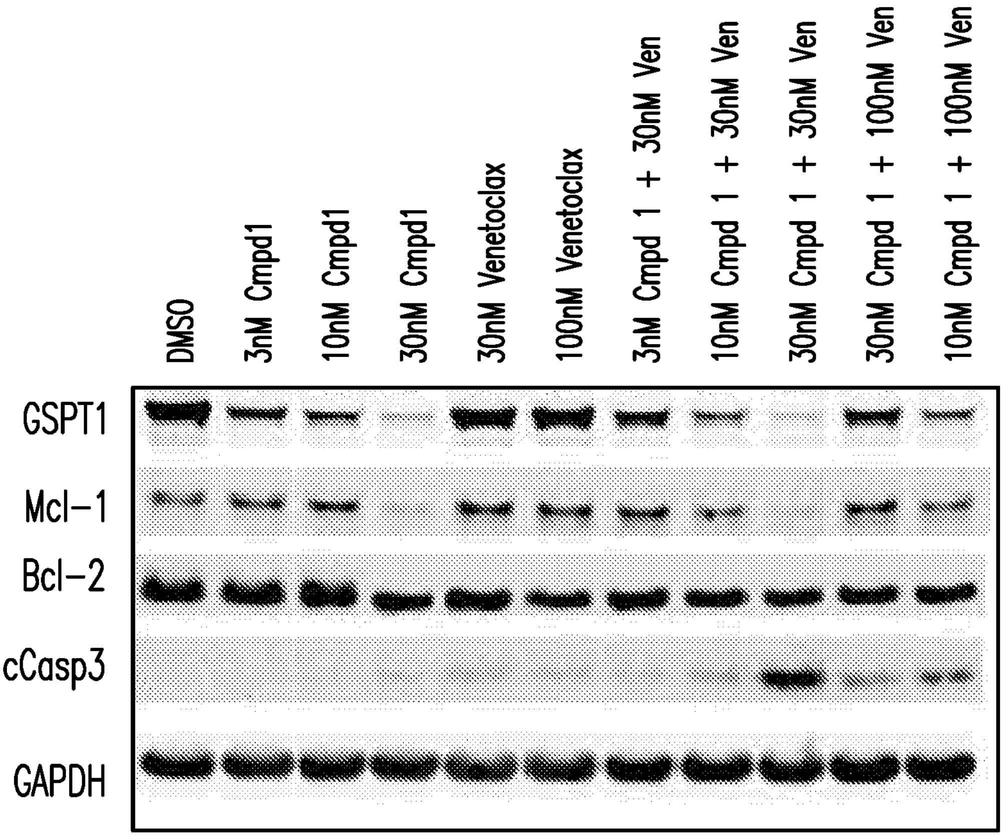
DMSO
3nM Cmpd1
10nM Cmpd1
30nM Cmpd1
30nM Venetoclax
100nM Venetoclax
3nM Cmpd 1 + 30nM Ven
10nM Cmpd 1 + 30nM Ven
30nM Cmpd 1 + 30nM Ven
30nM Cmpd 1 + 100nM Ven
10nM Cmpd 1 + 100nM Ven
GSPT1
Mcl-1
Bcl-2
cCasp3
GAPDH

FIG. 104

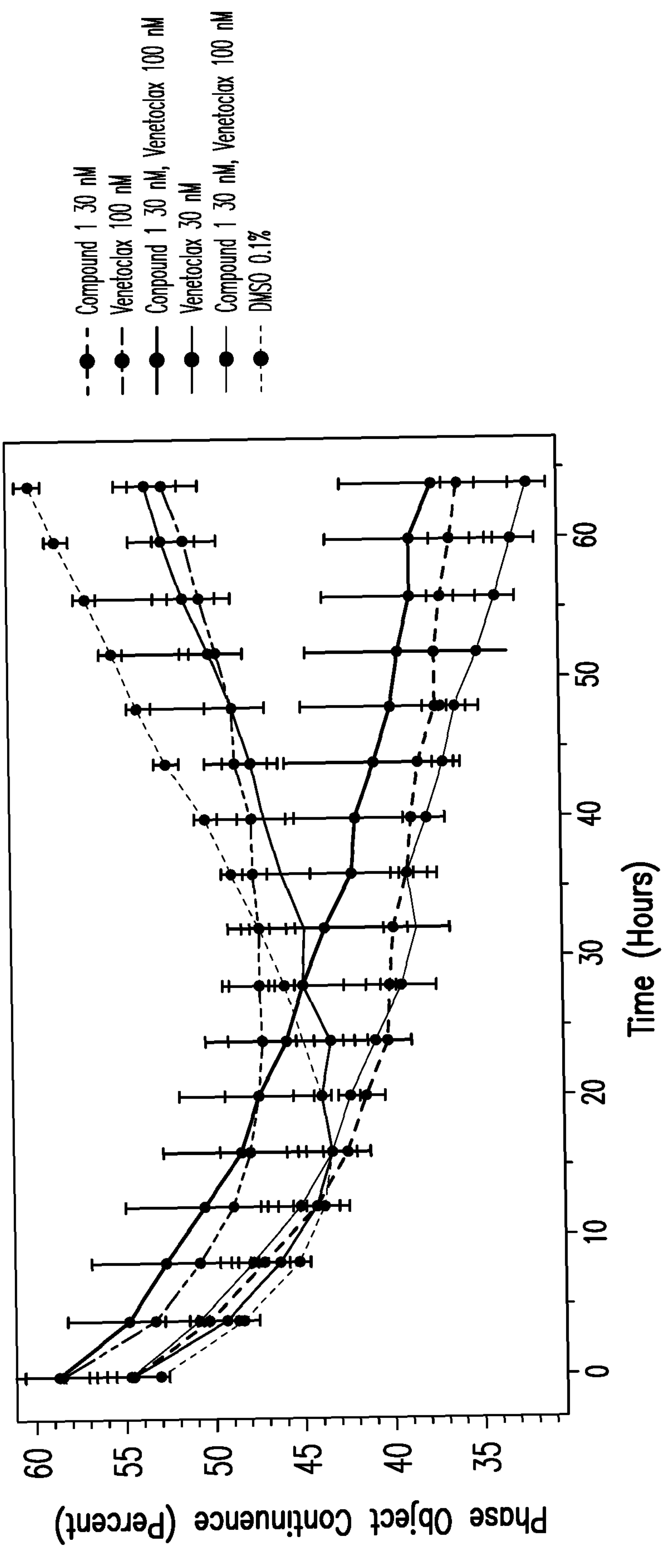
Compound 1 30 nM
Venetoclax 100 nM
Compound 1 30 nM, Venetoclax 100 nM
Venetoclax 30 nM
Compound 1 30 nM, Venetoclax 100 nM
DMSO 0.1%
Phase Object Confluence (Percent)
60
55
50
45
40
35
0
10
20
30
40
50
60
Time (Hours)

FIG. 105A

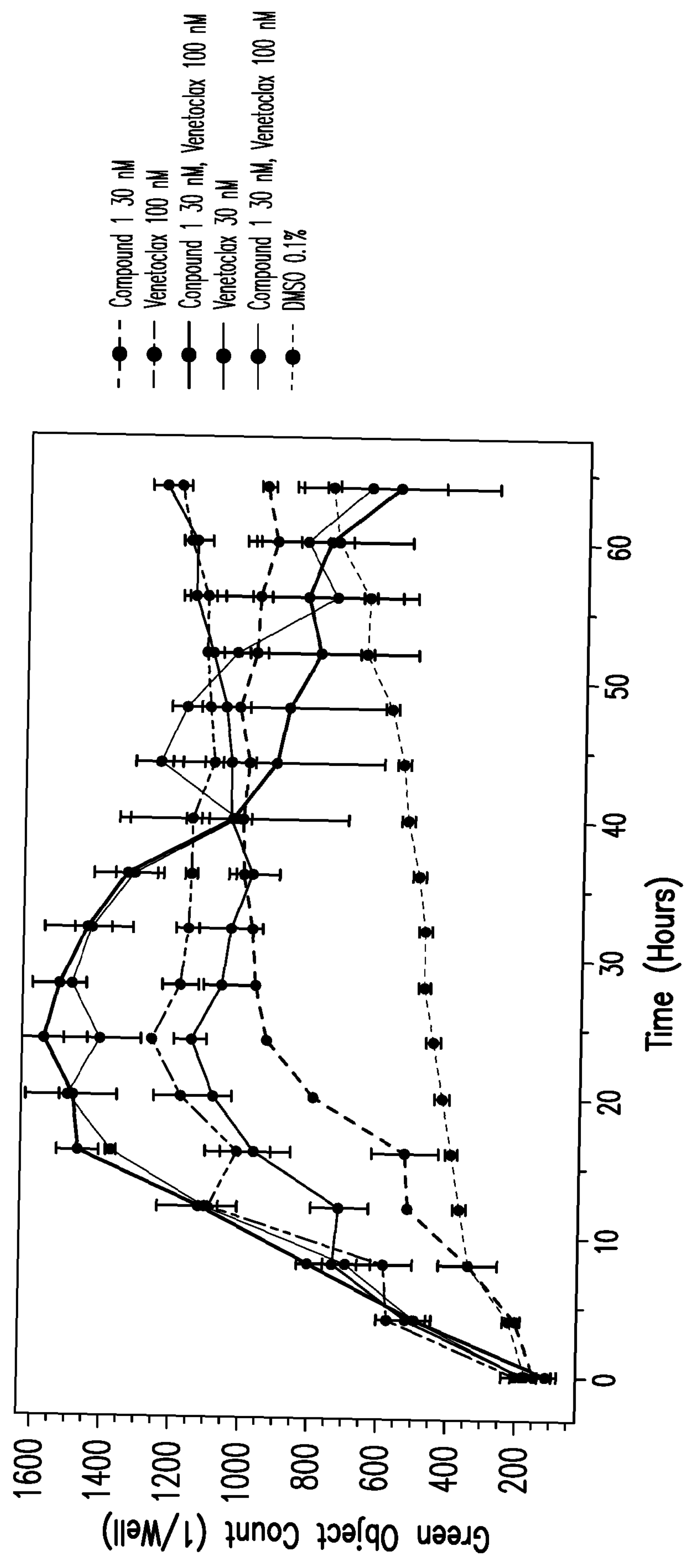
Compound 1 30 nM
Venetoclax 100 nM
Compound 1 30 nM, Venetoclax 100 nM
Venetoclax 30 nM
Compound 1 30 nM, Venetoclax 100 nM
DMSO 0.1%
Green Object Count (1/Well)
1600
1400
1200
1000
800
600
400
200
0
10
20
30
40
50
60
Time (Hours)

FIG. 105B

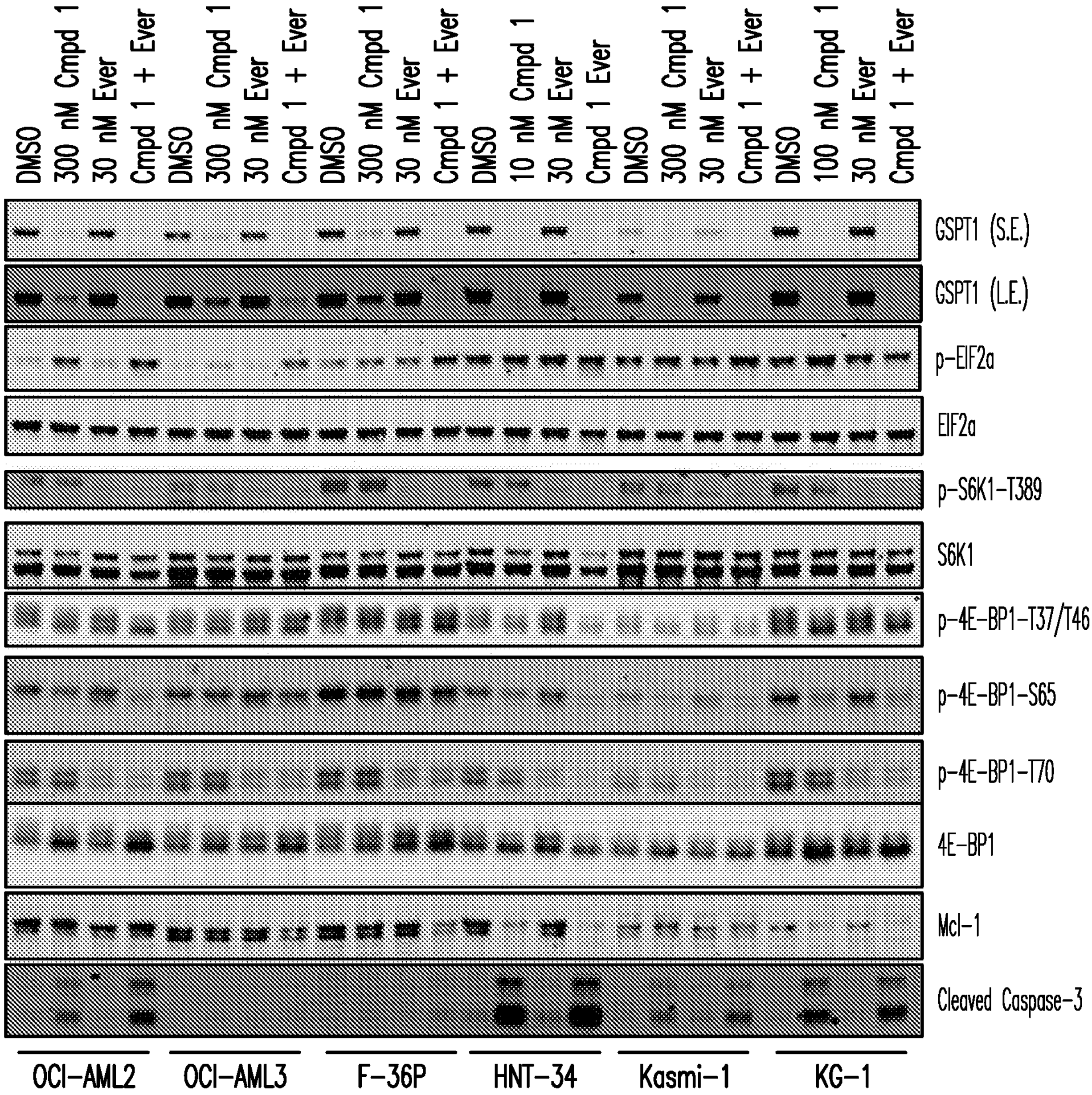
DMSO
300 nM Cmpd 1
30 nM Ever
Cmpd 1 + Ever
DMSO
300 nM Cmpd 1
30 nM Ever
Cmpd 1 + Ever
DMSO
300 nM Cmpd 1
30 nM Ever
Cmpd 1 + Ever
DMSO
10 nM Cmpd 1
30 nM Ever
Cmpd 1 Ever
DMSO
300 nM Cmpd 1
30 nM Ever
Cmpd 1 + Ever
DMSO
100 nM Cmpd 1
30 nM Ever
Cmpd 1 + Ever
GSPT1 (S.E.)
GSPT1 (L.E.)
p-EIF2a
EIF2a
p-S6K1-T389
S6K1
p-4E-BP1-T37/T46
p-4E-BP1-S65
p-4E-BP1-T70
4E-BP1
Mcl-1
Cleaved Caspase-3
OCI-AML2
OCI-AML3
F-36P
HNT-34
Kasmi-1
KG-1

FIG. 106

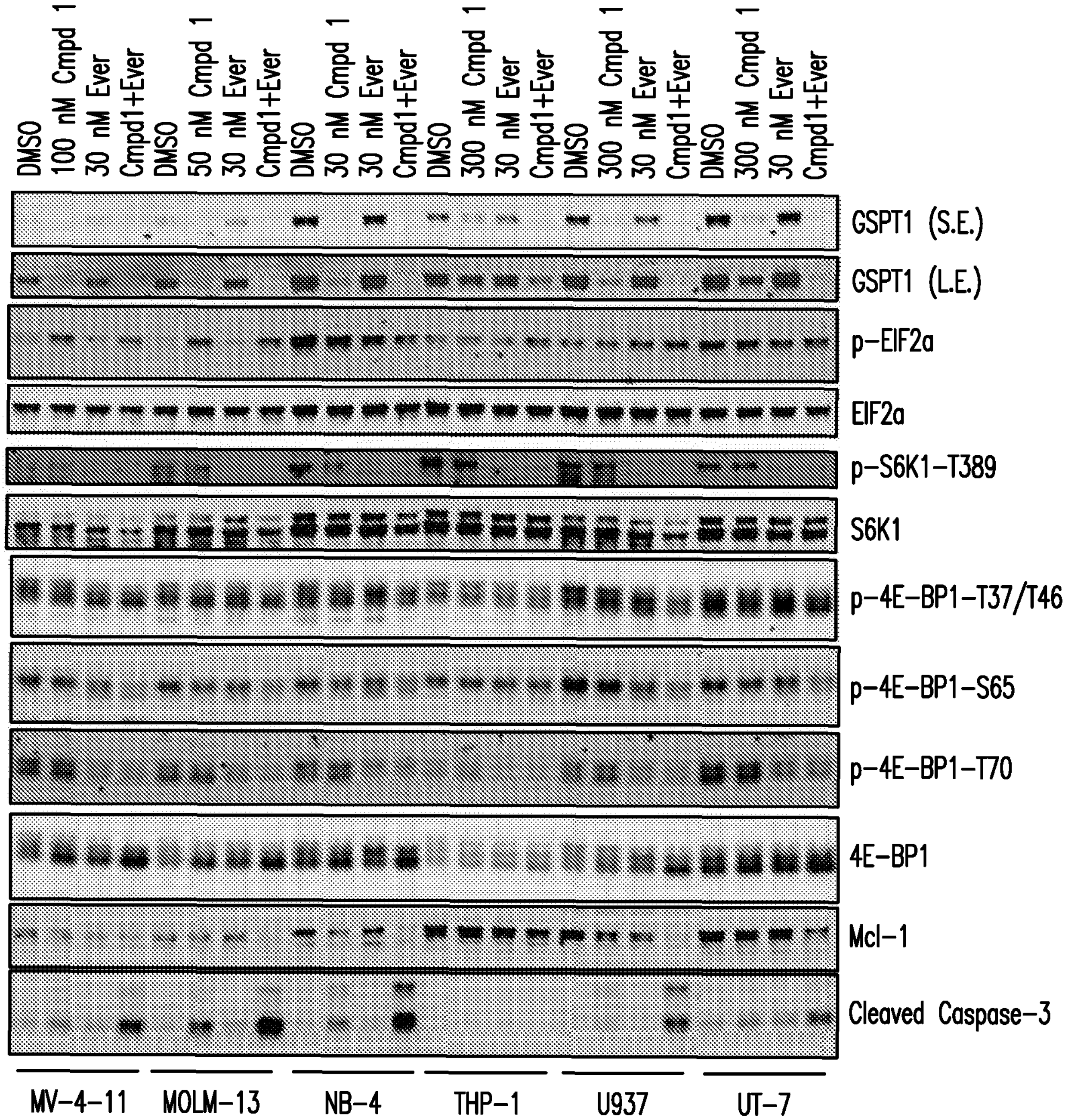


FIG. 106 (continued)

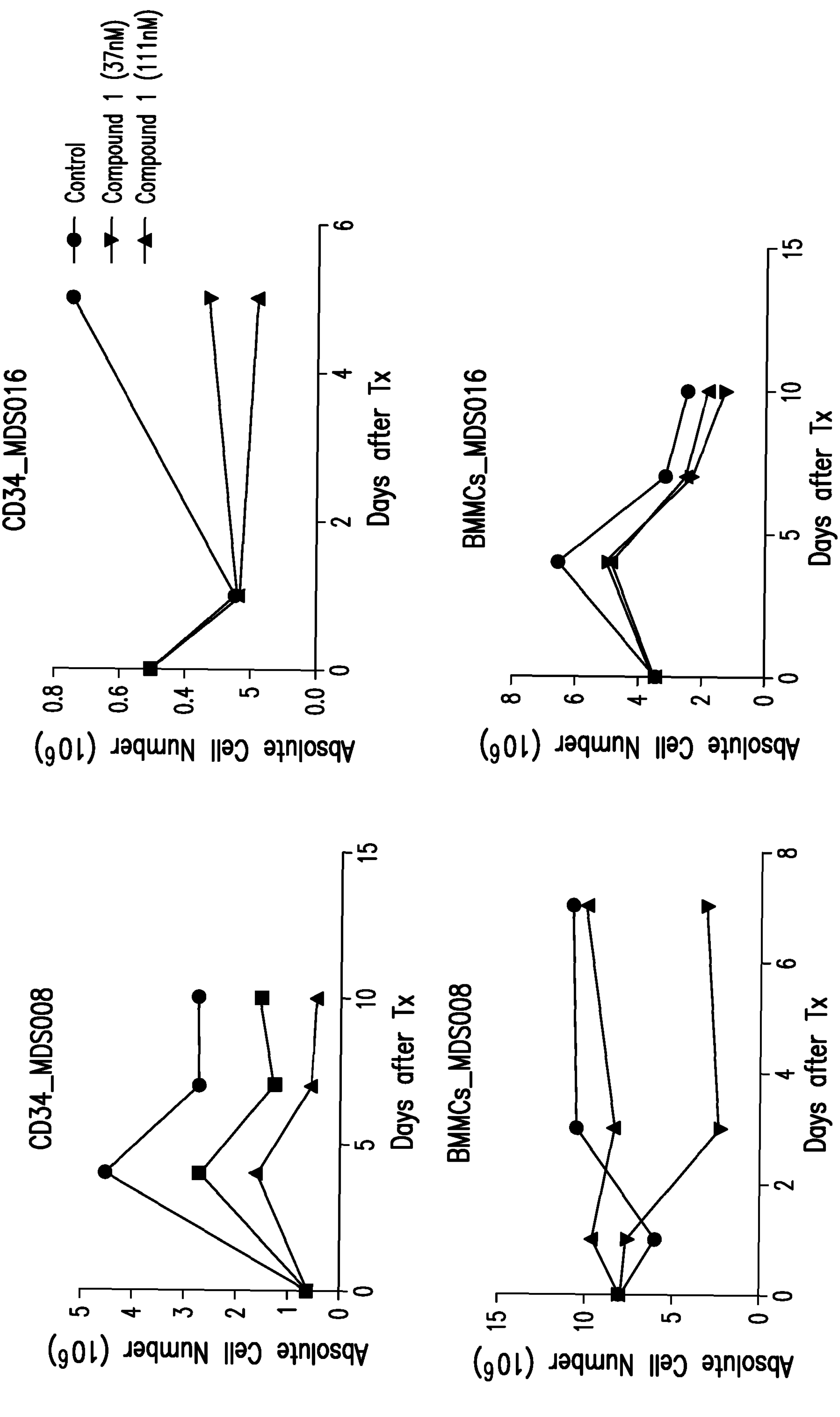
CD34_MDS008
Absolute Cell Number (10^6)
Days after Tx
CD34_MDS016
Absolute Cell Number (10^6)
Days after Tx
Control
Compound 1 (37nM)
Compound 1 (111nM)
BMMCs_MDS008
Absolute Cell Number (10^6)
Days after Tx
BMMCs_MDS016
Absolute Cell Number (10^6)
Days after Tx

FIG. 107

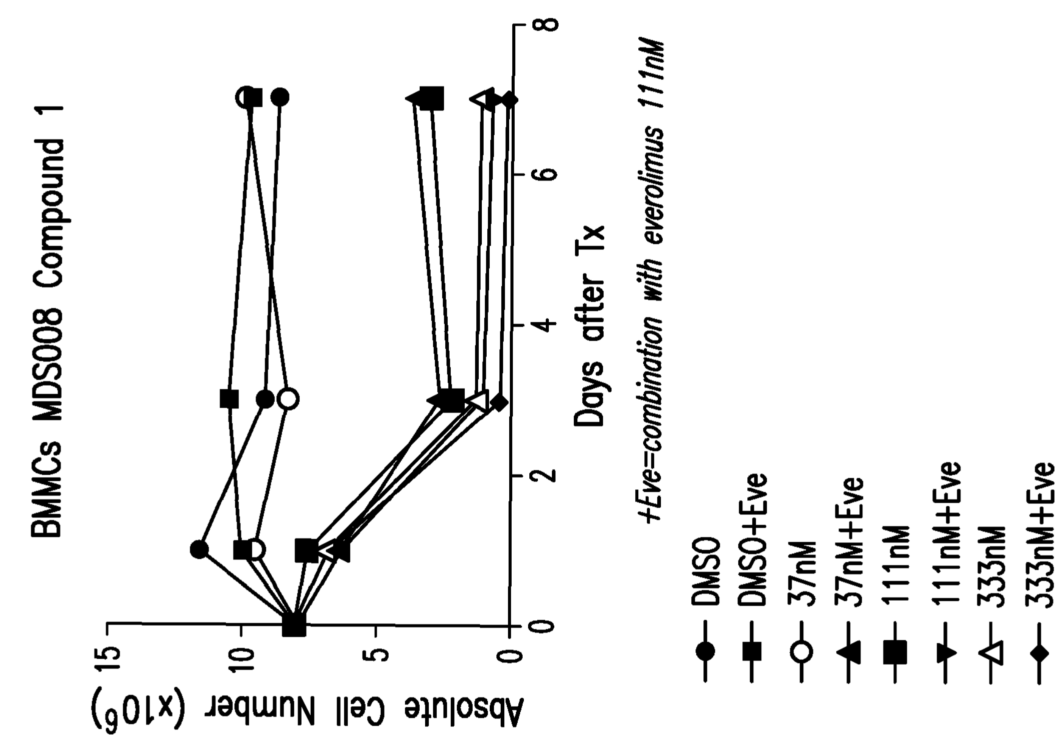


FIG. 108A

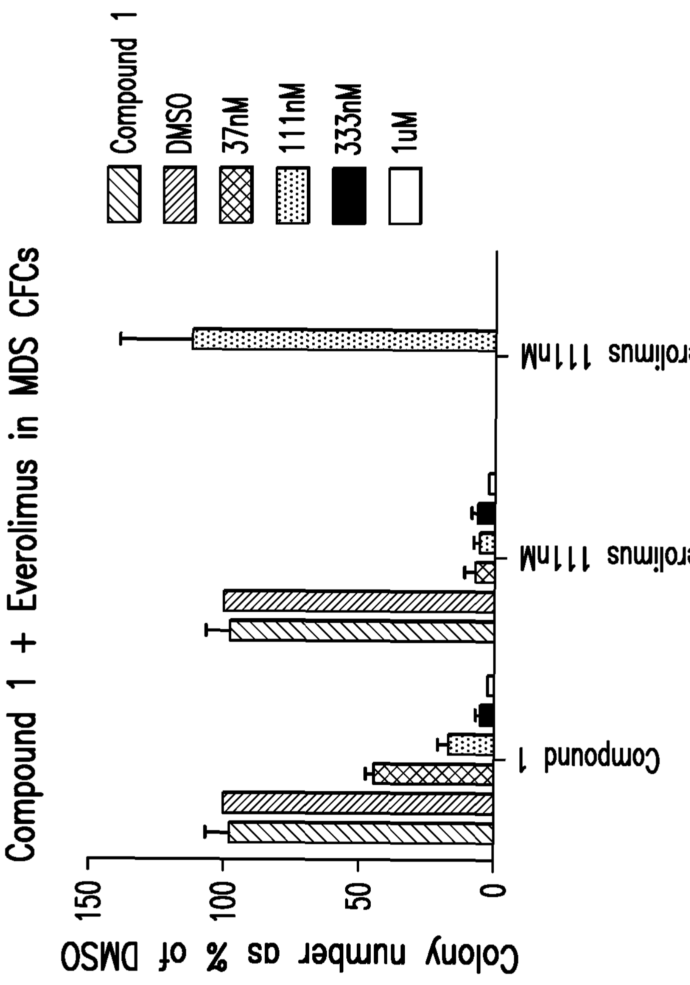


FIG. 108B

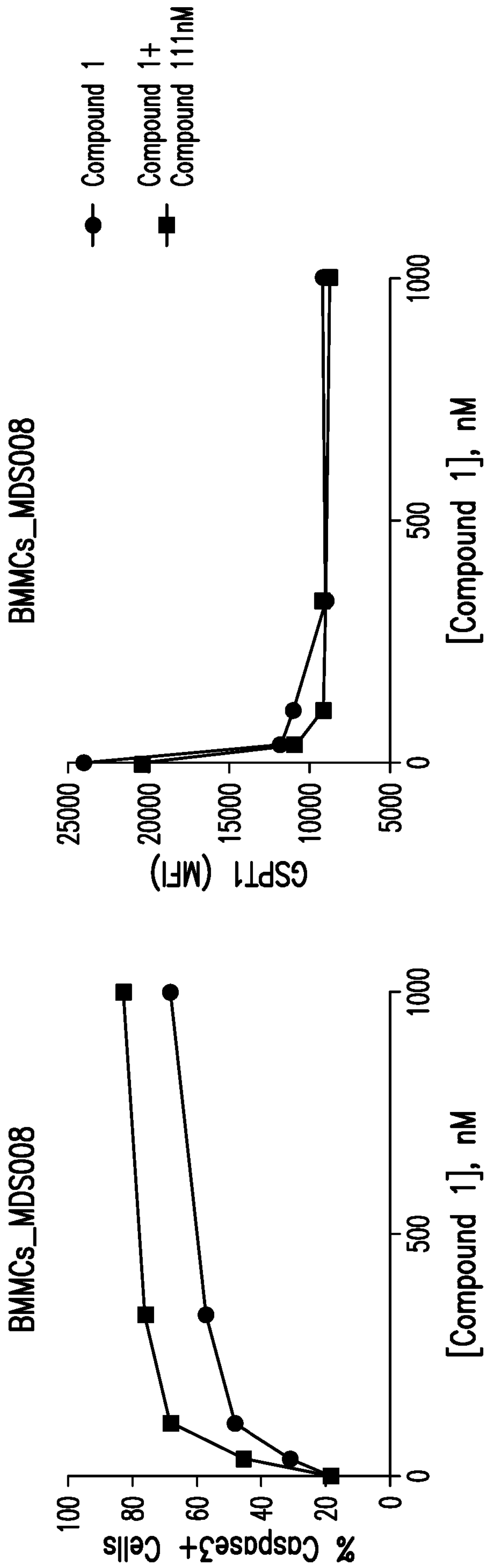
BMMCs_MDS008
% Caspase3+ Cells
[Compound 1], nM
BMMCs_MDS008
GSPT1 (MFI)
[Compound 1], nM
Compound 1
Compound 1+
Compound 111nM

FIG. 109

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62527744
- US 62653436 B
- US 62673064 B
- US 20170196847 A
- US 9499514 B
- WO 2016007848 A
- US 20170197934 A
- US 5391485 A
- US 5393870 A
- US 5229496 A
- US 4810643 A
- US 4999291 A
- US 5528823 A
- US 5580755 A
- US 5811097 A
- US 5855887 A
- US 6051227 A
- US 6207157 B
- US 6682736 B
- US 6984720 B
- US 7605238 B
- US 7488802 B
- US 7943743 B
- US 8008449 B
- US 8168757 B
- US 8217149 B
- WO 2003042402 A
- WO 2008156712 A
- WO 2010089411 A
- WO 2010036959 A
- WO 2011066342 A
- WO 2011159877 A
- WO 2011082400 A
- WO 2011161699 A
- US 5948893 A
- US 6534055 B
- US 6352694 B
- US 6692964 B
- US 6887466 B
- US 6905681 B
- US 9732062 B
- US 9724350 B
- US 9738625 B
- US 9579324 B
- US 20160159771 A
- US 20160158230 A1
- US 7498171 B
- US 20170196847 A1

### Non-patent literature cited in the description


- 11. *Biochem.,* 1972, 942-944
- **ROWE R.C. ; SHESKY P.J. ; QUINN M.E.** Handbook of Pharmaceutical Excipients. The Pharmaceutical Press, RPS Publishing, 2009
- **PASCHKA P et al.** *Blood,* 2013, vol. 121, 170-177
- **CATENACCI et al.** *Blood Rev,* 2005, vol. 19, 301-319
- **ARBER et al.** *Blood,* 2016, vol. 127 (20), 2391-2405
- **VARDIMAN et al.** *Blood.,* 2009, vol. 114 (5), 937-51
- **OKEN M et al.** Toxicity and response criteria of the Eastern Cooperative Oncology Group. *Am J Clin Oncol,* 1982, vol. 5 (6), 649-655
- **CHESON et al.** *J Clin Oncol,* 2003, vol. 21 (24), 4642-9
- **CHESON BD et al.** *J. Clin. Oncol,* 2007, (25), 579-586
- **HALLEK M et al.** *Blood,* 2008, vol. 12 (111), 5446-5456
- **DURIE et al.** *Leukemia,* 2006, vol. 10 (10), 1-7
- **THEREASSE P. et al.** *J. of the National Cancer Institute,* 2000, (92), 205-216
- **EISENHAUER et al.** *European J. Cancer,* 2009, (45), 228-247
- **CHESON et al.** *Blood,* 2006, vol. 108 (2), 419-25
- **GREENBURG et al.** *Blood.,* 2012, vol. 120 (12), 2454-65
- **GREENBURG et al.** *Blood,* 2012, vol. 120 (12), 2454-65
- **GREENBERG et al.** *Blood.,* 2012, vol. 120 (12), 2454-65
- **GUIDELINES.** *Journal of the National Cancer Institute,* 2000, vol. 92 (3), 205-216
- **PENICHET, M.L. ; MORRISON, S.L.** *J. Immunol. Methods,* 2001, vol. 248, 91-101

- **EMENS, L.A. et al.** *, Curr. Opinion Mol. Ther.,* 2001, vol. 3 (1), 77-84
- **PARDOLL.** *Nature Reviews Cancer,* 2012, vol. 12, 252-264
- **BRIGNONE et al.** *J. Immunol.,* 2007, vol. 179, 4202-4211
- **LOO et al.** *, Clin. Cancer Res.,* 2012, 3834
- **FOURCADE et al.** *J. Exp. Med.,* 2010, vol. 207, 2175-86
- **SAKUISHI et al.** *J. Exp. Med.,* 2010, vol. 207, 2187-94
- **S. ANGUILLE et al.** *Leukemia,* 2012, vol. 26, 2186-2196
- **STRAATHOF et al.** *, Blood,* 2005, vol. 105 (11), 4247-4254
- **A. GOPALSAMY et al.** *, Bioorg. Med Chem Lett.,* 2012, vol. 22, 6636-66412
- **FOUCQUIER.** *Pharma Res Per,* 2015, vol. 3 (3
- **VEROLI G.** *Bioinformatics,* 2016, vol. 32 (18
- **GEARY N.** *Am J Physiol Endocrinol Metab,* 2012
- *Leukemia,* 2017, vol. 31, 393-402
- *Blood,* 2011, vol. 118, 5162
- **SIMON et al.** *J Natl Cancer Inst,* 1997, vol. 89 (15), 1138-47
- **CHESON BD et al.** *Blood,* 2006, vol. 108 (2), 419-25